(19) Europäisches Patentamt
European Patent Office
Office européen des brevets

(11) EP 3 159 414 A1

(12) EUROPEAN PATENT APPLICATION

(43) Date of publication:
**26.04.2017 Bulletin 2017/17**

(21) Application number: **16201986.3**

(22) Date of filing: **12.02.2007**

(51) Int Cl.:
***C12N 15/85*** *(2006.01)*
***A01K 67/033*** *(2006.01)*
***C12N 15/90*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.02.2006 US 352177**
**25.10.2006 GB 0621234**

(62) Document number(s) of the earlier application(s) in accordance with Art. 76 EPC:
**07712717.3 / 1 984 512**

(71) Applicant: **Oxitec Limited**
**Oxfordshire OX14 4RX (GB)**

(72) Inventor: **ALPHEY, Luke**
**Abingdon, Oxfordshire OX14 4RX (GB)**

(74) Representative: **Zvesper, Thomas**
**Marks & Clerk LLP**
**90 Long Acre**
**London WC2E 9RA (GB)**

Remarks:
This application was filed on 02-12-2016 as a divisional application to the application mentioned under INID code 62.

(54) **EXPRESSION SYSTEM**

(57) A polynucleotide expression system is provided that is capable of alternative splicing of RNA transcripts of a polynucleotide sequence to be expressed in an organism.

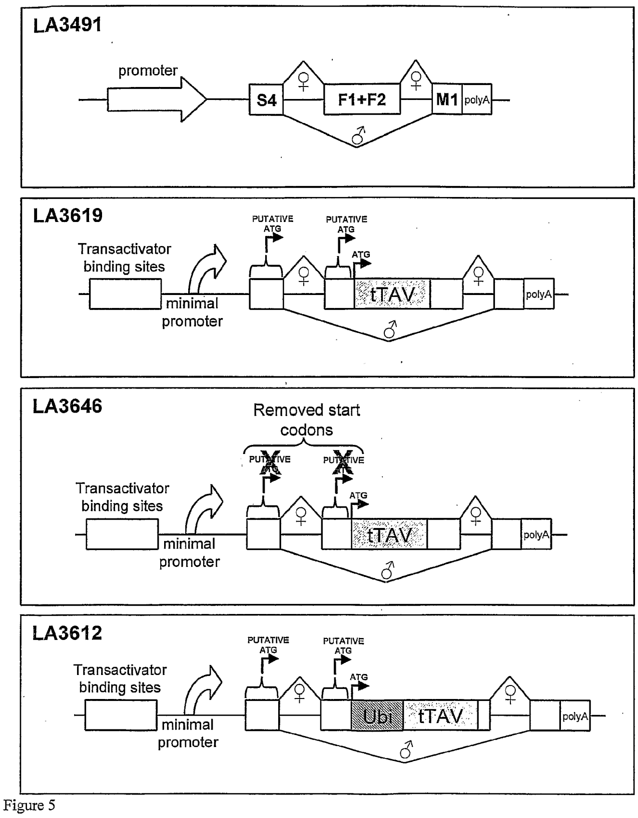


Figure 5



Printed by Jouve, 75001 PARIS (FR)

## Description

All references cited herein are hereby incorporated by reference, unless otherwise apparent.

## INTRODUCTION

The present invention relates to a gene expression system, in combination with splice control sequences, said control sequences providing a mechanism for alternative splicing.

Alternative splicing involves the removal of one or more introns and ligation of the flanking exons. This reaction is catalyzed by the spliceosome, a macromolecular machine composed of five RNAs and hundreds of proteins (Jurica, M. S. & Moore, M. J. (2003) Mol. Cell 12, 5-14). Alternative splicing generates multiple mRNAs from a single gene, thus increasing proteome diversity (Graveley, B. R. (2001) Trends Genet. 17, 100-107).

Alternative splicing also plays a key role in the regulation of gene expression in many developmental processes ranging from sex determination to apoptosis (Black, D. L. (2003) Annu. Rev. Biochem. 72, 291-336), and defects in alternative splicing have been linked to many human disorders (Caceres, J. F. & Kornblihtt, A. R. (2002) Trends Genet. 18, 186-193). In general, alternative splicing is regulated by proteins that associate with the pre-mRNA and function to either enhance or repress the ability of the spliceosome to recognize the splice site(s) flanking the regulated exon (Smith, C. W. & Valcarcel, J. (2000) Trends Biochem. Sci. 25, 381-388).

Whether a particular alternative exon will be included or excluded from a mature RNA in each cell is thought to be determined by the relative concentration of a number of positive and negative splicing regulators and the interactions of these factors with the pre-mRNA and components of the spliceosome (Smith, C. W. & Valcarcel, J. (2000) Trends Biochem. Sci. 25, 381-388).

Spliceosomes are large complexes of small nuclear RNA and protein particles (snRNPs) which assemble with pre-mRNA to achieve RNA splicing, by removing introns from eukaryotic nuclear RNAs, thereby producing mRNA which is then translated to protein in ribosomes.

Although at least 74% of human genes encode alternatively spliced mRNAs (Johnson, J. M., Castle, J., Garrett-Engele, P., Kan, Z., Loerch, P. M., Armour C. D., Santos, R., Schadt, E. E., Stoughton, R. & Shoemaker, D. D. (2003) Science 302, 2141-2144), relatively few splicing regulators have been identified.

## SUMMARY OF THE INVENTION

Thus, in a first aspect, the present invention provides a polynucleotide expression system comprising:

at least one heterologous polynucleotide sequence encoding a functional protein, defined between a start codon and a stop codon, and/or polynucleotides for interference RNA (RNAi), to be expressed in an organism;
at least one promoter operably linked thereto; and
at least one splice control sequence which, in cooperation with a spliceosome, is capable of (i) mediating splicing of an RNA transcript of the coding sequence to yield a first spliced messenger RNA (mRNA) product, and (ii) mediating at least one alternative splicing of said RNA transcript to yield an alternative spliced mRNA product;

wherein, when the at least one heterologous polynucleotide sequence encodes a functional protein, at least one of the mature mRNA products comprising a continuous Open Reading Frame (ORF) extending from said start codon to said stop codon, thereby defining a protein, which is said functional protein , or is related to said functional protein by at least one amino acid deletion, and which is functional when translated and, optionally, has undergone post-translational modification;
the mediation being selected from the group consisting of: sex-specific mediation, stage-specific mediation, germline-specific mediation, tissue-specific mediation, and combinations thereof.

The expression system may be DNA or RNA or a hybrid or combination of both. It is envisaged that the system comprises both ribo- and deoxy-ribonucleotides, i.e. portions of DNA and portions of RNA. These could correspond to different genetic elements, such that the system is a DNA/RNA hybrid, with some functional elements provided by DNA and others by RNA.

Preferably, the mediation is in a sex-specific, stage-specific, germline-specific or tissue-specific manner. In particular, sex-specific mediation is particularly preferred. However, it is also preferred that a combination of these four manners of mediation can be utilised. It is particularly preferred that, when a combination of these modes is used, that this includes sex-specific mediation. A particularly preferred example of such a combination is a combination of sex-specific, tissue-specific and stage-specific mediation of alternative splicing.

The system may be adapted for expression of a gene. Preferably, the polynucleotide sequence to be expressed comprises a coding sequence for a protein or polypeptide, i.e. at least one exon, and preferably 2 or more exons, capable of encoding a polypeptide, such as a protein or fragment thereof.

It will be understood that an exon is any region of DNA within a gene, that is present in a mature RNA molecule derived from that gene, rather than being spliced out from the transcribed RNA molecule. For protein coding genes, mature RNA molecules correspond to mature mRNA molecules, which may encode one or more proteins or polypeptides. Exons of many eukaryotic genes interleave with segments of non-coding DNA.

The at least one heterologous polynucleotide sequence may encode a functional protein, defined between a start codon and a stop codon to be expressed in an organism. Alternatively, or in addition, the at least one heterologous polynucleotide sequence encodes or comprises polynucleotides for interference RNA (RNAi), to be expressed in an organism.

These sequences, to be expressed in the organism, may also be referred to as sequences, the expression of which is to be regulated in said organism.

Preferably, the polynucleotide sequence to be expressed comprises two or more coding exons, being segments or sequences of polynucleotides that encode amino acids when translated from mRNA. Preferably, the different exons are differentially spliced together to provide alternative mRNAs. Preferably, said alternative spliced mRNAs have different coding potential, i.e. encode different proteins or polypeptide sequences. Thus, the expression of the coding sequence is regulated by alternative splicing in the above-mentioned manners of mediation.

The polynucleotide sequence to be expressed may comprise polynucleotides for interference RNA (RNAi). Such sequences are capable of providing, for instance, one or more stretches of double-stranded RNA (dsRNA), preferably in the form of a primary transcript, which in turn is capable of processing by the RNA Pol III-like enzyme "Dicer." Such stretches include, for instance, stretches of single-stranded RNA that can form loops, such as those found in short-hairpin RNA (shRNA), or with longer regions that are substantially self-complementary.

Thus, where the system is DNA, the polynucleotides for interference RNA are deoxyribonucleotides that, when transcribed into pre-RNA ribonucleotides, provide a stretch of dsRNA, as discussed above.

Polynucleotides for interference RNA are particularly preferred when said polynucleotides are positioned to minimise interference with alternative splicing. This may be achieved by distal positioning of these polynucleotides from the alternative splicing control sequences, preferably 3' to the control sequences. In another preferred embodiment, substantially self-complementary regions may be separated from each other by one or more splice control sequences, such as an intron, that mediate alternative splicing. Preferably, the self-complementary regions are arranged as a series of two or more inverted repeats, each inverted repeat separated by splice control sequence, preferably an intron, as defined elsewhere.

In this configuration, different alternatively spliced transcripts may have their substantially self-complementary regions separated by different lengths of non-self-complementary sequence in the mature (post-alternative-splicing) transcript. It will be appreciated that regions that are substantially self-complementary are those that are capable of forming hairpins, for instance, as portions of the sequence are capable of base-pairing with other portions of the sequence. These two portions do not have to be exactly complementary to each other, as there can be some mismatching or toleration of stretches in each portion that do not base-pair with each other. Such stretches may not have an equivalent in the other portion, such that symmetry is lost and "bulges" form, as is known with base-pair complementation in general.

In another preferred embodiment, one or more segment of sequence substantially complementary to another section of the primary transcript is positioned, relative to the at least one splice control sequence, so that it is not included in all of the transcripts produced by alternative splicing of the primary transcript. By this method, some transcripts are produced that tend to produce dsRNA while others do not; by mediation of the alternative splicing, e.g. sex-specific mediation, stage-specific mediation, germline-specific mediation, tissue-specific mediation, and combinations thereof, dsRNA may be produced in a sex-specific, stage-specific, germline-specific or tissue-specific manner, or combinations thereof.

The system is preferably capable of expressing at least one protein of interest, i.e. said functional protein to be expressed in an organism. Said at least one protein of interest may have a therapeutic effect or may, preferably, be a marker, for instance DsRed, Green Fluorescent Protein (GFP) or one or more of their mutants or variants, or other markers that are well known in the art.

Most preferably, the functional protein to be expressed in an organism has a lethal, deleterious or sterilizing effect. Where reference is made herein to a lethal effect, it will be appreciated that this extends to a deleterious or sterilizing effect, such as an effect capable of killing the organism *per se* or its offspring, or capable of reducing or destroying the function of certain tissues thereof, of which the reproductive tissues are particularly preferred, so that the organism or its offspring are sterile. Therefore, some lethal effects, such as poisons, will kill the organism or tissue in a short time-frame relative to their life-span, whilst others may simply reduce the organism's ability to function, for instance reproductively.

A lethal effect resulting in sterilization is particularly preferred, as this allows the organism to compete in the natural environment ("in the wild") with wild-type organisms, but the sterile insect cannot then produce viable offspring. In this way, the present invention achieve a similar result to techniques such as the Sterile Insect Technique (SIT) in insects, without the problems associated with SIT, such as the cost, danger to the user, and reduced competitiveness of the irradiated organism.

Preferably, the system comprises at least one positive feedback mechanism, namely at least functional protein to be differentially expressed, via alternative splicing, and at least one promoter therefor, wherein a product of a gene to be expressed serves as a positive transcriptional control factor for the at least one promoter, and whereby the product, or the expression of the product, is controllable. Preferably, an enhancer is associated with the promoter, the gene product serving to enhance activity of the promoter *via* the enhancer. Preferably, the control factor is the tTA gene product or an analogue thereof, and wherein one or more tetO operator units is operably linked with the promoter and is the enhancer, tTA or its analogue serving to enhance activity of the promoter *via* tetO. It is preferred that functional protein encodes the tTAV or tTAF product and preferably, the promoter is substantially inactive in the absence of the positive transcriptional control factor. Suitable, preferably minimal, promoters for this system can be selected from: hsp70, a P minimal promoter, a CMV minimal promoter, an Act5C-based minimal promoter, a BmA3 promoter fragment, a promoter fragment from hunchback, an Adh core promoter, and an Act5C minimal promoter, or combinations thereof.

In one embodiment, the functional protein is preferably an apoptosis-inducing factor, such as the AIF protein described for instance in Candé et al (Journal of Cell Science 115, 4727-4734 (2002)) or homologues thereof. AIF homologues are found in mammals and even in invertebrates, including insects, nematodes, fungi, and plants, meaning that the AIF gene has been conserved throughout the eukaryotic kingdom. Also preferred is Hid, the protein product of the *head involution defective* gene of *Drosophila melanogaster,* or Reaper (Rpr), the product of the *reaper* gene of *Drosophila,* or mutants thereof. Use of Hid was described by Heinrich and Scott (Proc. Natl Acad. Sci USA 97, 8229-8232 (2000). Use of a mutant derivative, $Hid^{Ala5}$ was described by Horn and Wimmer (Nature Biotechnology 21, 64-70 (2003)). Use of a mutant derivative of Rpr, $Rpr^{KR}$, is described herein (see also White et al 1996, Wing et al., 2001, and Olson et al., 2003). Both Rpr and Hid are pro-apoptotic proteins, thought to bind to IAP1. IAP1 is a well-conserved anti-apoptotic protein. Hid and Rpr are therefore expected to work across a wide phylogenetic range (Huang et al., 2002, Vernooy et al., 2000) even though their own sequence is not well conserved.

Also preferred is Nipp1Dm, the *Drosophila* homologue of mammalian Nipp1 (Parker et al Biochemical Journal 368, 789-797 (2002); Bennett et al., Genetics 164, 235-245 (2003)). Nipp1Dm is another example of a protein with lethal effect if expressed at a suitable level, as would be understood by the skilled person. Indeed, many other examples of proteins with a lethal effect will be known to the person skilled in the art.

It is also preferred that the functional protein itself a transcriptional transactivator, such as the tTAV system described above.

It is preferred that the promoter can be activated by environmental conditions, for instance the presence or absence of a particular factor such as tetracycline in the *tet* system described herein, such that the expression of the gene of interest can be easily manipulated by the skilled person. Alternatively, a preferred example of a suitable promoter is the *hsp70* heat shock promoter, allowing the user to control expression by variation of the environmental temperature to which the hosts are exposed in a lab or in the field, for instance. Another preferred example of temperature control is described in Fryxell and Miller (Journal of Economic Entomology 88, 1221-1232 (1995)).

Also preferred as a promoter is the *sry*$\alpha$ embryo-specific promoter (Horn & Wimmer (2003) from *Drosophila melanogaster,* or its homologues, or promoters from other embryo-specific or embryo-active genes, such as that of the *Drosophila* gene *slow as molasses (slam),* or its homologues from other species.

It is also preferred that the system comprises other upstream, 5' factors and/or downstream 3' factors for controlling expression. Examples include enhancers such as the fat-body enhancers from the *Drosophila* yolk protein genes, and the *homology region* (hr) enhancers from baculoviruses, for example AcMNPV. It will also be appreciated that the RNA products will include suitable 5' and 3' UTRs, for instance.

The splice control sequence allows an additional level of control of protein expression, in addition to the promoter and/or enhancer of the gene. For instance, tissue or sex-specific expression in insect embryos only would be extremely difficult by conventional methods. Promoters with this specificity are unknown, even in *Drosophila.* However, using combinatorial control according to the present invention, an embryo-specific promoter, for example *sry*$\alpha$, can be combined with a suitable alternative splicing system.

It is preferred that any combination of promoter and alternative splicing mechanism is envisaged. The promoter is preferably specific to a particular protein having a short temporal or confined spatial effect, for example a cell-autonomous effect.

Alternatively, it is preferred that the promoter may be specific for a broader class of proteins or a specific protein that has a long-term and/or wide system effect, such as a hormone, positive or negative growth factor, morphogen or other secreted or cell-surface signalling molecule. This would allow, for instance, a broader expression pattern so that a combination of a morphogen promoter with a stage-specific alternative splicing mechanism could result in the morphogen being expressed only once a certain life-cycle stage was reached, but the effect of the morphogen would still be felt (i.e. the morphogen can still act and have an effect) beyond that life-cycle stage. Preferred examples would be the morphogen/signaling molecules Hedgehog, Wingless/WNTs, TGFß/BMPs, EGF and their homologues, which are well-known evolutionarily-conserved signalling molecules.

It is also envisaged that a promoter that is activated by a range of protein factors, for instance transactivators, or which has a broad systemic effect, such as a hormone or morphogen, could be used in combination with an alternative splicing mechanism to achieve a tissue and sex-specific control or sex and stage-specific control, or other combinations of stage-, tissue, germ-line- and sex-specific control.

It is also envisaged that more than one promoter, and optionally an enhancer therefor, can be used in the present system, either as alternative means for initiating transcription of the same protein or by virtue of the fact that the genetic system comprises more than one gene expression system (i.e. more than one gene and its accompanying promoter).

In a further aspect, the present invention provides a method of transformation, comprising expressing two or more RNA molecules, derived from a single primary transcript, or substantially similar primary transcripts, by alternative splicing, said two or more RNA molecules preferably encoding different proteins or polypeptides, in an organism by contacting the organism with the expression system and preferably inducing expression of the expression system. Methods of introduction or transformation of the gene system and induction of expression are well known in the art with respect to the relevant organism.

Also provided are organisms (i.e. transformants) transformed by the present system.

Where reference to a particular nucleotide or protein sequence is made, it will be understood that this includes reference to any mutant or variant thereof, having substantially equivalent biological activity thereto. Preferably, the mutant or variant has at least 85%, preferably at least 90%, preferably at least 95%, preferably at least 99%, preferably at least 99.9%, and most preferably at least 99.99% sequence identity with the reference sequences.

The sequences provided can tolerate some sequence variation and still splice correctly. There are a few nucleotides known to be important. These are the ones required for all splicing, e.g. as shown in Figure 34 below. The initial GU and the final AG of the intron are particularly important and therefore preferred, as discussed elsewhere, though ~5% of introns start GC instead. This consensus sequence is preferred, although it applies to all splicing, not specifically to alternative splicing. In Figure 34, Pu = A or G; Py = C or U

Preferably, the system is or comprises a plasmid. As mentioned above, this can be either DNA, RNA or a mixture of both. If the system comprises RNA, then it may be preferable to reverse-translate the RNA into DNA by means of a Reverse Transcriptase. If reverse transcription is required, then the system may also comprise a coding sequence for the RT protein and a suitable promoter therefor. Alternatively, the RTase and promoter therefore may be provided on a separate system, such as a virus. In this case, the system would only be activated following infection with that virus. The need to include suitable cis-acting sequences for the reverse transcriptase or RNA-dependent RNA polymerase would be apparent to the person skilled in the art.

However, it is particularly preferred that the system is predominantly DNA and more preferably consists only of DNA, at least with respect to the sequences to be expressed in the organism.

Whilst in some embodiments the at least one heterologous polynucleotide sequence to be expressed in an organism is a polynucleotide sequence for interference RNA (RNAi), it is particularly preferred that it is a polynucleotide sequence capable off encoding a functional protein. The description will predominantly focus on polynucleotide sequences encoding a functional protein, but it will be understood that this also refers to polynucleotides for interference RNA (RNAi), unless otherwise apparent.

It will be understood that reference is made to start and stop codons between which the polynucleotide sequence to be expressed in an organism is defined, but that this does not exclude positioning of the at least one splice control sequence, elements thereof, or other sequences, such as introns, in this region. In fact, it will be apparent form the present description that the splice control sequence, can, in some embodiments, be positioned in this region.

Furthermore, the splice control sequence, for instance, can overlap with the start codon at least, in the sense that the G of the ATG can be, in some embodiments, be the initial 5' G of the splice control sequence. Thus, the term "between" can be thought of as referring to from the beginning (3' to the initial nucleotide, i.e. A) of the start codon, preferably 3' to the second nucleotide of the start codon (i.e. T), up to the 5' side of the first nucleotide of the stop codon. Alternatively, as will be apparent by a simple reading of a polynucleotide sequence, the stop codon may also be included.

The at least one heterologous polynucleotide sequence to be expressed in an organism is a heterologous sequence. By "heterologous", it would be understood that this refers to a sequence that would not, in the wild type, be normally found in association with, or linked to, at least one element or component of the at least one splice control sequence. For example, where the splice control sequence is derived from a particular organism, and the heterologous polynucleotide is a coding sequence for a protein or polypeptide, i.e. is a polynucleotide sequence encoding a functional protein, then the coding sequence could be derived, in part or in whole, from a gene from the same organism, provided that that the origin of at least some part of the transcribed polynucleotide sequence was not the same as the origin of the at least one splice control sequence. Alternatively, the coding sequence could be from a different organism and, in this context, could be thought of as "exogenous". The heterologous polynucleotide could also be thought of as "recombinant", in that the coding sequence for a protein or polypeptide are derived from different locations, either within the same genome (i.e. the genome of a single species or sub-species) or from different genomes (i.e. genomes from different species or subspecies).

Heterologous can refer to a sequence other than the splice control sequence and can, therefore, relate to the fact the promoter, and other sequences such as 5' UTR and/or 3'UTR can be heterologous to the polynucleotide sequence to be expressed in the organism, provided that said polynucleotide sequence is not found in association or operably linked to the promoter, 5' UTR and/or 3'UTR, in the wildtype, i.e. the natural context of said polynucleotide sequence, if any.

It will be understood that heterologous also applies to "designer" or hybrid sequences that are not derived from a particular organism but are based on a number of components from different organisms, as this would also satisfy the requirement that the sequence and at least one component of the splice control sequence are not linked or found in association in the wildtype, even if one part or element of the hybrid sequence is so found, as long as at least one part or element is not. Preferably, a portion of at least 50 nucleotides of the hybrid sequence is not found in association with the at least one component of the splice control sequence, more preferably 200 nucleotides and most preferably 500 nucleotides.

It will also be understood that synthetic versions of naturally occurring sequences are envisioned. Such synthetic sequences are also considered as heterologous, unless they are of identical sequence to a sequence which would, in the wild type or natural context, be normally found in association with, or linked to, at least one element or component of the at least one splice control sequence.

This applies equally to where the heterologous polynucleotide is a polynucleotide for interference RNA.

In one embodiment, where the polynucleotide sequence to be expressed comprises a coding sequence for a protein or polypeptide, it will be understood that reference to expression in an organism refers to the provision of one or more transcribed RNA sequences, preferably mature mRNAs, but this may, preferably, also refer to translated polypeptides in said organism.

RT-PCR, which demonstrates the presence of a transcript, not of a protein, may be used to identify transcribed RNA sequences. This is also particularly useful when the protein itself is not translated or is not functional or not identifiable by antibodies raised against the naturally-occurring or wildtype protein, due to RNAi, post-translational modification or distorted folding.

In another embodiment, where the polynucleotide sequence to be expressed comprises polynucleotides for interference RNA, it will also be understood that reference to expression in an organism refers to the interaction of the polynucleotides for interference RNA, or transcripts thereof, in the RNAi pathway, for instance by binding of Dicer or formation of small interfering RNA (siRNA). Indeed, it is particularly preferred that the polynucleotides for interference RNA comprise siRNA sequences and are, therefore, preferably 20-25 nucleotides long, especially where the organism is mammalian.

In insects and nematodes especially, it is preferred to provide portion of dsRNA, for instance by hairpin formation, which can then be processed by the Dicer system. Mammalian cells generally produce an interferon response against long dsRNA sequences, so for mammalian cells it is more common to provide shorter sequences, such as siRNAs. Antisense sequences or sequences having homology to microRNAs that are naturally occurring RNA molecules targeting protein 3' UTRs are also envisaged as sequences for RNAi according to an embodiment of the present invention.

Each splice control sequence in the system comprises at least one splice acceptor site and at least one splice donor site. The number of donor and acceptor sites may vary, depending on the number of segments of sequence that are to be spliced together. Preferably, branch sites are included in each splice control sequence. A branch site is the sequence to which the splice donor is initially joined, see figure 32, which shows that splicing occurs in two stages, in which the 5' exon is separated and then is joined to the 3' exon.

Referring to said figure, the A is the only essential nucleotide, and is, therefore, preferably included. Without being bound by theory, it is believed that pre-mRNA splicing proceeds via a lariat intermediate, just as it does in group II self-splicing. First, cleavage occurs at the 5' junction - sometimes called the splice donor site. The phosphate at the 5'end of the intron then becomes linked to the 2' OH of an adenine approximately 25 nucleotides upstream of the 3' end of the intron, which is sometimes called the acceptor site. This A residue is called the branch point. The next step is that cleavage occurs at the 3' splice junction and the 5' phosphate of the downstream exon is joined to the 3' OH of the upstream exon.

It is particularly preferred that the manner or mechanism of alternative splicing is sex-specific. Preferably, the splice control sequence is derived from a *tra* intron. However, it is particularly preferred that the alternative splicing mechanism is derived from the Medfly *transformer* gene *Cctra,* or from another ortholog or homolog of the *Drosophila transformer* gene, preferably from C. *rosa,* or *B. zonata* especially one derived from a tephritid fruit fly.

It is also preferred that the splice control sequence is derived from the alternative splicing mechanism of the *Actin-4* gene, in particular that from *Aedes spp.* and most preferably from *AaActin-4,* which is a gene from *Aedes*/*Stegomyia aegypti* which shows tissue, stage and sex-specific splicing.

Preferably, alternative splicing, particularly that mediated by *Actin-4,* may add sequences that affect RNA translation or stability, for instance.

It is also preferred that the splicing mechanism comprises at least a fragment of the *doublesex* (*dsx*) gene, preferably that derived from *Drosophila, B. mori,* Pink Boll Worm, Codling Moth, or a mosquito, in particular A. *gambiae* or especially *A. aegypti.*

It is preferred that the splice control sequence and the heterologous polynucleotide sequence encoding a functional protein, defined between a start codon and a stop codon, and/or polynucleotides for interference RNA (RNAi), to be expressed in an organism, are provided in the form of a minigene construct or a cassette exon.

This is particularly preferred when the splice control sequence is derived from *dsx* (preferably minigene 1 as described in the Examples and represented in SEQ ID NO. 149 (exons are present at positions 1-135, 1311-2446 and 3900-4389 of SEQ ID NO. 149) which was included in construct LA3491) or *Actin-4.*

Particularly preferred examples of the present invention are provided in the Examples, and can be selected from the group consisting of the plasmids or constructs, in particular any of those according to any one of Figures 19-31, especially any of the plasmids shown in Figs 16-18, 22-24, 26-32, 49, 52-55, and 61-69, and/or SEQ ID NOs 46-48, 50-56, 143-145 and 151-162.

Preferably, the functional protein to be expressed in an organism is tTAV, tTAV2 or tTAV3.

Further proteins to be expressed in the organism are, or course envisaged, in combination with said functional protein, preferably a lethal gene as discussed elsewhere.

A continuous ORF may be also be thought of as an uninterrupted ORF, i.e. a polynucleotide sequence in mature mRNA, which does not include non-coding nucleotides, for instance those having the potential to be translated into amino acids. In this definition, it is preferred that the stop codon is not included.

In some embodiments, the at least one splice control sequence regulates the alternative splicing by means of both intronic and exonic nucleotides. However, in one embodiment, it is particularly preferred that the at least one splice control sequence is an intronic splice control sequence. In other words, it is preferred that the at least one splice control sequence is substantially derived from polynucleotides that form part of an intron and are thus excised from the primary transcript by splicing, such that these nucleotides are not retained in the mature mRNA sequence.

Therefore, intronic sequences can be thought of as distinct from "exonic" sequences, which are retained in the processed (post-splicing) RNA molecule. Where the processed RNA molecule encodes a protein or polypeptide sequence, and is capable of being translated, i.e. has the correct structure and modifications such as a cap, and a polyadenylation signal, for instance, it is known as mature or processed mRNA and some of the exonic sequences then code for amino acids, when translated.

It will be understood that in alternative splicing, sequences may be intronic under some circumstances (i.e. in some alternative splicing variants), but exonic under other circumstances (i.e. in other variants). Thus, the at least one splice control sequence of the present invention is preferably substantially derived from polynucleotides that form part of an intron in at least one alternative splicing variant, i.e. in either the first spliced mRNA product or the at least one alternatively spliced mRNA product. Thus, introns or intronic sequences can be viewed as spliced out in at least one transcript or transcript type.

For example, consider the tra intron from *C. capitata* (Cctra intron), which is a particularly preferred example of an at least one splice control sequence according to the present invention. According to Figure 2A of Pane et al, reproduced as Figure 33, all 8 of the putative Tra/Tra2 binding sites highlighted are in intronic sequence in the sense that they are in portions of sequence spliced out in transcript F1, but on the other hand 6 out of the 8 are exonic in the sense that they are in exons that are included or retained in either transcript M1 or M2, or both. Thus, these Tra/Tra2 binding sites are intronic in the present sense as they are capable of controlling alternative splicing, but are spliced out, i.e. not present, in at least one alternative splicing variant, i.e. at least one mRNA that has been spliced in an alternative manner from pre-RNA.

In "normal" (non-alternative) splicing and in alternative splicing, introns are generally removed from the pre-RNA to form a spliced mRNA, which may then be translated into a polypeptide, such as a protein or protein fragment, having an amino acid sequence. Thus, it will be readily apparent to the skilled person how to determine those sequences of the present system that are to be considered intronic, rather than exonic.

It will, of course be appreciated that only part of an mRNA is actually translated, i.e. typically the part between the start codon and the stop codon, although it will be understood that sometimes multiple starts and stops are present. Thus, when reference is made herein to translation of an mRNA sequence, it will be appreciated that this is referring to translation of the portion starting at the first nucleotide of the start codon and ending after the last nucleotide before the start of the stop codon, which may be considered as the coding portion.

As mentioned above, exonic sequences may be involved in the mediation of the control of alternative splicing, but it is preferred that at least some intronic control sequences are involved in the mediation of the alternative splicing. In other words, the gene expression system of the present invention may also include splice control sequences present in exons, as long as there is some intronic involvement of control. Particularly preferred examples of these are splice control sequences derived from or containing elements of the *dsx* gene, where, without being bound by theory, it is thought that exonic sequences assist in the mechanism of alternative splicing.

Thus, in some embodiments, the at least one splice control sequence does comprise exonic sequence and it will be understood that this is envisaged by definitions used to describe the present invention. Thus, as will be apparent, it is possible for some nucleotides to be encompassed within the definition of the at least one splice control sequence and also within the definition of a polynucleotide sequence encoding a functional protein. In other words, the definition of these elements can overlap, such that certain nucleotides can be covered by the definition of more than one element.

However, the skilled person will recognise that this is not unusual in molecular biology, as nucleotides can often perform more than one role. For instance, in the present invention, a nucleotide can form part of a coding sequence for a functional protein, but could also form part of a sequence recognised and bound by a splicing factor, an example of which the TRA protein or TRA/TRA complex, as discussed elsewhere. This is not unusual as, for instance, some viruses have highly concentrated genome where the same stretch of polynucleotides can code for two or even three different proteins, each read in a different frame.

Of course, it may also be that the splice control sequence or sequences are solely intronic, i.e. with no exonic influence. Indeed, this is particularly preferred.

In some embodiments, it is preferred that the at least one splice control sequence is capable of being removed from the pre-RNA, by splicing. Preferably, the at least one splice control sequence does not result in a frameshift in at least one splice variant. Preferably this is a splice variant encoding a full-length functional protein. In other words, at least the one splice control sequence preferably does not mediate the removal of nucleotides that form part, or were intended to form part of, the polynucleotide sequence encoding a functional protein, defined between a start codon and a stop codon, and/or polynucleotides for interference RNA (RNAi), to be expressed in an organism. By this it is meant that nucleotides that are excised by splicing, in at least one splice variant, are not nucleotides that encode amino acids in the wild type form of the protein or gene. One or more splice variants may have said nucleotides excised, but at least one variant must retain these nucleotides, so that a frameshift is not induced in the at least one variant. These removed nucleotides are those that are removed in addition to the sequences that are normally spliced out such as the intron.

However, in view of the above, it is also envisaged that different splice variants may result in the same sequence being read in different frames.

Interaction of the at least one splice control sequence with cellular splicing machinery, e.g. the spliceosome, leads to or mediates the removal of a series of, preferably, at least 50 consecutive nucleotides from the primary transcript and ligation (splicing) together of nucleotide sequences that were not consecutive in the primary transcript (because they, or their complement if the antisense sequence is considered, were not consecutive in the original template sequence from which the primary transcript was transcribed). Said series of at least 50 consecutive nucleotides comprises an intron. This mediation acts preferably in a sex-specific, stage-specific, germline-specific or tissue-specific manner, or combination thereof, such that equivalent primary transcripts in different sexes, stages, tissue types, etc, tend to remove introns of different size or sequence, or in some cases may remove an intron in one case but not another. This phenomenon, the removal of introns of different size or sequence in different circumstances, or the differential removal of introns of a given size or sequence, in different circumstances, is known as alternative splicing. Alternative splicing is a well-known phenomenon in nature, and many instances are known, see above.

In some preferred embodiments, the at least one splice control sequence is associated with a heterologous open reading frame such that, in at least one splice variant, the heterologous open reading frame is disrupted, e.g. by a stop codon or frameshift, while in at least one alternative splice variant the heterologous open reading frame is not disrupted. Transcripts of the second type encode or potentially encode a functional protein, whereas those of the first type encode a protein with altered, disrupted or even no function, activity or stability relative to those of the second type.

In general, it will be apparent to the person skilled in the art that the heterologous open reading frame may itself be a composite or fusion of sequences from various sources. Splicing to produce a functional protein may still produce an altered protein relative to the prototype heterologous open reading frame, for example if the inserted alternatively spliced intron includes sequence that is exonic in all alternative splicing forms, and therefore retained in mature mRNAs of the second type. However, it is particularly preferred that at least one transcript removes all, or substantially all, of the inserted alternatively spliced sequence, such that the heterologous open reading frame is restored, or substantially restored, to intact form, with little or no sequence endogenously associated with the intron remaining in the mature mRNA. Endogenous is used here in contrast to heterologous, so it will be understood that this refers to a sequence that would, in the wild type, be normally found in association with, or linked to, at least one element or component of the at least one splice control sequence.

Alternatively, one or more transcripts may remove additional nucleotides, so that the heterologous open reading frame is disrupted, not by the insertion of extra nucleotides (for example stop codon or frame shift, but also potentially coding sequence that disrupts the function), but rather by deletion of nucleotides from the heterologous open reading frame, for example in such a way as to induce a frameshift. One or more splice variants may have said nucleotides excised, but at least one variant must retain these nucleotides, so that a frameshift is not induced in the at least one variant. These removed nucleotides are those that are removed in addition to the sequences that are normally spliced out such as the intron, where an intronic sequence may be considered as one that forms part of an intron in at least one alternative splicing variant of the natural analogue.

When exonic nucleotides are to be removed, then these must be removed in multiples of three, if it is desired to avoid to avoid a frameshift, but as a single nucleotide or multiples of two (that are not also multiples of three) if it is desired to induce a frameshift. It will be appreciated that if only one or certain multiples of two nucleotides are removed, then this could lead to a completely different protein sequence being encoded at or around the splice junction of the mRNA.

This is particularly the case in an embodiment of the system where cassette exons are used to interrupt an open reading frame in some splice variants but not others, such as in, for example, tra, especially Cctra.

In another preferred embodiment of the present invention, all or part of an open reading frame is on a cassette exon, for example some Dsx embodiments derived from *Aedes,* are provided with, for instance, a tTAV coding region on a cassette exon that is only present in female-specific splice variants.

Where mediation of alternative splicing is sex-specific, it is preferred that the splice variant encoding a functional protein to be expressed in an organism is the F1 splice variant, i.e. a splice variant found only or predominantly in females, and preferably is the most abundant variant found in females, although this is not essential. Correspondingly for configurations where all or part of a functional open reading frame is on a cassette exon, it is preferred that this cassette exon is included in transcripts found only or predominantly in females, and preferably such transcripts are, individually or in combination, the most abundant variants found in females, although this is not essential.

In one preferred embodiment, sequences are included in a hybrid or recombinant sequence or construct which are derived from naturally occurring intronic sequences which are themselves subject to alternative splicing, in their native or original context. Therefore, an intronic sequence may be considered as one that forms part of an intron in at least one alternative splicing variant of the natural analogue. Thus, sequences corresponding to single contiguous stretches of naturally occurring intronic sequence are envisioned, but also hybrids of such sequences, including hybrids from two different naturally occurring intronic sequences, and also sequences with deletions or insertions relative to single contiguous stretches of naturally occurring intronic sequence, and hybrids thereof. Said sequences derived from naturally occurring intronic sequences may themselves be associated, in the invention, with sequences not themselves part of any naturally occurring intron. If such sequences are transcribed, and preferably retained in the mature RNA in at least one splice variant, they may then be considered exonic.

It will also be appreciated that reference to a "frame shift" could also refer to the direct coding of a stop codon, which is also likely to lead to a non-functioning protein as would a disruption of the spliced mRNA sequence caused by insertion or deletion of nucleotides. Production from different splice variants of two or more different proteins or polypeptide sequences of differential function is also envisioned, in addition to the production of two or more different proteins or polypeptide sequences of which one or more has no predicted or discernable function. Also envisioned is the production from different splice variants of two or more different proteins or polypeptide sequences of similar function, but differing subcellular location, stability or capacity to bind to or associate with other proteins or nucleic acids.

Preferably, the at least one splice control sequence is intronic and comprises on its 5' end a guanine (G) nucleotide. In other words, the 5' nucleotide of the splice control sequence, 3' to the splice donor site, and preferably at the interface or junction of the exon with the splice control sequence, is Guanine (G), in the pre-RNA, or C in an antisense DNA sequence corresponding thereto.

Furthermore, the adjacent nucleotide (3' to said G) is preferably Cytosine (C) in the pre-RNA, or a corresponding G in a DNA sequence, but is most preferably Uracil (U) in the pre-RNA, or a corresponding A in a DNA antisense sequence. Thus, the two 5' nucleotides of the splice control sequence are preferably 5'GT with respect to the DNA sense strand, 5'-GU in the primary transcript.

Preferably, at least one intronic splice control sequence also comprises on its 3' end a 3' Guanine nucleotide and preferably AG-3' at the junction of the splice acceptor site with the exon, for instance, see Figure 34.

Preferably, the flanking sequence 5' to the splice donor site in the system comprises 5'-TG, so that the sequence can be represented 5'-TG-*-splice control sequence-**-3', where * represents the splice donor site and ** represents the splice acceptor site.

Preferably, the splice control sequence is also flanked on its 3' side by a G nucleotide, and most preferably by GT nucleotides, such that the sequence could be represented as: 5'-TG-*-splice control sequence-**-GT-3'. It will be appreciated that this is the sense strand DNA sequence (TG). Thus, the transcribed pre-RNA will read UG for instance, where U replaces T.

Derivatives of Guanine or Thymine having the same function are also envisaged.

It is particularly preferred that the splicing is sex-specific and further mediated or controlled by binding of the TRA protein or TRA/TRA2 protein complex, or homologues thereof. In insects, for instance, the TRA protein is differentially expressed in different sexes. In particular, the TRA protein is known to be present largely in females and, therefore, mediates alternative splicing in such a way that a coding sequence is expressed in a sex-specific manner, i.e. that in some cases a protein is expressed only in females or at a much higher level in females than in males or, alternatively, in other cases a protein is expressed only in males, or at a much higher level in males than in females. Whilst it is preferred that the protein is expressed only in males, it is particularly preferred that the protein is expressed only in females, however. The mechanism for achieving this sex-specific alternative splicing mediated by the TRA protein or the TRA/TRA-2 complex is known and is discussed, for instance, in Pane et al (Development 129, 3715-3725 (2002)).

Preferably, the at least one splice control sequence comprises, and more preferably consists of, the *tra* intron derived from the *tra* gene of *Ceratitis capitata* (*Cctra*), which has one alternatively spliced region. In the F1 transcript, as illustrated by Figure 33 (Figure 2A of Pane et al (2002) *supra*), this is the first intron. Homologues of the *tra* gene in other species, such as *Bactrocera oleae, Ceratitis rosa, Bactrocera zonata* and *Drosophila melanogaster* also have alternatively spliced regions in a similar location within the *tra* coding sequence. *tra* introns derived from these insects are also particularly preferred.

The splicing pattern in *Cctra* in particular is well conserved, with those transcripts found in males containing additional exonic material relative to the F1 transcript, such that these transcripts do not encode full-length, functional Tra protein. By contrast, the F1 transcript does encode full-length, functional Tra protein; this transcript is substantially female-specific at most life-cycle stages, though it is speculated that very early embryos of both sexes may contain a small amount of this transcript. We describe the sequence spliced out of the F1 transcript, but not the male-specific or non-sex-specific transcripts, as the tra intron, or even the tra F1 intron. Thus the version of this sequence found in the *Cctra* gene is the Cctra intron.

Thus the *tra* gene is regulated in part by sex-specific alternative splicing, while its key product, the Tra protein, is itself involved in alternative splicing. In insects, sex-specific alternative splicing mediated by the TRA protein, or a complex comprising the TRA and TRA2 proteins, include Dipteran splice control sequences derived from the *doublesex* (dsx) gene and also the *tra* intron itself, although this would exclude the *tra* intron from *Drosophila* (*Dmtra*), which is principally mediated by the *Sxl* gene product in *Drosophila,* rather than TRA or the TRA/TRA2 complex.

Outside of *Drosophila,* the *Sxl* gene product is not differentially expressed in the different sexes. *Sxl* is not thought to act in the mediation of sex-specific alternative splicing in non-Drosophilid insects.

Examples of the TRA protein that binds to the binding protein sites (the nucleotide sequences specifically recognised by the TRA protein) in the *tra* intron are preferably from Diptera, preferably from the family Tephritidae, more preferably from the genera *Ceratitis, Anastrepha* or *Bactrocera.* However, it is also envisaged that other Dipterans, such as Drosophilids or mosquitoes of the various forms discussed below, are also capable of providing the TRA protein or homologues thereof that are capable of binding to the appropriate sites on the splice control sequences derived from *dsx* gene, the *tra* gene or the *tra* intron, i.e. the alternatively spliced tra intron completely removed in the F1 transcript, even in those cases, such as *Drosophila,* where the natural tra gene (*Dmtra*) is not itself regulated by TRA protein. In some embodiments, the "tra intron" may be defined as a splice control sequence wherein alternative splicing of the RNA transcript is regulated by TRA, for instance binding thereof, alone or in combination (i.e. when complexed) with TRA2. This excludes the *tra* intron from Drosophila.

It is particularly preferred that the splice control sequences are derived from the *tra* intron. Said *tra* intron may be derived, as discussed elsewhere, from *Ceratitis, Anastrepha* or *Bactrocera.* The *Ceratitis capitata tra* intron from the *transformer* gene was initially characterised by Pane et al (2002), *supra.* However, it will be appreciated that homologues exist in other species, and can be easily identified in said species and also in their various genera. Thus, when reference is made to *tra* it will be appreciated that this also relates to *tra* homologues in other species, especially in *Ceratitis, Anastrapha* or *Bactrocera* species.

By "derived" it will be understood that, using reference to the *tra* intron, this refers to sequences that approximate to or replicate exactly the *tra* intron, as described in the art, in this case by Pane et al (2002), *supra.* However, it will be appreciated that, as these are intronic sequences, that some nucleotides can be added or deleted or substituted without a substantial loss in function.

Preferred examples of this include the dsx intron, preferably provided in the form of a minigene. In this instance, it may be preferable to delete, as we have done in the Examples, sizable amounts from alternatively spliced introns, e.g. 90% or more of an intron in some cases, whilst still retaining the alternative splicing function. Thus, whilst large deletions are envisioned, it is also envisaged that smaller, e.g. even single nucleotide insertions, substitutions or deletions are also preferred.

The exact length of the splice control sequence derived from the *tra* intron is not essential, provided that it is capable of mediating alternative splicing. In this regard, it is thought that around 55 to 60 nucleotides is the minimum length for a modified *tra* intron, although the wild type *tra* intron (F1 splice variant) from *C. capitata* is in the region of 1345 nucleotides long.

It is particularly preferred that the full length 1345 ntd sequence of *Cctra* is used.

As with all nucleotide sequences discussed herein, it is preferred that a certain degree of sequence homology is envisaged, unless otherwise apparent. Thus, it is preferred that the splice control sequence has at least 80% sequence homology with the reference SEQ ID NO., preferably at least 80% sequence homology with the reference SEQ ID NO., preferably at least 80% sequence homology with the reference SEQ ID NO., more preferably at least 90% sequence homology with the reference SEQ ID NO., more preferably at least 95% sequence homology with the reference SEQ ID NO., even more preferably at least 99% sequence homology with the reference SEQ ID NO., and most preferably at least 99.9% sequence homology with the reference SEQ ID NO. A suitable algorithm such as BLAST may be used to ascertain sequence homology. If large amounts of sequence are deleted cf the wildtype, then the sequence comparison may be over the full length of the wildtype or over aligned sequences of similar homology.

However, it will be understood that despite the above sequence homology, certain elements, in particular the flanking nucleotides and splice branch site must be retained, for efficient functioning of the system. In other words, whilst portions may be deleted or otherwise altered, alternative splicing functionality or activity, to at least 30%, preferably 50%, preferably 70%, more preferably 90%, and most preferably 95% compared to the wildtype should be retained. This could be increased cf the wildtype, as well, by suitably engineering the sites that bind alternative splicing factors or interact with the spliceosome, for instance.

In particular, it is preferred that where the splice control sequence comprises a modified TRA intron, this comprises at least 20 to 40 base pairs from the 5' and, preferably, so the 3' end of said intron. Furthermore, it is preferred that at least 3 or 4 and most preferably, at least 5, preferably 6, more preferably 7 and most preferably all 8 of the 8 putative TRA binding domains of the *C. capitata tra* intron, as taught by Pane et al (2002), or homologues thereof, are provided. Of course, if further such sites are discovered in due course, then it is envisaged that the splice control sequence could include more than 8 sites. In fact, it is envisaged that the more than 8 sites may be engineered in to the splice control sequence and that alternative splicing may be regulated in this way, especially if some sites are bound with differing affinities leading to different alternative splicing outcomes.

A consensus sequence for the putative TRA binding domains of the *C. capitata tra* intron is given below as SEQ ID NO 1, a DNA sequence, although the corresponding RNA equivalent is also preferred.

The preferred consensus sequences is 1: TC<u>WWCRAT</u>CAACA (SEQ ID NO. 1), where W = A or T and R = A or G.

Similar considerations apply to *doublesex,* where the consensus sequence for the TRA protein is also that given in SEQ ID NO. 1, as a protein complex comprising the Tra and TRA2 proteins is a key regulator of alternative splicing of *doublesex,* as it is for *tra* homologues (though not the *tra* homologues found in Drosophilids).

As mentioned above, the splice control sequences are preferably derived from the *tra* intron, preferably from the family *Tephritidae.* It is particularly preferred that the *tra* intron is derived from *B. zonata* or, preferably, from other non-Drosophilid fruit flies. However, it is particularly preferred that the *tra* intron is derived from the *Ceratitis* genus, in particular C. *rosa* and, most preferably, C. *capitata.* These are more widely known as the Natal and Mediterranean fruit flies, respectively.

With regard to the *tra* intron derived from *B. zonata,* we have shown that this can lead to sex-specific alternative splicing in transgenic Mexfly *(Anastrapha ludens)* and in transgenic Medfly (C. *capitata*). We have also shown that a variety of proteins can be expressed in a sex-specific manner via alternative splicing, including tTAV 3 and Rpr.

In relation to the *tra* intron derived from *C. rosa,* we have successfully provided alternative splicing in a sex-specific manner of a transgene in Medfly.

With regard to the *tra* intron derived from *C. capitata* (Medfly), we have shown that this can mediate sex-specific splicing in transgenic Medfly, and other Tephritids, and other Tephritids such as A. *ludens* (Mexfly). Not only that, we have shown that this intron can work successfully across a whole range of insects and, in particular, Dipterans. Indeed, we have shown that the TRA intron from *C. capitata* (referred to as *Cctra*) can provide sex-specific alternative splicing in transgenic Drosophila, which is not a Tephritid, and also in the mosquito *Aedes aegypti.* Although mosquitoes are Diptera, they diverged from Drosophila and the Tephritids about 250 million years ago and, therefore, are much more distantly related than Drosophilids are to Tephritids, for which the divergence time has been estimated as 120-150 million years. Thus, this shows the broad applicability of the present invention across a wide range of insects.

With regard to splice control sequences derived from the *dsx* intron, we have also shown that this can be used to alternatively splice, in a sex-specific manner, in a broad range of insects. Accordingly, it is particularly preferred that the dsx is derived from *Bombyx mori (silk moth), Pectinophora gossypiella (Pink Bollworm) Pectinophora gossypiella, Cydia. pomonella (codling moth*), *Drosophila,* and mosquitoes such as *Anopheles sp.,* for instance A. *gambiae.* Particularly preferred mosquitoes include *Stegomyia spp.,* particularly *S. aegypti* (also known as *Aedes aegypti*).

Indeed, in A. *aegypti,* we have shown a considerable number of DNA constructs, which are capable of providing sex-specific alternative splicing.

It will be appreciated that the system or construct is preferably administered as a plasmid, but generally tested after integrating into the genome. Administration can be by known methods in the art, such as parenterally, intra-venous intra-muscularly, orally, transdermally, delivered across a mucous membrane, and so forth. Injection into embryos is particularly preferred. The plasmid may be linearised before or during administration, and not all of the plasmid may be integrated into the genome. Where only part of the plasmid is integrated into the genome, it is preferred that this part include the at least one splice control sequence capable of mediating alternative splicing.

Preferably, the polynucleotide expression system is a recombinant dominant lethal genetic system, the lethal effect of which is conditional. Suitable conditions include temperature, so that the system is expressed at one temperature but not, or to a lesser degree, at another temperature, for example. The lethal genetic system may act on specific cells or tissues or impose its effect on the whole organism. Systems that are not strictly lethal but impose a substantial fitness cost are also envisioned, for example leading to blindness, flightlessness (for organisms that could normally fly), or sterility. Systems that interfere with sex determination are also envisioned, for example transforming or tending to transform all or part of an organism from one sexual type to another. It will be understood that all such systems and consequences are encompassed by the term lethal as used herein. Similarly, "killing", and similar terms refer to the effective expression of the lethal system and thereby the imposition of a deleterious or sex-distorting phenotype, for example death.

More preferably, the polynucleotide expression system is a recombinant dominant lethal genetic system, the lethal effect of which is conditional and is not expressed under permissive conditions requiring the presence of a substance which is absent from the natural environment of the organism, such that the lethal effect of the lethal system occurs in the natural environment of the organism

In other words, the coding sequences encode a lethal linked to a system such as the *tet* system described in WO 01/39599 and/or WO2005/012534.

Indeed it is preferred that the expression of said lethal gene is under the control of a repressible transactivator protein. It is also preferred that the gene whose expression is regulated by alternative splicing encode a transactivator protein such as tTA. This is not incompatible with the regulated protein being a lethal. Indeed, it is particularly preferred that it is both. In this regard, we particularly prefer that the system includes a positive feedback system as taught in WO2005/012534.

Preferably, the lethal effect of the dominant lethal system is conditionally suppressible.

Suitable organisms under which the present system can be used include mammals such as mice, rats and farm animals. Also preferred are fish, such as salmon and trout. Plants are also preferred, but it is particularly preferred that the host organism is an insect, preferably a Dipteran or tephritid. Preferably, the organism is not a human, preferably non-mammalian, preferably not a bird, preferably an invertebrate, preferably an arthropod.

In particular, it is preferred that the insect is from the Order Diptera, especially higher Diptera and particularly that it is a tephritid fruit fly, preferably Medfly (*Ceratitis capitata*), preferably Mexfly (*Anastrepha ludens*), preferably Oriental fruit fly *(Bactrocera dorsalis),* Olive fruit fly *(Bactrocera oleae),* Melon fly *(Bactrocera cucurbitae*), Natal fruit fly (*Ceratitis rosa*), Cherry fruit fly (*Rhagoletis cerasi*), Queensland fruit fly (*Bactrocera tyroni*), Peach fruit fly (*Bactrocera zonata*) Caribbean fruit fly (*Anastrepha suspensa*) or West Indian fruit fly (*Anastrepha obliqua*). It is also particularly preferred that the host organism is a mosquito, preferably from the genera *Stegomyia, Aedes, Anopheles* or *Culex.* Particularly preferred are *Stegomyia aegyptae,* also known as *Aedes aegypti, Stegomyia albopicta* (also known as *Aedes albopictus*), *Anopheles stephensi, Anopheles albimanus* and *Anopheles gambiae.*

Within Diptera, another preferred group is Calliphoridae, particularly the New world screwworm (*Cochliomyia hominivorax*), Old world screwworm (*Chrysomya bezziana*) and Australian sheep blowfly (*Lucilia cuprina*). Lepidoptera and Coleoptera are also preferred, especially moths, including codling moth (*Cydia pomonella*), and the silk worm (*Bombyx mori*), the pink bollworm (*Pectinophora gossypiella*), the diamondback moth (*Plutella xylostella*), the Gypsy moth (*Lymantria dispar*), the Navel Orange Worm (*Amyelois transitella*), the Peach Twig Borer (*Anarsia lineatella*) and the rice stem borer (*Tryporyza incertulas*), also the noctuid moths, especially Heliothinae. Among Coleoptera, Japanese beetle (*Popilla japonica*), White-fringed beetle (*Graphognatus* spp.), Boll weevil (*Anthonomous grandis*), corn root worm (*Diabrotica spp*) and Colorado potato beetle (*Leptinotarsa decemlineata*) are particularly preferred.

Preferably, the insect is not a Drosphilid, especially Dm. Thus, in some embodiments, expression in Drosophilids, especially Dm is excluded. In other embodiments, the splice control sequence is not derived from the *tra* intron of a Drosphilid, especially Dm.

It is preferred that the expression of the heterologous polynucleotide sequence leads to a phenotypic consequence in the organism. It is particularly preferred that the functional protein is not beta-galactosidase, but can be associated with visible markers (including fluorescence), viability, fertility, fecundity, fitness, flight ability, vision, and behavioural differences. It will be appreciated, of course, that, in some embodiments, the expression systems are typically conditional, with the phenotype being expressed only under some, for instance restrictive, conditions.

In a further aspect, there is also provided a method of population control of an organism in a natural environment therefor, comprising:

i) breeding a stock of the organism,

the organism carrying a gene expression system comprising a system according to the present invention which is a dominant lethal genetic system,

ii) distributing the said stock animals into the environment at a locus for population control; and

iii) achieving population control through early stage lethality by expression of the lethal system in offspring that result from interbreeding of the said stock individuals with individuals of the opposite sex of the wild population.

Preferably, the early stage lethality is embryonic or before sexual maturity, preferably early in development, most preferably in the early larval or embryonic life stages.

Preferably, the lethal effect of the lethal system is conditional and occurs in the said natural environment *via* the expression of a lethal gene,

the expression of said lethal gene being under the control of a repressible transactivator protein,
the said breeding being under permissive conditions in the presence of a substance, the substance being absent from the said natural environment and able to repress said transactivator.

Preferably, the lethal effect is expressed in the embryos of said offspring. Preferably, the organism is an invertebrate multicellular animal or is as discussed elsewhere.

Also provided is a method of biological control, comprising:

i) breeding a stock of males and female organisms transformed with the expression system according to the present invention under permissive conditions, allowing the survival of males and females, to give a dual sex biological control agent;
ii) optionally before the next step imposing or permitting restrictive conditions to cause death of individuals of one sex and thereby providing a single sex biological control agent comprising individuals of the other sex carrying the conditional lethal genetic system;
iii) releasing the dual sex or single sex biological control agent into the environment at a locus for biological control; and
iv) achieving biological control through expression of the genetic system in offspring resulting from interbreeding of the individuals of the biological control agent with individuals of the opposite sex of the wild population.:

Preferably, there is sex-separation prior to organism distribution by expression of a sex specific lethal genetic system.

Preferably, the lethal effect results in killing of greater than 90% of the target class of the progeny of matings between released organisms and the wild population.

Also provided is a method of sex separation comprising:

i) breeding a stock of male and female organisms transformed with the gene expression system under permissive or restrictive conditions, allowing the survival of males and females; and
ii) removing the permissive or restrictive conditions to induce the lethal effect of the lethal gene in one sex and not the other by sex-specific alternative splicing of the lethal gene.

Preferably, the lethal effect results in killing of greater than 90% of the target class of the progeny of matings between released organisms and the wild population.

Also provided is a method or biological or population control comprising;

i) breeding a stock of male and female organisms transformed with the gene expression system under permissive or restrictive conditions, allowing the survival of males and females;
ii) removing the permissive or restrictive conditions to induce the lethal effect of the lethal gene in one sex and not the other by sex-specific alternative splicing of the lethal gene to achieve sex separation;
iii) sterilising or partially sterilising the separated individuals and
iv) achieving said control through release of the separated sterile or partially sterile individuals in to the natural environment of the organism.

Preferably, the sterilising is achieved through the use of ionising radiation. In general, however, methods avoiding irradiation, as used in the Sterile Insect Technique (SIT) are especially preferred and have many cost and health advantages over methods associated with or followed by the use of radiation.

Also provided is a method to selectively eliminate females from a population. The equivalent for males is also envisaged.

Methods of sex separation are hugely important commercially in, for example silk worms, where males produce more and better silk than females. Thus, methods of sex separation that eliminate females and, in particular female silk worms are particularly preferred.

It is also envisaged that the functional protein may be a expressed differentially, but detectably in more than one splice variant and preferably, therefore, in both sexes, for instance. Such examples include a fluorescent protein, such as eGFP, CopGFP and DsRed2. This may be used in a method of non-lethal sex separation or sorting, so that one can separate the two types without killing either of them

We have also surprisingly discovered that the positioning of the splice control sequence can be altered and better results obtained. Preferably, the splice control sequence is the "first" splice control sequence, when read from the promoter, in 5' to 3' direction We have found that in certain constructs with an intron in the 5' UTR of the system that this leads to reduced levels or alternatively spliced protein expression mediated by the splice control sequence of the present invention.

Preferably, the splice control sequence is 3' to the start codon. Preferably, the splice control sequence is inserted within the first exon, i.e. the stretch of sequence immediately 3' to the transcription start site. It will be understood that such terms may refer to the DNA sequence which encodes the transcript, or to the RNA transcript itself.

Where the splice control sequence is 3' to the start codon, it is preferred that it is also 5' to the first in-frame stop codon (that is 3' to and in frame with the start codon), so that alternative splicing yields transcripts that encode different protein or polypeptide sequences. Thus in a preferred embodiment, the construct or polynucleotide sequence comprises the following elements in 5' to 3' order, with respect to the sense strand or primary transcript: transcription start, translation start, intron capable of alternative splicing, coding sequence for all or part of a protein, stop codon.

The splice control sequence may be defined as preferably up to and including the 5' G (GT/C) and its 3' G equivalent, especially in *tra,* but as mentioned above, this can include some exonic sequence and therefore, could include the 3' most (last) nucleotide of the exon (i.e. G).

It is particularly preferred that the splice control sequence is immediately adjacent, in the 3' direction, the start codon, so that the G of the ATG is 5' to the start (5' end) of the splice control sequence. This is particularly advantageous as it allows the G of the ATG start codon to be the 5'G flanking sequence to the splice control sequence.

Alternatively, the splice control sequence is 3' to the start codon but within 1000 exonic bp, preferably 500 exonic bp, preferably 300 exonic bp, preferably 200 exonic bp, preferably 150 exonic bp, preferably 100 exonic bp, more preferably 75 exonic bp, more preferably 50 exonic bp, more preferably 30 exonic bp, more preferably 20 exonic bp, and most preferably 10 or even 5, 4, 3, 2, or 1 exonic bp.

The present invention is an improvement on the system defined as LA1188 in WO2005/012534. This plasmid had a number of defects, principal of which is that exonic nucleotides were excised with the *Cctra* intron used therein, thereby resulting in an induced frameshift in the transcript.

Specifically, in addition to the sequence derived from *Cctra* (the *Cctra* intron), 4 nucleotides of tTAV sequence were removed in the female-specific transcript. Therefore, though several alternatively spliced transcripts were produced, including one female-specific transcript, none were capable of encoding functional tTAV protein. Therefore, this construct was not capable of providing sex-specific expression of functional tTAV protein.

Since splicing was not directed to the splice donor sequence (5'-GT...) normally used in the Cctra intron, clearly this construct did not contain all of the regulatory sequences necessary to direct splicing in the form of the Cctra intron in "its native context." However, this highlights another issue. Probably the only thing missing was the flanking TG...GT, of which it is possible that only the 5'G mattered.

A key benefit of the present invention is, in particular in relation to *tra,* that the requirements for exonic sequence are so minimal (e.g. 2 nucleotides at each end) that they can easily be designed into most coding sequences, using the redundancy in the genetic code. So the "extra" exonic nucleotides can both be part of the heterologous protein sequence, and the flanking sequence of the intron in its native context at the same time.

Furthermore, the *Cctra* intron in LA1188 was +132bp 3' to the G of the ATG start codon (to the last exonic nucleotide). Indeed, although the *Cctra* intron in LA1188 is the first intron read in the 5' to 3; direction from the ATG start codon, it is not the "first" intron when read in the 5' to 3' direction from promoter. In fact, it is the 2nd intron, as there is a further intron (derived from the *Drosophila melanogaster Adh* gene) upstream of the ATG start codon. This information is included in the Table 3.

It will be understood that where reference is made to ATG start codons or flanking G, or 5'-TG...GT-3' sequences, that this is in relation to a DNA sequence, but this is also covers the corresponding DNA antisense sequence and, equally, the corresponding RNA sequence.

### Description of the Sequences of the present invention

SEQ ID NO. 1 tra consensus sequence
SEQ ID NO. 2 LA3097 5' flanking sequence
SEQ ID NO. 3 LA3097 3' flanking sequence
SEQ ID NO. 4 primer 688 - ie1-transcr
SEQ ID NO. 5 primer 790 - Aedsx-m-r2
SEQ ID NO. 6 primer 761 - Aedsx-fem-r

SEQ ID NO. 7 primer AedsxR1
SEQ ID NO. 8 Pane et al consensus sequence
SEQ ID NO. 9 Scali et al 2005 consensus sequence
SEQ ID NOS. 10 - 33 and 107 - 138 consensus sequences of putative Tra/Tra2 binding sites deduced for *Drosophila* (see Table 2).
SEQ ID NO. 34: Open reading frame of tTAV
SEQ ID NO. 35: Protein sequence of tTAV
SEQ ID NO. 36: Open reading frame of tTAV2
SEQ ID NO. 37: Protein sequence of tTAV2
SEQ ID NO. 38: Open reading frame of tTAV3
SEQ ID NO. 39: Protein sequence of tTAV3
SEQ ID NO. 40: Pink Bollworm *dsx* female specific sequence fragment 1
SEQ ID NO. 41: Pink Bollworm (PBW, *Pectinophora gossypiella*) *dsx* female specific sequence fragment 2
SEQ ID NO. 42: Pink Bollworm (PBW, *Pectinophora gossypiella) dsx* male specific sequence
SEQ ID NO. 43: Partial gene sequence of *Aedes aegypti dsx.* All exonic sequence is included, but only partial intronic sequence- see Figures 47 and 48 for annotation.
SEQ ID NO. 44: Codling moth (*Cydia pomonella*) *dsx* female gene sequence: includes a stretch of unknown nucleotides, preferably than then 100, preferably less than 50, more preferably less than 20, more preferably less than 10, and most preferably less than 5.
SEQ ID NO. 45: Codling moth (*Cydia pomonella*) dsx-male sequence.
SEQ ID NO. 46: Sequence of pLA3435-Bombyx mori-dsx construct/plasmid.
SEQ ID NO. 47: Sequence of *pLA3359-Anopheles gambiae* dsx construct.
SEQ ID NO. 48: Sequence of pLA3433-Agdsx (*Anopheles gambiae*)construct with exon 2 included.
SEQ ID NO. 49: Sequence of pLA1188-cctra intron construct
SEQ ID NO. 50: Sequence of pLA3077-a Cctra intron-tTAV construct.
SEQ ID NO. 51: Sequence of pLA3097-a Cctra intron-tTAV construct.
SEQ ID NO. 52: Sequence of pLA3233-Cctra-intron-tTAV2 construct.
SEQ ID NO 53: Sequence of pLA3014-Cctra-intron-Ubiquitin-reaperKR construct.
SEQ ID NO. 54: Sequence of pLA3166-Cctra intron-Ubiquitin-reaperKR construct.
SEQ ID NO. 55: Sequence of pLA3376-Bztra intron-reaperKR and Bztra-intron-tTAV3.
SEQ ID NO. 56: Sequence of pLA3242-Crtra intron-reaperKR construct.
SEQ ID NO. 57: Partial sequence of a male transcript generated in *Drosophila melanogaster* from LA3077 transformants that differs to the sequence generated in Medfly LA3077 lines. This sequence corresponds to the M3 transcript depicted in Figure 36.
SEQ ID NO. 58: Partial sequence of *Bactrocera zonata* tra homologue. Sequence of intron predicted to be spliced out in a female-specific transcript of *B. zonata* tra (+3 to +970bp in sequence). Exonic flanking nucleotides are at positions 1-2 and 971-972, i.e. at the 5' and 3' ends of the intronic sequence. In fact, it is worth noting that the intronic sequence is flanked on its 5' end by a Guanine nucleotide, which is thought critical for a clean exit of the intron.
SEQ ID NO 59: Partial sequence of *Ceratitis rosa tra* homologue. Sequence of intron predicted to be spliced out in a female-specific transcript of *C. rosa* tra (+3 to 1311bp in sequence). Exonic flanking nucleotides are present at positions 1-2 and 1312-3. Again, it is noteworthy that the intronic sequence is flanked on its 5' end by a Guanine nucleotide, which is thought critical for a clean exit of the intron.
SEQ ID NOS. 60-70: Primers as referred to in Figures 44-46 and 50-51.
SEQ ID NO. 71: Pink Bollworm (PBW, *Pectinophora gossypiella*) *dsx* female specific fragment 3.
SEQ ID NO. 72: Open reading frame of *Drosophila melanogaster* ubiquitin.
SEQ ID NO. 73: Protein sequence of *Drosophila melanogaster* Ubiquitin.
SEQ ID NOS. 74-105 are primers as discussed above in the Examples.
SEQ ID NO. 106 is the LA1172 nucleotide sequence, including plasmid backbone.
SEQ ID NOs 107-138 are described above.

| | |
|---|---|
| SEQ ID NO. 139 HSP primer | SEQ ID NO. 151 LA3619 whole plasmid sequence |
| SEQ ID NO. 140 VP16 primer | |
| SEQ ID NO. 141 primer Agexon1F | SEQ ID NO. 152 LA3612 whole plasmid sequence |
| SEQ ID NO. 142 primer TETRR1 | |
| SEQ ID NO. 143 LA3576 plasmid sequence | SEQ ID NO. 153 LA3491 plasmid sequence |
| SEQ ID NO. 144 LA3582 plasmid sequence | SEQ ID NO. 154 LA3515 plasmid sequence |

(continued)

| | |
|---|---|
| SEQ ID NO. 145 LA3596 plasmid sequence | SEQ ID NO. 155 LA3545 plasmid sequence |
| SEQ ID NO. 146 PBW-dsx (Fig 6) | SEQ ID NO. 156 LA3604 plasmid sequence |
| SEQ ID NO. 147 bombyx-dsx (Fig 6) | SEQ ID NO. 157 LA3646 plasmid sequence |
| SEQ ID NO. 148 codling-dsx (Fig 6) | SEQ ID NO. 158 LA3054 plasmid sequence |
| SEQ ID NO. 149 DSX Minigene1 from construct LA3491 | SEQ ID NO. 159 LA3056 plasmid sequence |
| | SEQ ID NO. 160 LA3488 plasmid sequence |
| SEQ ID NO. 150 DSX Minigene2 from construct LA3534 | SEQ ID NO. 161 LA3641plasmid sequence |
| | SEQ ID NO. 162 LA3570 plasmid sequence |

The invention will now be described by reference to the following, non-limiting Examples.

## EXAMPLES

### Transformer

### Example *1-Ceratitis capitata tra* intron

We have prepared an insertion of a Cctra intron cassette into a synthetic open reading frame (ORF). Two versions of this splice correctly in Medfly, in other words the splicing of the Cctra intron cassette faithfully recapitulates what it would normally do in the context of the endogenous Cctra gene. This is to produce 3 (major or only) splice variants in females, one of which is female-specific (called F1), while the other two are found in both males and females (called M1 and M2). Since each of the non-sex-specific transcripts contain additional exonic material with stop codons, we have also arranged this so that only the female splice variant produces functional protein.

Each of these constructs (LA3077 and LA3097) has the Cctra intron flanked by TG and GT (to give 5'...TG¦*intron*¦GT... 3'. An older construct, which does not work perfectly, is LA1188. LA1188 is quite well characterized - splicing is exactly as above except that an additional 4 nucleotides are removed. The intron is in the context 5'...TGGCAC¦intron¦GT...3'; splicing removes an additional 4 bases, i.e. 5'...TG¦GCAC*intron*¦GT...3' (Figure 33).

In all cases the intron is invariant, and is simply the complete Cctra intron sequence. As is normal for introns, it begins GT and ends AG. Almost all introns start with GT, so the use of the rare alternative GC in LA1188 is surprising [GC-AG introns are a known alternative - in one large-scale survey, 0.5% of all introns were reported to use GC-AG (Burset et al., 2001), though this may be an underestimate, particularly for alternatively spliced introns, of which perhaps 5% might use GC-AG (Thanaraj and Clark, 2001)].

RT-PCR analysis was performed on LA3077, (a positive feedback construct with the CcTRA intron in the tTAV open reading frame). Transformed adult flies of both sexes were reared on diet substantially free of tetracycline ("off tetracycline") for 7 days. Flies were then collected for RNA extraction and RT PCR using primers (HSP- SEQ ID NO. 104 and VP16 SEQ ID NO. 105) were used to analyse the splicing pattern of the CcTRA intron (Figure 34). In two female samples we found the correct splice pattern of the Cctra (776bp, corresponding to precise removal of the Cctra intron) and saw no such band in males.

We found that LA3077 and LA3097 correspondingly gave repressible *female-specific* lethality. LA3077 was tested phenotypically through crossing flies heterozygous for LA3077 to wild type, on and off tetracycline. Female lethality ranged from 50 to 70%. LA3097 (a modified version of LA3077 whereby the Cctra intron immediately follows the start codon in the tTAV ORF), demonstrated a much higher level of female specific lethality, peaking at 100% (Figure 35). The Cctra intron was also inserted in tTAV2 at the same position as LA3097, in construct LA3233, and this gave a similar phenotypic result as LA3097 (Figure 35).

We have also prepared transformants of LA3077 in *Drosophila.* Phenotypically, the construct works perfectly, which is to say it is a highly effective female-specific lethal. However, sequencing of the splice variants of one of these insertions has shown that the splicing of this construct in *Drosophila* is not quite the same as it is in Medfly (SEQ ID NO. 57). The critical transcript, the female-specific one, is the same in both, but at least one of the non-sex-specific transcripts is different. It still incorporates extra exonic sequence, with stop codons, but the splice junctions are not quite the same (Figure 36). This observation is extremely important in that it shows that this method (regulation of gene expression by use of alternatively spliced introns) can be used across quite a wide phylogenetic range.

A simple test to determine whether an as yet uncharacterized exonic splice regulator (such as enhancers and suppressors) may be modifying the function of the alternatively spliced intron, could include making the construct and introducing it into a target tissue, then examining its splice pattern. In many cases this will not require germline transformation, so the test can be quite rapid, for instance by transient expression in suitable tissue culture cells or *in vivo.*

For instance, *in vivo* testing in insects could be achieved by delivering the DNA by microinjection. However, as the skilled person will appreciate, microinjection coupled with electroporation, or electroporation, chemical transformation, ballistic methods, for instance,have all been used in a number of various contexts and such methods of plasmid introduction and protein expression therefrom are will known in the art.

We have also recently made, and have obtained transgenics with, the Cctra intron in a different gene (LA3014) (all the above examples are in tTAV). LA3014 contains a ubiquitin-reaper$^{KR}$ fusion downstream of a Cctra intron. Phenotypic data (Figure 35) shows that LA3014 transgenic Medfly gave repressible *female-specific* lethality. RT-PCR analysis on RNA extracted from adult males and females raised off tetracycline, using primers (HSP, SEQ ID NO 74) and ReaperKR (SEQ ID NO. 75), demonstrate that correct splicing was occurring in females (508bp band) and no such band was found in males (Figure 37). LA3166 is another construct with the Cctra intron placed inside the ubiquitin coding region fused to reaper$^{KR}$, but placed in a different position in ubiquitin. LA3166 also produces a dominant repressible female-specific lethal effect in Medfly (Figure 35).

We have also recently made, and have obtained transgenics with, 'intron-only' Cctra-based constructs with the intron in a different gene (all the above examples are in tTAV or one of its variants, i.e. tTAV2 or tTAV3). These constructs work as predicted. This is an important result, thus showing that there are not essential exonic sequences in Cctra that we have simply duplicated (in function, if not necessarily in sequence) by chance, in tTAV. We also have ubi-rpr$^{KR}$ constructs of this type (LA3014 and LA3166), which also validates the ubiquitin fusion method described above.

In order to demonstrate the phylogenetic range of the Cctra intron we generated transgenic LA3097 and LA3233 *Anastrepha ludens.* LA3097 and LA3233 were selected for injection into *Anastrepha ludens* as they demonstrated the best female specific lethality in *Ceratitis capitata* (see Example 13). Phenotypic data was generated for 4 independent LA3097 lines and 1 LA3233 line (see Figure 38). Female specific lethality was generally somewhat lower in *Anastrepha ludens* when compared to C. *capitata* but reached 100% in one line.

*Anastrepha ludens* transformed with LA3097 and raised on tetracycline until eclosion were isolated and maintained off tetracycline for 7 days. RNA was then extracted and RT-PCR analysis was performed using primers HSP (SEQ ID NO. 76) and TETRR1 (SEQ ID NO. 77). The correct female specific (F1-like) splice pattern was observed RNA isolated from in females (348bp) but not from males demonstrating the function of the Cctra intron in a different species (Figure 39)

The brightest male band and the female specific band were purified and precipitated for sequencing. The female specific transcript was found to be correctly spliced in Mexfly females as expected for LA3097:

LA3097: AGCCACCATG GT...intron...AG GTCAGCCGCC

The two flanking sequences above are SEQ ID NOS. 2 and 3, respectively.

## Example 2: *Bactocera zonata* tra intron

We isolated the tra intron from *Bactocera zonata (B. zonata*) (SEQ ID NO. 58) using primers ROSA1 (SEQ ID NO. 78), ROSA2 (SEQ ID NO. 79), and ROSA3 (SEQ ID NO. 80).

These primer sequences were designed based on conserved coding sequence of *Ceratitis capitata* and *Bactrocera oleae* tra homologs. Using ROSA2 and ROSA3 or ROSA1 and ROSA3 as primers, the tra intron and its flanking coding region were amplified from *Bactrocera zonata* genomic DNA. Then we used these PCR products as a template and amplified the tra intron fragment to make the construct-LA3376 (Figure 31 and SEQ ID NO. 55). The primers (BZNHE-SEQ ID NO. 81 and BZR-SEQ ID NO. 82) were used for making the constructs; these primers contain additional sequences for cloning purposes. The Bztra intron in LA3376 is cloned into the ORF of tTAV3 and also of reaper$^{KR}$. Medfly transformants were generated and RNA extracted from male and female flies.

RT-PCR was then performed on both the reaper$^{KR}$ (HB- SEQ ID NO. 83) and Reaper KR- SEQ ID NO. 84) and tTAV3 (SRY- SEQ ID NO. 85) and AV3F- SEQ ID NO. 86) splice. The expected fragments of 200bp for reaper$^{KR}$ and 670bp for tTAV3, corresponding to splicing in a pattern equivalent to the F1 transcript of *Cctra* (Pane *et al.,* 2002), were generated in females (Figure 40).

## Example 3: Isolation and splicing of the *Ceratitis rosa (C. rosa,* Natal fruit fly) tra intron

Primers ROSA2 (SEQ ID NO. 87) and ROSA3 (SEQ ID NO. 88) were designed based on conserved coding sequence of *Ceratitis capitata* and *Bactrocera oleae.* Using ROSA2 and ROSA3 as primers, the tra intron and its flanking coding region were amplified from *Ceratitis rosa* genomic DNA (SEQ ID NO. 59). We then used the PCR products as a template and amplified the tra intron fragment to make constructs. The primers (CRNHE- SEQ ID NO 89 and CRR SEQ ID NO 90) were used during the construction of LA3242 (SEQ ID NO. 56 and Figure 32. LA3242 contains the *C. rosa* intron at the 5' end of the reaper$^{KR}$ ORF. *Ceratitis capitata* embryos were injected with DNA of LA3242, injected embryos were raised to adulthood on a diet substantially free of tetracycline. RNA was extracted from adult males and females; this was used as a template for RT PCR using primers HB (SEQ ID NO. 91) and ReaperKR (SEQ ID NO. 92). The expected female-specific splice band (200bp), corresponding to splicing in the equivalent pattern to that of transcript F1 of *Cctra,* was observed in females and not males (Figure 41).

## Double-sex

### Example 4 *Bombyx mori* dsx in PBW

The sequence of a *Bombyx mori* (silk moth) homolog of *Drosophila* Dsx (*Bmdsx*) has been previously described and a male- and a female-specific splice product have been identified (Suzuki et al, 2001). Both males and females use the same 3' polyA, and there are two female specific exons. One paper has suggested that the sex-specific splicing is not dependent on tra/tra2, in other words even though the pattern looks the same, the underlying mechanism may be different (Suzuki et al., 2001), though their data, principally the lack of recognisable tra-tra2 binding sites, however, is not compelling. In addition, a *B. mori* dsx mini-gene construct (containing exonic sequence and truncated intronic sequence) has been transformed into *B. mori* and the germline transformants show sex-specific splicing (Funaguma et al., 2005).

We have generated a Bmdsx minigene based on the sequence used in the Funaguma et al paper, with some significant changes, and injected this into the moth Pink Bollworm to ascertain if one can obtain sex-specific splicing in a divergent species. The mini-gene construct we generated does not included exon 1, which is present in both males and females. In addition, we removed the intron between exon 3 and 4 (the two female specific exons), included a heterologous sequence (containing multiple cloning sites, MCS), used the Hr5-IE1 enhancer/promoter sequence from the baculovirus *Ac*NPV and used a 3' transcriptional termination sequence derived from SV40 (see Figure 42 for a schematic). The individual exon/flanking intron fragments used were amplified and recombined together by PCR and ligated into a construct carrying a Hr5/IE1 enhancer promoter fragment and SV40 3'UTR (Figure 22 and SEQ ID NO. 22).

LA3435 was injected into pink bollworm (*Pectinophora gossypiella*) embryos. First instar larvae were collected after 5-7 days and analysed individually by RT-PCR (using primers IE1 transcr- SEQ ID NO. 93 and SV40-RT-P2- SEQ ID NO. 94) to determine if BMdsx can undergo male and female specific splicing (Figure 43). Our analysis detected the male specific band (predicted to be 442bp) in 4 samples (Lanes 1, 2, 3 and 4) and the female specific band (predicted to be 612bp) in 1 sample (Lane 5).

The correct splicing of *B. mori* dsx in PBW demonstrates that we can achieve (have achieved) sex-specific expression of a heterologous sequence (here, the MCS) in a Lepidopteran by utilizing an alternative splicing system. Furthermore, since this splicing system was derived from a heterologous species, this suggests that such constructs might work over a wide phylogenetic range. However, the identification of alternative splicing systems in the species of interest is also envisioned, and methods for identifying such alternative splicing systems are provided herein or will be known to the person skilled in the art. By providing a MCS in our Example (see Figure 42), the expression of a sequence of interest, for example a coding region for a protein of interest could readily be achieved by inserting said sequence. If said sequence encoded a suitable protein, a sex-specific phenotype, for example conditional sex-specific lethality, could thereby be introduced, for example into pink bollworm.

### Example 5: Isolation of Codling moth dsx

The dsx gene from Codling moth (*Cydia pomonella)* was isolated by performing 3' RACE using primers which were based on sequence alignments from *B. oleae, B. tyroni, C. capitata, D. melanogaster, B. mori,* and A. *gambiae.* RNA was isolated from a male and female codling moth and 3' RACE , to generate cDNA, was performed using the TT7T25 primer (SEQ ID NO. 95).

PCR was performed using the primers dslc (SEQ ID NO. 96) and TT7 (SEQ ID NO. 97). Two rounds of nested PCR were then performed on the product of the first PCR using the primers codling2a (SEQ ID NO. 98) and TT7 (SEQ ID NO. 99) and the product of the second round of PCR using Codling2b (SEQ ID NO. 100) and TT7. The isolated male and female specific sequences share sequence similarity to previously isolated dsx homologues (Male-SEQ ID NO. 43 and Female- SEQ ID NO. 42).

### Example 6: Isolation of PBW dsx

The dsx gene from pink bollworm was isolated by performing 3' RACE using primers which were based on sequence alignments from *B. oleae, B. tyroni, C. capitata, D. melanogaster, B. mori,* and *A. gambiae.* RNA was isolated from a male and female codling moth and 3' RACE , to generate cDNA, was performed using TT7T25 (sequence defined herein). PCR was performed using the primers Pbwdsx2 (SEQ ID NO. 101) and TT7 (SEQ ID NO. 102). Nested PCR was then performed on the product of the first PCR using the primers Pbwdsx3 (SEQ ID NO. 103) and TT7. Three female specific sequences were isolated: PBWdsx-F1 (SEQ ID NO. 40), PBWdsx-F2 (Figure 10), and PBWdsx-F3 (SEQ ID NO. 71) and one male specific sequence (SEQ ID NO. 42). The isolated male and female specific sequences share sequence similarity to previously isolated dsx homologues.

**Example 7: *dsx* in *Anopheles gambiae***

The sequence of the *dsx* gene of *Anopheles gambiae* has previously been described (Scali *et al* 2005). However, when we have tried to repeat the work described in the paper we find that there are some differences in the splicing that occurs. When we tried to repeat the amplification of the female specific transcript using primers designed from the mRNA sequence (Accession; AY903308 for female coding sequence and AY903307 for male coding sequence), the amplification failed. However, when Scali and colleagues showed that there was a shared exon, which had previously not been described, we designed primers to amplify the entire *dsx* transcript and gene. Using these primers and primers designed from genomic DNA sequence (Accession; GI:19611767) we find that the splicing of the female transcript is different from that described by Scali *et al* 2005 (Figure 44). The transcript showed that the female exon was in a different position. There are several explanations for these differences, but the most likely are either some sort of strain difference in the *Anopheles* that we used to get the data from, or the published sequence is not from *Anopheles gambiae,* or there is more than one female isoform as shown for *Stegomyia aegypti* in Example 20.

We have also successfully used primers, designed around our version of the *Anopheles gambiae dsx* splicing, that are able to distinguish between males and females of *Anopheles gambiae* (Figure 45). This provides good evidence that the system will be functional as a sex-specific splicing mechanism when fused to a protein of interest, such as tTAV or a killer.

The *Anopheles gambiae dsx* gene that we have isolated from genomic DNA, which has several changes in nucleotide sequence compared to the reported genomic sequence, was cloned into LA3359 (SEQ ID NO. 47) and LA3433 (SEQ ID NO. 48), schematics can be found in Figures 23 and Figure 24, respectively.

**Example 8: *dsx* in *Stegomyia aegypti***

The splicing of the gene appears to be similar to *Anopheles gambiae dsx* (Scali *et al* 2005). The *Stegomyia aegypti dsx* gene is illustrated diagrammatically in Figure 47 or 48. A male-specific transcript (M1) is produced which does not include exons 5a or 5b. Two female specific splice variants (F1 and F2) have the following structure; F1 comprises exons 1-4, 5a, 6 and 7 but not 5b, F2 comprises exons 1-4 and 5b (figure 46). In addition, a further transcript (C1) is present in both males and females; this comprises exons 1-4 and 7, but not exons 5a, 5b or 6.

The splicing of the gene appears to be similar to *Anopheles gambiae dsx* (Scali *et al* 2005). The *Stegomyia aegypti dsx* gene is illustrated diagrammatically in Figure 47 or 48.

**<u>Actin 4</u>**

**Example 9: *Stegomyia aegypti Actin-4* gene**

One way to get sex-, tissue- and stage-specific expression of a gene of interest is to link it with the *Stegomyia aegypti Actin-4 (AeAct-4)* gene. This gene is only expressed in the developing flight muscles of female *Stegomyia aegypti* (Munoz et al 2004). They used in-situ hybridisation to an RNA to detect the expression profile of *AeAct-4.* We have taken a fragment of the *Stegomyia aegypti Actin-4* gene, comprising a putative promoter region, an alternatively spliced intron, and a section of 5' untranslated region (UTR) and placed it in front of sequence coding for tTAV (Figure 49) to test the function of the sex specific splicing when fused to tTAV.

We integrated LA1172 into the *Stegomyia aegypti* genome using *piggyBac.* Two independent lines were generated (lines 2 and 8). Both of these lines show the correct splicing of the Actin-4-tTAV gene (Figures 50 and 51). The Actin-4 promoter and alternatively spliced intron can therefore be used successfully to provide sex-, tissue- and stage-specific splicing of a gene of interest in *Stegomyia aegypti.*

**<u>Description Of The Figures And Sequence Listings of Examples 1-9</u>**

**Figure 19: One use of the P element in generating germline-specific expression of a gene of interest (Gene E).**

Insertion of the P element IVS3 and flanking exonic sequences upstream of an ubiquitin-Gene E fusion with allow germline-specific expression of Gene E under a germline active promoter. A - Germline active promoter; B - P-element open reading frame; C - P intron 'IVS3'; D - Ubiquitin; E - Coding region for protein of Interest e.g. tTAV.

**Figure 20: Sex-specific expression using *dsx.***

A: Intron used as *Cctra* intron above, but giving male-specific expression. A fragment of *dsx* (here the *Anopheles* version) is inserted into a heterologous coding region (shaded boxes). The intron is completely removed in males, but in females the coding region is prematurely terminated.

B: An alternative approach to male-specific expression, in which a heterologous coding region is fused to a fragment of *dsx*.

C: Female-specific expression: the heterologous coding region is inserted into the female-specific exon, either as an in-frame fusion to a fragment of Dsx, or with its own start and stop codons.

D: Differential expression: designs B and C can be combined to give expression of gene *a* in females and *b* in males.

**Figure 21: Sex-specific alternative splicing of *Cctra***

*A: Cctra* is spliced in females to produce three transcripts: F1, which encodes functional Tra protein, and M1 and M2, which do not, because they include additional exons with stop codons (redrawn from Pane *et al.* 2002). Males produce only transcripts M1 and M2 and therefore do not produce functional Tra protein at all.

*B* If this intron were to function similarly in a heterologous coding region, this would similarly allow females, but not males, to produce functional protein X.

**Figure 22:** Diagrammatic representation of pLA3435 construct/plasmid (SEQ ID NO. 46).

**Figure 23:** Plasmid map of pLA3359 *Anopheles gambiae* dsx gene placed under the control of a Hr5-IE1 promoter for assessing splicing *via* transient expression.

**Figure 24:** pLA3433-Anopheles gambiae dsx gene placed under the contronl of a Hr5-IE1 promoter, with the addition of exon 2, for assessing splicing *via* transient expression.

**Figure 25:** Schematic representation of pLA1188 construct.

**Figure 26:** Schematic diagram of pLA3077 construct.

**Figure 27:** Schematic diagram of pLA3097 construct.

**Figure 28:** Schematic diagram of pLA3233 construct.

**Figure 29:** Schematic diagram of pLA3014 construct.

**Figure 30:** Schematic diagram of pLA3166 construct.

**Figure 31:** Schematic diagram of pLA3376 construct.

**Figure 32:** Schematic diagram of pLA3242 construct.

**Figure 33: Flanking sequence of *Cctra*** Splicing of the Cctra intron in LA3077 and LA3097 is exactly as you would see in the native Cctra intron. Splicing in LA1188 results in the removal of 4 additional nucleotides. In all cases the introns are flanked by 5' exonic TG and 3' GT.

**Figure 34: Gel showing correct sex- specific splicing of intron(s) derived from CcTra (776bp band in females) in *Ceratitis capitata* transformed with LA3077.** Lane 1: Marker (SmartLadder™ from Eurogentec, bands of approx 0.8, 1.0 and 1.5kb are indicated); Lanes 2 and 3: *Ceratitis capitata* LA3077/+ males; Lanes 4 and 5: *Ceratitis capitata* LA3077/+ females.

**Figure 35: Phenotypic data for transformed female specific constructs in *Ceratitis capitata.*** Column 1: Construct designation LA#, e.g. LA3077, LA3097, LA3233, etc, is indicated by number, with independent insertion lines referred to by letter; Columns 2 and 3: Non-tetracycline (NT) results for each transformed line given in total males (2) and total females (3). Columns 4 and 5: Tetracycline (TET) results for each transformed line given in total males (4) and total females (5).

**Figure 36: Transcripts of Cctra intron constructs in *Drosophila* and *Ceratitis capitata.*** The top line represents the construct DNA containing tra intron flanked by desired gene (the open box). The red box represents the male specific exons. Introns are represented by solid lines. Arrow above the first line represents the positions of the oligonucleotides used in the RT-PCR experiments. The bar indicates the scale of the figure.

**Figure 37: Gel showing correct female specific splicing of CcTRA-derived sequence (508bp band) in female *Ceratitis capitata* transformed with LA3014.** Lane 1: Marker (SmartLadder™ from Eurogentec, bands of approx 0.4 and 1.0kb are indicated); Lane 2 *Ceratitis capitata* LA3014/+ male; Lane 4: *Ceratitis capitata* LA3014/+ female; Lanes 3 and 5: no reverse transcriptase negative controls (background bands, probably from genomic DNA, can be seen in lanes 2 and 4).

**Figure 38: Phenotypic data for transgenic *Anastrepha ludens* transformed with LA3097 or LA3233.** Column 1: Construct LA# (LA3097 or LA3233) indicated, with independent insertion lines referred to by letter; Columns 2 and 3: Non-tetracycline (NT) results for each transformed line given in total males (2) and total females (3). Columns 4 and 5: Tetracycline (TET) results for each transformed line given in total males (4) and total females (5).

**Figure 39: Gel showing correct sex-specific splicing of CcTRA splicing (348bp band in females) in *Anastrepha ludens* transformed with LA3097.** Lane 1: Marker (SmartLadder™ from Eurogentec, bands of approx 0.4 and 1.0kb are indicated); Lanes 2, 3 and 4: A. *ludens* LA3097/+ males; Lanes 5, 6 and 7: A. *ludens* LA3097/+ females.

**Figure 40: Gel showing correct sex-specific splicing of BzTRA in reaperKR (200bp band in females) and tTAV3 (670bp band in females) regions of LA3376, in *Ceratitis capitata* transformed with LA3376.** Lane 1: Marker (SmartLadder™ from Eurogentec, bands of approx 0.2, 0.6 and 1.0kb are indicated); Lanes 2 and 3: C. *capitata* LA3376/+ males tested for splicing in reaperKR; Lanes 4 and 5: *C. capitata* LA3376/+ females tested for splicing in reaperKR; Lane 6: SmartLadder™; Lanes 7 and 8: *C. capitata* LA3376/+ males tested for splicing in tTAV; Lanes 9 and 10: *C. capitata* LA3376/+ females tested for splicing in tTAV; Lane 11: SmartLadder™.

**Figure 41: Gel showing correct sex-specific CrTRA splicing in CrTRA-reaperKR (200bp band in females) in *Ceratitis capitata* injected with LA3242.** Lane 1: Marker (SmartLadder™ from Eurogentec, bands of approx 0.2, 0.6 and 1.0kb are indicated); Lanes 2-7: *C. capitata* wild type males injected with LA3242; Lane 8: SmartLadder™; Lanes 9-14: *C. capitata* wild type females injected with LA3242; Lane 15: SmartLadder™.

**Figure 42: Schematic representation of Bmdsx minigene constructs.** Two minigene constructs derived from the *Bombyx mori dsx* gene are illustrated diagrammatically, together with the predicted alternative splicing of these constructs (female pattern shown above the construct, male pattern below). (A) is the *Bombyx mori dsx* mini-gene construct used in Funaguma et al., 2005) (B) is pLA3435. A and B differ from each other in several ways: (i) Exon 1 is excluded from pLA3435, (ii) the intron between female specific exons 3 and 4 has been removed and a short heterlogous sequence has been inserted in pLA3435 (iii) Funaguma et al., use the ie1 promoter from the baculovirus *Bm*NPV and a *Bm*A3 3'UTR compared with pLA3435 which uses the hr5-IE1 enhancer/promoter from the baculovirus *Ac*NPV and a 3'SV40 3'UTR. (iv) pLA3435 uses slightly longer intron sequences when compared with (A) (see Figure 15 for sequence). Two minigene constructs derived from the *Bombyx mori dsx* gene are illustrated diagrammatically, together with the predicted alternative splicing of these constructs (female pattern shown above the construct, male pattern below).

**Figure 43: Sex-specific splicing of BMdsx mini-gene construct in PBW.** Analysis of transient expression from pLA3435 using RT-PCR show the presence of a 442bp fragment (Lanes 1,2,3 and 4) in males and a 612bp fragment in females (Lane 5), showing that the BMdsx mini-gene with a heterologous fragment inserted between exon 3 and 4 is able to splice correctly in the divergent moth, PBW. Markers are SmartLadder™ from Eurogentec; bands of approx 0.2, 0.4 and 0.6 kb are indicated

**Figure 44: Sex-specific splicing of *Anopheles gambiae dsx.*** Anopheles (A) shows the splicing that was reported by Scali *et al* 2005. However, when RT-PCR was performed using our primers (spl-agdsx-e3 (SEQ ID NO. 60) and spl-agdsx-m (SEQ ID NO. 61)) a different splicing pattern for females was revealed, represented by Anopheles (B).

**Figure 45: Identification of male and female *Anopheles gambiae* using *dsx* primers.** RNA was extracted from male and female *Anopheles gambiae* and the *dsx* transcripts were amplified by RT-PCR using the primers spl-agdsx-e3 (SEQ ID NO. 62) and spl-agdsx-m (SEQ ID NO. 63); the resulting banding pattern is shown in the gel above. The expected bands for the male and female transcripts are indicated by the white arrows, the bands have been cloned and sequenced and are identical to the predicted sequence of our version of the *dsx* transcript (see SEQ ID NO. 47 (LA3359) and SEQ ID NO. 48 (LA3433)). The molecular weight markers are shown in kb (SmartLadder™ from Eurogentec; sizes are approximate).

**Figure 46; Identification of male and female *Stegomyia aegypti* using dsx primers.** The primers for the Stegomyia aegypti RT-PCR for A and B were aedesxF1 (SEQ ID NO. 64) and aedesxR5 (SEQ ID NO. 65) were tested initially on pupae, a life stage of *Stegomyia aegypti* that can be sexed conveniently and accurately; the resulting RT-PCR amplification is shown on gel image (A). The male and female pupae show a distinctive sex specific band. Then the primers were tested on RNA extractions from larvae, which can not be readily sexed by their morphology and the resulting RT-PCR amplification shown on gel image (B). The larvae show a clear banding pattern which distinguishes males from females unambiguously. Gel image (C) shows an approximately 600bp band from RT-PCR using the primers aedessxF1 and aedesxR2 (SEQ ID NO. 66) from individual male and female pupa. Sequencing of this band showed a female specific splice variant which does not appear to possess the male shared exon to which aedesxR5 is predicted to anneal (exon 7, see figure 56). The molecular weight markers are shown in kb (SmartLadder™ from Eurogentec; sizes are approximate).

**Figure 47: Diagrammatic representation of part of the *Stegomyia aegypti dsx* gene (not to scale).** A fragment of the *Stegomyia aegypti dsx* gene is represented above. Exons 5a and 5b are female specific and exon 6 is a male specific exon. Two female-specific splice variants have been found (F1 and F2) which comprise exons 1-4,5b,6 and 7 (F1) or 1-4,5a (F2); transcripts in males (M1) comprise exons 1-4,6 and 7 but not exon 5a or 5b and a transcript (C1) of 1-4 and 7 but not exons 5a, 5b or 6 is shown in males and females. The numbers for each of the exons after # relates to contig 1.370 (http://www.broad.mit.edu/annotation/disease vector/aedes aegypti/), which reads in the opposite orientation, and after * relate to the nucleotide sequence shown in SEQ ID NO. 43.

**Figure 48: Diagrammatic representation of the *Stegomyia aegypti dsx* gene.** The entire *Stegomyia aegypti*

*dsx* gene is represented above Exon 5 is the female specific exon and exon 6 is a putative male specific exon. In principle, transcripts in females comprise exons 1,2,3,4,5 and 7, and males comprise exons 1,2,3,4,6 and 7. The numbers for each of the exons after # relates to contig 1.370

(http://www.broad.mit.edu/annotation/disease vector/aedes aegypti/) reading in the opposite orientation, and after * relate to figure 12.

**Figure 49: Plasmid map of pLA 1172.**

A coding region for tTAV has been placed under the control of a fragment from the *Stegomyia aegypti* actin-4 gene (Munoz *et al* 2005) which includes the 5' UTR, first intron, and upstream sequences (putative promoter). The construct also contains a $tetO_7$ Nipper sequence. The construct has *piggyBac* ends and a DsRed2 marker for stable integration into a genome.

**Figure 50: Sex-specific splicing of tTAV in LA1172 transformants.**

Gel image of RT-PCR of RNA extracted from LA1172 line 2 male and female pupa. The primers used were Agexon1 (SEQ ID NO. 67) and Tra (tTAV) seq+ (SEQ ID NO. 68). Sequencing of the RT-PCR bands showed the expected splicing occurring in males and females. The data shown in the above diagram is for LA1172 line 2, line 8 showed exactly the same results (data not shown). Markers are SmartLadder™ from Eurogentec; approximate sizes are indicated, in kb).

**Figure 51: RT-PCR of wild type samples, showing sex-specific splice variants of the *Stegomyia aegypti* Actin-4 gene**.

Gel image of RT-PCR of RNA extracted from different developmental stages, and dissections of adults, of LA1172 line 8. The primers used were Agexon1 (SEQ ID NO. 69) and Exon 3 (SEQ ID NO. 70). The gel image shows that strong expression from the Actin-4 gene only occurs at the pupal stage, and that adult expression is generally limited to the female thorax where the flight muscles are found. Table 17, below show the contents of each lane.

Table 1.

| | |
|---|---|
| E = pool of ~100 embryos | MH = head from male adult |
| L4 = 4th instar larva | MT = thorax from male adult |
| ME = early male pupa (<4hours old) | MA = abdomen from male adult |
| FE = early female pupa (<4hours old) | FH = head from female adult |
| MP = male pupa | FT = thorax from female adult |
| FP = female pupae | FA = abdomen from female adult |
| | -ve = water control |

## Further Examples

**Example 10: Moths.**

We have newly made constructs based on our transient expression data using a recombinant minigene construct derived from Bombyx mori. This is discussed further below in the section entitled "Moth *dsx* sequence alignment and conserved motifs"

**Example 11: Use of Bztra**

We have newly made two *Bztra*-based constructs, expressed in Mexfly (LA3376). LA3376 gives repressible female-specific lethality. LA3376 we have previously shown to function and splice correctly in Medfly. Transformants in Mexfly (*Anastrepha ludens)* were also generated with LA3376. These were analysed for correct splicing of the Bztra intron in order to demonstrate the phylogenetic range of the Bztra intron by RT-PCR using primers SRY and AV3F (Figure 15 and "Medfly RT-PCR gels" section above). This shows correct splicing of the Bztra intron in Mexfly.

**Example 12: Dmdsx in Medfly (DmDsx in transgenic Medfly example: nipper fusion in #797)**

We also have newly made data on a Dmdsx construct in Medfly. The construct used a fragment of the *Drosophila melanogaster* gene *doublesex* to give sex-specific expression of a fragment of the *Drosophila melanogaster* gene *Nip-*

*plDm* (we call this fragment "nipper"). We didn't see clear sex-specific splicing. However, the phenotypic data shows some sex-specificity; we saw increased lethality of females, to about 75% penetration. Of course this incomplete penetrance could be due to expression level, lack of toxicity of nipper in Medfly, etc. We also had a significant reduction in the number of males, but the tTA source, LA670, used in this experiment could itself be killing some of the males.

We have tested three independent Medfly transgenic lines that carry a fusion of nipper to DmDsx sequence that was intended to be expressed specifically in females. This construct may not have worked perfectly possibly due to essential sequence for correct alternative splicing and/or the Sxl binding sites required by DmDsx, and since Medfly do not use Sxl in the sex-determining pathway, DmDsx may be unable to completely splice this fusion in the correct way in Medfly. However, we were successful in reproducibly causing increased lethality in females compared to males across all three lines at a very similar efficiency (approximately 75% more lethality observed in females than in males). This demonstrates the dsx system can work across quite distantly related species (evolutionary separation is around 120-150 Million years), and if the Ccdsx sequence were used it may have well worked due to the Sxl requirement of Dmdsx .

The 797 results are shown below, using a Tet014 dsx splice nipper (Pub EGFP) system. They show that this system is lethal at the larval stage (~50%), and is likely to be acting more successfully in females (~75%). 797 is marked with green (G), 670 with red (R). 670 is a tTAV source, so one expects to see a phenotype in the R+G flies; G (and R) only are controls. NF - non-fluorescent (i.e. wild type) is also a control where included. All progeny reared on tet-free media.

All three Independent Lines seem to act in similar way.

| | | | |
|---|---|---|---|
| 797A/797A M2 x 670A/+: | | | |
| | Pupae | Adults | Males: Females |
| G | 184 | 176 | 85: 91 |
| R+G | 74 | 57 | 44: 13 |
| 797C/797C M1 x 670A/+: | | | |
| | Pupae | Adults | Males: Females |
| G | 169 | 157 | 89: 68 |
| R+G | 94 | 67 | 54: 13 |
| 797C/797C M2 x 670A/+: | | | |
| | Pupae | Adults | Males: Females |
| G | 406 | 377 | 179: 198 |
| R+G | 171 | 147 | 121:26 |
| 670A/+ x 797C/+ M2: | | | |
| | Pupae | Adults | Males: Females |
| NF | 198 | 192 | 92: 100 |
| G | 162 | 147 | 67: 80 |
| R | 149 | 72 | 43: 29 |
| R+G | 45 | 22 | 20: 2 |

Average of all 3 lines: number of R+G females = 21 % of the number of R+G males, therefore substantial excess mortality in R+G females relative to males. This effect is not seen in R only or G only control females, nor in wild type.

**Examples 13-15:**

We have newly demonstrated:

(5) sex-specific splicing in recombinant *Aadsx*-based minigene constructs;
(6) sex-specific phenotype from a *Cctra*-based construct; and
(7) sex-specific splicing in Aedes-Actin4 -based constructs.

At least some of each of these examples not only shows minigenes, but actually shows splicing to generate tTAV/tTAV2 or ubi-tTAV2

**Example 13: Aedes doublesex (dsx) minigenes**

See also section entitled *Aedes dsx* Tra2 binding sites. We have isolated the *Aedes aegypti dsx* gene (*Aadsx*) and identified 6 transcripts from this region (Figure 1). These are: 2 male-specific transcripts (M1 and M2), 3 female-specific transcripts (F1, F2 and F3) and a transcript found in both males and females (MF). We made two minigene constructs. In these constructs, the large majority of the intronic sequence was deleted. For example, DSX minigene1 is approximately 4.4kb in length, whereas its terminal sequences are separated by approximately 26kb in its natural context, i.e. in the genomic DNA of *Aedes aegypti.*

The splicing in minigene2 of Figure 1 is illustrative as splicing occurs in the "female" form in both males and females. This may mean that this system depends on alternative splice acceptor use. In this model, there is competition between alternative splice acceptors, with some sex-specific factor biasing this, the sex-specific factor probably being Tra. But deleting the M1 and M2 3' splice acceptors forces splicing in the F forms, by removing the alternative.

Therefore, it is preferred that one or more of the female-specific (F1 and/or F2) 3' splice acceptors are provided together with an additional 3' splice acceptor. Most preferably, said additional splice acceptor is the 3' splice acceptor of M1 or M2 splice variant (or both), although it is envisaged that this is not essential as other known 3' splice acceptors are likely to function.

Figure 1 illustrates the various transcripts produced by alternative splicing of the *Aedes aegypti doublesex* gene (*Aadsx*). It will be appreciated that *Aedes aegypti* is also known as *Stegomyia aegypti.* The figure shows the *Aadsx* gene from the fourth exon, which is not alternatively spliced, i.e. is present in all transcripts discussed here. Numbering is from the first nucleotide of the fourth exon (acgacgaact...). Note that the diagram is not to scale - the introns are much longer than the exons. The total alternatively spliced region comprises over 43kb.

This minigene fragment was included in an expression construct (LA3515). Transgenic *Aedes aegypti* were generated by site-specific recombination into an *att*P site, using the method of Nimmo et al (2006 : Nimmo, D.D., Alphey, L. Meredith, J.M. and Eggleston, P (2006). High efficiency site-specific genetic engineering of the mosquito genome. Insect Molecular Biology, 15: 129-136)

A second, smaller minigene was constructed similarly (DSX minigene2) and an expression construct for this was inserted into the same *att*P site as DSX minigene1, to allow direct comparison (LA3534). DSX minigene2 did not show sex-specific splicing. This indicates that sequences present in DSX minigene1 but not in DSX minigene2 (approx 2029bp, see Fig 1 and SEQ ID NO. 150, where exons are found at positions 29-163 and 1535-2572) are essential for correct alternative splicing, even though the first alternatively spliced intron, and the exonic sequence immediately flanking it, is present in both constructs.

We have produced two transgenic lines (LA3491 and LA3534) using minigene constructs of *Aedes aegypti dsx* gene. LA3491 is a fusion of shared exon4, the female-specific cassette exons, and part of the first shared 3' exon (exon 5 in transcript M1).

Transcripts from the minigene region of LA3491 were analysed by reverse transcriptase PCR (RT-PCR) and sequencing. Transcripts corresponding to alternative splicing in the F2 form were found in females but not in males (Fig 2 and 3) and in the F1 form there was some male expression but it was very low (Fig 4). While transcripts corresponding to the M1 form were detected in males but not in females (Fig 2). Since the minigene did not contain the 3' splice acceptor of the M2 variant, this transcript was not possible from this construct. This minigene does not contain any exogenous sequence, though it clearly demonstrates sex-specific splicing of an Aadsx fragment, indeed a highly deleted "minigene" fragment.

It will be apparent that certain sequences are important for controlling splicing and should therefor be retained, as discussed elsewhere. This can be easily established by deletion of certain portions and testing for alternative splicing by RT-PCR for instance.

Figure 2 shows RT-PCR of males and females from LA3491 *Aedes aegypti* transgenic line using the primers 688 - ie1-transcr (SEQ ID NO. 4) and 790 - Aedsx-m-r2 (SEQ ID NO. 5). Using these primers, splicing in the F2 pattern would give a band of approximately 985bp while splicing in the M1 pattern would give a band of approximately 516bp. A band of approx 985bp (F2) appeared only in lanes representing females and a band of approx 516bp male specific transcript 1 (M1) appeared only in males. These bands have been sequenced and show that correct splicing had occurred, i.e. F2-type and M1-type respectively. The absence of bands in the no RT controls (-RT CON) shows that there was no genomic DNA contamination in the samples. Lanes 1 and 11 are Marker (SmartLadder™ from Eurogentec, bands from 1.5kb to 0.2kb are indicated). Lanes 2 and 3 are negative controls (no reverse transcriptase) and lanes 2-9 represent reactions performed on extracts from males or females as marked.

Figure 3 shows RT-PCR of males and females from LA3491 Aedes aegypti transgenic lines using the primers 688 - ie1-transcr (SEQ ID NO. 4) and 761 - Aedsx-fem-r (SEQ ID NO. 6). Using these primers, splicing in the F2 pattern would give a band of approximately 525bp. A band of approximately 525bp was present in reactions on extracts from females, but not from corresponding reactions on extracts from males. Sequencing of this 525bp band confirmed that correct, i.e. F2-type splicing had occurred. Marker (SmartLadder™ from Eurogentec, bands from 1.5kb to 0.2kb are indicated).

Figure 4 shows RT-PCR of males and females from LA3491 *Aedes aegypti* transgenic lines using the primers 688 - ie1-transcr (SEQ ID NO. 4) and AedsxR1 (SEQ ID NO. 4). Using these primers splicing in the F1 pattern would give a band of 283bp. A band of approximately 283bp is present predominantly in females, although there is evidence of a small amount of splicing in males. Sequencing confirmed that this band did indeed correspond to splicing in the F1 pattern. Marker (SmartLadder™ from Eurogentec, bands from 1.5kb to 0.2kb are indicated).

LA3534 is identical to LA3491 except for a 3' deletion of approx 2kb. This construct showed no differential splicing between male and females (Fig 1, minigene 2). RT-PCR gels have not been shown for this case. Based on these results several constructs have been designed to incorporate the sex-specific splicing of LA3491 (Fig 1, minigene 1) into a positive-feedback system. LA3612 (Fig 5), which incorporates a fusion of ubiquitin and tTAV2 into the *dsx* coding region, is designed so that when the F2 female transcript is produced, the ubiquitin is cleaved and the tTAV2 is released to initiate and sustain the positive feedback system. LA3619 (Fig 5) has tTAV2 without ubiquitin and using its own translation start codon. LA3646 (Fig 5) is identical to LA3619 except the start codons for the *dsx* gene have been mutated; this should improve the quantity of tTAV2 produced by removing non-specific translation.

Figure 5 is a diagrammatic representation of plasmids based around the splicing in *Aedes aegypti dsx* minigene. For clarity it will be understood that the first female intron represents any of F1, F2 or F3 splicing, and tTAV in the diagram refers to tTAV2 (it will be appreciated that other proteins or other versions of tTA or tTAV could alternatively be used). In each of these plasmids, apart from LA3491, heterologous sequence has been added to the F2 exon. "Putative ATG" represents any ATG triplet sequence in exonic sequence located 5' relative to the heterologous DNA. In LA3646 these putative translation start codons ("putative ATG") were removed or modified. In the case of construct LA3612, translation from an upstream (5') ATG that is in frame with the ubi-tTAV coding region will still (assuming no intervening stop codon) produce functional tTAV, following separation of the ubiquitin and tTAV moieties by protease action. The various alternative splicing cassettes are operably linked to a suitable promoter, transcriptional terminator and other regulatory sequences.

This example shows sex-specific splicing of a highly compressed "minigene" fragment in a heterologous context (i.e. heterologous promoter, 5' UTR and 3'UTR). Although it does not show differential expression of a non-*Aedes* sequence, as the alternatively spliced exons are derived from the *Aadsx* gene and do not contain additional material, it does clearly illustrate the feasibility of this approach. In any case, the promoter, 5' UTR and 3'UTR are heterologous. We have additional constructs which illustrate several different methods for obtaining differential (sex-specific) expression of a heterologous protein by this *dsx.*

### TRA sequence alignment

Pane et al. (2002) suggested that certain sequences related to the known binding sites of the Tra/Tra-2 complex in *Drosophila* might be important in regulating the splicing of Cctra, and this also known for *Drosophila dsx* and has also been suggested for *Anopheles gambiae dsx* (Scali et al 2005). The consensus sequence is variously described as
UC(U/A)(U/A)C(A/G)AUCAACA (Pane et al), SEQ ID NO. 8, or
UC(U/A)(U/A)CAAUCAACA (Scali et al 2005), SEQ ID NO. 9.

It is noteworthy that these definitions are extremely similar. Pane *et al* identify 8 partial matches to this consensus in the Cctra sequence (7 or more nucleotides matching the 13 nucleotide consensus sequence. Scali et al identify 6 matches in *Agdsx* (9/13 or better). Such sequences are also known to regulate the alternative splicing of the *Drosophila* gene *fruitless*; Scali *et al* review 3 matches in that sequence (12/13 or better). Correct splicing of *dsx* may also require a purine-rich region, as discussed by Scali *et al.*

As can be seen from the Table 2 and Figure 7, we have identified what are thought to be significant clusters of binding sites for Tra/Tra2 in our *Aedes aegypti dsx* minigene1.

### Moth *dsx* sequence alignment and conserved motifs

Figure 6 shows an alignment of the second female-specific exons and flanking sequences of dsx genes from pink bollworm (*Pectinophora gossypiella,* PBW-dsx, SEQ ID NO. 146), silk worm (*Bombyx mori,* bombyx-dsx, SEQ ID NO. 147) and codling moth (*Cydia pomonella,* codlingdsx, SEQ ID NO. 148). The second female-specific exon is shown in bold. We identified multiple copies of a short, repeated nucleotide sequence, conserved in sequence and approximate location between these relatively distantly related moths; these are located just 5' to the female-specific exon. The conserved repeats AGTGAC/T are underlined. Asterisks (*) represent identical nucleotides, dashes (-) represent gaps for best alignment. The exons are represented in the SEQ ID NOS. by the following nucleotide numbering: SEQ ID NO. 146 289-439; SEQ ID NO. 147 339-492; and SEQ ID NO. 148 285-439.

### *Aedes dsx* Tra2 binding sites.

In females of *Drosophila melanogaster,* Tra and a product from the constitutively active gene *tra2,* act as splicing regulators by binding to splice enhancer sites on the pre-mRNA of *dsx,* which activates the weak 3' acceptor site of the female-specific exon (Scali et al). In males there is no expression of TRA and the weak 3' acceptor site is not recognised and splicing occurs at the male exon. To look for putative Tra/Tra2 binding sites we used the consensus sequence of these binding sites deduced for *Drosophila* Tra/Tra2 and looked for the distribution of these in the *Aedes aegypti dsx* gene sequence. This is shown in Table 2, below.

Table 2: * = in 3491, only 9/13 but 6/6 in core. This table does not include 9/13 identities apart from the ones that are in 3491 with 6/6 identity with core sequence of wwcrat. This consensus core sequence (WWCRAT) is particularly preferred.

| Name | Sequence<br>w = T or A<br>r = A or G | Present in Minigenel 1 | Position | Identity with consensus | Identity with wwcrat | SEQ ID NO. |
|---|---|---|---|---|---|---|
| | | | | | | |
| Consensus | tc**wwcrat**caaca | / | / | /13 | /6 | 138 |
| | | | | | | |
| 1 | tcaacaagcaaca | Y | 14917 | 12 | 5 | 10 |
| 2 | ttatcaaacaaca | Y | 364 | 11 | 5 | 11 |
| 3 | tcatcaattaaaa | | 1015 | 11 | **6** | 12 |
| 4 | tcatcaatcaaac | | 6502 | 11 | **6** | 13 |
| 5 | tcttcaaccaacc | Y | 14958 | 11 | 5 | 14 |
| 6 | cctacaatctaca | Y | 14973 | 11 | **6** | 15 |
| 7 | tcttagatcaaaa | | 16553 | 11 | 5 | 16 |
| 8 | tcttcgatcatta | | 17386 | 11 | **6** | 17 |
| 9 | ccaacaatctaca | | 28802 | 11 | 6 | 18 |
| 10 | tcaaagatcacca | | 42096 | 11 | 5 | 19 |
| 11 | tcttcggtcgacg | Y | 256 | 11 | 5 | 20 |
| 12 | tcgacaaacaaaa | | 1277 | 11 | <5 | 21 |
| 13 | tattcaaacaacg | | 4061 | 11 | 5 | 22 |
| 14 | ttttcgataaaaa | | 4380 | 10 | **6** | 23 |
| 15 | tcttcagtctgca | | 5399 | 10 | **5** | 24 |
| 16 | gattcaatcatca | | 7723 | 10 | **6** | 25 |
| 17 | ttatcgagcaaaa | | 8137 | 10 | 5 | 26 |
| 18 | tcataactcaaga | | 9062 | 10 | <5 | 27 |
| 19 | tcagaaatcaaaa | | 9126 | 10 | <5 | 28 |
| 20 | tctttaatttaca | | 10639 | 10 | 5 | 29 |
| 21 | tttacaatcctca | | 10646 | 10 | **6** | 30 |
| 22 | tcatagatcagga | | 11214 | 10 | 5 | 31 |
| 23 | acctcaaacaaca | | 11989 | 10 | <5 | 32 |
| 24 | tcatcgaacaccc | | 12020 | 10 | 5 | 33 |
| 25 | tcaataatcgtca | | 12199 | 10 | 5 | 107 |
| 26 | tcatcaaacgtca | | 13287 | 10 | 5 | 108 |
| 27 | ttatcgttaaaca | Y | 13439 | 10 | 5 | 109 |
| 28 | taaacagtcaata | Y | 13446 | 10 | 5 | 110 |
| 29 | tacacgatcagca | Y | 14096 | 10 | 5 | 111 |

(continued)

| Name | Sequence w = T or A r = A or G | Present in Minigenel 1 | Position | Identity with consensus | Identity with wwcrat | SEQ ID NO. |
|---|---|---|---|---|---|---|
| 30 | aatacaaacaaca | Y | 14637 | 10 | 5 | 112 |
| 31 | tcatcaacaagca | Y | 14914 | 10 | 5 | 113 |
| 32 | tctacaaaccaga | Y | 14980 | 10 | 5 | 114 |
| 33 | acatcgattcaca | | 16085 | 10 | **6** | 115 |
| 34 | cgctcaatcaaca | | 16175 | 10 | 5 | 116 |
| 35 | tctaccataaaaa | | 16511 | 10 | 5 | 117 |
| 36 | aaatgaatcaaca | | 20044 | 10 | 5 | 118 |
| 37 | acatcgttcaacg | | 21374 | 10 | 5 | 119 |
| 38 | tcttgattcacca | | 21580 | 10 | <5 | 120 |
| 39 | tctgcagacaaca | | 22408 | 10 | <5 | 121 |
| 40 | tcttcggtaatca | | 23285 | 10 | 5 | 122 |
| 41 | tctataaacaata | Y | 25436 | 10 | <5 | 123 |
| 42 | taaacaataaata | Y | 25440 | 10 | **6** | 124 |
| 43 | taaacaagcaaaa | | 28242 | 10 | 5 | 125 |
| 44 | tcaacgatcggcg | | 30309 | 10 | **6** | 126 |
| 45 | tgatccatcatca | | 30910 | 10 | 5 | 127 |
| 46 | tcaacatgcaaga | | 32295 | 10 | <5 | 128 |
| 47 | tcttaaataaaga | | 32862 | 10 | 5 | 129 |
| 48 | tcaaagatctata | | 40551 | 10 | 5 | 130 |
| 49 | taatgaattaaca | | 40847 | 10 | 5 | 131 |
| 50 | tttaccatcaact | | 41712 | 10 | 5 | 132 |
| 51 | taatgaaacaaca | | 43380 | 10 | <5 | 133 |
| 52* | gtttcaattaaaa | Y | 13500 | 9 | **6** | 134 |
| 53* | tattcaattataa | Y | 13602 | 9 | **6** | 135 |
| 54* | tcttcaatcgttt | Y | 15002 | 9 | **6** | 136 |
| 55* | tcaacgatccttt | Y | 15533 | 9 | **6** | 137 |

Figure 7 is a diagrammatic representation of putative Tra/Tra2 binding sites within the *dsx* coding region of plasmid LA3491. This diagram is approximately to scale and represents a sequence of approximately 4kb. We can calculate the chance of a random match to the Tra/Tra2 consensus sequence. Assuming all 4 nucleotides occur at equal frequency, the chances of any given nucleotide in a random sequence being the first nucleotide of a 10/13 or better match to the consensus is approx $7x10^{-4}$. Therefore, one would expect slightly less than one such match per 1000 nucleotides of such random sequence. The calculation for this is below:

**Sex-specific splicing: probabilities**

**Questions**

A binding site consensus sequence consists of 13 bases. Ten of those (fixed) positions (call this set X) must each be one specific base. The other three (call this set Y) can each be one of two specific bases. Assuming that each possible base A, G, C and T is equally likely and that the base at each position is independent of the bases at the other positions, what is the probability of a 13-base sequence selected at random exactly matching this sequence? What are the probabilities of such a sequence being a near mismatch (allowing for up to one, two, three or four differences)? The answers are provided in Table 2 below and the workings are shown thereafter.

**Answers**

Table 3

| No. of positions mismatched | Probability (fraction) | Probability (to 3 d.p.) |
|---|---|---|
| none, i.e. exact match | $\frac{1}{2^{23}}$ | $1.192\times10^{-7}$ |
| up to 1, i.e. at least 12 positions match | $\frac{17}{2^{22}}$ | $4.053\times10^{-6}$ |
| up to 2, i.e. at least 11 positions match | $\frac{133}{2^{21}}$ | $6.342\times10^{-5}$ |
| up to 3, i.e. at least 10 positions match | $\frac{23}{2^{15}}$ | $7.019\times10^{-4}$ |
| up to 4, i.e. at least 9 positions match | $\frac{33863}{2^{23}}$ | $4.037\times10^{-3}$ |

**Workings**

P(exact match) = $P_0 = \left(\frac{1}{4}\right)^{10}\left(\frac{1}{2}\right)^{3} = \frac{1}{4^{10}\times2^{3}} = \frac{1}{2^{23}} = 1.192\times10^{-7}$ to 3 d.p. (*3 d.p. all below*)

P(mismatch in exactly 1 position) = P(mismatch at one of the 10 X positions or mismatch at one of the 3 Y positions)

$$= P_1 = 10\left(\frac{1}{4}\right)^{9}\left(\frac{3}{4}\right)\left(\frac{1}{2}\right)^{3} + 3\left(\frac{1}{4}\right)^{10}\left(\frac{1}{2}\right)^{3} = \frac{(10\times3)+3}{4^{10}\times2^{3}} = \frac{33}{2^{23}} = 3.934\times10^{-6}$$

P(mismatch in exactly 2 positions) = P(mismatches at 2 of the 10 X or mismatch at 1 of the 10 X and 1 of the 3 Y or mismatches at 2 of the 3 Y)

$$= P_2 = \frac{10!}{2!8!}\left(\frac{1}{4}\right)^{8}\left(\frac{3}{4}\right)^{2}\left(\frac{1}{2}\right)^{3} + 10\times3\left(\frac{1}{4}\right)^{9}\left(\frac{3}{4}\right)\left(\frac{1}{2}\right)^{3} + 3\left(\frac{1}{4}\right)^{10}\left(\frac{1}{2}\right)^{3}$$

$$= \frac{\left((45\times3^{2})+(30\times3)+3\right)}{2^{23}} = \frac{498}{2^{23}} = \frac{249}{2^{22}} = 5.937\times10^{-5}$$

P(mismatch in exactly 3 positions) = P(mismatches at 3 of the 10 X or mismatches at 2 of the 10 X and 1 of the 3 Y or mismatches at 1 of the 10 X and 2 of the 3 Y or mismatches at 3 of the 3 Y)

$$= P_3 = \frac{10!}{3!7!}\left(\frac{1}{4}\right)^7\left(\frac{3}{4}\right)^3\left(\frac{1}{2}\right)^3 + \frac{10!}{2!8!}3\left(\frac{1}{4}\right)^8\left(\frac{3}{4}\right)^2\left(\frac{1}{2}\right)^3 + 10\times 3\left(\frac{1}{4}\right)^9\left(\frac{3}{4}\right)\left(\frac{1}{2}\right)^3 + \left(\frac{1}{4}\right)^{10}\left(\frac{1}{2}\right)^3$$

$$= \frac{\left((120\times 3^3) + (45\times 3^3) + (30\times 3) + 1\right)}{2^{23}} = \frac{5356}{2^{23}} = \frac{1339}{2^{21}} = 6.385\times 10^{-4}$$

P(mismatch in exactly 4 positions) = P(mismatches at 4 of the 10 X or mismatches at 3 of the 10 X and 1 of the 3 Y or mismatches at 2 of the 10 X and 2 of the 3 Y or mismatches at 1 of the 10 X and 3 of the 3 Y)

$$= P_4 = \frac{10!}{4!6!}\left(\frac{1}{4}\right)^6\left(\frac{3}{4}\right)^4\left(\frac{1}{2}\right)^3 + \frac{10!}{3!7!}3\left(\frac{1}{4}\right)^7\left(\frac{3}{4}\right)^3\left(\frac{1}{2}\right)^3 + \frac{10!}{2!8!}3\left(\frac{1}{4}\right)^8\left(\frac{3}{4}\right)^2\left(\frac{1}{2}\right)^3 + 10\left(\frac{1}{4}\right)^9\left(\frac{3}{4}\right)\left(\frac{1}{2}\right)^3$$

$$= \frac{\left((210\times 3^4) + (120\times 3^4) + (45\times 3^3) + (10\times 3)\right)}{2^{23}} = \frac{27975}{2^{23}} = 3.335\times 10^{-3}$$

P(mismatch in up to 1 position)

$$= P_0 + P_1 = \frac{1+33}{2^{23}} = \frac{17}{2^{22}} = 4.053\times 10^{-6}$$

P(mismatch in up to 2 positions) $= P_0 + P_1 + P_2 = \frac{1+33+498}{2^{23}} = \frac{532}{2^{23}} = \frac{133}{2^{21}} = 6.342\times 10^{-5}$

P(mismatch in up to 3 positions)

$$= P_0 + P_1 + P_2 + P_3 = \frac{1+33+498+5356}{2^{23}} = \frac{5888}{2^{23}}$$

$$= \frac{23}{2^{15}} = 7.019\times 10^{-4}$$

P(mismatch in up to 4 positions)

$$= P_0 + P_1 + P_2 + P_3 + P_4 = \frac{1+33+498+5356+27975}{2^{23}} = \frac{33863}{2^{23}}$$

$$= 4.037\times 10^{-3}$$

**Experiment 14: Cctra**

We have one line of LA3097 (LA3097A) which shows very good expression of its fluorescent marker; it is unknown if this line is a single integration event. This line does show evidence of sex-specific splicing, when reared off tetracycline all the females die as embryos, and when it is on 30μg/ml of tetracycline both males and females survive.

This example is important. It shows that *Cctra* provides sex-specific alternative splicing in *Aedes,* and that this can be used to give sex-specific lethality. This, therefore, provides evidence of the phylogenetic range for *Cctra* splicing. Thus, it is entirely plausible that the present invention can be applied to all Diptera, as we have shown that *Cctra* works in *Drosophila,* tephritids and mosquitoes, which essentially spans the whole Dipteran Order.

It is surprising that Cctra works in *Aedes,* given the rapid sequence evolution of tra.

We transformed *Aedes aegypti* with construct LA3097. Heterozygous males from the resultant transgenic line were crossed to wild type and the progeny reared in aqueous medium supplemented with tetracycline to a final concentration of 30 μg/ml. Adults were recovered as follows: 14 males and one female, thus showing significant female-specific lethality.

This species and strain normally has a sex ratio of approximately 1:1, therefore this construct gave female-specific lethality in *Aedes aegypti.* Equivalent constructs which did not contain the *Cctra* intronic sequence gave non-sex-specific lethality. Therefore, the *Cctra* intron can be used to provide differential (i.e. sex-specific) regulation of gene expression in mosquitoes, and this can further be used to provide sex-specific lethality and a method for the selective elimination of females from a population.

In more detail: on 0 μg/ml tetracycline, males survive only to pupae, i.e. don't make it to adult. Females die so early that we don't see them, probably as embryos, so there is still a differential effect between the sexes. However, the pupal lethality in males suggests that the system is not completely switched off in males. The single insertion line that we recovered is unusual, in that it shows extremely strong expression of the marker; other insertions with more typical expression levels might well not show male lethality.

**Splicing in LA3097A**

Analysis of splicing of LA3097 from LA3097A transgenic mosquitoes by RT-PCR showed that males and females shared two transcripts, an approximately 950 bp band and a fainter band of approximately 800 bp (Figure 59). Sequencing of these bands showed that the ~900 bp band corresponds to a non-sex-specific splice variant (AeM2, ~920 bp), and the fainter band was a mixture of a non-sex-specific splice variant (AeM1, ~804bp) and the female form (AeF1, ~765bp), see Figure 60. The splicing of the AeF1 transcript was identical to that shown for this construct in Medfly (Figure 33). The splicing of the M transcripts differs somewhat from that seen in the native context (Cctra splicing in Medfly, either the native gene or as we observed from LA3097 in transgenic Medfly); in AeM1 the second alternatively spliced exon (ME1b) is not included in the mature AeM1 transcript and in AeM2 the second alternatively spliced exon (ME2b) is similarly not included in the mature AeM2 transcript. In other words, for each of these transcripts the first but not the second cassette exon is present, relative to the Medfly prototype. Note that, as a consequence of the absence of the second cassette exon in AeM1, and the reading frame of tTAV2 relative to the first cassette exon in this construct, splicing in the AeM1 pattern does not lead to interruption of the tTAV2 open reading frame, but rather to the addition of 39 nucleotides (corresponding to 13 amino acids) between the ATG and the rest of the tTAV2 open reading frame. It is likely that this variant of tTAV2 may retain some activity, relative to normal or prototypic tTAV2 (as encoded by the F1 splice variant). In the absence of tetracycline, a phenotypic effect was observed in males as well as in females, though weaker in males than females. Production of a partially active variant of tTAV2 from the AeM1 transcript in males (and females) may explain this.

Figure 59 - shows RT-PCR of males and females from LA3097A *Aedes aegypti* transgenic line using the primers HSP (SEQ ID NO. 139) and VP16 (SEQ ID NO. 140). Using these primers, splicing in the CcF1 pattern (i.e. corresponding to the F1 variant of *Ceratitis capitata*) would give a band of approximately 765bp and splicing in the CcM1 1005bp and CcM2 1094bp. In both males and females, a strong band of approximately 950bp (1) was observed along with a fainter band of approximately 800bp (2). Marker (SmartLadder™ from Eurogentec, bands from 1.5kb to 0.4kb are indicated).

Sequence analysis of several clones from band 2 (i.e. AeM1/AeF1 splice variants) from males and females showed that one of five clones from females showed AeM2 splicing (20%), whereas in males three of the four clones showed AeM2 splicing (75%); all the other clones showed AeF1 splicing. This indicates that there is more AeF1 transcript present in females than in males and this would explain the differential killing effect seen between them.

Figure 60 Illustrates the various transcripts produced by alternative splicing of Cctra from LA3097A *Aedes aegypti* transgenic line. 3097 represents the DNA sequence of Cctra and the numbers relate to figure described elsewhere. Shading and boxes also relate to Figure 33. Note that the diagram is not to scale.

**Example 15: Aedes *Actin-4*;**

We have eleven lines of LA3545, which uses the *Aedes actin-4* gene (*AeAct-4* or *AaAct4*) to drive expression of tTAV2. In construct LA3545, a sequence encoding tTAV2 has been inserted into the second exon of AaAct4 (fig 10).

For transcripts spliced in the pattern characteristic of *AaAct4* splicing in females, the ATG of the tTAV2 coding region will be the first (5'-most) ATG of the transcript. Splicing in the pattern characteristic of *AaAct4* splicing in males introduces an array of start and stop codons before the tTAV2 sequence which tends to inhibit or interfere with translation from the ATG of the tTAV2 coding region. These lines should only express tTAV2 in female pupae. The splicing is shown in Figure 8, below.

Figure 8 shows RT-PCR of male and female adults from LA3545AeC *Aedes aegypti* transgenic line using the primers Agexon1F (SEQ ID NO. 141) and TETRR1 (SEQ ID NO. 142). Using these primers, splicing in a pattern equivalent to that of the native AaAct4 gene would give bands of approx 347bp for the female-type splice variant and of approx 595bp for the male-type splice variant. A band of approx 347bp band (F) was found only in reactions on extracts from females; a band of approx 595bp (M) was found in both males and females. Sequencing has confirmed that the correct splicing occurred in males and females. Marker (SmartLadder™ from Eurogentec, bands from 1.5kb to 0.2kb are indicated).

We also have transgenic *Aedes aegypti* carrying construct LA3604, which is similar to LA3545 except it has an engineered start codon in the portion of exon 1 that is present in both male-type and female-type transcripts (Fig 10). This is arranged to be the first ATG in either transcript type. LA3604 encodes tTAV2 fused to ubiquitin (LA3545 codes tTAV, while LA3604 codes ubi-tTAV2). This construct should produce a fully functional tTAV2 protein in females only, even if the male form is expressed in females the extra male exon contains several start and stop codons that would prevent translation of the Ubi-tTAV2 fusion protein.

The alternative splicing of *AaAct4* occurs in the 5' UTR (of the native gene). It may or may not have a regulatory role in the native gene. One possibility is as follows: in the female-specific splice variant, the start codon of the *AaAct4* coding region is the first ATG of the transcript. However, in the male-specific splice variant there are several additional ATG sequences 5' to the start codon of the *AaAct4* coding region; most of these have in-frame stop codons a short distance 3'. This sequence arrangement may interfere with the efficient translation of the *AaAct4* protein and thereby reduce expression of the protein in males as compared with females. This is the arrangement in LA3545.

However, a greater differential effect between males and females would be expected if the intron was included in coding region (rather than 5' UTR), i.e. inserted between the start and stop codons of the polynucleotide for expression in the organism. In this case, the male-specific cassette exon would change the coding potential of the transcript, rather than simply interfering with translation.

This is achieved in construct LA3604. We modified the shared first exon to include an ATG sequence in a suitable sequence context for translational initiation. In this modified sequence, this is the first ATG in either the male-type (M) or female-type (F) splice variants. Following splicing in the F form, this (engineered) 5' ATG is in frame with the ubi-tTAV coding region. F-type transcripts would therefore encode a fusion protein, comprising sections encoded by (i) part of what is normally Act4 5' UTR (but here obviously translated, and so not UTR at all), (ii) ubiquitin coding region and (iii) tTAV2 coding region.

Activity of cellular ubiquitin proteases will release the tTAV2 protein. Translation from the engineered 5' ATG would be terminated by in-frame stop codons in the additional sequence (cassette exon) present in transcripts spliced in the M form. This would therefore prevent expression of functional tTAV2 in males, thereby giving sex-specific expression of tTAV2. Obviously, this gives a general method for sex-specific expression of a protein, by replacing the tTAV2 segment with another protein or sequence of interest. Using this strategy we have provided transgenics and shown sex-specific splicing (Fig 9).

Figure 9 shows RT-PCR of males and females from LA3604AeA *Aedes aegypti* transgenic line using the primers Agexon1F (SEQ ID NO. 141)and TETRR1 (SEQ ID NO. 142). Using these primers, splicing in the female form would give a band of approximately 575 bp, while inclusion of the male-specific cassette exon would increase this to approximately 823bp. A band of approx 575bp was seen from each female analyzed, while a band of approx 823bp was seen from each male analyzed. These bands appear to be substantially specific to the respective sexes. Sequencing of these bands showed the correct splicing had occurred in males and females. Marker: SmartLadder™ from Eurogentec, bands from 1.5kb to 0.2kb are indicated.

Figure 10, below, is a diagrammatic representation of plasmids LA3545 and LA3604. S1: shared exon 1; M1: additional sequence included in male-specific exon 1; S2: shared exon 2 (5' end only); ubi: sequence encoding ubiquitin; tTAV2: sequence encoding tTAV2.

In several of the LA3545 trangenic lines a sex- and tissue-specific effect was observed: females are flightless. Two of the lines show a 90-100% female flightless phenotype one line shows 70% flightless and another 50%. This phenotype is presumably due to female-specific expression of tTAV2 in the developing flight muscles. The difference in the phenotypes between the lines is due to positional effects on the expression of the *AaAct4* promoter. Due to a genes position in the genome expression can be influenced by a number of factors (heterochromatin or euchromatin regions, enhancer and suppressor elements, proximity to other genes) which can be seen readily in the fluorescent markers used to identify transgenics. All eleven lines of LA3545 were identified because they have different fluorescent profiles, even though they have the same promoters and marker. This variation is due to positional effects. This would then mean that we would expect some lines of LA3545 to express more tTAV2 than other because of positional effects, and those lines that do express more would give a female-specific flightless phenotype.

To test this hypothesis we developed a separate *Aedes aegypti* line with a tetO-DsRed2 reporter gene (LA3576 see Fig 17 and SEQ ID NO. 143), when crossed with the different LA3545 lines this would allow the visualisation of where and when the Actin4-tTAV2 was expressing. Out of 8 LA3545 lines crossed to LA3576 all showed female-specific indirect flight muscle fluorescence in late L4 larvae, pupae and adults. In four of the lines DsRed2 expression appeared to be specific (i.e. exclusive) to the female indirect flight muscles; in the other four additional tissues showed expression of DsRed2. This phenomenon, where expression of a transgene depends in part on the region or point in the genome into which it has inserted, is called position effect, and will be well known and understood by the person skilled in the art.

Using LA3576 proved that the expression of tTAV2 in LA3604 was female-specific, occurs mainly in the indirect flight muscles and is stage-specific. Several different tetO-effector constructs were then constructed to analyse their effects. The tetO-MichelobX transgenics (LA3582, see Fig 15 and SEQ ID NO. 144) when crossed to LA3545 all showed female-specific flightless phenotypes that could be repressed by tetracycline. This proves that Actin4 can be used to drive an effector gene in a stage, tissue and sex-specific manner.

Because some lines of LA3545 had a female-specific flightless phenotype without the presence of an induced effector gene, this showed that tTAV2 could act as an effector molecule. tTAV2 is composed of a tTA, a tetO binding domain and VP16, a herpes simplex virus protein. VP16 activates transcription of immediate early viral genes by using its amino-terminal sequences to attach to one or more host-encoded proteins that recognise DNA sequences in their promoters. In LA3604 a tetO-VP16 effector gene has been added to enhance the effect of tTAV2. In three transgenic lines of LA3604 this has caused a 100% female-specific flightless phenotype when reared without tetracycline, showing that VP16 is an effective effector molecule. Note that LA3604 has a potential start codon (ATG) engineered 5' to the alternatively spliced intron. Therefore, in this construct, the male-specific exon is expected to interrupt the open reading frame encoding tTAV (ubi-tTAV); since the male-specific sequence contains several stop codons, this will tend to reduce or eliminate production of functional tTAV in males. By way of comparison, the male-specific exon is 5' to the start codon of tTAV in LA3545. However, by inserting a number of start codons 5' to the start codon of tTAV (which is the first ATG of the female transcript but not of the male transcript), none of these additional start codons being suitable for efficient production of functional tTAV due to being out of frame or having intervening stop codons, this arrangement will also tend to reduce or eliminate production of functional tTAV in males, consistent with the phenotypic data above.

## Example 16: use of ubiquitin and intron positioning

We have newly made *Cctra*-based constructs with the Cctra intron cassette in a variety of different contexts, i.e. flanked by different sequences. Various lines of transgenic Medfly carrying these have been constructed. This shows that the system is general and robust, i.e. that it will work for a wide range of heterologous sequences of interest.

We also have at least one newly made example of a Cctra-ubi-tTAV fusion giving correct splicing (DsRed-cctra-ubi-tTAV).

Preferred examples of the functional protein place the coding sequence for either ubiquitin or tTA, or their functional mutants and or variants such as tTAV, tTAV2 or tTAV3, 3' to the intron. These are arranged so that these elements are substantially adjacent to the 3' end of the intron, more preferably such that the coding region starts within 20 nucleotides or less of the 3' intron boundary), and most preferably, immediately adjacent the 3' end of the intron, although this is less relevant if the Ubiquitin system is used.

Preferred examples of constructs according to the present invention are listed in Table 4, below. It will be appreciated that LA1188 is not within the scope of the present invention, as it does not encode a functional protein, i.e. it doesn't work properly. This is thought to be because of the unexpected use of a splice donor 4 bp 5' to the junction with Cctra intron sequence, leading to a frameshift that is induced in all splices. It is, therefore, included for the sake of information only.

Table 4

| Construct NO. (Figs #.) | Species tra intron is from | position from ATG (bp) | tra intron is fused to- |
|---|---|---|---|
| LA1188 (80) | Medfly | +132 | tTAV |
| LA3014 (29) | Medfly | +22 | ubiquitin |
| LA3166 (30) | Medfly | +136 | ubiquitin |
| LA3097 (27) | Medfly | +0 | tTAV |
| LA3077 (26) | Medfly | +61 | tTAV |

(continued)

| Construct NO. (Figs #.) | Species tra intron is from | position from ATG (bp) | tra intron is fused to- |
|---|---|---|---|
| LA3233 (28) | Medfly | +0 | tTAV2 |
| LA3376 (31) | Medfly | +0 | tTAV2 |
| LA3376 (31) | B. zonata | +3 | reaper KR |
| LA3376 (31) | B.zonata | +0 | tTAV3 |
| LA3242 (32) | C. rosa | +3 | reaperKR |
| LA1038 (14) | Medfly | +21 | Nipp1 (nipper) |
| LA3054 (61) | Medfly | +811 | DsRed-ubiquitin |
| LA3056 (62) | Medfly | +811 | DsRed-ubiquitin |
| LA3488 (63) | Medfly | +949 | Ubiquitin |
| LA3596 (67) | Medfly | +949 | Ubiquitin |

Table 4 shows constructs which contain a splice control sequence which is derived from a *tra* intron. The introns were derived from *C. capitata* (Medfly), *B. zonata* or *C. rosa* (see column 2). Said intron was inserted within the coding region such that the distance between the putative initiator ATG and the last nucleotide of the exon immediately preceding the tra intron was as should be indicated in column 3. Intron is inserted into or adjacent to coding region for either ubiquitin, tTAV, reaper$^{KR}$, nipper or ubiquitin-DsRed as shown in column 4. These were generated and shown to successfully splice, by RT-PCR or phenotypically in Medfly and, in some cases, also either in *Drosophila melanogaster* (LA3077) or *Anastrepha ludens* (LA3097, LA3233, LA3376). In addition, the distance between the ATG and the end of the exon immediately preceding the tra intron (assuming splicing in F1-like form) can range from 0bp to at least +949bp without adverse consequences to splicing (see Table 4, column 3). Thus, it is reasonable to assume that this distance can be up to at least 900 and preferably up to at least 949 bp.

Further information on these examples is summarized in Table 5. The preferred option is to use no endogenous sequence to achieve correct alternative splicing control of expression (+0bp in table 4). We prefer to insert the tra intron between the flanking dinucleotides TG...GT in the coding region of the protein of interest to be alternatively spliced to ensure correct splicing as this may be important, however we will not restrict ourselves to this if necessary as other flanking nucleotides may function correctly as well. Examples LA1038, LA3054 and LA3056 include some endogenous flanking exonic sequence from the natural Cctra gene. In Table 5, if 6 nucleotides or less (including the ATG start codon) are included of particular fusions to the 3' or 5' of the splice junction, for the summary purposes of this table these will not be considered to be part of the fusion. Table 4 can be correlated with table 3 to find which tra intron (Cctra, Bztra or Crtra) is used in each example. Again, LA1188 is included only for the purposes of information and falls outside the present invention.

Table 5

| Construct NO. (Figs #.) | tra intron is fused to 5' | tra intron is fused to 3' | exonic tra sequence fused to 5' (bp) | exonic tra sequence fused to 3' (bp) |
|---|---|---|---|---|
| LA1188 (80) | Hsp70-tTAV | tTAV | +0bp | +0bp |
| LA3014 (29) | Hsp70-ubiquitin | ubiquitin-reaperKR-sv40 | +0bp | +0bp |
| LA3166 (30) | Hsp70-ubiquitin | ubiquitin-reaperKR-sv40 | +0bp | +0bp |
| LA3097 (27) | Hsp70 | tTAV-K10 | +0bp | +0bp |
| LA3077 (26) | Hsp70-tTAV | tTAV-K10 | +0bp | +0bp |

(continued)

| Construct NO. (Figs #.) | tra intron is fused to 5' | tra intron is fused to 3' | exonic tra sequence fused to 5' (bp) | exonic tra sequence fused to 3' (bp) |
|---|---|---|---|---|
| LA3233 (28) | Hsp70 | tTAV2-K10 | +0bp | +0bp |
| LA3376 (31) | Hsp70 | tTAV2-K10 | +0bp | +0bp |
| LA3376 (31) | Sry-a | tTAV3-sv40 | +0bp | +0bp |
| LA3376 (31) | HB | reaperKR-sv40 | +0bp | +0bp |
| LA3242 (32) | HB | reaperKR-sv40 | +0bp | +0bp |
| LA1038 (14) | Hsp70-tra | Tra-Nipp1 (nipper)-sv40 | +22bp | +20bp |
| LA3054 (61) | Opie2-nls-DsRed-tra | tra-ubiquitin-tTAV-sv40 | +22bp | +20bp |
| LA3056 (62) | Opie2-nls-DsRed-tra | tra-ubiquitin-tTAV-sv40 | +22bp | +242bp |
| LA3488 (63) | Ie1-nls-TurboGreen-nls-ubiquitin | ubiquitin-nls-DsRed-nls-sv40 | +0bp | +0bp |
| LA3596 (67) | Ie1-nls-TurboGreen-nls-ubiquitin | ubiquitin-nls-DsRed-nls-sv40 | +0bp | +0bp |

As mentioned above when an intron is placed 5' to a protein coding region (ORF-X), it is preferred to position or use ubiquitin 3' to the intron, 5' to ORF-X, thus and providing female-specific regulation of ORF-X, whilst introducing physical separation between that sequence and the tra intron, thereby reducing the chance that sequences within ORF-X will interfere with the splicing of the tra intron.

Composite constructs and sequences are also envisaged, for example of the form:

*X-ubi-Y*

with the alternatively spliced intron inserted between coding region X and the region encoding ubiquitin (ubi), or within the ubiquitin coding region, or between the region encoding ubiquitin and coding region Y. Thus X will be expressed irrespective of the splicing of the intron, while Y will only be expressed when the intron is spliced in a suitable form. Further configurations and arrangements of this general type will be apparent to the person skilled in the art. Some examples of this are LA3014, LA3054, LA3056, LA3166, LA3488 and LA3596 which all use ubiquitin fusions in this way demonstrating the ability of this idea to be successfully applied in transgenic Medfly. Alternative examples in transgenic mosquitoes include LA3604 and LA3612, showing the wide phylogenetic applicability of this system in not only different species (mosquitoes and Medfly), but also in different contexts including AaActin4, Aadsx and Cctra.

LA3596 (see Fig 67 and SEQ ID NO. 145) is of similar design to LA3488, intended to generate green fluorescence (by expression of nuclear localised TurboGreen fluorescent protein) in both sexes, but red fluorescence only in females (by expression of nuclear localised DsRed2 fluorescent protein). This is accomplished by the fusion of these two proteins, driven by the Hr5-Ie1 enhancer/promoter cassette, linked together with a short 11 amino acid linker (SG4 linker) and a coding region comprising ubiquitin (with one intended point mutation to stabilize the resulting protein by reducing its propensity to ubiquitin-mediated degradation) and the Cctra intron to limit DsRed2 expression to females. Transgenic Medfly were generated with this construct. Red fluorescence was limited to females in this line as expected, while green fluorescence was observed in all males and females. This could be used for sex separation by fluorescence screening for a particular fluorescent protein, in this case red fluorescence representing expression of DsRed2.

**Example 17: Further *Cctra* exemplification**

Reference is also made to LA3014 and LA3166 and phenotypic data therefrom in other Examples.

We have previously made, and have obtained transgenics with, the Cctra intron in a functional protein other than tTAV, see LA3014 and LA3166. LA3014 contains a ubiquitin-reaper$^{KR}$ fusion downstream of a Cctra intron. Phenotypic data shows that LA3014 transgenic Medfly gave repressible *female-specific* lethality. RT-PCR analysis on RNA extracted from adult males and females raised off tetracycline, using primers and ReaperKR, demonstrate that correct splicing was occurring in females (508bp band) and no such band was found in males (Figure 37). LA3166 is another construct with the Cctra intron placed inside the ubiquitin coding region fused to reaper$^{KR}$, but placed in a different position in ubiquitin. LA3166 also produces a dominant repressible female-specific lethal effect in Medfly.

LA1038 is a new example of the use of the Cctra intron in a different sequence context, here placed in a fragment of Nipp1Dm called 'nipper' that also splices correctly in transgenic Medfly when analysed by RT-PCR (Figure 12). LA670 was required as a source of tTAV to drive expression of the alternatively spliced nipper.

We have also newly made, and have obtained transgenics with, 'intron-only' Cctra-based constructs with the intron in a different gene (many of the above examples, unless otherwise apparent, are in tTAV or one of its variants, i.e. tTAV2 or tTAV3). These constructs work as predicted. This is an important result, thus showing that there are not essential exonic sequences in Cctra that we have simply duplicated (in function, if not necessarily in sequence) by chance, in tTAV. We also have ubi-rpr$^{KR}$ constructs of this type (LA3014 and LA3166), which also validates the ubiquitin fusion method described above. The ubiquitin fusion method is further exemplified by RT-PCR analysis of LA3054, LA3056 and LA3488 (Figures 11, 13, 14), as described in Example 16, above.

**Example 17: Further *Cctra* exemplification**

Reference is also made to LA3014 and LA3166 and phenotypic data therefrom in other Examples.

We have previously made, and have obtained transgenics with, the Cctra intron in a functional protein other than tTAV, see LA3014 and LA3166. LA3014 contains a ubiquitin-reaper$^{KR}$ fusion downstream of a Cctra intron. Phenotypic data shows that LA3014 transgenic Medfly gave repressible *female-specific* lethality. RT-PCR analysis on RNA extracted from adult males and females raised off tetracycline, using primers and ReaperKR, demonstrate that correct splicing was occurring in females (508bp band) and no such band was found in males (Figure 37). LA3166 is another construct with the Cctra intron placed inside the ubiquitin coding region fused to reaper$^{KR}$, but placed in a different position in ubiquitin. LA3166 also produces a dominant repressible female-specific lethal effect in Medfly.

LA1038 is a new example of the use of the Cctra intron in a different sequence context, here placed in a fragment of Nipp1Dm called 'nipper' that also splices correctly in transgenic Medfly when analysed by RT-PCR (Figure 12). LA670 was required as a source of tTAV to drive expression of the alternatively spliced nipper.

We have also newly made, and have obtained transgenics with, 'intron-only' Cctra-based constructs with the intron in a different gene (many of the above examples, unless otherwise apparent, are in tTAV or one of its variants, i.e. tTAV2 or tTAV3). These constructs work as predicted. This is an important result, thus showing that there are not essential exonic sequences in Cctra that we have simply duplicated (in function, if not necessarily in sequence) by chance, in tTAV. We also have ubi-rpr$^{KR}$ constructs of this type (LA3014 and LA3166), which also validates the ubiquitin fusion method described above. The ubiquitin fusion method is further exemplified by RT-PCR analysis of LA3054, LA3056 and LA3488 (Figures 11, 13, 14), and as described in Example 16, above.

**Figure 11: Gel showing sex- specific splicing of intron(s) derived from Cctra (780bp band in females) in *Ceratitis capitata* transformed with LA3488.** Splicing in the F1 form would yield a product of approximately 780bp. A band of this size is clearly visible from females (lane 4), but not from males, nor in the lanes with reactions from which the reverse transcriptase enzyme was omitted ("no RT"). Therefore, the Cctra-derived intron is capable of sex-specific alternative splicing in this novel sequence context. Lane 1: Marker (SmartLadder™ from Eurogentec, bands of approx 0.8, 1.0 and 1.5kb are indicated); Lanes 2 and 3: *Ceratitis capitata* LA3488/+ males (RT and no RT control, respectively); Lanes 4 and 5: *Ceratitis capitata* LA3488/+ females (RT and noRT control, respectively).

**Figure 12: Gel showing sex- specific splicing of intron(s) derived from Cctra in** *Ceratitis* ***capitata*** **transformed with LA1038.** Splicing in the F1 form would yield a product of approximately 230bp. A band of this size is clearly visible from females (lanes 1, 2, 7, 8, 9 and 10), but not from males. Therefore, the Cctra-derived intron is capable of sex-specific alternative splicing in this novel sequence context. Lane 15: Marker (SmartLadder™ from Eurogentec, bands of approx 0.2, 0.4 and 0.6kb are indicated); Lanes 1, 2, 7, 8, 9 and 10: *Ceratitis capitata* LA670; LA1038 females; Lanes 3, 4, 5, 6, 11, 12, 13 and 14: *Ceratitis capitata* LA670; LA1038 males.

**Figure 13: Gel showing sex- specific splicing of intron(s) derived from CcTra in *Ceratitis capitata* transformed with LA3054.** Splicing in the F1 form would yield a product of approximately 340 bp. A band of this size is clearly visible in lane 7, but not from males. Therefore, the Cctra-derived intron is capable of sex-specific alternative splicing in this novel sequence context. Lane 1: Marker (SmartLadder™ from Eurogentec, bands of approx 0.4, 0.6, 0.8 and 1.0kb are indicated); Lanes 2-5: *Ceratitis capitata* LA3054 males; Lane 7: *Ceratitis capitata* LA3054 female.

**Figure 14: Gel showing sex- specific splicing of intron(s) derived from Cetra in *Ceratitis capitata* transformed with LA3056.** Splicing in the F1 form would yield a product of approximately 200 bp. A band of this size is clearly visible from a female (lane 6), but not from males (lanes 2-4). Therefore, the Cctra-derived intron is capable of sex-specific alternative splicing in this novel sequence context. Lane 1: Marker (SmartLadder™ from Eurogentec, bands of approx 0.2, 0.4, 0.6 and 0.8kb are indicated); Lanes 2-5: *Ceratitis capitata* LA3056/+ males; Lanes 6-7: *Ceratitis capitata* LA3056/+ females.

**Figure 15: Gel showing sex- specific splicing of intron(s) derived from BzTra in *Anastrepha ludens* transformed with LA3376.** Splicing in the F1 form would yield a product of approximately 672 bp. A band of this size is clearly visible from females (lane 4), but not from males, nor in the lanes with reactions from which the reverse transcriptase enzyme was omitted ("no RT"), primers used were SRY and AV3F. Therefore, the Bztra-derived intron is capable of sex-specific alternative splicing in this novel sequence context and species. Lane 1: Marker (SmartLadder™ from Eurogentec, bands of approx 0.6, 0.8, and 1.0kb are indicated); Lanes 2 and 3: *Anastrepha ludens* LA3376/+ males (RT and no RT control, respectively); Lanes 4 and 5: *Anastrepha ludens* LA3376/+ females (RT and no RT control, respectively).

**Figure 18** and SE ID NOs 149 and 150 show DSX minigene1, DSX minigene2 sequences and LA3619 plasmid map.

Figs 19-51 are as per Examples 1-9 above. Figs 52-58, 68 and 69 show various plasmid diagrams and sequences. Figs 59-60 are described above and Figs 61-66 show various further plasmid diagrams and sequences. Fig 67 is pLA3596, as discussed elsewhere.

***References:***

Allen ML, Christensen BM. Related 2004 Flight muscle-specific expression of act88F: GFP in transgenic Culex quinquefasciatus Say (Diptera: Culicidae). Parasitol Int. 53(4):307-14.

Bennett D, Szoor B, Gross S, Vereshchagina N, Alphey L. 2003 Ectopic expression of inhibitors of protein phosphatase type 1 (PP1) can be used to analyze roles of PP1 in Drosophila development. Genetics. 164(1): 235-45.

Black, D. (2003). Mechanisms of alternative pre-messenger RNA splicing. Annu Rev Biochem 72, 291-336.

Burset, M., Seledtsov, I., and Solovyev, V. (2001). SpliceDB: database of canonical and non-canonical splice sites in mammalian genomes. Nucleic Acids Research 29, 255-259.

Caceres JF, Kornblihtt AR. 2002 Alternative splicing: multiple control mechanisms and involvement in human disease. Trends Genet. 18(4):186-93.

Cande C, Cecconi F, Dessen P, Kroemer G. 2002 Apoptosis-inducing factor (AIF): key to the conserved caspase-independent pathways of cell death?

J Cell Sci. 115(24):4727-34.

Cartegni, L., Chew, S., and Krainer, A. (2002). Listening to silence and understanding nonsense: exonic mutations that affect splicing. Nature Reviews Genetics 3, 285-298.

Clark, F., and Thanaraj, T. (2002). Categorization and characterization of transcript-confirmed constitutively and alternatively spliced introns and exons from human. Human Molecular Genetics 11, 451-464.

Funaguma, S., Suzuki, M., Tamura, T., and Shimada, T. (2005). The Bmdsx transgene including trimmed introns is sex-specifically spliced in tissues of the silkworm, Bombyx mori. J Insect Sci 5, 17.

George, E.L., Ober, M.B. and Emerson Jr, C.P. (1989). Functional domains of the Drosophila melanogaster muscle myosin heavy-chain gene are encoded by alternatively spliced exons. Mol. Cell Biol. 9:2957-2974.

Graveley BR. 2001 Alternative splicing: increasing diversity in the proteomic world. Trends Genet. 17(2):100-7.

Hammes, A., Guo, J.K., Lutsch, G., Leheste, J.R., Landrock, D., Zeigler, U., Gubler, M.C. and Schedl, A. (2001). Two splice variants of the Wilms' Tumour 1 gene have distinct functions during sex determination and nephron formation. Cell 106:319-329.

Hastings, G.A. and Emerson Jr, C.P (1991). Myosin functional domains encoded by alternative exons are expressed in specific thoracic muscles of Drosophila. J. Cell Biol. 114: 263-276.

Hedley, M.L. and Maniatis (1991). Sex-specific splicing and polyadenylation of dsx pre-mRNA requires a sequence that binds specifically to a tra-2 protein in vivo. Cell 65:579-586.

Heinrich J.C. and Scott M.J. 2000 A repressible female-specific lethal genetic system for making transgenic insect strains suitable for a sterile-release program PNAS 97(15): 8229-8232

Horn C, Wimmer EA. 2003 A transgene-based, embryo-specific lethality system for insect pest management. Nat Biotechnol. 21(1):64-70.

Hoshijima, K.K, Inoue, L., Higuchi, I., Sakamoto, H. and Shimura, Y. (1991). Control of doublesex alternative splicing by transformer and transformer-2 in Drosophila. Science 252:833-836.
Huang, Q., Deveraux, Q.L., Maeda, S., Salvesen, G.S., Stennicke, H.R., Hammock, B.D. and Reed, J.C. (2002). Evolutionary conservation of apoptosis mechanisms:
Lepidopteran and baculoviral inhibitor of apoptosis proteins are inhibitor of mammalian caspase-9.Agricultural Sciences 97(4):1427-1432.
Ito, Y., Hirochicka, H. and Kurata, N. (2002). Organ-specific alternative transcripts of KNOX family class 2 homeobox genes of rice. Gene 288:41-47.
Johnson JM, Castle J, Garrett-Engele P, Kan Z, Loerch PM, Armour CD, Santos R, Schadt EE, Stoughton R, Shoemaker DD. 2003 Genome-wide survey of human alternative pre-mRNA splicing with exon junction microarrays. Science. 302(5653):2141-4.
Jurica MS, Moore MJ. 2003 Pre-mRNA splicing: awash in a sea of proteins. Mol Cell. 12(1):5-14.
Kazzaz JA, Rozek CE. 1989 Tissue-specific expression of the alternately processed Drosophila myosin heavy-chain messenger RNAs. Dev Biol. 133(2):550-61.
Maniatis, T., and Tasic, B. (2002). Alternative pre-mRNA splicing and proteome expansion in metazoans. Nature 418, 236-243.
Muñoz, D., Jimenez, A., Marinotti, O., and James, A. (2004). The AeAct-4 gene is expressed in the developing flight muscles of females Aedes aegypti. Insect Molecular Biology 13, 563-568.
Nishiyama, R., Mizuno, H., Okada, S., Yamaguchi, T., Takenaka, M., Fukuzawa, H. and Ohyama, K. (1999). Two mRNA species encoding calcium-dependent protein kinases are differentially expressed in sexual organs of Marchantia polymorpha through alternative splicing. Plant Cell Physiol.40(2):205-212.
Nishiyama, R.,Yamato, K.T., Miura, K., Sakida, M., Okada, S., Kono, K., Takahama, M., Sone, T., Takenaka, M., Fukuzawa, H. and Ohyama, K. (2000). Comparison of expressed sequence tags from male and female sexual organs of Marchantia polymorpha. DNA Res. 7:165-174.
Olson, M.R., Holley, C.L., Ji Yoo, S., Huh, J.R, Hay, B.A. and Kornbluth, S. (2003). Reaper is regulated by IAP-mediated Ubiquitination. J.Biol.Chem., 278(6):4028-4034.
Olson, M.R., Holley, C.L., Gan, E.C., Colon-Ramos, D.A., Kaplan, B. and Kornbluth, S. (2003). A GH3-like domain in reaper is required for mitochondrial localization and induction of IAP degradation. J. Biol. Chem. 278(45):44758-44768.
Pan, Q., Shai, O., Misquitta, C., Zhang, W., Saltzman, A., Mohammad, N., Babak, T., Siu, H., Hughes, T., Morris, Q., et al. (2004). Revealing global regulatory features of mammalian alternative splicing using a quantitative microarray platform. Mol Cell 16, 929-941.
Pane, A., Salvemini, M., Delli Bovi, P., Polito, C., and Saccone, G. (2002). The transformer gene in Ceratitis capitata provides a genetic basis for selecting and remembering the sexual fate. Development 129, 3715-3725.
Park, J., Parisky, K., Celotto, A., Reenan, R., and Graveley, B. (2004). Identification of alternative splicing regulators by RNA interference in Drosophila. Proc Nat'l Acad Sci (USA) 101, 15974-15979.
Parker L, Gross S, Beullens M, Bollen M, Bennett D, Alphey L. 2002 Functional interaction between nuclear inhibitor of protein phosphatase type 1 (NIPP1) and protein phosphatase type 1 (PP1) in Drosophila: consequences of over-expression of NIPP1 in flies and suppression by co-expression of PP1. Biochem J. 368(3):789-97.
Raphael, K.A., Whyard, S., Shearman, D., An, X. and Frommer, M. (2004). Bactrocera tyroni and closely related pest-tephritids-molecular analysis and prospects for transgenic control strategies. Insect Biochem. Mol. Biol. 34:167-176.
Ryner, L. and Baker, B.S. (1991). Regulation of doublesex pre-mRNA processing occurs by 3'-splice site activation. Genes Dev. 5:2071-2085.
Saccone, G., Pane, A., and Polito, C. (2002). Sex determination in flies, fruitfles and butterflies. Genetica 116, 15-23.
Scali, C., Catteruccia, F., Li, Q., and Crisanti, A. (2005). Identification of sex-specific transcripts of the Anopheles gambiae doublesex gene. J Exp Biol 208, 3701-3709.
Scott, M., Heinrich, J., and Li, X. (2004). Progress towards the development of a transgenic strain of the Australian sheep blowfly (Lucilia cuprina) suitable for a male-only sterile release program. Insect Biochem Mol Biol 34, 185-192.
Seo, S-J., Cheon, H-M., Sun, J., Sappington, T.W. and Raikhel, A.S. (2003). Tissue- and stage-specific expression of two lipophorin receptor variants with seven and eight ligand-binding repeats in the adult mosquito. J. Biol. Chem. 278(43):41954-41962.
Siebel CW, Fresco LD, Rio DC. 1992 The mechanism of somatic inhibition of Drosophila P-element pre-mRNA splicing: multiprotein complexes at an exon pseudo-5' splice site control U1 snRNP binding. Genes Dev. 6(8):1386-401.
Shivikrupa, Singh., R and Swarup, G. (1999). Identification of a novel splice variant of C3G which shows tissue-specific expression. DNA Cell Biol. 18: 701-708.
Smith, C., and Valcarcel, J. (2000). Alternative pre-mRNA splicing: the logic of combinatorial control. Trends Biochem Sci 25, 381-388.

Stoss, O., Stoilov, P., Hartmann, A. M., Nayler, O., and Stamm, S. (1999). The in vivo minigene approach to analyze tissue-specific splicing. Brain Research Protocols 4, 383-394.
Stoss, O., Olbrich, M, Hartmann, A. M., Konig, H., Memmott, J., Andreadis, A and Stamm, S. (2001). The STAR/GSG family protein rSLM-2 regulates the selection of alternative splice sites. J. Biol. Chem. 276(12):8665-8673.
Streuli, M. and Saito, H. (1989). Regulation of tissue-specific alternative splicing: exon-specific cis-elements govern the splicing of leukocyte common antigen pre-mRNA. EMBO J. 8(3): 787-796.
Suzuki, M., Ohbayashi, F., Mita, K., and Shimada, T. (2001). The mechanism of sex-specific splicing at the doublesex gene is different between Drosophila melanogaster and Bombyx mori. Insect Biochem Mol Biol 31, 1201-1211.
Thanaraj, T., and Clark, F. (2001). Human GC-AG alternative intron isoforms with weak donor sites show enhanced consensus at acceptor exon positions. Nucleic Acids Research 29, 2581-2593.
Thanaraj, T., Stamm, S., Clark, F., Reithoven, J., Le Texier, V., and Muilu, J. (2004). ASD: the Alternative Splicing Database. Nucleic Acids Research 32, D64-D69.
Varshavsky, A. (2000). Ubiquitin fusion technique and its descendants. Meth Enz 327.
Venables, J. (2002). Alternative splicing in the testes. Curr Opin Genet Dev 12, 615-619.
Venables JP. 2004 Aberrant and alternative splicing in cancer. Cancer Res. 64(21):7647-54.
Vernooy, S.Y., Copeland, J., Ghaboosi, N., Griffin, E.E., Yoo, S.J. and Hay, B.A. (2000). J. Cell Biol. 150(2):F69-F75.
White, K., Tahoaglu, E. and Steller, H. (1996). Cell killing by the Drosophila gene reaper. Science 271 (5250): 805-807.
Wing, J.P., Zhou, L., Schwartz, L.M. and Nambu, J.R. (2001) Distinct cell killing properties of the Drosophila reaper, head involution defective, and grim genes. Cell Death Diffn 5(11): 930-939
Yali Chiu A., and Pin Ouyang, A.B.,(2006).Loss of Pnn expression attenuates expression levels of SR family splicing factors and modulates alternative pre-mRNA splicing in vivo. Bioch. Biophys.Res. Comm.341:663-671.
Yoshimura, K., Yabuta, Y., Ishikawa, T. and Shigeoka, S. (2002). Idenitification of a cis element for tissue-specific alternative splicing of chloroplast Ascorbate Peroxidase pre-mRNA in higher plants. J. Biol.Chem 277 (43):40623-40632.

## SEQUENCE ANNOTATIONS

The following relates to the various plasmids of the present and highlights the position of certain preferred elements therein.

<223> Sequence of pLA3359 (SED ID NO. 47).

<***> Key features include:

1. Anopheles gambiae dsx (Agdsx) mini-gene, [a mini-gene is a recombinant sequence derived from a particular gene (the Agdsx gene in this example) by ligating together non-contiguous segments while retaining original 5'-3' order; this is equivalent to deletion of some internal segments from a longer fragment of genomic sequence derived from the gene], (1-3135): including Agdsx part of exon3, exon 4a (female), exon 4b (female) and part of exon5 (male and female).

<***> Exons derived from Agdsx from positions 426 to 560 (part of exon 3); 1068 to 2755 (including part of exon 4, found in females); 1809 to 2755 (including part of exon 4, found in females); and 2914 to 3135 (including part of exon 5, found in males).
<***> Alternatively spliced transcript starts in segment derived from baculovirus AcMNPV Ie1 (immediate early 1) at position ~8031 (Ie1 fragment is from position 7431 to 8060).
<***> Included feature:

1. additional intron derived from Drosophila scraps gene ('scraps intron') upstream to Agdsx sequence from position 8075 to 8137.

<223> Sequence of pLA3433 (SED ID NO. 48).

<***> Key features include:

1. Agdsx mini-gene (778-4623): including Agdsx part of exon 2, exon3, exon 4a (female), exon 4b (female) and part of exon5 (male and female).

<***> Exons derived from Agdsx from position 778 to 908 (part of exon 2); 1913 to 2048 (part of exon 3); 2556 to 2642 (part of exon 4a); 3297 to 4243 (part of exon 4b) and 4402 to 4623 (part of exon 5).
<***> Alternatively spliced transcript starts in segment derived from baculovirus AcMNPV Ie1 (immediate early 1) at position ~606 (Ie1 fragment is from position 6 to 635).
<***> Included feature:

1. additional intron derived from Drosophila scraps gene ('scraps intron') upstream to Agdsx sequence from position 650 to 712.

<223> Sequence of pLA3491.

<***> Key features include:

1. Aedes aegypti dsx (Aadsx) mini-gene: including part of Aadsx exon 4, exon5a (female), exon 5b (female), and part of exon6 (male and female).

<***> Exons derived from Aadsx from position 1316 to 1450 (part of exon 4); 2626 to 3761 (part of exon 5a); 3293 to 3761 (part of exon 5b); and 5215 to 5704 (part of exon 6).
<***> Part of the F1 transcript is predicted to comprise nucleotides ~1174-1450, 2626-3761, 5215-~5850.
<***> Part of the F2 transcript is predicted to comprise nucleotides ~1174-1450, 3293-3761, 5215-~5850.
<***> Part of the F3 transcript is predicted to comprise nucleotides ~1174-1450, 2626-3083, 3293-3761, 5215-~5850.
<***> Part of the M1 transcript is predicted to comprise nucleotides ~1174-1450, 5215-~5850. <***> Alternatively spliced transcript starts in segment derived from baculovirus AcMNPV Ie1 (immediate early 1) at position ~1174 (Ie1 fragment is from position 574 to 1203).
<***> Included feature:

1. additional intron derived from Drosophila scraps gene ('scraps intron') upstream to Aadsx sequence from position 1218 to 1280.

<223> Sequence of pLA3646.

<***> Key features include:

1. Aadsx mini-gene (17218-11707): including part of Aadsx exon 4 from position 17113 to 16979, exon 5a from position 15803 to 15025 + 14010 to 13650, exon 5b from position 15136 to 15025 + 14010 to 13650 and exon 6 from position 12196 to 11707 (note: reverse orientation). <***> part of exon 4 contains 4 point mutations relative to wild type at positions 17087 (ATG-ACG), 17053 (ATG-ACG), 17050 (ATG-ACG) and 17041 (ATG-ACG) (note: reverse orientation); part of exon 5a and 5b contain 3 point mutations relative to wild type at positions 15129 (ATG-ATA), 15116 (ATG-ATA) and 15113 (ATG-ATA) (note: reverse orientation). All of these mutations are to eliminate ATG sequences.

<***> tTAV2 is inserted in the overlapping exons 5a and 5b from position 15024 to 14011 (note: reverse orientation).
<***> Alternatively spliced transcript starts in hsp70 derived fragment at position ~17312 (hsp70 fragment is from position 17354 to 17225); (note: reverse orientation).
<***> Included feature:

1. additional intron derived from Drosophila scraps gene ('scraps intron') upstream to Aadsx sequence from position 1107 to 1045 (note: reverse orientation)

Sequence of pLA3435 (SED ID NO. 46).

<223> Key features include:

1. Bombyx mori dsx (Bmdsx) minigene (1411-3161) with an exogenous linker between fused female exons 3 and 4.

<***> Fragment of shared exon two (1411bp-1554bp)
<***> Part of female specific exon three (2121bp-2202) fused to part of female specific exon 4 (2225bp-2290bp) using an exogenous linker (2203bp-2224bp)
<***> Fragment of shared exon five (3007bp-3161bp)
<***> A female dsx mini-gene splicing product is encoded by 1411-1554 + 2121-2290 +3007-3161.
<***> A male dsx mini-gene splicing product is encoded by 1411-1554 +3007-3161.
<***> Transcription is predicted to start at approximately position ~1239 within the segment derived from baculovirus AcMNPV Ie1 (immediate early 1) promoter (639bp-1268bp).

<223> Sequence of pLA3534.

<***> Key features include:

1. Aadsx mini-gene (6996-4425): containing Aadsx exon 4, part of exon5a (female) and part of exon 5b (female), inclusive of Aadsx intron fragments.

<***> Exons derived from Aadsx from position 6968 to 6834 (part of exon 4), 5462 to 4425 (part of exon 5a) and 4795 to 4425 (part of exon 5b); (note reverse orientation).
<***> Part of the F1 transcript is predicted to comprise nucleotides ~7146-6834, 5462-~4300 (note: reverse orientation).
<***> Part of the F2 transcript is predicted to comprise nucleotides ~7146-6834, 4795-~4300 (note: reverse orientation).
<***> Part of the F3 transcript is predicted to comprise nucleotides ~7146-6834, 5462-5005, 4795-~4300 (note: reverse orientation).
<***> Alternatively spliced transcript starts in segment derived from baculovirus AcMNPV Ie1 (immediate early 1) at position ~7146 (Ie1 fragment is from position 7746 to 7117, reverse orientation).

<223> Sequence of pLA3612.

<***> Key features include:

1. Ubiquitin-tTAV2 coding region inserted into a female exon of Aadsx gene.

<***> Ubiquitin-tTAV2 is from position 15185-16429 in Aadsx (ubiquitin is from 15185-15412; tTAV2 is from 15413-16429), inclusive of start and stop codon.
<***> Sequence derived from Aadsx: 13150-15184, 16438-18805.
<***> Aadsx-ubiquitin-tTAV2 alternatively spliced transcript starts in hsp70 derived segment (hsp70 fragment is from 13014-13143).

<223> Sequence of pLA3619.

<***> Key features include:

1. tTAV2 coding region inserted into a female exon of Aadsx gene.

<***> Sequence derived from Aadsx: 5635-3641, 2610-243 (note: reverse orientation).
<***> Aadsx-tTAV2 alternatively spliced transcript starts in hsp70 derived segment from 5642-5771 (note: reverse orientation).
<***> tTAV2 transcript is predicted to be translated between 2619-3635, inclusive of start and stop codon (note: reverse orientation).

<223> Sequence of pLA3545.

<***> Key features include:

1. AaActin4 promoter and 5' UTR including first intron regulates tTAV expression.

<***> Sequence derived from AaActin4 is from position 923-4285.
<***> Alternatively spliced transcript is predicted to start from approximately ~2366.
<***> The first intron from AaActin4 (female splice variant) is from 2458-4259.
<***> tTAV is predicted to be translated between 4300-5316, inclusive of start and stop codon.

<223> Sequence of pLA3604.

<***> Key features include:

1. AaActin4 promoter and 5' UTR regulates ubiquitin-tTAV2 expression.

<***> Sequence derived from AaActin4 is from position 5795-2407 (note: reverse orientation).
<***> Alternatively spliced transcript is predicted to start from approximately ~4353 (note: reverse orientation).
<***> The first intron from AaActin4 (female splice variant) is from 2455-4254 (note: reverse orientation).
<***> Ubquitin-tTAV2 transcript is predicted to be translated from a start codon engineered in the first exon of AaAct4 gene at 4299-4297 (ubiquitin is from 2406-2179; tTAV2 is from 2178-1162); (note: reverse orientation).

<223> Sequence of pLA3641.

<***> Key features include:

1. tTAV coding region inserted into a female exon of CodlingDsx gene.

<***> tTAV is from position 2731-3747 in CodlingDsx gene.
<***> Dsx-tTAV alternatively spliced transcript starts in hsp70 derived segment (hsp70 fragment is from 4811-4940).
<***> tTAV transcript is predicted to be translated between 2731-3747, inclusive of start and stop codon (note: reverse orientation).

<223> Sequence of pLA3570

<***> Key features include:

1. tTAV coding region inserted into a female exon of PBW-Dsx gene.

<***> tTAV coding region is from 2336-3352.
<***> Dsx-tTAV alternatively spliced transcript starts in hsp70 derived segment (hsp70 fragment is from 4683-4812).

<***> tTAV transcript is predicted to be translated between 2336-3352, inclusive of start and stop codon (note: reverse orientation).

<223> Sequence of pLA1188 (SED ID NO.49)

<***> Key features include:

1. tTAV coding region with inserted Cctra intron.

<***> Cctra intron is from position 3905-2561 in tTAV (note: reverse orientation).
<***> tTAV alternatively spliced transcript starts in hsp70 derived segment at position 4217 (hsp70 fragment is from 4260-4131); (note: reverse orientation).
<***> tTAV F1 transcript is predicted to be translated between 4040-1679 (note: reverse orientation).
<***> Included feature:

1. Adh intron within predicted F1 transcript from position 4118-4049 (note: reverse orientation).

<223> Sequence of pLA3077 (SED ID NO. 50).

<***> Key features include:

1. tTAV coding region with inserted Cctra intron.

<***> Cctra intron is from position 3975-2631 in tTAV (note: reverse orientation).
<***> tTAV alternatively spliced transcript starts in hsp70 derived segment at position ~4217 (hsp70 fragment is from 4260-4131); (note: reverse orientation).
<***> tTAV F1 transcript is predicted to be translated between 4039-1678, inclusive of start and stop codon (note: reverse orientation).
<***> Included feature:

1. Adh intron within predicted F1 transcript from position 4117-4048 (note: reverse orientation).

<223> Sequence of pLA3097 (SED ID NO. 51).

<***> Key features include:

1. tTAV coding region with inserted Cctra intron.

<***> Cctra intron is from position 3282-1938 in tTAV (note: reverse orientation).
<***> tTAV alternatively spliced transcript starts in hsp70 derived segment at position ~3382 (hsp70 fragment is from 3425-3296); (note: reverse orientation).
<***> tTAV F1 transcript is predicted to be translated between 3285-924, inclusive of start and stop codon (note: reverse orientation).

<223> Sequence of pLA3233 (SED ID NO. 52).

<***> Key features include:

1. tTAV2 coding region with inserted Cctra intron.

<***> Cctra intron is from position 3289-1945 in tTAV2 (note: reverse orientation).
<***> tTAV2 alternatively spliced transcript starts in hsp70 derived segment at position ~3389 (hsp70 fragment is from 3432-3303); (note: reverse orientation).
<***> tTAV2 F1 transcript is predicted to be translated between 3292-931, inclusive of start and stop codon (note: reverse orientation).

<223> Sequence of pLA3014 (SED ID NO. 53).

<***> Key features include:

1. ubi-reaper[KR] coding region with inserted Cctra intron.

<***> Cctra intron is from position 3356-4700 in ubi-reaper[KR].
<***> ubi-reaper[KR] alternatively spliced transcript starts in hsp70 derived segment at position ~3234 (hsp70 fragment is from 3191-3320).
<***> ubi-reaper[KR] F1 transcript is predicted to be translated between 3331-5143, inclusive of start and stop codon (ubiquitin is from 3331-3355, 4701-4948; reaper[KR] is from 4949-5143).

<223> Sequence of pLA3166 (SED ID NO. 54).

<***> Key features include:

1. ubi-reaper[KR] coding region with inserted Cctra intron.

<***> Cctra intron is from position 9987-8643 in ubi-reaper[KR] (note: reverse orientation).
<***> ubi-reaper[KR] alternatively spliced transcript starts in hsp70 derived segment at position ~10227 (hsp70 fragment is from 10270-10141); (note: reverse orientation).
<***> ubi-reaper[KR] F1 transcript is predicted to be translated between 10126-8359, inclusive of start and stop codon (ubiquitin is from 10126-9988, 8642-8554; reaper[KR] is from 8553-8359); (note: reverse orientation).
<223> Sequence of pLA3376 (SED ID NO. 55).
<***> Key features include:

1. tTAV2 coding region with inserted Cctra intron.
2. tTAV3 coding region with inserted Bztra intron.
3. reaper[KR] coding region with inserted Bztra intron.

<***> Cctra intron is from position 3289-1945 in tTAV2 (note: reverse orientation).
<***> Bztra intron is from position 5981-5014 in tTAV3 (note: reverse orientation).
<***> Bztra intron is from position 16391-17358 in reaper[KR].
<***> tTAV2 alternatively spliced transcript starts in hsp70 derived segment at position ~3389 (hsp70 fragment is from 3432-3303); (note: reverse orientation).
<***> tTAV3 alternatively spliced transcript starts in sry-alpha derived segment at position ~6019 (sry-alpha fragment is from 6243-5999); (note: reverse orientation).
<***> reaper[KR] alternatively spliced transcript starts in hunchback derived segment at position ~16339 (hunchback fragment is from 16289-16372).
<***> tTAV2 F1 transcript is predicted to be translated between 3292-931, inclusive of start and stop codon (note: reverse orientation).
<***> tTAV3 F1 transcript is predicted to be translated between 5984-4006, inclusive of start and stop codon (note: reverse orientation).
<***> reaper[KR] F1 transcript is predicted to be translated between 16385-17550, inclusive of start and stop codon.

<223> Sequence of pLA3242 (SED ID NO. 56).

<***> Key features include:

1) tTAV coding region with inserted Cctra intron.
2) reaper[KR] coding region with inserted Crtra intron.

<***> Cctra intron is from position 3282-1938 in tTAV (note: reverse orientation).
<***> Crtra intron is from position 5488-4180 in reaperKR (note: reverse orientation).
<***> reaperKR alternatively spliced transcript starts in hunchback derived segment at position ~5540 (hunchback fragment is from 5590-5507); (note: reverse orientation).
<***> tTAV alternatively spliced transcript starts in hsp70 derived segment at position ~3382 (hsp70 fragment is from 3425-3296); (note: reverse orientation).
<***> reaperKR F1 transcript is predicted to be mainly translated between 4088-5494, inclusive of start and stop codon (note: reverse orientation).
<***> tTAV F1 transcript is predicted to be mainly translated between 924-3285, inclusive of start and stop codon (note: reverse orientation).

<223> Sequence of pLA1172 (SED ID NO. 106).

<***> Key features include:

1. tTAV coding region between AaActin4 derived fragments.

<***> AaActin4 derived fragments are from 7868-11257 and 12366-13100.
<***> tTAV transcript is predicted to be translated between 11342-12358, inclusive of start and stop codon.
<***> AaActin4-tTAV transcript is predicted to start at position ~9312.
<***> AaActin4 contains an intron (female-type splice variant) from position 9403-11204.

<223> Sequence of pLA1038 (Fig 12).

<***> Key features include:

1. Fragment of Nipp1Dm ('nipper') coding region with inserted Cctra intron with flanking tra exonic sequence.

<***> Cctra intron is from position 3365-4709 in nipper.
<***> Cctra intron is flanked by Cctra exonic sequence at positions 3343-3364 and 4710-4729.
<***> nipper alternatively spliced transcript starts in hsp70 derived segment at position ~3243 (hsp70 fragment is from 3200-3329).
<***> nipper F1 transcript is predicted to be translated between 3340-5014, inclusive of start and stop codon.

<223> Sequence of pLA3054 (SED ID NO. 158).

<***> Key features include:

1. DsRed-ubi-tTAV coding region with inserted Cctra intron with flanking tra exonic sequence.

<***> Cctra intron is from position 3509-2165 in DsRed-ubi-tTAV (note: reverse orientation).
<***> Cctra intron is flanked by Cctra exonic sequence at positions 3531-3510 and 2164-2145 (note: reverse orientation).
<***> DsRed-ubi-tTAV alternatively spliced transcript starts either in hsp70 derived segment at position ~3243 (hsp70 fragment is from 4930-4801) or Opie2 derived segment at position ~4353 (Opie2 fragment is from 4795-4255); (note: reverse orientation).
<***> DsRed-ubi-tTAV F1 transcript is predicted to be translated between 4320-888, inclusive of start and stop codon (DsRed is from 4212-3538; ubiquitin is from 2135-1908; tTAV is from 1907-888); (note: reverse orientation).

<223> Sequence of pLA3056 (SED ID NO. 159).

<***> Key features include:

1. DsRed-ubi-tTAV coding region with inserted Cctra intron with flanking tra exonic sequence.

<***> Cctra intron is from position 3731-2387 in DsRed-ubi-tTAV (note: reverse orientation).
<***> Cctra intron is flanked by Cctra exonic sequence at positions 3753-3732 and 2386-2145 (note: reverse orientation).
<***> DsRed-ubi-tTAV alternatively spliced transcript starts either in hsp70 derived segment at position ~5109 (hsp70 fragment is from 5152-5023) or Opie2 derived segment at position ~4575 (Opie2 fragment is from 5017-4477); (note: reverse orientation).
<***> DsRed-ubi-tTAV F1 transcript is predicted to be translated between 4542-888, inclusive of start and stop codon (DsRed is from 4434-3760; ubiquitin is from 2135-1908; tTAV is from 1907-888); (note: reverse orientation).
<***> Included feature:

1. additional intron derived from Cctra gene (second intron of Cctra F1 transcript) within predicted F1 transcript from position 2222-2168 (note: reverse orientation).

<223> Sequence of pLA3488 (SED ID NO. 160).

<***> Key features include:

1. TurboGreen-ubi-DsRed coding region with inserted Cctra intron.

<***> Cctra intron is from position 2263-3607 in TurboGreen-ubi-DsRed.
<***> TurboGreen-ubi-DsRed alternatively spliced transcript starts in segment derived from baculovirus AcMNPV Ie1 (immediate early 1) at position ~1180 (Ie1 fragment is from 580-1209).
<***> TurboGreen-ubi-DsRed F1 transcript is predicted to be translated between 1311-4467, inclusive of start and stop codon (TurboGreen is from 1311-2093; SG4 linker is from 2094-2123; ubiquitin is from 2124-3696, inclusive of Cctra intron; DsRed is from 3697-4467).
<***> Included feature:

1. additional intron derived from Drosophila scraps gene ('scraps intron') within predicted F1 transcript from position 1224-1286.

<223> Sequence of pLA3596 (SED ID NO. 145).

<***> Key features include:

1. TurboGreen-ubi-DsRed2 coding region with inserted Cctra intron.

<***> Cctra intron is from position 5947-7291 in TurboGreen-ubi-DsRed2.
<***> TurboGreen-ubi-DsRed2 alternatively spliced transcript starts in segment derived from baculovirus AcMNPV Ie1 (immediate early 1) at position ~4864 (Ie1 fragment is from 4264-4893).
<***> TurboGreen-ubi-DsRed2 F1 transcript is predicted to be translated between 4995-8148, inclusive of start and stop codon (TurboGreen is from 4995-5777; SG4 linker is from 5778-5807; ubiquitin is from 5808-7380, inclusive of Cctra intron; DsRed2 is from 7381-8151).
<***> Included feature:

1. additional intron derived from Drosophila scraps gene ('scraps intron') within predicted F1 transcript from position 4908-4970.
2. intended amino acid mutation compared to LA3488 at position 7294-7296.

Exemplary aspects of the invention are described in the following clauses:

1. A polynucleotide expression system comprising:

at least one heterologous polynucleotide sequence encoding a functional protein, defined between a start codon and a stop codon, and/or polynucleotides for interference RNA (RNAi), to be expressed in an organism;
at least one promoter operably linked thereto; and
at least one splice control sequence which, in cooperation with a spliceosome, is capable of (i) mediating splicing of an RNA transcript of the coding sequence to yield a first spliced messenger RNA (mRNA) product, and (ii) mediating at least one alternative splicing of said RNA transcript to yield an alternative spliced mRNA product;

wherein, when the at least one heterologous polynucleotide sequence encodes a functional protein, at least one of the mature mRNA products comprising a continuous Open Reading Frame (ORF) extending from said start codon to said stop codon, thereby defining a protein, which is said functional protein, or is related to said functional protein by at least one amino acid deletion, and which is functional when translated and, optionally, has undergone post-translational modification;
the mediation being selected from the group consisting of: sex-specific mediation, stage-specific mediation, germline-specific mediation, tissue-specific mediation, and combinations thereof.

2. A polynucleotide expression system according to clause 1, wherein mediation is the sex-specific.

3. A polynucleotide expression system according to clause 1 or 2, wherein the polynucleotide sequence to be expressed comprises two or more coding exons for the functional protein.

4. A polynucleotide expression system according to any preceding clause, wherein the protein is a marker, or has a lethal, deleterious or sterilizing effect.

5. A polynucleotide expression system according to clause 4, wherein the protein has a lethal effect resulting in sterilization.

6. A polynucleotide expression system according to clause 5, wherein the lethal effect of the protein is conditionally suppressible.

7. A polynucleotide expression system according to clause 4, wherein the protein is selected from the group consisting of an apoptosis-inducing factor, Hid, Reaper (Rpr), and Nipp1Dm.

8. A polynucleotide expression system according to any preceding clause, wherein the system comprises at least one positive feedback mechanism, being at least a functional protein to be differentially expressed, via alternative splicing, and at least one promoter therefor, wherein a product of a gene to be expressed serves as a positive transcriptional control factor for the at least one promoter, and whereby the product, or the expression of the product, is controllable.

9. A polynucleotide expression system according to clause 8, wherein an enhancer is associated with the promoter, the gene product serving to enhance activity of the promoter *via* the enhancer.

10. A polynucleotide expression system according to clause 9, wherein the control factor is the tTA gene product or an analogue thereof, and wherein one or more tetO operator units is operably linked with the promoter and is the enhancer, tTA or its analogue serving to enhance activity of the promoter *via* tetO.

11. A polynucleotide expression system according to any preceding clause, wherein the functional protein itself a transcriptional transactivator, such as the tTAV system, comprising tTAV, tTAV2 or tTAV3.

12. A polynucleotide expression system according to any preceding clause, wherein the promoter is activated by environmental conditions, for instance the presence or absence of a particular factor such as tetracycline in the *tet* system or by variation of the environmental temperature.

13. A polynucleotide expression system according to any of clauses 1-11, wherein the promoter is selected from the group consisting of the *sryα* embryo-specific promoter, or its homologues, the *Drosophila* gene *slow as molasses (slam),* or its homologues.

14. A polynucleotide expression system according to any preceding clause, further comprising an enhancer.

15. A polynucleotide expression system according to any preceding clause, wherein the mediation of alternative splicing is sex-specific and the splice control sequence is derived from a *tra* intron.

16. A polynucleotide expression system according to clause 15, wherein the the splice control sequence is derived from the Medfly *transformer* gene *Cctra,* or from another ortholog or homolog of the *Drosophila transformer* gene.

17. A polynucleotide expression system according to clause 16, wherein, wherein said another ortholog or homolog of the *Drosophila transformer* gene is from a tephritid fruit fly.

18. A polynucleotide expression system according to clause 17, wherein, wherein the tephritid fruit fly is C. *rosa,* or *B. zonata.*

19. A polynucleotide expression system according to any of clauses 1-14, wherein the splice control sequence is derived from the alternative splicing mechanism of the *Actin-4* gene.

20. A polynucleotide expression system according to clause 19, wherein the the *Actin-4* gene is from *Aedes spp.*

21. A polynucleotide expression system according to clause 19, wherein the the *Actin-4* gene is from *Aedes aegypti AeActin-4.*

22. A polynucleotide expression system according to any of clauses 1-14, wherein the splicing mechanism comprises at least a fragment of the *doublesex* (*dsx*) gene, preferably that derived from *Drosophila, B. mori,* Pink Boll Worm, Codling Moth, or a mosquito, in particular *Aedes gambiae* or especially *Aedes aegypti.*

23. A polynucleotide expression system according to any of clauses 19-22, wherein the splice control sequence and the heterologous polynucleotide sequence encoding a functional protein, defined between a start codon and a stop codon, and/or polynucleotides for interference RNA (RNAi), to be expressed in an organism, are provided in the form of a minigene construct or a cassette exon.

24. A polynucleotide expression system according to clause 4, wherein the system is a plasmid or construct selected from the group consisting of any one of Figures 16-18, 22-24, 26-32, 49, 52-55, and 61-69, and/or SEQ ID NOs 46-48, 50-56, 143-145 and 151-162.

25. A polynucleotide expression system according to any preceding clause, wherein the at least one splice control sequence is intronic and comprises on its 5' end guanine (G) nucleotide, in RNA.

26. A polynucleotide expression system according to any preceding clause, wherein the at least one splice control sequence is intronic and comprises on its 5' end UG nucleotides and UT at its 3' end, in RNA.

27. A polynucleotide expression system according to any preceding clause, wherein the mediation is sex-specific and further mediated or controlled by binding of the TRA protein or TRA/TRA2 protein complex, or homologues thereof.

28. A polynucleotide expression system according to clause 27, wherein the system comprises the consensus sequence: TCWWCRATCAACA, where W = A or T and R = A or G.

29. A polynucleotide expression system according to any preceding clause, wherein the organism is a mammal, a fish an invertebrate, an arthropod, an insect or a plant.

30. A polynucleotide expression system according to any preceding clause, wherein the organism is an insect from the Order Diptera.

31. A polynucleotide expression system according to clause 30, wherein the insect is a tephritid fruit fly selected from the group consisting of: Medfly (*Ceratitis capitata*), Mexfly (*Anastrepha ludens),* Oriental fruit fly (*Bactrocera dorsalis*), Olive fruit fly (*Bactrocera oleae*), Melon fly (*Bactrocera cucurbitae*), Natal fruit fly (*Ceratitis rosa*), Cherry fruit fly *(Rhagoletis cerasi*), Queensland fruit fly *(Bactrocera tyroni),* Peach fruit fly *(Bactrocera zonata*) Caribbean fruit fly (*Anastrepha suspensa*) and West Indian fruit fly (*Anastrepha obliqua*).

32. A polynucleotide expression system according to clause 30, wherein the insect is a mosquito from the genera *Stegomyia, Aedes, Anopheles* or *Culex.*

33. A polynucleotide expression system according to clause 32, wherein the mosquito is selected from *Aedes aegypti, Aedes albopictus, Anopheles stephensi, Anopheles albimanus* and *Anopheles gambiae.*

34. A polynucleotide expression system according to clause 30, wherein the insect is selected from the group consisting of: the New world screwworm *(Cochliomyia hominivorax),* Old world screwworm (*Chrysomya bezziana*) and Australian sheep blowfly *(Lucilia cuprina*), codling moth (*Cydia pomonella),* the silk worm *(Bombyx mori),* the pink bollworm (*Pectinophora gossypiella),* the diamondback moth *(Plutella xylostella),* the Gypsy moth (*Lymantria dispar*), the Navel Orange Worm *(Amyelois transitella),* the Peach Twig Borer *(Anarsia lineatella)* and the rice stem borer (*Tryporyza incertulas*), the noctuid moths, especially Heliothinae, the Japanese beetle *(Popilla japonica*), White-fringed beetle (*Graphognatus* spp.), Boll weevil (*Anthonomous grandis*), corn root worm (*Diabrotica spp*) and Colorado potato beetle (*Leptinotarsa decemlineata*).

35. A polynucleotide expression system according to clause 30, wherein the insect is not a Drosphilid.

36. A polynucleotide expression system according to any preceding clause, wherein the expression of the heterologous polynucleotide sequence leads to a phenotypic consequence in the organism.

37. A polynucleotide expression system according to clause 1 or 2, wherein the polynucleotide sequence to be expressed comprises a polynucleotides for interference RNA (RNAi).

38. A method of population control of an organism in a natural environment therefor, comprising:

i) breeding a stock of the organism,

the organism carrying a gene expression system comprising a system according to any of clauses 1-36 which is a dominant lethal genetic system,

ii) distributing the said stock animals into the environment at a locus for population control; and
iii) achieving population control through early stage lethality by expression of the lethal system in offspring that result from interbreeding of the said stock individuals with individuals of the opposite sex of the wild population.

39. A method according to clause 38, wherein the early stage lethality is embryonic or before sexual maturity.

40. A method according to clause 39, wherein the early stage lethality occurs early in development.

41. A method according to clause 38 or 39, wherein the lethal effect of the lethal system is conditional and occurs in the said natural environment *via* the expression of a lethal gene, the expression of said lethal gene being under the control of a repressible transactivator protein, the said breeding being under permissive conditions in the presence of a substance, the substance being absent from the said natural environment and able to repress said transactivator.

42. A method of biological control, comprising:

i) breeding a stock of males and female organisms transformed with the system according to any of clauses 1-36 under permissive conditions, allowing the survival of males and females, to give a dual sex biological control agent;
ii) optionally before the next step imposing or permitting restrictive conditions to cause death of individuals of one sex and thereby providing a single sex biological control agent comprising individuals of the other sex carrying the conditional lethal genetic system;
iii) releasing the dual sex or single sex biological control agent into the environment at a locus for biological control; and
iv) achieving biological control through expression of the genetic system in offspring resulting from interbreeding of the individuals of the biological control agent with individuals of the opposite sex of the wild population.

43. A method of sex separation comprising:

i) breeding a stock of male and female organisms transformed with the expression system according to any of clauses 1-36 under permissive or restrictive conditions, allowing the survival of males and females; and
ii) removing the permissive or restrictive conditions to induce the lethal effect of the lethal gene in one sex and not the other by sex-specific alternative splicing of the lethal gene.

44. A method or biological or population control comprising;

i) breeding a stock of male and female organisms transformed with the gene expression system according to any of clauses 1-36 under permissive or restrictive conditions, allowing the survival of males and females;
ii) removing the permissive or restrictive conditions to induce the lethal effect of the lethal gene in one sex and not the other by sex-specific alternative splicing of the lethal gene to achieve sex separation;
iii) sterilising or partially sterilising the separated individuals and
iv) achieving said control through release of the separated sterile or partially sterile individuals in to the natural environment of the organism.

**Claims**

**1.** A polynucleotide expression system, comprising:

a heterologous polynucleotide sequence, which encodes an RNA for RNA interference (RNAi) and/or a functional protein, the coding sequence of which is defined between a start codon and a stop codon;
a promoter operably linked to the heterologous polynucleotide sequence; and
a splice control sequence, which, in cooperation with a spliceosome, is capable of mediating:

(i) a first splicing of an RNA transcript of the polynucleotide sequence to produce a first spliced RNA product, and
(ii) an alternative splicing of said RNA transcript to yield an alternatively spliced RNA product,

wherein the splice control sequence is derived from a *transformer* (*tra*) gene, a *doublesex* (*dsx*) gene, or an *Actin-4* gene.

**2.** The polynucleotide expression system of claim 1, wherein the mediation of the alternative splicing is a sex-specific mediation, a developmental stage-specific mediation, a germline-specific mediation, a tissue-specific mediation, or a combination thereof.

**3.** The polynucleotide expression system of claim 1 or 2, wherein the functional protein comprises a marker, or has a lethal, deleterious, or sterilizing effect, optionally wherein the effect of the functional protein is dominantly lethal.

**4.** The polynucleotide expression system of claim 3, wherein the lethal, deleterious, or sterilizing effect of the functional protein is conditionally suppressible.

**5.** The polynucleotide expression system of any of claims 1-4, wherein the promoter is selected from the group consisting of the *hsp70* heat shock protein promoter, the *srya* embryo-specific promoter from *Drosophila melanogaster* or a homologue thereof, and the *Drosophila* gene *slow as molasses (slam)* or a homologue thereof.

**6.** The polynucleotide expression system of any of claims 1-5, wherein the functional protein comprises an apoptosis-inducing factor, Hid, Reaper (Rpr), and Nipp1Dm.

**7.** The polynucleotide expression system of any of claims 1-6, wherein the functional protein comprises a positive transcriptional control factor for the promoter, such that the functional protein or its expression is controlled by a positive feedback mechanism.

**8.** The polynucleotide expression system of any of claims 1-7, further comprising an enhancer associated with the promoter, wherein the functional protein is capable of enhancing activity of the promoter via the enhancer, optionally wherein the functional protein comprises a tTA gene product or analogue thereof (such as tTAV, tTAV2, and tTAV3) and the enhancer comprises one or more tetO operator units operably linked to the promoter.

**9.** The polynucleotide expression system of any of claims 1-8, wherein:

the splice control sequence is derived from a *tra* intron, such as one from the Medfly transformer gene *Cctra* or from an ortholog or homolog of the *Drosophila transformer* gene, for example, an ortholog or homolog of the *Drosophila transformer* gene in a tephritid fruit fly such as *C. rosa* or *B. zonata*;
the splice control sequence is derived from a *dsx* intron, such as one from *Drosophila,* silk worm (*Bombyx mori*), pink bollworm (*Pectinophora gossypiella),* codling moth (*Cydia pomonella*), or a mosquito, such as *Aedes gambiae* or *Aedes aegypti*;
the splice control sequence is derived from an *Actin-4* gene, such as one from *Aedes* spp. or *Aedes aegypti.*

**10.** The polynucleotide expression system of any of claims 1-9, wherein the splice control sequence comprises:

on its 5' end, guanine (G) nucleotide, in RNA, or
on its 5' end, GU nucleotides and AG at its 3' end, in RNA.

**11.** The polynucleotide expression system of any of claims 1-10, wherein the splice control sequence is intronic and comprises:

a protein binding domain, such as a binding domain for a TRA protein or a TRA/TRA2 protein complex, and
a splice donor sequence GT on its 5' end (5'-GT),

wherein the intronic splice control sequence is flanked by a 5' guanine (G) nucleotide and is 3' to the ATG start codon of the heterologous polynucleotide sequence.

**12.** The polynucleotide expression system of any of claims 1-11, wherein the splice control sequence comprises the nucleotide sequence of SEQ ID NO: 1 (TCWWCRATCAACA, wherein W =A or T and R =A or G) or its RNA equivalent.

**13.** The polynucleotide expression system of any of claims 1-12, wherein transcription from the promoter is activated by an environmental condition, such as the environmental temperature and the presence or absence of tetracycline.

**14.** The polynucleotide expression system of any of claims 1-13, which comprises a nucleotide sequence selected from the group consisting of SEQ ID NOs: 46-48, 50-56, 106, 143-145, and 151-162.

**15.** A method of generating a transgenic organism, comprising transforming an organism with the polynucleotide expression system of any of claims 1-14.

**16.** A transgenic organism generated by the method of claim 15.

**17.** A transgenic organism comprising the polynucleotide expression system of any of claims 1-14.

**18.** The transgenic organism of claim 16 or 17, wherein the transgenic organism is optionally a non-human mammal, a fish, an invertebrate, an arthropod, an insect, or a plant, wherein the insect is optionally from the Order Diptera, and wherein the insect is optionally:

a tephritid fruit fly selected from the group consisting of Medfly (*Ceratitis capitata*), Mexfly (*Anastrepha ludens),* Oriental fruit fly (*Bactrocera dorsalis*), Olive fruit fly (*Bactrocera oleae*), Melon fly (*Bactrocera cucurbitae*), Natal fruit fly (*Ceratitis rosa*), Cherry fruit fly (*Rhagoletis cerasi*), Queensland fruit fly (*Bactrocera tyroni*), Peach fruit fly (*Bactrocera zonata*), Caribbean fruit fly (*Anastrepha suspensa*) and West Indian fruit fly (*Anastrepha obliqua*);
a mosquito from the genera *Stegomyia, Aedes, Anopheles,* or *Culex,* such as *Aedes aegypti, Stegomyia albopicta* (also known as *Aedes albopictus*), *Anopheles stephensi, Anopheles albimanus,* and *Anopheles gambiae*;
a Calliphoridae, such as New world screwworm (*Cochliomyia hominivorax*)*,* Old world screwworm (*Chrysomya bezziana*), and Australian sheep blowfly (*Lucilia cuprina*);
a Lepidoptera, such as a moth including the codling moth (*Cydia pomonella*), silk worm (*Bombyx mori*), pink bollworm (*Pectinophora gossypiella*), diamondback moth (*Plutella xylostella),* Gypsy moth (*Lymantria dispar*), Navel Orange Worm (*Amyelois transitella*), Peach Twig Borer (*Anarsia lineatella*), rice stem borer (*Tryporyza incertulas*), and a noctuid moth such as Heliothinae; or
a Coleoptera, such as Japanese beetle (*Popilla japonica*), White-fringed beetle (*Graphognatus* spp.), Boll weevil (*Anthonomous* grandis), corn root worm (*Diabrotica* spp.), and Colorado potato beetle (*Leptinotarsa decemlineata*).

**19.** A breeding stock comprising the transgenic organism of any of claims 16-18.

**20.** The breeding stock of claim 19, which is maintained under a permissive condition allowing the survival of both male and female organisms of the stock, wherein organisms from the stock are capable of breeding with organisms of the opposite sex in a wild-type population to produce offspring expressing the functional protein or RNAi, thereby achieving biological control of the wild-type population.

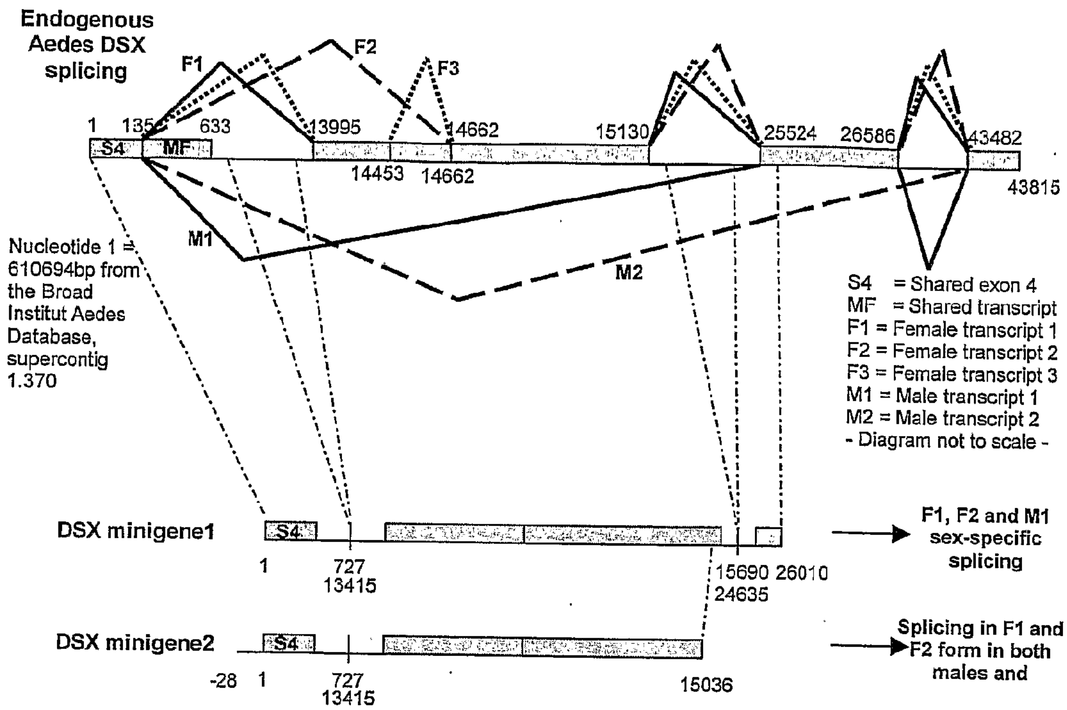


Figure 1

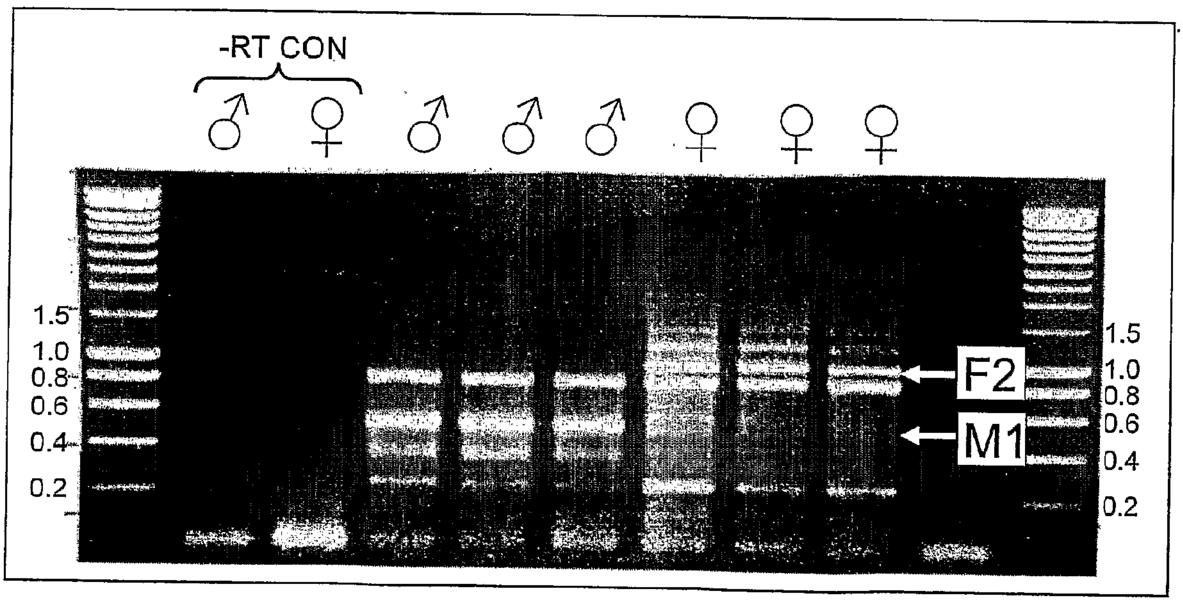


Figure 2

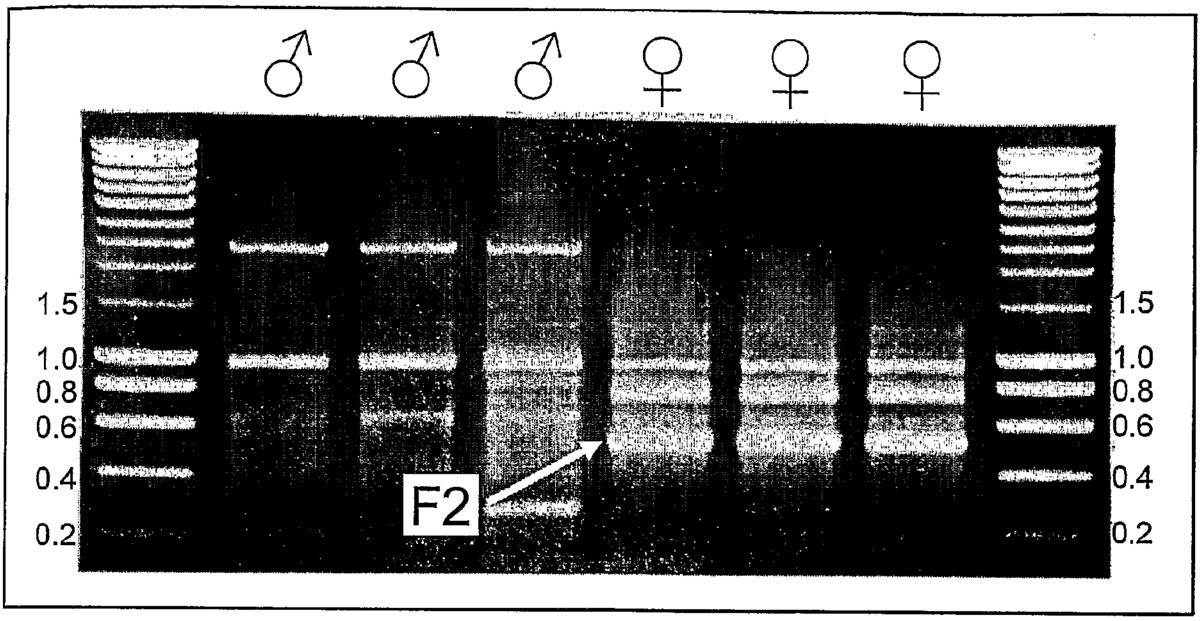


Figure 3

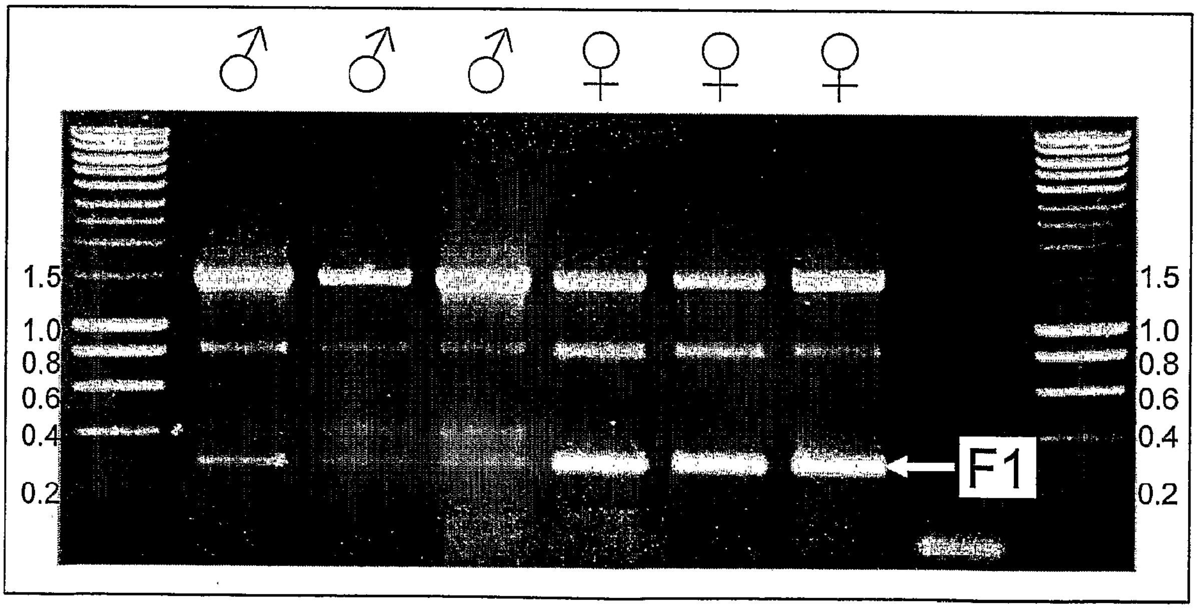


Figure 4

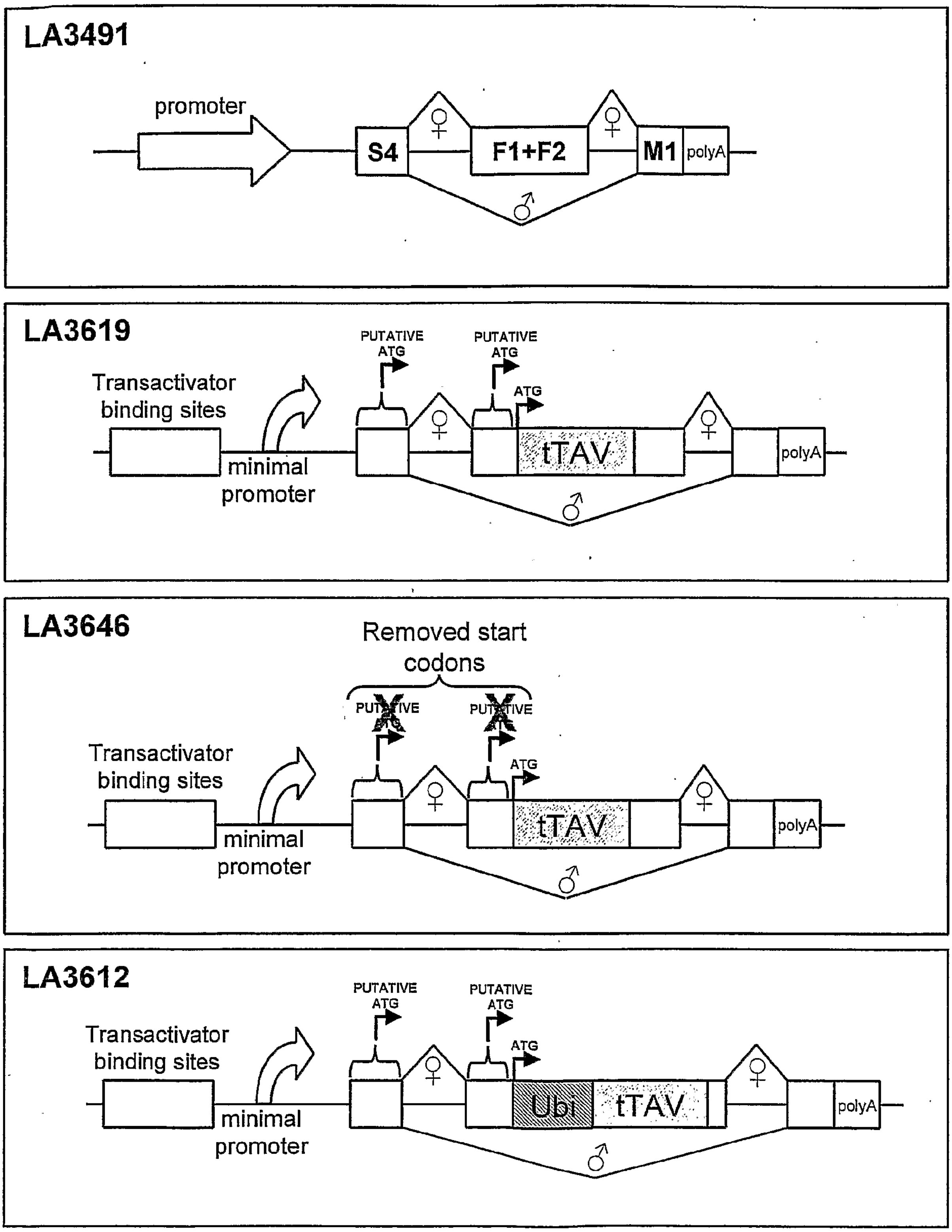
LA3491
promoter
S4
F1+F2
M1
polyA
LA3619
PUTATIVE ATG
PUTATIVE ATG
ATG
Transactivator binding sites
minimal promoter
tTAV
polyA
LA3646
Removed start codons
ATG
Transactivator binding sites
minimal promoter
tTAV
polyA
LA3612
PUTATIVE ATG
PUTATIVE ATG
ATG
Transactivator binding sites
minimal promoter
Ubi
tTAV
polyA

Figure 5

```
PBW-dsx         GTCCAATCGATCACAATGTATCACAACGTTGCGAATTCAGTTTCACAATCACACGCAACA 3910
Bombyx-dsx      G--CGATTATTTA-ATTCTATAT--ATTTTTCAAATTCAGTTTCTATTCCACTAACAATG 1170
codling-dsx     TTACAAACAAT-GTACGGAGCTACAACGTTGCAAGTTCGGTCCCCACACAAC--ACAATG 3034
                   * *    *   *          *  ** * * *** **  *      **   ***

PBW-dsx         AA-CRCGRCACGT------------TACAAATTAGTTACTT-TGAATCGATCGATTA-TG 3955
bombyx-dsx      TA-CACTACACGTACACA-------TACACACAACAAAATAGTGAATCGATAAATTAGTG 1222
codling-dsx     TGTCATAACACATTAACAACATTGTTACACACCCACACATACAAATTTGCTAAGTTGATA 3094
                   *    *** *            **** *        *    * * * *   **  *

PBW-dsx         ATGCGGCCGACTCARCGGCCCC-CG--GCAGCACTAACC-AGTAGTGATTTCCACTTTGC 4011
bombyx-dsx      GTCGAATAATCGGAATGGTTCG-CATCACACTACTAACC-AGTCGTGATTTCCACTTTAC 1280
codling-dsx     AAAGAGTGGTGTGTCCGACGAATCAGAACATCACTAACCCAGTCGTGATTTCATTTCCAC 3154
                                *      *    **   ******* *** ********   *   *

PBW-dsx         AGTGACCGGACCAA-------AACTTCGAAATTCGAATTGTAAAGTGACAGTTC--ATTT 4062
bombyx-dsx      AGTGACCGGACGAAGGTGGAGAAATTCGAAATTTAAATATAAAAGTGACAATTCGAATTT 1340
codling-dsx     AGTGACCGGACGAAGGTGGAGAAGTTCGAAATTTAAA---AAAAGTGACCACAT---TTT 3208
                *********** **       ** *********  **    ********        ***

PBW-dsx         CC-CGC-------CAAGTGTTGTGCCAGTGTC---ATGTCGATATT-TATTTTATTTTCT 4110
bombyx-dsx      CCACGCGCGCGCTCTAGTGATGTGCCAGTGTGTGAATATCAATATTATTTTTTTATTTCT 1400
codling-dsx     ATTTAA--------TAGTGATGTGCAAGTG-----ATAC---TATTTTTATTTTGTTTTT 3252
                               **** ***** ****     **     **** *  ***  *** *

PBW-dsx         TTTTTGTAGGAAAATGCTGAGCGAAATTAATAATATAAGTGGTGTACTATCGTCGTCCAT 4170
bombyx-dsx      TTTTTGTAGGAAAATGCTG---GAAATTAATAATATAAGTGGTGTACTGTCTTCGTCAAT 1457
codling-dsx     CTTTTGTAGGAAAATGCTGAGCGAAATAAATAATTTTAGTGGTGTGCTATCGTCATCGAT 3312
                 *****************    ***** ****** * ******** ** ** ** ** **

PBW-dsx         GAAGTTATTTTGCGAATGATACTTTGTTTTGTATGTGCTGTGTGTTGTGTGGACTTTTGC 4230
bombyx-dsx      GAAGTTATTTTGCGAATGATACTTAGTTTTACAAGTGCCGTGGTGTGTGTTGACACTTGC 1517
codling-dsx     GAAGTTGTTTTGCGAATGATACTATGTTCTTCAAGTGCTGTGTTTTGTG-GACTGTGGGG 3371
                ****** ***************   *** *  * **** ***   ****         *
```

```
PBW-dsx         TGTGCGTTGCTGTT--TGCGATGGAAGGACTAT-TGTGTCGTCGCCACGCTGGACTATTC 4287
bombyx-dsx      TGTGCGATGCTGTG--CGAATTTCAACGGAAATATTTGTTGTCGTAACATTGGATCTATG 1575
codling-dsx     TGACTGTTCCTGTAAATAAGCTTCGTTGGACAT-TGTGTC-TCAC-ACATCGGATCTCAT 3428
                **   * * ****        *     *   ** * ***  **   **   ***

PBW-dsx         GGTGAGTGG------TAGAATAATA-TTTTATCTA-------------TTTCATCGCGGT 4327
bombyx-dsx      GGTAAGTT-------TAGTATAATAACTTTACTCT-------------GTTCACATTAGT 1615
codling-dsx     GGTAAGTGCTAGTGCTAGCATYRMAACTTAACTCTCTGAGCGAATTCCTTTGACTCTAAA 3488
                *** ***        *** **   *  ** *                  ** *

PBW-dsx         ACAATTGACTTTTTATTACTACTCACTGCTATGGAGGAATCTCAGGAACAT----CGTAA 4383
bombyx-dsx      GAAACATACATTTG--TAAAATTTG-TGTTTT--ACTAATGTGAAATTTAT----TTTTG 1666
codling-dsx     GTCACACGRACAGCCATACAATCAA-AGCTACGCTCTAATTTTAAGATGACAWTCTGTAA 3547
                   *            **  *      * *       *** * *     *       *
```

Figure 6: the second female-specific exon is marked by bold nucleotides. The conserved repeats AGTGAC/T are underlined.

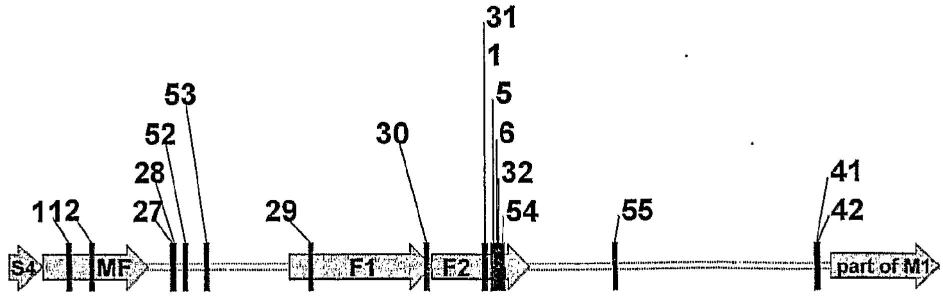


Figure 7

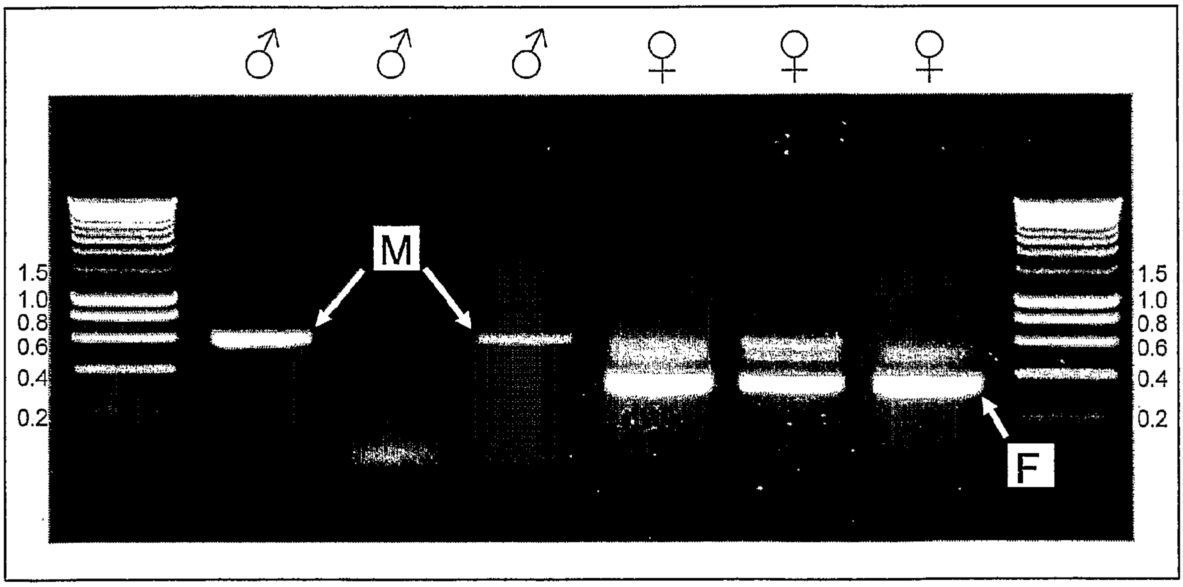


Figure 8

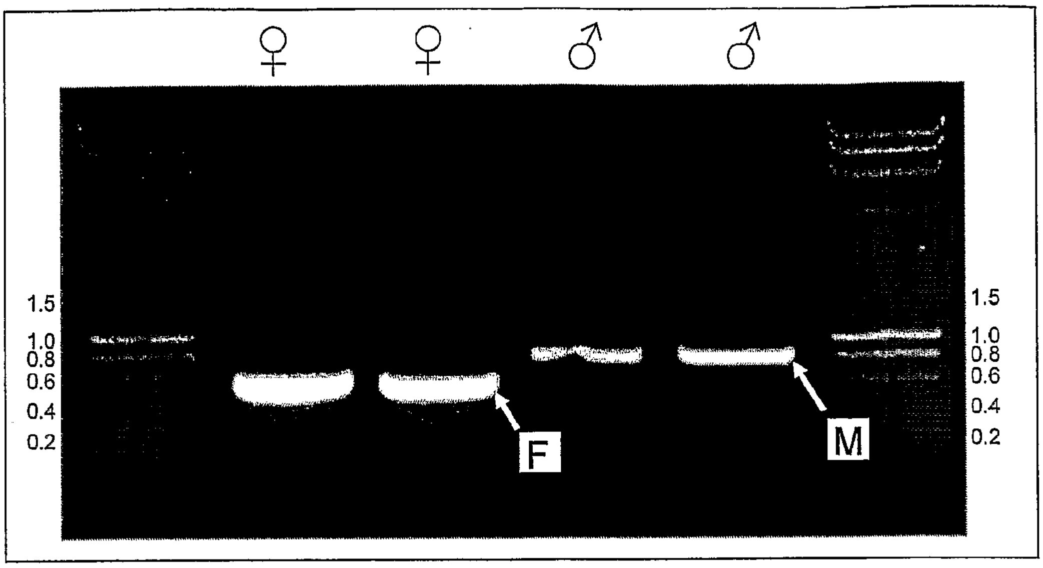


Figure 9

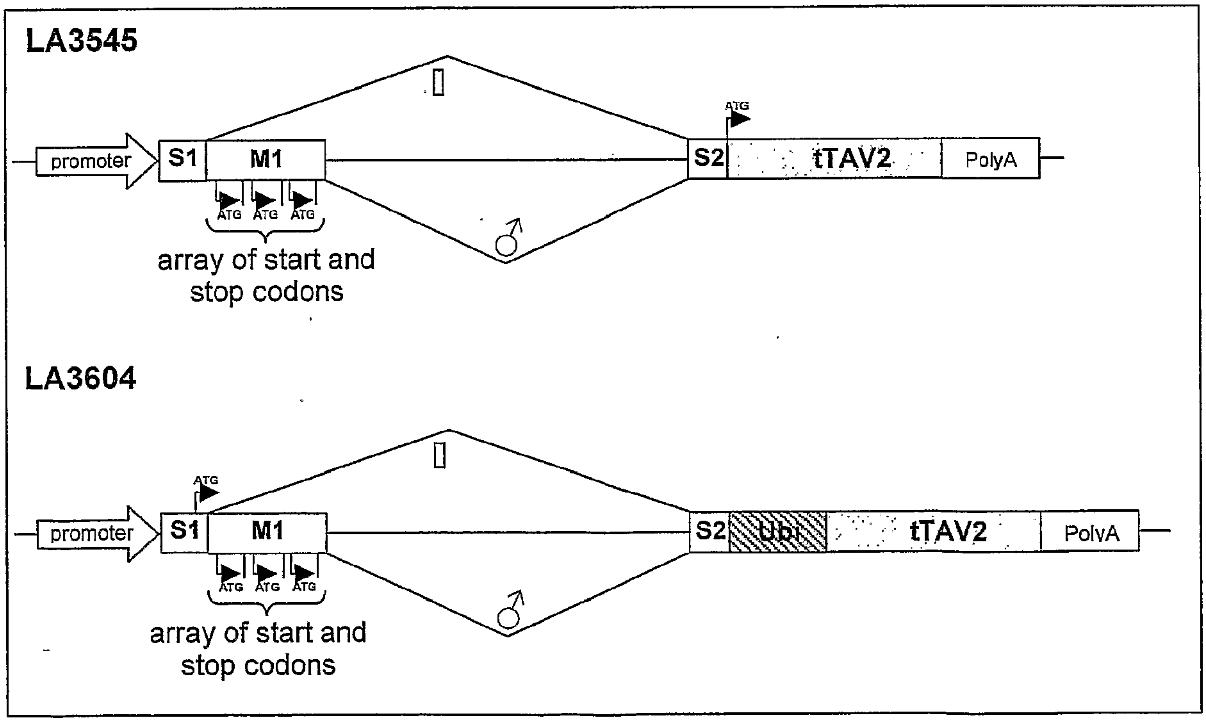


Figure 10

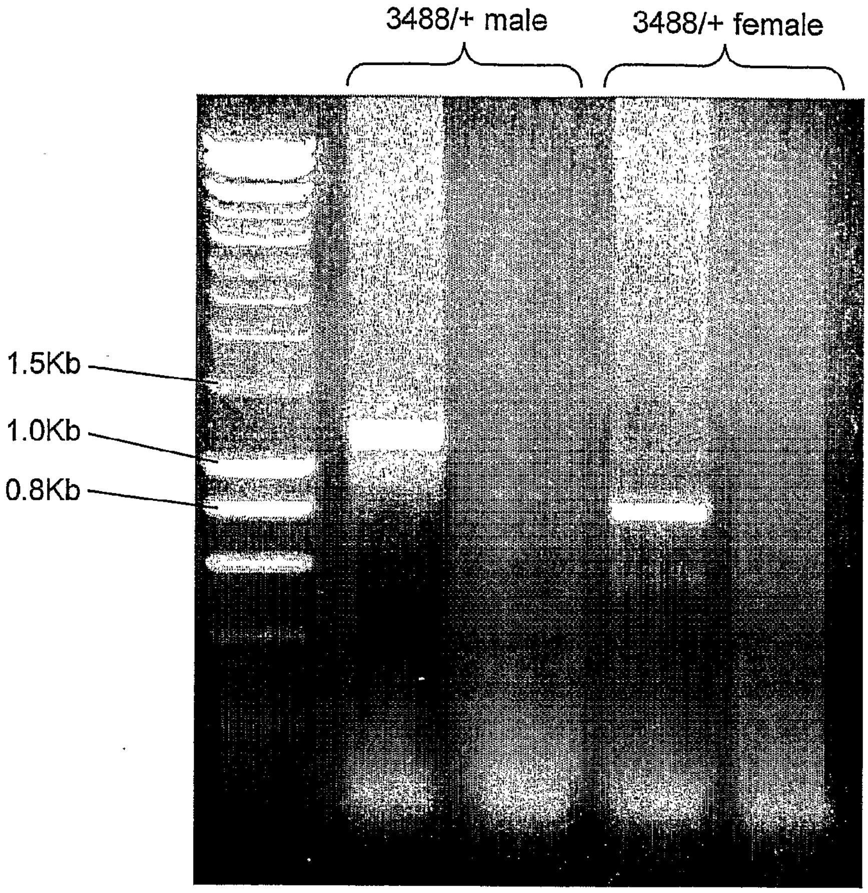


Figure 11

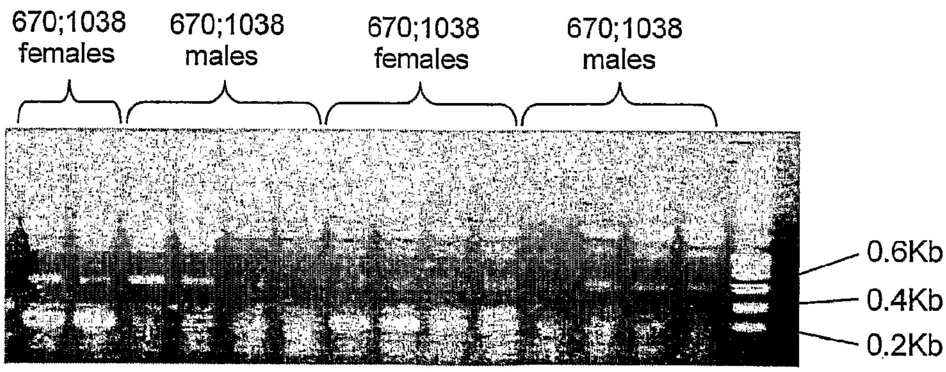


Figure 12

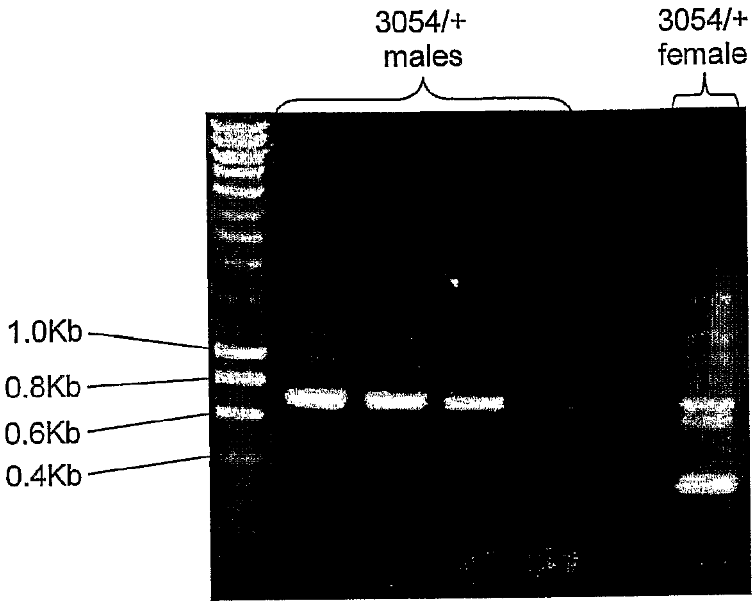


Figure 13

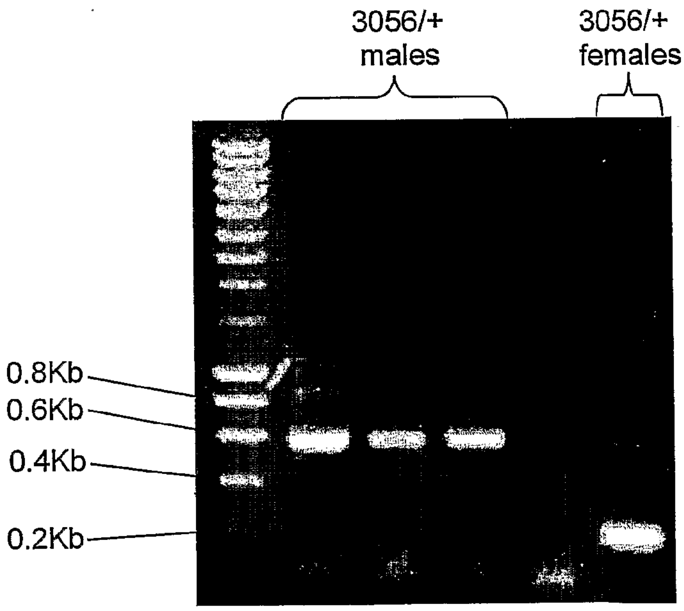


Figure 14

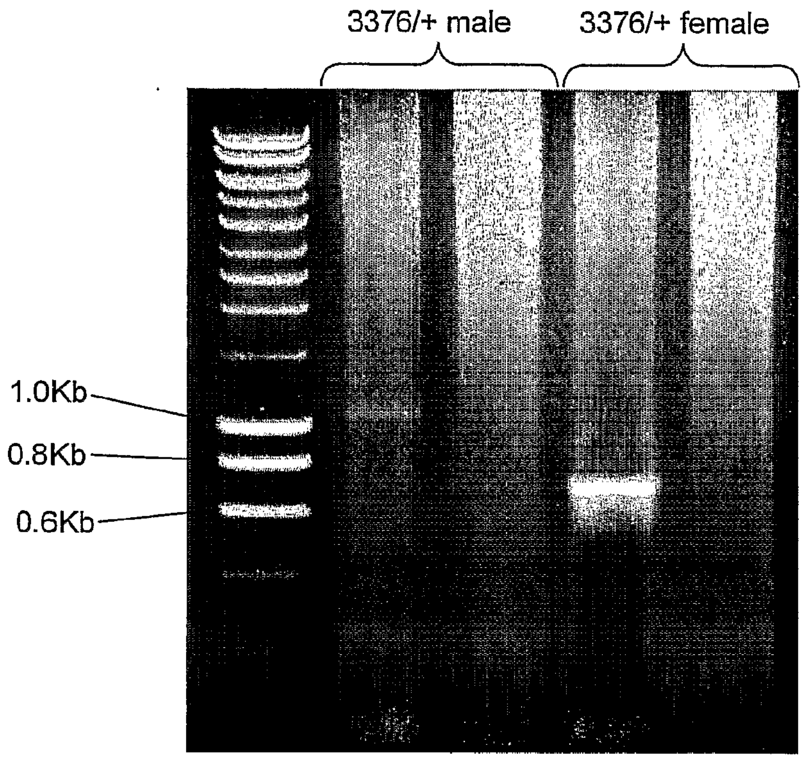


Figure 15

Figure 16

plasmid LA3582

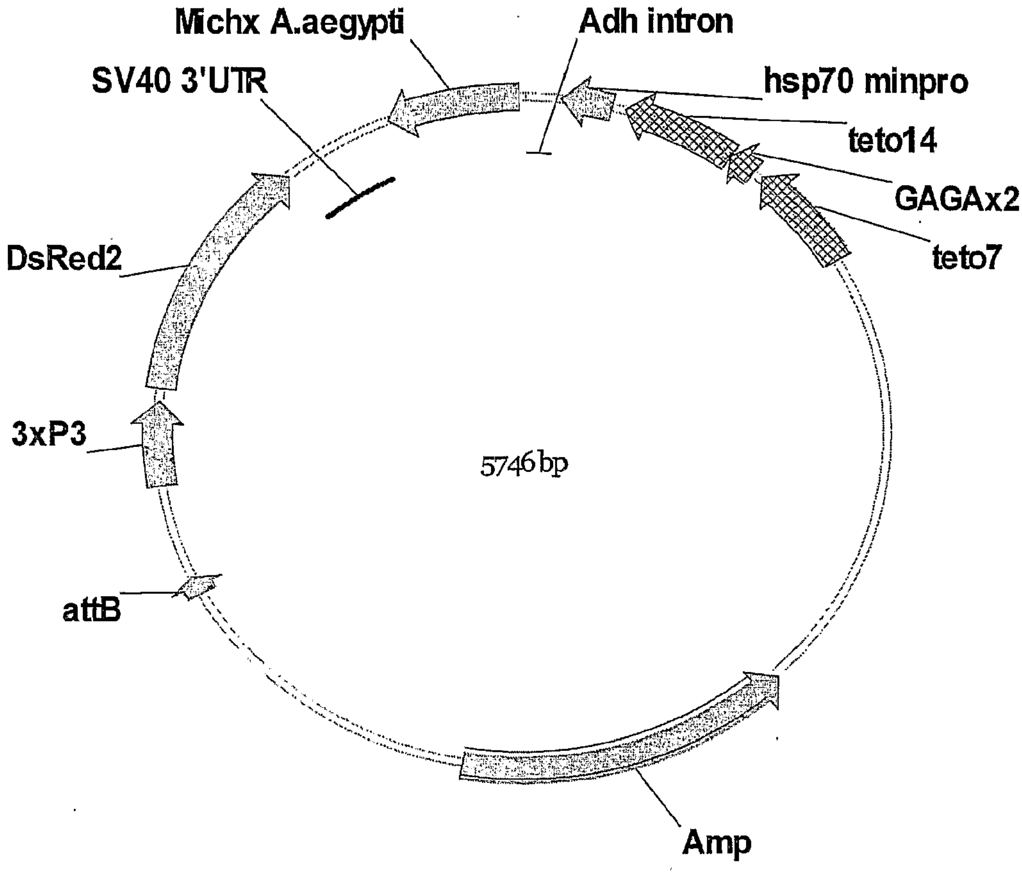


#3581,2 AttB-3xP3DsRed2-teto21-hsp-adh-michxc

Figure 17

plasmid LA3576

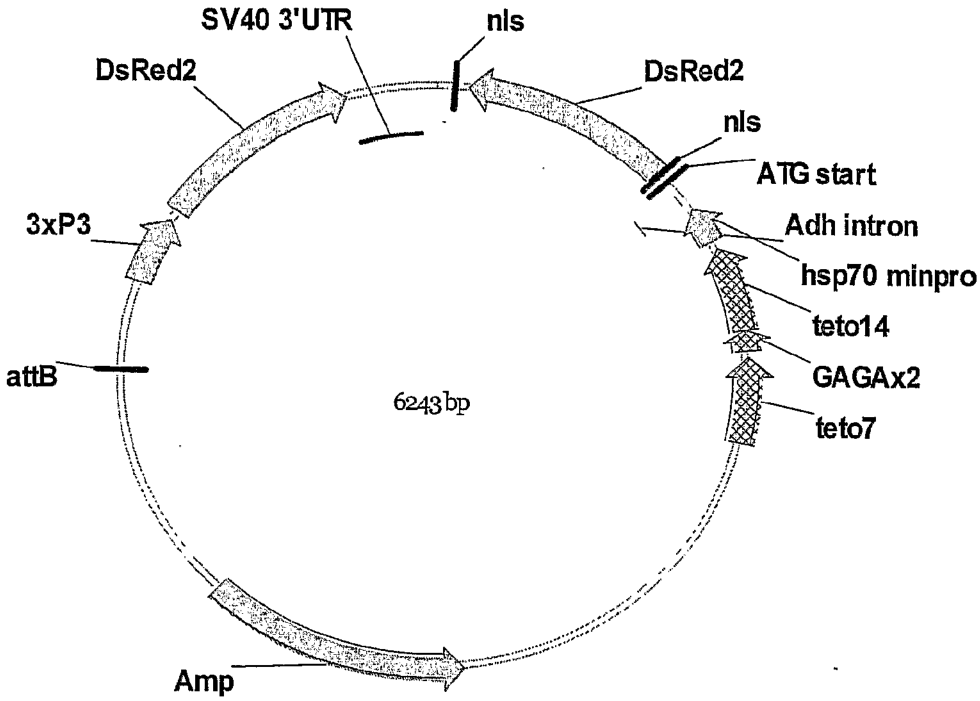


#3575,6 AttB-3xP3DsRed2-teto21-hsp-adh-dsred

Figure 18 - LA3619 plasmid map

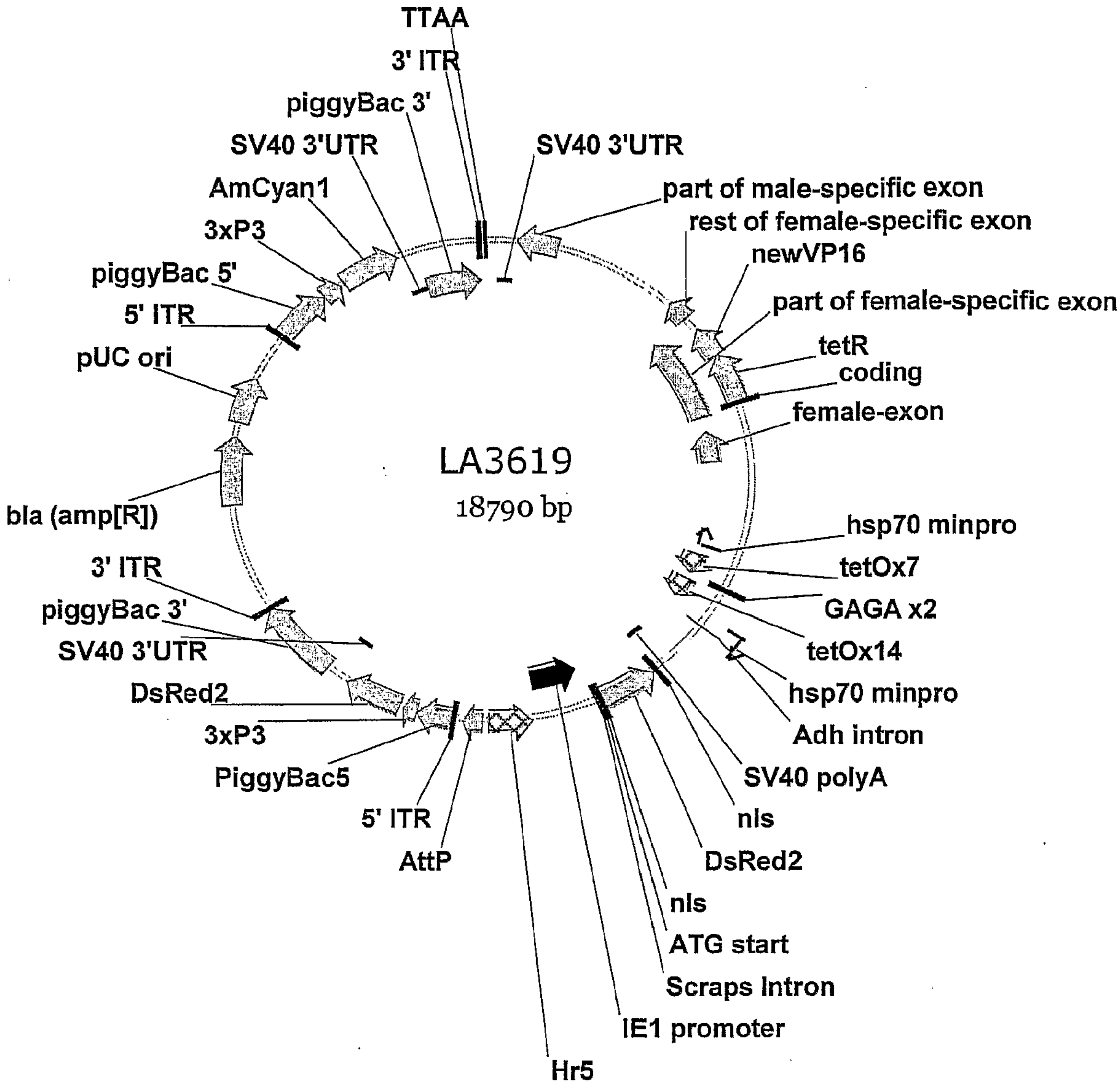

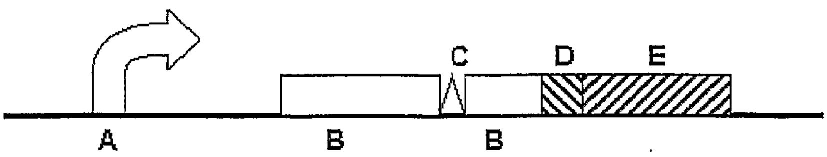


Figure 19

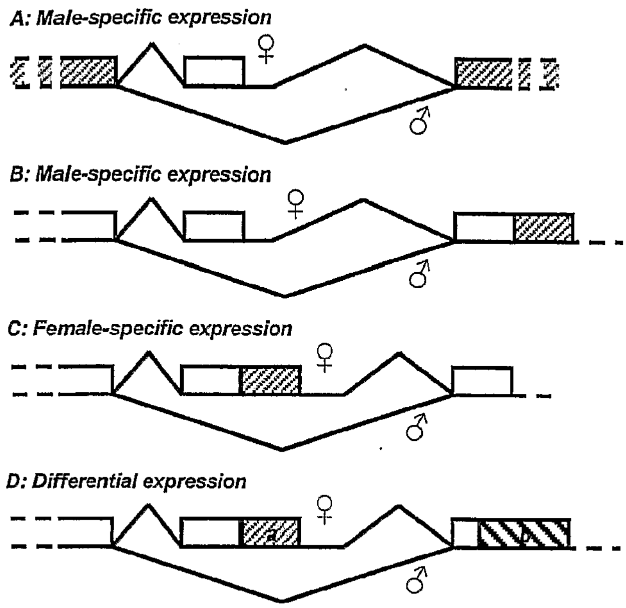
A: Male-specific expression
♀
♂
B: Male-specific expression
♀
♂
C: Female-specific expression
♀
♂
D: Differential expression
♀
♂

Figure 20

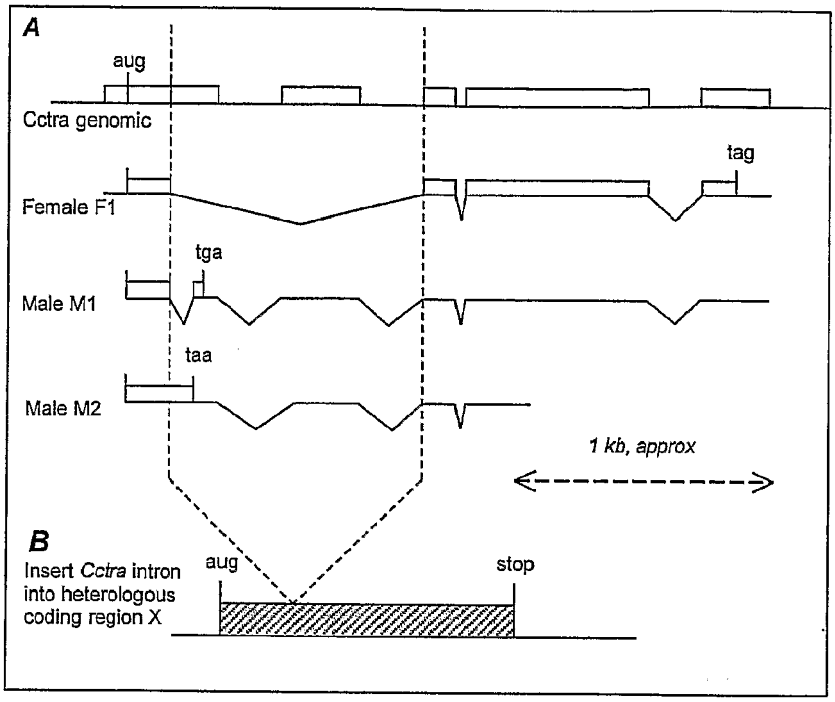
A
aug
Cctra genomic
tag
Female F1
tga
Male M1
taa
Male M2
1 kb, approx
B
Insert Cctra intron into heterologous coding region X
aug
stop

Figure 21

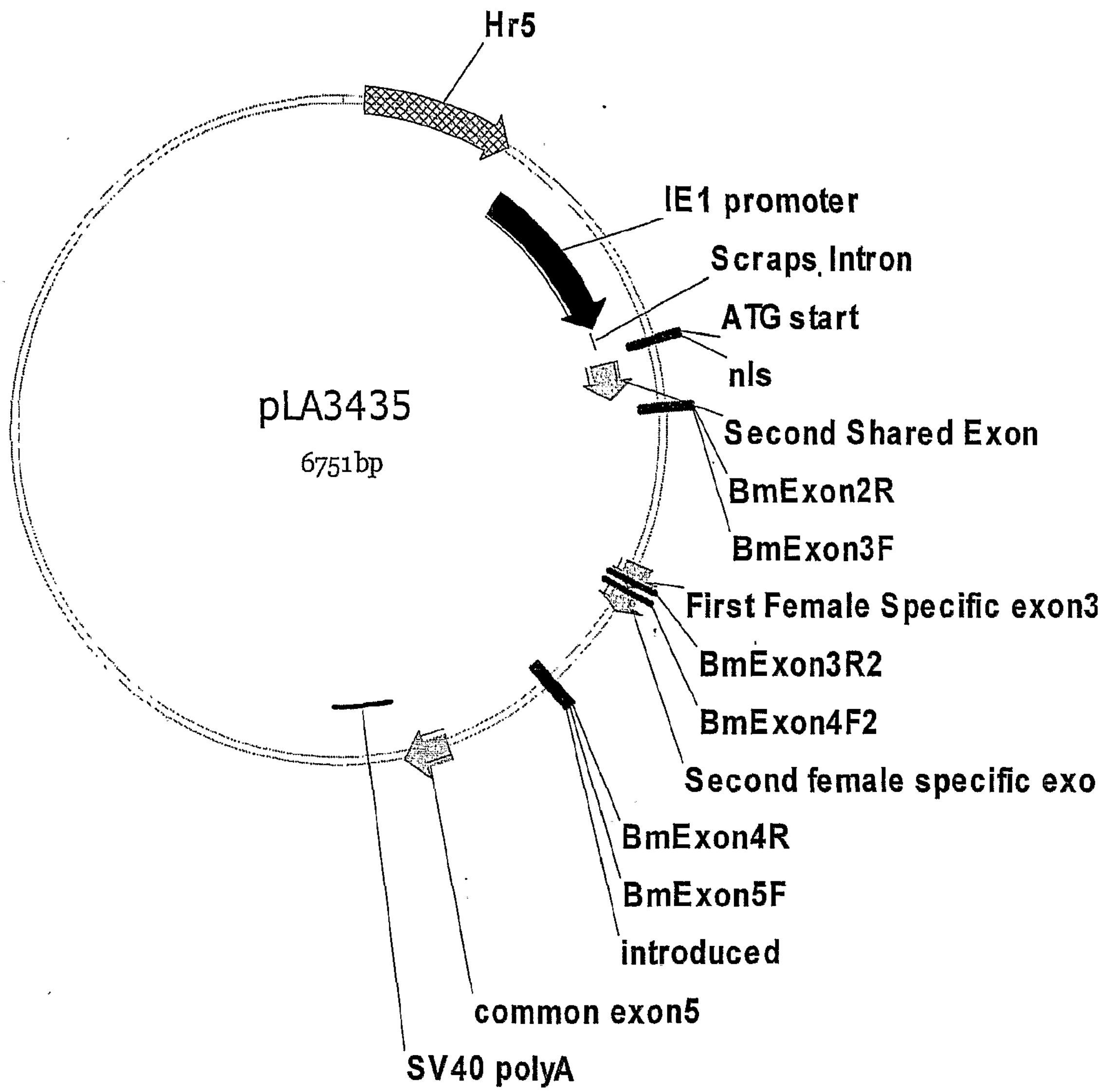
Hr5
IE1 promoter
Scraps Intron
ATG start
nls
pLA3435
6751bp
Second Shared Exon
BmExon2R
BmExon3F
First Female Specific exon3
BmExon3R2
BmExon4F2
Second female specific exo
BmExon4R
BmExon5F
introduced
common exon5
SV40 polyA

Figure 22

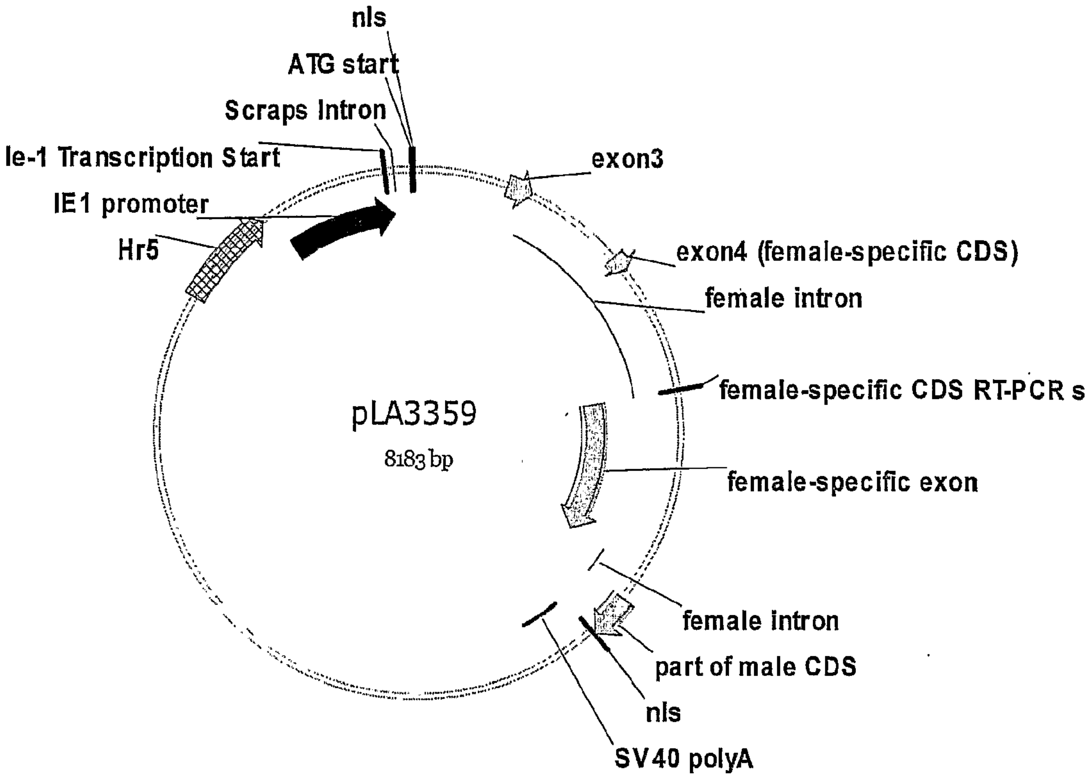
nls
ATG start
Scraps Intron
Ie-1 Transcription Start
IE1 promoter
Hr5
exon3
exon4 (female-specific CDS)
female intron
female-specific CDS RT-PCR s
pLA3359
8183 bp
female-specific exon
female intron
part of male CDS
nls
SV40 polyA

Figure 23

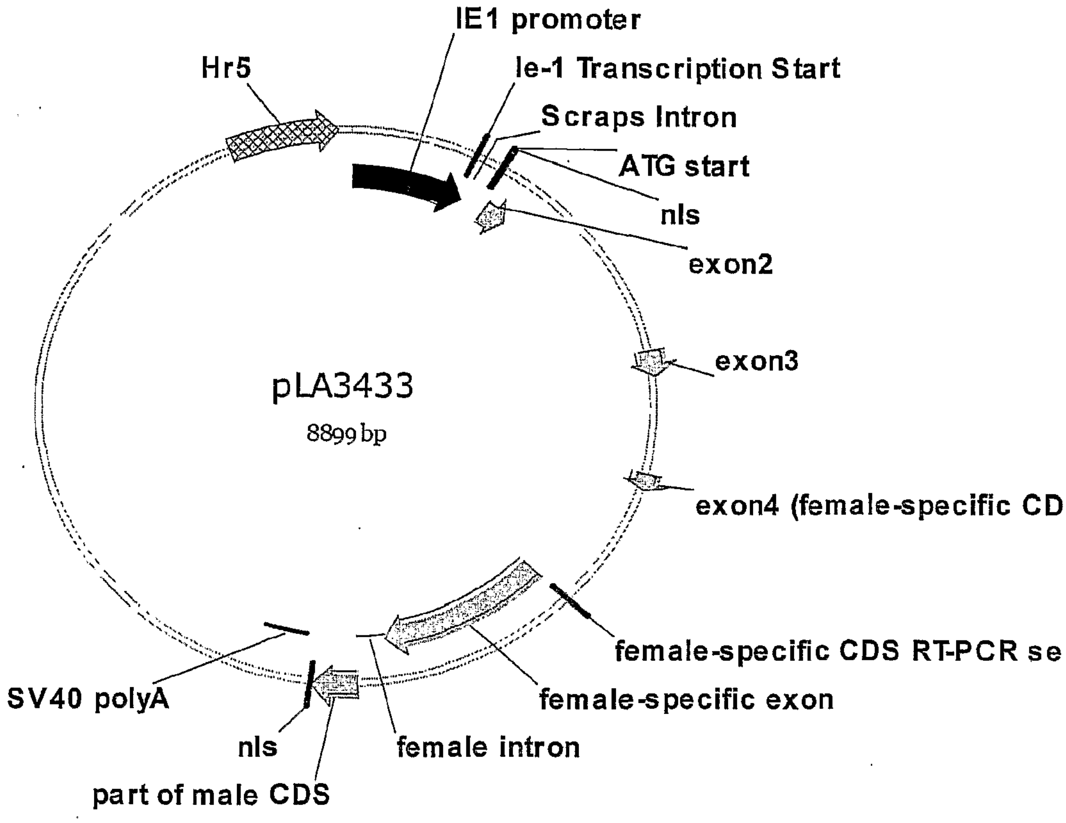
IE1 promoter
Hr5
Ie-1 Transcription Start
Scraps Intron
ATG start
nls
exon2
exon3
pLA3433
8899 bp
exon4 (female-specific CD
female-specific CDS RT-PCR se
female-specific exon
SV40 polyA
nls
female intron
part of male CDS

Figure 24

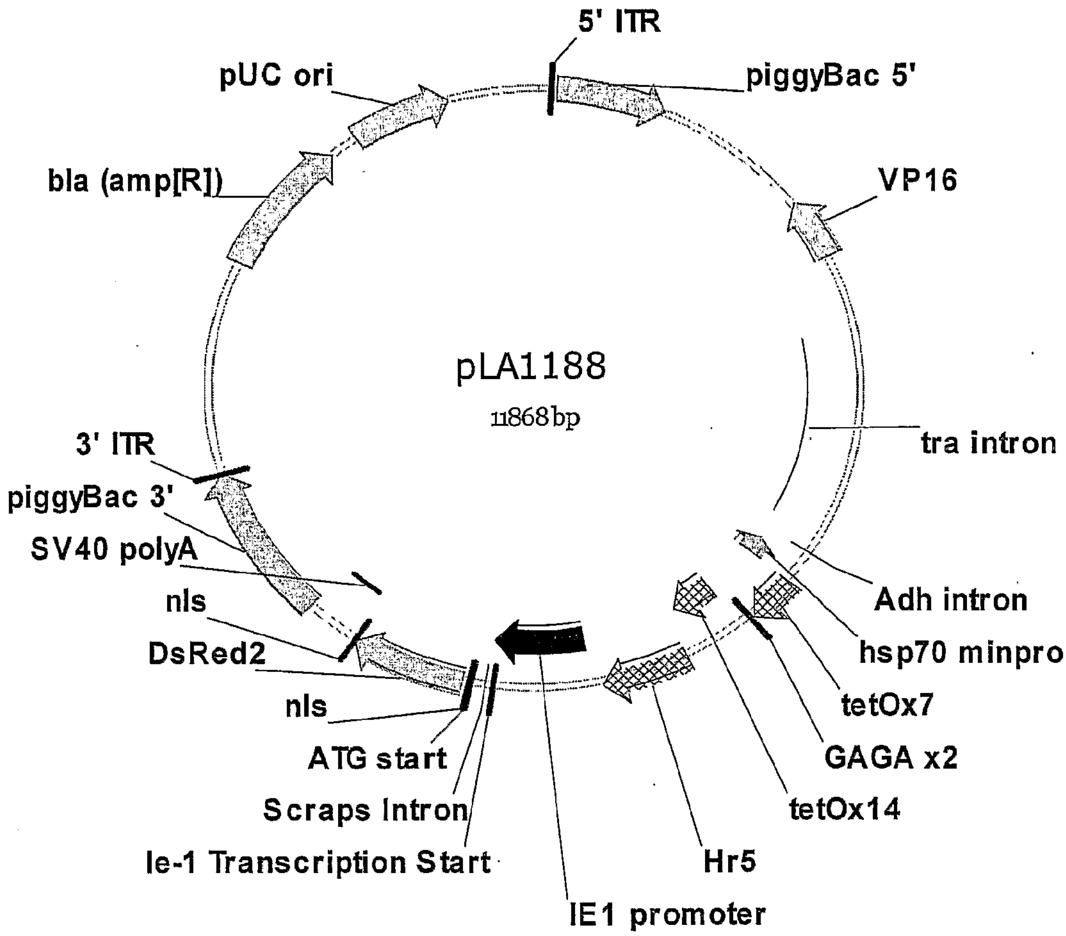
5' ITR
pUC ori
piggyBac 5'
bla (amp[R])
VP16
pLA1188
11868bp
tra intron
3' ITR
piggyBac 3'
SV40 polyA
Adh intron
nls
hsp70 minpro
DsRed2
tetOx7
nls
GAGA x2
ATG start
Scraps Intron
tetOx14
Ie-1 Transcription Start
Hr5
IE1 promoter

Figure 25

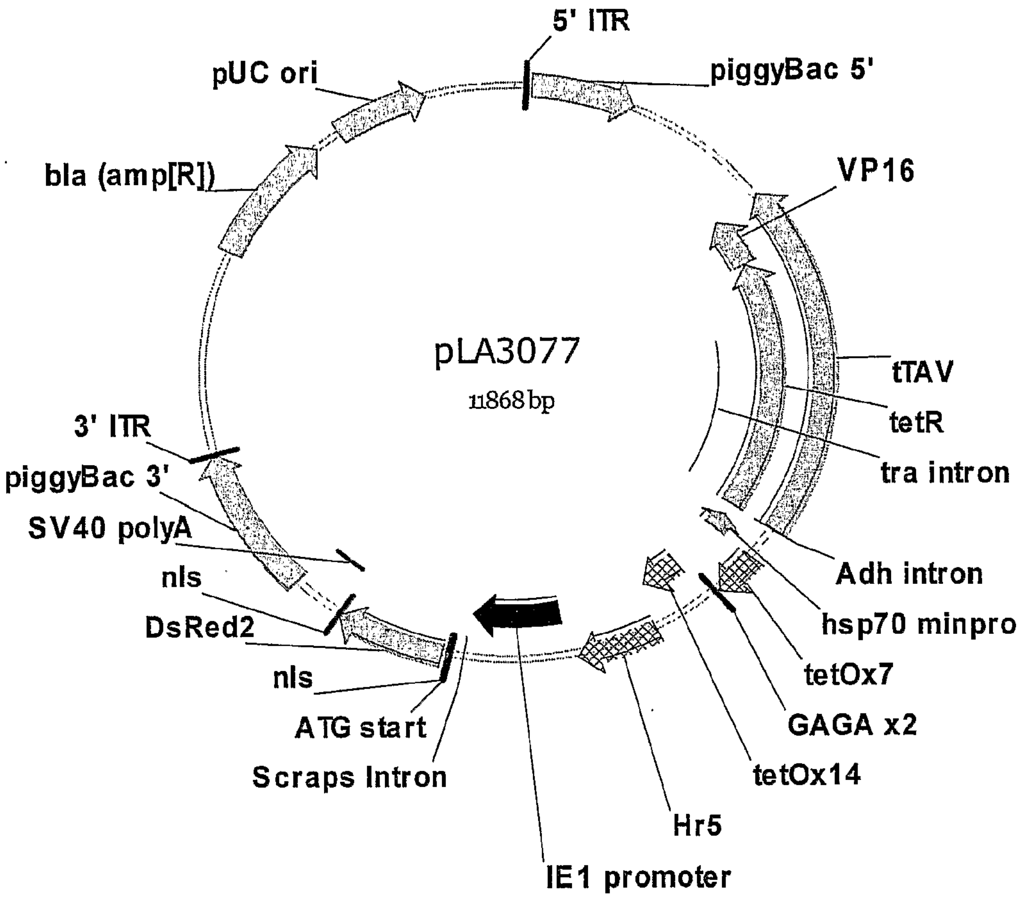
5' ITR
piggyBac 5'
pUC ori
bla (amp[R])
VP16
pLA3077
11868 bp
tTAV
tetR
tra intron
3' ITR
piggyBac 3'
SV40 polyA
Adh intron
nls
hsp70 minpro
DsRed2
tetOx7
nls
GAGA x2
ATG start
Scraps Intron
tetOx14
Hr5
IE1 promoter

Figure 26

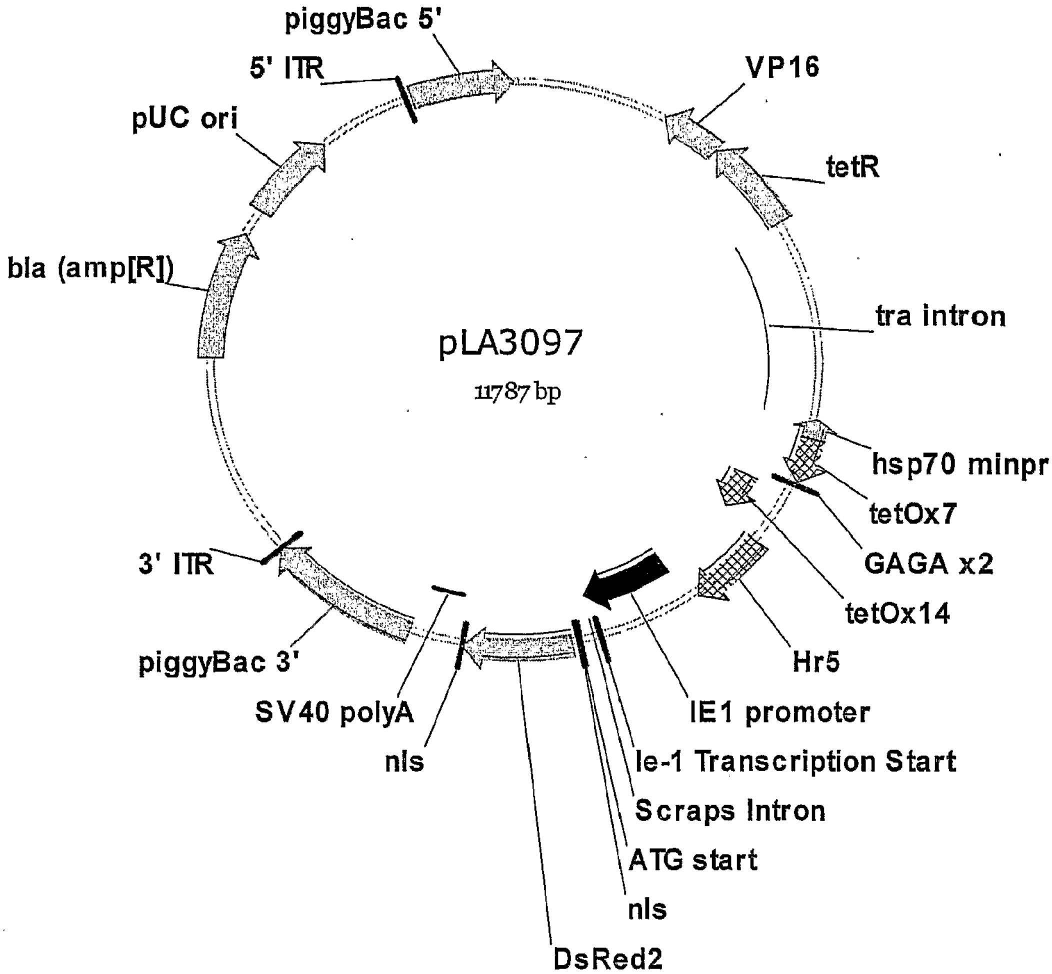
piggyBac 5'
5' ITR
pUC ori
bla (amp[R])
VP16
tetR
tra intron
pLA3097
11787 bp
hsp70 minpr
tetOx7
GAGA x2
tetOx14
Hr5
IE1 promoter
Ie-1 Transcription Start
Scraps Intron
ATG start
nls
DsRed2
nls
SV40 polyA
piggyBac 3'
3' ITR

Figure 27

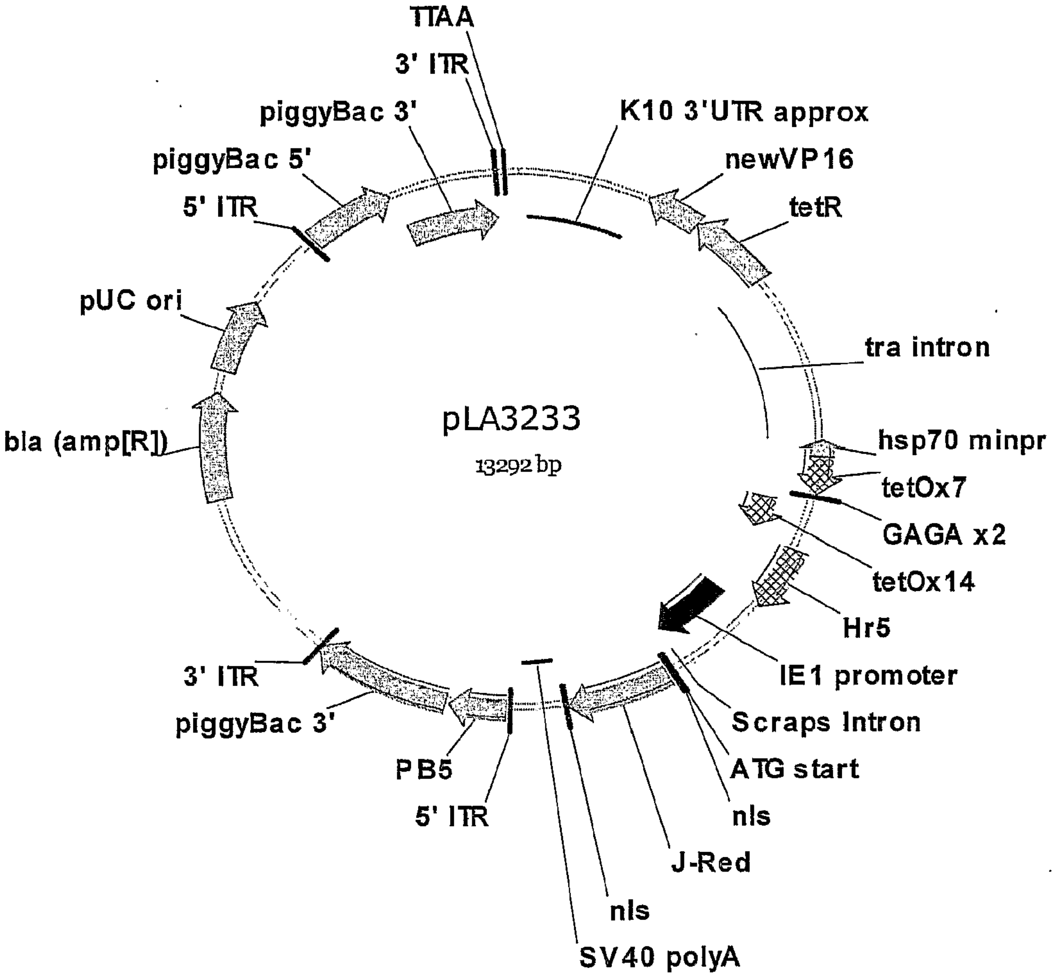
TTAA
3' ITR
piggyBac 3'
K10 3'UTR approx
piggyBac 5'
newVP16
5' ITR
tetR
pUC ori
tra intron
pLA3233
13292 bp
bla (amp[R])
hsp70 minpr
tetOx7
GAGA x2
tetOx14
Hr5
IE1 promoter
3' ITR
Scraps Intron
piggyBac 3'
ATG start
PB5
nls
5' ITR
J-Red
nls
SV40 polyA

Figure 28.

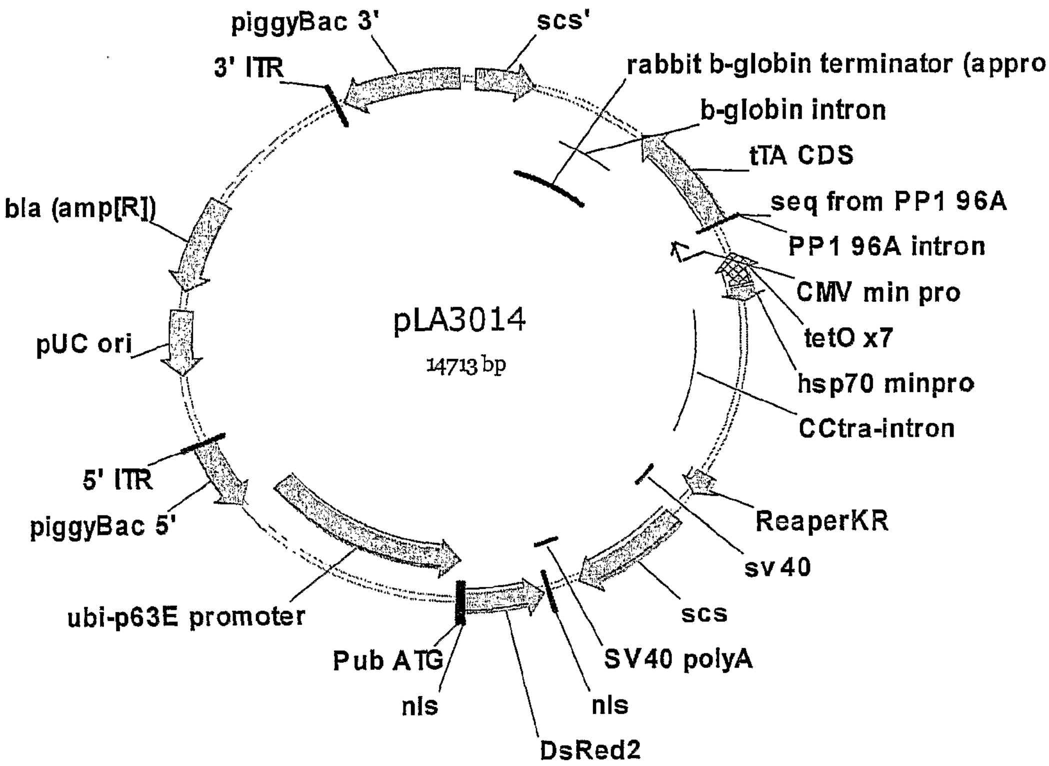
piggyBac 3'
scs'
3' ITR
rabbit b-globin terminator (appro
b-globin intron
tTA CDS
seq from PP1 96A
bla (amp[R])
PP1 96A intron
CMV min pro
pLA3014
14713 bp
pUC ori
tetO x7
hsp70 minpro
CCtra-intron
5' ITR
ReaperKR
piggyBac 5'
sv40
ubi-p63E promoter
scs
Pub ATG
SV40 polyA
nls
nls
DsRed2

Figure 29

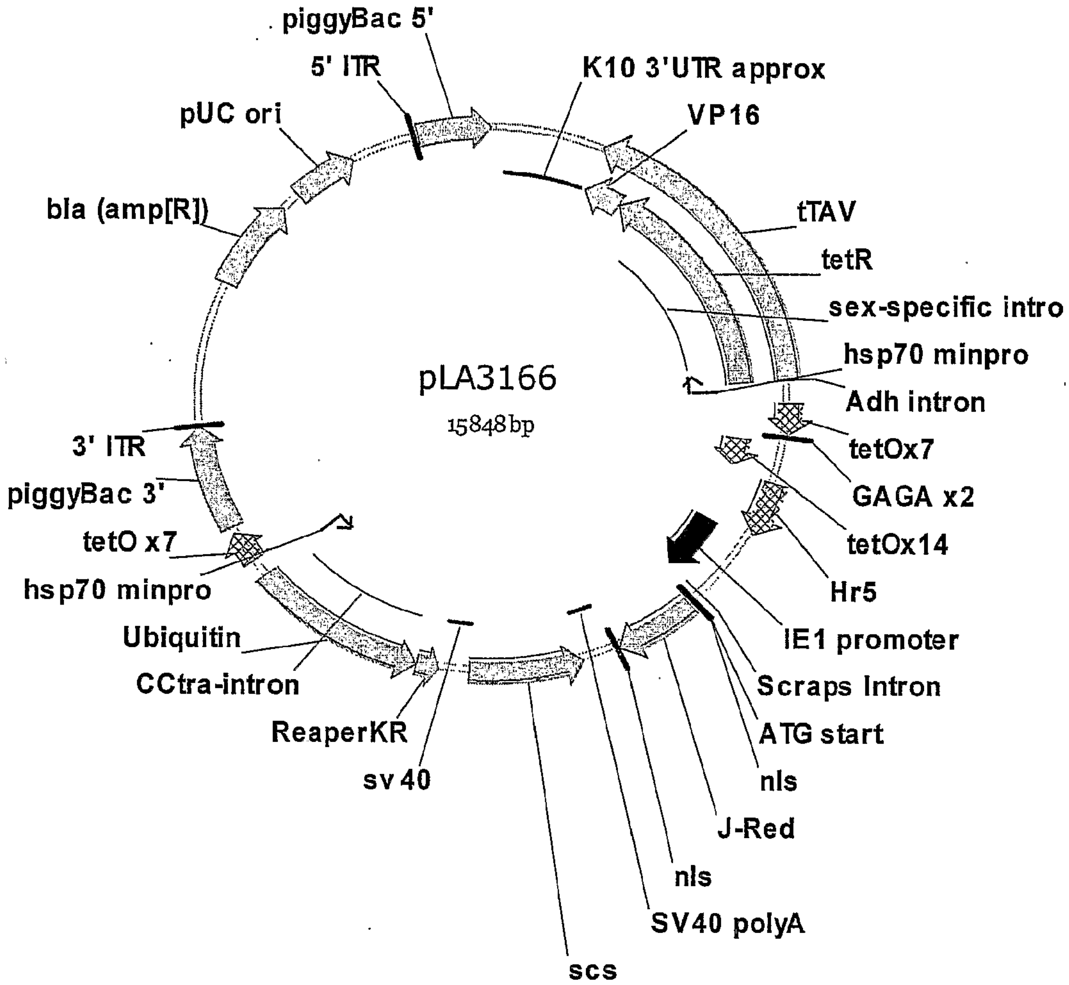
piggyBac 5'
5' ITR
pUC ori
bla (amp[R])
K10 3'UTR approx
VP16
tTAV
tetR
sex-specific intro
hsp70 minpro
Adh intron
tetOx7
GAGA x2
tetOx14
Hr5
IE1 promoter
Scraps Intron
ATG start
nls
J-Red
nls
SV40 polyA
scs
sv40
ReaperKR
CCtra-intron
Ubiquitin
hsp70 minpro
tetO x7
piggyBac 3'
3' ITR
pLA3166
15848bp

Figure 30

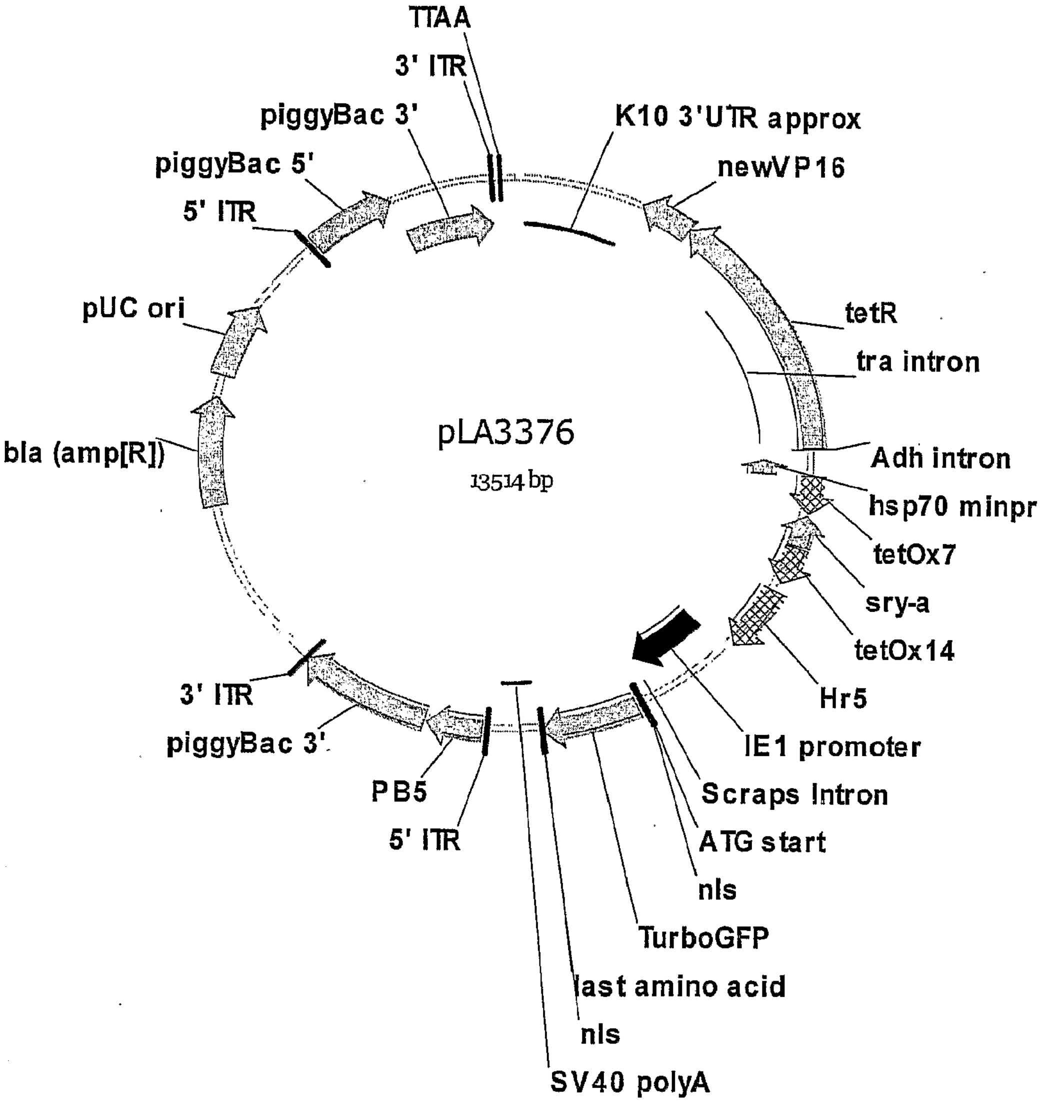
TTAA
3' ITR
piggyBac 3'
K10 3'UTR approx
piggyBac 5'
newVP16
5' ITR
pUC ori
tetR
tra intron
pLA3376
13514 bp
bla (amp[R])
Adh intron
hsp70 minpr
tetOx7
sry-a
tetOx14
Hr5
3' ITR
IE1 promoter
piggyBac 3'
Scraps Intron
PB5
ATG start
5' ITR
nls
TurboGFP
last amino acid
nls
SV40 polyA

Figure 31

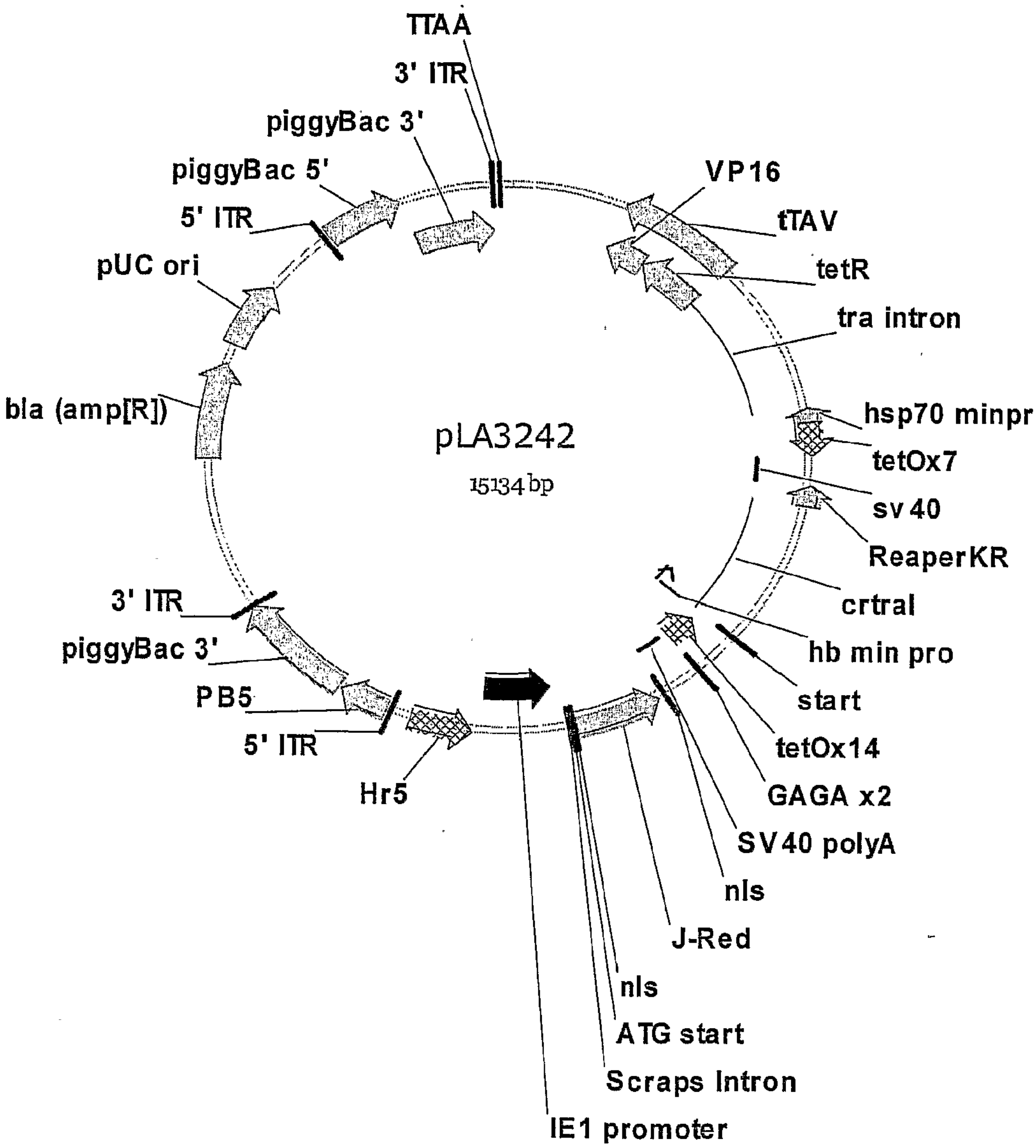
TTAA
3' ITR
piggyBac 3'
piggyBac 5'
5' ITR
pUC ori
bla (amp[R])
VP16
tTAV
tetR
tra intron
hsp70 minpr
tetOx7
sv40
ReaperKR
crtral
hb min pro
start
tetOx14
GAGA x2
SV40 polyA
nls
J-Red
nls
ATG start
Scraps Intron
IE1 promoter
Hr5
5' ITR
PB5
piggyBac 3'
3' ITR
pLA3242
15134 bp

Figure 32

Native: CGTAGATTTG¦GT...intron...AG¦GTGAAGGCTC
LA1188: CTACTG¦GCACGT...intron...AG¦GTGAAGAATA
LA3077: AACGAAGTTG¦GT...intron...AG¦GTATTGAGGG
LA3097: AGCCACCATG¦GT...intron...AG¦GTCAGCCGCC

Figure 33

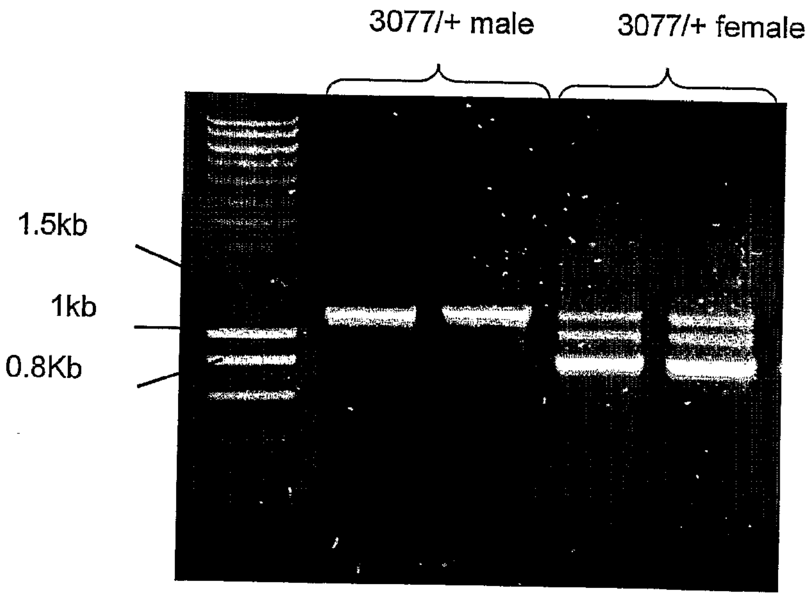


Figure 34

| [illegible] | NT Males | NT Females | TET Males | TET Females |
|---|---|---|---|---|
| 3077A | 111 | 32 | 73 | 44 |
| 3077B | 314 | 157 | 132 | 121 |
| 3077C | 161 | 116 | 60 | 84 |
| 3077D | 445 | 85 | 194 | 190 |
| | | | | |
| 3097A | 179 | 5 | 89 | 90 |
| 3097B | 440 | 0 | 59 | 27 |
| 3097C | 172 | 0 | 46 | 44 |
| | | | | |
| 3233A | 457 | 1 | 79 | 58 |
| 3233B | 171 | 0 | 14 | 13 |
| | | | | |
| 3014;1217 | 136 | 0 | 48 | 10 |
| 3166;1217 | 64 | 0 | 5 | 7 |

Figure 35

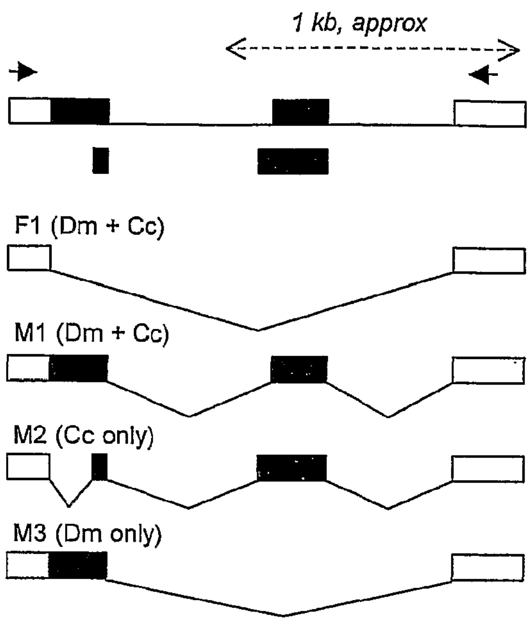


Figure 36

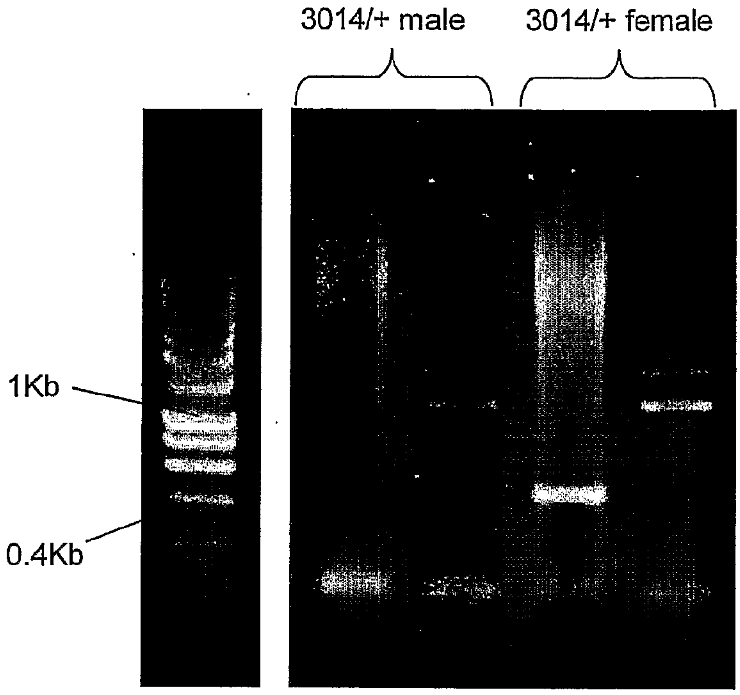


Figure 37

| | NT males | NTfemales | TET males | TET females |
|---|---|---|---|---|
| 3097A | 136 | 0 | 21 | 19 |
| 3097B | 295 | 11 | 14 | 11 |
| 3097C | 96 | 12 | 22 | 21 |
| 3097D | 103 | 15 | 82 | 67 |
| 3233A | 78 | 6 | 32 | 5 |

Figure 38

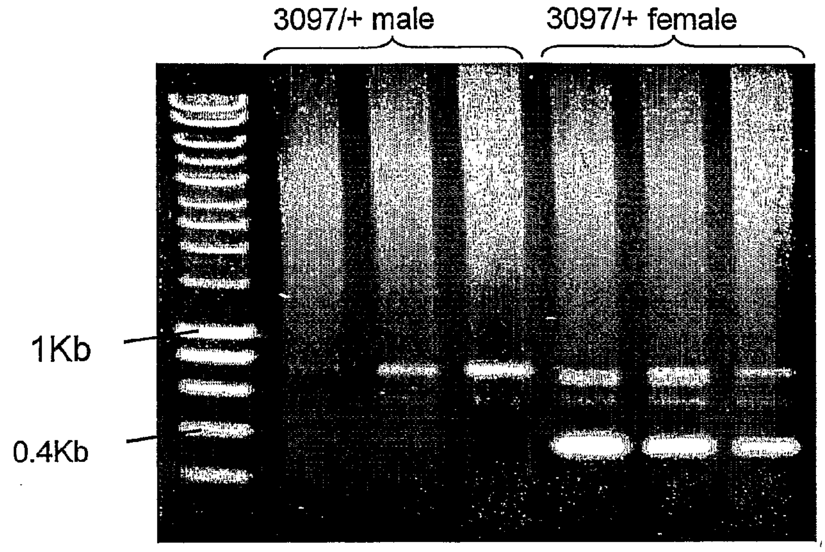


Figure 39

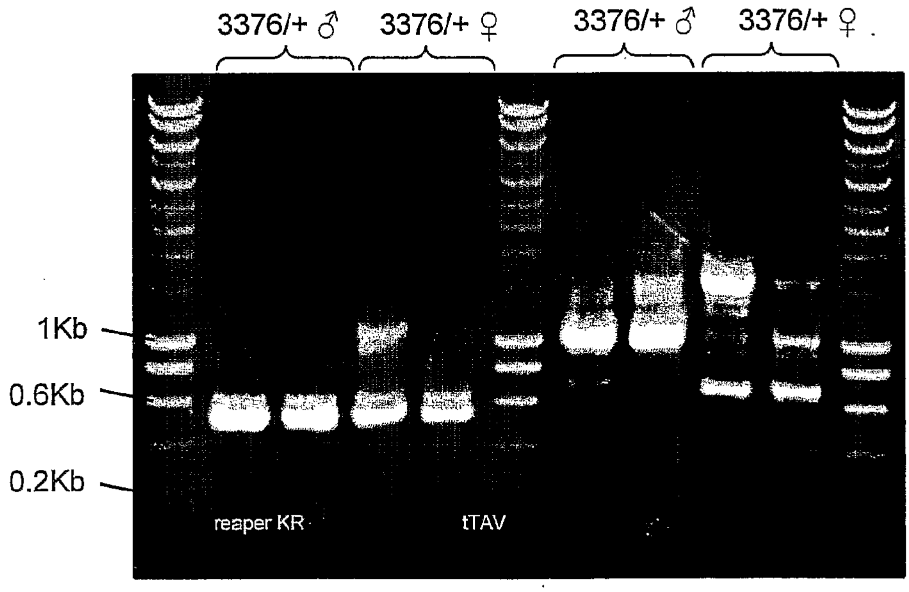


Figure 40

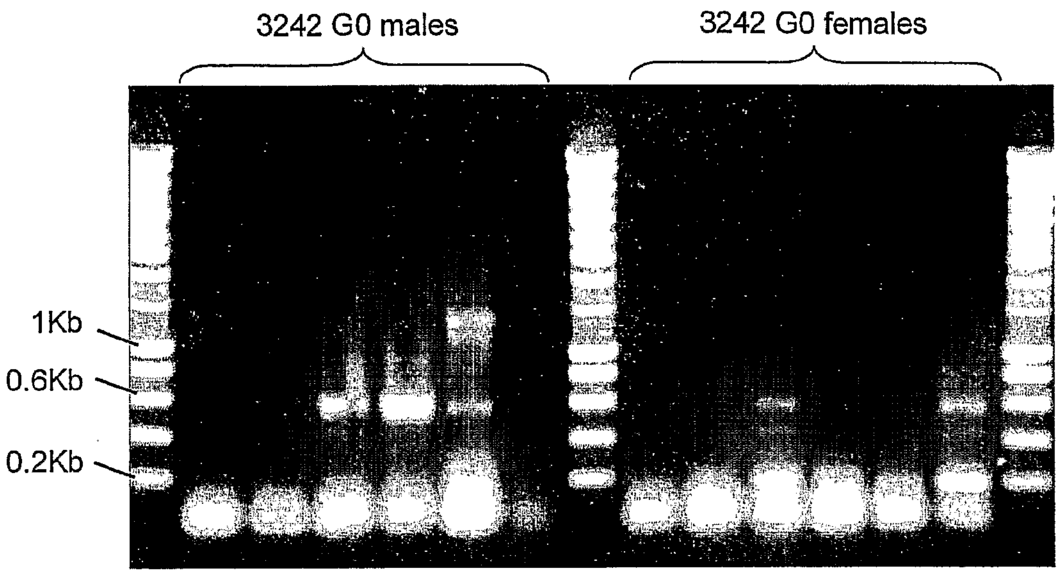


Figure 41

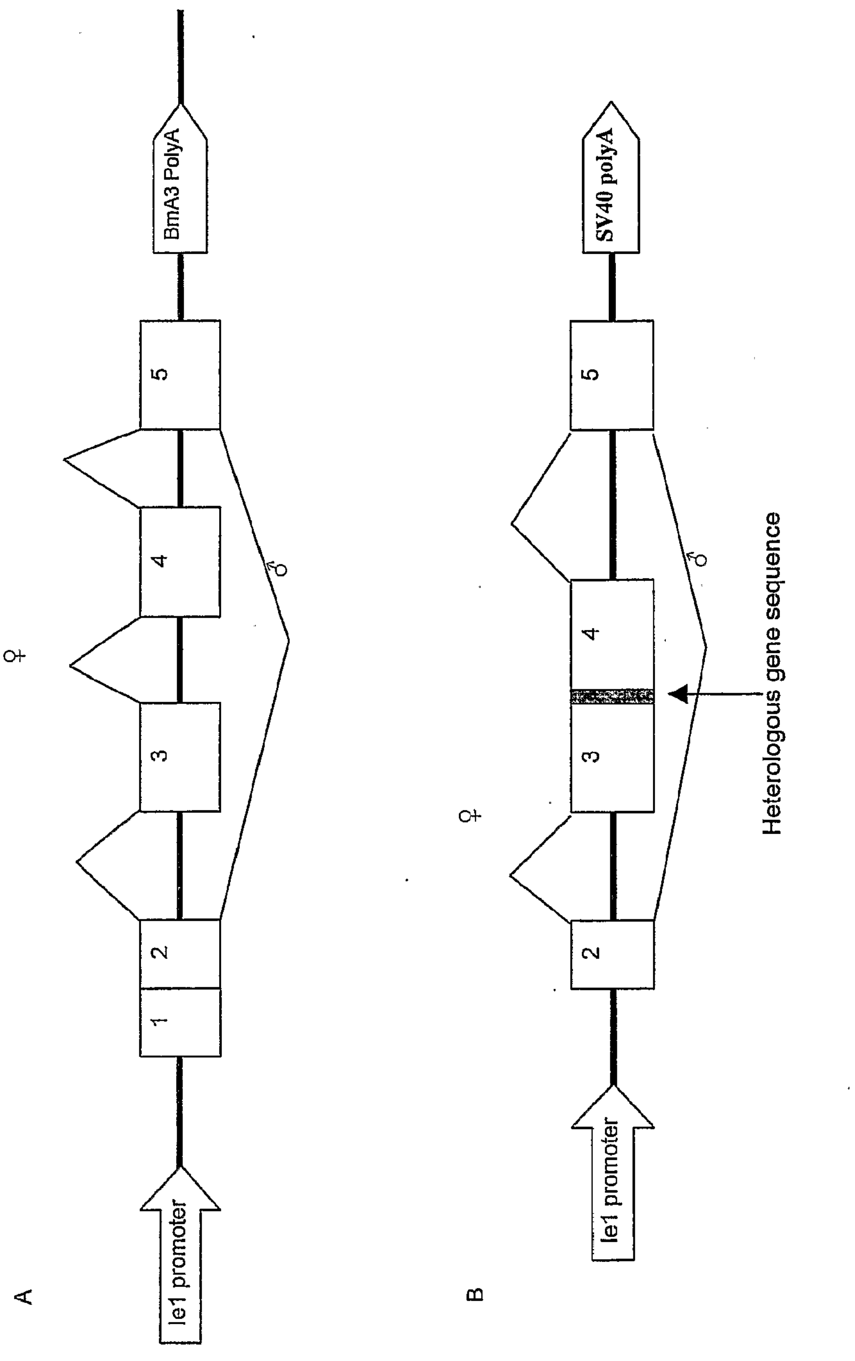
A
le1 promoter
1
2
3
4
5
BmA3 PolyA
♀
♂
B
le1 promoter
2
3
4
5
SV40 polyA
♀
♂
Heterologous gene sequence

Figure 42

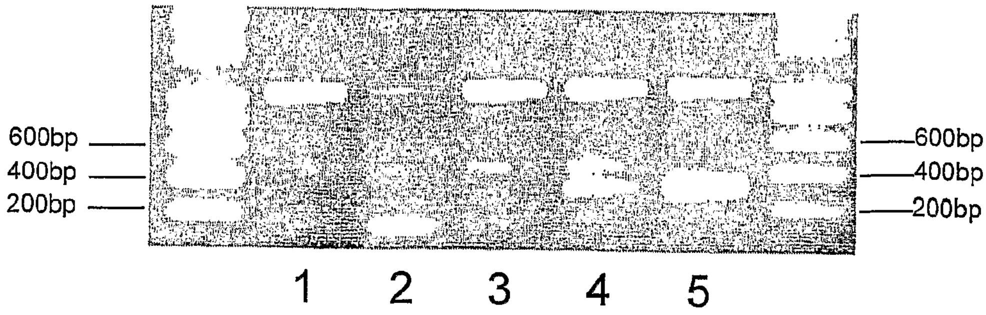


Figure 43

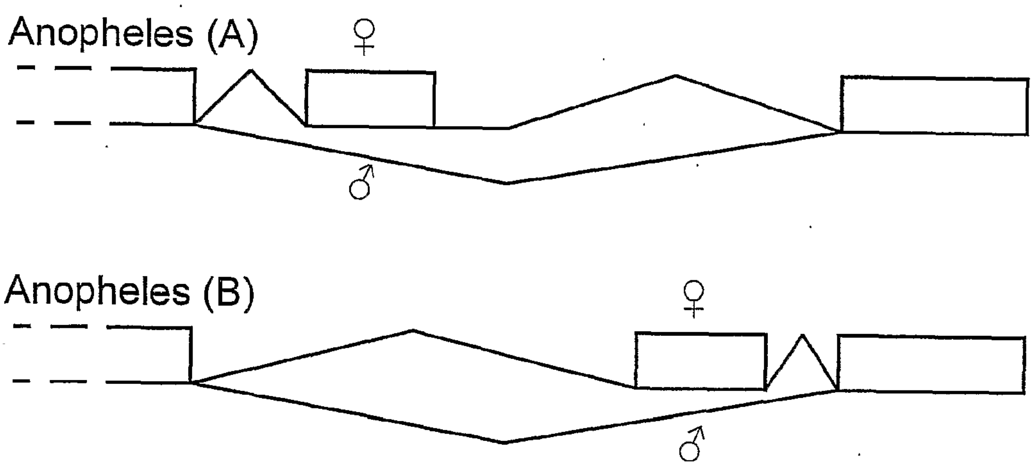


Figure 44

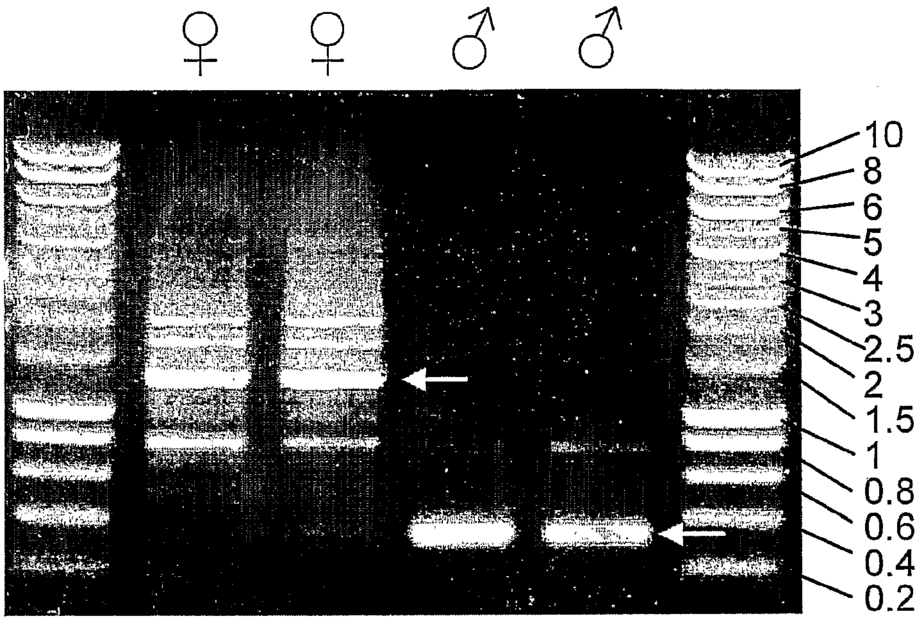


Figure 45

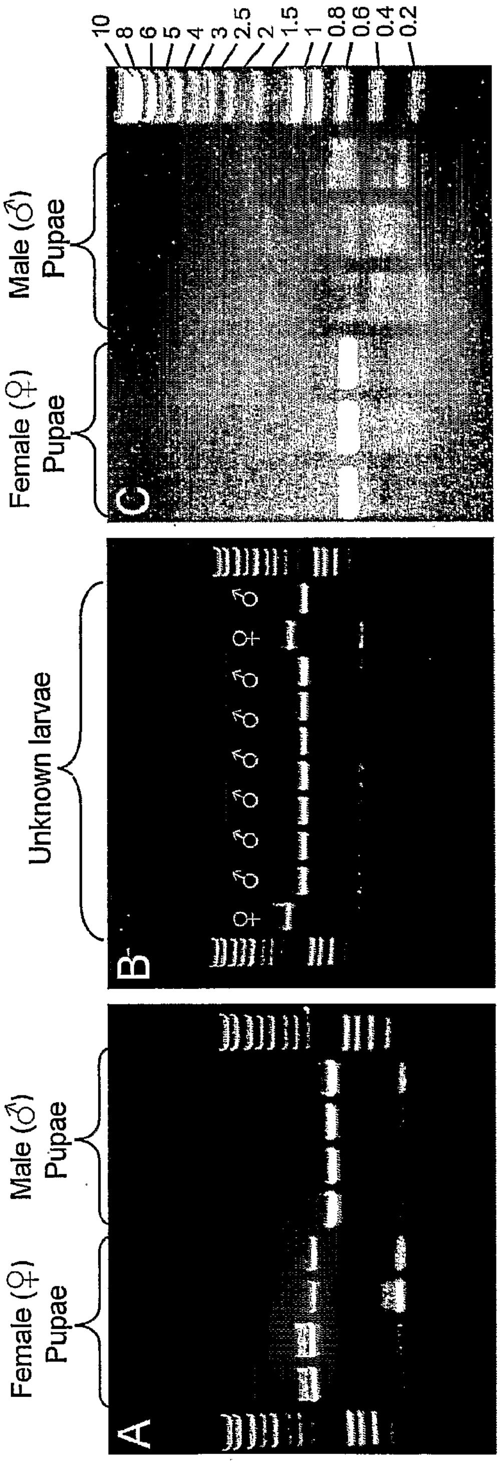
A
Female (♀) Pupae
Male (♂) Pupae
B
Unknown larvae
♀ ♂ ♂ ♂ ♂ ♂ ♂ ♂ ♀ ♂
C
Female (♀) Pupae
Male (♂) Pupae
10
8
6
5
4
3
2.5
2
1.5
1
0.8
0.6
0.4
0.2

Figure 46

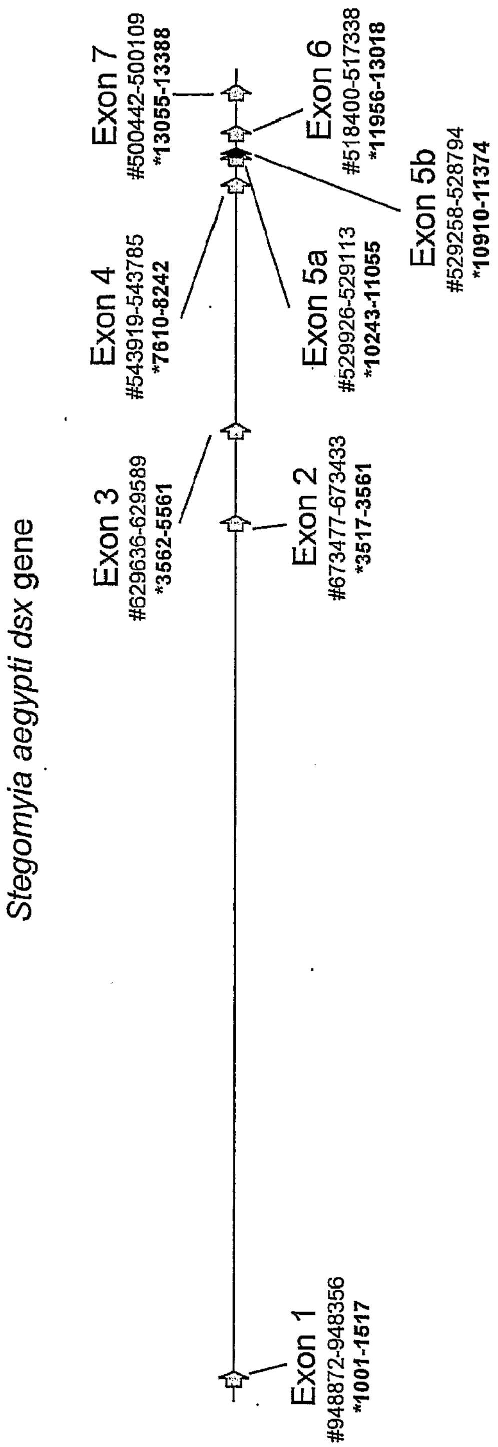
*Stegomyia aegypti dsx* gene
Exon 1
#948872-948356
*1001-1517
Exon 2
#673477-673433
*3517-3561
Exon 3
#629636-629589
*3562-5561
Exon 4
#543919-543785
*7610-8242
Exon 5a
#529926-529113
*10243-11055
Exon 5b
#529258-528794
*10910-11374
Exon 6
#518400-517338
*11956-13018
Exon 7
#500442-500109
*13055-13388

Figure 47

*Stegomyia aegypti dsx* gene

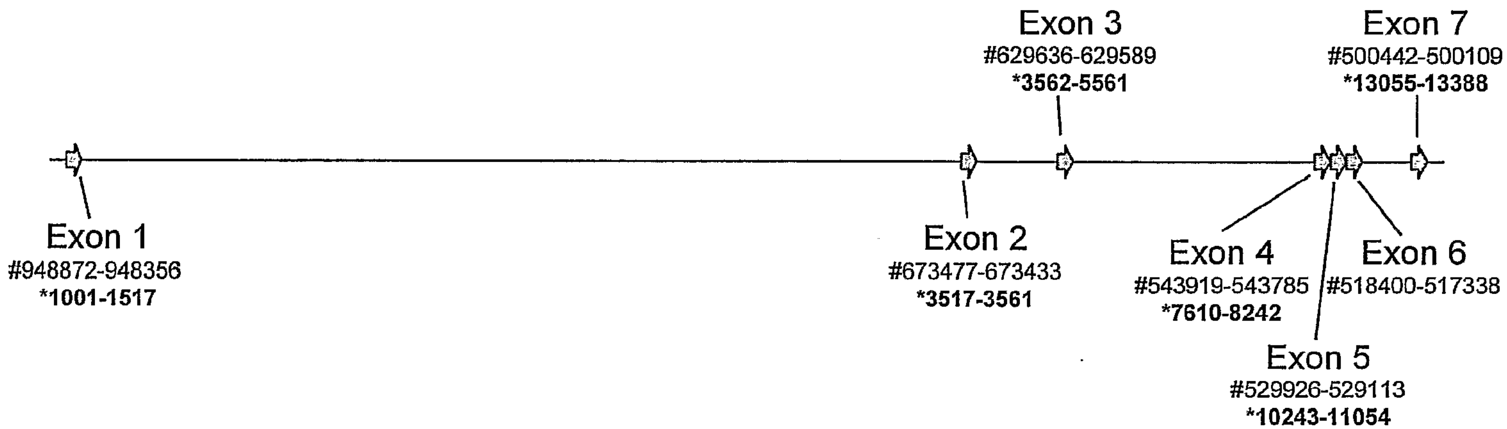


Figure 48

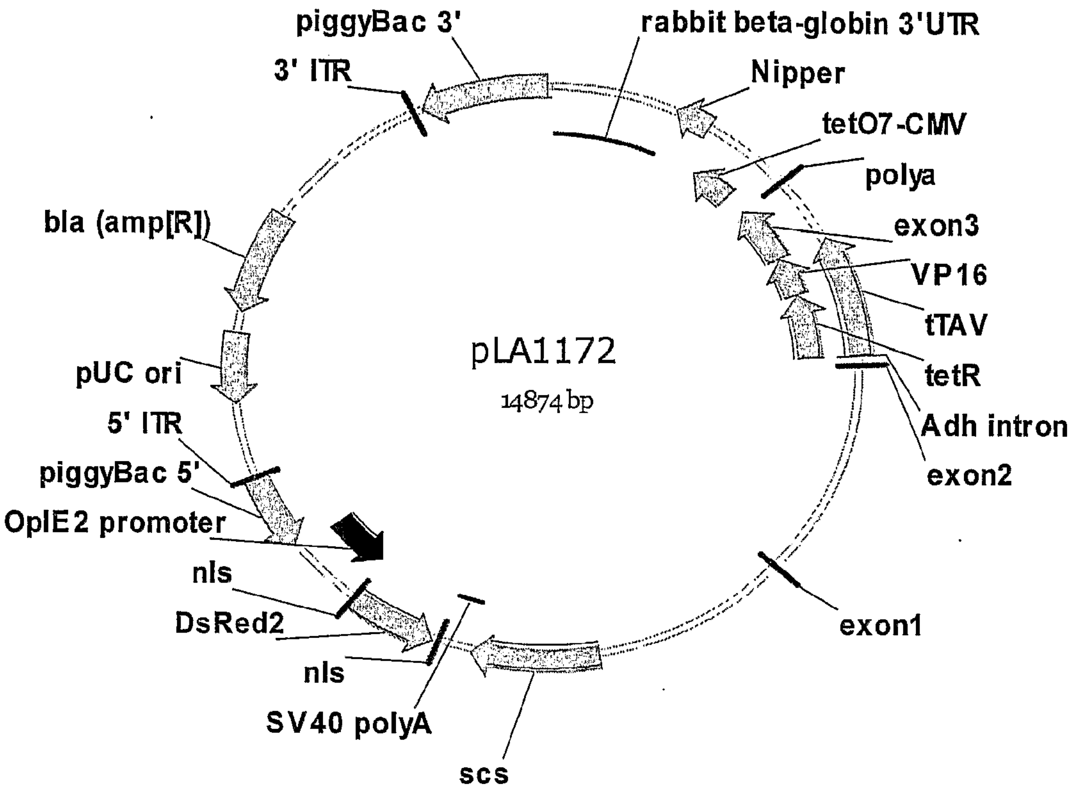
piggyBac 3'
3' ITR
rabbit beta-globin 3'UTR
Nipper
tetO7-CMV
polya
exon3
VP16
tTAV
tetR
Adh intron
exon2
bla (amp[R])
pUC ori
pLA1172
14874 bp
5' ITR
piggyBac 5'
OpIE2 promoter
nls
DsRed2
nls
SV40 polyA
scs
exon1

Figure 49

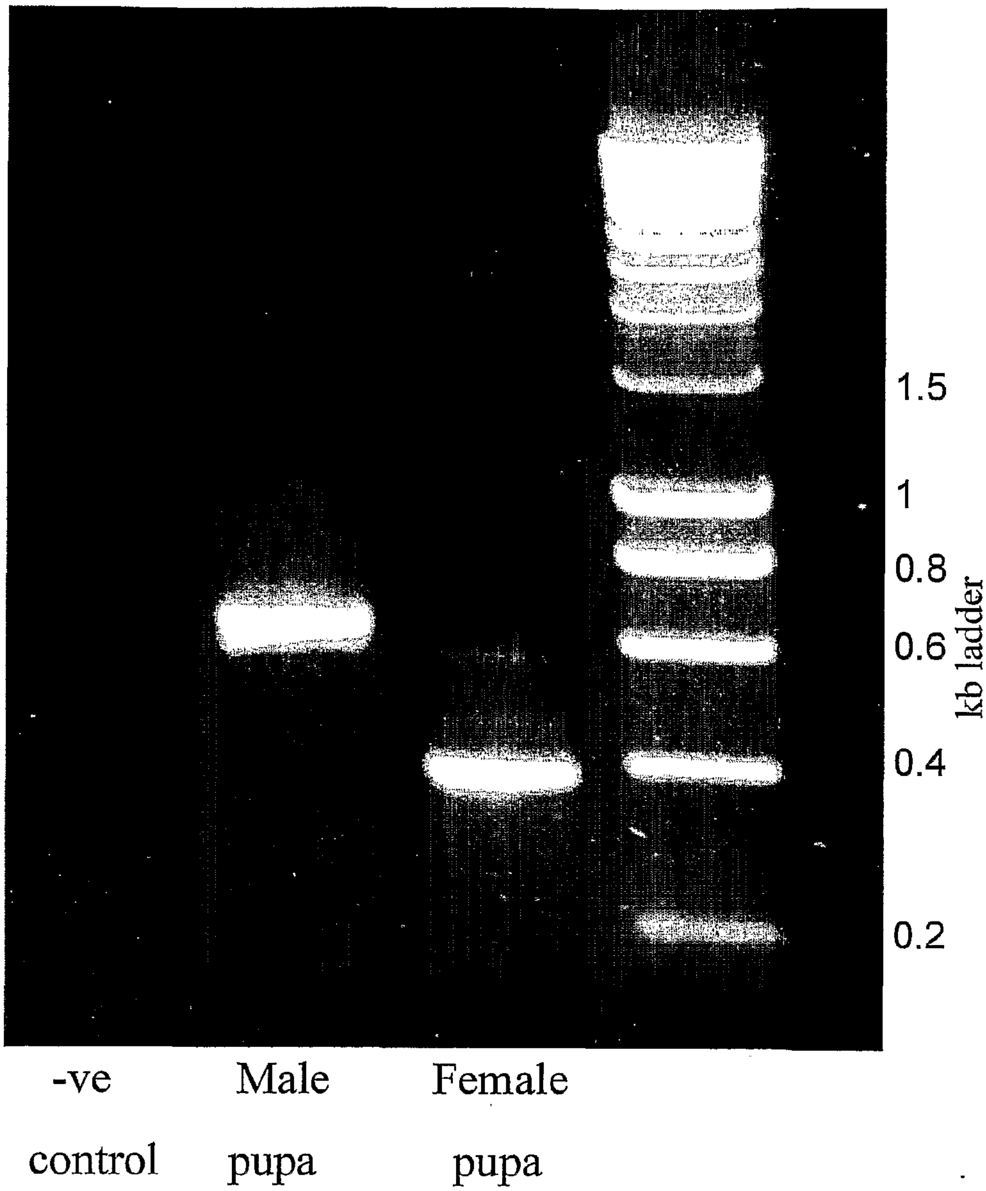


Figure 50

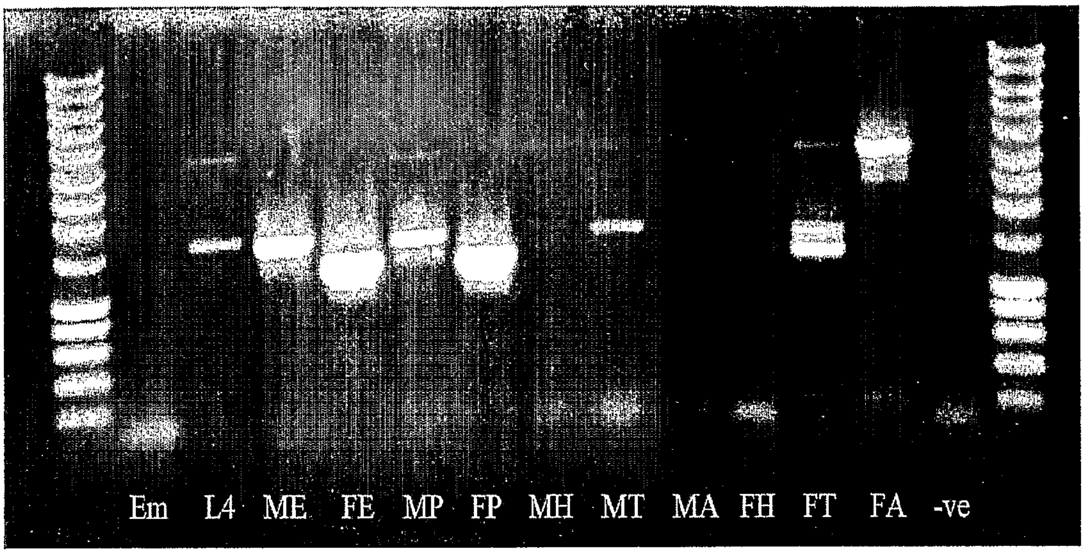


Figure 51

Figure 52- LA3515 Plasmid map

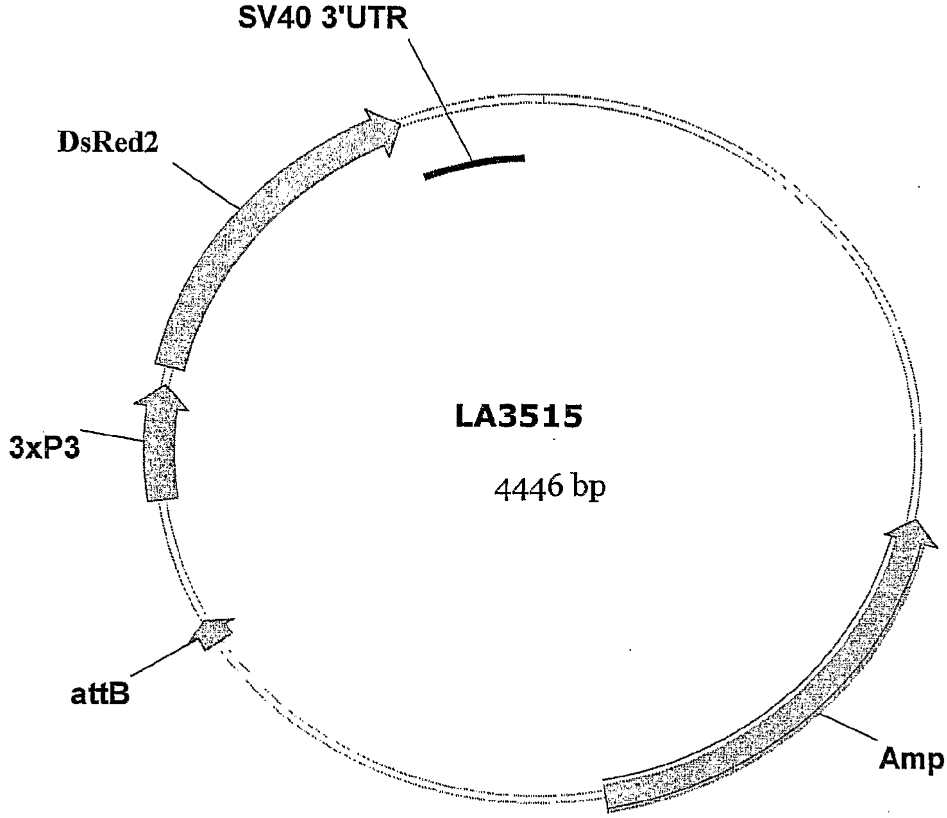

Figure 53 LA3545 Plasmid map

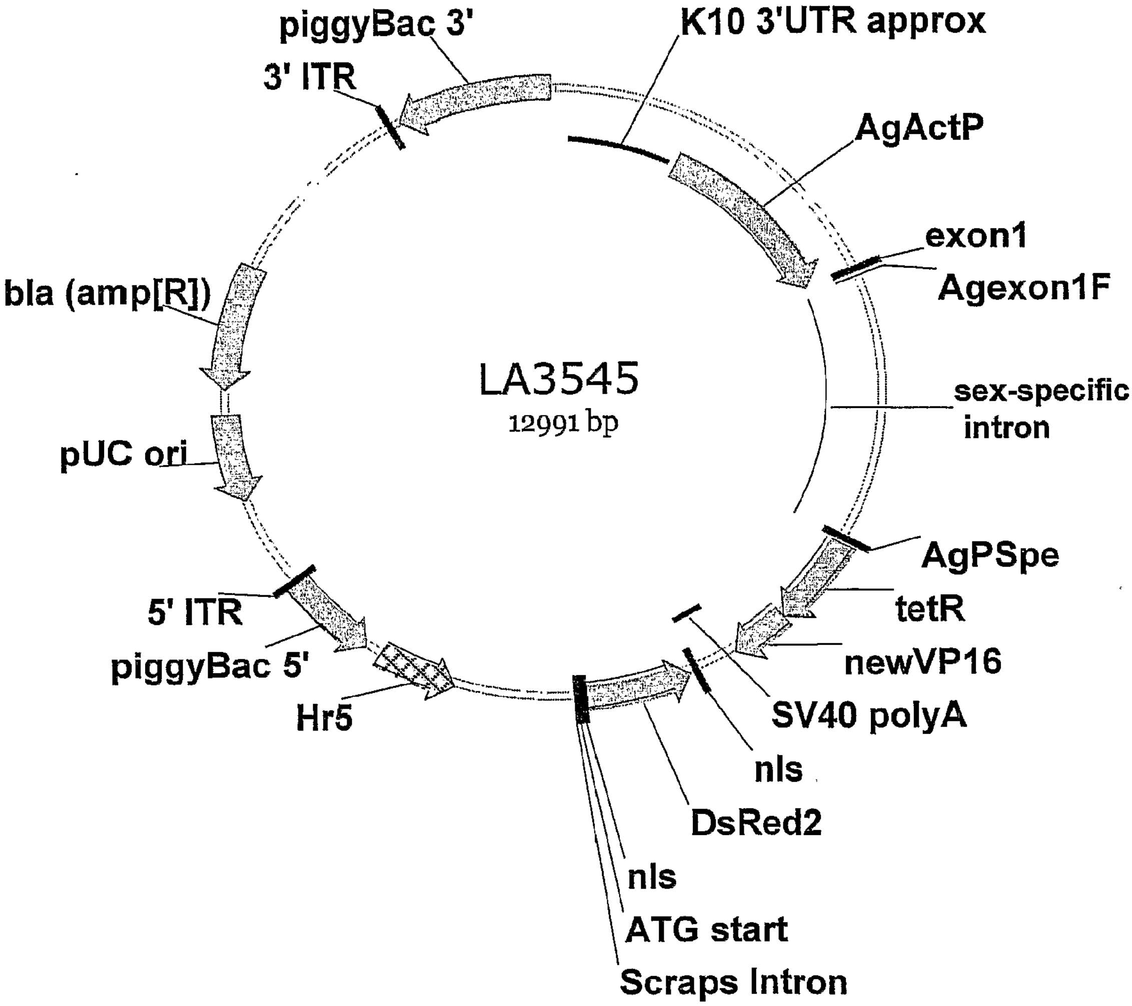

Figure 54 LA3604 Plasmid map

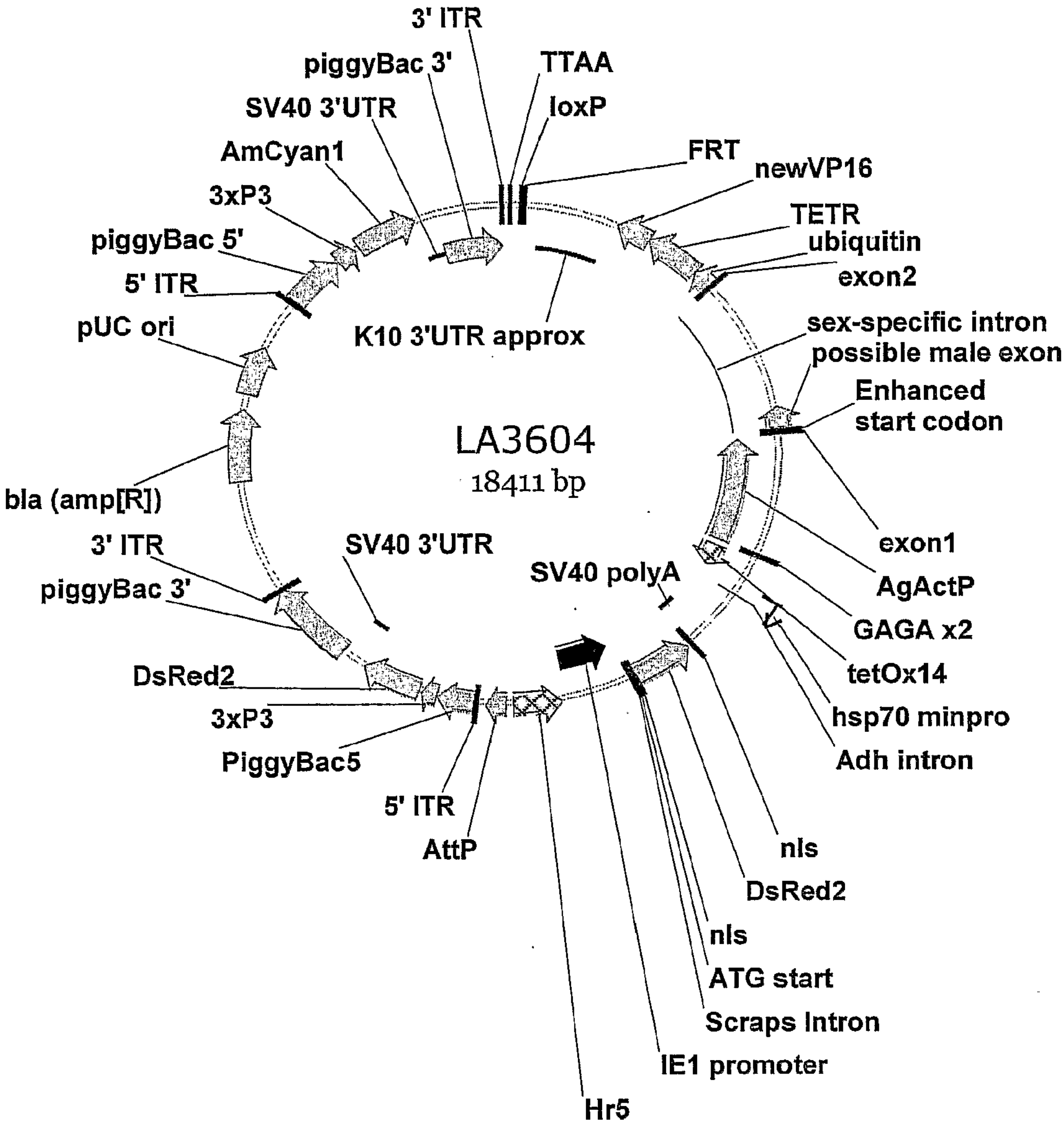

Figure 55 LA3646 Plasmid map

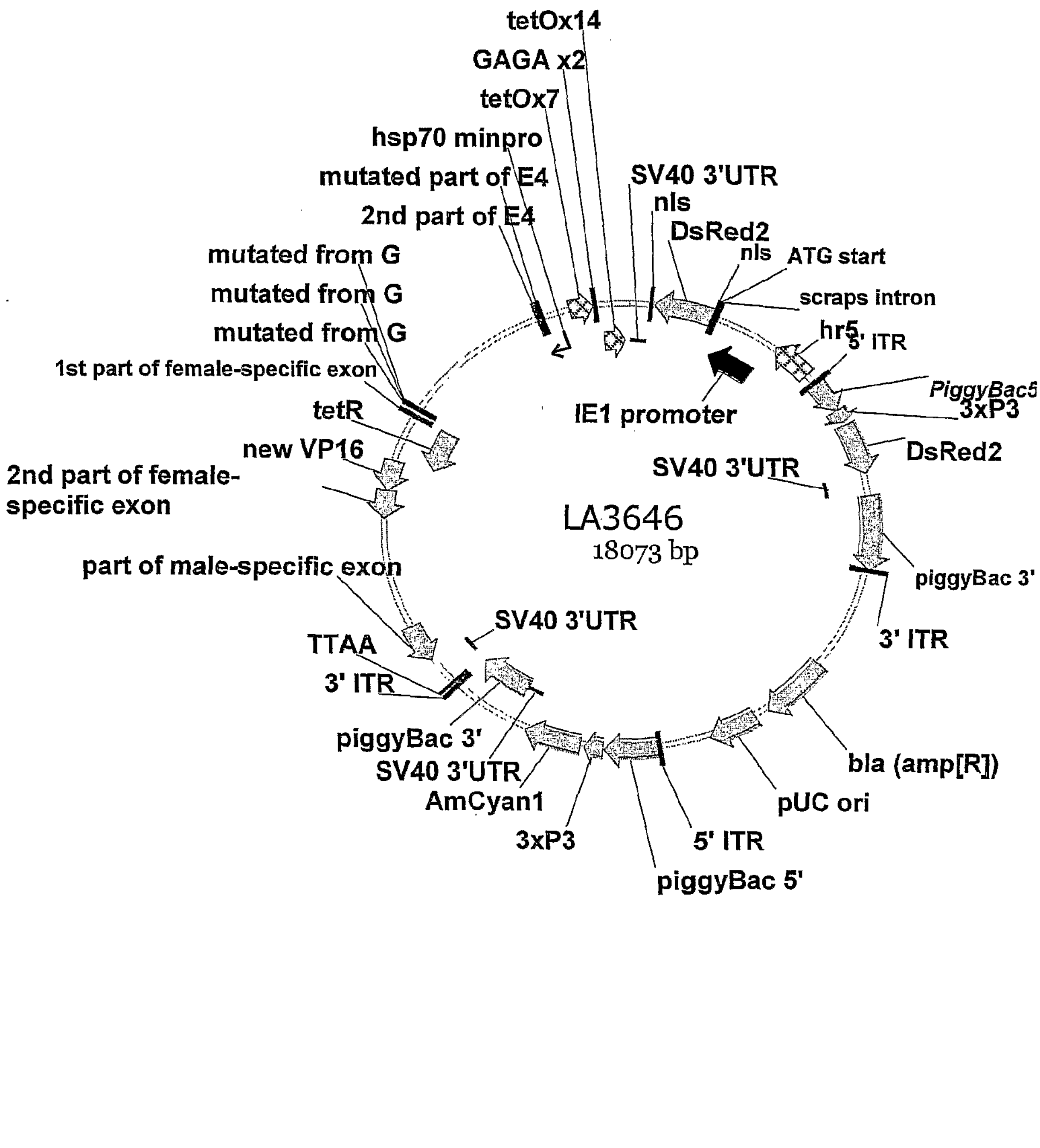

Figure 56

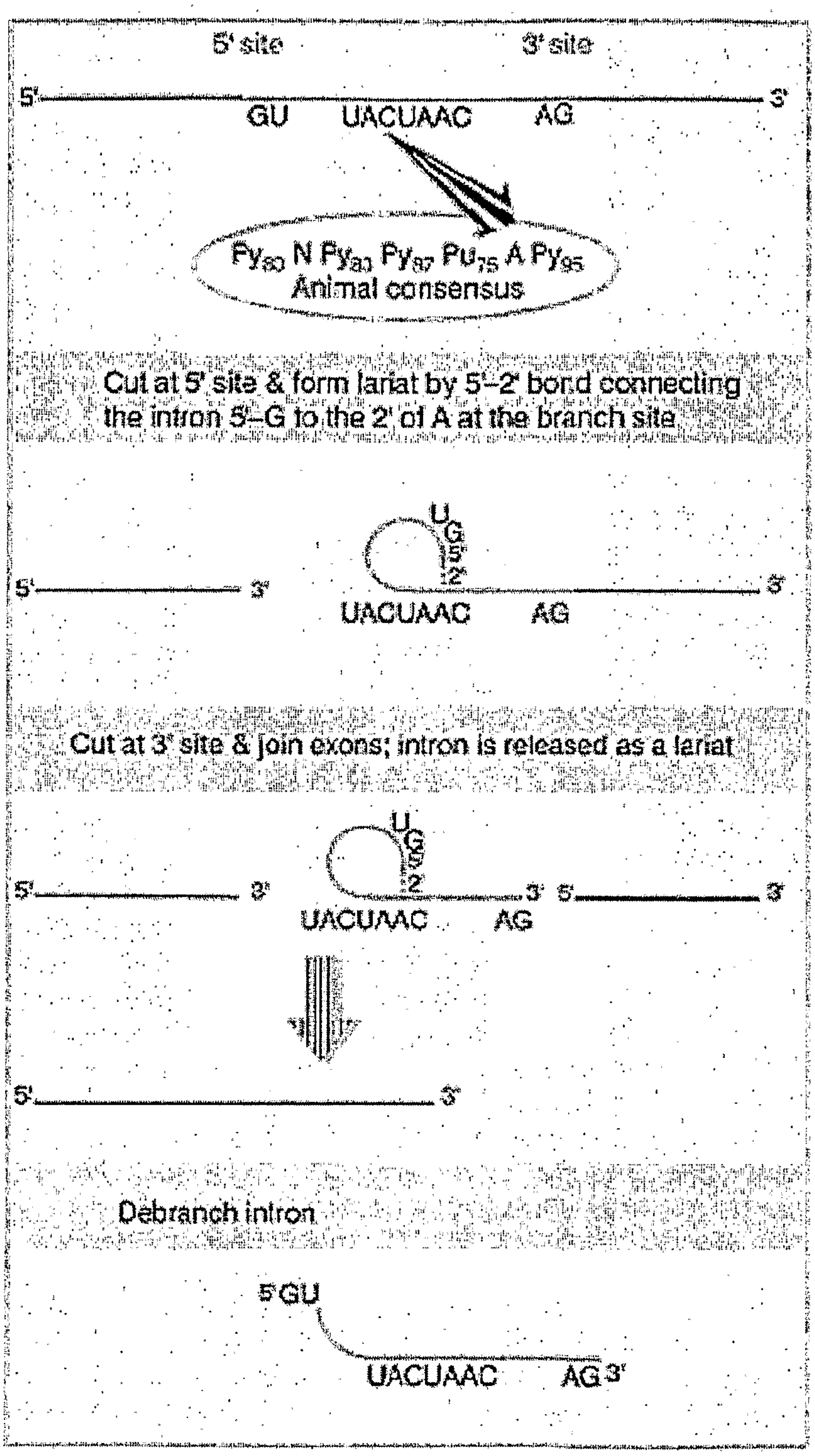
5' site
3' site
5'
3'
GU
UACUAAC
AG
Py80 N Py80 Py87 Pu75 A Py95
Animal consensus
Cut at 5' site & form lariat by 5'-2' bond connecting the intron 5'-G to the 2' of A at the branch site
U
G
5'
2'
5'
3'
UACUAAC
AG
3'
Cut at 3' site & join exons; intron is released as a lariat
UACUAAC
AG
Debranch intron
5'GU
UACUAAC
AG 3'

Figure 57

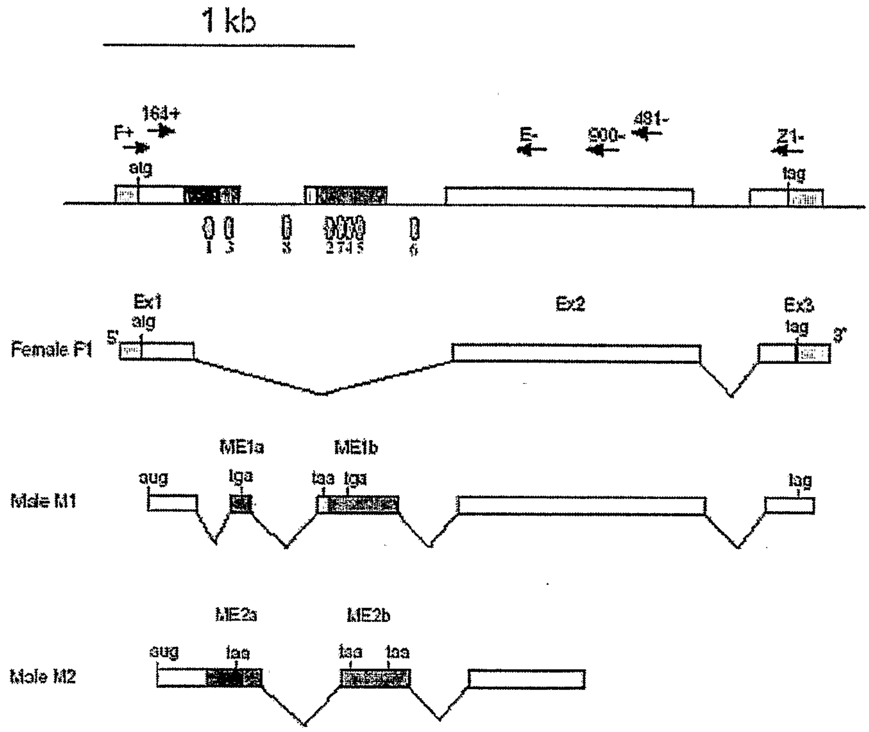
1 kb
164+
F+
atg
E-
900-
481-
Z1-
tag
1 3
8
2 7 4 5
6
Ex1
atg
5'
Female F1
Ex2
Ex3
tag
3'
ME1a
ME1b
aug
tga
taa tga
tag
Male M1
ME2a
ME2b
aug
taa
taa taa
Male M2

A

Figure 58

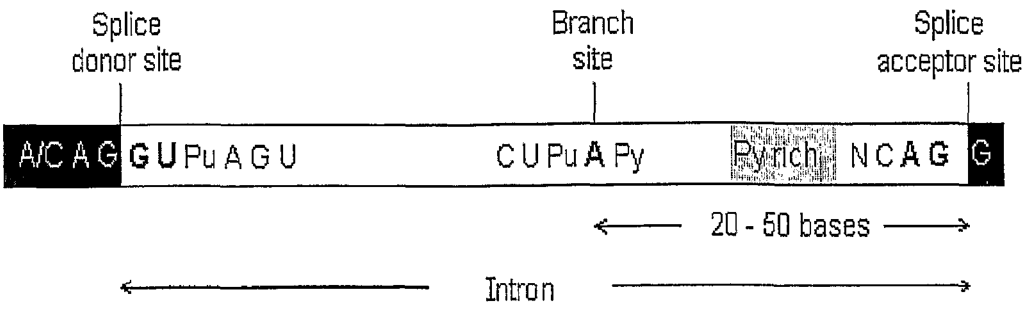


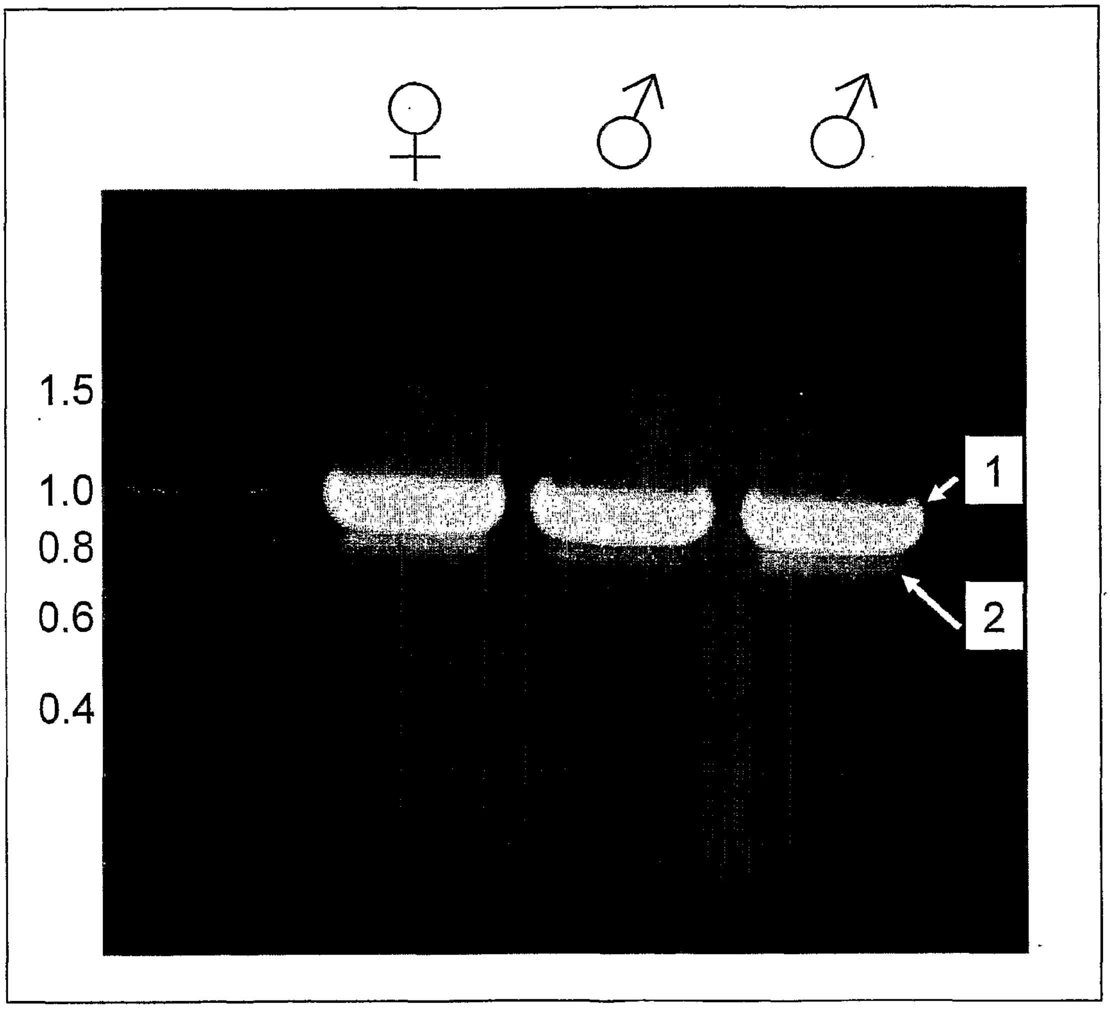


Figure 59

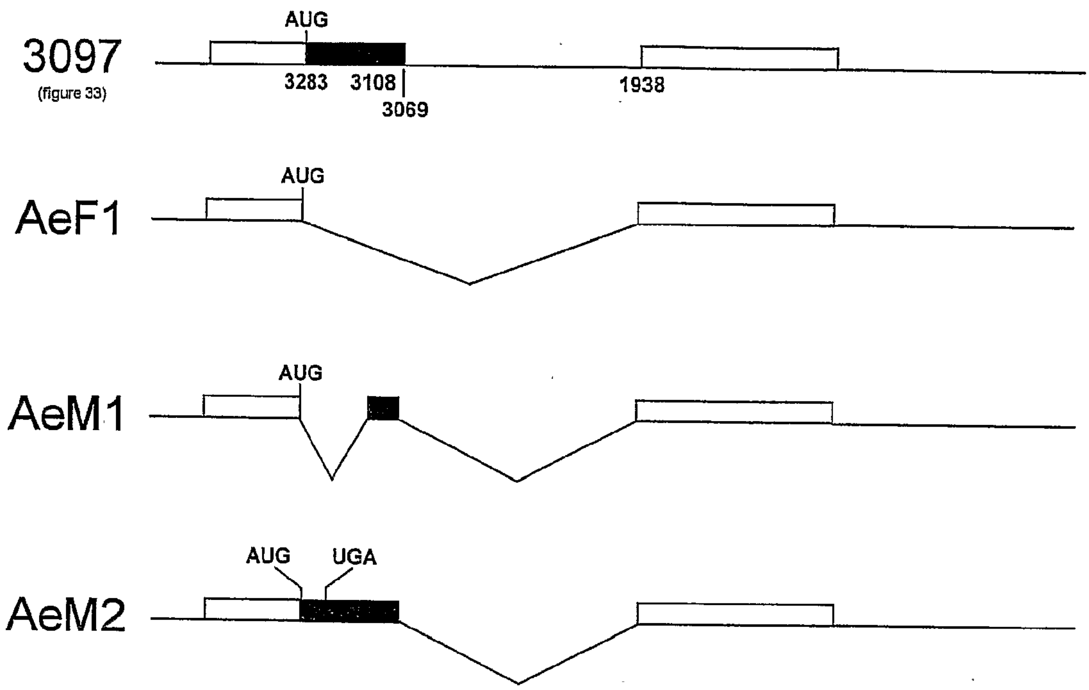
3097
(figure 33)
AUG
3283
3108
3069
1938
AeF1
AUG
AeM1
AUG
AeM2
AUG
UGA

Figure 60

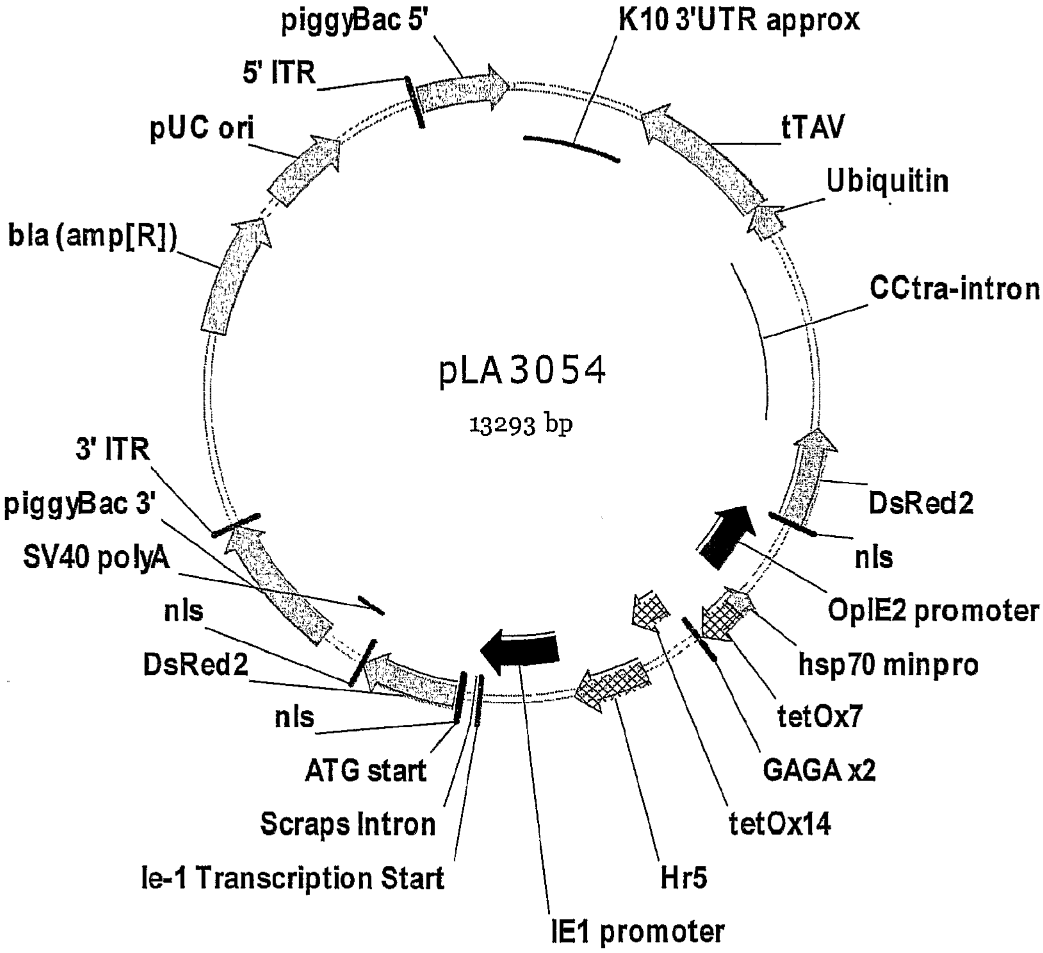
piggyBac 5'
K10 3'UTR approx
5' ITR
pUC ori
tTAV
Ubiquitin
bla (amp[R])
CCtra-intron
pLA3054
13293 bp
3' ITR
DsRed2
piggyBac 3'
nls
SV40 polyA
OpIE2 promoter
nls
hsp70 minpro
DsRed2
tetOx7
nls
GAGA x2
ATG start
Scraps Intron
tetOx14
Ie-1 Transcription Start
Hr5
IE1 promoter

Figure 61

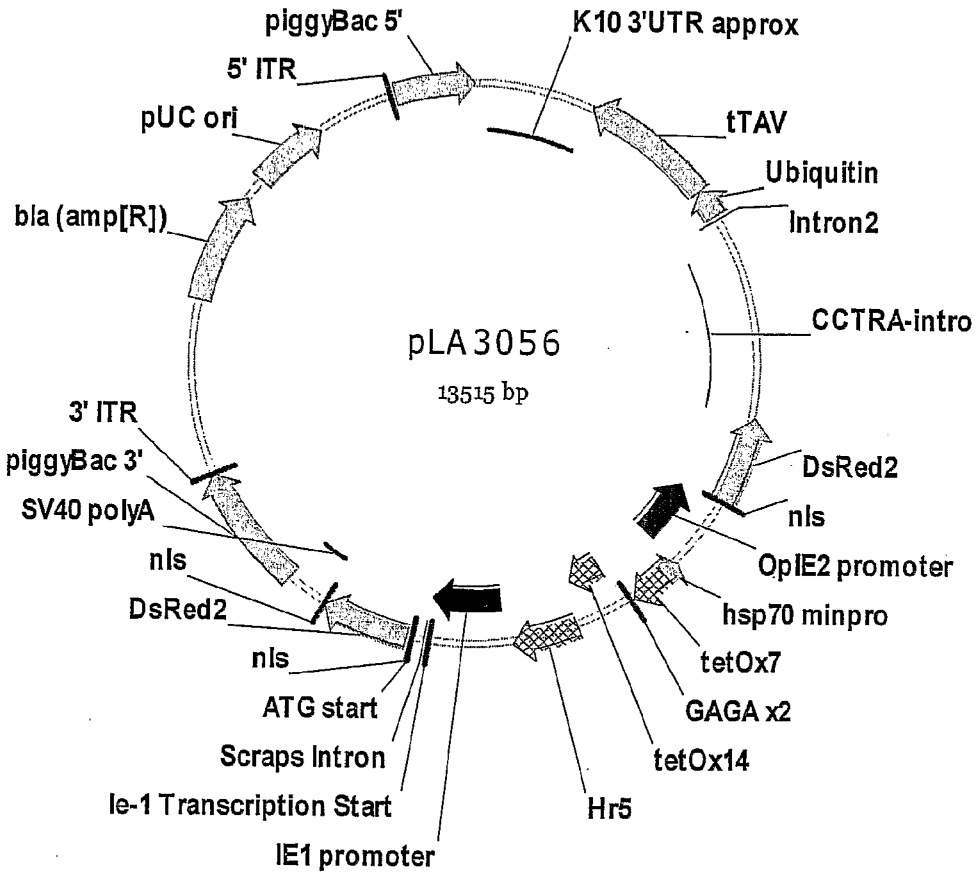
piggyBac 5'
K10 3'UTR approx
5' ITR
pUC ori
tTAV
Ubiquitin
Intron2
bla (amp[R])
pLA3056
13515 bp
CCTRA-intro
3' ITR
piggyBac 3'
DsRed2
nls
SV40 polyA
OpIE2 promoter
nls
hsp70 minpro
DsRed2
tetOx7
nls
ATG start
GAGA x2
Scraps Intron
tetOx14
Ie-1 Transcription Start
Hr5
IE1 promoter

Figure 62

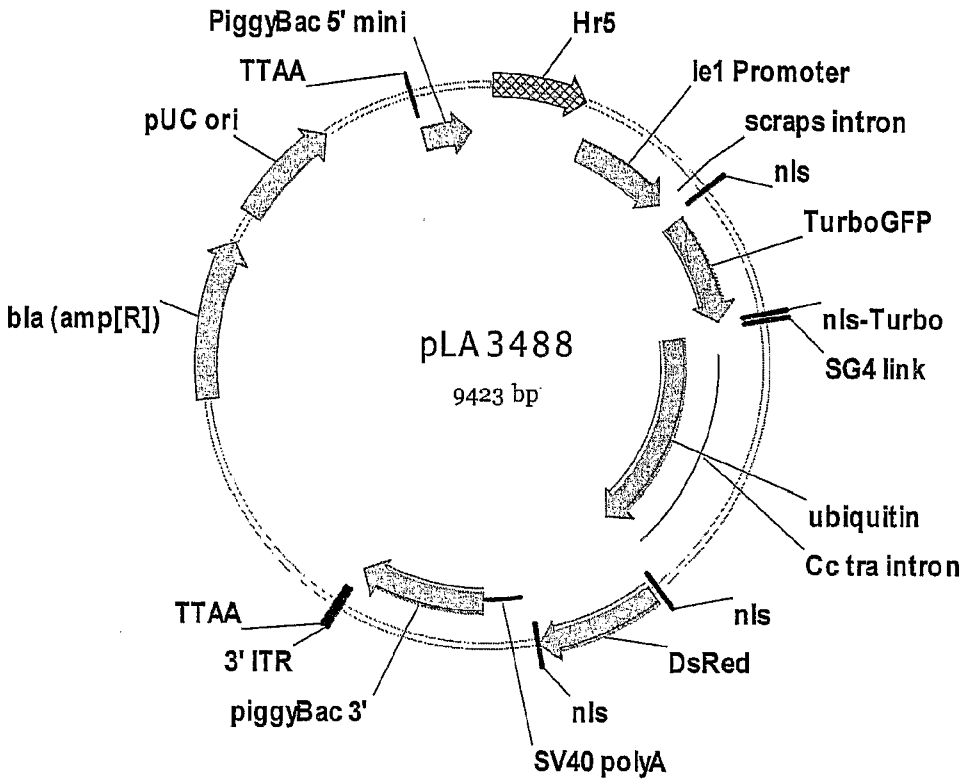
PiggyBac 5' mini
Hr5
TTAA
Ie1 Promoter
pUC ori
scraps intron
nls
TurboGFP
bla (amp[R])
pLA3488
9423 bp
nls-Turbo
SG4 link
ubiquitin
Cc tra intron
TTAA
nls
3' ITR
DsRed
piggyBac 3'
nls
SV40 polyA

Figure 63

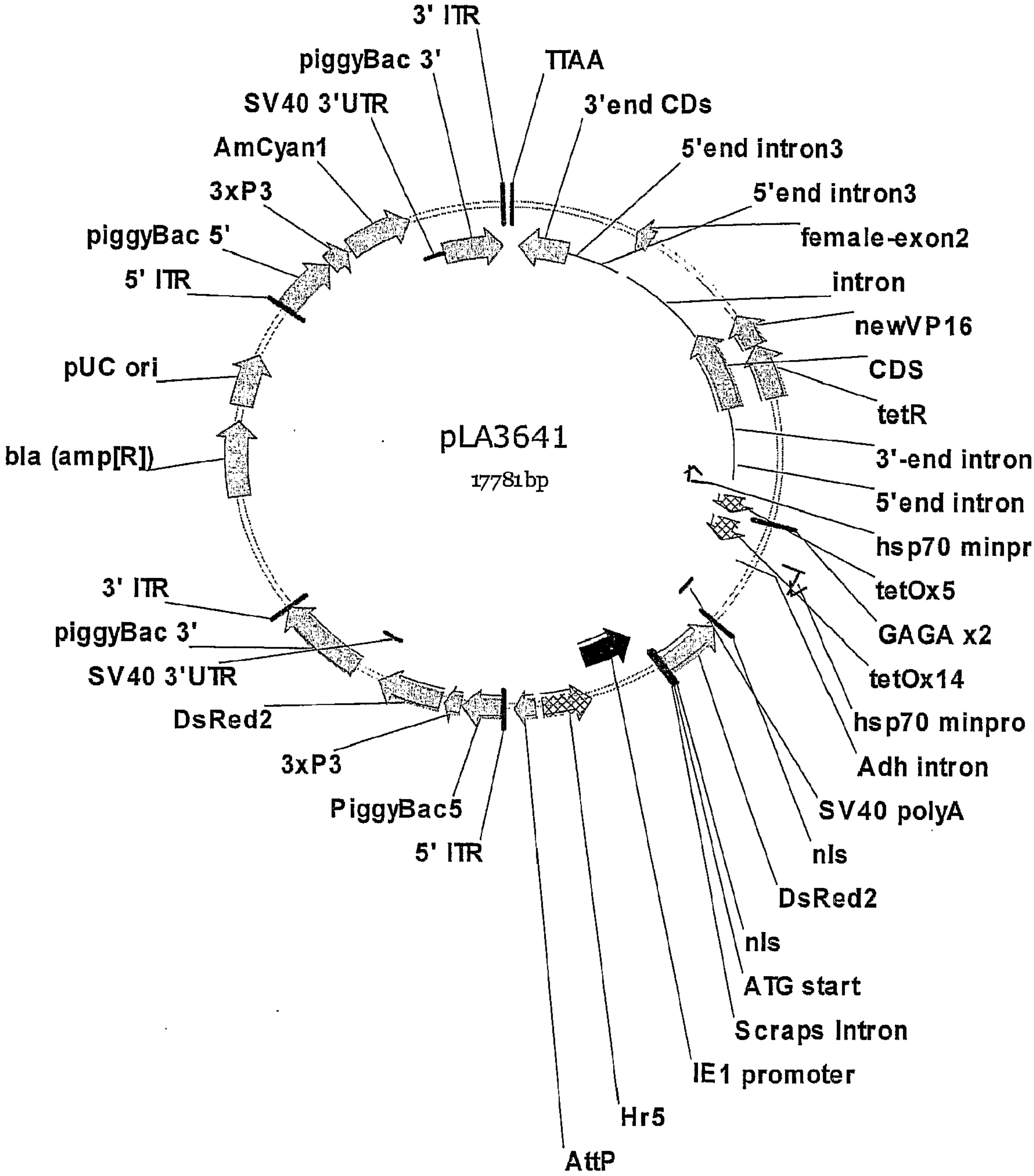
3' ITR
piggyBac 3'
TTAA
SV40 3'UTR
3'end CDs
AmCyan1
5'end intron3
3xP3
5'end intron3
piggyBac 5'
female-exon2
5' ITR
intron
newVP16
pUC ori
CDS
tetR
bla (amp[R])
pLA3641
17781bp
3'-end intron
5'end intron
hsp70 minpr
tetOx5
3' ITR
GAGA x2
piggyBac 3'
tetOx14
SV40 3'UTR
hsp70 minpro
DsRed2
Adh intron
3xP3
SV40 polyA
PiggyBac5
nls
5' ITR
DsRed2
nls
ATG start
Scraps Intron
IE1 promoter
Hr5
AttP

Figure 64

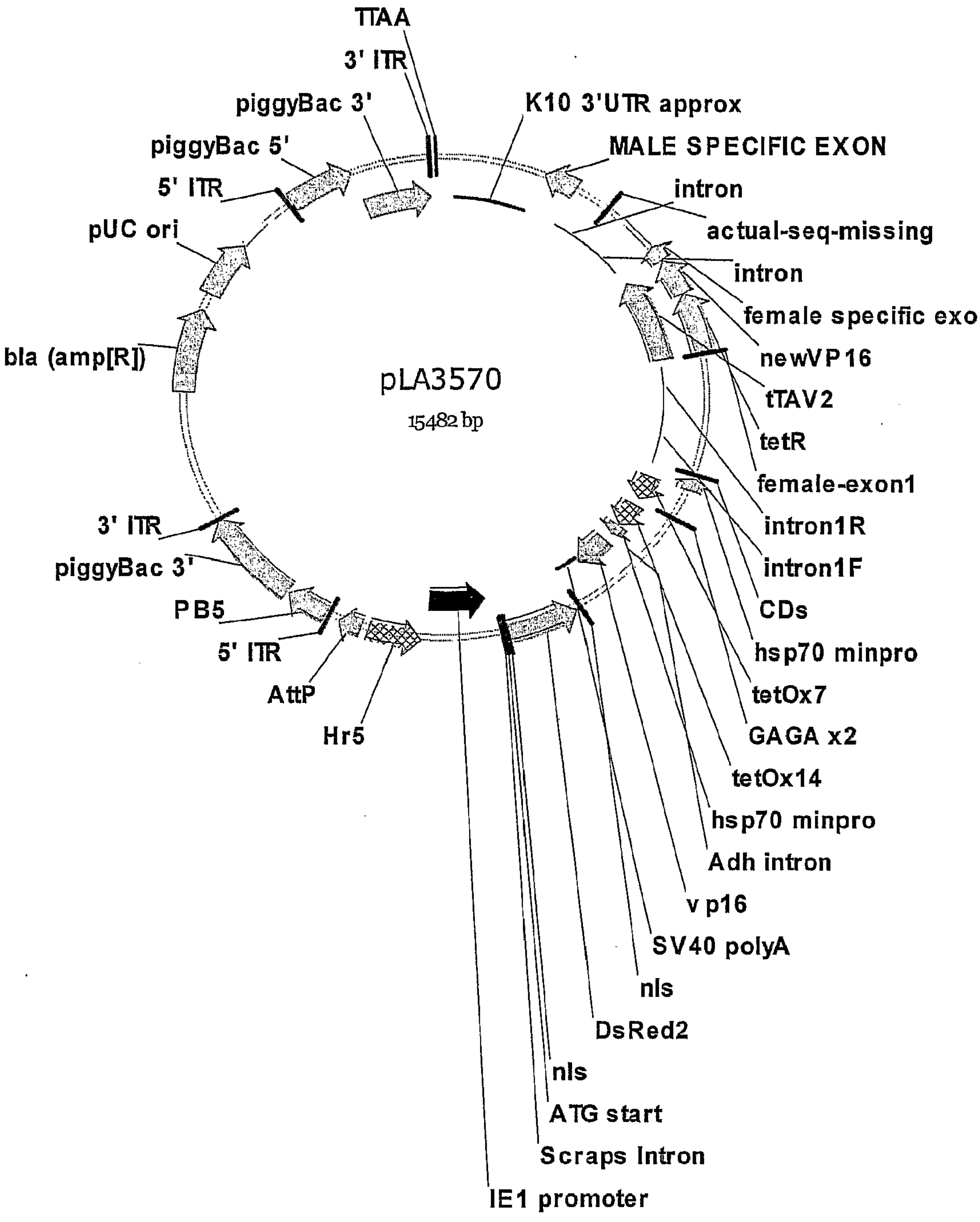
TTAA
3' ITR
piggyBac 3'
K10 3'UTR approx
piggyBac 5'
MALE SPECIFIC EXON
5' ITR
intron
pUC ori
actual-seq-missing
intron
female specific exo
newVP16
bla (amp[R])
pLA3570
15482 bp
tTAV2
tetR
female-exon1
intron1R
3' ITR
intron1F
piggyBac 3'
CDs
PB5
hsp70 minpro
5' ITR
tetOx7
AttP
GAGA x2
Hr5
tetOx14
hsp70 minpro
Adh intron
vp16
SV40 polyA
nls
DsRed2
nls
ATG start
Scraps Intron
IE1 promoter

Figure 65

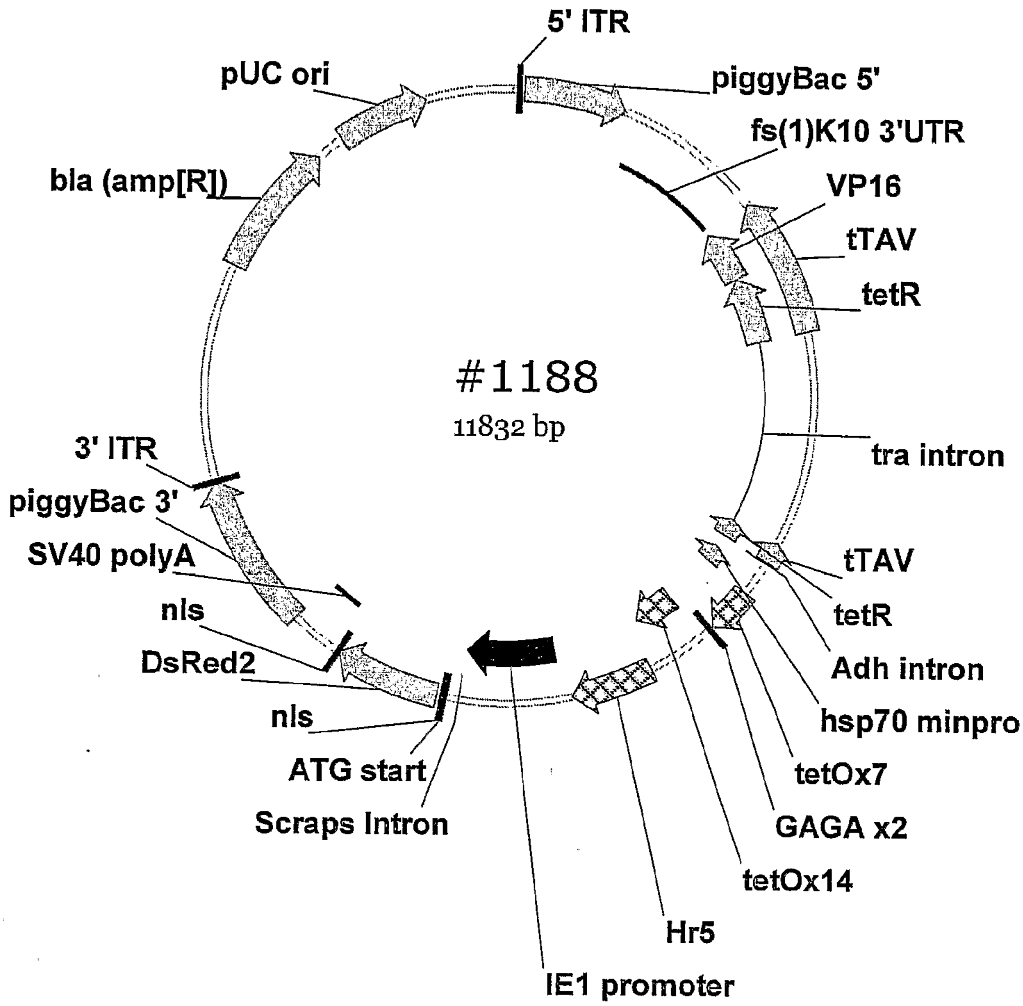
5' ITR
piggyBac 5'
fs(1)K10 3'UTR
VP16
tTAV
tetR
tra intron
tTAV
tetR
Adh intron
hsp70 minpro
tetOx7
GAGA x2
tetOx14
Hr5
IE1 promoter
Scraps Intron
ATG start
nls
DsRed2
nls
SV40 polyA
piggyBac 3'
3' ITR
bla (amp[R])
pUC ori
#1188
11832 bp

Figure 66

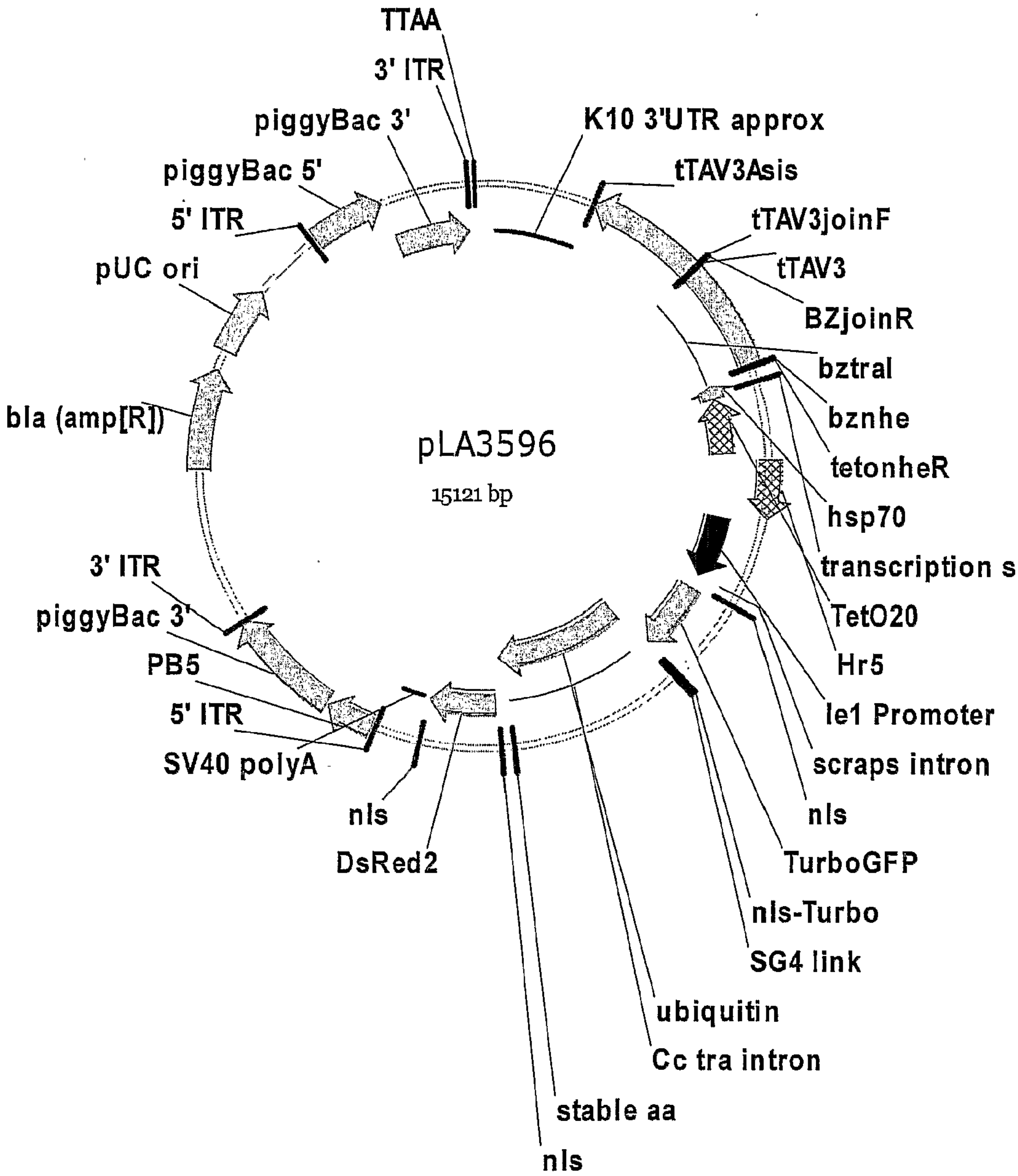
TTAA
3' ITR
piggyBac 3'
K10 3'UTR approx
piggyBac 5'
tTAV3Asis
5' ITR
tTAV3joinF
pUC ori
tTAV3
BZjoinR
bztral
bla (amp[R])
bznhe
pLA3596
15121 bp
tetonheR
hsp70
transcription s
3' ITR
TetO20
piggyBac 3'
Hr5
PB5
Ie1 Promoter
5' ITR
scraps intron
SV40 polyA
nls
nls
TurboGFP
DsRed2
nls-Turbo
SG4 link
ubiquitin
Cc tra intron
stable aa
nls

Figure 67

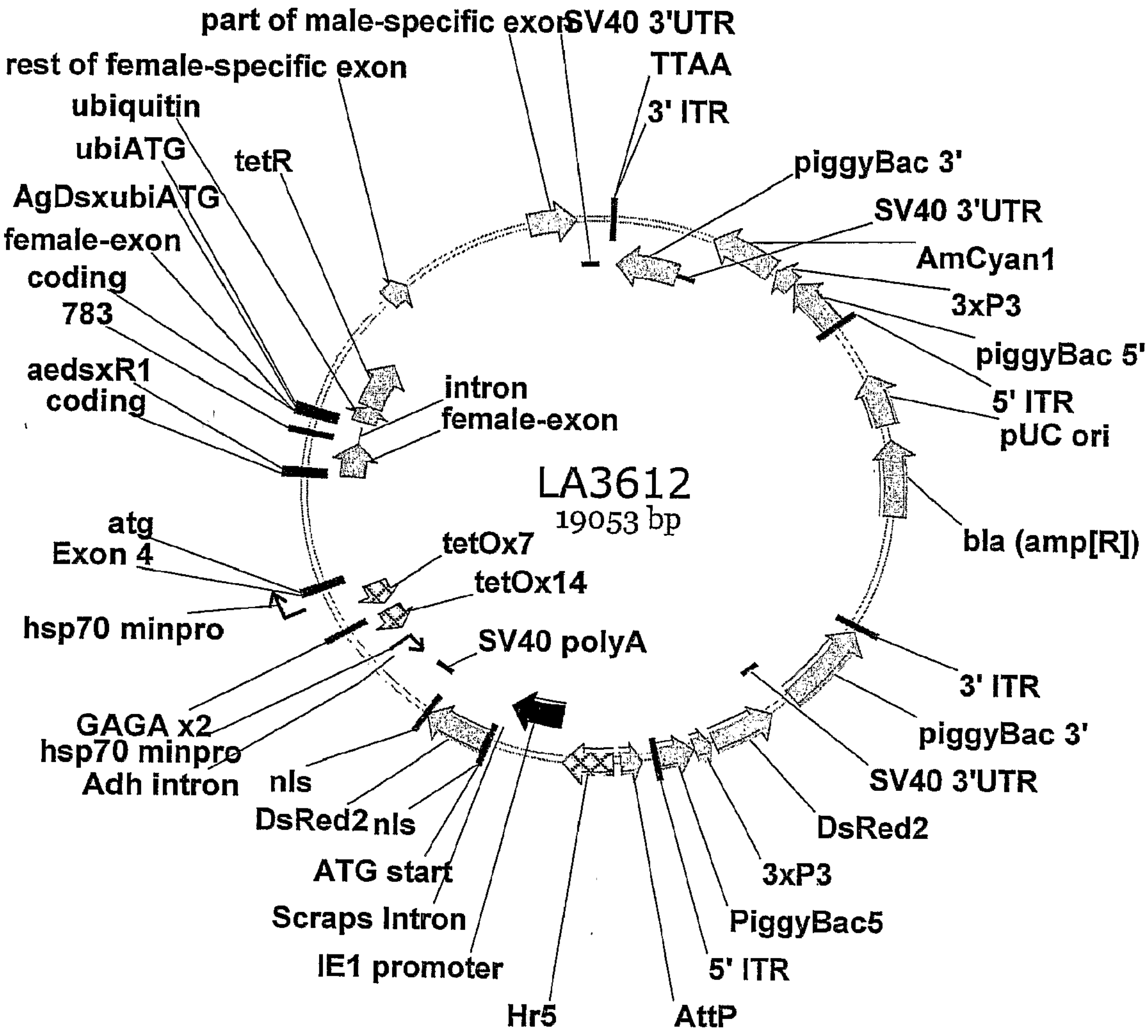

LA3612
19053 bp
part of male-specific exon
SV40 3'UTR
TTAA
3' ITR
rest of female-specific exon
ubiquitin
ubiATG
tetR
AgDsxubiATG
female-exon
coding
783
aedsxR1
coding
intron
female-exon
piggyBac 3'
SV40 3'UTR
AmCyan1
3xP3
piggyBac 5'
5' ITR
pUC ori
bla (amp[R])
atg
Exon 4
tetOx7
tetOx14
hsp70 minpro
SV40 polyA
GAGA x2
hsp70 minpro
Adh intron
nls
DsRed2
nls
ATG start
Scraps Intron
IE1 promoter
Hr5
AttP
5' ITR
PiggyBac5
3xP3
DsRed2
SV40 3'UTR
piggyBac 3'
3' ITR


Figure 68

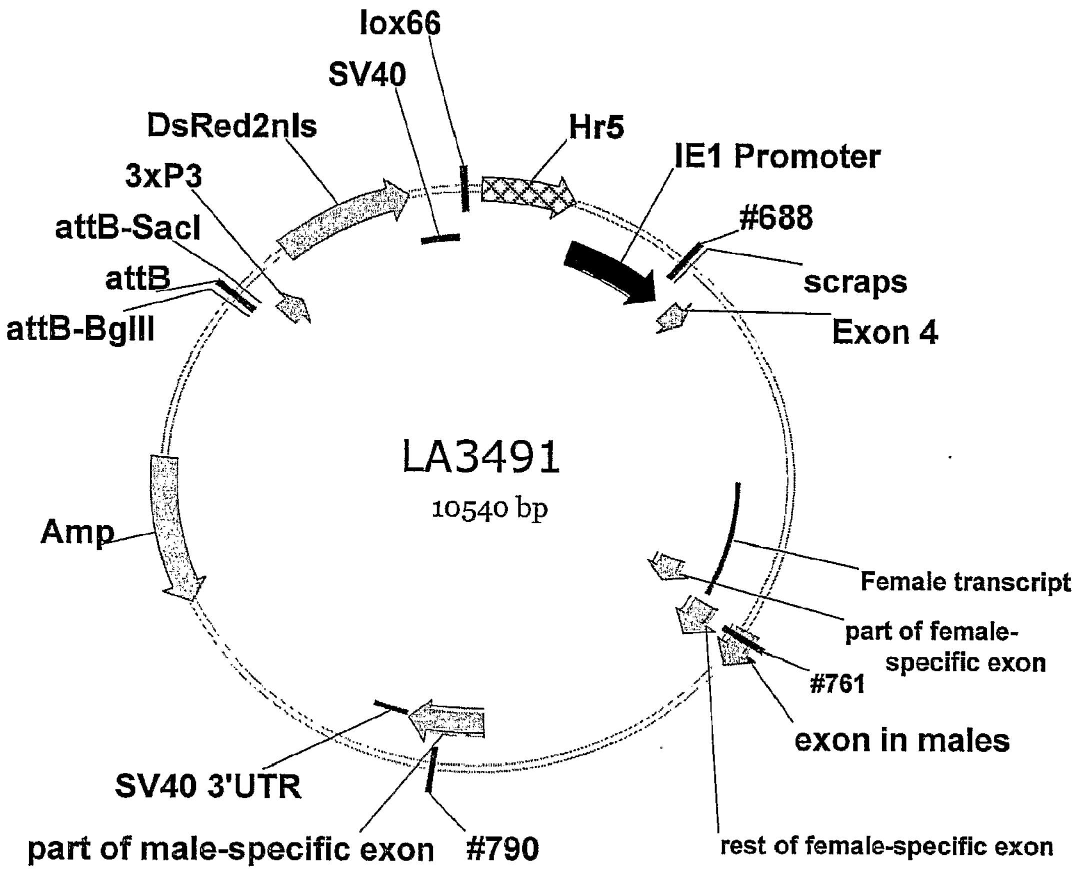
lox66
SV40
DsRed2nls
Hr5
IE1 Promoter
3xP3
attB-SacI
#688
scraps
attB
attB-BglII
Exon 4
LA3491
10540 bp
Amp
Female transcript
part of female-specific exon
#761
exon in males
SV40 3'UTR
part of male-specific exon
#790
rest of female-specific exon

Figure 69

Europäisches Patentamt
European Patent Office
Office européen des brevets

# EUROPEAN SEARCH REPORT

Application Number
EP 16 20 1986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/012534 A (OXITEC LTD [GB]; ALPHEY LUKE [GB]) 10 February 2005 (2005-02-10)<br>* example 12 *<br>----- | 1-20 | INV.<br>C12N15/85<br>A01K67/033<br>C12N15/90 |
| X | US 2004/082032 A1 (BOVI PASQUALE DELLI [IT] ET AL) 29 April 2004 (2004-04-29)<br>* paragraphs [0003], [0004], [0039]; claims 45-46 *<br>----- | 1-3,9, 10,15-20 | |
| X | WILLIAM MATTOX ET AL: "Aternative splicing of the sex determination gene transformer-2 is sex-specific in tile germ line but not m the soma",<br>GENES & DEVELOPMENT,<br>vol. 4, 1 May 1990 (1990-05-01), pages 789-805, XP055348674,<br>* abstract; figure 6 *<br>----- | 1-3, 9-12, 15-20 | |
| X | W MATTOX ET AL: "Autoregulation of the splicing of transcripts from the transformer-2 gene of Drosophila.",<br>GENES AND DEVELOPMENT.,<br>vol. 5, no. 5, 1 May 1991 (1991-05-01), pages 786-796, XP055348669,<br>US<br>ISSN: 0890-9369, DOI: 10.1101/gad.5.5.786<br>* abstract; figure 5 *<br>----- | 1-3, 9-12, 15-20 | TECHNICAL FIELDS SEARCHED (IPC)<br>C12N<br>C12Q |
| X | WO 01/39599 A2 (ISIS INNOVATION [GB]; ALPHEY LUKE [GB]; THOMAS DEAN [GB])<br>7 June 2001 (2001-06-07)<br>* pages 10,12,15 *<br>* pages 21,24,68 *<br>* page 70 *<br>----- | 1-8,10, 13,15-20 | |

-/--

The present search report has been drawn up for all claims

1

| Place of search | Date of completion of the search | Examiner |
|---|---|---|
| The Hague | 22 February 2017 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 20 1986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | FU GUOLIANG ET AL: "Female-specific insect lethality engineered using alternative splicing.", NATURE BIOTECHNOLOGY MAR 2007, vol. 25, no. 3, March 2007 (2007-03), pages 353-357, XP002428609, ISSN: 1087-0156 * the whole document * ----- | 1-20 | |

TECHNICAL FIELDS SEARCHED (IPC)

The present search report has been drawn up for all claims

1

| Place of search | Date of completion of the search | Examiner |
|---|---|---|
| The Hague | 22 February 2017 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 20 1986

This annex lists the patent family members relating to the patent documents cited in the above-mentioned European search report. The members are as contained in the European Patent Office EDP file on
The European Patent Office is in no way liable for these particulars which are merely given for the purpose of information.

22-02-2017

| Patent document cited in search report | Publication date | Patent family member(s) | Publication date |
|---|---|---|---|
| WO 2005012534 A | 10-02-2005 | AU 2004261782 A1 | 10-02-2005 |
| | | BR PI0413024 A | 03-10-2006 |
| | | CN 1860235 A | 08-11-2006 |
| | | EP 1649027 A1 | 26-04-2006 |
| | | ES 2447422 T3 | 12-03-2014 |
| | | GB 2404382 A | 02-02-2005 |
| | | PT 1649027 E | 25-02-2014 |
| | | US 2006242717 A1 | 26-10-2006 |
| | | US 2007056051 A1 | 08-03-2007 |
| | | US 2016060651 A1 | 03-03-2016 |
| | | WO 2005012534 A1 | 10-02-2005 |
| | | ZA 200600728 B | 30-05-2007 |
| US 2004082032 A1 | 29-04-2004 | AU 2002236148 A1 | 19-09-2002 |
| | | EP 1368374 A2 | 10-12-2003 |
| | | IT RM20010120 A1 | 09-09-2002 |
| | | US 2004082032 A1 | 29-04-2004 |
| | | WO 02070686 A2 | 12-09-2002 |
| WO 0139599 A2 | 07-06-2001 | AT 383434 T | 15-01-2008 |
| | | AU 784705 B2 | 01-06-2006 |
| | | CA 2392111 A1 | 07-06-2001 |
| | | CN 1433475 A | 30-07-2003 |
| | | DE 60037752 T2 | 24-12-2008 |
| | | EP 1246927 A2 | 09-10-2002 |
| | | ES 2298167 T3 | 16-05-2008 |
| | | GB 2355459 A | 25-04-2001 |
| | | IL 149885 A | 29-12-2008 |
| | | MX PA02005337 A | 02-04-2004 |
| | | NZ 519175 A | 27-02-2004 |
| | | SK 7352002 A3 | 06-11-2002 |
| | | US 2003213005 A1 | 13-11-2003 |
| | | US 2008115233 A1 | 15-05-2008 |
| | | US 2013298266 A1 | 07-11-2013 |
| | | US 2016044902 A1 | 18-02-2016 |
| | | WO 0139599 A2 | 07-06-2001 |
| | | ZA 200204167 B | 29-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0139599 A
- WO 2005012534 A


### Non-patent literature cited in the description


- **JURICA, M. S. ; MOORE, M. J.** *Mol. Cell,* 2003, vol. 12, 5-14
- **GRAVELEY, B. R.** *Trends Genet.,* 2001, vol. 17, 100-107
- **BLACK, D. L.** *Annu. Rev. Biochem.,* 2003, vol. 72, 291-336
- **CACERES, J. F. ; KORNBLIHTT, A. R.** *Trends Genet.,* 2002, vol. 18, 186-193
- **SMITH, C. W. ; VALCARCEL, J.** *Trends Biochem. Sci.,* 2000, vol. 25, 381-388
- **JOHNSON, J. M. ; CASTLE, J. ; GARRETT-ENGELE, P. ; KAN, Z. ; LOERCH, P. M. ; ARMOUR C. D. ; SANTOS, R. ; SCHADT, E. E. ; STOUGHTON, R. ; SHOEMAKER, D. D.** *Science,* 2003, vol. 302, 2141-2144
- **CANDÉ et al.** *Journal of Cell Science,* 2002, vol. 115, 4727-4734
- **HEINRICH ; SCOTT.** Proc. Natl Acad. Sci USA. 2000, vol. 97, 8229-8232
- **HORN ; WIMMER.** *Nature Biotechnology,* 2003, vol. 21, 64-70
- **PARKER et al.** *Biochemical Journal,* 2002, vol. 368, 789-797
- **BENNETT et al.** *Genetics,* 2003, vol. 164, 235-245
- **FRYXELL ; MILLER.** *Journal of Economic Entomology,* 1995, vol. 88, 1221-1232
- **PANE et al.** *Development,* 2002, vol. 129, 3715-3725
- **NIMMO, D.D. ; ALPHEY, L. ; MEREDITH, J.M. ; EGGLESTON, P.** High efficiency site-specific genetic engineering of the mosquito genome. *Insect Molecular Biology,* 2006, vol. 15, 129-136
- **ALLEN ML ; CHRISTENSEN BM.** Related 2004 Flight muscle-specific expression of act88F: GFP in transgenic Culex quinquefasciatus Say (Diptera: Culicidae). *Parasitol Int.,* vol. 53 (4), 307-14
- **BENNETT D ; SZOOR B ; GROSS S ; VERESHCHAGINA N ; ALPHEY L.** Ectopic expression of inhibitors of protein phosphatase type 1 (PP1) can be used to analyze roles of PP1 in Drosophila development. *Genetics,* 2003, vol. 164 (1), 235-45
- **BLACK, D.** Mechanisms of alternative pre-messenger RNA splicing. *Annu Rev Biochem,* 2003, vol. 72, 291-336
- **BURSET, M. ; SELEDTSOV, I. ; SOLOVYEV, V.** SpliceDB: database of canonical and non-canonical splice sites in mammalian genomes. *Nucleic Acids Research,* 2001, vol. 29, 255-259
- **CACERES JF ; KORNBLIHTT AR ; 2002.** Alternative splicing: multiple control mechanisms and involvement in human disease. *Trends Genet,* vol. 18 (4), 186-93
- **CANDE C, CECCONI F, DESSEN P, KROEMER G.** Apoptosis-inducing factor (AIF): key to the conserved caspase-independent pathways of cell death?. *J Cell Sci.,* 2002, vol. 115 (24), 4727-34
- **CARTEGNI, L. ; CHEW, S. ; KRAINER, A.** Listening to silence and understanding nonsense: exonic mutations that affect splicing. *Nature Reviews Genetics,* 2002, vol. 3, 285-298
- **CLARK, F. ; THANARAJ, T.** Categorization and characterization of transcript-confirmed constitutively and alternatively spliced introns and exons from human. *Human Molecular Genetics,* 2002, vol. 11, 451-464
- **FUNAGUMA, S. ; SUZUKI, M. ; TAMURA, T. ; SHIMADA, T.** The Bmdsx transgene including trimmed introns is sex-specifically spliced in tissues of the silkworm, Bombyx mori. *J Insect Sci,* 2005, vol. 5, 17
- **GEORGE, E.L. ; OBER, M.B. ; EMERSON JR, C.P.** Functional domains of the Drosophila melanogaster muscle myosin heavy-chain gene are encoded by alternatively spliced exons. *Mol. Cell Biol.,* 1989, vol. 9, 2957-2974
- **GRAVELEY BR.** Alternative splicing: increasing diversity in the proteomic world. *Trends Genet.,* 2001, vol. 17 (2), 100-7
- **HAMMES, A. ; GUO, J.K. ; LUTSCH, G. ; LEHESTE, J.R. ; LANDROCK, D. ; ZEIGLER, U. ; GUBLER, M.C. ; SCHEDL, A.** Two splice variants of the Wilms' Tumour 1 gene have distinct functions during sex determination and nephron formation. *Cell,* 2001, vol. 106, 319-329

- **HASTINGS, G.A. ; EMERSON JR, C.P.** Myosin functional domains encoded by alternative exons are expressed in specific thoracic muscles of Drosophila. *J. Cell Biol.,* 1991, vol. 114, 263-276
- **HEDLEY, M.L. ; MANIATIS.** Sex-specific splicing and polyadenylation of dsx pre-mRNA requires a sequence that binds specifically to a tra-2 protein in vivo. *Cell,* 1991, vol. 65, 579-586
- **HEINRICH J.C. ; SCOTT M.J.** A repressible female-specific lethal genetic system for making transgenic insect strains suitable for a sterile-release program. *PNAS,* 2000, vol. 97 (15), 8229-8232
- **HORN C ; WIMMER EA.** A transgene-based, embryo-specific lethality system for insect pest management. *Nat Biotechnol.,* 2003, vol. 21 (1), 64-70
- **HOSHIJIMA, K.K ; INOUE, L. ; HIGUCHI, I. ; SAKAMOTO, H. ; SHIMURA, Y.** Control of doublesex alternative splicing by transformer and transformer-2 in Drosophila. *Science,* 1991, vol. 252, 833-836
- **HUANG, Q., DEVERAUX, Q.L., MAEDA, S., SALVESEN, G.S., STENNICKE, H.R., HAMMOCK, B.D., REED, J.C.** Evolutionary conservation of apoptosis mechanisms: Lepidopteran and baculoviral inhibitor of apoptosis proteins are inhibitor of mammalian caspase-9. *Agricultural Sciences,* 2002, vol. 97 (4), 1427-1432
- **ITO, Y. ; HIROCHICKA, H. ; KURATA, N.** Organ-specific alternative transcripts of KNOX family class 2 homeobox genes of rice. *Gene,* 2002, vol. 288, 41-47
- **JOHNSON JM ; CASTLE J ; GARRETT-ENGELE P ; KAN Z ; LOERCH PM ; ARMOUR CD ; SANTOS R ; SCHADT EE ; STOUGHTON R ; SHOEMAKER DD.** Genome-wide survey of human alternative pre-mRNA splicing with exon junction microarrays. *Science,* 2003, vol. 302 (5653), 2141-4
- **JURICA MS ; MOORE MJ.** Pre-mRNA splicing: awash in a sea of proteins. *Mol Cell,* 2003, vol. 12 (1), 5-14
- **KAZZAZ JA ; ROZEK CE.** Tissue-specific expression of the alternately processed Drosophila myosin heavy-chain messenger RNAs. *Dev Biol.,* 1989, vol. 133 (2), 550-61
- **MANIATIS, T. ; TASIC, B.** Alternative pre-mRNA splicing and proteome expansion in metazoans. *Nature,* 2002, vol. 418, 236-243
- **MUÑOZ, D. ; JIMENEZ, A. ; MARINOTTI, O. ; JAMES, A.** The AeAct-4 gene is expressed in the developing flight muscles of females Aedes aegypti. *Insect Molecular Biology,* 2004, vol. 13, 563-568
- **NISHIYAMA, R. ; MIZUNO, H. ; OKADA, S. ; YAMAGUCHI, T. ; TAKENAKA, M. ; FUKUZAWA, H. ; OHYAMA, K.** Two mRNA species encoding calcium-dependent protein kinases are differentially expressed in sexual organs of Marchantia polymorpha through alternative splicing. *Plant Cell Physiol,* 1999, vol. 40 (2), 205-212
- **NISHIYAMA, R. ; YAMATO, K.T. ; MIURA, K. ; SAKIDA, M. ; OKADA, S. ; KONO, K. ; TAKAHAMA, M. ; SONE, T. ; TAKENAKA, M. ; FUKUZAWA, H.** Comparison of expressed sequence tags from male and female sexual organs of Marchantia polymorpha. *DNA Res.,* 2000, vol. 7, 165-174
- **OLSON, M.R. ; HOLLEY, C.L. ; JI YOO, S. ; HUH, J.R ; HAY, B.A. ; KORNBLUTH, S.** Reaper is regulated by IAP-mediated Ubiquitination. *J.Biol.Chem.,* 2003, vol. 278 (6), 4028-4034
- **OLSON, M.R. ; HOLLEY, C.L. ; GAN, E.C. ; COLON-RAMOS, D.A. ; KAPLAN, B. ; KORNBLUTH, S.** A GH3-like domain in reaper is required for mitochondrial localization and induction of IAP degradation. *J. Biol. Chem.,* 2003, vol. 278 (45), 44758-44768
- **PAN, Q. ; SHAI, O. ; MISQUITTA, C. ; ZHANG, W. ; SALTZMAN, A. ; MOHAMMAD, N. ; BABAK, T. ; SIU, H. ; HUGHES, T. ; MORRIS, Q. et al.** Revealing global regulatory features of mammalian alternative splicing using a quantitative microarray platform. *Mol Cell,* 2004, vol. 16, 929-941
- **PANE, A. ; SALVEMINI, M. ; DELLI BOVI, P. ; POLITO, C. ; SACCONE, G.** The transformer gene in Ceratitis capitata provides a genetic basis for selecting and remembering the sexual fate. *Development,* 2002, vol. 129, 3715-3725
- **PARK, J. ; PARISKY, K. ; CELOTTO, A. ; REENAN, R. ; GRAVELEY, B.** Identification of alternative splicing regulators by RNA interference in Drosophila. *Proc Nat'l Acad Sci,* 2004, vol. 101, 15974-15979
- **PARKER L ; GROSS S ; BEULLENS M ; BOLLEN M ; BENNETT D ; ALPHEY L.** Functional interaction between nuclear inhibitor of protein phosphatase type 1 (NIPP1) and protein phosphatase type 1 (PP1) in Drosophila: consequences of over-expression of NIPP1 in flies and suppression by co-expression of PP1. *Biochem J.,* 2002, vol. 368 (3), 789-97
- **RAPHAEL, K.A. ; WHYARD, S. ; SHEARMAN, D. ; AN, X. ; FROMMER, M.** Bactrocera tyroni and closely related pest-tephritids-molecular analysis and prospects for transgenic control strategies. *Insect Biochem. Mol. Biol.,* 2004, vol. 34, 167-176
- **RYNER, L. ; BAKER, B.S.** Regulation of doublesex pre-mRNA processing occurs by 3'-splice site activation. *Genes Dev.,* 1991, vol. 5, 2071-2085
- **SACCONE, G. ; PANE, A. ; POLITO, C.** Sex determination in flies, fruitfles and butterflies. *Genetica,* 2002, vol. 116, 15-23

- **SCALI, C. ; CATTERUCCIA, F. ; LI, Q. ; CRISANTI, A.** Identification of sex-specific transcripts of the Anopheles gambiae doublesex gene. *J Exp Biol,* 2005, vol. 208, 3701-3709
- **SCOTT, M. ; HEINRICH, J. ; LI, X.** Progress towards the development of a transgenic strain of the Australian sheep blowfly (Lucilia cuprina) suitable for a male-only sterile release program. *Insect Biochem Mol Biol,* 2004, vol. 34, 185-192
- **SEO, S-J. ; CHEON, H-M. ; SUN, J. ; SAPPINGTON, T.W. ; RAIKHEL, A.S.** Tissue- and stage-specific expression of two lipophorin receptor variants with seven and eight ligand-binding repeats in the adult mosquito. *J. Biol. Chem.,* 2003, vol. 278 (43), 41954-41962
- **SIEBEL CW ; FRESCO LD ; RIO DC.** The mechanism of somatic inhibition of Drosophila P-element pre-mRNA splicing: multiprotein complexes at an exon pseudo-5' splice site control U1 snRNP binding. *Genes Dev.,* 1992, vol. 6 (8), 1386-401
- **SHIVIKRUPA ; SINGH., R ; SWARUP, G.** Identification of a novel splice variant of C3G which shows tissue-specific expression. *DNA Cell Biol.,* 1999, vol. 18, 701-708
- **SMITH, C. ; VALCARCEL, J.** Alternative pre-mRNA splicing: the logic of combinatorial control. *Trends Biochem Sci,* 2000, vol. 25, 381-388
- **STOSS, O. ; STOILOV, P. ; HARTMANN, A. M. ; NAYLER, O. ; STAMM, S.** The in vivo minigene approach to analyze tissue-specific splicing. *Brain Research Protocols,* 1999, vol. 4, 383-394
- **STOSS, O. ; OLBRICH, M ; HARTMANN, A. M. ; KONIG, H. ; MEMMOTT, J. ; ANDREADIS, A ; STAMM, S.** The STAR/GSG family protein rSLM-2 regulates the selection of alternative splice sites. *J. Biol. Chem.,* 2001, vol. 276 (12), 8665-8673
- **STREULI, M. ; SAITO, H.** Regulation of tissue-specific alternative splicing: exon-specific cis-elements govern the splicing of leukocyte common antigen pre-mRNA. *EMBO J.,* 1989, vol. 8 (3), 787-796
- **SUZUKI, M. ; OHBAYASHI, F. ; MITA, K. ; SHIMADA, T.** The mechanism of sex-specific splicing at the doublesex gene is different between Drosophila melanogaster and Bombyx mori. *Insect Biochem Mol Biol,* 2001, vol. 31, 1201-1211
- **THANARAJ, T. ; CLARK, F.** Human GC-AG alternative intron isoforms with weak donor sites show enhanced consensus at acceptor exon positions. *Nucleic Acids Research,* 2001, vol. 29, 2581-2593
- **THANARAJ, T. ; STAMM, S. ; CLARK, F. ; REITHOVEN, J. ; LE TEXIER, V. ; MUILU, J.** ASD: the Alternative Splicing Database. *Nucleic Acids Research,* 2004, vol. 32, D64-D69
- **VARSHAVSKY, A.** Ubiquitin fusion technique and its descendants. *Meth Enz,* 2000, vol. 327
- **VENABLES, J.** Alternative splicing in the testes. *Curr Opin Genet Dev,* 2002, vol. 12, 615-619
- **VENABLES JP.** Aberrant and alternative splicing in cancer. *Cancer Res.,* 2004, vol. 64 (21), 7647-54
- **VERNOOY, S.Y. ; COPELAND, J. ; GHABOOSI, N. ; GRIFFIN, E.E. ; YOO, S.J. ; HAY, B.A.** *J. Cell Biol.,* 2000, vol. 150 (2), F69-F75
- **WHITE, K. ; TAHOAGLU, E. ; STELLER, H.** Cell killing by the Drosophila gene reaper. *Science,* 1996, vol. 271 (5250), 805-807
- **WING, J.P. ; ZHOU, L. ; SCHWARTZ, L.M. ; NAMBU, J.R.** Distinct cell killing properties of the Drosophila reaper, head involution defective, and grim genes. *Cell Death Diffn,* 2001, vol. 5 (11), 930-939
- **YALI CHIU A. ; PIN OUYANG, A.B.** Loss of Pnn expression attenuates expression levels of SR family splicing factors and modulates alternative pre-mRNA splicing in vivo. *Bioch. Biophys.Res. Comm.,* 2006, vol. 341, 663-671
- **YOSHIMURA, K. ; YABUTA, Y. ; ISHIKAWA, T. ; SHIGEOKA, S.** Idenitification of a cis element for tissue-specific alternative splicing of chloroplast Ascorbate Peroxidase pre-mRNA in higher plants. *J. Biol.Chem,* 2002, vol. 277 (43), 40623-40632

SEQUENCE LISTING

<110> Oxitec Limited

<120> Gene Expression System Using Alternative Splicing in Insects

<130> PN755663EPA

<150> GB 0621234.4
<151> 2006-10-25

<150> US 11/352,177
<151> 2006-02-10

<160> 170

<170> PatentIn version 3.5

<210> 1
<211> 13
<212> DNA
<213> artificial

<220>
<223> Ceratitis capitata tra consensus sequence

<400> 1
tcwwcratca aca 13

<210> 2
<211> 10
<212> DNA
<213> Artificial

<220>
<223> LA3097 flanking sequence

<400> 2
agccaccatg 10

<210> 3
<211> 10
<212> DNA
<213> artificial

<220>
<223> LA3097 flanking sequence

<400> 3
gtcagccgcc 10

<210> 4
<211> 21
<212> DNA
<213> artificial

<220>
<223> primer 688 - ie1-transcr

<400> 4

gttgcaagtt gacactggcg g 21

<210> 5
<211> 21
<212> DNA
<213> artificial

<220>
<223> primer 790 - Aedsx-m-r2

<400> 5
ccactgtgta aggcttcctc c 21

<210> 6
<211> 21
<212> DNA
<213> artificial

<220>
<223> primer 761 - Aedsx-fem-r

<400> 6
ggatggttgg ttgaagatcc g 21

<210> 7
<211> 21
<212> DNA
<213> artificial

<220>
<223> primer AedsxR1

<400> 7
actgcgcaac tctacaccgt c 21

<210> 8
<211> 13
<212> RNA
<213> artificial

<220>
<223> Pane et al consensus sequence

<400> 8
ucwwcrauca aca 13

<210> 9
<211> 13
<212> RNA
<213> artificial

<220>
<223> Scali et al 2005 consensus sequence

<400> 9
ucwwcaauca aca 13

<210> 10
<211> 13
<212> DNA
<213> Drosophila sp.

<400> 10
tcaacaagca aca 13

<210> 11
<211> 13
<212> DNA
<213> Drosophila sp.

<400> 11
ttatcaaaca aca 13

<210> 12
<211> 13
<212> DNA
<213> Drosophila sp.

<400> 12
tcatcaatta aaa 13

<210> 13
<211> 13
<212> DNA
<213> Drosophila sp.

<400> 13
tcatcaatca aac 13

<210> 14
<211> 13
<212> DNA
<213> Drosophila sp.

<400> 14
tcttcaacca acc 13

<210> 15
<211> 13
<212> DNA
<213> Drosophila sp.

<400> 15
cctacaatct aca 13

<210> 16
<211> 13
<212> DNA
<213> Drosophila sp.

<400> 16
tcttagatca aaa 13

<210> 17
<211> 13
<212> DNA
<213> Drosophila sp.

<400> 17
tcttcgatca tta 13

<210> 18
<211> 13
<212> DNA
<213> Drosophila sp.

<400> 18
ccaacaatct aca 13

<210> 19
<211> 13
<212> DNA
<213> Drosophila sp.

<400> 19
tcaaagatca cca 13

<210> 20
<211> 13
<212> DNA
<213> Drosophila sp.

<400> 20
tcttcggtcg acg 13

<210> 21
<211> 13
<212> DNA
<213> Drosophila sp.

<400> 21
tcgacaaaca aaa 13

<210> 22
<211> 13
<212> DNA
<213> Drosophila sp.

<400> 22
tattcaaaca acg 13

<210> 23
<211> 13
<212> DNA
<213> Drosophila sp.

<400> 23
ttttcgataa aaa 13

<210>  24
<211>  13
<212>  DNA
<213>  Drosophila sp.

<400>  24
tcttcagtct gca    13

<210>  25
<211>  13
<212>  DNA
<213>  Drosophila sp.

<400>  25
gattcaatca tca    13

<210>  26
<211>  13
<212>  DNA
<213>  Drosophila sp.

<400>  26
ttatcgagca aaa    13

<210>  27
<211>  13
<212>  DNA
<213>  Drosophila sp.

<400>  27
tcataactca aga    13

<210>  28
<211>  13
<212>  DNA
<213>  Drosophila sp.

<400>  28
tcagaaatca aaa    13

<210>  29
<211>  13
<212>  DNA
<213>  Drosophila sp.

<400>  29
tctttaattt aca    13

<210>  30
<211>  13
<212>  DNA
<213>  Drosophila sp.

<400>  30
tttacaatcc tca    13

<210> 31
<211> 13
<212> DNA
<213> Drosophila sp.

<400> 31
tcatagatca gga 13

<210> 32
<211> 13
<212> DNA
<213> Drosophila sp.

<400> 32
acctcaaaca aca 13

<210> 33
<211> 13
<212> DNA
<213> Drosophila sp.

<400> 33
tcatcgaaca ccc 13

<210> 34
<211> 1014
<212> DNA
<213> artificial

<220>
<223> Open reading frame of tTAV construct

<400> 34
atgggcagcc gcctggataa gtccaaagtc atcaactccg cgttggagct gttgaacgaa 60

gttggcattg agggactgac gacccgcaag ttggcgcaga agctgggcgt ggagcagccc 120

accctctact ggcacgtgaa gaataagcgg gcgctgctgg atgccctggc catcgagatg 180

ctcgaccgcc accacacgca tttttgcccg ttggaaggcg agtcctggca ggacttcctc 240

cgcaataacg ccaagtcgtt ccgctgcgct ctgctgtccc accgagacgg tgccaaagtc 300

catctcggca cgcgcccgac cgaaaagcaa tacgagacac tggagaacca gctcgcgttc 360

ctgtgccagc aaggcttcag cctggaaaat gctctctacg ctctgagcgc cgtcggtcac 420

tttaccctgg gctgcgtgct ggaggaccaa gagcatcaag tcgcaaaaga ggagcgcgag 480

accccaacaa ccgattcgat gcccccactg ctgcgtcagg caatcgagct gttcgatcat 540

caaggagccg agccggcatt cctgttcggc ttggagctga ttatctgcgg attggaaaag 600

caactgaaat gcgagtcggg ctcgggcccc gcgtacagcc gcgcgcgtac gaaaaacaat 660

tacgggtcta ccatcgaggg cctgctcgat ctcccggacg acgacgcccc cgaagaggcg 720

gggctggcgg ctccgcgcct gtcctttctc cccgcgggac acacgcgcag actgtcgacg 780

gcccccccga ccgatgtcag cctgggggac gagctccact tagacggcga ggacgtggcg 840

atggcgcatg ccgacgcgct agacgatttc gatctggaca tgttggggga cggggattcc 900

ccgggtccgg gatttacccc ccacgactcc gccccctacg gcgctctgga tatggccgac 960

ttcgagtttg agcagatgtt taccgatgcc cttggaattg acgagtacgg tggg 1014

<210> 35
<211> 338
<212> PRT
<213> artificial

<220>
<223> Protein sequence of tTAV

<400> 35

Met Gly Ser Arg Leu Asp Lys Ser Lys Val Ile Asn Ser Ala Leu Glu
1 5 10 15

Leu Leu Asn Glu Val Gly Ile Glu Gly Leu Thr Thr Arg Lys Leu Ala
20 25 30

Gln Lys Leu Gly Val Glu Gln Pro Thr Leu Tyr Trp His Val Lys Asn
35 40 45

Lys Arg Ala Leu Leu Asp Ala Leu Ala Ile Glu Met Leu Asp Arg His
50 55 60

His Thr His Phe Cys Pro Leu Glu Gly Glu Ser Trp Gln Asp Phe Leu
65 70 75 80

Arg Asn Asn Ala Lys Ser Phe Arg Cys Ala Leu Leu Ser His Arg Asp
85 90 95

Gly Ala Lys Val His Leu Gly Thr Arg Pro Thr Glu Lys Gln Tyr Glu
100 105 110

Thr Leu Glu Asn Gln Leu Ala Phe Leu Cys Gln Gln Gly Phe Ser Leu
115 120 125

Glu Asn Ala Leu Tyr Ala Leu Ser Ala Val Gly His Phe Thr Leu Gly
130 135 140

Cys Val Leu Glu Asp Gln Glu His Gln Val Ala Lys Glu Glu Arg Glu
145 150 155 160

Thr Pro Thr Thr Asp Ser Met Pro Pro Leu Leu Arg Gln Ala Ile Glu
165 170 175

Leu Phe Asp His Gln Gly Ala Glu Pro Ala Phe Leu Phe Gly Leu Glu

180 185 190

Leu Ile Ile Cys Gly Leu Glu Lys Gln Leu Lys Cys Glu Ser Gly Ser
195 200 205

Gly Pro Ala Tyr Ser Arg Ala Arg Thr Lys Asn Asn Tyr Gly Ser Thr
210 215 220

Ile Glu Gly Leu Leu Asp Leu Pro Asp Asp Asp Ala Pro Glu Glu Ala
225 230 235 240

Gly Leu Ala Ala Pro Arg Leu Ser Phe Leu Pro Ala Gly His Thr Arg
245 250 255

Arg Leu Ser Thr Ala Pro Pro Thr Asp Val Ser Leu Gly Asp Glu Leu
260 265 270

His Leu Asp Gly Glu Asp Val Ala Met Ala His Ala Asp Ala Leu Asp
275 280 285

Asp Phe Asp Leu Asp Met Leu Gly Asp Gly Asp Ser Pro Gly Pro Gly
290 295 300

Phe Thr Pro His Asp Ser Ala Pro Tyr Gly Ala Leu Asp Met Ala Asp
305 310 315 320

Phe Glu Phe Glu Gln Met Phe Thr Asp Ala Leu Gly Ile Asp Glu Tyr
325 330 335

Gly Gly

<210> 36
<211> 1014
<212> DNA
<213> artificial

<220>
<223> Open reading frame of tTAV2

<400> 36
atgagccgcc tggataagtc caaagtcatc aactccgcgt tggagctgtt gaacgaagtt 60

ggcattgagg gactgacgac ccgcaagttg gcgcagaagc tgggcgtgga gcagcccacc 120

ctctactggc acgtgaagaa taagcgggcg ctgctggatg ccctggccat cgagatgctc 180

gaccgccacc acacgcattt ttgcccgttg gaaggcgagt cctggcagga cttcctccgc 240

aataacgcca agtcgttccg ctgcgctctg ctgtcccacc gagacggtgc caaagtccat 300

ctcggcacgc gcccgaccga aaagcaatac gagacactgg agaaccagct cgcgttcctg 360

tgccagcaag gcttcagcct ggaaaatgct ctctacgctc tgagcgccgt cggtcacttt 420

accctgggct gcgtgctgga ggaccaagag catcaagtcg caaaagagga gcgcgagacc 480

ccaacaaccg attcgatgcc cccactgctg cgtcaggcaa tcgagctgtt cgatcatcaa 540

ggagccgagc cggcattcct gttcggcttg gagctgatta tctgcggatt ggaaaagcaa 600

ctgaaatgcg agtcgggctc gggccccgcc tacagccgcg cccgcaccaa gaacaactac 660

ggcagcacca tcgagggcct gctggatctg ccggatgatg atgccccgga ggaggcgggc 720

ctggccgccc cgcgcctgag cttcctgccg gccggacaca cccgccgcct gtcgaccgcc 780

ccgccgaccg acgtgagcct gggcgatgag ctgcacctgg atggcgagga tgtggcgatg 840

gcccacgccg atgccctgga cgacttcgac ctggacatgc tgggcgatgg cgatagcccg 900

ggaccgggat tcaccccgca cgatagcgcc ccctacggcg ccctggatat ggccgatttc 960

gagttcgagc agatgttcac cgacgccctg ggcatcgatg agtacggcgg ctaa 1014

<210> 37
<211> 337
<212> PRT
<213> artificial

<220>
<223> Protein sequence of tTAV2

<400> 37

Met Ser Arg Leu Asp Lys Ser Lys Val Ile Asn Ser Ala Leu Glu Leu
1 5 10 15

Leu Asn Glu Val Gly Ile Glu Gly Leu Thr Thr Arg Lys Leu Ala Gln
20 25 30

Lys Leu Gly Val Glu Gln Pro Thr Leu Tyr Trp His Val Lys Asn Lys
35 40 45

Arg Ala Leu Leu Asp Ala Leu Ala Ile Glu Met Leu Asp Arg His His
50 55 60

Thr His Phe Cys Pro Leu Glu Gly Glu Ser Trp Gln Asp Phe Leu Arg
65 70 75 80

Asn Asn Ala Lys Ser Phe Arg Cys Ala Leu Leu Ser His Arg Asp Gly
85 90 95

Ala Lys Val His Leu Gly Thr Arg Pro Thr Glu Lys Gln Tyr Glu Thr
100 105 110

Leu Glu Asn Gln Leu Ala Phe Leu Cys Gln Gln Gly Phe Ser Leu Glu

```
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Ser Gly
        195                 200                 205

Pro Ala Tyr Ser Arg Ala Arg Thr Lys Asn Asn Tyr Gly Ser Thr Ile
    210                 215                 220

Glu Gly Leu Leu Asp Leu Pro Asp Asp Asp Ala Pro Glu Glu Ala Gly
225                 230                 235                 240

Leu Ala Ala Pro Arg Leu Ser Phe Leu Pro Ala Gly His Thr Arg Arg
                245                 250                 255

Leu Ser Thr Ala Pro Pro Thr Asp Val Ser Leu Gly Asp Glu Leu His
            260                 265                 270

Leu Asp Gly Glu Asp Val Ala Met Ala His Ala Asp Ala Leu Asp Asp
        275                 280                 285

Phe Asp Leu Asp Met Leu Gly Asp Gly Asp Ser Pro Gly Pro Gly Phe
    290                 295                 300

Thr Pro His Asp Ser Ala Pro Tyr Gly Ala Leu Asp Met Ala Asp Phe
305                 310                 315                 320

Glu Phe Glu Gln Met Phe Thr Asp Ala Leu Gly Ile Asp Glu Tyr Gly
                325                 330                 335

Gly


<210>  38
<211>  1011
<212>  DNA
<213>  artificial
```

<220>
<223> Open reading frame of tTAV3

<400> 38
atgggcagcc gcctggacaa gagcaaggtg atcaacagcg ccctggagct gctgaacgaa 60

gttggtatcg agggcctgac cacccgcaag ctggcccaga agctgggcgt ggaacagccg 120

accctgtact ggcacgtgaa gaacaagcgc gccctgctgg acgccctggc catcgaaatg 180

ctggatcgcc accacaccca cttctgcccg ctggagggcg agagctggca ggatttcctg 240

cgcaacaacg ccaagagctt ccgctgcgcc ctgctgtcgc accgcgatgg cgccaaggtg 300

cacctgggca cccgcccgac cgagaagcag tacgagaccc tggagaacca gctggccttc 360

ctgtgccagc agggcttcag cctggagaac gccctgtacg ccctgagcgc cgtgggccac 420

ttcaccctgg gctgtgtgct ggaggatcag gagcaccagg tggccaagga ggagcgcgag 480

accccgacca ccgatagcat gccgccgctg ctgcgccagg ccatcgagct gttcgatcac 540

cagggcgccg agccggcctt cctgttcggc ctggagctga tcatctgcgg cctggaaaag 600

cagctgaagt gcgagagcgg cagcgcctac agccgcgccc gtaccaagaa caactatggc 660

agcaccatcg agggactgct ggacctgccg gatgacgatg ccccggagga agccggcctg 720

gccgcccccc gcctgagctt cctgcccgcc ggacacacgc gccgcctgag caccgccccg 780

ccgaccgatg tgagcctggg cgacgagctg cacctggatg gagaggatgt ggcaatggcc 840

cacgccgacg ccctggacga tttcgacctg gatatgctgg gcgatggaga tagcccggga 900

ccgggcttca cgccccacga tagcgccccg tacggcgccc tggacatggc cgacttcgag 960

ttcgagcaaa tgttcaccga cgcgctgggc atcgatgagt atggcgggta g 1011

<210> 39
<211> 336
<212> PRT
<213> artificial

<220>
<223> Protein sequence of tTAV3

<400> 39

Met Gly Ser Arg Leu Asp Lys Ser Lys Val Ile Asn Ser Ala Leu Glu
1               5                   10                  15

Leu Leu Asn Glu Val Gly Ile Glu Gly Leu Thr Thr Arg Lys Leu Ala
            20                  25                  30

Gln Lys Leu Gly Val Glu Gln Pro Thr Leu Tyr Trp His Val Lys Asn
        35                  40                  45

Lys Arg Ala Leu Leu Asp Ala Leu Ala Ile Glu Met Leu Asp Arg His

```
    50                  55                  60

His Thr His Phe Cys Pro Leu Glu Gly Glu Ser Trp Gln Asp Phe Leu
65                  70                  75                  80

Arg Asn Asn Ala Lys Ser Phe Arg Cys Ala Leu Leu Ser His Arg Asp
                85                  90                  95

Gly Ala Lys Val His Leu Gly Thr Arg Pro Thr Glu Lys Gln Tyr Glu
            100                 105                 110

Thr Leu Glu Asn Gln Leu Ala Phe Leu Cys Gln Gln Gly Phe Ser Leu
        115                 120                 125

Glu Asn Ala Leu Tyr Ala Leu Ser Ala Val Gly His Phe Thr Leu Gly
    130                 135                 140

Cys Val Leu Glu Asp Gln Glu His Gln Val Ala Lys Glu Glu Arg Glu
145                 150                 155                 160

Thr Pro Thr Thr Asp Ser Met Pro Pro Leu Leu Arg Gln Ala Ile Glu
                165                 170                 175

Leu Phe Asp His Gln Gly Ala Glu Pro Ala Phe Leu Phe Gly Leu Glu
            180                 185                 190

Leu Ile Ile Cys Gly Leu Glu Lys Gln Leu Lys Cys Glu Ser Gly Ser
        195                 200                 205

Ala Tyr Ser Arg Ala Arg Thr Lys Asn Asn Tyr Gly Ser Thr Ile Glu
    210                 215                 220

Gly Leu Leu Asp Leu Pro Asp Asp Asp Ala Pro Glu Glu Ala Gly Leu
225                 230                 235                 240

Ala Ala Pro Arg Leu Ser Phe Leu Pro Ala Gly His Thr Arg Arg Leu
                245                 250                 255

Ser Thr Ala Pro Pro Thr Asp Val Ser Leu Gly Asp Glu Leu His Leu
            260                 265                 270

Asp Gly Glu Asp Val Ala Met Ala His Ala Asp Ala Leu Asp Asp Phe
        275                 280                 285

Asp Leu Asp Met Leu Gly Asp Gly Asp Ser Pro Gly Pro Gly Phe Thr
    290                 295                 300
```

```
Pro His Asp Ser Ala Pro Tyr Gly Ala Leu Asp Met Ala Asp Phe Glu
305                 310                 315                 320

Phe Glu Gln Met Phe Thr Asp Ala Leu Gly Ile Asp Glu Tyr Gly Gly
                325                 330                 335

<210>  40
<211>  568
<212>  DNA
<213>  Pectinophora gossypiella

<400>  40
gctagtggag aactgccaca aactgctgga aaagttccac tactcctggg aaatgatgcc      60

cctggtgctg gtcattctaa actacgccgg ctccgacctc gacgaggctt ctagaaaaat     120

tgatgaaggg aagatgatca tcaacgagta cgcgaggaag cacaatctga acatcttcga     180

tggccacgag ctaaggaact cgactcgcca gtacggactt taatacagta atattagttt     240

tctccaacaa cactaaacac gacataacac gctacacgca aaaaatacac gagtctttaa     300

tgttttacac gctcagtaaa ttattcactt acacgcttaa ctaaaatttt acacaatcgg     360

taaaaaaata caacaattta ttatcgtaaa aattacacaa aataaatgag atttaaatgt     420

cgtttaataa aataaaataa aaatagcatc gggaatatct tttcacctat tgccggagaa     480

cagtttaaat ggatactctc atttgaatca ttttaattgt agtagcattt tattttatta     540

ttaatagcaa taagtacaca aacataaa                                        568

<210>  41
<211>  610
<212>  DNA
<213>  Pectinophora gossypiella

<400>  41
gtagtggaga actgccacaa actgctggaa aagttccact actcctggga aatgatgccc      60

ctggtgctgg tcattctaaa ctacgccggc tccgacctcg acgaggcttc tagaaaaatt     120

gatgaaggga agatgatcat caacgagtac gcgaggaagc acaatctgaa catcttcgat     180

ggccacgagc tgaggaactc gactcgccag tacggacttt aatacagaaa atgctgagcg     240

aaattaataa tataagtggt gtactatcgt cgtccatgaa gttattttgc gaatgatact     300

ttgttttgta tgtgctgtgt gttgtgtgga cttttgctgt gcgttgctgt ttgcgatgga     360

aggactattg tgtcgtcgcc acgctggact attcgcacat tgggtggtcc accagtggcg     420

gatgtacgag cggtcgctgt gctcgctcct ggagctgcaa gcgcgcaaag ggacgtactc     480

ggtgtgctgc tcaccccgct acgtcatcgc gcccgagtac gcgtcacacc tgttgcctct     540

gccgcttacc acgcagagat catccccgcc gcccgcgcac ttgtagcgat gcgaacctgc     600

gccgcgggaa                                                            610
```

<210> 42
<211> 449
<212> DNA
<213> Pectinophora gossypiella

<220>
<221> misc_feature
<222> (26)..(26)
<223> n is a, c, g, or t

<400> 42
gctagtggag aactgccaca aactgntgga aaagttccac tactcctggg aaatgatgcc 60

cctggtgctg gtcattctaa actacgccgg ctccgacctc gacgaggctt ctagaaaaat 120

tgatgaagca cattgggtgg tccaccagtg gcggatgtac gagcggtcgc tgtgctcgct 180

cctggagctg caagcgcgca aagggacgta ctcggtgtgc tgctcacccc gctacgtcat 240

cgcgcccgag tgcgcgtcac acctgttgcc tctgccgctt accacgcaga gatcatcccc 300

gccgcccgcg cacttgtagc gatgcgaacc tgcgccgcgg gaagtaagta ctatttcatt 360

tattattctt tttatttttg gttttaaggt gctgacagac ttgaatttca agcaaatagt 420

gtctgacaaa gagctcaaaa tagacatgt 449

<210> 43
<211> 28774
<212> DNA
<213> Aedes aegypti

<400> 43
acagtgaaat ttgatcgatc actcatcgaa acgagatcac tttcgattga tcgtgacaat 60

tttttagaat ccatttcaca gtcgttggga ctgttgaccc tgtcacttta aactagctag 120

tgagtagctt tgctctagtg aaagctaact agcactgtta aaaaatctta ggtaaagtgt 180

cagcaaccct gacaactggg ccacctcttg ccgaccataa gcaaatgaaa tcaaatggtt 240

cgctacgaag gttaattggg tttcgatcta cttcgtccta agcgctattt ttcgtcatac 300

ggtggagaac ggctggtatt cgtttacttt agtttaccaa gcgatgcttc caattaaccc 360

aaagctagat gaagcaggat tcgcgataaa aagcagtatg cgaacttaaa atgttctact 420

acattacggc gggtattcaa atttacctgc cacataaatt tattttccaa gtataatttg 480

cgaaagctgc aatggttcat gcttgaattt tacaagatga tgtaatgccg cccataagtt 540

taaatggacg gtgtatttaa ataaaaggtt catattaaac gctttcgacg ttaccaagta 600

ccatttgtac acaaacatgt aataaaacta ttgtatttct ataaataact tcagttcaat 660

catccacttt gcacattttc accgaaatcg catggacgaa ggtaaacatg tgtttgtaca 720

ttattttgat aacataaaga tatttattga agtcaagtta gtaggtgaaa cgtgtaaaag 780

tggctttagc gtacctgctt gacgtaccga gcgaaatctg attagcggtc gactaagcca 840

```
taaaacttct acaattcaca aaattttgaa aaattccctc gctgccacga tactaatgca      900
ctgcatggct cgctttagac taatcgccag ctgattcggt attttgaaga tgttaagtgt      960
tttaaaactt tttaagggag cgacggtgct atgattacgt aatcaaatgt tctttctttt     1020
actttcagac caattgcaga acaagcttta tcctaatcca tctcattttg ggaacagcac     1080
tagccgcgac cattagccgt ttagtttaca agaaagaaaa tgaaagtctg gttaacgtct     1140
tgttcgaaat aggattaggt agagtaaaac ccttgtcgtg atcggcgctg gtaatcggca     1200
tctgcgtaga gaacatgttg tacttcctcg aggacgattg ctcgcgctcg cacggttctt     1260
attgctacca tggtgaaacc actagcgccg aggaagtgct agacgcatct cttgtacaac     1320
atgaaggagc tcctgctaac gagcgcgagc gtgccaagaa taacgatggt accactttgg     1380
tgatcgcggc tccttcacga taccgttgtg aaggttttct gaattgcgca tcgtctccga     1440
agggtgtgtc caggtgcatt gtctcccaac tgacctgttc ccgacaatat cgagcactaa     1500
atggcaacac ttccaaaaga cttaacgcgt agcagaggct tcccacacag gtccacgtaa     1560
cagagggttg actggacaag ggctgttata gctcgtgatt ggtttccatt agagagcagt     1620
atctcgtagt agcgtaggag agtccattag agtgcgatat tccgtgagtt tgtgtgaccg     1680
gcgatagaga agccctgacg ccaaaggtaa tctctcgtca tagagcatca tcgcatcctc     1740
tcaggtaatc tcacgctata aggcactcaa acacactggc cgctatctct tcgggactgc     1800
cgcgcttcaa gacgattgta actcggaaac tgacctgatt agtacataaa aagagaccta     1860
ttgcgtaagc ttataagaaa cgagtttgtc cacacggttg gcgcgaagtt ctgctaacat     1920
tgagcctttg actggactaa tcatgtattt ttctctggat aacgcattcg aatattcttt     1980
gctcaaacag gtgtgccaac atggtttcgc aagatcgctg gatggtaaag atgtccgagg     2040
cagggtacga taaccgggcg gatggcagtg gagcttccag cagcagcctg aacccgcgaa     2100
taccaaagcg ttctagcgac ctaccatttc tacaggctcc gtcccatgct attggcccgc     2160
ctaccgtcac ctcgaaggtc gtcgtcggac ttgggcgctt cgccgccgaa ctgtgcccgc     2220
tgccggaacc acggtcacaa gatcggcctg aagggacaca agcgctattg taagtatcgc     2280
aattgtacct gcgaaaagtg gcggcggctt gacacgggcg acggccttgg tgccagtgtt     2340
ctagccggac ttccctgtgt tcgcgataac attcatagcg ttaacatgga cgcttttcac     2400
ctgcctgacg gccgaacggc agcgggtcat ggccctgcag acggctctcc gaagggcgca     2460
aacccaggac gaacagcggt tgctggtaga cggagaggtg gacggactgc cggcttgccg     2520
tcgcccagta ccgggacgtc tgccgagagg cttcccgcgt ttgggtcctg cttgtcgcca     2580
acgaccatct gcctctccac cccgccgaac cggtacatag ccttcaaata ccaaaattgt     2640
ctgacctaaa agagatgatc cataattctc agcagaggtc gttgatcgac tgcgactcgt     2700
```

gggcggcttg gccatgtatc ggaagtttat ggttttaaca gactggattt tctctactag 2760
gtattaagag tcgtctccag caactagctg acgctgagca ccaccggctc gatgaactcc 2820
accccgggca gctcgttggt aacgctgtcc cagcaccgaa gatcaccctg ctccgccgcg 2880
tcggtccacc ccagcgaggc ggtggccgag ctacttgagg tggggcccgt cgagcaacca 2940
ttgcgacagg gtcgtggctt ctagtgggac gaggcggcgc agccaggtgg ggtcgctccg 3000
tcagcaaaac gttgcaggta ggtgtgaggc atatctattt cgttattctc tcaatgtttg 3060
tggagaaccg gccggaattc aacatcgaag tcggtttctg agtcgttttg caacgtccat 3120
ccacactccg tatagataaa gcaataagag agttacaaac acctcttggc cggccttaag 3180
ttgtagcttc agccaaagac ttctattgat ttatgataaa tttctctcaa atgtttgcgc 3240
ggagggtgga tttttgagag ctgagtggtg tagaaacgaa atgggcatca aacgttatgc 3300
aagataacta aatactattt aaagagagtt tacaaacgcg cctcccacct aaaaactctc 3360
gactcaccac atctttgctt tacccgtagt ttgcaatacg ggcgctgctt gaaacaggtt 3420
tatgttaggg gtttcctgtg tttcatacag tcaccccatt gttatgtata gcacacagat 3480
atggataaaa gttggattaa ccgcgacgaa ctttgtccaa atacaatccc caaaggacac 3540
aaagtatgtc agtggggtaa caatacatat cgtgtgtcta tacctatttt caacctaatt 3600
gcagtgaata tcccatcaaa tagagttgca attgagtaga acacatttta ccaacgtata 3660
aagcatcgta atcaattata atatacttaa gcaaaataca cgtcacttat agggtagttt 3720
atctcaacgt taactcatct tgtgtaaaat ggttgcatat ttcgtagcat tagttaatat 3780
tatatgaatt cgttttatgt atggggaaat aatttgtcaa ccacatttct agaaaagttg 3840
attcatacat gtgtgctttt gaaagccata taccacatta tgtttgattc atatctctta 3900
tacccccttta ttaaacagtt ggtgtaaaga tcttttcaac taagtatgta cacacgaaaa 3960
ctttcggtat atggtgtaat acaaactaag tatagagaat taatatgagt cgatttatcg 4020
cgaaattttt caaaatgtcc tatgtaccaa tgaaagatac tctcttatct cgctctgttt 4080
tgaacataac aactgaaact attatactca gctaaatagc gctttaaaaa gttttacagg 4140
atacatggtt actttctatg agagaataga gcgagacaaa acttgtattg ttgactttga 4200
tttgggaagt ttttcactat agataaaaaa atgtccttga ctagcgtttc atacaaaaaa 4260
aaaaaaaaac gcaaccaaaa atgttaatgt ggttcagtga aaacccttca aaaagtgata 4320
tctatttttt tacaggaact gatcgcaaag tatgtttttt tttttttttg cgttggtttt 4380
tacaattaca ccaagtcact ttgattaaag aggaagtaaa ctaagatagt gtctcaatgt 4440
tggataggtc atttagaaaa ggtccgcgag attggatcca taataatgat tctcctctct 4500
aactaatttc tccttcattt gattctatca cagagttaca acctatccag taaatctttt 4560
ccaggcgctc taacctaggt attattacta agaggagaga cactgatccg catctgtggg 4620

atggacaacg tttgtaattt ctatcggtat cgaaaataat cgcgcatttt cgggcgtatt 4680
ccagaaaaca acaatgaaat gtgactaggc gtagacaccc tacctgttgc aaacattaaa 4740
gatagccata gcttttatta gcgcgtaaaa gcccgcataa ggtcttttgt tgttacttta 4800
atactgaagc aaatgtgcac aattttcatt acatgatatt attcaatggg gtaggtgggc 4860
gacaaaatag attcattaat gttggataat aggggcgttt tatgacttcg tttacacgtg 4920
ttaaaagtaa tgtactataa taagttaccc catccacccg ctgttttatc taagtaatta 4980
caacctatta tccccgcaaa gtcattatcc ctaaatgctc cacctcagct ggtggccccg 5040
tcagtcagtt gatcgggaaa gcagcaatca atccggagac aggtcgacct ccatcgaaca 5100
cagtaatagg gatttacgag gtggagtcga ccaccggggc agtcagtcaa ctagcccttt 5160
cgtcgttagt taggcctctg tccagctgga ggtagcttgt ggaaccgaac aacactagat 5220
gttcgatttc taacgaccga ctaagaacat cgtcggaagc gtctggttca ttcgacgagc 5280
cggaaggggt tcatctttcg ccttggcttg ttgtgatcta caagctaaag attgctggct 5340
gattcttgta gcagccttcg cagaccaagt aagctgctcg gccttcccca agtagaaagc 5400
ctcgtcgtcg aacgaatagc tgctgctaca cttcgcgtcg ttatcgtcgt cgggggattg 5460
gtgtttgtaa ctgcgcactc gtttatacat tgttgtttgc gagcagcagc ttgcttatcg 5520
acgacgatgt gaagcgcagc aatagcagca gccccctaac cacaaacatt gacgcgtgag 5580
caaatatgta acaacaaacg cgatcggcgg gcgctgtaac tgcctgcagt cacgcgttca 5640
ttcgcagtcg ttgtcgtagt catacacacg ccgtcgttcc tttgtatcag ctgtgtagca 5700
gctagccgcc cgcgacattg acggacgtca gtgcgcaagt aagcgtcagc aacagcatca 5760
gtatgtgtgc ggcagcaagg aaacatagtc gacacatcgt tttagtggtg ttacaacatt 5820
gagctacttt ttgcgtttcg ctttcgtgct gcggcggcgg cggcgggact tcgctgcact 5880
gataggaacg gaatgcatgc aaatcaccac aatgttgtaa ctcgatgaaa aacgcaaagc 5940
gaaagcacga cgccgccgcc gccgccctga agcgacgtga ctatccttgc cttacgtacg 6000
tgctccggtt gaagagagct ctgcgccact tgtggcgggt ttcactcaaa aggcatcgtc 6060
gcgtcgcaac aaagtgcgca cattcgacgc gtaactgtaa acgaggccaa cttctctcga 6120
gacgcggtga acaccgccca aagtgagttt tccgtagcag cgcagcgttg tttcacgcgt 6180
gtaagctgcg cattgacatt gtaaatagaa agactttggt gcgtttagaa aaagggtcac 6240
aaagggtggc aagtgagtat gtatgtgagc tcatttcatt ctcgatggca ttgagacgta 6300
catttatctt tctgaaacca cgcaaatctt tttcccagtg tttcccaccg ttcactcata 6360
catacactcg agtaaagtaa gagctaccgt aactctgcat atctattctg agaacgaaag 6420
ttcaatggat gcattttatg caatgccacc ggaattttcc tatgaactgc tttcacactt 6480

```
cttttaagaa aattttgcag tagataagac tcttgctttc aagttaccta cgtaaaatac     6540
gttacggtgg ccttaaaagg atacttgacg aaagtgtgaa gaaaattctt ttaaaacgtc     6600
atttaattta ttcactccat ttagttctga cgtaacattc cagataacac acttcaaagt     6660
catggtcagt tcatgttgaa cgaatgtgca ccgcgatcca taaattaaat aagtgaggta     6720
aatcaagact gcattgtaag gtctattgtg tgaagtttca gtaccagtca agtacaactt     6780
gcttacacgt ggcgctaggt cgcagaacga ttccatgtct taatgtcgtc acttatcata     6840
taatcaccca gtttttgccc cacttaaaaa aacgatgtcc actttttatc tgagtttctt     6900
gcgtcttgct aaggtacaga attacagcag tgaatagtat attagtgggt caaaaacggg     6960
gtgaattttt ttgctacagg tgaaaaatag actcaaagaa tctcctctct tttcagccaa     7020
ccactccagc ggaacccctg aacccggaaa catggtacca ggtgagttcg ctgttgaaat     7080
actaatttgc agaaaacata agaggagaga aaagtcggtt ggtgaggtcg ccttggggac     7140
ttgggccttt gtaccatggt ccactcaagc gacaacttta tgattaaacg tcttttgtat     7200
agaaattttg ctaccgattt accataactg gaatcgaaga caatatgact tcatcacacc     7260
agcagtaaac acggcgtaaa aatgattcat caggacccgc tctttaaaac gatggctaaa     7320
tggtattgac cttagcttct gttatactga agtagtgtgg tcgtcatttg tgccgcattt     7380
ttactaagta gtcctgggcg tcaatagccc tgtttttcca cgctcatctt gggtttcaca     7440
tcggtgaaca ccacttggag acgttttcac acaatgttca tgttcttctt tgagtaaatg     7500
agttatcggg acaaaaaggt gcgagtagaa cccaaagtgt agccacttgt ggtgaacctc     7560
tgcaaaagtg tgttacaagt acaagaagaa actcatttac aagttatgcg tggtcccgtg     7620
ctcatcaaga tagtgtgcca cacataagaa ttatcttaat tgaggccttc tgcgggccgt     7680
gagcttgttt gctacgccct ttcaatacgc accagggcac gagtagttct atcacacggt     7740
gtgtattctt aatagaatta actccggaag acgcccggca ctcgaacaaa cgatgcggga     7800
tccttggcgt tgagttttag tttctttgac agagaaagac ttttgataat ctactttctg     7860
cagctacgac ctttctctga actatttgga aaattataac aggaaccgca actcaaaatc     7920
aaagaaactg tctctttctg aaaactatta gatgaaagac gtcgatgctg gaaagagact     7980
tgataaacct tttaatattg ttatgttgac aatatttatc ccttcgatta acaaaaaact     8040
tcaagccagg gaaacatcca gtgtgaaaac actaagcggc gcactttggt tcatttcatt     8100
aatacaactg ttataaatag ggaagctaat tgttttttga agttcggtcc ctttgtaggt     8160
cacacttttg tgattcgccg cgtgaaacca agtaaagtaa cgtatcgatc actcttaatt     8220
caagatgaca aagtggttga gtagtagagt acgtggctca caatcggaag gttcttggct     8280
cgaatctcaa tgtatgctat gcatagctag tgagaattaa gttctactgt ttcaccaact     8340
catcatctca tgcaccgagt gttagccttc caagaaccga gcttagagtt acatacgata     8400
```

ttttaacttt ttttttattt tgtcgatcat aaacggatgc gcgactcagc atttttggca 8460
tttgaatcat gattccgagt aatcagctac aaaaacctaa aaaattgaaa aaaaaataaa 8520
acagctagta tttgcctacg cgctgagtcg taaaaaccgt aaacttagta ctaaggctca 8580
ttagtcgatg tttttggatt cgcgtgtgtt gcgttacggc aatctgactc atgatatcat 8640
gagtccaaat catggtgtat tttcataaga cgaaaacacg ctggaatcat gatatcatga 8700
gcgcacacaa cgcaatgccg ttagactgag tactatagta ctcaggttta gtaccacata 8760
aaagtattct gcttttgtgc gaccttagta ctatagtact gtaataatct tgtttttgga 8820
ttctgatttc tacccgtgca tttctaaagt ttgcaaagaa ggaagcttca aaaaacttcc 8880
aaaagcttat gttacagaag cattattaga acaaaaacct aagactaaag atgggcacgt 8940
aaagatttca aacgtttctt ccttcgaagt tttttgaagg ttttcgaata caatgtcttc 9000
cttggaaagc ttaagttaca gcagtttccg taccagaacg ttggaaagct tatattacga 9060
aacagtaata gggtttctat gcggtggaag tgctgttata gaacctttcg aattcaatgt 9120
cgtcaaaggc atggtcttgc aacctttcga atataatgct ttgtcattat cccaaagata 9180
cgccaccttc acgacaatat tggcgtgtaa gcatttataa tacatctggg tatcatcgaa 9240
atcattagaa aaaatgcggt ataagtttca cttgaattca gatcagtgat cgattgttac 9300
accgcacatt cgtaaatatt atgtagaccc atagtagctt tagtaatctt ttttacgcca 9360
tattcaaagt gaacttaagt ctagtcacta gctaacaatg agttcaaata gatccaaata 9420
tatgagggtg aaacgtcatt gcgatccact gtgaactgca gttgattggc cgcaatttca 9480
aaatatgtac acccgagtga tcaagtttat ctaggtttat atactcccac tttgcagtaa 9540
cgctaggtga cacttgacgt caactaaccg gcgttaaagt tttatacatg tgggctcact 9600
tctgcacggc tgttcagctg acatccttca ttgtcccagt cgttcataca aacttgcccg 9660
tcaagatcaa ggaagttggc gcttgatcaa tgttctgttt agacgtgccg acaagtcgac 9720
tgtaggaagt aacagggtca gcaagtatgt ttgaacgggc agttctagtt ccttcaaccg 9780
cgaactagtt acaagacaaa catttctttt ttcttaagta gtattgggcg ctgcggtcac 9840
ctcatttatc tttttgaaat tgtttcggaa ataatgcacg agatgcaata acggttcttg 9900
gtaaagaaaa aagaattcat cataacccgc gacgccagtg gagtaaatag aaaaacttta 9960
acaaagcctt tattacgtgc tctacgttat tgccaagaac aacatagtca tgtagaacct 10020
tacaaatgat cagaattgat ttgatcaatt catttccagc tttcaaactg acgatcgccc 10080
aatgctaccg tccatcacga ttgtatcagt acatcttgga atgtttacta gtcttaacta 10140
aactagttaa gtaaaggtcg aaagtttgac tgctagcggg ttacgatggc aggtagtgct 10200
tattccacgc actggctgtc atgttccctg ccagatttac gtagtgttct tttgtaaagg 10260

caacactgct gcactgctcc aagtcactcc aagcttcatc ataaggtgcg tgaccgacag 10320

tacaagggac ggtctaaatg catcacaaga aaacatttcc gttgtgacga cgtgacgagg 10380

ttcagtgagg ttcgaagtag tgcgagttga agcaaactgt gaaggattga tattttgaat 10440

taaatcaagc tctcgcgttg caggcagctg taacttgcca ccaagtatga tcggtcttcc 10500

acgctcaact tcgtttgaca cttcctaact ataaaactta atttagttcg agagcgcaac 10560

gtccgtcgac attgaacggt ggttcatact agccagaagg gacttcgttc cataaaaagt 10620

ggaatgctcc tcgtccgatt tccagaaaca gtcggttatg caataaaaca ggatcaggtt 10680

cgatgactct tggcgatatc ctgaagcaag gtatttttca ccttacgagg agcaggctaa 10740

aggtctttgt cagccaatac gttattttgt cctagtccaa gctactgaga accgctatag 10800

tgaattggag tcgttaccta tcccccgata aagatatcct ctcgcaattc gaggggggatt 10860

aggattagaa accgtttgct gatatttgcg agatataaaa acttaacctc agcaatggat 10920

agggggctat ttctatagga gagcgttaag ctccccctaa tcctaatctt tggcaaacga 10980

ctataaacgc tctatatttt actaataaaa tcttcaattc gctaaaagca cttcaattct 11040

tgttttctct tctggtttca gttgaccccc atatgcgagt gcagcatcac ggaccggact 11100

tgattatttt agaagttaag cgattttcgt gaagttaaga acaaaagaga agaccaaagt 11160

caactggggg tatacgctca cgtcgtagtg cctggcctga caggaacagg tgcgtacttc 11220

cttaacttca ctatcaataa aaccgtacct cctccagtcc atcgaaacaa caataaaata 11280

ctgcaccgat cagctggaat gtccttgtcc acgcatgaag gaattgaagt gatagttatt 11340

ttggcatgga ggaggtcagg tagctttgtt gttattttat gacgtggcta gtcgacctta 11400

ttctatcccg ggaggtccaa tcgctacaat ttatgcacat ttaattccac tggagccatg 11460

tgcgttcggg catcttatca ggcgttcggg aattgaaact aagatagggc cctccaggtt 11520

agcgatgtta aatacgtgta aattaaggtg acctcggtac acgcaagccc gtagaatagt 11580

ccgcaagccc ttaactttga ttacgacctc atttgtcatt aacgggatgc attcgtacgc 11640

agtcagcgtc ttatcggcat atatgcggta gccccccgag tgacaattaa accatggagc 11700

aatgctggag taaacagtaa ttgccctacg taagcatgcg tcagtcgcag aatagccgta 11760

tatacgccat cggggggctc actgttaatt tggtacctcg cgaaaccaat ttcacagcgg 11820

tccaccaact accgaatgcg atgcattttt atacgacagt ggcgttacta ggtgcttaac 11880

atatcaaaac ttggaagctt gctttggtta aagtgtcgcc aggtggttga tggcttacgc 11940

tacgtaaaaa tatgctgtca ccgcaatgat ccacgaattg tatagttttg aaccttcgaa 12000

cctttcaaaa gcttgcaaag cttccttcca ggagcttgga aagcttcctt ccaggagctt 12060

ggaaagcttc cttccaggag cttggaaagc ttccttccag ggaaagtttt cgaacgtttc 12120

gaaggaaggt cctcgaacct ttcgaaggaa ggtcctcgaa cctttcgaag gaaggtcctc 12180

gaacctttcg aaggaaggtc gagcttggaa agcttccttc caggagcttg gaaagcttcc 12240

ttccagtagc ttggaaagct tccttccagg agcttggaaa gcttccttcc aggagcttgg 12300

ctcgaacctt tcgaaggaag gtcctcgaac ctttcgaagg aaggtcatcg aacctttcga 12360

aggaaggtcc tcgaaccttt cgaaggaagg tcctcgaacc aaagcttcct tccaggagct 12420

tggaaagctt ccttccagga gcttggaaag cttccttcca ggagcttgga aagcttcctt 12480

ccaggagctt ggaaagcttc tttcgaagga aggtcctcga acctttcgaa ggaaggtcct 12540

cgaacctttc gaaggaaggt cctcgaacct ttcgaaggaa ggtcctcgaa cctttcgaag 12600

cttccaggag cttggaaagc ttccttccag gagcttggaa agcttccttc caggagcttg 12660

gaaagcttcc ttccaggagc ttggaaagct tccttccagg gaaggtcctc gaacctttcg 12720

aaggaaggtc ctcgaacctt tcgaaggaag gtcctcgaac ctttcgaagg aaggtcctcg 12780

aacctttcga aggaaggtcc agcttggaaa gcttccttcc aggagcttgg aaagcttcct 12840

tccaggagct tggaaagctt ccttccagga gcttggaaag cttccttcca ggagcttgga 12900

tcgaaccttt cgaaggaagg tcctcgaacc tttcgaagga aggtcctcga acctttcgaa 12960

ggaaggtcct cgaacctttc gaaggaaggt cctcgaacct aagcttcctt ccaggagctt 13020

ggaaagcttc cttccaggag cttggaaagc ttccttccag gagcttggaa agcttccttc 13080

caggagcttg gaaagcttcc ttcgaaggaa ggtcctcgaa cctttcgaag gaaggtcctc 13140

gaacctttcg aaggaaggtc ctcgaacctt tcgaaggaag gtcctcgaac ctttcgaagg 13200

ttccaggagt ggaaaagatt cctgaaaagt acttggagaa attcctcgag ttatttcagt 13260

aaagattata ctggaggaac caatggtgga atcacttgag aaggtcctca ccttttctaa 13320

ggacttttca tgaacctctt taaggagctc aataaagtca tttctaatat gacctccttg 13380

gttaccacct tagtgaactc gcatttcggc agaaatccct ggcaaaatcg ctatggaaaa 13440

atccctgcaa aaaatcctgg aataatcctt gccggaatct catgaggaac tcctggtaaa 13500

cgtaaagccg tctttaggga ccgttttagc gataccttttt tagggacgtt ttttaggacc 13560

ttattaggaa cggccttaga gtactccttg aggaccattt attctttaac aaatttctgt 13620

ttattttctc tacaaagtta cagctccttt accgtgccga ttggccagaa atgaccccaa 13680

agactcatgg ggtacgatct taagaaattg tttaaagaca aataaaagag atgtttcaat 13740

gtcgaggaaa tggcacggct aaccggtctt tactggggtt tctgagtacc ccatgctaga 13800

tatttctgcc aaatatactg tatgtttgtt tctttctgat atgcttttaa gctcaatttt 13860

ctttggaatg gtggagattt gttttggcct ccaatatact ataaagacgg tttatatgac 13920

atacaaacaa agaaagacta tacgaaaatt cgagttaaaa gaaaccttac cacctctaaa 13980

caaaaccgga ggttatatga tgctagctcg tagttcgtac ctgaagtcaa ctcctcaatt 14040

cctaaatgct acaataatat ataaaatttt aggaaataac tgcaaaatat tctgaaggcc 14100

acgatcgagc atcaagcatg gacttcagtt gaggagttaa ggatttacga tgttattata 14160

tattttaaaa tcctttattg acgttttata agacttccgg atgtcttgat ctatcttgat 14220

gtatctaata tgtaatccca gaagcattct agttttttct gataatctgt gaaataagtt 14280

gtttttacga actttgactt tacagaacta gatagaacta catagattat acattagggt 14340

cttcgtaaga tcaaaaaaga ctattagaca ctttattcaa caaaaatgct tgaaactgaa 14400

ttcgggattt gaggtacaag ctttcaaata tattggaggt tctgcgatat taacttcaat 14460

gaattattgg aaattagaaa tcgtcttgtg catacgggtt aagccctaaa ctccatgttc 14520

gaaagtttat ataacctcca agacgctata attgaagtta cttaataacc tttaatcttt 14580

agcagaacac gtatgcccaa aatcgatttt agtctctggt agatttcgag agggaatgtc 14640

tgaagaaatt ttctgaccta catgtgaagt attgtctgtc aaattcaaaa tattttctgt 14700

ttagctaaaa tcagagacca tctaaagctc tcccttacag acttctttaa aagactggat 14760

gtacacttca taacagacag tttaagtttt ataaaagaca aggaaattaa aattttttgg 14820

ggaaaactcg aaactccttg gatatccaag gaaacaaaaa aaaaagaaat atctgaagaa 14880

gtgcatcgtc ctttttcctt tcctttaatt ttaaaaaacc ccttttgagc tttgaggaac 14940

ctataggttc ctttgttttt tttttcttta tagacttctt cacgtagcag gaaaaaggaa 15000

aattattgtt ttaattaact aatagttctg ctagaaaggt ttttggcaga accccaaaat 15060

gatattcaaa gcaactaaca gctcgatttc ccctcgtttc ttaataacaa aattaattga 15120

ttatcaagac gatctttcca aaaaccgtct tggggtttta ctataagttt cgttgattgt 15180

cgagctaaag gggagcaaag caatttcaga cgacgaactt gtcaaacgat ctcaatggct 15240

cctggagaag ctgcgatacc cctgggagat gatgcccctg atgtacgtga tactgaaagg 15300

gttaaagtct gctgcttgaa cagtttgcta gagttaccga ggacctcttc gacgctatgg 15360

ggaccctcta ctacggggac tacatgcact atgactttcc cgccgacgga gacgtcaata 15420

aagcgcgcca acggattgac gaaggtatgg gggttcttac cggttgggac tgtttccgag 15480

gtatcgatcg ggtgtcactc gcggctgcct ctgcagttat ttcgcgcggt tgcctaactg 15540

cttccatacc cccaagaatg gccaaccctg acaaaggctc catagctagc ccacagtgag 15600

acttcctggg tgctcccatt ttgtaactgc taacgcttat tattgagttt caggacatct 15660

gggatcttcg gtcgacggag tctattccca acagtgccct tgaaggaccc acgagggtaa 15720

aacattgacg attgcgaata ataactcaaa gtcctgtaga ccctagaagc cagctgcctc 15780

agataagggt tgtcacggga ggatcaaaca ctgccatcat gcagtttccg tagcctgttg 15840

ggctacgctc cccgacttga catcccccat tcttatcaaa caacaactca aggcctgaga 15900

cctagtttgt gacggtagta cgtcaaaggc atcggacaac ccgatgcgag ggctgaact 15960

gtagggggta agaatagttt gttgttgagt tccggactct caacgagtgg tggaatttgc 16020
gcacgaagtc attggtttgt cctggtaaaa gttaaaaggg ttaactggag ggttaattga 16080
cacggtttca actgatggcc gttgctcacc accttaaacg cgtgcttcag taaccaaaca 16140
ggaccatttt caattttccc aattgacctc ccaattaact gtgccaaagt tgactaccgg 16200
ttattgacac acggatgaaa gacttgcacg cttgaccttc tgtctgtact aataaaagtt 16260
acgttggctg ggttttgggg tcataatggc cccaaaatcg aataactgtg tgcctacttt 16320
ctgaacgtgc gaactggaag acagacatga ttattttcaa tgcaaccgac ccaaaacccc 16380
agtattaccg gggttttagc aatcgtcata acttcttgaa atacaactca cgtttaagac 16440
cattcaagag tattagatca tcgtctataa tagcagattt gaaatttact tcacatttcg 16500
ttagcagtat tgaagaactt tatgttgagt gcaaattctg gtaagttctc ataatctagt 16560
agcagatatt atcgtctaaa ctttaaatga agtgtaaagc gtattgcagt gccccttgct 16620
tccacaatgg aattagttaa agtttcgaga gcattgtcaa tatcaagtgt tgttagcaaa 16680
caaatgctaa catcaagatt cataacgtca cggggaacga aggtgttacc ttaatcaatt 16740
tcaaagctct cgtaacagtt atagttcaca acaatcgttt gtttacgatt gtagttctaa 16800
actatcgatg tttgattcac atgtattcca atcagctcgt aaaaaatgga aagtggagct 16860
gatagggttg agaatcgctt catgggataa ttggaaacag tgatagctac aaactaagtg 16920
tacataaggt tagtcgagca ttttttacct ttcacctcga ctatcccaac tcttagcgaa 16980
gtaccctatt aacctttgtc ggacatgatc agaatgaaaa tcagcgtgag taaccagttg 17040
actacaaaga tgactagagt cggttaagaa aaattcaagt agggctatca ggttattgaa 17100
cctgtactag tcttactttt agtcgcactc attggtcaac tgatgtttct actgatctca 17160
gccaattctt tttaagttca tcccgatagt ccaataactt ttgaaaaata tcccgaaggg 17220
ccctcatcaa ttaaaatttt gcctttggaa atgtttggca ttcaagtagc aaattttaac 17280
atactgcgat tcgatttccg aactttttat agggcttccc gggagtagtt aattttaaaa 17340
cggaaacctt tacaaaccgt aagttcatcg tttaaaattg tatgacgcta agctaaaggc 17400
caagttagtt tgaaacaaat taacttgcta cccagtgcat taaaaaggca agtaggcagc 17460
tttggaagta taaacttagc tgtgttttaa cagaagcact gttcaatcaa actttgttta 17520
attgaacgat gggtcacgta atttttccgt tcatccgtcg aaaccttcat atttgaatcg 17580
acacaaaatt gtcttcgtga cgcaagtttc aaaaattttg gtttcgaatg acaaaaaaag 17640
ttgatgttat atacgcctat tgaatgatga ttccagttga tcatttcgac aaacaaaaaa 17700
gcgttcaaag tttttaaaac caaagcttac tgttttttttc aactacaata tatgcggata 17760
acttactact aaggtcaact agtaaagctg tttgtttttt gaatctcttt tgatttcaga 17820

tccaggattc aaataacatt ccgttatcag ataaagggtt aatgccacaa tcgtgtggtc 17880

cattatcccc ggaaacttca cttagagaaa actaaagtct aggtcctaag tttattgtaa 17940

ggcaatagtc tatttcccaa ttacggtgtt agcacaccag gtaatagggg cctttgaagt 18000

caccgtcaca ctcgatccag atctgatgtg atctctgccg tcgggcgcct cagaagcgaa 18060

aaccacattc gcccgcgctc tccggaatta tgtcgtaaaa gtggcagtgt gagctaggtc 18120

tagactacac tagagacggc agcccgcgga gtcttcgctt ttggtgtaag cgggcgcgag 18180

aggccttaat acagcatttt taaaacttta caaccataat tattcagaac ttcgacgact 18240

gcgcgatgac ttggccgcgg tgtgcctgct tgggatggac ctccgagcac tgaaagcagt 18300

attttgaaat gttggtatta ataagtcttg aagctgctga cgcgctactg aaccggcgcc 18360

acacggacga accctacctg gaggctcgtg actttcgtca ggtttgtaca aattgaatgg 18420

gctatttgaa attaattggg ctgcgataac ttcaaagtgt gacatcaaaa tggtgtgagt 18480

tttttactgc acaaattcca ccaaacatgt ttaacttacc cgataaactt taattaaccc 18540

gacgctattg aagtttcaca ctgtagtttt accacactca aaaaatgacg tgtttaaggt 18600

agttatttcc tacttcatat caatcggagc tccaggagtg aagatccaaa ttaccaagct 18660

tggccatttc gtatgaaaaa cggcaaaatg atcttttttt tcaataaagg atgaagtata 18720

gttagcctcg aggtcctcac ttctaggttt aatggttcga accggtaaag catacttttt 18780

gccgttttac tagaaaaaaa cgccagtcac tgtatctcat gatccagatg agataaaaaa 18840

gttcgagtct tcgacaaagt tgttttggaa gtcatggaca ttcttaagca aacaacttag 18900

gcggtcagtg acatagagta ctaggtctac tctatttttt caagctcaga agctgtttca 18960

acaaaacctt cagtacctgt aagaattcgt ttgttgaatc ttttgccact aggtggcgcc 19020

agtaagcata ttcgtcatca aacgtcaaca tcccaccgca aaatcgctag tgtttggagg 19080

ggattttaac ctccaaattg aaaacggtga tccaccgcgg tcattcgtat aagcagtagt 19140

ttgcagttgt agggtggcgt tttagcgatc acaaacctcc cctaaaattg gaggtttaac 19200

ccaaataacc tccaaatcat cacctccaag ttagttctaa tacactccgt tatatgaaat 19260

atggtggtgc gtcgatcgtc gcaagtttat cgttaaacag ggtttattgg aggtttagta 19320

gtggaggttc aatcaagatt atgtgaggca atatacttta taccaccacg cagctagcag 19380

cgttcaaata gcaatttgtc tcaataaaat gagcatttta tatcgtgata catatgagaa 19440

gatagaggtt tcaattaaaa caaatccaca tggtgtcgct aataaaattg tgcattttaa 19500

agttatttta ctcgtaaaat atagcactat gtatactctt ctatctccaa agttaatttt 19560

gtttaggtgt accacagcga ttattttaac acgtaaaatt gcgagttata tcctctgatc 19620

aagataaaat agaaaattcg atttttgaat attcaattat aagagcctga ataactacaa 19680

catgtagtga atcgaaactg cgctcaatat aggagactag ttctatttta tcttttaagc 19740

taaaaactta taagttaata ttctcggact tattgatgtt gtacatcact tagctttgac 19800
atttatgacg gtttgtgaag gttacacgtc ctaagcattt ggattcaaga aaagcaagag 19860
atatgacgaa tgtaaacttt atcgtatcaa tgaagtaact taaatactgc caaacacttc 19920
caatgtgcag gattcgtaaa cctaagttct tttcgttctc tatactgctt acatttgaaa 19980
tagcatagtt acttcattga agcgtccaga acagtacaaa ccaacatcgt accgtcgtat 20040
tccactccgg tcgttgcaat atctctaggt ccaccgaaaa acactcatga ccaagatcgt 20100
tcgcaggtct tgtcatgttt ggttgtagca tggcagcata aggtgaggcc agcaacgtta 20160
tagagatcca ggtggctttt tgtgagtact ggttctagca gtcgtcgatc ttggtccacc 20220
gaaacaccga tgtccatatc gtttcgtcga acttggacca acgattcatg caactgatga 20280
caacgcggcc cccgggtcgt cagcagctag aaccaggtgg ctttgtggct acaggtatag 20340
caaagcagct tgaacctggt tgctaagtac gttgactact gttgcgccgg gggcccagca 20400
accaatatcc gaaaaatcca actgttcttc tctgcctcgc aggtcaagcc gtggtcaatg 20460
aatactcacg attgcacaat ctgaacatgt tcgacggtgt tggttatagg ctttttaggt 20520
tgacaagaag agacggagcg tccagttcgg caccagttac ttatgagtgc taacgtgtta 20580
gacttgtaca agctgccaca agagttgcgc agtacgacgc gccagtccgg atgatagact 20640
ttttacacga tcagcacgac ccactgcgct gcggcaaagg tcgaaccgaa acaagaataa 20700
tctcaacgcg tcatgctgcg cggtcaggcc tactatctga aaaatgtgct agtcgtgctg 20760
ggtgacgcga cgccgtttcc agcttggctt tgttcttatt accacgaaga tcagatcgat 20820
tcgacggaag aagcaatcga atgcaaagaa gaatcggaac gaagaaaact ctaaagcatc 20880
gcatatttac aaagcataac tggtgcttct agtctagcta agctgccttc ttcgttagct 20940
tacgtttctt cttagccttg cttcttttga gatttcgtag cgtataaatg tttcgtattg 21000
ggaaaacccg caagttcaaa ctagtgatta gtgtaagatg aagcaaagca gaaatgtagt 21060
atctagattt ttcgacgtta gtttacaaag ataaaaaatg ccttttgggc gttcaagttt 21120
gatcactaat cacattctac ttcgtttcgt ctttacatca tagatctaaa aagctgcaat 21180
caaatgtttc tattttttac aggttggaca tacaatcgtg ggtattcgtc tgagttcgtc 21240
acaactgcac cggaaactgt gaaacagaat agagccaacc tgtgcgcgga gaatgttgag 21300
tccaacctgt atgttagcac ccataagcag actcaagcag tgttgacgtg gcctttgaca 21360
ctttgtctta tctcggttgg acacgcgcct cttacaactc gtcattataa gcttccttag 21420
catccacggg tgaaagtcga tcgacggaag cctgcaagac tctgtcgatg ggctttcgtc 21480
ctagaagaat aagattaaac cagtaatatt cgaaggaatc gtaggtgccc actttcagct 21540
agctgccttc ggacgttctg agacagctac ccgaaagcag gatcttctta ttctaatttg 21600

ctgaaatgta ttctcccgtg gaatggtttc atttgagtaa ttctgtatct tctccttccc 21660
aattccacga acgcgacgaa ctctaataca aacaacataa gactttacat aagagggcac 21720
cttaccaaag taaactcatt aagacataga agaggaaggg ttaaggtgct tgcgctgctt 21780
gagattatgt ttgttgtatt tgaccacagt gcaaatgctg tttaacgata atagcgacat 21840
gcagccattc tggggctacc acgtgtagct ctacttgtga gacagcgttc ctaaagagtg 21900
actggtgtca cgtttacgac aaattgctat tatcgctgta cgtcggtaag accccgatgg 21960
tgcacatcga gatgaacact ctgtcgcaag gatttctcac tgaaagtgca aacaagtgat 22020
gaaaccaata gtgcaaagca agtttagagg gaaaatttaa aaaatgcaaa acagcagtag 22080
tacttaactt ttaagattgt actttcacgt ttgttcacta ctttggttat cacgtttcgt 22140
tcaaatctcc cttttaaatt ttttacgttt tgtcgtcatc atgaattgaa aattctaaca 22200
gtttcgaaag ccgaagtgtg ttccatctgc caccggaaaa aaacgacgac agcagaatca 22260
tcaacaagca acatccatcc gaaaaaatcc gggaaaccgg caaagctttc ggcttcacac 22320
aaggtagacg gtggcctttt tttgctgctg tcgtcttagt agttgttcgt tgtaggtagg 22380
ctttttttagg ccctttggcc atcttcaacc aaccatccta caatctacaa accagagatt 22440
atatctcttc aatcgtttcc gacatcggtc ggtttcggtg cccaaaatga tctgataaac 22500
tagaagttgg ttggtaggat gttagatgtt tggtctctaa tatagagaag ttagcaaagg 22560
ctgtagccag ccaaagccac gggttttact agactatttg acttatctct ctgtagcttg 22620
catgccattg cgagcgtatt ttggtagctg gccgttgcca aacggctccg acaggtactg 22680
ctattggagg ttgtgcacga tgaatagaga gacatcgaac gtacggtaac gctcgcataa 22740
aaccatcgac cggcaacggt ttgccgaggc tgtccatgac gataacctcc aacacgtgct 22800
ccacgttgag tttgcctttt gagttggaga gtgtgtcttt tcgtcatata tttggccttt 22860
tcaagggtga ttttcaggct gcgtaaagat tgtatagttt ggtgcaactc aaacggaaaa 22920
ctcaacctct cacacagaaa agcagtatat aaaccggaaa agttcccact aaaagtccga 22980
cgcatttcta acatatcaaa aaccagctaa aacatattga tgacaagttc tatttcagca 23040
ccacaaacaa gcctgttaat gtctctcacc gcaaccattg ttctgcgcgc gttataatca 23100
ttggtcgatt ttgtataact actgttcaag ataaagtcgt ggtgtttgtt cggacaatta 23160
cagagagtgg cgttggtaac aagacgcgcg caatattagt gcatagaagt ttattttctt 23220
tgggatgatt caaatattac gtgacgcaaa gtttgccaat tttagaaccc ctccctcctc 23280
cacgtaacgg cttttgtgtg cgtatcttca aataaaagaa accctactaa gtttataatg 23340
cactgcgttt caaacggtta aaatcttggg gagggaggag gtgcattgcc gaaaacacac 23400
aaaaatttaa attttgtgta tagaccgtag catttcggaa gaccccctcc cttactctgt 23460
tgagttacgt aaaatttcaa cgatcctttt gtagttctga tttttaaatt taaaacacat 23520

atctggcatc gtaaagcctt ctgggggagg gaatgagaca actcaatgca ttttaaagtt 23580

gctaggaaaa catcaagact attttatatc agcgtgcagt gttatgaaga tatccacagt 23640

ataaaatatt attttatttt aaattctatg ctgattatca atgtgttact agtggctttt 23700

taaaatatag tcgcacgtca caatacttct ataggtgtca tattttataa taaaataaaa 23760

tttaagatac gactaatagt tacacaatga tcaccgaaaa catactcatg ttgcgagctc 23820

gatttggcgc acggggtcat ctacacctga tacctttagg gtcgttgggg gaccacttag 23880

cgtgcacgta cggacattca gtatgagtac aacgctcgag ctaaaccgcg tgccccagta 23940

gatgtggact atggaaatcc cagcaacccc ctggtgaatc gcacgtgcat gcctgtaagt 24000

aaatgttgtt caaatttttt tcttaccaag acgagcactt tacaatgaca aactctggct 24060

ctgctctggc tctgctctgg ctctgctctg gctctgctct tttacaacaa gtttaaaaaa 24120

agaatggttc tgctcgtgaa atgttactgt ttgagaccga gacgagaccg agacgagacc 24180

gagacgagac cgagacgaga ggctctgctc tggctctgct ctggctctgc tctggctctg 24240

ctctggctct gctctggctc tgctctggct ctgctctggc tctgctctgg ctctgctctg 24300

ccgagacgag accgagacga gaccgagacg agaccgagac gagaccgaga cgagaccgag 24360

acgagaccga gacgagaccg agacgagacc gagacgagac gctctgctct ggctctgctc 24420

tggctctgct ctggctctgc tctggctctg ctctggctct gctctggctc tgctctggct 24480

ctgctctggc tctgctctgg cgagacgaga ccgagacgag accgagacga gaccgagacg 24540

agaccgagac gagaccgaga cgagaccgag acgagaccga gacgagaccg agacgagacc 24600

ctctgctctg caaaatgctc tggattaatt tattgctcac actcttttgc tgttggacca 24660

ctattcattt caaatcttca atatgttcct attacccccca gagacgagac gttttacgag 24720

acctaattaa ataacgagtg tgagaaaacg acaacctggt gataagtaaa gtttagaagt 24780

tatacaagga taatgggggt aacacggtcc acacggatcg atttcaacta actccactct 24840

cgtatgcata ttttgtgtat aaattttgaa taatcgaaaa gggttgctgc aaatgttaat 24900

ttgtgccagg tgtgcctagc taaagttgat tgaggtgaga gcatacgtat aaaacacata 24960

tttaaaactt attagctttt cccaacgacg tttacaatta attttttccc tctacccccct 25020

cactctgtcg ttggcgttgg aaaaaaatca ccactgcata caaaacactc attggttggg 25080

tggaaggacg gtttagcaga taaaaaaggg agatggggga gtgagacagc aaccgcaacc 25140

tttttttagt ggtgacgtat gttttgtgag taaccaaccc accttcctgc caaatcgtct 25200

gttgctaaat tttccatatc acgctgattg atttgtgatt aaaaataaat ataaatagaa 25260

aatgaataat tcccacatgt gtttcggtat taggcaccgg caacgattta aaaggtatag 25320

tgcgactaac taaacactaa tttttattta tatttatctt ttacttatta agggtgtaca 25380

caaagccata atccgtggcc catggggcgg cgaagtgcag acggttctag ttctcattat 25440

ttggcatcga ttggcggtca aactacaacc tccatggaga aacaggcccc atccgtactt 25500

gtaccccgcc gcttcacgtc tgccaagatc aagagtaata aaccgtagct aaccgccagt 25560

ttgatgttgg aggtacctct ttgtccgggg taggcatgaa agttattaat aaataacaat 25620

gatttgaatt tgaatcattc atgctgcggc gtggctgatt tcggtgaatt gttgttctct 25680

tagagaaaga gggggatttg tcaataatta tttattgtta ctaaacttaa acttagtaag 25740

tacgacgccg caccgactaa agccacttaa caacaagaga atctctttct cccccctaaac 25800

aatttggacg agtaaataac attgaatatt acactttatg actaatcacc agtaatgaaa 25860

caacacgggt gatgatttca aaagcttcat tctaaatgca ttaaacctgc tcatttattg 25920

taacttataa tgtgaaatac tgattagtgg tcattacttt gttgtgccca ctactaaagt 25980

tttcgaagta agatttacgt tggttcactt ttggtggcag atttaaaact cttatcttcc 26040

tcttttcttc aacaggtttc acgccatcaa agacgcttgg cagccgcttc catttgcgta 26100

accaagtgaa aaccaccgtc taaattttga gaatagaagg agaaaagaag ttgtccaaag 26160

tgcggtagtt tctgcgaacc gtcggcgaag gtaaacgcat gcaaacgtat gttaacctta 26220

ggttttaatg ttaaaagtat caccaaaaat caagtcccaa gacttctgca agaatggttt 26280

atgctgaatt tattcgaaat cgtttgcata caattggaat ccaaaattac aattttcata 26340

gtggttttta gttcagggtt ctgaagacgt tcttaccaaa tacgacttaa ataagcttta 26400

ggttttattt tcatcgaaac atgtgtgatg taggctacta ttttggtaaa accgttggca 26460

acgactgtat ttaaactcac aaaatttgaa ccaaacttat ccaaaataaa agtagctttg 26520

tacacactac atccgatgat aaaaccattt tggcaaccgt tgctgacata aatttgagtg 26580

ttttaaactt ggtttgaata aattgtaact tttaattgag taaacatagg cgaaagagag 26640

tgattcaaat gggattcgga atcgaacggt tcttctaagt aagacaaacg aaaaaaacaa 26700

ttaacattga aaattaactc atttgtatcc gctttctctc actaagttta ccctaagcct 26760

tagcttgcca agaagattca ttctgtttgc tttttttgtt ccaaacgagt caaagctgca 26820

aaaacttcaa gtttgaactg tgatatcaat gaaattaaat acgaactatg tatcaagatt 26880

acagtaaaat ttaaagaaga ggtttgctca gtttcgacgt ttttgaagtt caaacttgac 26940

actatagtta ctttaattta tgcttgatac atagttctaa tgtcatttta aatttcttct 27000

ctttcaacgc atgaaacagg agggtggcaa ccgaaaagtg actgaatcaa ttgcgggtta 27060

tcattcgaga tatccagggg ttgaattgtg agaaaacttc gaaagttgcg tactttgtcc 27120

tcccaccgtt ggcttttcac tgacttagtt aacgcccaat agtaagctct ataggtcccc 27180

aacttaacac tcttttgaag ttcttcttct tattcttggc aatacgtcct cactgggata 27240

gagtctgctt cctaacttca tgttcaatga ccacttccac agttattaac tgagagcttt 27300

aagaagaaga ataagaaccg ttatgcagga gtgaccctat ctcagacgaa ggattgaagt 27360

acaagttact ggtgaaggtg tcaataattg actctcgaaa ctttgccaaa gttgccattt 27420

tcgcattcgt atatcgtgtg gcagcagtgt tgtgaaaaac tcaatttctc ataactaacg 27480

cttgagattt ttcatgcgtg gaaacggttt caacggtaaa agcgtaagca tatagcacac 27540

cgtcgtcaca acacttttg agttaaagag tattgattgc gaactctaaa aagtacgcac 27600

agttgtcaat cacgcaactc agcagtcaaa attttccaca gtatacttac acacggcaat 27660

aatttcttgc tagtctggta aaattatagt aatcttttct tcaacagtta gtgcgttgag 27720

tcgtcagttt taaaaggtgt catatgaatg tgtgccgtta ttaaagaacg atcagaccat 27780

tttaatatca ttagaaaaga aacgtaaaca acaaaattcg ggtttcaaga gtttttgacg 27840

ggagcaagca aaataggatt tagaattttg catgagacga agtttgaaaa ttttattgtc 27900

ttgcatttgt tgttttaagc ccaaagttct caaaaactgc cctcgttcgt tttatcctaa 27960

atcttaaaac gtactctgct tcaaactttt aaaataacag aaatttagta tcggttcaat 28020

cgaattttcg aacacaattg taggctctat ataaactaca tttattccct tattttgcca 28080

gatacaatac tcgcataact tttaaatcat agccaagtta gcttaaaagc ttgtgttaac 28140

atccgagata tatttgatgt aaataaggga ataaaacggt ctatgttatg agcgtattga 28200

tgagatctcg cctaaaaagc cattggtaac cgagtgtgta gctctttgtt tctaagccaa 28260

ttaatggacc tggatgaaaa ctatcatcac tgggaaatag actctagagc ggatttttcg 28320

gtaaccattg gctcacacat cgagaaacaa agattcggtt aattacctgg acctactttt 28380

gatagtagtg accctttatc aggaggaact tgtctttatc gtagcattgt taaataacgt 28440

gtaaacccat ttgtttcctc ggtagctgca agctacacac tcgattacca atggctttta 28500

tcctccttga acagaaatag catcgtaaca atttattgca catttgggta aacaaaggag 28560

ccatcgacgt tcgatgtgtg agctaatggt taccgaaaat gggcgagatc acaagttatg 28620

cgagaatact tcccgaaatc accacctttt acccttttaa ataacgaaat tactacaaac 28680

ttcgttaccc gctctagtgt tcaatacgct cttatgaagg gctttagtgg tggaaaatgg 28740

gaaaatttat tgctttaatg atgtttgaag caat 28774

<210> 44
<211> 3399
<212> DNA
<213> Cydia pomonella

<220>
<221> misc_feature
<222> (1179)..(1184)
<223> n is a, c, g, or t

<400> 44

```
catcagacgg gcccaggctc aggatgaagc tagagcgcgg gcggcggacg cagggctcca      60
ccctcccggg atcgagctag atcggcctga gccgccagtg gtgaaagcgc cgaggagtcc     120
cgtgatcccg ccgccgccgc cgcgctccat gggatcggcg agctgcgact ccgttccggg     180
atcgcccggg gtatcgccgt atgcgccgaa cccgccgtcc gctccgcctc cgccgatgcc     240
gccgctcccg cctccgcaac cagtggccct ggactccctg gtagaaaact gccacaagct     300
gctggaaaaa ttccactaca gttgggagat gatgccgctc gtgctggtca tcctcaacta     360
cgccggctcc gacctggagg aggcctcgcg gaagattgac gaaggtaagt ttaaatttaa     420
gtacataaca atgcttacag acgaattgaa agggaatgtg actcggctaa tccaccagga     480
tataattttg tagagtgcgc taaagaattc tagcaacgga cgctgttatt ctgccaccgc     540
cgttgatgcc gccgtcttct gatagtgata ctttaagatc cgtatactac gctcacttcc     600
attcacttat gtcgtacgga gtattaatat gggtaaactc gcggacacga aacgattacg     660
aaaacgcaga gtacttagat tggagcaaag cccagggatt cgccgagact tttttgttac     720
ggaagattga tgaaggtaag caagttggga ctgtggcgag ttgacacatg aaacaagtca     780
aggtcacagc tggagttcca ttaaagctgg atgctaccgc tagtcatcct gaggccggct     840
ccgacttcgt gcaatgaggt attaagctgc tggaattgaa tggaatatag tggtgaaaca     900
ctactactag gtttaagcgt ttagttatat ggttgttttc ttatttttaa tttttaaatg     960
ctctgctaag ctaaaacggc waatgtctat ttttgattat aaagacttat ataaaacaac    1020
ttgtttagct tctttkacgt ctttttgtta agctgtgccc tggttttaaa wkgggcgaac    1080
acytcacgaa taagacgtaa ttttaaaaag aaaatagata tcggccctct tggttcgcat    1140
ttatacatat gtattgctgc ccgtgcgaat gttggggann nnnnaaacag tacccctagt    1200
gtaartaaat tcgatttcga aacgtgacgt acgcgtttgc gtttagtctc mwtttgtatt    1260
ggatttagaa agagcgcgcc aagcgggacg ttttggaaac tcaaaatcct atacaaaatg    1320
agacttaacg caaasgcgtt tcgtcacgtt atgatgtcga tcaaatttac actaggggta    1380
cagaggtatt gcagtaactg tacaaatact aaactaaatt aataaattag ctaaatctaa    1440
aatataccct tcaggcattg tactaaggat gctggcggaa ttacttgtgc gaggaagccg    1500
ccagcttttc ggtcaccatt tacgagtacg tataccaaac gcttcgttgc tgcaaaaaag    1560
tttcaacgcc aaatggtaca aaatgcttta tattgttctc tatatattat attaacacat    1620
cgttatttta acctaggtct tagttatgta caaggttaca taaaatagat gttcctagtc    1680
cattcctccg tgtatgttgt gtctattata aagcaaggct gcattttgta atcagtcaat    1740
ttcaatataa aaaagttgca tcgttttttt ttactkttcg acaattaaat tcaagtagca    1800
aaaaataacc caccttaatt tgtcatggtc ataatgaaac aatgacaarg ttttttttat    1860
```

cgcccgatac atgtacgtgt tctccaaaat gcagtctccg cgccgccaag cgaacgttca 1920

aactgtgcga tttccgttgt ccccaggcaa aatgatcatc aacgattacg ccaggaagca 1980

taatctgaac atcttcgacg ggctcgagct gaggaactcg acacgccact ccatttcgga 2040

tggcgatgaa aaacgcccac cgcaacctaa gcaagtctca aagtaaggtt ccatttaaat 2100

catctcaaaa ccgttagaaa cactcaaaaa gaaaccaaaa ttctgttcgg aaaccgacct 2160

ttgtttttta cacacactta gaccgaattt gcaaatttta acccccttatt cctaaaacta 2220

gcaatggtaa gctcggctga atttcacata caaacggagt ttcgttctca ttataaaact 2280

gcgtgttgga ttgtaatgga actttgcaca tacaatgaca tgaggtatgt ctagggctga 2340

aattagttta tacttggtat ctgaggctac ataaactaat tacagcctta gacttggagg 2400

atttaacaac tggaaacacc ttgtctgtaa ttctctgtac aacgatttta cgggggagga 2460

gcaaatatgt cagttaaacg tcagtccaaa caatacatat gactattggc cgtggtattt 2520

cgacggaggg gtaataagct cttaaaggcg actccgatat gcctaatcct attgttagta 2580

caaagtttca gagcaattta gctagtcgtt ttaaaatgag agcgtaacta cgttagcttg 2640

ctcttcttcc tcctgctctt atcccacgtt atgtggggtc ggcacaacat gttcctctct 2700

tctcactcct ttctttctca tatcctcttt cacacaatcc atccatcgtt tacttacaac 2760

cgagcttgct ggggaccgtt aaggcgccgc gagttcaggt tcttctctca ctctcactct 2820

cactggtgtg agcggagcga gacagcgttt tattttcgcc ttatcgaggt tccactgtat 2880

tataaataac ttacatttat aaagacgctg taatcgataa gaagttgagt cacgcttacg 2940

tcgcttacgt actacgtata gtaacgtagc ctgccgttta caaacaatgt acggagctac 3000

aacgttgcaa gttcggtccc cacacaacac aatgtgtcat aacacattaa caacattgtt 3060

acacacccac acatacaaat ttgctaagtt gataaaagag tggtgtgtcc gacgaatcag 3120

aacatcacta acccagtcgt gatttcattt ccacagtgac cggacgaagg tggagaagtt 3180

cgaaatttaa aaaaagtgac cacattttat ttaatagtga tgtgcaagtg atactatttt 3240

tattttgttt ttcttttgta ggaaaatgct gagcgaaata aataatttta gtggtgtgct 3300

atcgtcatcg atgaagttgt tttgcgaatg atactatgtt cttcaagtgc tgtgttttgt 3360

ggactgtggg gtgactgttc ctgtaaataa gcttcgttg 3399

<210> 45
<211> 996
<212> DNA
<213> Cydia pomonella

<400> 45
catcagacgg gcccaggctc aggatgaagc tagagcgcgg gcggcggacg cagggctcca 60

ccctcccggg atcgagctag atcggcctga gccgccagtg gtgaaagcgc cgaggagtcc 120

```
cgtgatcccg ccgccgccgc cgcgctccat gggatcggcg agctgcgact ccgttccggg      180

atcgcccggg gtatcgccgt atgcgccgca cccgccgtcc gctccgcctc cgccgatgcc      240

gccgctcccg cctccgcaac cagtggcctt ggactccctg gtagaaaact gccacaagct      300

gctggaaaaa ttccactaca gttgggagat gatgccgctc gtgctggtca tcctcaacta      360

cgccggctcc gacctggagg aggcctcgcg gaagattgac gaagcctcct gggtggtgca      420

ccagtggcgg ctgtacgagc gctcactgtg ctcgctgctg gagctgcaag cgcgcaaaga      480

gtcgttttgc tgctcgccgc gctatgtgct gtcgcgcgag tacgcgccgc acctgcccgt      540

gccgctcatg cgctcgccgc cgccagcgca cttgtagccc cacaccgcgc cgcgacagac      600

ggcgcacgag cccactgagc catctacttc ggccaaaccc gagtaggccc gaggccgacc      660

cgagcccgac ccgagaggac ccgagtgggc tattccggac tttacctagt tttatatgtg      720

ctatacgtgt tacaacacgc atatttgtat attatcacgg acattaagtt ggagagcggt      780

taccttatct tgttaacccg gtccttgaag taattattcc cagatatatt aagaaaacca      840

gtgaatactt tgcctgatgt ataattaaca gttgttaagc aaccatgaga attatggtat      900

ttcttgtgga catgttgcag ctagaaattt catatcatcg gtgataaaat ttaaccacac      960

tgtggttggc ggaaaaccac attgtttgta atattg                                996


<210>  46
<211>  6751
<212>  DNA
<213>  artificial

<220>
<223>  Sequence of pLA3435-Bombyx mori-dsx construct/plasmid.


<220>
<221>  misc_feature
<222>  (1617)..(1622)
<223>  n is a, c, g, or t

<400>  46
ggccgcatgg tacccattgc ttgtcattta ttaatttgga tgatgtcatt tgtttttaaa       60

attgaactgg ctttacgagt agaattctac gcgtaaaaca caatcaagta tgagtcataa      120

tctgatgtca tgttttgtac acggctcata accgaactgg ctttacgagt agaattctac      180

ttgtaatgca cgatcagtgg atgatgtcat ttgtttttca aatcgagatg atgtcatgtt      240

ttgcacacgg ctcataaact cgctttacga gtagaattct acgtgtaacg cacgatcgat      300

tgatgagtca tttgttttgc aatatgatat catacaatat gactcatttg tttttcaaaa      360

ccgaacttga tttacgggta gaattctact tgtaaagcac aatcaaaaag atgatgtcat      420

ttgtttttca aaactgaact cgctttacga gtagaattct acgtgtaaaa cacaatcaag      480

aaatgatgtc atttgttata aaaataaaag ctgatgtcat gttttgcaca tggctcataa      540
```

ctaaactcgc tttacgggta gaattctacg cgtaaaacat gattgataat taaataattc 600
atttgcaagc tatacgttaa atcaaacgga cgctcgaggt tgcacaacac tattatcgat 660
ttgcagttcg ggacataaat gtttaaatat atcgatgtct ttgtgatgcg cgcgacattt 720
ttgtaggtta ttgataaaat gaacggatac gttgcccgac attatcatta aatccttggc 780
gtagaatttg tcgggtccat tgtccgtgtg cgctagcatg cccgtaacgg acctcgtact 840
tttggcttca aaggttttgc gcacagacaa aatgtgccac acttgcagct ctgcatgtgt 900
gcgcgttacc acaaatccca acggcgcagt gtacttgttg tatgcaaata aatctcgata 960
aaggcgcggc gcgcgaatgc agctgatcac gtacgctcct cgtgttccgt tcaaggacgg 1020
tgttatcgac ctcagattaa tgtttatcgg ccgactgttt tcgtatccgc tcaccaaacg 1080
cgtttttgca ttaacattgt atgtcggcgg atgttctata tctaatttga ataaataaac 1140
gataaccgcg ttggttttag agggcataat aaaagaaata ttgttatcgt gttcgccatt 1200
agggcagtat aaattgacgt tcatgttgga tattgtttca gttgcaagtt gacactggcg 1260
gcgacaagca attctaattg ggtaagttt tcccgttctt ttctgggttc ttcccttttg 1320
ctcatccttg ctgcactacc ttcaggtgca agttgagatt caggccacca tgggagatcc 1380
caccccaccc aagaagaagc gcaaaccggt ccgtcccctc ggagacgctt gtggagaact 1440
gtcacagact cctcgagaag ttccattact cgtgggagat gatgccgctt gtgctcgtca 1500
tcatgaacta cgcccgcagc gacttggatg aggcttcaag gaaaatctac gaaggtaccg 1560
aatgtgtaaa tacgagtgta gcgttgatta gaaaacggac attgttcgtg agtttannnn 1620
nnggtctctc tggccagcaa gacatttgaa acactgtaaa aaaattcatt gaaaaaaaag 1680
aacactgtaa tgaaaatatt ctgaatgctt aatctggtat ttcagggatt aaactgattg 1740
tgatgaaaag tgattaaact attttcttta agtaccaaat taaccgaaca ggtttgggtc 1800
tttcctttca gtaacaaaca aaatctatcg aaggtaagaa ataaacaaca ggatattttc 1860
ttttactaaa aatcaataag gagactgcac tatttcaatg ttcaacttcc tttatcgaat 1920
gcatgaaaaa tttaattgtc taaaaatcta aattactaat taacgcaaag gaacctttgc 1980
ctaaaaaaaa aaataagcta ttaaacgaat gcctaaaata cgtaacagtg ttgccagttg 2040
taaaaattgc gaatccgaga agtgcagttt cctgaaatgc ccagcgatac gaatttccta 2100
tgttagagtc ttgtccgcag ggaagatgat cgtcgacgag tacgcgagga agcacaactt 2160
gaacgtgttc gacggactag aactaaggaa ctcgacacgc caggcgcgcc ggatccggcc 2220
ggccgaaaat gctggaaatt aataatataa gtggtgtact gtcttcgtca atgaagttat 2280
tttgcgaatg atacttagtt ttacaagtgc cgtggtgtgt gttgacactt gctgtgcgat 2340
gctgtgcgaa tttcaacgga aatatttgtt gtcgtaacat tggatctatg ggtaagttta 2400

gtataataac tttactctgt tcacattagt gaaacataca tttgtaaaat ttgtgtttta 2460

ctaatgtgaa atttattttt ggaaattcac gttaacacta ttgaataaaa aaaaatcgat 2520

aatgtaattt aaaaaaaata caaaaatata gttttcgctt attgttagaa agaaaatttt 2580

acatacgcca ttttgaataa ttccttccgg gtacattggg ccctaaacca gcgatcgggg 2640

aactttttta attattaccc taaaatattt ttatgtaagt tgatattacc gatggcgaag 2700

aacaacaaaa aaaaaaacga aatcgcttct ttttagcatc tttcatatta tagaccccac 2760

gataatttta aatcacaacg attataaaga agtttcactt caatatatac tttttactca 2820

caaaagtttc atttttaccc catttgggat aatttagccc ggttcccccc ccgaccgctg 2880

gcctaaacgt atcaccgaca atagctaaaa taacaaggta cgttcgattt gccgagctga 2940

actaacatta cacagctttg cattattcat atgtacattg cgactgaaac gtccggaccg 3000

ttacaggtta ttggatgatg catcaatggc gattgcagca gtattcgttg tgctacggag 3060

cgctggagtt gtcggcgcgc aaggatgtgg ccgcgctatg ttgcctccga gatacgtgct 3120

ggcgcccgag gtcccgccgc gtctggtgcc cctccagctg atctagataa ctgatcataa 3180

tcagccatac cacatttgta gaggttttac ttgctttaaa aaacctccca cacctccccc 3240

tgaacctgaa acataaaatg aatgcaattg ttgttgttaa cttgtttatt gcagcttata 3300

atggttacaa ataaagcaat agcatcacaa atttcacaaa taaagcattt ttttcactgc 3360

attctagttg tggtttgtcc aaactcatca atgtatctta acgcgagtta attaagtgcg 3420

cgtaaattgt aagcgttaat attttgttaa aattcgcgtt aaatttttgt taaatcagct 3480

catttttaa ccaataggcc gaaatcggca aaatccctta taaatcaaaa gaatagaccg 3540

agatagggtt gagtgttgtt ccagtttgga acaagagtcc actattaaag aacgtggact 3600

ccaacgtcaa agggcgaaaa accgtctatc agggcgatgg cccactacgt gaaccatcac 3660

cctaatcaag ttttttgggg tcgaggtgcc gtaaagcact aaatcggaac cctaaaggga 3720

gcccccgatt tagagcttga cggggaaagc cggcgaacgt ggcgagaaag gaagggaaga 3780

aagcgaaagg agcgggcgct agggcgctgg caagtgtagc ggtcacgctg cgcgtaacca 3840

ccacacccgc cgcgcttaat gcgccgctac agggcgcgtc aggtggcact tttcggggaa 3900

atgtgcgcgg aacccctatt tgtttatttt tctaaataca ttcaaatatg tatccgctca 3960

tgagacaata accctgataa atgcttcaat aatattgaaa aaggaagagt cctgaggcgg 4020

aaagaaccag ctgtggaatg tgtgtcagtt agggtgtgga aagtccccag gctccccagc 4080

aggcagaagt atgcaaagca tgcatctcaa ttagtcagca accaggtgtg gaaagtcccc 4140

aggctcccca gcaggcagaa gtatgcaaag catgcatctc aattagtcag caaccatagt 4200

cccgccccta actccgccca tcccgcccct aactccgccc agttccgccc attctccgcc 4260

ccatggctga ctaatttttt ttatttatgc agaggccgag gccgcctcgg cctctgagct 4320

attccagaag tagtgaggag gcttttttgg aggcctaggc ttttgcaaag atcgatcaag 4380

agacaggatg aggatcgttt cgcatgattg aacaagatgg attgcacgca ggttctccgg 4440

ccgcttgggt ggagaggcta ttcggctatg actgggcaca acagacaatc ggctgctctg 4500

atgccgccgt gttccggctg tcagcgcagg ggcgcccggt tctttttgtc aagaccgacc 4560

tgtccggtgc cctgaatgaa ctgcaagacg aggcagcgcg gctatcgtgg ctggccacga 4620

cgggcgttcc ttgcgcagct gtgctcgacg ttgtcactga agcgggaagg gactggctgc 4680

tattgggcga agtgccgggg caggatctcc tgtcatctca ccttgctcct gccgagaaag 4740

tatccatcat ggctgatgca atgcggcggc tgcatacgct tgatccggct acctgcccat 4800

tcgaccacca agcgaaacat cgcatcgagc gagcacgtac tcggatggaa gccggtcttg 4860

tcgatcagga tgatctggac gaagagcatc aggggctcgc gccagccgaa ctgttcgcca 4920

ggctcaaggc gagcatgccc gacggcgagg atctcgtcgt gacccatggc gatgcctgct 4980

tgccgaatat catggtggaa aatggccgct tttctggatt catcgactgt ggccggctgg 5040

gtgtggcgga ccgctatcag gacatagcgt tggctacccg tgatattgct gaagagcttg 5100

gcggcgaatg ggctgaccgc ttcctcgtgc tttacggtat cgccgctccc gattcgcagc 5160

gcatcgcctt ctatcgcctt cttgacgagt tcttctgagc gggactctgg ggttcgaaat 5220

gaccgaccaa gcgacgccca acctgccatc acgagatttc gattccaccg ccgccttcta 5280

tgaaaggttg ggcttcggaa tcgttttccg ggacgccggc tggatgatcc tccagcgcgg 5340

ggatctcatg ctggagttct tcgcccaccc taggggaggg ctaactgaaa cacggaagga 5400

gacaataccg gaaggaaccc gcgctatgac ggcaataaaa agacagaata aaacgcacgg 5460

tgttgggtcg tttgttcata aacgcggggt tcggtcccag ggctggcact ctgtcgatac 5520

cccaccgaga ccccattggg gccaatacgc ccgcgtttct tccttttccc caccccaccc 5580

cccaagttcg ggtgaaggcc cagggctcgc agccaacgtc ggggcggcag gccctgccat 5640

agcctcaggt tactcatata tactttagat tgatttaaaa cttcattttt aatttaaaag 5700

gatctaggtg aagatccttt ttgataatct catgaccaaa atcccttaac gtgagttttc 5760

gttccactga gcgtcagacc ccgtagaaaa gatcaaagga tcttcttgag atcctttttt 5820

tctgcgcgta atctgctgct tgcaaacaaa aaaaccaccg ctaccagcgg tggtttgttt 5880

gccggatcaa gagctaccaa ctctttttcc gaaggtaact ggcttcagca gagcgcagat 5940

accaaatact gtccttctag tgtagccgta gttaggccac cacttcaaga actctgtagc 6000

accgcctaca tacctcgctc tgctaatcct gttaccagtg gctgctgcca gtggcgataa 6060

gtcgtgtctt accgggttgg actcaagacg atagttaccg gataaggcgc agcggtcggg 6120

ctgaacgggg ggttcgtgca cacagcccag cttggagcga acgacctaca ccgaactgag 6180

atacctacag cgtgagctat gagaaagcgc cacgcttccc gaagggagaa aggcggacag 6240

gtatccggta agcggcaggg tcggaacagg agagcgcacg agggagcttc caggggggaaa 6300

cgcctggtat ctttatagtc ctgtcgggtt tcgccacctc tgacttgagc gtcgattttt 6360

gtgatgctcg tcaggggggc ggagcctatg gaaaaacgcc agcaacgcgg cctttttacg 6420

gttcctggcc ttttgctggc cttttgctca catgttcttt cctgcgttat cccctgattc 6480

tgtggataac cgtattaccg ccatgcatta gttattaata gtaatcaatt acggggtcat 6540

tagttcatag cccatatatg gagttccgcg ttacataact tacggtaaat ggcccgcctg 6600

gctgaccgcc caacgacccc cgcccattga cgtcaataat gacgtatgtt cccatagtaa 6660

cgccaatagg gactttccat tgacgtcaat gggtggagta tttacggtaa actgcccact 6720

tggcagtaca tcaagtgtat catagcgatg c 6751

<210> 47
<211> 8183
<212> DNA
<213> artificial

<220>
<223> Sequence of pLA3359-Anopheles gambiae dsx construct

<400> 47
ccggtgctgc tgttgctgat gctacgatcc tcgacagtga ttggaaacgc ctggagatgg 60

tgggaaaaaa tcaaacacaa aaacggtcct aatgaacatc gtgtgttctc attcgctgcc 120

acgattgaca ccttcgataa gacgcacata atgagctaaa ggagagggga cagggtcttg 180

tctttgccac gagcgataag attgcaatca ctcgtgagcg tgtgctgctg ggctgaagaa 240

gaaacacttt ccacagcagt aggtgggaag tgggattgtg gaacgtggca ttgaaaagaa 300

cctattttct aaagcccgag agcccgttct cgaactggaa aacgagatgc agaagttttt 360

tattgtcccc cgccaggaaa acaaatgtat ttaatgcttt ctctgccttt tccgccccgt 420

ttcagacgac gagctagtga agcgagccca atggctgttg gagaaactcg gctacccgtg 480

ggagatgatg cccctgatgt acgtcatact gaagagcgcc gatggcgatg tacaaaaagc 540

acaccagcgg atcgacgaag gtaagctggc gatgatggtg tcgttcgaca tcactttcat 600

caccgtgtca gacatctact gtgcctagca ccggtccagt ggtcacaggg tgtagcaaaa 660

acgtgttctt ttttgcgaga gactctacct catgatgcag ctgttaagga aaggtttcag 720

atgaagacaa tttttcctag gataatatga tcttaagtta cctgcgtatg agtgtttaac 780

attgtcgtct caactccaag gaatgtttta accgtctagg gctagtttat ttatactgtt 840

ctcattgaaa tgtcgttaaa tccaacatgt taagttagct agctcagaca cgagaagtta 900

ggagtatctg catcttgaag gtagcggcat atggtgttat gccacgttca ctgacttcaa 960

aattcgatac aaaaaaaaac aaaatcaaaa acaaaattgt gaattccgtc agccagcagc 1020

agtgaccttc aaagccttac ctttccattc atttatgttt aacacaggtc aagcggtggt 1080

caacgaatac tcacgattgc ataatctgaa catgtttgat ggcgtggagt tgcgcaatac 1140

cacccgtcag agtggatgat aaactttccg caccactgta actgtccgta tctttgtatg 1200

tgggtgtgtg tatgtgtgtt tggtgaaacg aattcaattg ttctgtgcta ttttaaatca 1260

agccgcgtgc gcaactgatg ccgataagtt caaactagtg tttaaggagt ggagagagag 1320

agccgcacca cggtacagaa gggcagcaga atgggtcggc agcctagctg cactggtgcg 1380

gtgcgtccgg cgtctcgggg ggagggcggg gaaattctag tgttaaatcg gagcagcaaa 1440

aacaaaacag tggtcgtccc gttcaagaaa cggcctgtac acacacagaa aacactgcag 1500

catgtttgta catagtagat cctagagcag gtggtcgttg ctcctcgaac gctctggacg 1560

cacggcttcg cgcgtacttg cgtagcgttc caccgatcgt gggtattcgt actgccacaa 1620

gcccgctttc tcccatgcaa tctctgcaac caaaccaaca aacaacaaca aaataccaat 1680

cgacacaatg aatcacaccc cttttgtatc atctgtatat tcttgttctt tgcgttcttt 1740

tccatgtggc ccacgccccg gcgggtacgt aattgcgtcg aaaaccccga aaaccccggc 1800

acatacagtg tacatacggt ttgaggacaa ctttgacctg cagcccttct ggggctgcca 1860

cgtgtagcta tacttgtgag atcgggcgcc gacggtgtaa agcgcgaatg gccgccacac 1920

agtgtgtcca ctccaacact acccctctgg aactaccccg tccagggatg caccggctcg 1980

gctcatgccc ctgcaaaaca gtccgggctc cactgtagta gctccggcgt tgctctgaga 2040

gaaggatgcc cttcgaagtg tcgaaagcgt gcattgggcg ttcaagtgtg tgtctgtgtt 2100

aggtttagcg agaaacagca gcagttgcgt gtgctgaaaa gcgaaggagt aatagagtgc 2160

ataatgaaaa tgaaaatgaa aatgaagcaa aagtagaagg cggaggagag caacctgtgt 2220

tccactagta gcgaatagtt tagtctagtt tcgtcaccaa tcaaccttcc aaccatcgtt 2280

caaccaatac ctgagtcaac atcgtcatcg ttatcgtgcc acaactttat taaaaatgaa 2340

ccttgtccgc gccaccgtag ggtgatctga ggcgaccttt cttacgggcg cgactcacat 2400

gccatcgtca ccttctccaa tcaaaaccaa cagcctgtac cgatggtgtg caattgtgcg 2460

tgcgtgtgtg ttattagcaa aaaaagagaa agagacggcg agagagagat agatcgagat 2520

cgagagtaca aaagagcagt agaaatgttc gttgtttgtt ttccgtaaca cagttgttta 2580

gccaaaatgg gaatttccaa taatcccggg ggcggggaaa tgcgggaata ctgcgtacac 2640

acatacatca atcaaaaaga aaaatccttg cgctacatca ctaccgtttg cgcggtgctg 2700

atctagagca gaccactttc cacgccattc tacaatcaat caatctgtgc agaaggtatg 2760

gtaagacggc ctttgagcga gtcacggtcg ccaccataac gccgtccgac gagggctgaa 2820

tgcgaacttt gctaatcgat tttccgcttt ctttttatcc caccccctt tctctctctc 2880

tcttttgcac cgccccttgt aacccccaaa aaggtaaacg acacattaag acctacgaag 2940
cgctggtgaa gtcatcgctc gatccgaaca gcgaccggct gacggaagac gacgacgagg 3000
acgagaacat ctcggtgacc cgcaccaact ccaccattcg gtcgaggtcc agctcgctgt 3060
cgcggtcccg gtcctgctcg cgccaggccg aaactccccg ggccgacgat cgggccctga 3120
accttgacac caaatagatc tcgacccaag aaaaagcgga aggtggagga cccgtaagat 3180
ccaccggatc tagataactg atcataatca gccataccac atttgtagag gttttacttg 3240
ctttaaaaaa cctcccacac ctccccctga acctgaaaca taaaatgaat gcaattgttg 3300
ttgttaactt gtttattgca gcttataatg gttacaaata aagcaatagc atcacaaatt 3360
tcacaaataa agcatttttt tcactgcatt ctagttgtgg tttgtccaaa ctcatcaatg 3420
tatcttaacg cgagttaatt aagtgcgcgt aaattgtaag cgttaatatt ttgttaaaat 3480
tcgcgttaaa tttttgttaa atcagctcat tttttaacca ataggccgaa atcggcaaaa 3540
tcccttataa atcaaaagaa tagaccgaga tagggttgag tgttgttcca gtttggaaca 3600
agagtccact attaaagaac gtggactcca acgtcaaagg gcgaaaaacc gtctatcagg 3660
gcgatggccc actacgtgaa ccatcaccct aatcaagttt tttggggtcg aggtgccgta 3720
aagcactaaa tcggaaccct aaagggagcc cccgatttag agcttgacgg ggaaagccgg 3780
cgaacgtggc gagaaaggaa gggaagaaag cgaaaggagc gggcgctagg gcgctggcaa 3840
gtgtagcggt cacgctgcgc gtaaccacca cacccgccgc gcttaatgcg ccgctacagg 3900
gcgcgtcagg tggcactttt cggggaaatg tgcgcggaac ccctatttgt ttatttttct 3960
aaatacattc aaatatgtat ccgctcatga gacaataacc ctgataaatg cttcaataat 4020
attgaaaaag gaagagtcct gaggcggaaa gaaccagctg tggaatgtgt gtcagttagg 4080
gtgtggaaag tccccaggct ccccagcagg cagaagtatg caaagcatgc atctcaatta 4140
gtcagcaacc aggtgtggaa agtccccagg ctccccagca ggcagaagta tgcaaagcat 4200
gcatctcaat tagtcagcaa ccatagtccc gcccctaact ccgcccatcc cgcccctaac 4260
tccgcccagt tccgcccatt ctccgcccca tggctgacta atttttttta tttatgcaga 4320
ggccgaggcc gcctcggcct ctgagctatt ccagaagtag tgaggaggct tttttggagg 4380
cctaggcttt tgcaaagatc gatcaagaga caggatgagg atcgtttcgc atgattgaac 4440
aagatggatt gcacgcaggt tctccggccg cttgggtgga gaggctattc ggctatgact 4500
gggcacaaca gacaatcggc tgctctgatg ccgccgtgtt ccggctgtca gcgcaggggc 4560
gcccggttct ttttgtcaag accgacctgt ccggtgccct gaatgaactg caagacgagg 4620
cagcgcggct atcgtggctg gccacgacgg gcgttccttg cgcagctgtg ctcgacgttg 4680
tcactgaagc gggaagggac tggctgctat tgggcgaagt gccggggcag gatctcctgt 4740
catctcacct tgctcctgcc gagaaagtat ccatcatggc tgatgcaatg cggcggctgc 4800

atacgcttga tccggctacc tgcccattcg accaccaagc gaaacatcgc atcgagcgag 4860
cacgtactcg gatggaagcc ggtcttgtcg atcaggatga tctggacgaa gagcatcagg 4920
ggctcgcgcc agccgaactg ttcgccaggc tcaaggcgag catgcccgac ggcgaggatc 4980
tcgtcgtgac ccatggcgat gcctgcttgc cgaatatcat ggtggaaaat ggccgctttt 5040
ctggattcat cgactgtggc cggctgggtg tggcggaccg ctatcaggac atagcgttgg 5100
ctacccgtga tattgctgaa gagcttggcg gcgaatgggc tgaccgcttc ctcgtgcttt 5160
acggtatcgc cgctcccgat tcgcagcgca tcgccttcta tcgccttctt gacgagttct 5220
tctgagcggg actctggggt tcgaaatgac cgaccaagcg acgcccaacc tgccatcacg 5280
agatttcgat tccaccgccg ccttctatga aaggttgggc ttcggaatcg ttttccggga 5340
cgccggctgg atgatcctcc agcgcgggga tctcatgctg gagttcttcg cccaccctag 5400
ggggaggcta actgaaacac ggaaggagac aataccggaa ggaacccgcg ctatgacggc 5460
aataaaaaga cagaataaaa cgcacggtgt tgggtcgttt gttcataaac gcggggttcg 5520
gtcccagggc tggcactctg tcgatacccc accgagaccc cattggggcc aatacgcccg 5580
cgtttcttcc ttttccccac cccacccccc aagttcgggt gaaggcccag ggctcgcagc 5640
caacgtcggg gcggcaggcc ctgccatagc ctcaggttac tcatatatac tttagattga 5700
tttaaaactt catttttaat ttaaaaggat ctaggtgaag atcctttttg ataatctcat 5760
gaccaaaatc ccttaacgtg agttttcgtt ccactgagcg tcagaccccg tagaaaagat 5820
caaaggatct tcttgagatc ctttttttct gcgcgtaatc tgctgcttgc aaacaaaaaa 5880
accaccgcta ccagcggtgg tttgtttgcc ggatcaagag ctaccaactc tttttccgaa 5940
ggtaactggc ttcagcagag cgcagatacc aaatactgtc cttctagtgt agccgtagtt 6000
aggccaccac ttcaagaact ctgtagcacc gcctacatac ctcgctctgc taatcctgtt 6060
accagtggct gctgccagtg gcgataagtc gtgtcttacc gggttggact caagacgata 6120
gttaccggat aaggcgcagc ggtcgggctg aacggggggt tcgtgcacac agcccagctt 6180
ggagcgaacg acctacaccg aactgagata cctacagcgt gagctatgag aaagcgccac 6240
gcttcccgaa gggagaaagg cggacaggta tccggtaagc ggcagggtcg gaacaggaga 6300
gcgcacgagg gagcttccag ggggaaacgc ctggtatctt tatagtcctg tcgggtttcg 6360
ccacctctga cttgagcgtc gatttttgtg atgctcgtca ggggggcgga gcctatggaa 6420
aaacgccagc aacgcggcct ttttacggtt cctggccttt tgctggcctt ttgctcacat 6480
gttctttcct gcgttatccc ctgattctgt ggataaccgt attaccgcca tgcattagtt 6540
attaatagta atcaattacg gggtcattag ttcatagccc atatatggag ttccgcgtta 6600
cataacttac ggtaaatggc ccgcctggct gaccgcccaa cgacccccgc ccattgacgt 6660

caataatgac gtatgttccc atagtaacgc caatagggac tttccattga cgtcaatggg 6720

tggagtattt acggtaaact gcccacttgg cagtacatca agtgtatcat agcgatgcgg 6780

ccgcatggta cccattgctt gtcatttatt aatttggatg atgtcatttg tttttaaaat 6840

tgaactggct ttacgagtag aattctacgc gtaaaacaca atcaagtatg agtcataatc 6900

tgatgtcatg ttttgtacac ggctcataac cgaactggct ttacgagtag aattctactt 6960

gtaatgcacg atcagtggat gatgtcattt gtttttcaaa tcgagatgat gtcatgtttt 7020

gcacacggct cataaactcg ctttacgagt agaattctac gtgtaacgca cgatcgattg 7080

atgagtcatt tgttttgcaa tatgatatca tacaatatga ctcatttgtt tttcaaaacc 7140

gaacttgatt tacgggtaga attctacttg taaagcacaa tcaaaaagat gatgtcattt 7200

gtttttcaaa actgaactcg ctttacgagt agaattctac gtgtaaaaca caatcaagaa 7260

atgatgtcat ttgttataaa aataaaagct gatgtcatgt tttgcacatg gctcataact 7320

aaactcgctt tacgggtaga attctacgcg taaaacatga ttgataatta aataattcat 7380

ttgcaagcta tacgttaaat caaacggacg ctcgaggttg cacaacacta ttatcgattt 7440

gcagttcggg acataaatgt ttaaatatat cgatgtcttt gtgatgcgcg cgacattttt 7500

gtaggttatt gataaaatga acggatacgt tgcccgacat tatcattaaa tccttggcgt 7560

agaatttgtc gggtccattg tccgtgtgcg ctagcatgcc cgtaacggac ctcgtacttt 7620

tggcttcaaa ggttttgcgc acagacaaaa tgtgccacac ttgcagctct gcatgtgtgc 7680

gcgttaccac aaatcccaac ggcgcagtgt acttgttgta tgcaaataaa tctcgataaa 7740

ggcgcggcgc gcgaatgcag ctgatcacgt acgctcctcg tgttccgttc aaggacggtg 7800

ttatcgacct cagattaatg tttatcggcc gactgttttc gtatccgctc accaaacgcg 7860

tttttgcatt aacattgtat gtcggcggat gttctatatc taatttgaat aaataaacga 7920

taaccgcgtt ggttttagag ggcataataa aagaaatatt gttatcgtgt tcgccattag 7980

ggcagtataa attgacgttc atgttggata ttgtttcagt tgcaagttga cactggcggc 8040

gacaagcaat tctaattggg gtaagttttc ccgttctttt ctgggttctt ccctttttgct 8100

catccttgct gcactacctt caggtgcaag ttgagattca ggccaccatg ggagatccca 8160

ccccacccaa gaagaagcgc aaa 8183

<210> 48
<211> 7342
<212> DNA
<213> artificial

<220>
<223> Sequence of pLA3433-Agdsx (Anopheles gambiae) construct with exon 2 included

<400> 48

ctagtgtcga cgatgtaggt cacggtctcg aagccgcggt gcgggtgcca gggcgtgccc 60
ttgggctccc cgggcgcgta ctccacctca cccatctggt ccatcatgat gaacgggtcg 120
aggtggcggt agttgatccc ggcgaacgcg cggcgcaccg ggaagccctc gccctcgaaa 180
ccgctgggcg cggtggtcac ggtgagcacg ggacgtgcga cggcgtcggc gggtgcggat 240
acgcggggca gcgtcagcgg gttctcgacg gtcacggcgg gcatgtcgac cgccggcgcc 300
ttaattaact cgcgttaaga tacattgatg agtttggaca aaccacaact agaatgcagt 360
gaaaaaaatg ctttatttgt gaaatttgtg atgctattgc tttatttgta accattataa 420
gctgcaataa acaagttaac aacaacaatt gcattcattt tatgtttcag gttcaggggg 480
aggtgtggga ggttttttaa agcaagtaaa acctctacaa atgtggtatg gctgattatg 540
atcagttatc tagatccggt ggatcttacg ggtcctccac cttccgcttt ttcttgggtc 600
gagatctgag tccggaatcc tcgtcgctac cgatggcgct ggtgatgcgg ggcacgctgt 660
gggcgtaggt cacctcgcgc tggcacacgt ggtcgcgctt gtcgctggtg tccctcatct 720
gcttggtgat gatggtcacg aagtgggggc cggggatctt gatggcgcgg ctgccgttga 780
aggtcatctt gctgtcgaag tggcccatca tcaggccgcc gtcggcggtg gtgaagccga 840
tgaaggccag ctggcgcacg gcgttggggc cgtgggggaa catgtgggtc tcgttgggca 900
ggatgtccac cagctggtcg cgcatgatgg ggccgtcggg ctggaagccg tcgcagttca 960
cggtgatgcg gctgaccacg caggtgccgt ccagctcgta ggtgtggtgg ctggtcatgg 1020
tgccgtcgtt ctcgaagcgc acggtgcggt cgatgctcag gccctcgggg aagcactcct 1080
gggcgaagtg gctgatgccg ttggggtagc gggcgaagaa gggctcgccg tactggatca 1140
ggtggcagat gggcttccag ctcatgggca gcttgccggt ctcgcacacg gcgtgcacgt 1200
tgaagtcgcc gtgggggaac ttgctgctgc cgtcggccac gatggtgaac ttctggccgt 1260
tcacctcgcc gtcgatgaag attttgaagg tcatgtcgct ctggaacagg gcggggccgc 1320
cctctgaacc atcctcgtcc atggtggcga ccggtttgcg cttcttcttg ggtggggtgg 1380
gatccaccag agacaggttg cggcggcggt tggatggcgt gggcgcgttg gcgttgttgg 1440
accggctcat gttgtgtcgc tgtaacagat gctgttcaac tgtgtttacc agatcgttgc 1500
gggctgtatt tataggcgcg ataagcggga cggcgcctc gtgtccggtc acgcgcatga 1560
gataacgcgc ggctgatatg gaggcgcgtc ctgttccgat aaggagttgc gtccggctgc 1620
ggttagcaac acaggaagct ggcgtcctgt cacgataaga caacactcgt ccggtccgat 1680
aatgtgattc gtacgtgaca ggacgcgacc cgataaggcc ggcctacgtg actgccgaca 1740
cgtacttttt tgcactgcaa aaaggttcaa tgtgtggtag tgtatttgga gcgtatacaa 1800
cggtgtagac tatttatgta aaatagtcta cgaaacgtag agtttgtact atgtatgggc 1860
ccgcgtgcaa aagcgtgttt ttttgcagtg caaaaaagtt ggtggtgggg aggccaccga 1920

gtatggtacc atgcggccgc gtacgcgccc ggggagccca agggcacgcc ctggcacccg 1980
tccggtgctt atctagagcg cgcttggcgt aatcatggtc atagctgttt cctgtgtgaa 2040
attgttatcc gctcacaatt ccacacaaca tacgagccgg aagcataaag tgtaaagcct 2100
ggggtgccta atgagtgagc taactcacat taattgcgtt gcgctcactg cccgctttcc 2160
agtcgggaaa cctgtcgtgc cagctgcatt aatgaatcgg ccaacgcgcg gggagaggcg 2220
gtttgcgtat tgggcgctct tccgcttcct cgctcactga ctcgctgcgc tcggtcgttc 2280
ggctgcggcg agcggtatca gctcactcaa aggcggtaat acggttatcc acagaatcag 2340
gggataacgc aggaaagaac atgtgagcaa aaggccagca aaaggccagg aaccgtaaaa 2400
aggccgcgtt gctggcgttt ttccataggc tccgcccccc tgacgagcat cacaaaaatc 2460
gacgctcaag tcagaggtgg cgaaacccga caggactata aagataccag gcgtttcccc 2520
ctggaagctc cctcgtgcgc tctcctgttc cgaccctgcc gcttaccgga tacctgtccg 2580
cctttctccc ttcgggaagc gtggcgcttt ctcatagctc acgctgtagg tatctcagtt 2640
cggtgtaggt cgttcgctcc aagctgggct gtgtgcacga accccccgtt cagcccgacc 2700
gctgcgcctt atccggtaac tatcgtcttg agtccaaccc ggtaagacac gacttatcgc 2760
cactggcagc agccactggt aacaggatta gcagagcgag gtatgtaggc ggtgctacag 2820
agttcttgaa gtggtggcct aactacggct acactagaag gacagtattt ggtatctgcg 2880
ctctgctgaa gccagttacc ttcggaaaaa gagttggtag ctcttgatcc ggcaaacaaa 2940
ccaccgctgg tagcggtggt ttttttgttt gcaagcagca gattacgcgc agaaaaaaag 3000
gatctcaaga agatcctttg atcttttcta cggggtctga cgctcagtgg aacgaaaact 3060
cacgttaagg gattttggtc atgagattat caaaaaggat cttcacctag atccttttaa 3120
attaaaaatg aagttttaaa tcaatctaaa gtatatatga gtaaacttgg tctgacagtt 3180
accaatgctt aatcagtgag gcacctatct cagcgatctg tctatttcgt tcatccatag 3240
ttgcctgact ccccgtcgtg tagataacta cgatacggga gggcttacca tctggcccca 3300
gtgctgcaat gataccgcga gacccacgct caccggctcc agatttatca gcaataaacc 3360
agccagccgg aagggccgag cgcagaagtg gtcctgcaac tttatccgcc tccatccagt 3420
ctattaattg ttgccgggaa gctagagtaa gtagttcgcc agttaatagt ttgcgcaacg 3480
ttgttgccat tgctacaggc atcgtggtgt cacgctcgtc gtttggtatg gcttcattca 3540
gctccggttc ccaacgatca aggcgagtta catgatcccc catgttgtgc aaaaaagcgg 3600
ttagctcctt cggtcctccg atcgttgtca gaagtaagtt ggccgcagtg ttatcactca 3660
tggttatggc agcactgcat aattctctta ctgtcatgcc atccgtaaga tgcttttctg 3720
tgactggtga gtactcaacc aagtcattct gagaatagtg tatgcggcga ccgagttgct 3780

cttgcccggc gtcaatacgg gataataccg cgccacatag cagaacttta aaagtgctca 3840
tcattggaaa acgttcttcg gggcgaaaac tctcaaggat cttaccgctg ttgagatcca 3900
gttcgatgta acccactcgt gcacccaact gatcttcagc atcttttact ttcaccagcg 3960
tttctgggtg agcaaaaaca ggaaggcaaa atgccgcaaa aaagggaata agggcgacac 4020
ggaaatgttg aatactcata ctcttccttt ttcaatatta ttgaagcatt tatcagggtt 4080
attgtctcat gagcggatac atatttgaat gtatttagaa aaataaacaa ataggggttc 4140
cgcgcacatt tccccgaaaa gtgccaccta aattgtaagc gttaatattt tgttaaaatt 4200
cgcgttaaat ttttgttaaa tcagctcatt ttttaaccaa taggccgaaa tcggcaaaat 4260
cccttataaa tcaaaagaat agaccgagat agggttgagt gttgttccag tttggaacaa 4320
gagtccacta ttaaagaacg tggactccaa cgtcaaaggg cgaaaaaccg tctatcaggg 4380
cgatggccca ctacgtgaac catcacccta atcaagtttt ttggggtcga ggtgccgtaa 4440
agcactaaat cggaacccta aagggagccc ccgatttaga gcttgacggg gaaagccggc 4500
gaacgtggcg agaaaggaag ggaagaaagc gaaaggagcg ggcgctaggg cgctggcaag 4560
tgtagcggtc acgctgcgcg taaccaccac acccgccgcg cttaatgcgc cgctacaggg 4620
cgcgtcccat tcgccattca ggctgcgcaa ctgttgggaa gggcgatcgg tgcgggcctc 4680
ttcgctatta cgccagctgg cgaaaggggg atgtgctgca aggcgattaa gttgggtaac 4740
gccagggttt tcccagtcac gacgttgtaa aacgacggcc agtgagcgcg ctagcgttta 4800
aacgagctct aagatacatt gatgagtttg gacaaaccac aactagaatg cagtgaaaaa 4860
aatgctttat ttgtgaaatt tgtgatgcta ttgctttatt tgtaaccatt ataagctgca 4920
ataaacaagt taacaacaac aattgcattc attttatgtt tcaggttcag ggggaggtgt 4980
gggaggtttt ttaaagcaag taaaacctct acaaatgtgg tatggctgat tatgatcctg 5040
cagctacgcc gctacgtctt ccgtgccgtc ctgggcgtcg tcttcgtcgt cgtcggtcgg 5100
cggcttcgcc cacgtgatcg aagcgcgctt ctcgatgggc gttccctgcc ccctgcccgt 5160
agtcgacttc gtgacaacga tcttgtctac gaagagcccg acgaacacgc gcttgtcgtc 5220
tactgacgcg cgcccccacc acgacttagg gccggtcggg tcagcgtcgg cgtcttcggg 5280
gaaccattgg tcaaggggaa gcttcggggc ttcggcggct tcaagttcgg caagccgctc 5340
ttccgcccct tgctgccgga gcgtcagcgc tgcctgttgc ttccggaagt gcttcctgcc 5400
aacgggtccg tcgtacgcgc ctgccgcgcg gtcttcgtac agctcttcaa gggcgttcag 5460
ggcgtcggcg cgctccgcaa caaggttcgc ccgttcgccg ctcttctcag gcgcctcagt 5520
gagcttgccg aagcgtcggg cggcttccca cagaagcgcc aacgtctctt cgtcgccttc 5580
ggcgtgcctg atcttgttga agatgcgttc cgcaacgaac ttgtcgagtg ccgccatgct 5640
gacgttgcac gtgccttcgt gctgcccagg tgcggacggg tcgaccacct tccggcgacg 5700

gcagcggtaa gagtccttga tcgattcttc cccgcgcttc gaagtcatga cggcgccaca 5760

ctcgcagtac agcttgtcca tggcggacag aatggcttgc ccccgggaaa gccccttgcc 5820

gcgccccctg ccgtccaacc acgcctgaag ctcataccac tcagcgggct cgatgatcgg 5880

tccgcaatca agctcgaccg gccggagcgt gatcgggtcg cgctgaatgc ggtaaccctc 5940

aatcttcgtg gtcggcgtgc cgtccggctt cttcttgtag atcacctcag cggcgaagcc 6000

cgcaatacgc gggtcccgaa ggattcgcat aacggttgcc gggtcccagg cgcttgaagc 6060

ggtcttcttc ccaatcgtct cgccccgggt cggcacggcg tcagcgtcca tgcgcttaca 6120

aagccccgtg atgctgcccg ggtgaatggc ggcttgactg cccggcttga agggaaggtg 6180

tttgtgcgtc ttgatctcac gccaccacca ccggattacg tcgggctcga actcgaaggg 6240

tccggtaagg ggagtggtcg agtgcgcaag cttgttgatg acgacattga ccattcggcc 6300

gttgcgcgtg atctccttcg tctccgaaac aagctcgaag ccgtaaggcg ccttcccgcc 6360

gacgtacccg cccaattcgc gctgaaggtt cttcgtgtcg agaatcttcg ccgacttcag 6420

cgaagattct ttgtgcgacg cgtcgagccg cataatcagg tgaatcaggt ccatgacgtt 6480

tccctgccgg aagacgcctt cctgagtgga aacaatcgtc acgcccaggg cgagcaattc 6540

cgagacaatc ggaatcgcgt ccatgacctt caggcgcgag aagcgcgaca cgtcatagac 6600

aatgatcatg ttgagccgcc cggcgcggca ttcgttcagg atgcgttcga actccgggcg 6660

ctccgccgtc ccgaacgccg acgtgcccgg cgcttcgctg aaatgcccga cgaacctgaa 6720

ccggcccccg tcgcgctcga cttcgcgctg aaggtcggcc gccttgtctt cgttggcgct 6780

acgctgtgtc gctgggcttg ctgcgctcga attctcgcgc tcgcgcgact gacggtcgta 6840

agcacccgcg tacgtgtcca tggcggatcc gtgtcgctgt aacagatgct gttcaactgt 6900

gtttaccaga tcgttgcggg ctgtatttat aggcgcgata agcgggacgg gcgcctcgtg 6960

tccggtcacg cgcatgagat aacgcgcggc tgatatggag gcgcgtcctg ttccgataag 7020

gagttgcgtc cggctgcggt tagcaacaca ggaagctggc gtcctgtcac gataagacaa 7080

cactcgtccg gtccgataat gtgattcgta cgtgacagga cgcgacccga taaggccggc 7140

ctacgtgact gccgacacgt actttttgc actgcaaaaa ggttcaatgt gtggtagtgt 7200

atttggagcg tatacaacgg tgtagactat ttatgtaaaa tagtctacga aacgtagagt 7260

ttgtactatg tatgggcccg cgtgcaaaag cgtgtttttt tgcagtgcaa aaaagttggt 7320

ggtggggagg ccaccgagta ta 7342

<210> 49
<211> 11868
<212> DNA
<213> artificial

<220>
<223> 49 Sequence of pLA1188-cctra intron construct

<400> 49
gtggttttg tcaaacgaag attctatgac gtgtttaaag tttaggtcga gtaaagcgca 60

aatctttttt aaccctagaa agatagtctg cgtaaaattg acgcatgcat tcttgaaata 120

ttgctctctc tttctaaata gcgcgaatcc gtcgctgtgc atttaggaca tctcagtcgc 180

cgcttggagc tcccgtgagg cgtgcttgtc aatgcggtaa gtgtcactga ttttgaacta 240

taacgaccgc gtgagtcaaa atgacgcatg attatctttt acgtgacttt taagatttaa 300

ctcatacgat aattatattg ttatttcatg ttctacttac gtgataactt attatatata 360

tattttcttg ttatagatat cgtgactaat atataataaa atgggtagtt ctttagacga 420

tgagcatatc ctctctgctc ttctgcaaag cgatgacgag cttgttggtg aggattctga 480

cagtgaaata tcagatcacg taagtgaaga tgacgtccag agcgatacag aagaagcgtt 540

tatagatgag gtacatgaag tgcagccaac gtcaagcggt agtgaaatat tagacgaaca 600

aaatgttatt gaacaaccag gttcttcatt ggcttctaac agaatcttga ccttgccaca 660

gaggactatt agaggtaaga ataaacattg ttggtcaact tcaaagtcca cgaggcgtag 720

ccgagtctct gcactgaaca ttgtcagatc ggcccgggcg gccgtttttc ttgaaatatt 780

gctctctctt tctaaatagc gcgaatccgt cgctgtgcat ttaggacatc tcagtcgccg 840

cttggagctc ccaaacgcgc cagtggtagt acacagtact gtgggtgttc agtttgaaat 900

cctcttgctt ctccattgtc tcggttacct ttggtcaaat ccatgggttc tattgcctat 960

atactcttgc gattaccagt gattgcgcta ttagctatta gatggattgt tggccaaact 1020

tgtcgcttaa gtggctggga attgtaaccg taggcccgag tgtaatgatc ccccataaaa 1080

agttttcgca atgcctttat tttttgttgc aaatctctct ttattctgcg gtattcttca 1140

ttattgcggg gatggggaaa gtgtttatat agaagcaact tacgattgaa cccaaatgca 1200

cctgacaagc aaggtcaaag ggccagattt ttaaatatat tatttagtct taggactctc 1260

tatttgcaat taaattactt tgctacctga gggttaaatc ttccccattg ataataataa 1320

ttccactata tgttcaattg ggtttcaccg cgcttagtta catgacgagc cctaatgagc 1380

cgtcggtggt ctataaactg tgccttacaa atacttgcaa ctcttctcgt tttgaagtca 1440

gcagagttat tgctaattgc taattgctaa ttgcttttaa ctgatttctt cgaaattggt 1500

gctatgttta tggcgctatt aacaagtatg aatgtcaggt ttaaccaggg gatgcttaat 1560

tgtgttctca acttcaaagg cagaaatgtt tactcttgac catgggttta ggtataatgt 1620

tatcaagctc ctcgagttaa cgttacgtta acgttaacgt tcgaggtcga ctctagaact 1680

acccaccgta ctcgtcaatt ccaagggcat cggtaaacat ctgctcaaac tcgaagtcgg 1740

ccatatccag agcgccgtag gggcggagt cgtgggggt aaatcccgga cccggggaat 1800

ccccgtcccc caacatgtcc agatcgaaat cgtctagcgc gtcggcatgc gccatcgcca 1860
cgtcctcgcc gtctaagtgg agctcgtccc ccaggctgac atcggtcggg ggggccgtcg 1920
acagtctgcg cgtgtgtccc gcggggagaa aggacaggcg cggagccgcc agccccgcct 1980
cttcgggggc gtcgtcgtcc gggagatcga gcaggccctc gatggtagac ccgtaattgt 2040
ttttcgtacg cgcgcggctg tacgcggggc ccgagcccga ctcgcatttc agttgctttt 2100
ccaatccgca gataatcagc tccaagccga acaggaatgc cggctcggct ccttgatgat 2160
cgaacagctc gattgcctga cgcagcagtg gggcatcga atcggttgtt ggggtctcgc 2220
gctcctcttt tgcgacttga tgctcttggt cctccagcac gcagcccagg gtaaagtgac 2280
cgacggcgct cagagcgtag agagcatttt ccaggctgaa gccttgctgg cacaggaacg 2340
cgagctggtt ctccagtgtc tcgtattgct tttcggtcgg gcgcgtgccg agatggactt 2400
tggcaccgtc tcggtgggac agcagagcgc agcggaacga cttggcgtta ttgcggagga 2460
agtcctgcca ggactcgcct tccaacgggc aaaaatgcgt gtggtggcgg tcgagcatct 2520
cgatggccag ggcatccagc agcgcccgct tattcttcac ctatagatac catagatgta 2580
tggattagta tcatatacat acaaaggcta tttttgggac atattaatat taacaatttc 2640
cgtgatagtt ttcaccattt ttgttgaatg ttacgttgaa aatttaaatt tgttttaaat 2700
taattttacc agtcatgtgt tcttaaaagt ttttatgatt gaaacggcat aaagtggttc 2760
aaaaatttat caagaaaggc tttccttttt taaatcttat ctttttctct taaaaatcac 2820
tagtcaattc attattaatt tgttaacttg aatttggaat gtctatttac tttcagataa 2880
attaaagcaa gaaacttaat attcgaaaaa aattgattct aaatggaatt tcacttgatc 2940
ttcatgtatg catatcaatt tttatttaca ttgtataata agtttcgagt tgattgttgt 3000
aatccacagg tgtcccagag aattaaattc caaattaccc aagtttattg aatgttgatt 3060
gtagtttcag ttgctttgtt gctgcaacaa tggcttgttg attgtagata ttttcccttt 3120
ccttggttta cttattacat agactgaaaa agaggtttac ttttttgata cttatgaaaa 3180
atttctatta gtgattacta accaatcgct atatgtttac tagaaaacaa ataaactctt 3240
tacattaaca ttcaataatg tttgctctgt aaccgacaat tgaaggcgtt acagcaacag 3300
taatataact agcttcttaa ccctcatcta ttaaccccat cgtttaaaac actatgttaa 3360
atggtctaac aaatctagat actaatagat gtcttattac ttagcagcca cagctgcaac 3420
atccaagaca atttttgaaa cttcttattg agctcttggc agcagaaatg ttggtatttt 3480
tcacagcttt ctgaaagacc ggcaccttcc tccggttccc gtttctgaat tcaagaggat 3540
ttccgacccc caattaatcc cgaaacaaat aaggtatatt caaaatgatg gaaaagtcat 3600
ggctgctgac cttattttta ttcctattga tagaatatta ttccccttttt aaatacactg 3660

tactaagagg tccggctata attttactca cttgtcgatt atcccataga atgttgattg 3720
tagttggttg cttttccagg tgagagttga tcaagtcaca aaagttagcg tgtgttgatt 3780
gtagatttga aggtaaaata atttttgcac ccattcatcg ggtaaaacgt tctccataga 3840
atacatttcc atcgataatt gataacttat gaatttcaaa gaaaaaaata tgcttttaaa 3900
attacgtgcc agtagagggt gggctgctcc acgcccagct tctgcgccaa cttgcgggtc 3960
gtcagtccct caatgccaac ttcgttcaac agctccaacg cggagttgat gactttggac 4020
ttatccaggc ggctgcccat ggtggtttct aaaggtgtta taaatcaaat tagttttgtt 4080
ttttcttgaa aactttgcgt ttcctttgat caacttaccg ccagggtacc gcagattgtt 4140
tagcttgttc agctgcgctt gtttatttgc ttagctttcg cttagcgacg tgttcacttt 4200
gcttgtttga attgaattgt cgctccgtag acgaagcgcc tctatttata ctccggcgct 4260
cgttttcgag tttaccactc cctatcagtg atagagaaaa gtgaaagtcg agtttaccac 4320
tccctatcag tgatagagaa aagtgaaagt cgagtttacc actccctatc agtgatagag 4380
aaaagtgaaa gtcgagttta ccactcccta tcagtgatag agaaaagtga aagtcgagtt 4440
taccactccc tatcagtgat agagaaaagt gaaagtcgag tttaccactc cctatcagtg 4500
atagagaaaa gtgaaagtcg agtttaccac tccctatcag tgatagagaa aagtgaaagt 4560
cgaaacctgg cgcgccccgg ccatcgagaa agagagagag aagagaagag agagaacatt 4620
cgagaaagag agagagaaga gaagagagag aacatactcc ctatcagtga tagagaagtc 4680
cctatcagtg atagagatgt ccctatcagt gatagagagt tccctatcag tgatagagac 4740
gtccctatca gtgatagaga agtccctatc agtgatagag agatccctat cagtgataga 4800
gatttcccta tcagtgatag agaggtccct atcagtgata gagacttccc tatcagtgat 4860
agagaaatcc ctatcagtga tagagacatc cctatcagtg atagagaact ccctatcagt 4920
gatagagacc tccctatcag tgatagagat cgatgcggcc gcatggtacc cattgcttgt 4980
catttattaa tttggatgat gtcatttgtt tttaaaattg aactggcttt acgagtagaa 5040
ttctacgcgt aaaacacaat caagtatgag tcataatctg atgtcatgtt ttgtacacgg 5100
ctcataaccg aactggcttt acgagtagaa ttctacttgt aatgcacgat cagtggatga 5160
tgtcatttgt ttttcaaatc gagatgatgt catgttttgc acacggctca taaactcgct 5220
ttacgagtag aattctacgt gtaacgcacg atcgattgat gagtcatttg ttttgcaata 5280
tgatatcata caatatgact catttgtttt tcaaaaccga acttgattta cgggtagaat 5340
tctacttgta aagcacaatc aaaaagatga tgtcatttgt ttttcaaaac tgaactcgct 5400
ttacgagtag aattctacgt gtaaaacaca atcaagaaat gatgtcattt gttataaaaa 5460
taaaagctga tgtcatgttt tgcacatggc tcataactaa actcgcttta cgggtagaat 5520
tctacgcgta aaacatgatt gataattaaa taattcattt gcaagctata cgttaaatca 5580

aacggacgct cgaggttgca caacactatt atcgatttgc agttcgggac ataaatgttt 5640
aaatatatcg atgtctttgt gatgcgcgcg acatttttgt aggttattga taaaatgaac 5700
ggatacgttg cccgacatta tcattaaatc cttggcgtag aatttgtcgg gtccattgtc 5760
cgtgtgcgct agcatgcccg taacggacct cgtacttttg gcttcaaagg ttttgcgcac 5820
agacaaaatg tgccacactt gcagctctgc atgtgtgcgc gttaccacaa atcccaacgg 5880
cgcagtgtac ttgttgtatg caaataaatc tcgataaagg cgcggcgcgc gaatgcagct 5940
gatcacgtac gctcctcgtg ttccgttcaa ggacggtgtt atcgacctca gattaatgtt 6000
tatcggccga ctgttttcgt atccgctcac caaacgcgtt tttgcattaa cattgtatgt 6060
cggcggatgt tctatatcta atttgaataa ataaacgata accgcgttgg ttttagaggg 6120
cataataaaa gaaatattgt tatcgtgttc gccattaggg cagtataaat tgacgttcat 6180
gttggatatt gtttcagttg caagttgaca ctggcggcga caagcaattc taattggggt 6240
aagttttccc gttcttttct gggttcttcc cttttgctca tccttgctgc actaccttca 6300
ggtgcaagtt gagattcagg ccaccatggg agatcccacc ccacccaaga agaagcgcaa 6360
accggtcgcc accatggcct cctccgagaa cgtcatcacc gagttcatgc gcttcaaggt 6420
gcgcatggag ggcaccgtga acggccacga gttcgagatc gagggcgagg gcgagggccg 6480
cccctacgag ggccacaaca ccgtgaagct gaaggtgacc aagggcggcc ccctgccctt 6540
cgcctgggac atcctgtccc cccagttcca gtacggctcc aaggtgtacg tgaagcaccc 6600
cgccgacatc cccgactaca agaagctgtc cttccccgag ggcttcaagt gggagcgcgt 6660
gatgaacttc gaggacggcg gcgtggcgac cgtgacccag gactcctccc tgcaggacgg 6720
ctgcttcatc tacaaggtga agttcatcgg cgtgaacttc ccctccgacg gccccgtgat 6780
gcagaagaag accatgggct gggaggcctc caccgagcgc ctgtaccccc gcgacggcgt 6840
gctgaagggc gagacccaca aggccctgaa gctgaaggac ggcggccact acctggtgga 6900
gttcaagtcc atctacatgg ccaagaagcc cgtgcagctg cccggctact actacgtgga 6960
cgccaagctg gacatcacct cccacaacga ggactacacc atcgtggagc agtacgagcg 7020
caccgagggc cgccaccacc tgttcctgag atctcgaccc aagaaaaagc ggaaggtgga 7080
ggacccgtaa gatccaccgg atctagataa ctgatcataa tcagccatac cacatttgta 7140
gaggttttac ttgctttaaa aaacctccca cacctccccc tgaacctgaa acataaaatg 7200
aatgcaattg ttgttgttaa cttgtttatt gcagcttata atggttacaa ataaagcaat 7260
agcatcacaa atttcacaaa taaagcattt ttttcactgc attctagttg tggtttgtcc 7320
aaactcatca atgtatctta acgcgagtta attaaggccg ctcatttaaa tctggccggc 7380
cgcaaccatt gtgggaaccg tgcgatcaaa caaacgcgag ataccggaag tactgaaaaa 7440

cagtcgctcc aggccagtgg gaacatcgat gttttgtttt gacggacccc ttactctcgt 7500
ctcatataaa ccgaagccag ctaagatggt atacttatta tcatcttgtg atgaggatgc 7560
ttctatcaac gaaagtaccg gtaaaccgca aatggttatg tattataatc aaactaaagg 7620
cggagtggac acgctagacc aaatgtgttc tgtgatgacc tgcagtagga agacgaatag 7680
gtggcctatg gcattattgt acggaatgat aaacattgcc tgcataaatt cttttattat 7740
atacagccat aatgtcagta gcaagggaga aaaggtccaa agtcgcaaaa aatttatgag 7800
aaacctttac atgagcctga cgtcatcgtt tatgcgtaag cgtttagaag ctcctacttt 7860
gaagagatat ttgcgcgata atatctctaa tattttgcca aatgaagtgc ctggtacatc 7920
agatgacagt actgaagagc cagtaatgaa aaaacgtact tactgtactt actgcccctc 7980
taaaataagg cgaaaggcaa atgcatcgtg caaaaaatgc aaaaaagtta tttgtcgaga 8040
gcataatatt gatatgtgcc aaagttgttt ctgactgact aataagtata atttgtttct 8100
attatgtata agttaagcta attacttatt ttataataca acatgactgt ttttaaagta 8160
caaaataagt ttatttttgt aaaagagaga atgtttaaaa gttttgttac tttatagaag 8220
aaattttgag tttttgtttt tttttaataa ataaataaac ataaataaat tgtttgttga 8280
atttattatt agtatgtaag tgtaaatata ataaaactta atatctattc aaattaataa 8340
ataaacctcg atatacagac cgataaaaca catgcgtcaa ttttacgcat gattatcttt 8400
aacgtacgtc acaatatgat tatctttcta gggttaaata atagtttcta atttttttat 8460
tattcagcct gctgtcgtga ataccgtata tctcaacgct gtctgtgaga ttgtcgtatt 8520
ctagcctttt tagtttttcg ctcatcgact tgatattgtc cgacacattt tcgtcgattt 8580
gcgttttgat caaagacttg agcagagaca cgttaatcaa ctgttcaaat tgatccatat 8640
taacgatatc aacccgatgc gtatatggtg cgtaaaatat atttttaac cctcttatac 8700
tttgcactct gcgttaatac gcgttcgtgt acagacgtaa tcatgttttc ttttttggat 8760
aaaactccta ctgagtttga cctcatatta gaccctcaca agttgcaaaa cgtggcattt 8820
tttaccaatg aagaatttaa agttatttta aaaaatttca tcacagattt aaagaagaac 8880
caaaaattaa attatttcaa cagtttaatc gaccagttaa tcaacgtgta cacagacgcg 8940
tcggcaaaaa acacgcagcc cgacgtgttg gctaaaatta ttaaatcaac ttgtgttata 9000
gtcacggatt tgccgtccaa cgtgttcctc aaaaagttga agaccaacaa gtttacggac 9060
actattaatt atttgatttt gccccacttc attttgtggg atcacaattt tgttatattt 9120
taaacaaagc ttggcactgg ccgtcgtttt acaacgtcgt gactgggaaa accctggcgt 9180
tacccaactt aatcgccttg cagcacatcc ccctttcgcc agctggcgta atagcgaaga 9240
ggcccgcacc gatcgccctt cccaacagtt gcgcagcctg aatggcgaat ggcgcctgat 9300
gcggtatttt ctccttacgc atctgtgcgg tatttcacac cgcatatggt gcactctcag 9360

tacaatctgc tctgatgccg catagttaag ccagccccga cacccgccaa cacccgctga 9420
cgcgccctga cgggcttgtc tgctcccggc atccgcttac agacaagctg tgaccgtctc 9480
cgggagctgc atgtgtcaga ggttttcacc gtcatcaccg aaacgcgcga gacgaaaggg 9540
cctcgtgata cgcctatttt tataggttaa tgtcatgata ataatggttt cttagacgtc 9600
aggtggcact tttcggggaa atgtgcgcgg aacccctatt tgtttatttt tctaaataca 9660
ttcaaatatg tatccgctca tgagacaata accctgataa atgcttcaat aatattgaaa 9720
aaggaagagt atgagtattc aacatttccg tgtcgccctt attccctttt ttgcggcatt 9780
ttgccttcct gtttttgctc acccagaaac gctggtgaaa gtaaaagatg ctgaagatca 9840
gttgggtgca cgagtgggtt acatcgaact ggatctcaac agcggtaaga tccttgagag 9900
ttttcgcccc gaagaacgtt ttccaatgat gagcactttt aaagttctgc tatgtggcgc 9960
ggtattatcc cgtattgacg ccgggcaaga gcaactcggt cgccgcatac actattctca 10020
gaatgacttg gttgagtact caccagtcac agaaaagcat cttacggatg gcatgacagt 10080
aagagaatta tgcagtgctg ccataaccat gagtgataac actgcggcca acttacttct 10140
gacaacgatc ggaggaccga aggagctaac cgcttttttg cacaacatgg gggatcatgt 10200
aactcgcctt gatcgttggg aaccggagct gaatgaagcc ataccaaacg acgagcgtga 10260
caccacgatg cctgtagcaa tggcaacaac gttgcgcaaa ctattaactg gcgaactact 10320
tactctagct tcccggcaac aattaataga ctggatggag gcggataaag ttgcaggacc 10380
acttctgcgc tcggcccttc cggctggctg gtttattgct gataaatctg gagccggtga 10440
gcgtgggtct cgcggtatca ttgcagcact ggggccagat ggtaagccct cccgtatcgt 10500
agttatctac acgacgggga gtcaggcaac tatggatgaa cgaaatagac agatcgctga 10560
gataggtgcc tcactgatta agcattggta actgtcagac caagtttact catatatact 10620
ttagattgat ttaaaacttc atttttaatt taaaaggatc taggtgaaga tcctttttga 10680
taatctcatg accaaaatcc cttaacgtga gttttcgttc cactgagcgt cagaccccgt 10740
agaaaagatc aaaggatctt cttgagatcc tttttttctg cgcgtaatct gctgcttgca 10800
aacaaaaaaa ccaccgctac cagcggtggt ttgtttgccg gatcaagagc taccaactct 10860
ttttccgaag gtaactggct tcagcagagc gcagatacca aatactgtcc ttctagtgta 10920
gccgtagtta ggccaccact tcaagaactc tgtagcaccg cctacatacc tcgctctgct 10980
aatcctgtta ccagtggctg ctgccagtgg cgataagtcg tgtcttaccg ggttggactc 11040
aagacgatag ttaccggata aggcgcagcg gtcgggctga acggggggtt cgtgcacaca 11100
gcccagcttg gagcgaacga cctacaccga actgagatac ctacagcgtg agcattgaga 11160
aagcgccacg cttcccgaag ggagaaaggc ggacaggtat ccggtaagcg gcagggtcgg 11220

aacaggagag cgcacgaggg agcttccagg gggaaacgcc tggtatcttt atagtcctgt 11280

cgggtttcgc cacctctgac ttgagcgtcg atttttgtga tgctcgtcag gggggcggag 11340

cctatggaaa aacgccagca acgcggcctt tttacggttc ctggcctttt gctggccttt 11400

tgctcacatg ttctttcctg cgttatcccc tgattctgtg gataaccgta ttaccgcctt 11460

tgagtgagct gataccgctc gccgcagccg aacgaccgag cgcagcgagt cagtgagcga 11520

ggaagcggaa gagcgcccaa tacgcaaacc gcctctcccc gcgcgttggc cgattcatta 11580

atgcagctgg cacgacaggt ttcccgactg gaaagcgggc agtgagcgca acgcaattaa 11640

tgtgagttag ctcactcatt aggcacccca ggctttacac tttatgcttc cggctcgtat 11700

gttgtgtgga attgtgagcg gataacaatt tcacacagga aacagctatg accatgatta 11760

cgaatttcga cctgcaggca tgcaagcttg catgcctgca ggtcgacgct cgcgcgactt 11820

ggtttgccat tctttagcgc gcgtcgcgtc acacagcttg gccacaat 11868

<210> 50
<211> 11868
<212> DNA
<213> artificial

<220>
<223> Sequence of pLA3077-a Cctra intron-tTAV construct.

<400> 50
gtggtttttg tcaaacgaag attctatgac gtgtttaaag tttaggtcga gtaaagcgca 60

aatctttttt aaccctagaa agatagtctg cgtaaaattg acgcatgcat tcttgaaata 120

ttgctctctc tttctaaata gcgcgaatcc gtcgctgtgc atttaggaca tctcagtcgc 180

cgcttggagc tcccgtgagg cgtgcttgtc aatgcggtaa gtgtcactga ttttgaacta 240

taacgaccgc gtgagtcaaa atgacgcatg attatctttt acgtgacttt taagatttaa 300

ctcatacgat aattatattg ttatttcatg ttctacttac gtgataactt attatatata 360

tattttcttg ttatagatat cgtgactaat atataataaa atgggtagtt ctttagacga 420

tgagcatatc ctctctgctc ttctgcaaag cgatgacgag cttgttggtg aggattctga 480

cagtgaaata tcagatcacg taagtgaaga tgacgtccag agcgatacag aagaagcgtt 540

tatagatgag gtacatgaag tgcagccaac gtcaagcggt agtgaaatat tagacgaaca 600

aaatgttatt gaacaaccag gttcttcatt ggcttctaac agaatcttga ccttgccaca 660

gaggactatt agaggtaaga ataaacattg ttggtcaact tcaaagtcca cgaggcgtag 720

ccgagtctct gcactgaaca ttgtcagatc ggcccgggcg ccgtttttct tgaaatattg 780

ctctctcttt ctaaatagcg cgaatccgtc gctgtgcatt taggacatct cagtcgccgc 840

ttggagctcc caaacgcgcc agtggtagta cacagtactg tgggtgttca gtttgaaatc 900

ctcttgcttc tccattgtct cggttacctt tggtcaaatc catgggttct attgcctata 960

tactcttgcg attaccagtg attgcgctat tagctattag atggattgtt ggccaaactt 1020
gtcgcttaag tggctgggaa ttgtaaccgt aggcccgagt gtaatgatcc cccataaaaa 1080
gttttcgcaa tgcctttatt ttttgttgca aatctctctt tattctgcgg tattcttcat 1140
tattgcgggg atggggaaag tgtttatata gaagcaactt acgattgaac ccaaatgcac 1200
ctgacaagca aggtcaaagg gccagatttt taaatatatt atttagtctt aggactctct 1260
atttgcaatt aaattacttt gctacctgag ggttaaatct tccccattga taataataat 1320
tccactatat gttcaattgg gtttcaccgc gcttagttac atgacgagcc ctaatgagcc 1380
gtcggtggtc tataaactgt gccttacaaa tacttgcaac tcttctcgtt ttgaagtcag 1440
cagagttatt gctaattgct aattgctaat tgcttttaac tgatttcttc gaaattggtg 1500
ctatgtttat ggcgctatta acaagtatga atgtcaggtt taaccagggg atgcttaatt 1560
gtgttctcaa cttcaaaggc agaaatgttt actcttgacc atgggtttag gtataatgtt 1620
atcaagctcc tcgagttaac gttacgttaa cgttaacgtt cgaggtcgac tctagaacta 1680
cccaccgtac tcgtcaattc caagggcatc ggtaaacatc tgctcaaact cgaagtcggc 1740
catatccaga gcgccgtagg gggcggagtc gtggggggta aatcccggac ccggggaatc 1800
cccgtccccc aacatgtcca gatcgaaatc gtctagcgcg tcggcatgcg ccatcgccac 1860
gtcctcgccg tctaagtgga gctcgtcccc caggctgaca tcggtcgggg gggccgtcga 1920
cagtctgcgc gtgtgtcccg cggggagaaa ggacaggcgc ggagccgcca gccccgcctc 1980
ttcgggggcg tcgtcgtccg ggagatcgag caggccctcg atggtagacc cgtaattgtt 2040
tttcgtacgc gcgcggctgt acgcggggcc cgagcccgac tcgcatttca gttgcttttc 2100
caatccgcag ataatcagct ccaagccgaa caggaatgcc ggctcggctc cttgatgatc 2160
gaacagctcg attgcctgac gcagcagtgg gggcatcgaa tcggttgttg gggtctcgcg 2220
ctcctctttt gcgacttgat gctcttggtc ctccagcacg cagcccaggg taaagtgacc 2280
gacggcgctc agagcgtaga gagcattttc caggctgaag ccttgctggc acaggaacgc 2340
gagctggttc tccagtgtct cgtattgctt ttcggtcggg cgcgtgccga gatggacttt 2400
ggcaccgtct cggtgggaca gcagagcgca gcggaacgac ttggcgttat tgcggaggaa 2460
gtcctgccag gactcgcctt ccaacgggca aaaatgcgtg tggtggcggt cgagcatctc 2520
gatggccagg gcatccagca gcgcccgctt attcttcacg tgccagtaga gggtgggctg 2580
ctccacgccc agcttctgcg ccaacttgcg ggtcgtcagt ccctcaatac ctatagatac 2640
catagatgta tggattagta tcatatacat acaaaggcta tttttgggac atattaatat 2700
taacaatttc cgtgatagtt ttcaccattt ttgttgaatg ttacgttgaa aatttaaatt 2760
tgttttaaat taattttacc agtcatgtgt tcttaaaagt tttatgatt gaaacggcat 2820

aaagtggttc aaaaatttat caagaaaggc tttccttttt taaatcttat ctttttctct 2880
taaaaatcac tagtcaattc attattaatt tgttaacttg aatttggaat gtctatttac 2940
tttcagataa attaaagcaa gaaacttaat attcgaaaaa aattgattct aaatggaatt 3000
tcacttgatc ttcatgtatg catatcaatt tttatttaca ttgtataata agtttcgagt 3060
tgattgttgt aatccacagg tgtcccagag aattaaattc caaattaccc aagtttattg 3120
aatgttgatt gtagtttcag ttgctttgtt gctgcaacaa tggcttgttg attgtagata 3180
ttttcccttt ccttggttta cttattacat agactgaaaa agaggtttac ttttttgata 3240
cttatgaaaa atttctatta gtgattacta accaatcgct atatgtttac tagaaaacaa 3300
ataaactctt tacattaaca ttcaataatg tttgctctgt aaccgacaat tgaaggcgtt 3360
acagcaacag taatataact agcttcttaa ccctcatcta ttaaccccat cgtttaaaac 3420
actatgttaa atggtctaac aaatctagat actaatagat gtcttattac ttagcagcca 3480
cagctgcaac atccaagaca atttttgaaa cttcttattg agctcttggc agcagaaatg 3540
ttggtatttt tcacagcttt ctgaaagacc ggcaccttcc tccggttccc gtttctgaat 3600
tcaagaggat ttccgacccc caattaatcc cgaaacaaat aaggtatatt caaaatgatg 3660
gaaaagtcat ggctgctgac cttattttta ttcctattga tagaatatta ttccccttttt 3720
aaatacactg tactaagagg tccggctata attttactca cttgtcgatt atcccataga 3780
atgttgattg tagttggttg cttttccagg tgagagttga tcaagtcaca aaagttagcg 3840
tgtgttgatt gtagatttga aggtaaaata atttttgcac ccattcatcg ggtaaaacgt 3900
tctccataga atacatttcc atcgataatt gataacttat gaatttcaaa gaaaaaaata 3960
tgcttttaaa attaccaact tcgttcaaca gctccaacgc ggagttgatg actttggact 4020
tatccaggcg gctgcccatg gtggtttcta aaggtgttat aaatcaaatt agttttgttt 4080
tttcttgaaa actttgcgtt tcctttgatc aacttaccgc cagggtacct gcagattgtt 4140
tagcttgttc agctgcgctt gtttatttgc ttagctttcg cttagcgacg tgttcacttt 4200
gcttgtttga attgaattgt cgctccgtag acgaagcgcc tctatttata ctccggcgct 4260
cgttttcgag tttaccactc cctatcagtg atagagaaaa gtgaaagtcg agtttaccac 4320
tccctatcag tgatagagaa aagtgaaagt cgagtttacc actccctatc agtgatagag 4380
aaaagtgaaa gtcgagttta ccactcccta tcagtgatag agaaaagtga aagtcgagtt 4440
taccactccc tatcagtgat agagaaaagt gaaagtcgag tttaccactc cctatcagtg 4500
atagagaaaa gtgaaagtcg agtttaccac tccctatcag tgatagagaa aagtgaaagt 4560
cgaaacctgg cgcgccccgg ccatcgagaa agagagagag aagagaagag agagaacatt 4620
cgagaaagag agagagaaga gaagagagag aacatactcc ctatcagtga tagagaagtc 4680
cctatcagtg atagagatgt ccctatcagt gatagagagt tccctatcag tgatagagac 4740

gtccctatca gtgatagaga agtccctatc agtgatagag agatccctat cagtgataga 4800
gatttcccta tcagtgatag agaggtccct atcagtgata gagacttccc tatcagtgat 4860
agagaaatcc ctatcagtga tagagacatc cctatcagtg atagagaact ccctatcagt 4920
gatagagacc tccctatcag tgatagagat cgatgcggcc gcatggtacc cattgcttgt 4980
catttattaa tttggatgat gtcatttgtt tttaaaattg aactggcttt acgagtagaa 5040
ttctacgcgt aaaacacaat caagtatgag tcataatctg atgtcatgtt ttgtacacgg 5100
ctcataaccg aactggcttt acgagtagaa ttctacttgt aatgcacgat cagtggatga 5160
tgtcatttgt ttttcaaatc gagatgatgt catgttttgc acacggctca taaactcgct 5220
ttacgagtag aattctacgt gtaacgcacg atcgattgat gagtcatttg ttttgcaata 5280
tgatatcata caatatgact catttgtttt tcaaaaccga acttgattta cgggtagaat 5340
tctacttgta aagcacaatc aaaaagatga tgtcatttgt ttttcaaaac tgaactcgct 5400
ttacgagtag aattctacgt gtaaaacaca atcaagaaat gatgtcattt gttataaaaa 5460
taaaagctga tgtcatgttt tgcacatggc tcataactaa actcgcttta cgggtagaat 5520
tctacgcgta aaacatgatt gataattaaa taattcattt gcaagctata cgttaaatca 5580
aacggacgct cgaggttgca caacactatt atcgatttgc agttcgggac ataaatgttt 5640
aaatatatcg atgtctttgt gatgcgcgcg acatttttgt aggttattga taaaatgaac 5700
ggatacgttg cccgacatta tcattaaatc cttggcgtag aatttgtcgg gtccattgtc 5760
cgtgtgcgct agcatgcccg taacggacct cgtacttttg gcttcaaagg ttttgcgcac 5820
agacaaaatg tgccacactt gcagctctgc atgtgtgcgc gttaccacaa atcccaacgg 5880
cgcagtgtac ttgttgtatg caaataaatc tcgataaagg cgcggcgcgc gaatgcagct 5940
gatcacgtac gctcctcgtg ttccgttcaa ggacggtgtt atcgacctca gattaatgtt 6000
tatcggccga ctgttttcgt atccgctcac caaacgcgtt tttgcattaa cattgtatgt 6060
cggcggatgt tctatatcta atttgaataa ataaacgata accgcgttgg ttttagaggg 6120
cataataaaa gaaatattgt tatcgtgttc gccattaggg cagtataaat tgacgttcat 6180
gttggatatt gtttcagttg caagttgaca ctggcggcga caagcaattc taattggggt 6240
aagttttccc gttcttttct gggttcttcc cttttgctca tccttgctgc actaccttca 6300
ggtgcaagtt gagattcagg ccaccatggg agatcccacc ccacccaaga agaagcgcaa 6360
accggtcgcc accatggcct cctccgagaa cgtcatcacc gagttcatgc gcttcaaggt 6420
gcgcatggag ggcaccgtga acggccacga gttcgagatc gagggcgagg gcgagggccg 6480
cccctacgag ggccacaaca ccgtgaagct gaaggtgacc aagggcggcc ccctgccctt 6540
cgcctgggac atcctgtccc cccagttcca gtacggctcc aaggtgtacg tgaagcaccc 6600

cgccgacatc cccgactaca agaagctgtc cttccccgag ggcttcaagt gggagcgcgt 6660
gatgaacttc gaggacggcg gcgtggcgac cgtgacccag gactcctccc tgcaggacgg 6720
ctgcttcatc tacaaggtga agttcatcgg cgtgaacttc ccctccgacg gccccgtgat 6780
gcagaagaag accatgggct gggaggcctc caccgagcgc ctgtaccccc gcgacggcgt 6840
gctgaagggc gagacccaca aggccctgaa gctgaaggac ggcggccact acctggtgga 6900
gttcaagtcc atctacatgg ccaagaagcc cgtgcagctg cccggctact actacgtgga 6960
cgccaagctg gacatcacct cccacaacga ggactacacc atcgtggagc agtacgagcg 7020
caccgagggc cgccaccacc tgttcctgag atctcgaccc aagaaaaagc ggaaggtgga 7080
ggacccgtaa gatccaccgg atctagataa ctgatcataa tcagccatac cacatttgta 7140
gaggttttac ttgctttaaa aaacctccca cacctccccc tgaacctgaa acataaaatg 7200
aatgcaattg ttgttgttaa cttgtttatt gcagcttata atggttacaa ataaagcaat 7260
agcatcacaa atttcacaaa taaagcattt ttttcactgc attctagttg tggtttgtcc 7320
aaactcatca atgtatctta acgcgagtta attaaggccg ctcatttaaa tctggccggc 7380
cgcaaccatt gtgggaaccg tgcgatcaaa caaacgcgag ataccggaag tactgaaaaa 7440
cagtcgctcc aggccagtgg gaacatcgat gttttgtttt gacggacccc ttactctcgt 7500
ctcatataaa ccgaagccag ctaagatggt atacttatta tcatcttgtg atgaggatgc 7560
ttctatcaac gaaagtaccg gtaaaccgca aatggttatg tattataatc aaactaaagg 7620
cggagtggac acgctagacc aaatgtgttc tgtgatgacc tgcagtagga agacgaatag 7680
gtggcctatg gcattattgt acggaatgat aaacattgcc tgcataaatt cttttattat 7740
atacagccat aatgtcagta gcaagggaga aaaggtccaa agtcgcaaaa aatttatgag 7800
aaacctttac atgagcctga cgtcatcgtt tatgcgtaag cgtttagaag ctcctacttt 7860
gaagagatat ttgcgcgata atatctctaa tattttgcca aatgaagtgc ctggtacatc 7920
agatgacagt actgaagagc cagtaatgaa aaaacgtact tactgtactt actgcccctc 7980
taaaataagg cgaaaggcaa atgcatcgtg caaaaaatgc aaaaaagtta tttgtcgaga 8040
gcataatatt gatatgtgcc aaagttgttt ctgactgact aataagtata atttgtttct 8100
attatgtata agttaagcta attacttatt ttataataca acatgactgt ttttaaagta 8160
caaaataagt ttatttttgt aaaagagaga atgtttaaaa gttttgttac tttatagaag 8220
aaattttgag tttttgtttt tttttaataa ataaataaac ataaataaat tgtttgttga 8280
atttattatt agtatgtaag tgtaaatata ataaaactta atatctattc aaattaataa 8340
ataaacctcg atatacagac cgataaaaca catgcgtcaa ttttacgcat gattatcttt 8400
aacgtacgtc acaatatgat tatctttcta gggttaaata atagtttcta atttttttat 8460
tattcagcct gctgtcgtga ataccgtata tctcaacgct gtctgtgaga ttgtcgtatt 8520

ctagcctttt tagtttttcg ctcatcgact tgatattgtc cgacacattt tcgtcgattt 8580
gcgttttgat caaagacttg agcagagaca cgttaatcaa ctgttcaaat tgatccatat 8640
taacgatatc aacccgatgc gtatatggtg cgtaaaatat atttttaac cctcttatac 8700
tttgcactct gcgttaatac gcgttcgtgt acagacgtaa tcatgttttc ttttttggat 8760
aaaactccta ctgagtttga cctcatatta gaccctcaca agttgcaaaa cgtggcattt 8820
tttaccaatg aagaatttaa agttatttta aaaaatttca tcacagattt aaagaagaac 8880
caaaaattaa attatttcaa cagtttaatc gaccagttaa tcaacgtgta cacagacgcg 8940
tcggcaaaaa acacgcagcc cgacgtgttg gctaaaatta ttaaatcaac ttgtgttata 9000
gtcacggatt tgccgtccaa cgtgttcctc aaaaagttga agaccaacaa gtttacggac 9060
actattaatt atttgatttt gccccacttc attttgtggg atcacaattt tgttatattt 9120
taaacaaagc ttggcactgg ccgtcgtttt acaacgtcgt gactgggaaa accctggcgt 9180
tacccaactt aatcgccttg cagcacatcc ccctttcgcc agctggcgta atagcgaaga 9240
ggcccgcacc gatcgccctt cccaacagtt gcgcagcctg aatggcgaat ggcgcctgat 9300
gcggtatttt ctccttacgc atctgtgcgg tatttcacac cgcatatggt gcactctcag 9360
tacaatctgc tctgatgccg catagttaag ccagccccga cacccgccaa cacccgctga 9420
cgcgccctga cgggcttgtc tgctcccggc atccgcttac agacaagctg tgaccgtctc 9480
cgggagctgc atgtgtcaga ggttttcacc gtcatcaccg aaacgcgcga gacgaaaggg 9540
cctcgtgata cgcctatttt tataggttaa tgtcatgata ataatggttt cttagacgtc 9600
aggtggcact tttcggggaa atgtgcgcgg aacccctatt tgtttatttt tctaaataca 9660
ttcaaatatg tatccgctca tgagacaata accctgataa atgcttcaat aatattgaaa 9720
aaggaagagt atgagtattc aacatttccg tgtcgccctt attccctttt ttgcggcatt 9780
ttgccttcct gtttttgctc acccagaaac gctggtgaaa gtaaaagatg ctgaagatca 9840
gttgggtgca cgagtgggtt acatcgaact ggatctcaac agcggtaaga tccttgagag 9900
ttttcgcccc gaagaacgtt ttccaatgat gagcactttt aaagttctgc tatgtggcgc 9960
ggtattatcc cgtattgacg ccgggcaaga gcaactcggt cgccgcatac actattctca 10020
gaatgacttg gttgagtact caccagtcac agaaaagcat cttacggatg gcatgacagt 10080
aagagaatta tgcagtgctg ccataaccat gagtgataac actgcggcca acttacttct 10140
gacaacgatc ggaggaccga aggagctaac cgcttttttg cacaacatgg gggatcatgt 10200
aactcgcctt gatcgttggg aaccggagct gaatgaagcc ataccaaacg acgagcgtga 10260
caccacgatg cctgtagcaa tggcaacaac gttgcgcaaa ctattaactg gcgaactact 10320
tactctagct tcccggcaac aattaataga ctggatggag gcggataaag ttgcaggacc 10380

acttctgcgc tcggcccttc cggctggctg gtttattgct gataaatctg gagccggtga 10440

gcgtgggtct cgcggtatca ttgcagcact ggggccagat ggtaagccct cccgtatcgt 10500

agttatctac acgacgggga gtcaggcaac tatggatgaa cgaaatagac agatcgctga 10560

gataggtgcc tcactgatta agcattggta actgtcagac caagtttact catatatact 10620

ttagattgat ttaaaacttc atttttaatt taaaaggatc taggtgaaga tcctttttga 10680

taatctcatg accaaaatcc cttaacgtga gttttcgttc cactgagcgt cagaccccgt 10740

agaaaagatc aaaggatctt cttgagatcc tttttttctg cgcgtaatct gctgcttgca 10800

aacaaaaaaa ccaccgctac cagcggtggt ttgtttgccg gatcaagagc taccaactct 10860

ttttccgaag gtaactggct tcagcagagc gcagatacca aatactgtcc ttctagtgta 10920

gccgtagtta ggccaccact tcaagaactc tgtagcaccg cctacatacc tcgctctgct 10980

aatcctgtta ccagtggctg ctgccagtgg cgataagtcg tgtcttaccg ggttggactc 11040

aagacgatag ttaccggata aggcgcagcg gtcgggctga acggggggtt cgtgcacaca 11100

gcccagcttg gagcgaacga cctacaccga actgagatac ctacagcgtg agcattgaga 11160

aagcgccacg cttcccgaag ggagaaaggc ggacaggtat ccggtaagcg gcagggtcgg 11220

aacaggagag cgcacgaggg agcttccagg gggaaacgcc tggtatcttt atagtcctgt 11280

cgggtttcgc cacctctgac ttgagcgtcg atttttgtga tgctcgtcag gggggcggag 11340

cctatggaaa aacgccagca acgcggcctt tttacggttc ctggcctttt gctggccttt 11400

tgctcacatg ttctttcctg cgttatcccc tgattctgtg gataaccgta ttaccgcctt 11460

tgagtgagct gataccgctc gccgcagccg aacgaccgag cgcagcgagt cagtgagcga 11520

ggaagcggaa gagcgcccaa tacgcaaacc gcctctcccc gcgcgttggc cgattcatta 11580

atgcagctgg cacgacaggt ttcccgactg gaaagcgggc agtgagcgca acgcaattaa 11640

tgtgagttag ctcactcatt aggcacccca ggctttacac tttatgcttc cggctcgtat 11700

gttgtgtgga attgtgagcg gataacaatt tcacacagga aacagctatg accatgatta 11760

cgaatttcga cctgcaggca tgcaagcttg catgcctgca ggtcgacgct cgcgcgactt 11820

ggtttgccat tctttagcgc gcgtcgcgtc acacagcttg gccacaat 11868

<210> 51
<211> 11788
<212> DNA
<213> artificial

<220>
<223> 51 Sequence of pLA3097-a Cctra intron-tTAV construct.

<400> 51
gggcggccgt tttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180
caaatccatg ggttctattg cctatatact cttgcgatta ccagtgattg cgctattagc 240
tattagatgg attgttggcc aaacttgtcg cttaagtggc tgggaattgt aaccgtaggc 300
ccgagtgtaa tgatccccca taaaaagttt tcgcaatgcc tttatttttt gttgcaaatc 360
tctctttatt ctgcggtatt cttcattatt gcggggatgg ggaaagtgtt tatatagaag 420
caacttacga ttgaacccaa atgcacctga caagcaaggt caaagggcca gatttttaaa 480
tatattattt agtcttagga ctctctattt gcaattaaat tactttgcta cctgagggtt 540
aaatcttccc cattgataat aataattcca ctatatgttc aattgggttt caccgcgctt 600
agttacatga cgagccctaa tgagccgtcg gtggtctata aactgtgcct tacaaatact 660
tgcaactctt ctcgttttga agtcagcaga gttattgcta attgctaatt gctaattgct 720
tttaactgat ttcttcgaaa ttggtgctat gtttatggcg ctattaacaa gtatgaatgt 780
caggtttaac caggggatgc ttaattgtgt tctcaacttc aaaggcagaa atgtttactc 840
ttgaccatgg gtttaggtat aatgttatca agctcctcga gttaacgtta cgttaacgtt 900
aacgttcgag gtcgactcta gaactaccca ccgtactcgt caattccaag ggcatcggta 960
aacatctgct caaactcgaa gtcggccata tccagagcgc cgtagggggc ggagtcgtgg 1020
ggggtaaatc ccggacccgg ggaatccccg tcccccaaca tgtccagatc gaaatcgtct 1080
agcgcgtcgg catgcgccat cgccacgtcc tcgccgtcta agtggagctc gtcccccagg 1140
ctgacatcgg tcgggggggc cgtcgacagt ctgcgcgtgt gtcccgcggg gagaaaggac 1200
aggcgcggag ccgccagccc cgcctcttcg gggcgtcgt cgtccgggag atcgagcagg 1260
ccctcgatgg tagacccgta attgtttttc gtacgcgcgc ggctgtacgc ggggcccgag 1320
cccgactcgc atttcagttg cttttccaat ccgcagataa tcagctccaa gccgaacagg 1380
aatgccggct cggctccttg atgatcgaac agctcgattg cctgacgcag cagtgggggc 1440
atcgaatcgg ttgttggggt ctcgcgctcc tcttttgcga cttgatgctc ttggtcctcc 1500
agcacgcagc ccagggtaaa gtgaccgacg gcgctcagag cgtagagagc attttccagg 1560
ctgaagcctt gctggcacag gaacgcgagc tggttctcca gtgtctcgta ttgcttttcg 1620
gtcgggcgcg tgccgagatg gactttggca ccgtctcggt gggacagcag agcgcagcgg 1680
aacgacttgg cgttattgcg gaggaagtcc tgccaggact cgccttccaa cgggcaaaaa 1740
tgcgtgtggt ggcggtcgag catctcgatg gccagggcat ccagcagcgc ccgcttattc 1800
ttcacgtgcc agtagagggt gggctgctcc acgcccagct tctgcgccaa cttgcgggtc 1860
gtcagtccct caatgccaac ttcgttcaac agctccaacg cggagttgat gactttggac 1920
ttatccaggc ggctgaccta tagataccat agatgtatgg attagtatca tatacataca 1980

aaggctattt ttgggacata ttaatattaa caatttccgt gatagttttc accatttttg 2040
ttgaatgtta cgttgaaaat ttaaatttgt tttaaattaa ttttaccagt catgtgttct 2100
taaaagtttt tatgattgaa acggcataaa gtggttcaaa aatttatcaa gaaaggcttt 2160
cctttttaa atcttatctt tttctcttaa aaatcactag tcaattcatt attaatttgt 2220
taacttgaat ttggaatgtc tatttacttt cagataaatt aaagcaagaa acttaatatt 2280
cgaaaaaaat tgattctaaa tggaatttca cttgatcttc atgtatgcat atcaattttt 2340
atttacattg tataataagt ttcgagttga ttgttgtaat ccacaggtgt cccagagaat 2400
taaattccaa attacccaag tttattgaat gttgattgta gtttcagttg ctttgttgct 2460
gcaacaatgg cttgttgatt gtagatattt tccctttcct tggtttactt attacataga 2520
ctgaaaaaga ggtttacttt tttgatactt atgaaaaatt tctattagtg attactaacc 2580
aatcgctata tgtttactag aaaacaaata aactctttac attaacattc aataatgttt 2640
gctctgtaac cgacaattga aggcgttaca gcaacagtaa tataactagc ttcttaaccc 2700
tcatctatta accccatcgt ttaaaacact atgttaaatg gtctaacaaa tctagatact 2760
aatagatgtc ttattactta gcagccacag ctgcaacatc caagacaatt tttgaaactt 2820
cttattgagc tcttggcagc agaaatgttg gtatttttca cagctttctg aaagaccggc 2880
accttcctcc ggttcccgtt tctgaattca agaggatttc cgaccccaa ttaatcccga 2940
aacaaataag gtatattcaa aatgatggaa aagtcatggc tgctgacctt atttttattc 3000
ctattgatag aatattattc ccttttaaa tacactgtac taagaggtcc ggctataatt 3060
ttactcactt gtcgattatc ccatagaatg ttgattgtag ttggttgctt ttccaggtga 3120
gagttgatca agtcacaaaa gttagcgtgt gttgattgta gatttgaagg taaaataatt 3180
tttgcaccca ttcatcgggt aaaacgttct ccatagaata catttccatc gataattgat 3240
aacttatgaa tttcaaagaa aaaaatatgc ttttaaaatt accatggtgg ctagcgcaga 3300
ttgtttagct tgttcagctg cgcttgttta tttgcttagc tttcgcttag cgacgtgttc 3360
actttgcttg tttgaattga attgtcgctc cgtagacgaa gcgcctctat ttatactccg 3420
gcgctcgttt tcgagtttac cactccctat cagtgataga gaaaagtgaa agtcgagttt 3480
accactccct atcagtgata gagaaaagtg aaagtcgagt ttaccactcc ctatcagtga 3540
tagagaaaag tgaaagtcga gtttaccact ccctatcagt gatagagaaa agtgaaagtc 3600
gagtttacca ctccctatca gtgatagaga aaagtgaaag tcgagtttac cactccctat 3660
cagtgataga gaaaagtgaa agtcgagttt accactccct atcagtgata gagaaaagtg 3720
aaagtcgaaa cctggcgcgc cccggccatc gagaaagaga gagagaagag aagagagaga 3780
acattcgaga aagagagaga gaagagaaga gagagaacat actccctatc agtgatagag 3840
aagtccctat cagtgataga gatgtcccta tcagtgatag agagttccct atcagtgata 3900

gagacgtccc tatcagtgat agagaagtcc ctatcagtga tagagagatc cctatcagtg 3960

atagagattt ccctatcagt gatagagagg tccctatcag tgatagagac ttccctatca 4020

gtgatagaga aatccctatc agtgatagag acatccctat cagtgataga gaactcccta 4080

tcagtgatag agacctccct atcagtgata gagatcgatg cggccgcatg gtacccattg 4140

cttgtcattt attaatttgg atgatgtcat ttgtttttaa aattgaactg gctttacgag 4200

tagaattcta cgcgtaaaac acaatcaagt atgagtcata atctgatgtc atgttttgta 4260

cacggctcat aaccgaactg gctttacgag tagaattcta cttgtaatgc acgatcagtg 4320

gatgatgtca tttgtttttc aaatcgagat gatgtcatgt tttgcacacg gctcataaac 4380

tcgctttacg agtagaattc tacgtgtaac gcacgatcga ttgatgagtc atttgttttg 4440

caatatgata tcatacaata tgactcattt gtttttcaaa accgaacttg atttacgggt 4500

agaattctac ttgtaaagca caatcaaaaa gatgatgtca tttgtttttc aaaactgaac 4560

tcgctttacg agtagaattc tacgtgtaaa acacaatcaa gaaatgatgt catttgttat 4620

aaaaataaaa gctgatgtca tgttttgcac atggctcata actaaactcg ctttacgggt 4680

agaattctac gcgtaaaaca tgattgataa ttaaataatt catttgcaag ctatacgtta 4740

aatcaaacgg acgctcgagg ttgcacaaca ctattatcga tttgcagttc gggacataaa 4800

tgtttaaata tatcgatgtc tttgtgatgc gcgcgacatt tttgtaggtt attgataaaa 4860

tgaacggata cgttgcccga cattatcatt aaatccttgg cgtagaattt gtcgggtcca 4920

ttgtccgtgt gcgctagcat gcccgtaacg gacctcgtac ttttggcttc aaaggttttg 4980

cgcacagaca aaatgtgcca cacttgcagc tctgcatgtg tgcgcgttac cacaaatccc 5040

aacggcgcag tgtacttgtt gtatgcaaat aaatctcgat aaaggcgcgg cgcgcgaatg 5100

cagctgatca cgtacgctcc tcgtgttccg ttcaaggacg gtgttatcga cctcagatta 5160

atgtttatcg gccgactgtt ttcgtatccg ctcaccaaac gcgtttttgc attaacattg 5220

tatgtcggcg gatgttctat atctaatttg aataaataaa cgataaccgc gttggtttta 5280

gagggcataa taaaagaaat attgttatcg tgttcgccat tagggcagta taaattgacg 5340

ttcatgttgg atattgtttc agttgcaagt tgacactggc ggcgacaagc aattctaatt 5400

ggggtaagtt ttcccgttct tttctgggtt cttccctttt gctcatcctt gctgcactac 5460

cttcaggtgc aagttgagat tcaggccacc atgggagatc ccaccccacc caagaagaag 5520

cgcaaaccgg tcgccaccat ggcctcctcc gagaacgtca tcaccgagtt catgcgcttc 5580

aaggtgcgca tggagggcac cgtgaacggc cacgagttcg agatcgaggg cgagggcgag 5640

ggccgcccct acgagggcca caacaccgtg aagctgaagg tgaccaaggg cggccccctg 5700

cccttcgcct gggacatcct gtcccccag ttccagtacg gctccaaggt gtacgtgaag 5760

cacccgccg acatccccga ctacaagaag ctgtccttcc ccgagggctt caagtgggag 5820
cgcgtgatga acttcgagga cggcggcgtg gcgaccgtga cccaggactc ctccctgcag 5880
gacggctgct tcatctacaa ggtgaagttc atcggcgtga acttcccctc cgacggcccc 5940
gtgatgcaga agaagaccat gggctgggag gcctccaccg agcgcctgta cccccgcgac 6000
ggcgtgctga agggcgagac ccacaaggcc ctgaagctga aggacggcgg ccactacctg 6060
gtggagttca agtccatcta catggccaag aagcccgtgc agctgcccgg ctactactac 6120
gtggacgcca agctggacat cacctcccac aacgaggact acaccatcgt ggagcagtac 6180
gagcgcaccg agggccgcca ccacctgttc ctgagatctc gacccaagaa aaagcggaag 6240
gtggaggacc cgtaagatcc accggatcta gataactgat cataatcagc cataccacat 6300
ttgtagaggt tttacttgct ttaaaaaacc tcccacacct ccccctgaac ctgaaacata 6360
aaatgaatgc aattgttgtt gttaacttgt ttattgcagc ttataatggt tacaaataaa 6420
gcaatagcat cacaaatttc acaaataaag catttttttc actgcattct agttgtggtt 6480
tgtccaaact catcaatgta tcttaacgcg agttaattaa ggccgctcat ttaaatctgg 6540
ccggccgcaa ccattgtggg aaccgtgcga tcaaacaaac gcgagatacc ggaagtactg 6600
aaaaacagtc gctccaggcc agtgggaaca tcgatgtttt gttttgacgg acccottact 6660
ctcgtctcat ataaaccgaa gccagctaag atggtatact tattatcatc ttgtgatgag 6720
gatgcttcta tcaacgaaag taccggtaaa ccgcaaatgg ttatgtatta taatcaaact 6780
aaaggcggag tggacacgct agaccaaatg tgttctgtga tgacctgcag taggaagacg 6840
aataggtggc ctatggcatt attgtacgga atgataaaca ttgcctgcat aaattctttt 6900
attatataca gccataatgt cagtagcaag ggagaaaagg tccaaagtcg caaaaaattt 6960
atgagaaacc tttacatgag cctgacgtca tcgtttatgc gtaagcgttt agaagctcct 7020
actttgaaga gatatttgcg cgataatatc tctaatattt tgccaaatga agtgcctggt 7080
acatcagatg acagtactga agagccagta atgaaaaaac gtacttactg tacttactgc 7140
ccctctaaaa taaggcgaaa ggcaaatgca tcgtgcaaaa aatgcaaaaa agttatttgt 7200
cgagagcata atattgatat gtgccaaagt tgtttctgac tgactaataa gtataatttg 7260
tttctattat gtataagtta agctaattac ttattttata atacaacatg actgttttta 7320
aagtacaaaa taagtttatt tttgtaaaag agagaatgtt taaaagtttt gttactttat 7380
agaagaaatt ttgagttttt gttttttttt aataaataaa taaacataaa taaattgttt 7440
gttgaattta ttattagtat gtaagtgtaa atataataaa acttaatatc tattcaaatt 7500
aataaataaa cctcgatata cagaccgata aaacacatgc gtcaatttta cgcatgatta 7560
tctttaacgt acgtcacaat atgattatct ttctagggtt aaataatagt ttctaatttt 7620
tttattattc agcctgctgt cgtgaatacc gtatatctca acgctgtctg tgagattgtc 7680

gtattctagc ctttttagtt tttcgctcat cgacttgata ttgtccgaca cattttcgtc 7740
gatttgcgtt ttgatcaaag acttgagcag agacacgtta atcaactgtt caaattgatc 7800
catattaacg atatcaaccc gatgcgtata tggtgcgtaa aatatatttt ttaaccctct 7860
tatactttgc actctgcgtt aatacgcgtt cgtgtacaga cgtaatcatg ttttcttttt 7920
tggataaaac tcctactgag tttgacctca tattagaccc tcacaagttg caaaacgtgg 7980
catttttttac caatgaagaa tttaaagtta ttttaaaaaa tttcatcaca gatttaaaga 8040
agaaccaaaa attaaattat ttcaacagtt taatcgacca gttaatcaac gtgtacacag 8100
acgcgtcggc aaaaaacacg cagcccgacg tgttggctaa aattattaaa tcaacttgtg 8160
ttatagtcac ggatttgccg tccaacgtgt tcctcaaaaa gttgaagacc aacaagttta 8220
cggacactat taattatttg attttgcccc acttcatttt gtgggatcac aattttgtta 8280
tattttaaac aaagcttggc actggccgtc gttttacaac gtcgtgactg ggaaaaccct 8340
ggcgttaccc aacttaatcg ccttgcagca catccccctt tcgccagctg gcgtaatagc 8400
gaagaggccc gcaccgatcg cccttcccaa cagttgcgca gcctgaatgg cgaatggcgc 8460
ctgatgcggt attttctcct tacgcatctg tgcggtattt cacaccgcat atggtgcact 8520
ctcagtacaa tctgctctga tgccgcatag ttaagccagc cccgacaccc gccaacaccc 8580
gctgacgcgc cctgacgggc ttgtctgctc ccggcatccg cttacagaca agctgtgacc 8640
gtctccggga gctgcatgtg tcagaggttt tcaccgtcat caccgaaacg cgcgagacga 8700
aagggcctcg tgatacgcct atttttatag gttaatgtca tgataataat ggtttcttag 8760
acgtcaggtg gcacttttcg gggaaatgtg cgcggaaccc ctatttgttt atttttctaa 8820
atacattcaa atatgtatcc gctcatgaga caataaccct gataaatgct tcaataatat 8880
tgaaaaagga agagtatgag tattcaacat ttccgtgtcg cccttattcc cttttttgcg 8940
gcattttgcc ttcctgtttt tgctcaccca gaaacgctgg tgaaagtaaa agatgctgaa 9000
gatcagttgg gtgcacgagt gggttacatc gaactggatc tcaacagcgg taagatcctt 9060
gagagttttc gccccgaaga acgttttcca atgatgagca cttttaaagt tctgctatgt 9120
ggcgcggtat tatcccgtat tgacgccggg caagagcaac tcggtcgccg catacactat 9180
tctcagaatg acttggttga gtactcacca gtcacagaaa agcatcttac ggatggcatg 9240
acagtaagag aattatgcag tgctgccata accatgagtg ataacactgc ggccaactta 9300
cttctgacaa cgatcggagg accgaaggag ctaaccgctt ttttgcacaa catgggggat 9360
catgtaactc gccttgatcg ttgggaaccg gagctgaatg aagccatacc aaacgacgag 9420
cgtgacacca cgatgcctgt agcaatggca acaacgttgc gcaaactatt aactggcgaa 9480
ctacttactc tagcttcccg gcaacaatta atagactgga tggaggcgga taaagttgca 9540

ggaccacttc tgcgctcggc ccttccggct ggctggttta ttgctgataa atctggagcc 9600
ggtgagcgtg ggtctcgcgg tatcattgca gcactggggc cagatggtaa gccctcccgt 9660
atcgtagtta tctacacgac ggggagtcag gcaactatgg atgaacgaaa tagacagatc 9720
gctgagatag gtgcctcact gattaagcat tggtaactgt cagaccaagt ttactcatat 9780
atactttaga ttgatttaaa acttcatttt taatttaaaa ggatctaggt gaagatcctt 9840
tttgataatc tcatgaccaa aatcccttaa cgtgagtttt cgttccactg agcgtcagac 9900
cccgtagaaa agatcaaagg atcttcttga gatccttttt ttctgcgcgt aatctgctgc 9960
ttgcaaacaa aaaaaccacc gctaccagcg gtggtttgtt tgccggatca agagctacca 10020
actctttttc cgaaggtaac tggcttcagc agagcgcaga taccaaatac tgtccttcta 10080
gtgtagccgt agttaggcca ccacttcaag aactctgtag caccgcctac atacctcgct 10140
ctgctaatcc tgttaccagt ggctgctgcc agtggcgata agtcgtgtct taccgggttg 10200
gactcaagac gatagttacc ggataaggcg cagcggtcgg gctgaacggg gggttcgtgc 10260
acacagccca gcttggagcg aacgacctac accgaactga gatacctaca gcgtgagcat 10320
tgagaaagcg ccacgcttcc cgaagggaga aaggcggaca ggtatccggt aagcggcagg 10380
gtcggaacag gagagcgcac gagggagctt ccagggggaa acgcctggta tctttatagt 10440
cctgtcgggt ttcgccacct ctgacttgag cgtcgatttt tgtgatgctc gtcagggggg 10500
cggagcctat ggaaaaacgc cagcaacgcg gcctttttac ggttcctggc cttttgctgg 10560
ccttttgctc acatgttctt tcctgcgtta tcccctgatt ctgtggataa ccgtattacc 10620
gcctttgagt gagctgatac cgctcgccgc agccgaacga ccgagcgcag cgagtcagtg 10680
agcgaggaag cggaagagcg cccaatacgc aaaccgcctc tccccgcgcg ttggccgatt 10740
cattaatgca gctggcacga caggtttccc gactggaaag cgggcagtga gcgcaacgca 10800
attaatgtga gttagctcac tcattaggca ccccaggctt tacactttat gcttccggct 10860
cgtatgttgt gtggaattgt gagcggataa caatttcaca caggaaacag ctatgaccat 10920
gattacgaat ttcgacctgc aggcatgcaa gcttgcatgc ctgcaggtcg acgctcgcgc 10980
gacttggttt gccattcttt agcgcgcgtc gcgtcacaca gcttggccac aatgtggttt 11040
ttgtcaaacg aagattctat gacgtgttta aagtttaggt cgagtaaagc gcaaatcttt 11100
tttaacccta gaaagatagt ctgcgtaaaa ttgacgcatg cattcttgaa atattgctct 11160
ctctttctaa atagcgcgaa tccgtcgctg tgcatttagg acatctcagt cgccgcttgg 11220
agctcccgtg aggcgtgctt gtcaatgcgg taagtgtcac tgattttgaa ctataacgac 11280
cgcgtgagtc aaaatgacgc atgattatct tttacgtgac ttttaagatt taactcatac 11340
gataattata ttgttatttc atgttctact tacgtgataa cttattatat atatattttc 11400
ttgttataga tatcgtgact aatatataat aaaatgggta gttctttaga cgatgagcat 11460

atcctctctg ctcttctgca aagcgatgac gagcttgttg gtgaggattc tgacagtgaa 11520

atatcagatc acgtaagtga agatgacgtc cagagcgata cagaagaagc gtttatagat 11580

gaggtacatg aagtgcagcc aacgtcaagc ggtagtgaaa tattagacga acaaaatgtt 11640

attgaacaac caggttcttc attggcttct aacagaatct tgaccttgcc acagaggact 11700

attagaggta agaataaaca ttgttggtca acttcaaagt ccacgaggcg tagccgagtc 11760

tctgcactga acattgtcag atcggccc 11788

<210> 52
<211> 13292
<212> DNA
<213> artificial

<220>
<223> Sequence of pLA3233-Cctra-intron-tTAV2 construct.

<400> 52
gggcggccgt ttttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600

agttacatga cgagccctaa tgagccgtcg gtggtctata aactgtgcct tacaaatact 660

tgcaactctt ctcgttttga agtcagcaga gttattgcta attgctaatt gctaattgct 720

tttaactgat ttcttcgaaa ttggtgctat gtttatggcg ctattaacaa gtatgaatgt 780

caggtttaac caggggatgc ttaattgtgt tctcaacttc aaaggcagaa atgtttactc 840

ttgaccatgg gtttaggtat aatgttatca agctcctcga gttaacgtta cgttaacgtt 900

aacgttcgag gtcgactcta gacaccggtg ttagccgccg tactcatcga tgcccagggc 960

gtcggtgaac atctgctcga actcgaaatc ggccatatcc agggcgccgt agggggcgct 1020

atcgtgcggg gtgaatcccg gtcccgggct atcgccatcg cccagcatgt ccaggtcgaa 1080

gtcgtccagg gcatcggcgt gggccatcgc cacatcctcg ccatccaggt gcagctcatc 1140

gcccaggctc acgtcggtcg gcggggcggt cgacaggcgg cgggtgtgtc cggccggcag 1200

gaagctcagg cgcggggcgg ccaggcccgc ctcctccggg gcatcatcat ccggcagatc 1260

cagcaggccc tcgatggtgc tgccgtagtt gttcttggtg cgggcgcggc tgtaggcggg 1320

gcccgagccc gactcgcatt tcagttgctt ttccaatccg cagataatca gctccaagcc 1380

gaacaggaat gccggctcgg ctccttgatg atcgaacagc tcgattgcct gacgcagcag 1440

tgggggcatc gaatcggttg ttggggtctc gcgctcctct tttgcgactt gatgctcttg 1500

gtcctccagc acgcagccca gggtaaagtg accgacggcg ctcagagcgt agagagcatt 1560

ttccaggctg aagccttgct ggcacaggaa cgcgagctgg ttctccagtg tctcgtattg 1620

cttttcggtc gggcgcgtgc cgagatggac tttggcaccg tctcggtggg acagcagagc 1680

gcagcggaac gacttggcgt tattgcggag gaagtcctgc caggactcgc cttccaacgg 1740

gcaaaaatgc gtgtggtggc ggtcgagcat ctcgatggcc agggcatcca gcagcgcccg 1800

cttattcttc acgtgccagt agagggtggg ctgctccacg cccagcttct gcgccaactt 1860

gcgggtcgtc agtccctcaa tgccaacttc gttcaacagc tccaacgcgg agttgatgac 1920

tttggactta tccaggcggc tgacctatag ataccataga tgtatggatt agtatcatat 1980

acatacaaag gctatttttg ggacatatta atattaacaa tttccgtgat agttttcacc 2040

atttttgttg aatgttacgt tgaaaattta aatttgtttt aaattaattt taccagtcat 2100

gtgttcttaa aagtttttat gattgaaacg gcataaagtg gttcaaaaat ttatcaagaa 2160

aggctttcct tttttaaatc ttatcttttt ctcttaaaaa tcactagtca attcattatt 2220

aatttgttaa cttgaatttg gaatgtctat ttactttcag ataaattaaa gcaagaaact 2280

taatattcga aaaaaattga ttctaaatgg aatttcactt gatcttcatg tatgcatatc 2340

aatttttatt tacattgtat aataagtttc gagttgattg ttgtaatcca caggtgtccc 2400

agagaattaa attccaaatt acccaagttt attgaatgtt gattgtagtt tcagttgctt 2460

tgttgctgca acaatggctt gttgattgta gatattttcc ctttccttgg tttacttatt 2520

acatagactg aaaaagaggt ttactttttt gatacttatg aaaaatttct attagtgatt 2580

actaaccaat cgctatatgt ttactagaaa acaaataaac tctttacatt aacattcaat 2640

aatgtttgct ctgtaaccga caattgaagg cgttacagca acagtaatat aactagcttc 2700

ttaaccctca tctattaacc ccatcgttta aaacactatg ttaaatggtc taacaaatct 2760

agatactaat agatgtctta ttacttagca gccacagctg caacatccaa gacaattttt 2820

gaaacttctt attgagctct tggcagcaga aatgttggta tttttcacag ctttctgaaa 2880

gaccggcacc ttcctccggt tcccgtttct gaattcaaga ggatttccga cccccaatta 2940

atcccgaaac aaataaggta tattcaaaat gatggaaaag tcatggctgc tgaccttatt 3000

tttattccta ttgatagaat attattcccc ttttaaatac actgtactaa gaggtccggc 3060

tataatttta ctcacttgtc gattatccca tagaatgttg attgtagttg gttgcttttc 3120

caggtgagag ttgatcaagt cacaaaagtt agcgtgtgtt gattgtagat ttgaaggtaa 3180
aataattttt gcacccattc atcgggtaaa acgttctcca tagaatacat ttccatcgat 3240
aattgataac ttatgaattt caaagaaaaa aatatgcttt taaaattacc atggtggcta 3300
gcgcagattg tttagcttgt tcagctgcgc ttgtttattt gcttagcttt cgcttagcga 3360
cgtgttcact ttgcttgttt gaattgaatt gtcgctccgt agacgaagcg cctctattta 3420
tactccggcg ctcgttttcg agtttaccac tccctatcag tgatagagaa aagtgaaagt 3480
cgagtttacc actccctatc agtgatagag aaaagtgaaa gtcgagttta ccactcccta 3540
tcagtgatag agaaaagtga aagtcgagtt taccactccc tatcagtgat agagaaaagt 3600
gaaagtcgag tttaccactc cctatcagtg atagagaaaa gtgaaagtcg agtttaccac 3660
tccctatcag tgatagagaa aagtgaaagt cgagtttacc actccctatc agtgatagag 3720
aaaagtgaaa gtcgaaacct ggcgcgcccc ggccatcgag aaagagagag agaagagaag 3780
agagagaaca ttcgagaaag agagagagaa gagaagagag agaacatact ccctatcagt 3840
gatagagaag tccctatcag tgatagagat gtccctatca gtgatagaga gttccctatc 3900
agtgatagag acgtccctat cagtgataga gaagtcccta tcagtgatag agagatccct 3960
atcagtgata gagatttccc tatcagtgat agagaggtcc ctatcagtga tagagacttc 4020
cctatcagtg atagagaaat ccctatcagt gatagagaca tccctatcag tgatagagaa 4080
ctccctatca gtgatagaga cctccctatc agtgatagag atcgatgcgg ccgcatggta 4140
cccattgctt gtcatttatt aatttggatg atgtcatttg tttttaaaat tgaactggct 4200
ttacgagtag aattctacgc gtaaaacaca atcaagtatg agtcataatc tgatgtcatg 4260
ttttgtacac ggctcataac cgaactggct ttacgagtag aattctactt gtaatgcacg 4320
atcagtggat gatgtcattt gtttttcaaa tcgagatgat gtcatgtttt gcacacggct 4380
cataaactcg ctttacgagt agaattctac gtgtaacgca cgatcgattg atgagtcatt 4440
tgttttgcaa tatgatatca tacaatatga ctcatttgtt tttcaaaacc gaacttgatt 4500
tacgggtaga attctacttg taaagcacaa tcaaaaagat gatgtcattt gtttttcaaa 4560
actgaactcg ctttacgagt agaattctac gtgtaaaaca caatcaagaa atgatgtcat 4620
ttgttataaa aataaaagct gatgtcatgt tttgcacatg gctcataact aaactcgctt 4680
tacgggtaga attctacgcg taaaacatga ttgataatta aataattcat ttgcaagcta 4740
tacgttaaat caaacggacg ctcgaggttg cacaacacta ttatcgattt gcagttcggg 4800
acataaatgt ttaaatatat cgatgtcttt gtgatgcgcg cgacattttt gtaggttatt 4860
gataaaatga acggatacgt tgcccgacat tatcattaaa tccttggcgt agaatttgtc 4920
gggtccattg tccgtgtgcg ctagcatgcc cgtaacggac ctcgtacttt tggcttcaaa 4980

ggttttgcgc acagacaaaa tgtgccacac ttgcagctct gcatgtgtgc gcgttaccac 5040
aaatcccaac ggcgcagtgt acttgttgta tgcaaataaa tctcgataaa ggcgcggcgc 5100
gcgaatgcag ctgatcacgt acgctcctcg tgttccgttc aaggacggtg ttatcgacct 5160
cagattaatg tttatcggcc gactgttttc gtatccgctc accaaacgcg tttttgcatt 5220
aacattgtat gtcggcggat gttctatatc taatttgaat aaataaacga taaccgcgtt 5280
ggttttagag ggcataataa aagaaatatt gttatcgtgt tcgccattag ggcagtataa 5340
attgacgttc atgttggata ttgtttcagt tgcaagttga cactggcggc gacaagcaat 5400
tctaattggg gtaagttttc ccgttctttt ctgggttctt ccctttttgct catccttgct 5460
gcactacctt caggtgcaag ttgagattca ggccaccatg ggagatccca ccccacccaa 5520
gaagaagcgc aaaccggtcg ccaccatgga cgaggatggt tcagagggcg gccccgccct 5580
gttccagagc gacatgacct tcaaaatctt catcgacggc gaggtgaacg gccagaagtt 5640
caccatcgtg gccgacggca gcagcaagtt cccccacggc gacttcaacg tgcacgccgt 5700
gtgcgagacc ggcaagctgc ccatgagctg gaagcccatc tgccacctga tccagtacgg 5760
cgagcccttc ttcgcccgct accccaacgg catcagccac ttcgcccagg agtgcttccc 5820
cgagggcctg agcatcgacc gcaccgtgcg cttcgagaac gacggcacca tgaccagcca 5880
ccacacctac gagctggacg gcacctgcgt ggtcagccgc atcaccgtga actgcgacgg 5940
cttccagccc gacggcccca tcatgcgcga ccagctggtg gacatcctgc ccaacgagac 6000
ccacatgttc ccccacggcc ccaacgccgt gcgccagctg gccttcatcg gcttcaccac 6060
cgccgacggc ggcctgatga tgggccactt cgacagcaag atgaccttca acggcagccg 6120
cgccatcaag atccccggcc cccacttcgt gaccatcatc accaagcaga tgagggacac 6180
cagcgacaag cgcgaccacg tgtgccagcg cgaggtgacc tacgcccaca gcgtgccccg 6240
catcaccagc gccatcggta gcgacgagga ttccggactc agatctcgac ccaagaaaaa 6300
gcggaaggtg gaggacccgt aagatccacc ggatctagat aactgatcat aatcagccat 6360
accacatttg tagaggtttt acttgcttta aaaaacctcc cacacctccc cctgaacctg 6420
aaacataaaa tgaatgcaat tgttgttgtt aacttgttta ttgcagctta taatggttac 6480
aaataaagca atagcatcac aaatttcaca aataaagcat ttttttcact gcattctagt 6540
tgtggtttgt ccaaactcat caatgtatct taacgcgagt taattaacac cgaaatcgta 6600
attcacggca tcattacaaa atattttgac gttttggacc tcgtccctaa tgacaccata 6660
acggtggcct tgaagtatat ttaaccctag aaagatagtc tgcgtaaaat tgacgcatgc 6720
attcttgaaa tattgctctc tctttctaaa tagcgcgaat ccgtcgctgt gcatttagga 6780
catctcagtc gccgcttgga gctcccgtga ggcgtgcttg tcaatgcggt aagtgtcact 6840
gattttgaac tataacgacc gcgtgagtca aaatgacgca tgattatctt ttacgtgact 6900

tttaagattt aactcatacg ataattatat tgttatttca tgttctactt acgtgataac 6960
ttattatata tatattttct tgttatagat atcgtgacta atatataata aaatgggtag 7020
ttctttagac gatgagcata tcctctctgc tcttctgcaa agcgatgacg agcttgttgg 7080
tgaggattct gacagtgaaa tatcagatca cgtaagtgaa gatgacgtcc aggaaatctg 7140
gccggccgca accattgtgg gaaccgtgcg atcaaacaaa cgcgagatac cggaagtact 7200
gaaaaacagt cgctccaggc cagtgggaac atcgatgttt tgttttgacg gaccccttac 7260
tctcgtctca tataaaccga agccagctaa gatggtatac ttattatcat cttgtgatga 7320
ggatgcttct atcaacgaaa gtaccggtaa accgcaaatg gttatgtatt ataatcaaac 7380
taaaggcgga gtggacacgc tagaccaaat gtgttctgtg atgacctgca gtaggaagac 7440
gaataggtgg cctatggcat tattgtacgg aatgataaac attgcctgca taaattcttt 7500
tattatatac agccataatg tcagtagcaa gggagaaaag gtccaaagtc gcaaaaaatt 7560
tatgagaaac ctttacatga gcctgacgtc atcgtttatg cgtaagcgtt tagaagctcc 7620
tactttgaag agatatttgc gcgataatat ctctaatatt ttgccaaatg aagtgcctgg 7680
tacatcagat gacagtactg aagagccagt aatgaaaaaa cgtacttact gtacttactg 7740
cccctctaaa ataaggcgaa aggcaaatgc atcgtgcaaa aaatgcaaaa aagttatttg 7800
tcgagagcat aatattgata tgtgccaaag ttgtttctga ctgactaata agtataattt 7860
gtttctatta tgtataagtt aagctaatta cttattttat aatacaacat gactgttttt 7920
aaagtacaaa ataagtttat ttttgtaaaa gagagaatgt ttaaaagttt tgttacttta 7980
tagaagaaat tttgagtttt tgtttttttt taataaataa ataaacataa ataaattgtt 8040
tgttgaattt attattagta tgtaagtgta aatataataa aacttaatat ctattcaaat 8100
taataaataa acctcgatat acagaccgat aaaacacatg cgtcaatttt acgcatgatt 8160
atctttaacg tacgtcacaa tatgattatc tttctagggt taaataatag tttctaattt 8220
ttttattatt cagcctgctg tcgtgaatac cgtatatctc aacgctgtct gtgagattgt 8280
cgtattctag cctttttagt ttttcgctca tcgacttgat attgtccgac acattttcgt 8340
cgatttgcgt tttgatcaaa gacttgagca gagacacgtt aatcaactgt tcaaattgat 8400
ccatattaac gatatcaacc cgatgcgtat atggtgcgta aaatatattt tttaaccctc 8460
ttatactttg cactctgcgt taatacgcgt tcgtgtacag acgtaatcat gttttctttt 8520
ttggataaaa ctcctactga gtttgacctc atattagacc ctcacaagtt gcaaaacgtg 8580
gcattttttta ccaatgaaga atttaaagtt attttaaaaa atttcatcac agatttaaag 8640
aagaaccaaa aattaaatta tttcaacagt ttaatcgacc agttaatcaa cgtgtacaca 8700
gacgcgtcgg caaaaaacac gcagcccgac gtgttggcta aaattattaa atcaacttgt 8760

gttatagtca cggatttgcc gtccaacgtg ttcctcaaaa agttgaagac caacaagttt 8820

acggacacta ttaattattt gattttgccc cacttcattt tgtgggatca caattttgtt 8880

atattttaaa caaagcttgg cactggccgt cgttttacaa cgtcgtgact gggaaaaccc 8940

tggcgttacc caacttaatc gccttgcagc acatccccct ttcgccagct ggcgtaatag 9000

cgaagaggcc cgcaccgatc gcccttccca acagttgcgc agcctgaatg gcgaatggcg 9060

cctgatgcgg tattttctcc ttacgcatct gtgcggtatt tcacaccgca tatggtgcac 9120

tctcagtaca atctgctctg atgccgcata gttaagccag ccccgacacc cgccaacacc 9180

cgctgacgcg ccctgacggg cttgtctgct cccggcatcc gcttacagac aagctgtgac 9240

cgtctccggg agctgcatgt gtcagaggtt ttcaccgtca tcaccgaaac gcgcgagacg 9300

aaagggcctc gtgatacgcc tatttttata ggttaatgtc atgataataa tggtttctta 9360

gacgtcaggt ggcacttttc ggggaaatgt gcgcggaacc cctatttgtt tatttttcta 9420

aatacattca aatatgtatc cgctcatgag acaataaccc tgataaatgc ttcaataata 9480

ttgaaaaagg aagagtatga gtattcaaca tttccgtgtc gcccttattc ccttttttgc 9540

ggcattttgc cttcctgttt ttgctcaccc agaaacgctg gtgaaagtaa aagatgctga 9600

agatcagttg ggtgcacgag tgggttacat cgaactggat ctcaacagcg gtaagatcct 9660

tgagagtttt cgccccgaag aacgttttcc aatgatgagc acttttaaag ttctgctatg 9720

tggcgcggta ttatcccgta ttgacgccgg gcaagagcaa ctcggtcgcc gcatacacta 9780

ttctcagaat gacttggttg agtactcacc agtcacagaa aagcatctta cggatggcat 9840

gacagtaaga gaattatgca gtgctgccat aaccatgagt gataacactg cggccaactt 9900

acttctgaca acgatcggag gaccgaagga gctaaccgct tttttgcaca acatggggga 9960

tcatgtaact cgccttgatc gttgggaacc ggagctgaat gaagccatac caaacgacga 10020

gcgtgacacc acgatgcctg tagcaatggc aacaacgttg cgcaaactat taactggcga 10080

actacttact ctagcttccc ggcaacaatt aatagactgg atggaggcgg ataaagttgc 10140

aggaccactt ctgcgctcgg cccttccggc tggctggttt attgctgata aatctggagc 10200

cggtgagcgt gggtctcgcg gtatcattgc agcactgggg ccagatggta agccctcccg 10260

tatcgtagtt atctacacga cggggagtca ggcaactatg gatgaacgaa atagacagat 10320

cgctgagata ggtgcctcac tgattaagca ttggtaactg tcagaccaag tttactcata 10380

tatactttag attgatttaa aacttcattt ttaatttaaa aggatctagg tgaagatcct 10440

ttttgataat ctcatgacca aaatccctta acgtgagttt tcgttccact gagcgtcaga 10500

ccccgtagaa aagatcaaag gatcttcttg agatcctttt tttctgcgcg taatctgctg 10560

cttgcaaaca aaaaaaccac cgctaccagc ggtggtttgt ttgccggatc aagagctacc 10620

aactcttttt ccgaaggtaa ctggcttcag cagagcgcag ataccaaata ctgtccttct 10680

agtgtagccg tagttaggcc accacttcaa gaactctgta gcaccgccta catacctcgc 10740
tctgctaatc ctgttaccag tggctgctgc cagtggcgat aagtcgtgtc ttaccgggtt 10800
ggactcaaga cgatagttac cggataaggc gcagcggtcg ggctgaacgg ggggttcgtg 10860
cacacagccc agcttggagc gaacgaccta caccgaactg agatacctac agcgtgagca 10920
ttgagaaagc gccacgcttc ccgaagggag aaaggcggac aggtatccgg taagcggcag 10980
ggtcggaaca ggagagcgca cgagggagct tccaggggga aacgcctggt atctttatag 11040
tcctgtcggg tttcgccacc tctgacttga gcgtcgattt ttgtgatgct cgtcaggggg 11100
gcggagccta tggaaaaacg ccagcaacgc ggccttttta cggttcctgg ccttttgctg 11160
gccttttgct cacatgttct ttcctgcgtt atcccctgat tctgtggata accgtattac 11220
cgcctttgag tgagctgata ccgctcgccg cagccgaacg accgagcgca gcgagtcagt 11280
gagcgaggaa gcggaagagc gcccaatacg caaaccgcct ctccccgcgc gttggccgat 11340
tcattaatgc agctggcacg acaggtttcc cgactggaaa gcgggcagtg agcgcaacgc 11400
aattaatgtg agttagctca ctcattaggc accccaggct ttacacttta tgcttccggc 11460
tcgtatgttg tgtggaattg tgagcggata acaatttcac acaggaaaca gctatgacca 11520
tgattacgaa tttcgacctg caggcatgca agcttgcatg cctgcaggtc gacgctcgcg 11580
cgacttggtt tgccattctt tagcgcgcgt cgcgtcacac agcttggcca caatgtggtt 11640
tttgtcaaac gaagattcta tgacgtgttt aaagtttagg tcgagtaaag cgcaaatctt 11700
ttttaaccct agaaagatag tctgcgtaaa attgacgcat gcattcttga aatattgctc 11760
tctctttcta aatagcgcga atccgtcgct gtgcatttag gacatctcag tcgccgcttg 11820
gagctcccgt gaggcgtgct tgtcaatgcg gtaagtgtca ctgattttga actataacga 11880
ccgcgtgagt caaaatgacg catgattatc ttttacgtga cttttaagat ttaactcata 11940
cgataattat attgttattt catgttctac ttacgtgata acttattata tatatatttt 12000
cttgttatag atatcgtgac taatatataa taaaatgggt agttctttag acgatgagca 12060
tatcctctct gctcttctgc aaagcgatga cgagcttgtt ggtgaggatt ctgacagtga 12120
aatatcagat cacgtaagtg aagatgacgt ccagagcgat acagaagaag cgtttataga 12180
tgaggtacat gaagtgcagc caacgtcaag cggtagtgaa atattagacg aacaaaatgt 12240
tattgaacaa ccaggttctt cattggcttc taacagaatc ttgaccttgc cacagaggac 12300
tattagaggt aagaataaac attgttggtc aacttcaaag tccacgaggc gtagccgagt 12360
ctctgcactg aacattgtca gatcggcccg gcggagtgga cacgctagac caaatgtgtt 12420
ctgtgatgac ctgcagtagg aagacgaata ggtggcctat ggcattattg tacggaatga 12480
taaacattgc ctgcataaat tcttttatta tatacagcca taatgtcagt agcaaggggag 12540

aaaaggtcca aagtcgcaaa aaatttatga gaaaccttta catgagcctg acgtcatcgt 12600

ttatgcgtaa gcgtttagaa gctcctactt tgaagagata tttgcgcgat aatatctcta 12660

atattttgcc aaatgaagtg cctggtacat cagatgacag tactgaagag ccagtaatga 12720

aaaaacgtac ttactgtact tactgcccct ctaaaataag gcgaaaggca aatgcatcgt 12780

gcaaaaaatg caaaaaagtt atttgtcgag agcataatat tgatatgtgc caaagttgtt 12840

tctgactgac taataagtat aatttgtttc tattatgtat aagttaagct aattacttat 12900

tttataatac aacatgactg tttttaaagt acaaaataag tttatttttg taaaagagag 12960

aatgtttaaa agttttgtta ctttatagaa gaaattttga gtttttgttt ttttttaata 13020

aataaataaa cataaataaa ttgtttgttg aatttattat tagtatgtaa gtgtaaatat 13080

aataaaactt aatatctatt caaattaata aataaacctc gatatacaga ccgataaaac 13140

acatgcgtca attttacgca tgattatctt taacgtacgt cacaatatga ttatctttct 13200

agggttaaaa tgaatgtaag cactttatta acgaaatctt tgggaatatt tcgctcatca 13260

gcattttatt tgagcaggag tccgagatgc cc 13292

<210> 53
<211> 14713
<212> DNA
<213> artificial

<220>
<223> Sequence of pLA3014-Cctra-intron-Ubiquitin-reaperKR construct.

<400> 53
cgcgccggac gcggcaagtc tgcgagctta tatttacgtg gatctccggt gtgtccatga 60

ttcggcatca tatcataaac gacgaattcc aataaaaact ttgcttgttg ataacacctg 120

atgttcagag atgcccgata aaatcacagc tgttctggtt cacagtcacc agaaataaaa 180

aatattggaa ttgagatgta cacaattaac gatatttata aatatcttcc gatagtctat 240

cgtccggtta atcaaaataa agtgcgacga attaacatat tttcaaaatt aagacgcttt 300

gatagatgta tttgtataga gatagaaatt aaggttaaaa taacataaat gccaaagttt 360

agagcactat tcaataattc tcttgatttc aaattgaaat aatacacaat ataacatttt 420

ctaacactac aaagtcacga tattcttcca ccaaccgata gtatcgcaca cttgccattc 480

gcctcatcac gcacacgccc gcttcacaat tcaaacgaac ggcattttat tttcacagga 540

tcccgggagt cgtgaatgtt ttacccaata tcgactttca ttgttaactg accaaaattg 600

taatctgttc tgttagttgt cgagtgcctg tgccgcgatc gctatgggca tatgttgcca 660

aactctaaac caaatactca ttctgatgtt ttaaatgatt tgccctccca tatgtccttc 720

cgagtgagag acacaaaaaa ttccaacaca ctattgcaat gaaaataaat ttcctttatt 780

agccagaagt cagatgctca aggggcttca tgatgtcccc ataattttg gcagagggaa 840

aaagatctca gtggtatttg tgagccaggg cattggccac accagccacc accttctgat 900

aggcagcctg cacctgagga gtgaattctt tgccaaaatg atgagacagc acaacaacca 960

gcacgttgcc caggagctgt aggaaagaga agaaggcatg aacatggtta gcagaggggc 1020

ccggtttgga ctcagagtat tttatcctca tctcaaacag tgtatatcat tgtaaccata 1080

aagagaaagg caggatgatg accagggtgt agttgtttct accaataaga atatttccac 1140

gccagccaga atttatatgc agaaatattc taccttatca tttaattata acaattgttc 1200

tctaaaactg tgctgaagta caatataata taccctgatt gccttgaaaa aaaagtgatt 1260

agagaaagta cttacaatct gacaaataaa caaaagtgaa tttaaaaatt cgttacaaat 1320

gcaagctaaa gtttaacgaa aaagttacag aaaatgaaaa gaaaataaga ggagacaatg 1380

gttgtcaaca gagtagaaag tgaaagaaac aaaattatca tgagggtcca tggtgataca 1440

agggacatct tcccattcta aacaacaccc tgaaaacttt gccccctcca tataacatga 1500

attttacaat agcgaaaaag aaagaacaat caagggtccc caaactcacc ctgaagttct 1560

cagctctaga cgcgtttcac tacccaccgt actcgtcaat tccaagggca tcggtaaaca 1620

tctgctcaaa ctcgaagtcg gccatatcca gagcgccgta gggggcggag tcgtgggggg 1680

taaatcccgg acccggggaa tccccgtccc ccaacatgtc cagatcgaaa tcgtctagcg 1740

cgtcggcatg cgccatcgcc acgtcctcgc cgtctaagtg gagctcgtcc cccaggctga 1800

catcggtcgg gggggccgtc gacagtctgc gcgtgtgtcc cgcggggaga aaggacaggc 1860

gcggagccgc cagccccgcc tcttcggggg cgtcgtcgtc cgggagatcg agcaggccct 1920

cgatggtaga cccgtaattg tttttcgtac gcgcgcggct gtacgcggac ccactttcac 1980

atttaagttg tttttctaat ccgcatatga tcaattcaag gccgaataag aaggctggct 2040

ctgcaccttg gtgatcaaat aattcgatag cttgtcgtaa taatggcggc atactatcag 2100

tagtaggtgt ttccctttct tctttagcga cttgatgctc ttgatcttcc aatacgcaac 2160

ctaaagtaaa atgccccaca gcgctgagtg catataatgc attctctagt gaaaaacctt 2220

gttggcataa aaaggctaat tgattttcga gagtttcata ctgtttttct gtaggccgtg 2280

tacctaaatg tacttttgct ccatcgcgat gacttagtaa agcacatcta aaacttttag 2340

cgttattacg taaaaaatct tgccagcttt ccccttctaa agggcaaaag tgagtatggt 2400

gcctatctaa catctcaatg gctaaggcgt cgagcaaagc ccgcttattt tttacatgcc 2460

aatacaatgt aggctgctct acacctagct tctgggcgag tttacgggtt gttaaacctt 2520

cgattccgac ctcattaagc agctctaatg cgctgttaat cactttactt ttatctaatc 2580

tcaattccat ggtggcaacc tgcaaggcga atgaataaac aagattgtgg cgaacagtgt 2640

aatgcgaaga acccacctct gctccaattc ccaattccct attcagctcg agcggggatc 2700

cccgggtacc gagctcgaat tcggggccgc ggaggctgga tcggtcccgg tgtcttctat 2760
ggaggtcaaa acagcgtgga tggcgtctcc aggcgatctg acggttcact aaacgagctc 2820
tgcttatata ggcctcccac cgtacacgcc tacctcgacc cgggtaccga gctcgacttt 2880
cacttttctc tatcactgat agggagtggt aaactcgact ttcacttttc tctatcactg 2940
atagggagtg gtaaactcga ctttcacttt tctctatcac tgatagggag tggtaaactc 3000
gactttcact tttctctatc actgataggg agtggtaaac tcgactttca cttttctcta 3060
tcactgatag ggagtggtaa actcgacttt cacttttctc tatcactgat agggagtggt 3120
aaactcgact ttcacttttc tctatcactg atagggagtg gtaaactcga aatgtcgact 3180
atgcggaccg agcgccggag tataaataga ggcgcttcgt ctacggagcg acaattcaat 3240
tcaaacaagc aaagtgaaca cgtcgctaag cgaaagctaa gcaaataaac aagcgcagct 3300
gaacaagcta aacaatctgc gctagccacc atggttgtta ttaaacgtag atttggtaat 3360
tttaaaagca tatttttttc tttgaaattc ataagttatc aattatcgat ggaaatgtat 3420
tctatggaga acgttttacc cgatgaatgg gtgcaaaaat tattttacct tcaaatctac 3480
aatcaacaca cgctaacttt tgtgacttga tcaactctca cctggaaaag caaccaacta 3540
caatcaacat tctatgggat aatcgacaag tgagtaaaat tatagccgga cctcttagta 3600
cagtgtattt aaaaggggaa taatattcta tcaataggaa taaaaataag gtcagcagcc 3660
atgacttttc catcattttg aatatacctt atttgtttcg ggattaattg ggggtcggaa 3720
atcctcttga attcagaaac gggaaccgga ggaaggtgcc ggtctttcag aaagctgtga 3780
aaaataccaa catttctgct gccaagagct caataagaag tttcaaaaat tgtcttggat 3840
gttgcagctg tggctgctaa gtaataagac atctattagt atctagattt gttagaccat 3900
ttaacatagt gttttaaacg atggggttaa tagatgaggg ttaagaagct agttatatta 3960
ctgttgctgt aacgccttca attgtcggtt acagagcaaa cattattgaa tgttaatgta 4020
aagagtttat ttgttttcta gtaaacatat agcgattggt tagtaatcac taatagaaat 4080
ttttcataag tatcaaaaaa gtaaacctct ttttcagtct atgtaataag taaaccaagg 4140
aaagggaaaa tatctacaat caacaagcca ttgttgcagc aacaaagcaa ctgaaactac 4200
aatcaacatt caataaactt gggtaatttg gaatttaatt ctctgggaca cctgtggatt 4260
acaacaatca actcgaaact tattatacaa tgtaaataaa aattgatatg catacatgaa 4320
gatcaagtga aattccattt agaatcaatt tttttcgaat attaagtttc ttgctttaat 4380
ttatctgaaa gtaaatagac attccaaatt caagttaaca aattaataat gaattgacta 4440
gtgattttta agagaaaaag ataagattta aaaaaggaaa gcctttcttg ataaattttt 4500
gaaccacttt atgccgtttc aatcataaaa acttttaaga acacatgact ggtaaaatta 4560
atttaaaaca aatttaaatt ttcaacgtaa cattcaacaa aaatggtgaa aactatcacg 4620

gaaattgtta atattaatat gtcccaaaaa tagcctttgt atgtatatga tactaatcca 4680
tacatctatg gtatctatag gtgaaggctc aaagcctctg atgcagatct ttgtgaagac 4740
tttgaccgga aagaccatca ccctcgaggt agagcccatcg gacaccattg agaatgtaaa 4800
ggccaagatt caggataagg agggaatccc cccagatcag cagcgtctga tcttcgctgg 4860
caagcaactg gaagacggac gcaccctgtc cgattacaac atccagaagg agtccaccct 4920
tcacttggtc cttcgtctcc gtggtggcgc cgtggccttc tacatcccgg atcaggccac 4980
cctgctgcgc gaggccgagc agcgcgagca gcagatcctg cgcctgcgcg agagccagtg 5040
gcgcttcctg gccaccgtgg tgctggagac cctgcgccag tacaccagct gccacccgcg 5100
caccggccgc cgcagcggcc gttaccgccg tccgagccag taacaccggt gatcataatc 5160
agccatacca catttgtaga ggttttactt gctttaaaaa acctcccaca cctccccctg 5220
aacctgaaac ataaaatgaa tgcaattgtt gttgttaact tgtttattgc agcttataat 5280
ggttacaaat aaagcaatag catcacaaat ttcacaaata aagcattttt ttcactgcat 5340
tctagttgtg gtttgtccaa actcatcaat gtatcttaac gcgagtttaa acgcgtccgc 5400
atacgtccgc tcacgttaag ttccgcagag agaagttgtt gaaaacataa acagaatcac 5460
ttgttgcact ctttgagaaa actggggcta ttgcggaaaa aaccaactaa aaatattgca 5520
ggttaggggt actacgctcg attggcgtac ggccaccact tttgcgactt cactgttaac 5580
cgctaccttc atagagactt ttacccgata aatgttatgt agtttgactt tctctgttaa 5640
tcacaagaaa aaatattgtg gaaattaaaa ttatctcaaa ctcaataagg aaataataat 5700
atatacacct atgttttata gaagtcaaca gtaaataagt tatttggaaa accattgtag 5760
ccgtttaaat aaatctcctt gagtgtgttt taaataacgg tcattaagta tattacttgg 5820
ccctctgaat ttcttgaatt acaccatttt ttgaaataaa tcaatccaaa agactacttt 5880
ttggtggcaa atgaactgca taaaaagtaa caaaagaaat atgtttttga aataacagta 5940
tagctgaagt gtattaaaaa ataccgtcat atgagcgacc cgctgttacc gcttcgctgc 6000
gaatgacaaa acgggctgag caagaaaatg gcgtagaagg cgacgaaaat tcgtttcact 6060
cgtgaagaaa acctcgataa ctgaggaata cagctgggat ttaaagagca tattcgaact 6120
acaagcagag atgtttcctg gtggaaacgg aaacgccgat ttgggctaca acaagcatgc 6180
ccacgtccat ggacttggac aacatggcca tgggcacaac cataatcaca atcagttcct 6240
gcgcagcccc caccaccccc cacacatttt tcactgccct ccgggggcgg tcagggcatg 6300
gtgacgccca tggtagccgc cggcctgccg ctcgccatgc agggtggcgt tggcatcgat 6360
tggcgcagct cgcccagcaa tggattaatt aactcgcgtt aagatacatt gatgagtttg 6420
gacaaaccac aactagaatg cagtgaaaaa aatgctttat ttgtgaaatt tgtgatgcta 6480

ttgctttatt tgtaaccatt ataagctgca ataaacaagt taacaacaac aattgcattc 6540
attttatgtt tcaggttcag ggggaggtgt gggaggtttt ttaaagcaag taaaacctct 6600
acaaatgtgg tatggctgat tatgatcagt tatctagatc cggtggatct tacgggtcct 6660
ccaccttccg ctttttcttg ggtcgagatc tcaggaacag gtggtggcgg ccctcggtgc 6720
gctcgtactg ctccacgatg gtgtagtcct cgttgtggga ggtgatgtcc agcttggcgt 6780
ccacgtagta gtagccgggc agctgcacgg gcttcttggc catgtagatg gacttgaact 6840
ccaccaggta gtggccgccg tccttcagct tcagggcctt gtgggtctcg cccttcagca 6900
cgccgtcgcg ggggtacagg cgctcggtgg aggcctccca gcccatggtc ttcttctgca 6960
tcacggggcc gtcggagggg aagttcacgc cgatgaactt caccttgtag atgaagcagc 7020
cgtcctgcag ggaggagtcc tgggtcacgg tcgccacgcc gccgtcctcg aagttcatca 7080
cgcgctccca cttgaagccc tcggggaagg acagcttctt gtagtcgggg atgtcggcgg 7140
ggtgcttcac gtacaccttg gagccgtact ggaactgggg ggacaggatg tcccaggcga 7200
agggcagggg gccgcccttg gtcaccttca gcttcacggt gttgtggccc tcgtaggggc 7260
ggccctcgcc ctcgccctcg atctcgaact cgtggccgtt cacggtgccc tccatgcgca 7320
ccttgaagcg catgaactcg gtgatgacgt tctcggagga ggccatggtg gcgaccggtt 7380
tgcgcttctt cttgggtggg gtgggatccc cgatctgcat tttggattat tctgcgggtc 7440
aaaatagaga tgtggaaaat tagtacgaaa tcaaatgagt ttcgttgaaa ttacaaaact 7500
attgaaacta acttcctggc tggggaataa aaatgggaaa cttatttatc gacgccaact 7560
ttgttgagaa acccctatta accctctacg aatattggaa caaaggaaag cgaagaaaca 7620
ggaacaaagg tagttgagaa acctgttccg ttgctcgtca tcgttttcat aatgcgagtg 7680
tgtgcatgta tatatacaca gctgaaacgc atgcatacac attattttgt gtgtatatgg 7740
tgacgtcaca actactaagc aataagaaat tttccagacg tggctttcgt ttcaagcaac 7800
ctactctatt tcagctaaaa ataagtggat ttcgttggta aaatacttca attaagcaaa 7860
gaactaacta actaataaca tgcacacaaa tgctcgagtg cgttcgtgat ttctcgaatt 7920
ttcaaatgcg tcactgcgaa tttcacaatt tgccaataaa tcttggcgaa aatcaacacg 7980
caagttttat ttatagattt gtttgcgttt tgatgccaat tgattgggaa aacaagatgc 8040
gtggctgcca atttcttatt ttgtaattac gtagagcgtt gaataaaaaa aaaatggccg 8100
aacaaagacc ttgaaatgca gtttttcttg aaattactca acgtcttgtt gctcttatta 8160
ctaattggta acagcgagtt aaaaacttac gtttcttgtg actttcgaga atgttctttt 8220
aattgtactt taatcaccaa caattaagta taaatttttc gctgattgcg ctttactttc 8280
tgcttgtact tgctgctgca aatgtcaatt ggttttgaag gcgaccgttc gcgaacgctg 8340
tttatatacc ttcggtgtcc gttgaaaatc actaaaaaat accgtagtgt tcgtaacact 8400

ttagtacaga gaaaaaaaat tgtgccgaaa tgtttttgat acgtacgaat accttgtatt 8460
aaaatttttt atgatttctg tgtatcactt tttttttgtg tttttcgttt aaactcacca 8520
cagtacaaaa caataaaata tttttaagac aatttcaaat tgagaccttt ctcgtactga 8580
cttgaccggc tgaatgagga tttctaccta gacgacctac ttcttaccat gacattgaat 8640
gcaatgccac ctttgatcta aacttacaaa agtccaaggc ttgttaggat tggtgtttat 8700
ttagtttgct tttgaaatag cactgtcttc tctaccggct ataattttga aactcgcagc 8760
ttgactggaa atttaaaaag taattctgtg taggtaaagg gtgttttaaa agtgtgatgt 8820
gttgagcgtt gcggcaacga ctgctattta tgtatatatt ttcaaaactt attgtttttg 8880
aagtgtttta aatggagcta tctggcaacg ctgcgcataa tcttacacaa gcttttctta 8940
atccattttt aagtgaaatt tgtttttact ctttcggcaa ataattgtta aatcgcttta 9000
agtgggctta catctggata agtaatgaaa acctgcatat tataatatta aaacatataa 9060
tccactgtgc tttccccgtg tgtggccata tacctaaaaa agtttatttt cgcagagccc 9120
cgcacggtca cactacggtt cggcgatttt cgattttgga cagtactgat tgcaagcgca 9180
ccgaaagcaa aatggagctg gagattttga acgcgaagaa cagcaagccg tacggcaagg 9240
tgaaggtgcc ctccggcgcc acgcccatcg gcgatctgcg cgccctaatt cacaagaccc 9300
tgaagcagac cccacacgcg aatcgccagt cgcttcgtct ggaactgaag ggcaaaagcc 9360
tgaaagatac ggacacattg gaatctctgt cgctgcgttc cggcgacaag atcggggtac 9420
catgcggccg ctcatttaaa tctggccggc ctggccgatc tgacaatgtt cagtgcagag 9480
actcggctac gcctcgtgga ctttgaagtt gaccaacaat gtttattctt acctctaata 9540
gtcctctgtg gcaaggtcaa gattctgtta gaagccaatg aagaacctgg ttgttcaata 9600
acattttgtt cgtctaatat ttcactaccg cttgacgttg gctgcacttc atgtacctca 9660
tctataaacg cttcttctgt atcgctctgg acgtcatctt cacttacgtg atctgatatt 9720
tcactgtcag aatcctcacc aacaagctcg tcatcgcttt gcagaagagc agagaggata 9780
tgctcatcgt ctaaagaact acccatttta ttatatatta gtcacgatat ctataacaag 9840
aaaatatata tataataagt tatcacgtaa gtagaacatg aaataacaat ataattatcg 9900
tatgagttaa atcttaaaag tcacgtaaaa gataatcatg cgtcattttg actcacgcgg 9960
tcgttatagt tcaaaatcag tgacacttac cgcattgaca agcacgcctc acgggagctc 10020
caagcggcga ctgagatgtc ctaaatgcac agcgacggat tcgcgctatt tagaaagaga 10080
gagcaatatt tcaagaatgc atgcgtcaat tttacgcaga ctatctttct agggttaaaa 10140
aagatttgcg ctttactcga cctaaacttt aaacacgtca tagaatcttc gtttgacaaa 10200
aaccacattg tggccaagct gtgtgacgcg acgcgcgcta aagaatggca aaccaagtcg 10260

cgcgagcgtc gacctgcagg catgcaagct tgcatgcctg caggtcgaaa ttcgtaatca 10320
tggtcatagc tgtttcctgt gtgaaattgt tatccgctca caattccaca caacatacga 10380
gccggaagca taaagtgtaa agcctggggt gcctaatgag tgagctaact cacattaatt 10440
gcgttgcgct cactgcccgc tttccagtcg ggaaacctgt cgtgccagct gcattaatga 10500
atcggccaac gcgcggggag aggcggtttg cgtattgggc gctcttccgc ttcctcgctc 10560
actgactcgc tgcgctcggt cgttcggctg cggcgagcgg tatcagctca ctcaaaggcg 10620
gtaatacggt tatccacaga atcaggggat aacgcaggaa agaacatgtg agcaaaaggc 10680
cagcaaaagg ccaggaaccg taaaaaggcc gcgttgctgg cgtttttcca taggctccgc 10740
ccccctgacg agcatcacaa aaatcgacgc tcaagtcaga ggtggcgaaa cccgacagga 10800
ctataaagat accaggcgtt tccccctgga agctccctcg tgcgctctcc tgttccgacc 10860
ctgccgctta ccggatacct gtccgccttt ctcccttcgg gaagcgtggc gctttctcaa 10920
tgctcacgct gtaggtatct cagttcggtg taggtcgttc gctccaagct gggctgtgtg 10980
cacgaacccc ccgttcagcc cgaccgctgc gccttatccg gtaactatcg tcttgagtcc 11040
aacccggtaa gacacgactt atcgccactg gcagcagcca ctggtaacag gattagcaga 11100
gcgaggtatg taggcggtgc tacagagttc ttgaagtggt ggcctaacta cggctacact 11160
agaaggacag tatttggtat ctgcgctctg ctgaagccag ttaccttcgg aaaaagagtt 11220
ggtagctctt gatccggcaa acaaaccacc gctggtagcg gtggtttttt tgtttgcaag 11280
cagcagatta cgcgcagaaa aaaaggatct caagaagatc ctttgatctt ttctacgggg 11340
tctgacgctc agtggaacga aaactcacgt taagggattt tggtcatgag attatcaaaa 11400
aggatcttca cctagatcct tttaaattaa aaatgaagtt ttaaatcaat ctaaagtata 11460
tatgagtaaa cttggtctga cagttaccaa tgcttaatca gtgaggcacc tatctcagcg 11520
atctgtctat ttcgttcatc catagttgcc tgactccccg tcgtgtagat aactacgata 11580
cgggagggct taccatctgg ccccagtgct gcaatgatac cgcgagaccc acgctcaccg 11640
gctccagatt tatcagcaat aaaccagcca gccggaaggg ccgagcgcag aagtggtcct 11700
gcaactttat ccgcctccat ccagtctatt aattgttgcc gggaagctag agtaagtagt 11760
tcgccagtta atagtttgcg caacgttgtt gccattgcta caggcatcgt ggtgtcacgc 11820
tcgtcgtttg gtatggcttc attcagctcc ggttcccaac gatcaaggcg agttacatga 11880
tcccccatgt tgtgcaaaaa agcggttagc tccttcggtc ctccgatcgt tgtcagaagt 11940
aagttggccg cagtgttatc actcatggtt atggcagcac tgcataattc tcttactgtc 12000
atgccatccg taagatgctt ttctgtgact ggtgagtact caaccaagtc attctgagaa 12060
tagtgtatgc ggcgaccgag ttgctcttgc ccggcgtcaa tacgggataa taccgcgcca 12120
catagcagaa ctttaaaagt gctcatcatt ggaaaacgtt cttcggggcg aaaactctca 12180

aggatcttac cgctgttgag atccagttcg atgtaaccca ctcgtgcacc caactgatct 12240

tcagcatctt ttactttcac cagcgtttct gggtgagcaa aaacaggaag gcaaaatgcc 12300

gcaaaaaagg gaataagggc gacacggaaa tgttgaatac tcatactctt cctttttcaa 12360

tattattgaa gcatttatca gggttattgt ctcatgagcg gatacatatt tgaatgtatt 12420

tagaaaaata aacaaatagg ggttccgcgc acatttcccc gaaaagtgcc acctgacgtc 12480

taagaaacca ttattatcat gacattaacc tataaaaata ggcgtatcac gaggcccttt 12540

cgtctcgcgc gtttcggtga tgacggtgaa aacctctgac acatgcagct cccggagacg 12600

gtcacagctt gtctgtaagc ggatgccggg agcagacaag cccgtcaggg cgcgtcagcg 12660

ggtgttggcg ggtgtcgggg ctggcttaac tatgcggcat cagagcagat tgtactgaga 12720

gtgcaccata tatgcggtgt gaaataccgc acagatgcgt aaggagaaaa taccgcatca 12780

ggcgccattc gccattcagg ctgcgcaact gttgggaagg gcgatcggtg cgggcctctt 12840

cgctattacg ccagctggcg aaagggggat gtgctgcaag gcgattaagt tgggtaacgc 12900

cagggttttc ccagtcacga cgttgtaaaa cgacggccag tgccaagctt tgtttaaaat 12960

ataacaaaat tgtgatccca caaaatgaag tggggcaaaa tcaaataatt aatagtgtcc 13020

gtaaacttgt tggtcttcaa ctttttgagg aacacgttgg acggcaaatc cgtgactata 13080

acacaagttg atttaataat tttagccaac acgtcgggct gcgtgttttt tgccgacgcg 13140

tctgtgtaca cgttgattaa ctggtcgatt aaactgttga aataatttaa tttttggttc 13200

ttctttaaat ctgtgatgaa attttttaaa ataactttaa attcttcatt ggtaaaaaat 13260

gccacgtttt gcaacttgtg agggtctaat atgaggtcaa actcagtagg agttttatcc 13320

aaaaaagaaa acatgattac gtctgtacac gaacgcgtat taacgcagag tgcaaagtat 13380

aagagggtta aaaaatatat tttacgcacc atatacgcat cgggttgata tcgttaatat 13440

ggatcaattt gaacagttga ttaacgtgtc tctgctcaag tctttgatca aaacgcaaat 13500

cgacgaaaat gtgtcggaca atatcaagtc gatgagcgaa aaactaaaaa ggctagaata 13560

cgacaatctc acagacagcg ttgagatata cggtattcac gacagcaggc tgaataataa 13620

aaaaattaga aactattatt taaccctaga aagataatca tattgtgacg tacgttaaag 13680

ataatcatgc gtaaaattga cgcatgtgtt ttatcggtct gtatatcgag gtttatttat 13740

taatttgaat agatattaag ttttattata tttacactta catactaata ataaattcaa 13800

caaacaattt atttatgttt atttatttat taaaaaaaaa caaaaactca aaatttcttc 13860

tataaagtaa caaaactttt aaacattctc tcttttacaa aaataaactt attttgtact 13920

ttaaaaacag tcatgttgta ttataaaata agtaattagc ttaacttata cataatagaa 13980

acaaattata cttattagtc agtcagaaac aactttggca catatcaata ttatgctctc 14040

gacaaataac ttttttgcat tttttgcacg atgcatttgc ctttcgcctt attttagagg 14100

ggcagtaagt acagtaagta cgttttttca ttactggctc ttcagtactg tcatctgatg 14160

taccaggcac ttcatttggc aaaatattag agatattatc gcgcaaatat ctcttcaaag 14220

taggagcttc taaacgctta cgcataaacg atgacgtcag gctcatgtaa aggtttctca 14280

taaatttttt gcgactttgg accttttctc ccttgctact gacattatgg ctgtatataa 14340

taaaagaatt tatgcaggca atgtttatca ttccgtacaa taatgccata ggccacctat 14400

tcgtcttcct actgcaggtc atcacagaac acatttggtc tagcgtgtcc actccgcctt 14460

tagtttgatt ataatacata accatttgcg gtttaccggt actttcgttg atagaagcat 14520

cctcatcaca agatgataat aagtatacca tcttagctgg cttcggttta tatgagacga 14580

gagtaagggg tccgtcaaaa caaaacatcg atgttcccac tggcctggag cgactgtttt 14640

tcagtacttc cggtatctcg cgtttgtttg atcgcacggt tcccacaatg gttgcggcca 14700

gcccgggcta tgg 14713

<210> 54
<211> 15848
<212> DNA
<213> artificial

<220>
<223> Sequence of pLA3166-Cctra intron-Ubiquitin-reaperKR construct.

<400> 54
gggcggccgt tttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600

agttacatga cgagccctaa tgagccgtcg gtggtctata aactgtgcct tacaaatact 660

tgcaactctt ctcgttttga agtcagcaga gttattgcta attgctaatt gctaattgct 720

tttaactgat ttcttcgaaa ttggtgctat gtttatggcg ctattaacaa gtatgaatgt 780

caggtttaac caggggatgc ttaattgtgt tctcaacttc aaaggcagaa atgtttactc 840

ttgaccatgg gtttaggtat aatgttatca agctcctcga gttaacgtta cgttaacgtt 900

aacgttcgag gtcgactcta gaactaccca ccgtactcgt caattccaag ggcatcggta 960
aacatctgct caaactcgaa gtcggccata tccagagcgc cgtagggggc ggagtcgtgg 1020
ggggtaaatc ccggacccgg ggaatccccg tcccccaaca tgtccagatc gaaatcgtct 1080
agcgcgtcgg catgcgccat cgccacgtcc tcgccgtcta agtggagctc gtcccccagg 1140
ctgacatcgg tcgggggggc cgtcgacagt ctgcgcgtgt gtcccgcggg gagaaaggac 1200
aggcgcggag ccgccagccc cgcctcttcg ggggcgtcgt cgtccgggag atcgagcagg 1260
ccctcgatgg tagacccgta attgtttttc gtacgcgcgc ggctgtacgc ggggcccgag 1320
cccgactcgc atttcagttg cttttccaat ccgcagataa tcagctccaa gccgaacagg 1380
aatgccggct cggctccttg atgatcgaac agctcgattg cctgacgcag cagtgggggc 1440
atcgaatcgg ttgttggggt ctcgcgctcc tcttttgcga cttgatgctc ttggtcctcc 1500
agcacgcagc ccagggtaaa gtgaccgacg gcgctcagag cgtagagagc attttccagg 1560
ctgaagcctt gctggcacag gaacgcgagc tggttctcca gtgtctcgta ttgcttttcg 1620
gtcgggcgcg tgccgagatg gactttggca ccgtctcggt gggacagcag agcgcagcgg 1680
aacgacttgg cgttattgcg gaggaagtcc tggaaatggg atagatattg gtgttattgt 1740
tcatgtggca tataaaggac aagcaacaaa aaacgaacat aacatgagag atggttctga 1800
atcagaactt ctgaatatta tcctcccaaa agggttaaag tttttattaa gcatattacg 1860
ttttatacca cttccttatg taaaattttc ttcgtagttt aatatcatgt gaaatcatat 1920
ataatttcta tcgaacgttt gttcaaattg aatgatgtca tttttttgaat aattggttat 1980
aattttataa catctcccga cttcgacatg tggttggtac taatgattgc gaaatcgccc 2040
tccgagaatg agaacaaccg aggtccaccg tctggtcgag attaaaacac ttgaggagtg 2100
ctttggtgac tcgatcaata ggtacagggc tcgttgccaa caatctggcc agctggacat 2160
ccgggacctc gttccccccct ggggtatcaa aatttttgta gtgtaaatag tagtacactc 2220
ttaaaaataa tgaaaattac tgcggacgta attcacatta tgattgaatg acactatcat 2280
tgacatttcc cgaatcagac accatcgtat ttaaaatgtg acacaaattc acctcatttg 2340
gctcgcttct tttatgtgca tccaaaagac gtaaaatcgc atgatttttt cggagtgtgt 2400
agtaagattg tcaaatttta attttaaata accagagccc ataaagcaaa gcaacactag 2460
gaaaaaaccc acaaactcaa cctgtccaaa aaaaaatata acaatcaaag ttgagggaat 2520
cggggtcaaa cgtcatgtaa aaatattttt tgtaaaaacc aaaccaggaa taaatatgaa 2580
tttaatcgga aaaaattgca aaatcgcata atttaatcct ccaactgtac tttatccagc 2640
ctgttgcaga aatgatgttt aaaggttcta atctgtaatt gttattagcc ttcaatactg 2700
atgtagtatt tatttcttat tgaaacattg agagctttat tttccaaagt tgtcattttc 2760

tcattcgtat atcgtaatat gtatattcgt aaatggcaag cacaatgata cttagggtag 2820
tcaaggatat ttcaattacg aaaagatcct gaaacgaccg ggaatcgaac ccttcagcat 2880
ggttttgctt tgtagctgct gaatctaacc actaggctga tgaagatccc attttagggt 2940
tgcaagttct caaagagcaa gaatgccaaa atagtgtcaa aagaagccct atttgacgat 3000
ataccttttta gtctctacgt taatttgcta tgataattta tcatcaatta attggcaaag 3060
cctgatgcac gaaaagatct tcttctaaaa tttcagttgt tcttttcaac acattatgta 3120
atcataaaat ttaattaata aacctttttt ttttgtaact atccacagtt gatcaggcat 3180
aattttcttg gaaagtaaag tccatattta ggttgatgtt gaataaaaaa actttcaatt 3240
cactcttctg tttcacttca gaacttacgt aatacgacat tatgcatggt gcacacggaa 3300
caggataaga cgttcacaag ggatcaacat cacatcggat cgtaatcact ggatctggaa 3360
cacatatgac gccacaagac agcacatttt acacgatcac cagacgtgaa caaggaactg 3420
gatccacaag acgtcacagg aagacggcac atttccaacg gcttcgatgg aacttttctc 3480
gagtcttttt ccaccaatca taaacaccga cctgccagga ctcgccttcc aacgggcaaa 3540
aatgcgtgtg gtggcggtcg agcatctcga tggccagggc atccagcagc gcccgcttat 3600
tcttcacgtg ccagtagagg gtgggctgct ccacgcccag cttctgcgcc aacttgcggg 3660
tcgtcagtcc ctcaatgcca acttcgttca acagctccaa cgcggagttg atgactttgg 3720
acttatccag gcggctgccc atggtggttt ctaaaggtgt tataaatcaa attagttttg 3780
tttttcttg aaaactttgc gtttcctttg atcaacttac cgccagggta ccgcagattg 3840
tttagcttgt tcagctgcgc ttgtttattt gcttagcttt cgcttagcga cgtgttcact 3900
ttgcttgttt gaattgaatt gtcgctccgt agacgaagcg cctctattta tactccggcg 3960
ctcgttttcg agtttaccac tccctatcag tgatagagaa aagtgaaagt cgagtttacc 4020
actccctatc agtgatagag aaaagtgaaa gtcgagttta ccactcccta tcagtgatag 4080
agaaaagtga aagtcgagtt taccactccc tatcagtgat agagaaaagt gaaagtcgag 4140
tttaccactc cctatcagtg atagagaaaa gtgaaagtcg agtttaccac tccctatcag 4200
tgatagagaa aagtgaaagt cgagtttacc actccctatc agtgatagag aaaagtgaaa 4260
gtcgaaacct ggcgcgcccc ggccatcgag aaagagagag agaagagaag agagagaaca 4320
ttcgagaaag agagagagaa gagaagagag agaacatact ccctatcagt gatagagaag 4380
tccctatcag tgatagagat gtccctatca gtgatagaga gttccctatc agtgatagag 4440
acgtccctat cagtgataga gaagtcccta tcagtgatag agagatccct atcagtgata 4500
gagatttccc tatcagtgat agagaggtcc ctatcagtga tagagacttc cctatcagtg 4560
atagagaaat ccctatcagt gatagagaca tccctatcag tgatagagaa ctccctatca 4620
gtgatagaga cctccctatc agtgatagag atcgatgcgg ccgcatggta cccattgctt 4680

gtcatttatt aatttggatg atgtcatttg tttttaaaat tgaactggct ttacgagtag 4740

aattctacgc gtaaaacaca atcaagtatg agtcataatc tgatgtcatg ttttgtacac 4800

ggctcataac cgaactggct ttacgagtag aattctactt gtaatgcacg atcagtggat 4860

gatgtcattt gtttttcaaa tcgagatgat gtcatgtttt gcacacggct cataaactcg 4920

ctttacgagt agaattctac gtgtaacgca cgatcgattg atgagtcatt tgttttgcaa 4980

tatgatatca tacaatatga ctcatttgtt tttcaaaacc gaacttgatt tacgggtaga 5040

attctacttg taaagcacaa tcaaaaagat gatgtcattt gtttttcaaa actgaactcg 5100

ctttacgagt agaattctac gtgtaaaaca caatcaagaa atgatgtcat ttgttataaa 5160

aataaaagct gatgtcatgt tttgcacatg gctcataact aaactcgctt tacgggtaga 5220

attctacgcg taaaacatga ttgataatta aataattcat ttgcaagcta tacgttaaat 5280

caaacggacg ctcgaggttg cacaacacta ttatcgattt gcagttcggg acataaatgt 5340

ttaaatatat cgatgtcttt gtgatgcgcg cgacattttt gtaggttatt gataaaatga 5400

acggatacgt tgcccgacat tatcattaaa tccttggcgt agaatttgtc gggtccattg 5460

tccgtgtgcg ctagcatgcc cgtaacggac ctcgtacttt tggcttcaaa ggttttgcgc 5520

acagacaaaa tgtgccacac ttgcagctct gcatgtgtgc gcgttaccac aaatcccaac 5580

ggcgcagtgt acttgttgta tgcaaataaa tctcgataaa ggcgcggcgc gcgaatgcag 5640

ctgatcacgt acgctcctcg tgttccgttc aaggacggtg ttatcgacct cagattaatg 5700

tttatcggcc gactgttttc gtatccgctc accaaacgcg tttttgcatt aacattgtat 5760

gtcggcggat gttctatatc taatttgaat aaataaacga taaccgcgtt ggttttagag 5820

ggcataataa aagaaatatt gttatcgtgt tcgccattag ggcagtataa attgacgttc 5880

atgttggata ttgtttcagt tgcaagttga cactggcggc gacaagcaat tctaattggg 5940

gtaagttttc ccgttctttt ctgggttctt ccctttttgct catccttgct gcactacctt 6000

caggtgcaag ttgagattca ggccaccatg ggagatccca ccccacccaa gaagaagcgc 6060

aaaccggtcg ccaccatgga cgaggatggt tcagagggcg gccccgccct gttccagagc 6120

gacatgacct tcaaaatctt catcgacggc gaggtgaacg gccagaagtt caccatcgtg 6180

gccgacggca gcagcaagtt cccccacggc gacttcaacg tgcacgccgt gtgcgagacc 6240

ggcaagctgc ccatgagctg gaagcccatc tgccacctga tccagtacgg cgagcccttc 6300

ttcgcccgct accccaacgg catcagccac ttcgcccagg agtgcttccc cgagggcctg 6360

agcatcgacc gcaccgtgcg cttcgagaac gacggcacca tgaccagcca ccacacctac 6420

gagctggacg gcacctgcgt ggtcagccgc atcaccgtga actgcgacgg cttccagccc 6480

gacggcccca tcatgcgcga ccagctggtg gacatcctgc ccaacgagac ccacatgttc 6540

ccccacggcc ccaacgccgt gcgccagctg gccttcatcg gcttcaccac cgccgacggc 6600
ggcctgatga tgggccactt cgacagcaag atgaccttca acggcagccg cgccatcaag 6660
atccccggcc cccacttcgt gaccatcatc accaagcaga tgagggacac cagcgacaag 6720
cgcgaccacg tgtgccagcg cgaggtgacc tacgcccaca gcgtgccccg catcaccagc 6780
gccatcggta gcgacgagga ttccggactc agatctcgac ccaagaaaaa gcggaaggtg 6840
gaggacccgt aagatccacc ggatctagat aactgatcat aatcagccat accacatttg 6900
tagaggtttt acttgcttta aaaaacctcc cacacctccc cctgaacctg aaacataaaa 6960
tgaatgcaat tgttgttgtt aacttgttta ttgcagctta taatggttac aaataaagca 7020
atagcatcac aaatttcaca aataaagcat ttttttcact gcattctagt tgtggtttgt 7080
ccaaactcat caatgtatct taacgcgagt taattaatcc attgctgggc gagctgcgcc 7140
aatcgatgcc aacgccaccc tgcatggcga gcggcaggcc ggcggctacc atgggcgtca 7200
ccatgccctg accgcccccg gagggcagtg aaaaatgtgt gggggggtggt gggggctgcg 7260
caggaactga ttgtgattat ggttgtgccc atggccatgt tgtccaagtc catggacgtg 7320
ggcatgcttg ttgtagccca aatcggcgtt tccgtttcca ccaggaaaca tctctgcttg 7380
tagttcgaat atgctcttta aatcccagct gtattcctca gttatcgagg ttttcttcac 7440
gagtgaaacg aattttcgtc gccttctacg ccattttctt gctcagcccg ttttgtcatt 7500
cgcagcgaag cggtaacagc gggtcgctca tatgacggta tttttttaata cacttcagct 7560
atactgttat ttcaaaaaca tatttctttt gttacttttt atgcagttca tttgccacca 7620
aaaagtagtc ttttggattg atttatttca aaaaatggtg taattcaaga aattcagagg 7680
gccaagtaat atacttaatg accgttattt aaaacacact caaggagatt tatttaaacg 7740
gctacaatgg ttttccaaat aacttattta ctgttgactt ctataaaaca taggtgtata 7800
tattattatt tccttattga gtttgagata attttaattt ccacaatatt ttttcttgtg 7860
attaacagag aaagtcaaac tacataacat ttatcgggta aaagtctcta tgaaggtagc 7920
ggttaacagt gaagtcgcaa aagtggtggc cgtacgccaa tcgagcgtag taccccctaac 7980
ctgcaatatt tttagttggt tttttccgca atagccccag ttttctcaaa gagtgcaaca 8040
agtgattctg tttatgtttt caacaacttc tctctgcgga acttaacgtg agcggacgta 8100
tgcggacgcg tttaaactcg cgttaagata cattgatgag tttggacaaa ccacaactag 8160
aatgcagtga aaaaaatgct ttatttgtga aatttgtgat gctattgctt tatttgtaac 8220
cattataagc tgcaataaac aagttaacaa caacaattgc attcatttta tgtttcaggt 8280
tcagggggag gtgtgggagg ttttttaaag caagtaaaac ctctacaaat gtggtatggc 8340
tgattatgat caccggtgtt actggctcgg acggcggtaa cggccgctgc ggcggccggt 8400
gcgcgggtgg cagctggtgt actggcgcag ggtctccagc accacggtgg ccaggaagcg 8460

ccactggctc tcgcgcaggc gcaggatctg ctgctcgcgc tgctcggcct cgcgcagcag 8520
ggtggcctga tccgggatgt agaaggccac ggcgccacca cggagacgaa ggaccaagtg 8580
aagggtggac tccttctgga tgttgtaatc ggacagggtg cgtccgtctt ccagttgctt 8640
acctatagat accatagatg tatggattag tatcatatac atacaaaggc tatttttggg 8700
acatattaat attaacaatt tccgtgatag ttttcaccat ttttgttgaa tgttacgttg 8760
aaaatttaaa tttgttttaa attaatttta ccagtcatgt gttcttaaaa gtttttatga 8820
ttgaaacggc ataaagtggt tcaaaaattt atcaagaaag gctttccttt tttaaatctt 8880
atctttttct cttaaaaatc actagtcaat tcattattaa tttgttaact tgaatttgga 8940
atgtctattt actttcagat aaattaaagc aagaaactta atattcgaaa aaaattgatt 9000
ctaaatggaa tttcacttga tcttcatgta tgcatatcaa tttttattta cattgtataa 9060
taagtttcga gttgattgtt gtaatccaca ggtgtcccag agaattaaat tccaaattac 9120
ccaagtttat tgaatgttga ttgtagtttc agttgctttg ttgctgcaac aatggcttgt 9180
tgattgtaga tattttccct ttccttggtt tacttattac atagactgaa aaagaggttt 9240
actttttga tacttatgaa aaatttctat tagtgattac taaccaatcg ctatatgttt 9300
actagaaaac aaataaactc tttacattaa cattcaataa tgtttgctct gtaaccgaca 9360
attgaaggcg ttacagcaac agtaatataa ctagcttctt aaccctcatc tattaacccc 9420
atcgtttaaa acactatgtt aaatggtcta acaaatctag atactaatag atgtcttatt 9480
acttagcagc cacagctgca acatccaaga caatttttga aacttcttat tgagctcttg 9540
gcagcagaaa tgttggtatt tttcacagct ttctgaaaga ccggcacctt cctccggttc 9600
ccgtttctga attcaagagg atttccgacc cccaattaat cccgaaacaa ataaggtata 9660
ttcaaaatga tggaaaagtc atggctgctg accttatttt tattcctatt gatagaatat 9720
tattcccctt ttaaatacac tgtactaaga ggtccggcta taattttact cacttgtcga 9780
ttatcccata gaatgttgat tgtagttggt tgcttttcca ggtgagagtt gatcaagtca 9840
caaaagttag cgtgtgttga ttgtagattt gaaggtaaaa taatttttgc acccattcat 9900
cgggtaaaac gttctccata gaatacattt ccatcgataa ttgataactt atgaatttca 9960
aagaaaaaaa tatgctttta aaattaccag cgaagatcag acgctgctga tctgggggga 10020
ttccctcctt atcctgaatc ttggccttta cattctcaat ggtgtccgat ggctctacct 10080
cgagggtgat ggtctttccg gtcaaagtct tcacaaagat ctgcattttg gattgctagc 10140
gcagattgtt tagcttgttc agctgcgctt gtttatttgc ttagctttcg cttagcgacg 10200
tgttcacttt gcttgtttga attgaattgt cgctccgtag acgaagcgcc tctatttata 10260
ctccggcgct cggtccgcat agtcgacatt tcgagtttac cactccctat cagtgataga 10320

gaaaagtgaa agtcgagttt accactccct atcagtgata gagaaaagtg aaagtcgagt 10380
ttaccactcc ctatcagtga tagagaaaag tgaaagtcga gtttaccact ccctatcagt 10440
gatagagaaa agtgaaagtc gagtttacca ctccctatca gtgatagaga aaagtgaaag 10500
tcgagtttac cactccctat cagtgataga gaaaagtgaa agtcgagttt accactccct 10560
atcagtgata gagaaaagtg aaagtcgagc tcggtacccg ggtcgaggta ggcgtgtacg 10620
gtgggaggaa atctggccgg ccgcaaccat tgtgggaacc gtgcgatcaa acaaacgcga 10680
gataccggaa gtactgaaaa acagtcgctc caggccagtg ggaacatcga tgttttgttt 10740
tgacggaccc cttactctcg tctcatataa accgaagcca gctaagatgg tatacttatt 10800
atcatcttgt gatgaggatg cttctatcaa cgaaagtacc ggtaaaccgc aaatggttat 10860
gtattataat caaactaaag gcggagtgga cacgctagac caaatgtgtt ctgtgatgac 10920
ctgcagtagg aagacgaata ggtggcctat ggcattattg tacggaatga taaacattgc 10980
ctgcataaat tcttttatta tatacagcca taatgtcagt agcaagggag aaaaggtcca 11040
aagtcgcaaa aaatttatga gaaaccttta catgagcctg acgtcatcgt ttatgcgtaa 11100
gcgtttagaa gctcctactt tgaagagata tttgcgcgat aatatctcta atattttgcc 11160
aaatgaagtg cctggtacat cagatgacag tactgaagag ccagtaatga aaaaacgtac 11220
ttactgtact tactgcccct ctaaaataag gcgaaaggca aatgcatcgt gcaaaaaatg 11280
caaaaaagtt atttgtcgag agcataatat tgatatgtgc caaagttgtt tctgactgac 11340
taataagtat aatttgtttc tattatgtat aagttaagct aattacttat tttataatac 11400
aacatgactg tttttaaagt acaaaataag tttatttttg taaaagagag aatgtttaaa 11460
agttttgtta ctttatagaa gaaattttga gtttttgttt ttttttaata aataaataaa 11520
cataaataaa ttgtttgttg aatttattat tagtatgtaa gtgtaaatat aataaaactt 11580
aatatctatt caaattaata aataaacctc gatatacaga ccgataaaac acatgcgtca 11640
attttacgca tgattatctt taacgtacgt cacaatatga ttatctttct agggttaaat 11700
aatagtttct aatttttta ttattcagcc tgctgtcgtg aataccgtat atctcaacgc 11760
tgtctgtgag attgtcgtat tctagccttt ttagtttttc gctcatcgac ttgatattgt 11820
ccgacacatt ttcgtcgatt tgcgttttga tcaaagactt gagcagagac acgttaatca 11880
actgttcaaa ttgatccata ttaacgatat caacccgatg cgtatatggt gcgtaaaata 11940
tattttttaa ccctcttata ctttgcactc tgcgttaata cgcgttcgtg tacagacgta 12000
atcatgtttt cttttttgga taaaactcct actgagtttg acctcatatt agaccctcac 12060
aagttgcaaa acgtggcatt ttttaccaat gaagaattta aagttatttt aaaaaatttc 12120
atcacagatt taaagaagaa ccaaaaatta aattatttca acagtttaat cgaccagtta 12180
atcaacgtgt acacagacgc gtcggcaaaa aacacgcagc ccgacgtgtt ggctaaaatt 12240

attaaatcaa cttgtgttat agtcacggat ttgccgtcca acgtgttcct caaaaagttg 12300
aagaccaaca agtttacgga cactattaat tatttgattt tgccccactt cattttgtgg 12360
gatcacaatt ttgttatatt ttaaacaaag cttggcactg gccgtcgttt tacaacgtcg 12420
tgactgggaa aaccctggcg ttacccaact taatcgcctt gcagcacatc ccccttttcgc 12480
cagctggcgt aatagcgaag aggcccgcac cgatcgccct tcccaacagt tgcgcagcct 12540
gaatggcgaa tggcgcctga tgcggtattt tctccttacg catctgtgcg gtatttcaca 12600
ccgcatatgg tgcactctca gtacaatctg ctctgatgcc gcatagttaa gccagccccg 12660
acacccgcca acacccgctg acgcgccctg acgggcttgt ctgctcccgg catccgctta 12720
cagacaagct gtgaccgtct ccgggagctg catgtgtcag aggttttcac cgtcatcacc 12780
gaaacgcgcg agacgaaagg gcctcgtgat acgcctattt ttataggtta atgtcatgat 12840
aataatggtt tcttagacgt caggtggcac ttttcgggga aatgtgcgcg gaacccctat 12900
ttgtttattt ttctaaatac attcaaatat gtatccgctc atgagacaat aaccctgata 12960
aatgcttcaa taatattgaa aaaggaagag tatgagtatt caacatttcc gtgtcgccct 13020
tattcccttt tttgcggcat tttgccttcc tgtttttgct cacccagaaa cgctggtgaa 13080
agtaaaagat gctgaagatc agttgggtgc acgagtgggt tacatcgaac tggatctcaa 13140
cagcggtaag atccttgaga gttttcgccc cgaagaacgt tttccaatga tgagcacttt 13200
taaagttctg ctatgtggcg cggtattatc ccgtattgac gccgggcaag agcaactcgg 13260
tcgccgcata cactattctc agaatgactt ggttgagtac tcaccagtca cagaaaagca 13320
tcttacggat ggcatgacag taagagaatt atgcagtgct gccataacca tgagtgataa 13380
cactgcggcc aacttacttc tgacaacgat cggaggaccg aaggagctaa ccgctttttt 13440
gcacaacatg ggggatcatg taactcgcct tgatcgttgg gaaccggagc tgaatgaagc 13500
cataccaaac gacgagcgtg acaccacgat gcctgtagca atggcaacaa cgttgcgcaa 13560
actattaact ggcgaactac ttactctagc ttcccggcaa caattaatag actggatgga 13620
ggcggataaa gttgcaggac cacttctgcg ctcggccctt ccggctggct ggtttattgc 13680
tgataaatct ggagccggtg agcgtgggtc tcgcggtatc attgcagcac tggggccaga 13740
tggtaagccc tcccgtatcg tagttatcta cacgacgggg agtcaggcaa ctatggatga 13800
acgaaataga cagatcgctg agataggtgc ctcactgatt aagcattggt aactgtcaga 13860
ccaagtttac tcatatatac tttagattga tttaaaactt catttttaat ttaaaaggat 13920
ctaggtgaag atcctttttg ataatctcat gaccaaaatc ccttaacgtg agttttcgtt 13980
ccactgagcg tcagaccccg tagaaaagat caaaggatct tcttgagatc cttttttct 14040
gcgcgtaatc tgctgcttgc aaacaaaaaa accaccgcta ccagcggtgg tttgtttgcc 14100

ggatcaagag ctaccaactc tttttccgaa ggtaactggc ttcagcagag cgcagatacc 14160

aaatactgtc cttctagtgt agccgtagtt aggccaccac ttcaagaact ctgtagcacc 14220

gcctacatac ctcgctctgc taatcctgtt accagtggct gctgccagtg gcgataagtc 14280

gtgtcttacc gggttggact caagacgata gttaccggat aaggcgcagc ggtcgggctg 14340

aacggggggt tcgtgcacac agcccagctt ggagcgaacg acctacaccg aactgagata 14400

cctacagcgt gagcattgag aaagcgccac gcttcccgaa gggagaaagg cggacaggta 14460

tccggtaagc ggcagggtcg gaacaggaga gcgcacgagg gagcttccag ggggaaacgc 14520

ctggtatctt tatagtcctg tcgggtttcg ccacctctga cttgagcgtc gatttttgtg 14580

atgctcgtca ggggggcgga gcctatggaa aaacgccagc aacgcggcct ttttacggtt 14640

cctggccttt tgctggcctt ttgctcacat gttctttcct gcgttatccc ctgattctgt 14700

ggataaccgt attaccgcct ttgagtgagc tgataccgct cgccgcagcc gaacgaccga 14760

gcgcagcgag tcagtgagcg aggaagcgga agagcgccca atacgcaaac cgcctctccc 14820

cgcgcgttgg ccgattcatt aatgcagctg gcacgacagg tttcccgact ggaaagcggg 14880

cagtgagcgc aacgcaatta atgtgagtta gctcactcat taggcacccc aggctttaca 14940

ctttatgctt ccggctcgta tgttgtgtgg aattgtgagc ggataacaat ttcacacagg 15000

aaacagctat gaccatgatt acgaatttcg acgctcgcgc gacttggttt gccattcttt 15060

agcgcgcgtc gcgtcacaca gcttggccac aatgtggttt ttgtcaaacg aagattctat 15120

gacgtgttta aagtttaggt cgagtaaagc gcaaatcttt tttaacccta gaaagatagt 15180

ctgcgtaaaa ttgacgcatg cattcttgaa atattgctct ctctttctaa atagcgcgaa 15240

tccgtcgctg tgcatttagg acatctcagt cgccgcttgg agctcccgtg aggcgtgctt 15300

gtcaatgcgg taagtgtcac tgattttgaa ctataacgac cgcgtgagtc aaaatgacgc 15360

atgattatct tttacgtgac ttttaagatt taactcatac gataattata ttgttatttc 15420

atgttctact tacgtgataa cttattatat atatattttc ttgttataga tatcgtgact 15480

aatatataat aaaatgggta gttctttaga cgatgagcat atcctctctg ctcttctgca 15540

aagcgatgac gagcttgttg gtgaggattc tgacagtgaa atatcagatc acgtaagtga 15600

agatgacgtc cagagcgata cagaagaagc gtttatagat gaggtacatg aagtgcagcc 15660

aacgtcaagc ggtagtgaaa tattagacga acaaaatgtt attgaacaac caggttcttc 15720

attggcttct aacagaatct tgaccttgcc acagaggact attagaggta agaataaaca 15780

ttgttggtca acttcaaagt ccacgaggcg tagccgagtc tctgcactga acattgtcag 15840

atcggccc 15848

<210> 55
<211> 17802

<212> DNA
<213> artificial

<220>
<223> Sequence of pLA3376-Bztra intron-reaperKR and Bztra-intron-tTAV3.

<400> 55
gggcggccgt ttttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600

agttacatga cgagccctaa tgagccgtcg gtggtctata aactgtgcct tacaaatact 660

tgcaactctt ctcgttttga agtcagcaga gttattgcta attgctaatt gctaattgct 720

tttaactgat ttcttcgaaa ttggtgctat gtttatggcg ctattaacaa gtatgaatgt 780

caggtttaac caggggatgc ttaattgtgt tctcaacttc aaaggcagaa atgtttactc 840

ttgaccatgg gtttaggtat aatgttatca agctcctcga gttaacgtta cgttaacgtt 900

aacgttcgag gtcgactcta gacaccggtg ttagccgccg tactcatcga tgcccagggc 960

gtcggtgaac atctgctcga actcgaaatc ggccatatcc agggcgccgt agggggcgct 1020

atcgtgcggg gtgaatcccg gtcccgggct atcgccatcg cccagcatgt ccaggtcgaa 1080

gtcgtccagg gcatcggcgt gggccatcgc cacatcctcg ccatccaggt gcagctcatc 1140

gcccaggctc acgtcggtcg gcggggcggt cgacaggcgg cgggtgtgtc cggccggcag 1200

gaagctcagg cgcggggcgg ccaggcccgc ctcctccggg gcatcatcat ccggcagatc 1260

cagcaggccc tcgatggtgc tgccgtagtt gttcttggtg cgggcgcggc tgtaggcggg 1320

gcccgagccc gactcgcatt tcagttgctt ttccaatccg cagataatca gctccaagcc 1380

gaacaggaat gccggctcgg ctccttgatg atcgaacagc tcgattgcct gacgcagcag 1440

tgggggcatc gaatcggttg ttggggtctc gcgctcctct tttgcgactt gatgctcttg 1500

gtcctccagc acgcagccca gggtaaagtg accgacggcg ctcagagcgt agagagcatt 1560

ttccaggctg aagccttgct ggcacaggaa cgcgagctgg ttctccagtg tctcgtattg 1620

cttttcggtc gggcgcgtgc cgagatggac tttggcaccg tctcggtggg acagcagagc 1680

gcagcggaac gacttggcgt tattgcggag gaagtcctgc caggactcgc cttccaacgg 1740
gcaaaaatgc gtgtggtggc ggtcgagcat ctcgatggcc agggcatcca gcagcgcccg 1800
cttattcttc acgtgccagt agagggtggg ctgctccacg cccagcttct gcgccaactt 1860
gcgggtcgtc agtccctcaa tgccaacttc gttcaacagc tccaacgcgg agttgatgac 1920
tttggactta tccaggcggc tgacctatag ataccataga tgtatggatt agtatcatat 1980
acatacaaag gctatttttg ggacatatta atattaacaa tttccgtgat agttttcacc 2040
atttttgttg aatgttacgt tgaaaattta aatttgtttt aaattaattt taccagtcat 2100
gtgttcttaa aagtttttat gattgaaacg gcataaagtg gttcaaaaat ttatcaagaa 2160
aggctttcct tttttaaatc ttatcttttt ctcttaaaaa tcactagtca attcattatt 2220
aatttgttaa cttgaatttg gaatgtctat ttactttcag ataaattaaa gcaagaaact 2280
taatattcga aaaaaattga ttctaaatgg aatttcactt gatcttcatg tatgcatatc 2340
aatttttatt tacattgtat aataagtttc gagttgattg ttgtaatcca caggtgtccc 2400
agagaattaa attccaaatt acccaagttt attgaatgtt gattgtagtt tcagttgctt 2460
tgttgctgca acaatggctt gttgattgta gatattttcc ctttccttgg tttacttatt 2520
acatagactg aaaaagaggt ttactttttt gatacttatg aaaaatttct attagtgatt 2580
actaaccaat cgctatatgt ttactagaaa acaaataaac tctttacatt aacattcaat 2640
aatgtttgct ctgtaaccga caattgaagg cgttacagca acagtaatat aactagcttc 2700
ttaaccctca tctattaacc ccatcgttta aaacactatg ttaaatggtc taacaaatct 2760
agatactaat agatgtctta ttacttagca gccacagctg caacatccaa gacaattttt 2820
gaaacttctt attgagctct tggcagcaga aatgttggta tttttcacag ctttctgaaa 2880
gaccggcacc ttcctccggt tcccgtttct gaattcaaga ggatttccga cccccaatta 2940
atcccgaaac aaataaggta tattcaaaat gatggaaaag tcatggctgc tgaccttatt 3000
tttattccta ttgatagaat attattcccc ttttaaatac actgtactaa gaggtccggc 3060
tataatttta ctcacttgtc gattatccca tagaatgttg attgtagttg gttgcttttc 3120
caggtgagag ttgatcaagt cacaaaagtt agcgtgtgtt gattgtagat ttgaaggtaa 3180
aataattttt gcacccattc atcgggtaaa acgttctcca tagaatacat ttccatcgat 3240
aattgataac ttatgaattt caaagaaaaa aatatgcttt taaaattacc atggtggcta 3300
gcgcagattg tttagcttgt tcagctgcgc ttgtttattt gcttagcttt cgcttagcga 3360
cgtgttcact ttgcttgttt gaattgaatt gtcgctccgt agacgaagcg cctctattta 3420
tactccggcg ctcgttttcg agtttaccac tccctatcag tgatagagaa aagtgaaagt 3480
cgagtttacc actccctatc agtgatagag aaaagtgaaa gtcgagttta ccactcccta 3540
tcagtgatag agaaaagtga aagtcgagtt taccactccc tatcagtgat agagaaaagt 3600

gaaagtcgag tttaccactc cctatcagtg atagagaaaa gtgaaagtcg agtttaccac 3660

tccctatcag tgatagagaa aagtgaaagt cgagtttacc actccctatc agtgatagag 3720

aaaagtgaaa gtcgaaacct gcgcgccgtt taaactcgcg ttaagataca ttgatgagtt 3780

tggacaaacc acaactagaa tgcagtgaaa aaaatgcttt atttgtgaaa tttgtgatgc 3840

tattgcttta tttgtaacca ttataagctg caataaacaa gttaacaaca acaattgcat 3900

tcattttatg tttcaggttc agggggaggt gtgggaggtt ttttaaagca agtaaaacct 3960

ctacaaatgt ggtatggctg attatgatcg ctctagacac cggtgctacc cgccatactc 4020

atcgatgccc agcgcgtcgg tgaacatttg ctcgaactcg aagtcggcca tgtccagggc 4080

gccgtacggg gcgctatcgt ggggcgtgaa gcccggtccc gggctatctc catcgcccag 4140

catatccagg tcgaaatcgt ccagggcgtc ggcgtgggcc attgccacat cctctccatc 4200

caggtgcagc tcgtcgccca ggctcacatc ggtcggcggg gcggtgctca ggcggcgcgt 4260

gtgtccggcg ggcaggaagc tcaggcgggg ggcggccagg ccggcttcct ccggggcatc 4320

gtcatccggc aggtccagca gtccctcgat ggtgctgcca tagttgttct tggtacgggc 4380

gcggctgtag gcgctgccgc tctcgcactt cagctgcttt tccaggccgc agatgatcag 4440

ctccaggccg aacaggaagg ccggctcggc gccctggtga tcgaacagct cgatggcctg 4500

gcgcagcagc ggcggcatgc tatcggtggt cggggtctcg cgctcctcct tggccacctg 4560

gtgctcctga tcctccagca cacagcccag ggtgaagtgg cccacggcgc tcagggcgta 4620

cagggcgttc tccaggctga agccctgctg gcacaggaag gccagctggt tctccagggt 4680

ctcgtactgc ttctcggtcg ggcgggtgcc caggtgcacc ttggcgccat cgcggtgcga 4740

cagcagggcg cagcggaagc tcttggcgtt gttgcgcagg aaatcctgcc agctctcgcc 4800

ctccagcggg cagaagtggg tgtggtggcg atccagcatt tcgatggcca gggcgtccag 4860

cagggcgcgc ttgttcttca cgtgccagta cagggtcggc tgttccacgc ccagcttctg 4920

ggccagcttg cgggtggtca ggccctcgat accaacttcg ttcagcagct ccagggcgct 4980

gttgatcacc ttgctcttgt ccaggcggct gacctgtgaa tacggttaat gtcactatta 5040

gtgatttata aaaataaatt tgatttatat atcaacaatt tttcatcgca gccttcagct 5100

ttttgttgaa taattataat gatatttttt acgattcaaa tcatttaatt gttactcaac 5160

gaaataagtt taattcaaat tttaaaacaa gattatatat taagattaga ataagaaaga 5220

actttgttag attatttaat taaaaagatt aaaatttaag tctccagtca ctatttaaag 5280

atcatctttc aaacgttaaa gtgaattcaa acgagacgtt caaatttcga ttaaacagta 5340

attaactcta aatttctatc acgaattaag ttattgaata tgaaggttta tatttattta 5400

catcatctaa taggtttgag ttgattgttg taatccgcat gtgccagaag atatcaattt 5460

ccaaattgtc cgagttcatg gaatgttgat tgttgtttgt gttgctttgt aattgttgca 5520
gggagtattt atggtttgtt gattgtagta taaggctgtt tctaaaggct agaaaataat 5580
tttatttatt tgaaaataag taaatataca taatattact aacaataggt cgtcctattt 5640
tttgatattc tgcacaaatt tttaaaacac aaagattgca atacttttag acactaatac 5700
tgcacactct gaaaaattat taaattattt ttaaaaactt accttaatac tttagagaaa 5760
aatattatac cgcacctttc tactttatac tcactttatt ataccagttg catgttgatt 5820
gtagttcttt gacaagaaaa tattccatat tgctccaaat tatcttggta agttgattgg 5880
tgcgtcattt gagcaagcta acaccttgtc tcatttaagt tcgcctcaag atctcatagc 5940
atttttaaat atcactatat ttagtaagta attagaatta ccatggtggt ttgctagccg 6000
ttctatcaga tgtgctccgg gaaacagaaa tgttcaacta agttctggcg gacgacgcaa 6060
cacctttata tactttgcca agcgcacagg tagaaaggac ctattttggg gattaaaaaa 6120
catctgcctg ttttattgcc atacccgcga aaattcgcga aatccgctac tttacctact 6180
ggggttcctg gaaaatgggc gaagaacggc aaagaactgg tactttccgt caataattgt 6240
ttagaagaga gagaacatac tccctatcag tgatagagaa gtccctatca gtgatagaga 6300
tgtccctatc agtgatagag agttccctat cagtgataga gacgtcccta tcagtgatag 6360
agaagtccct atcagtgata gagagatccc tatcagtgat agagatttcc ctatcagtga 6420
tagagaggtc cctatcagtg atagagactt ccctatcagt gatagagaaa tccctatcag 6480
tgatagagac atccctatca gtgatagaga actccctatc agtgatagag acctccctat 6540
cagtgataga gatcgatgcg gccgcatggt acccattgct tgtcatttat taatttggat 6600
gatgtcattt gtttttaaaa ttgaactggc tttacgagta gaattctacg cgtaaaacac 6660
aatcaagtat gagtcataat ctgatgtcat gttttgtaca cggctcataa ccgaactggc 6720
tttacgagta gaattctact tgtaatgcac gatcagtgga tgatgtcatt tgtttttcaa 6780
atcgagatga tgtcatgttt tgcacacggc tcataaactc gctttacgag tagaattcta 6840
cgtgtaacgc acgatcgatt gatgagtcat ttgttttgca atatgatatc atacaatatg 6900
actcatttgt tttcaaaac cgaacttgat ttacgggtag aattctactt gtaaagcaca 6960
atcaaaaaga tgatgtcatt tgtttttcaa aactgaactc gctttacgag tagaattcta 7020
cgtgtaaaac acaatcaaga aatgatgtca tttgttataa aaataaaagc tgatgtcatg 7080
ttttgcacat ggctcataac taaactcgct ttacgggtag aattctacgc gtaaaacatg 7140
attgataatt aaataattca tttgcaagct atacgttaaa tcaaacggac gctcgaggtt 7200
gcacaacact attatcgatt tgcagttcgg gacataaatg tttaaatata tcgatgtctt 7260
tgtgatgcgc gcgacatttt tgtaggttat tgataaaatg aacggatacg ttgcccgaca 7320
ttatcattaa atccttggcg tagaatttgt cgggtccatt gtccgtgtgc gctagcatgc 7380

ccgtaacgga cctcgtactt ttggcttcaa aggttttgcg cacagacaaa atgtgccaca 7440
cttgcagctc tgcatgtgtg cgcgttacca caaatcccaa cggcgcagtg tacttgttgt 7500
atgcaaataa atctcgataa aggcgcggcg cgcgaatgca gctgatcacg tacgctcctc 7560
gtgttccgtt caaggacggt gttatcgacc tcagattaat gtttatcggc cgactgtttt 7620
cgtatccgct caccaaacgc gtttttgcat taacattgta tgtcggcgga tgttctatat 7680
ctaatttgaa taaataaacg ataaccgcgt tggttttaga gggcataata aaagaaatat 7740
tgttatcgtg ttcgccatta gggcagtata aattgacgtt catgttggat attgtttcag 7800
ttgcaagttg acactggcgg cgacaagcaa ttctaattgg ggtaagtttt cccgttcttt 7860
tctgggttct tcccttttgc tcatccttgc tgcactacct tcaggtgcaa gttgagattc 7920
aggccaccat gggagatccc accccaccca agaagaagcg caaaccggtc gccaccatgg 7980
agagcgacga gagcggcctg cccgccatgg agatcgagtg ccgcatcacc ggcaccctga 8040
acggcgtgga gttcgagctg gtgggcggcg gagagggcac ccccgagcag ggccgcatga 8100
ccaacaagat gaagagcacc aaaggcgccc tgaccttcag cccctacctg ctgagccacg 8160
tgatgggcta cggcttctac cacttcggca cctaccccag cggctacgag aaccccttcc 8220
tgcacgccat caacaacggc ggctacacca acacccgcat cgagaagtac gaggacggcg 8280
gcgtgctgca cgtgagcttc agctaccgct acgaggccgg ccgcgtgatc ggcgacttca 8340
aggtgatggg caccggcttc cccgaggaca gcgtgatctt caccgacaag atcatccgca 8400
gcaacgccac cgtggagcac ctgcacccca tgggcgataa cgatctggat ggcagcttca 8460
cccgcacctt cagcctgcgc gacggcggct actacagctc cgtggtggac agccacatgc 8520
acttcaagag cgccatccac cccagcatcc tgcagaacgg gggccccatg ttcgccttcc 8580
gccgcgtgga ggaggatcac agcaacaccg agctgggcat cgtggagtac cagcacgcct 8640
tcaagacccc ggatgcagat gccggtgaag aaagatctcg acccaagaaa aagcggaagg 8700
tggaggaccc gtaagatcca ccggatctag ataactgatc ataatcagcc ataccacatt 8760
tgtagaggtt ttacttgctt taaaaaacct cccacacctc cccctgaacc tgaaacataa 8820
aatgaatgca attgttgttg ttaacttgtt tattgcagct tataatggtt acaaataaag 8880
caatagcatc acaaatttca caaataaagc atttttttca ctgcattcta gttgtggttt 8940
gtccaaactc atcaatgtat cttaacgcga gttatcgcgc tcgcgcgact gacggtcgta 9000
agcacccgcg tacgtgtcca ccccggtcac aaccccttgt gtcatgtcgg cgaccctacg 9060
cccccaactg agagaactca aaggttaccc cagttggggc actactcccg aaaaccgctt 9120
ctgacctggg aaaacgtgaa gccccggggc atccgctgag ggttgccgcc ggggcttcgg 9180
tgtgtccgtc agtacttaat taacaccgaa atcgtaattc acggcatcat tacaaaatat 9240

tttgacgttt tggacctcgt ccctaatgac accataacgg tggccttgaa gtatatttaa 9300
ccctagaaag atagtctgcg taaaattgac gcatgcattc ttgaaatatt gctctctctt 9360
tctaaatagc gcgaatccgt cgctgtgcat ttaggacatc tcagtcgccg cttggagctc 9420
ccgtgaggcg tgcttgtcaa tgcggtaagt gtcactgatt ttgaactata acgaccgcgt 9480
gagtcaaaat gacgcatgat tatcttttac gtgactttta agatttaact catacgataa 9540
ttatattgtt atttcatgtt ctacttacgt gataacttat tatatatata ttttcttgtt 9600
atagatatcg tgactaatat ataataaaat gggtagttct ttagacgatg agcatatcct 9660
ctctgctctt ctgcaaagcg atgacgagct tgttggtgag gattctgaca gtgaaatatc 9720
agatcacgta agtgaagatg acgtccagga aatctggccg gccgcaacca ttgtgggaac 9780
cgtgcgatca aacaaacgcg agataccgga agtactgaaa aacagtcgct ccaggccagt 9840
gggaacatcg atgttttgtt ttgacggacc ccttactctc gtctcatata aaccgaagcc 9900
agctaagatg gtatacttat tatcatcttg tgatgaggat gcttctatca acgaaagtac 9960
cggtaaaccg caaatggtta tgtattataa tcaaactaaa ggcggagtgg acacgctaga 10020
ccaaatgtgt tctgtgatga cctgcagtag gaagacgaat aggtggccta tggcattatt 10080
gtacggaatg ataaacattg cctgcataaa ttcttttatt atatacagcc ataatgtcag 10140
tagcaaggga gaaaaggtcc aaagtcgcaa aaaatttatg agaaaccttt acatgagcct 10200
gacgtcatcg tttatgcgta agcgtttaga agctcctact ttgaagagat atttgcgcga 10260
taatatctct aatattttgc caaatgaagt gcctggtaca tcagatgaca gtactgaaga 10320
gccagtaatg aaaaaacgta cttactgtac ttactgcccc tctaaaataa ggcgaaaggc 10380
aaatgcatcg tgcaaaaaat gcaaaaaagt tatttgtcga gagcataata ttgatatgtg 10440
ccaaagttgt ttctgactga ctaataagta taatttgttt ctattatgta taagttaagc 10500
taattactta ttttataata caacatgact gtttttaaag tacaaaataa gtttattttt 10560
gtaaaagaga gaatgtttaa aagttttgtt actttataga agaaattttg agtttttgtt 10620
ttttttaat aaataaataa acataaataa attgtttgtt gaatttatta ttagtatgta 10680
agtgtaaata taataaaact taatatctat tcaaattaat aaataaacct cgatatacag 10740
accgataaaa cacatgcgtc aattttacgc atgattatct ttaacgtacg tcacaatatg 10800
attatctttc tagggttaaa taatagtttc taattttttt attattcagc ctgctgtcgt 10860
gaataccgta tatctcaacg ctgtctgtga gattgtcgta ttctagcctt tttagttttt 10920
cgctcatcga cttgatattg tccgacacat tttcgtcgat ttgcgttttg atcaaagact 10980
tgagcagaga cacgttaatc aactgttcaa attgatccat attaacgata tcaacccgat 11040
gcgtatatgg tgcgtaaaat atatttttta accctcttat actttgcact ctgcgttaat 11100
acgcgttcgt gtacagacgt aatcatgttt tctttttggg ataaaactcc tactgagttt 11160

gacctcatat tagaccctca caagttgcaa aacgtggcat tttttaccaa tgaagaattt 11220

aaagttattt taaaaaattt catcacagat ttaaagaaga accaaaaatt aaattatttc 11280

aacagtttaa tcgaccagtt aatcaacgtg tacacagacg cgtcggcaaa aaacacgcag 11340

cccgacgtgt tggctaaaat tattaaatca acttgtgtta tagtcacgga tttgccgtcc 11400

aacgtgttcc tcaaaaagtt gaagaccaac aagtttacgg acactattaa ttatttgatt 11460

ttgccccact tcattttgtg ggatcacaat tttgttatat tttaaacaaa gcttggcact 11520

ggccgtcgtt ttacaacgtc gtgactggga aaaccctggc gttacccaac ttaatcgcct 11580

tgcagcacat ccccctttcg ccagctggcg taatagcgaa gaggcccgca ccgatcgccc 11640

ttcccaacag ttgcgcagcc tgaatggcga atggcgcctg atgcggtatt ttctccttac 11700

gcatctgtgc ggtatttcac accgcatatg gtgcactctc agtacaatct gctctgatgc 11760

cgcatagtta agccagcccc gacacccgcc aacacccgct gacgcgccct gacgggcttg 11820

tctgctcccg gcatccgctt acagacaagc tgtgaccgtc tccgggagct gcatgtgtca 11880

gaggttttca ccgtcatcac cgaaacgcgc gagacgaaag ggcctcgtga tacgcctatt 11940

tttataggtt aatgtcatga taataatggt ttcttagacg tcaggtggca cttttcgggg 12000

aaatgtgcgc ggaaccccta tttgtttatt tttctaaata cattcaaata tgtatccgct 12060

catgagacaa taaccctgat aaatgcttca ataatattga aaaaggaaga gtatgagtat 12120

tcaacatttc cgtgtcgccc ttattccctt ttttgcggca ttttgccttc ctgtttttgc 12180

tcacccagaa acgctggtga aagtaaaaga tgctgaagat cagttgggtg cacgagtggg 12240

ttacatcgaa ctggatctca acagcggtaa gatccttgag agttttcgcc ccgaagaacg 12300

ttttccaatg atgagcactt ttaaagttct gctatgtggc gcggtattat cccgtattga 12360

cgccgggcaa gagcaactcg gtcgccgcat acactattct cagaatgact tggttgagta 12420

ctcaccagtc acagaaaagc atcttacgga tggcatgaca gtaagagaat tatgcagtgc 12480

tgccataacc atgagtgata acactgcggc caacttactt ctgacaacga tcggaggacc 12540

gaaggagcta accgcttttt tgcacaacat gggggatcat gtaactcgcc ttgatcgttg 12600

ggaaccggag ctgaatgaag ccataccaaa cgacgagcgt gacaccacga tgcctgtagc 12660

aatggcaaca acgttgcgca aactattaac tggcgaacta cttactctag cttcccggca 12720

acaattaata gactggatgg aggcggataa agttgcagga ccacttctgc gctcggccct 12780

tccggctggc tggtttattg ctgataaatc tggagccggt gagcgtgggt ctcgcggtat 12840

cattgcagca ctggggccag atggtaagcc ctcccgtatc gtagttatct acacgacggg 12900

gagtcaggca actatggatg aacgaaatag acagatcgct gagataggtg cctcactgat 12960

taagcattgg taactgtcag accaagttta ctcatatata ctttagattg atttaaaact 13020

tcatttttaa tttaaaagga tctaggtgaa gatccttttt gataatctca tgaccaaaat 13080

cccttaacgt gagttttcgt tccactgagc gtcagacccc gtagaaaaga tcaaaggatc 13140

ttcttgagat cctttttttc tgcgcgtaat ctgctgcttg caaacaaaaa aaccaccgct 13200

accagcggtg gtttgtttgc cggatcaaga gctaccaact ctttttccga aggtaactgg 13260

cttcagcaga gcgcagatac caaatactgt ccttctagtg tagccgtagt taggccacca 13320

cttcaagaac tctgtagcac cgcctacata cctcgctctg ctaatcctgt taccagtggc 13380

tgctgccagt ggcgataagt cgtgtcttac cgggttggac tcaagacgat agttaccgga 13440

taaggcgcag cggtcgggct gaacgggggg ttcgtgcaca cagcccagct tggagcgaac 13500

gacctacacc gaactgagat acctacagcg tgagcattga gaaagcgcca cgcttcccga 13560

agggagaaag gcggacaggt atccggtaag cggcagggtc ggaacaggag agcgcacgag 13620

ggagcttcca gggggaaacg cctggtatct ttatagtcct gtcgggtttc gccacctctg 13680

acttgagcgt cgatttttgt gatgctcgtc aggggggcgg agcctatgga aaaacgccag 13740

caacgcggcc tttttacggt tcctggcctt ttgctggcct tttgctcaca tgttctttcc 13800

tgcgttatcc cctgattctg tggataaccg tattaccgcc tttgagtgag ctgataccgc 13860

tcgccgcagc cgaacgaccg agcgcagcga gtcagtgagc gaggaagcgg aagagcgccc 13920

aatacgcaaa ccgcctctcc ccgcgcgttg gccgattcat taatgcagct ggcacgacag 13980

gtttcccgac tggaaagcgg gcagtgagcg caacgcaatt aatgtgagtt agctcactca 14040

ttaggcaccc caggctttac actttatgct tccggctcgt atgttgtgtg gaattgtgag 14100

cggataacaa tttcacacag gaaacagcta tgaccatgat tacgaatttc gacctgcagg 14160

catgcaagct tgcatgcctg caggtcgacg ctcgcgcgac ttggtttgcc attctttagc 14220

gcgcgtcgcg tcacacagct tggccacaat gtggtttttg tcaaacgaag attctatgac 14280

gtgtttaaag tttaggtcga gtaaagcgca aatctttttt aaccctagaa agatagtctg 14340

cgtaaaattg acgcatgcat tcttgaaata ttgctctctc tttctaaata gcgcgaatcc 14400

gtcgctgtgc atttaggaca tctcagtcgc cgcttggagc tcccgtgagg cgtgcttgtc 14460

aatgcggtaa gtgtcactga ttttgaacta taacgaccgc gtgagtcaaa atgacgcatg 14520

attatctttt acgtgacttt taagatttaa ctcatacgat aattatattg ttatttcatg 14580

ttctacttac gtgataactt attatatata tattttcttg ttatagatat cgtgactaat 14640

atataataaa atgggtagtt ctttagacga tgagcatatc ctctctgctc ttctgcaaag 14700

cgatgacgag cttgttggtg aggattctga cagtgaaata tcagatcacg taagtgaaga 14760

tgacgtccag agcgatacag aagaagcgtt tatagatgag gtacatgaag tgcagccaac 14820

gtcaagcggt agtgaaatat tagacgaaca aaatgttatt gaacaaccag gttcttcatt 14880

ggcttctaac agaatcttga ccttgccaca gaggactatt agaggtaaga ataaacattg 14940

ttggtcaact tcaaagtcca cgaggcgtag ccgagtctct gcactgaaca ttgtcagatc 15000
ggcccggcgg agtggacacg ctagaccaaa tgtgttctgt gatgacctgc agtaggaaga 15060
cgaataggtg gcctatggca ttattgtacg gaatgataaa cattgcctgc ataaattctt 15120
ttattatata cagccataat gtcagtagca agggagaaaa ggtccaaagt cgcaaaaaat 15180
ttatgagaaa cctttacatg agcctgacgt catcgtttat gcgtaagcgt ttagaagctc 15240
ctactttgaa gagatatttg cgcgataata tctctaatat tttgccaaat gaagtgcctg 15300
gtacatcaga tgacagtact gaagagccag taatgaaaaa acgtacttac tgtacttact 15360
gcccctctaa aataaggcga aaggcaaatg catcgtgcaa aaaatgcaaa aaagttattt 15420
gtcgagagca taatattgat atgtgccaaa gttgtttctg actgactaat aagtataatt 15480
tgtttctatt atgtataagt taagctaatt acttatttta taatacaaca tgactgtttt 15540
taaagtacaa aataagttta tttttgtaaa agagagaatg tttaaaagtt ttgttacttt 15600
atagaagaaa ttttgagttt ttgttttttt ttaataaata aataaacata aataaattgt 15660
ttgttgaatt tattattagt atgtaagtgt aaatataata aaacttaata tctattcaaa 15720
ttaataaata aacctcgata tacagaccga taaaacacat gcgtcaattt tacgcatgat 15780
tatctttaac gtacgtcaca atatgattat ctttctaggg ttaaaatgaa tgtaagcact 15840
ttattaacga aatctttggg aatatttcgc tcatcagcat tttatttgag caggagtccg 15900
agatgcccgg ccgcgccggc catcgagaaa gagagagaga agagaagaga gagaacattc 15960
gagaaagaga gagagaagag aagagagaga acatactccc tatcagtgat agagaagtcc 16020
ctatcagtga tagagatgtc cctatcagtg atagagagtt ccctatcagt gatagagacg 16080
tccctatcag tgatagagaa gtccctatca gtgatagaga gatccctatc agtgatagag 16140
atttccctat cagtgataga gaggtcccta tcagtgatag agacttccct atcagtgata 16200
gagaaatccc tatcagtgat agagacatcc ctatcagtga tagagaactc cctatcagtg 16260
atagagacct ccctatcagt gatagagatc gatccgtcta cctgagcgat atataaacta 16320
atgcctgttg caattgttca gtcagtcacg agtttgttac cactgcgaca agctagcaac 16380
caccatggcg gtaattctaa ttacttacta aatatagtga tatttaaaaa tgctatgaga 16440
tcttgaggcg aacttaaatg agacaaggtg ttagcttgct caaatgacgc accaatcaac 16500
ttaccaagat aatttggagc aatatggaat attttcttgt caaagaacta caatcaacat 16560
gcaactggta taataaagtg agtataaagt agaaaggtgc ggtataatat ttttctctaa 16620
agtattaagg taagttttta aaaataattt aataattttt cagagtgtgc agtattagtg 16680
tctaaaagta ttgcaatctt tgtgttttaa aaatttgtgc agaatatcaa aaaataggac 16740
gacctattgt tagtaatatt atgtatattt acttattttc aaataaataa aattattttc 16800

tagcctttag aaacagcctt atactacaat caacaaacca taaatactcc ctgcaacaat 16860

tacaaagcaa cacaaacaac aatcaacatt ccatgaactc ggacaatttg gaaattgata 16920

tcttctggca catgcggatt acaacaatca actcaaacct attagatgat gtaaataaat 16980

ataaaccttc atattcaata acttaattcg tgatagaaat ttagagttaa ttactgttta 17040

atcgaaattt gaacgtctcg tttgaattca ctttaacgtt tgaaagatga tctttaaata 17100

gtgactggag acttaaattt taatcttttt aattaaataa tctaacaaag ttctttctta 17160

ttctaatctt aatatataat cttgttttaa aatttgaatt aaacttattt cgttgagtaa 17220

caattaaatg atttgaatcg taaaaaatat cattataatt attcaacaaa aagctgaagg 17280

ctgcgatgaa aaattgttga tatataaatc aaatttattt ttataaatca ctaatagtga 17340

cattaaccgt attcacaggt ggccttctac atcccggatc aggccaccct gctgcgcgag 17400

gccgagcagc gcgagcagca gatcctgcgc ctgcgcgaga gccagtggcg cttcctggcc 17460

accgtggtgc tggagaccct gcgccagtac accagctgcc acccgcgcac cggccgccgc 17520

agcggccgtt accgccgtcc gagccagtaa caccggtgat cataatcagc cataccacat 17580

ttgtagaggt tttacttgct ttaaaaaacc tcccacacct ccccctgaac ctgaaacata 17640

aaatgaatgc aattgttgtt gttaacttgt ttattgcagc ttataatggt tacaaataaa 17700

gcaatagcat cacaaatttc acaaataaag catttttttc actgcattct agttgtggtt 17760

tgtccaaact catcaatgta tcttaacgcg agtttaggcg cg 17802

<210> 56
<211> 15134
<212> DNA
<213> artificial

<220>
<223> Sequence of pLA3242-Crtra intron-reaperKR construct.

<400> 56
gggcggccgt ttttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600

agttacatga cgagccctaa tgagccgtcg gtggtctata aactgtgcct tacaaatact 660
tgcaactctt ctcgttttga agtcagcaga gttattgcta attgctaatt gctaattgct 720
tttaactgat ttcttcgaaa ttggtgctat gtttatggcg ctattaacaa gtatgaatgt 780
caggtttaac caggggatgc ttaattgtgt tctcaacttc aaaggcagaa atgtttactc 840
ttgaccatgg gtttaggtat aatgttatca agctcctcga gttaacgtta cgttaacgtt 900
aacgttcgag gtcgactcta gaactaccca ccgtactcgt caattccaag ggcatcggta 960
aacatctgct caaactcgaa gtcggccata tccagagcgc cgtagggggc ggagtcgtgg 1020
ggggtaaatc ccggacccgg ggaatccccg tcccccaaca tgtccagatc gaaatcgtct 1080
agcgcgtcgg catgcgccat cgccacgtcc tcgccgtcta agtggagctc gtcccccagg 1140
ctgacatcgg tcggggggc cgtcgacagt ctgcgcgtgt gtcccgcggg gagaaaggac 1200
aggcgcggag ccgccagccc cgcctcttcg gggcgtcgt cgtccgggag atcgagcagg 1260
ccctcgatgg tagacccgta attgtttttc gtacgcgcgc ggctgtacgc ggggcccgag 1320
cccgactcgc atttcagttg cttttccaat ccgcagataa tcagctccaa gccgaacagg 1380
aatgccggct cggctccttg atgatcgaac agctcgattg cctgacgcag cagtgggggc 1440
atcgaatcgg ttgttggggt ctcgcgctcc tcttttgcga cttgatgctc ttggtcctcc 1500
agcacgcagc ccagggtaaa gtgaccgacg gcgctcagag cgtagagagc attttccagg 1560
ctgaagcctt gctggcacag gaacgcgagc tggttctcca gtgtctcgta ttgcttttcg 1620
gtcgggcgcg tgccgagatg gactttggca ccgtctcggt gggacagcag agcgcagcgg 1680
aacgacttgg cgttattgcg gaggaagtcc tgccaggact cgccttccaa cgggcaaaaa 1740
tgcgtgtggt ggcggtcgag catctcgatg gccagggcat ccagcagcgc ccgcttattc 1800
ttcacgtgcc agtagagggt gggctgctcc acgcccagct tctgcgccaa cttgcgggtc 1860
gtcagtccct caatgccaac ttcgttcaac agctccaacg cggagttgat gactttggac 1920
ttatccaggc ggctgaccta tagataccat agatgtatgg attagtatca tatacataca 1980
aaggctattt ttgggacata ttaatattaa caatttccgt gatagttttc accatttttg 2040
ttgaatgtta cgttgaaaat ttaaatttgt tttaaattaa ttttaccagt catgtgttct 2100
taaaagtttt tatgattgaa acggcataaa gtggttcaaa aatttatcaa gaaaggcttt 2160
cctttttttaa atcttatctt tttctcttaa aaatcactag tcaattcatt attaatttgt 2220
taacttgaat ttggaatgtc tatttacttt cagataaatt aaagcaagaa acttaatatt 2280
cgaaaaaaat tgattctaaa tggaatttca cttgatcttc atgtatgcat atcaattttt 2340
atttacattg tataataagt ttcgagttga ttgttgtaat ccacaggtgt cccagagaat 2400
taaattccaa attacccaag tttattgaat gttgattgta gtttcagttg ctttgttgct 2460

gcaacaatgg cttgttgatt gtagatattt tccctttcct tggtttactt attacataga 2520
ctgaaaaaga ggtttacttt tttgatactt atgaaaaatt tctattagtg attactaacc 2580
aatcgctata tgtttactag aaaacaaata aactctttac attaacattc aataatgttt 2640
gctctgtaac cgacaattga aggcgttaca gcaacagtaa tataactagc ttcttaaccc 2700
tcatctatta accccatcgt ttaaaacact atgttaaatg gtctaacaaa tctagatact 2760
aatagatgtc ttattactta gcagccacag ctgcaacatc caagacaatt tttgaaactt 2820
cttattgagc tcttggcagc agaaatgttg gtatttttca cagctttctg aaagaccggc 2880
accttcctcc ggttcccgtt tctgaattca agaggatttc cgacccccaa ttaatcccga 2940
aacaaataag gtatattcaa aatgatggaa aagtcatggc tgctgacctt atttttattc 3000
ctattgatag aatattattc cccttttaaa tacactgtac taagaggtcc ggctataatt 3060
ttactcactt gtcgattatc ccatagaatg ttgattgtag ttggttgctt ttccaggtga 3120
gagttgatca agtcacaaaa gttagcgtgt gttgattgta gatttgaagg taaaataatt 3180
tttgcaccca ttcatcgggt aaaacgttct ccatagaata catttccatc gataattgat 3240
aacttatgaa tttcaaagaa aaaaatatgc ttttaaaatt accatggtgg ctagcgcaga 3300
ttgtttagct tgttcagctg cgcttgttta tttgcttagc tttcgcttag cgacgtgttc 3360
actttgcttg tttgaattga attgtcgctc cgtagacgaa gcgcctctat ttatactccg 3420
gcgctcgttt tcgagtttac cactccctat cagtgataga gaaaagtgaa agtcgagttt 3480
accactccct atcagtgata gagaaaagtg aaagtcgagt ttaccactcc ctatcagtga 3540
tagagaaaag tgaaagtcga gtttaccact ccctatcagt gatagagaaa agtgaaagtc 3600
gagtttacca ctccctatca gtgatagaga aaagtgaaag tcgagtttac cactccctat 3660
cagtgataga gaaaagtgaa agtcgagttt accactccct atcagtgata gagaaaagtg 3720
aaagtcgaaa cctggcgcgc ctaaactcgc gttaagatac attgatgagt ttggacaaac 3780
cacaactaga atgcagtgaa aaaaatgctt tatttgtgaa atttgtgatg ctattgcttt 3840
atttgtaacc attataagct gcaataaaca agttaacaac aacaattgca ttcattttat 3900
gtttcaggtt caggggggagg tgtgggaggt tttttaaagc aagtaaaacc tctacaaatg 3960
tggtatggct gattatgatc accggtgtta ctggctcgga cggcggtaac ggccgctgcg 4020
gcggccggtg cgcgggtggc agctggtgta ctggcgcagg gtctccagca ccacggtggc 4080
caggaagcgc cactggctct cgcgcaggcg caggatctgc tgctcgcgct gctcggcctc 4140
gcgcagcagg gtggcctgat ccgggatgta gaaggccacc taaagatacc atggatgtat 4200
gaattagtat catatacata taaatgcttt tttttttggc atattaatgt taaaaatatc 4260
aacaatttcc gtgatagttt ttaccatttt tgttgaatgt ttactttgaa aacttaaata 4320
ttttttaact aattttacca gtcatgtgtt attaaaagta tttatgaata aaactgcaag 4380

taaagcgttt caaaaattta tcaagtaaaa ctttactttt tttaaatctt aactgtcaat 4440

tcattattaa tttattaatt taaatttgca atgtctattt actttaagac aaattaaagc 4500

aagaaactaa atattcgaat caattctttt ttaaatgaaa ttttacttca tcatcatgta 4560

tgtgtgtatc aatttttatt tacattgtat aataagtttc gagttgattg ttgtaatccg 4620

caggtgtccc gaagtattaa attccgaatt cccaagttta ttgaatgttg attgtagttt 4680

cagttgtttt gttattgcaa caatggcttg ttgattggag atattttcct tttccttggt 4740

ttacttacta catagactga aaaagatgtt tgactttttt gatactattg taaaatttct 4800

attagtgatt actaaccaat cgctataagt ttaatagaaa acaaataaac tctttgcatc 4860

cagatatacc tagcttctta acccttatct attaactcca ttgcttgtaa caaatctaga 4920

tattaataga tgtctaatta cttagcaaaa cttctttttg attaagcagc cacagctgtc 4980

gattttggtc atatttaaag gaaataaatg cgtttaaaat aataattaat ataagttttg 5040

aaactttttta ctaacacttg gcagcaggaa gtaggtgttt ttcacagctt tctgaaccac 5100

cggcaccttc cccggtctcc gttgtcggag ttcagcagga tttccggccc ccaattaacc 5160

ccgaaacaaa acatgtctta ttaataaggt gtattcaaaa tagtgggaat gtcatgactg 5220

ctgaccttat ttttattcct attgtaagtg ttccggctat aattttactc acttgtccat 5280

tatcccatag aatgttatgt tgattgtagt tgtttgcttt tccaggtgag agttgatcaa 5340

gtcgcaaaag ttagcgtgtg ttgattgtag atttgaaggt aaaataattt tgtacacatt 5400

catcaggcaa aacgttctcc atcgaataaa cttccatcga taattgatag cttatgaatt 5460

tcaaaaaaaa atatgctttt aaaattaccg ccatggtggt tgctagcttg tcgcagtggt 5520

aacaaactcg tgactgactg aacaattgca acaggcatta gtttatatat cgctcaggta 5580

gacggatcga tctctatcac tgatagggag gtctctatca ctgataggga gttctctatc 5640

actgataggg atgtctctat cactgatagg gatttctcta tcactgatag ggaagtctct 5700

atcactgata gggacctctc tatcactgat agggaaatct ctatcactga tagggatctc 5760

tctatcactg atagggactt ctctatcact gatagggacg tctctatcac tgatagggaa 5820

ctctctatca ctgataggga catctctatc actgataggg acttctctat cactgatagg 5880

gagtatgttc tctctcttct cttctctctc tctttctcga atgttctctc tcttctcttc 5940

tctctctctt tctcgatggc cggcctggct taattaactc gcgttaagat acattgatga 6000

gtttggacaa accacaacta gaatgcagtg aaaaaaatgc tttatttgtg aaatttgtga 6060

tgctattgct ttatttgtaa ccattataag ctgcaataaa caagttaaca acaacaattg 6120

cattcatttt atgtttcagg ttcaggggga ggtgtgggag gttttttaaa gcaagtaaaa 6180

cctctacaaa tgtggtatgg ctgattatga tcagttatct agatccggtg gatcttacgg 6240

gtcctccacc ttccgctttt tcttgggtcg agatctgagt ccggaatcct cgtcgctacc 6300

gatggcgctg gtgatgcggg gcacgctgtg ggcgtaggtc acctcgcgct ggcacacgtg 6360

gtcgcgcttg tcgctggtgt ccctcatctg cttggtgatg atggtcacga agtgggggcc 6420

ggggatcttg atggcgcggc tgccgttgaa ggtcatcttg ctgtcgaagt ggcccatcat 6480

caggccgccg tcggcggtgg tgaagccgat gaaggccagc tggcgcacgg cgttggggcc 6540

gtgggggaac atgtgggtct cgttgggcag gatgtccacc agctggtcgc gcatgatggg 6600

gccgtcgggc tggaagccgt cgcagttcac ggtgatgcgg ctgaccacgc aggtgccgtc 6660

cagctcgtag gtgtggtggc tggtcatggt gccgtcgttc tcgaagcgca cggtgcggtc 6720

gatgctcagg ccctcgggga agcactcctg ggcgaagtgg ctgatgccgt tggggtagcg 6780

ggcgaagaag ggctcgccgt actggatcag gtggcagatg ggcttccagc tcatgggcag 6840

cttgccggtc tcgcacacgg cgtgcacgtt gaagtcgccg tgggggaact tgctgctgcc 6900

gtcggccacg atggtgaact tctggccgtt cacctcgccg tcgatgaaga ttttgaaggt 6960

catgtcgctc tggaacaggg cggggccgcc ctctgaacca tcctcgtcca tggtggcgac 7020

cggtttgcgc ttcttcttgg gtggggtggg atctcccatg gtggcctgaa tctcaacttg 7080

cacctgaagg tagtgcagca aggatgagca aaagggaaga acccagaaaa gaacgggaaa 7140

acttacccca attagaattg cttgtcgccg ccagtgtcaa cttgcaactg aaacaatatc 7200

caacatgaac gtcaatttat actgccctaa tggcgaacac gataacaata tttcttttat 7260

tatgccctct aaaaccaacg cggttatcgt ttatttattc aaattagata tagaacatcc 7320

gccgacatac aatgttaatg caaaaacgcg tttggtgagc ggatacgaaa acagtcggcc 7380

gataaacatt aatctgaggt cgataacacc gtccttgaac ggaacacgag gagcgtacgt 7440

gatcagctgc attcgcgcgc cgcgccttta tcgagattta tttgcataca acaagtacac 7500

tgcgccgttg ggatttgtgg taacgcgcac acatgcagag ctgcaagtgt ggcacatttt 7560

gtctgtgcgc aaaacctttg aagccaaaag tacgaggtcc gttacgggca tgctagcgca 7620

cacggacaat ggacccgaca aattctacgc caaggattta atgataatgt cgggcaacgt 7680

atccgttcat tttatcaata acctacaaaa atgtcgcgcg catcacaaag acatcgatat 7740

atttaaacat ttatgtcccg aactgcaaat cgataatagt gttgtgcaac ctcgagcgtc 7800

cgtttgattt aacgtatagc ttgcaaatga attatttaat tatcaatcat gttttacgcg 7860

tagaattcta cccgtaaagc gagtttagtt atgagccatg tgcaaaacat gacatcagct 7920

tttattttta taacaaatga catcatttct tgattgtgtt ttacacgtag aattctactc 7980

gtaaagcgag ttcagttttg aaaaacaaat gacatcatct tttgattgt gctttacaag 8040

tagaattcta cccgtaaatc aagttcggtt ttgaaaaaca aatgagtcat attgtatgat 8100

atcatattgc aaaacaaatg actcatcaat cgatcgtgcg ttacacgtag aattctactc 8160

gtaaagcgag tttatgagcc gtgtgcaaaa catgacatca tctcgatttg aaaaacaaat 8220
gacatcatcc actgatcgtg cattacaagt agaattctac tcgtaaagcc agttcggtta 8280
tgagccgtgt acaaaacatg acatcagatt atgactcata cttgattgtg ttttacgcgt 8340
agaattctac tcgtaaagcc agttcaattt taaaaacaaa tgacatcatc caaattaata 8400
aatgacaagc aatgggtacc atgcggccgc accgaaatcg taattcacgg catcattaca 8460
aaatattttg acgttttgga cctcgtccct aatgacacca taacggtggc cttgaagtat 8520
atttaaccct agaaagatag tctgcgtaaa attgacgcat gcattcttga aatattgctc 8580
tctctttcta aatagcgcga atccgtcgct gtgcatttag gacatctcag tcgccgcttg 8640
gagctcccgt gaggcgtgct tgtcaatgcg gtaagtgtca ctgattttga actataacga 8700
ccgcgtgagt caaaatgacg catgattatc ttttacgtga cttttaagat ttaactcata 8760
cgataattat attgttattt catgttctac ttacgtgata acttattata tatatatttt 8820
cttgttatag atatcgtgac taatatataa taaaatgggt agttctttag acgatgagca 8880
tatcctctct gctcttctgc aaagcgatga cgagcttgtt ggtgaggatt ctgacagtga 8940
aatatcagat cacgtaagtg aagatgacgt ccaggaaatc tggccggccg caaccattgt 9000
gggaaccgtg cgatcaaaca aacgcgagat accggaagta ctgaaaaaca gtcgctccag 9060
gccagtggga acatcgatgt tttgttttga cggacccctt actctcgtct catataaacc 9120
gaagccagct aagatggtat acttattatc atcttgtgat gaggatgctt ctatcaacga 9180
aagtaccggt aaaccgcaaa tggttatgta ttataatcaa actaaaggcg gagtggacac 9240
gctagaccaa atgtgttctg tgatgacctg cagtaggaag acgaataggt ggcctatggc 9300
attattgtac ggaatgataa acattgcctg cataaattct tttattatat acagccataa 9360
tgtcagtagc aagggagaaa aggtccaaag tcgcaaaaaa tttatgagaa acctttacat 9420
gagcctgacg tcatcgttta tgcgtaagcg tttagaagct cctactttga agagatattt 9480
gcgcgataat atctctaata ttttgccaaa tgaagtgcct ggtacatcag atgacagtac 9540
tgaagagcca gtaatgaaaa aacgtactta ctgtacttac tgcccctcta aaataaggcg 9600
aaaggcaaat gcatcgtgca aaaaatgcaa aaaagttatt tgtcgagagc ataatattga 9660
tatgtgccaa agttgtttct gactgactaa taagtataat ttgtttctat tatgtataag 9720
ttaagctaat tacttatttt ataatacaac atgactgttt ttaaagtaca aaataagttt 9780
atttttgtaa aagagagaat gtttaaaagt tttgttactt tatagaagaa attttgagtt 9840
tttgtttttt tttaataaat aaataaacat aaataaattg tttgttgaat ttattattag 9900
tatgtaagtg taaatataat aaaacttaat atctattcaa attaataaat aaacctcgat 9960
atacagaccg ataaaacaca tgcgtcaatt ttacgcatga ttatctttaa cgtacgtcac 10020

aatatgatta tctttctagg gttaaataat agtttctaat ttttttatta ttcagcctgc 10080
tgtcgtgaat accgtatatc tcaacgctgt ctgtgagatt gtcgtattct agccttttta 10140
gtttttcgct catcgacttg atattgtccg acacattttc gtcgatttgc gttttgatca 10200
aagacttgag cagagacacg ttaatcaact gttcaaattg atccatatta acgatatcaa 10260
cccgatgcgt atatggtgcg taaaatatat tttttaaccc tcttatactt tgcactctgc 10320
gttaatacgc gttcgtgtac agacgtaatc atgttttctt ttttggataa aactcctact 10380
gagtttgacc tcatattaga ccctcacaag ttgcaaaacg tggcattttt taccaatgaa 10440
gaatttaaag ttattttaaa aaatttcatc acagatttaa agaagaacca aaaattaaat 10500
tatttcaaca gtttaatcga ccagttaatc aacgtgtaca cagacgcgtc ggcaaaaaac 10560
acgcagcccg acgtgttggc taaaattatt aaatcaactt gtgttatagt cacggatttg 10620
ccgtccaacg tgttcctcaa aaagttgaag accaacaagt ttacggacac tattaattat 10680
ttgattttgc ccccacttcat tttgtgggat cacaattttg ttatatttta aacaaagctt 10740
ggcactggcc gtcgttttac aacgtcgtga ctgggaaaac cctggcgtta cccaacttaa 10800
tcgccttgca gcacatcccc ctttcgccag ctggcgtaat agcgaagagg cccgcaccga 10860
tcgcccttcc caacagttgc gcagcctgaa tggcgaatgg cgcctgatgc ggtattttct 10920
ccttacgcat ctgtgcggta tttcacaccg catatggtgc actctcagta caatctgctc 10980
tgatgccgca tagttaagcc agccccgaca cccgccaaca cccgctgacg cgccctgacg 11040
ggcttgtctg ctcccggcat ccgcttacag acaagctgtg accgtctccg ggagctgcat 11100
gtgtcagagg ttttcaccgt catcaccgaa acgcgcgaga cgaaagggcc tcgtgatacg 11160
cctattttta taggttaatg tcatgataat aatggtttct tagacgtcag gtggcacttt 11220
tcggggaaat gtgcgcggaa cccctatttg tttatttttc taaatacatt caaatatgta 11280
tccgctcatg agacaataac cctgataaat gcttcaataa tattgaaaaa ggaagagtat 11340
gagtattcaa catttccgtg tcgcccttat tccctttttt gcggcatttt gccttcctgt 11400
ttttgctcac ccagaaacgc tggtgaaagt aaaagatgct gaagatcagt tgggtgcacg 11460
agtgggttac atcgaactgg atctcaacag cggtaagatc cttgagagtt ttcgccccga 11520
agaacgtttt ccaatgatga gcacttttaa agttctgcta tgtggcgcgg tattatcccg 11580
tattgacgcc gggcaagagc aactcggtcg ccgcatacac tattctcaga atgacttggt 11640
tgagtactca ccagtcacag aaaagcatct tacggatggc atgacagtaa gagaattatg 11700
cagtgctgcc ataaccatga gtgataacac tgcggccaac ttacttctga caacgatcgg 11760
aggaccgaag gagctaaccg cttttttgca caacatgggg gatcatgtaa ctcgccttga 11820
tcgttgggaa ccggagctga atgaagccat accaaacgac gagcgtgaca ccacgatgcc 11880
tgtagcaatg gcaacaacgt tgcgcaaact attaactggc gaactactta ctctagcttc 11940

ccggcaacaa ttaatagact ggatggaggc ggataaagtt gcaggaccac ttctgcgctc 12000
ggcccttccg gctggctggt ttattgctga taaatctgga gccggtgagc gtgggtctcg 12060
cggtatcatt gcagcactgg ggccagatgg taagccctcc cgtatcgtag ttatctacac 12120
gacggggagt caggcaacta tggatgaacg aaatagacag atcgctgaga taggtgcctc 12180
actgattaag cattggtaac tgtcagacca agtttactca tatatacttt agattgattt 12240
aaaacttcat ttttaattta aaaggatcta ggtgaagatc ctttttgata atctcatgac 12300
caaaatccct taacgtgagt tttcgttcca ctgagcgtca gaccccgtag aaaagatcaa 12360
aggatcttct tgagatcctt tttttctgcg cgtaatctgc tgcttgcaaa caaaaaaacc 12420
accgctacca gcggtggttt gtttgccgga tcaagagcta ccaactcttt ttccgaaggt 12480
aactggcttc agcagagcgc agataccaaa tactgtcctt ctagtgtagc cgtagttagg 12540
ccaccacttc aagaactctg tagcaccgcc tacatacctc gctctgctaa tcctgttacc 12600
agtggctgct gccagtggcg ataagtcgtg tcttaccggg ttggactcaa gacgatagtt 12660
accggataag gcgcagcggt cgggctgaac ggggggttcg tgcacacagc ccagcttgga 12720
gcgaacgacc tacaccgaac tgagatacct acagcgtgag cattgagaaa gcgccacgct 12780
tcccgaaggg agaaaggcgg acaggtatcc ggtaagcggc agggtcggaa caggagagcg 12840
cacgagggag cttccagggg gaaacgcctg gtatctttat agtcctgtcg ggtttcgcca 12900
cctctgactt gagcgtcgat ttttgtgatg ctcgtcaggg gggcggagcc tatggaaaaa 12960
cgccagcaac gcggcctttt tacggttcct ggccttttgc tggccttttg ctcacatgtt 13020
ctttcctgcg ttatcccctg attctgtgga taaccgtatt accgcctttg agtgagctga 13080
taccgctcgc cgcagccgaa cgaccgagcg cagcgagtca gtgagcgagg aagcggaaga 13140
gcgcccaata cgcaaaccgc ctctccccgc gcgttggccg attcattaat gcagctggca 13200
cgacaggttt cccgactgga aagcgggcag tgagcgcaac gcaattaatg tgagttagct 13260
cactcattag gcaccccagg ctttacactt tatgcttccg gctcgtatgt tgtgtggaat 13320
tgtgagcgga taacaatttc acacaggaaa cagctatgac catgattacg aatttcgacc 13380
tgcaggcatg caagcttgca tgcctgcagg tcgacgctcg cgcgacttgg tttgccattc 13440
tttagcgcgc gtcgcgtcac acagcttggc cacaatgtgg tttttgtcaa acgaagattc 13500
tatgacgtgt ttaaagttta ggtcgagtaa agcgcaaatc ttttttaacc ctagaaagat 13560
agtctgcgta aaattgacgc atgcattctt gaaatattgc tctctctttc taaatagcgc 13620
gaatccgtcg ctgtgcattt aggacatctc agtcgccgct tggagctccc gtgaggcgtg 13680
cttgtcaatg cggtaagtgt cactgatttt gaactataac gaccgcgtga gtcaaaatga 13740
cgcatgatta tcttttacgt gacttttaag atttaactca tacgataatt atattgttat 13800

ttcatgttct acttacgtga taacttatta tatatatatt ttcttgttat agatatcgtg 13860

actaatatat aataaaatgg gtagttcttt agacgatgag catatcctct ctgctcttct 13920

gcaaagcgat gacgagcttg ttggtgagga ttctgacagt gaaatatcag atcacgtaag 13980

tgaagatgac gtccagagcg atacagaaga agcgtttata gatgaggtac atgaagtgca 14040

gccaacgtca agcggtagtg aaatattaga cgaacaaaat gttattgaac aaccaggttc 14100

ttcattggct tctaacagaa tcttgacctt gccacagagg actattagag gtaagaataa 14160

acattgttgg tcaacttcaa agtccacgag gcgtagccga gtctctgcac tgaacattgt 14220

cagatcggcc cggcggagtg gacacgctag accaaatgtg ttctgtgatg acctgcagta 14280

ggaagacgaa taggtggcct atggcattat tgtacggaat gataaacatt gcctgcataa 14340

attcttttat tatatacagc cataatgtca gtagcaaggg agaaaaggtc caaagtcgca 14400

aaaaatttat gagaaacctt tacatgagcc tgacgtcatc gtttatgcgt aagcgtttag 14460

aagctcctac tttgaagaga tatttgcgcg ataatatctc taatattttg ccaaatgaag 14520

tgcctggtac atcagatgac agtactgaag agccagtaat gaaaaaacgt acttactgta 14580

cttactgccc ctctaaaata aggcgaaagg caaatgcatc gtgcaaaaaa tgcaaaaaag 14640

ttatttgtcg agagcataat attgatatgt gccaaagttg tttctgactg actaataagt 14700

ataatttgtt tctattatgt ataagttaag ctaattactt attttataat acaacatgac 14760

tgtttttaaa gtacaaaata agtttatttt tgtaaaagag agaatgttta aaagttttgt 14820

tactttatag aagaaatttt gagtttttgt ttttttttaa taaataaata aacataaata 14880

aattgtttgt tgaatttatt attagtatgt aagtgtaaat ataataaaac ttaatatcta 14940

ttcaaattaa taaataaacc tcgatataca gaccgataaa acacatgcgt caattttacg 15000

catgattatc tttaacgtac gtcacaatat gattatcttt ctagggttaa aatgaatgta 15060

agcactttat taacgaaatc tttgggaata tttcgctcat cagcatttta tttgagcagg 15120

agtccgagat gccc 15134

<210> 57
<211> 1403
<212> DNA
<213> artificial

<220>
<223> SEQ ID NO. 57 Partial sequence of a male transcript generated in Drosophila melanogaster from LA3077 transformants that differs to the sequence generated in Medfly LA3077 lines. T

<400> 57
ggccagatct gttgttatta aacgtagatt tggtaatttt aaaagcatat ttttttcttt 60

gaaattcata agttatcaat tatcgatgga aatgtattct atggagaacg ttttacccga 120

tgaatgggtg caaaaattat tttaccttca aatctacaat caacacacgc taacttttgt 180

gacttgatca actctcacct ggaaaagcaa ccaactacaa tcaacattct atgggataat 240

cgacaagtga gtaaaattat agccggacct cttagtacag tgtatttaaa aggggaataa 300

tattctatca ataggaataa aaataaggtc agcagccatg acttttccat cattttgaat 360

ataccttatt tgtttcggga ttaattgggg gtcggaaatc ctcttgaatt cagaaacggg 420

aaccggagga aggtgccggt ctttcagaaa gctgtgaaaa ataccaacat ttctgctgcc 480

aagagctcaa taagaagttt caaaaattgt cttggatgtt gcagctgtgg ctgctaagta 540

ataagacatc tattagtatc tagatttgtt agaccattta acatagtgtt ttaaacgatg 600

gggttaatag atgagggtta agaagctagt tatattactg ttgctgtaac gccttcaatt 660

gtcggttaca gagcaaacat tattgaatgt taatgtaaag agtttatttg ttttctagta 720

aacatatagc gattggttag taatcactaa tagaaatttt tcataagtat caaaaaagta 780

aacctctttt tcagtctatg taataagtaa accaaggaaa gggaaaatat ctacaatcaa 840

caagccattg ttgcagcaac aaagcaactg aaactacaat caacattcaa taaacttggg 900

taatttggaa tttaattctc tgggacacct gtggattaca acaatcaact cgaaacttat 960

tatacaatgt aaataaaaat tgatatgcat acatgaagat caagtgaaat tccatttaga 1020

atcaattttt ttcgaatatt aagtttcttg ctttaattta tctgaaagta aatagacatt 1080

ccaaattcaa gttaacaaat taataatgaa ttgactagtg atttttaaga gaaaaagata 1140

agatttaaaa aaggaaagcc tttcttgata aatttttgaa ccactttatg ccgtttcaat 1200

cataaaaact tttaagaaca catgactggt aaaattaatt taaaacaaat ttaaattttc 1260

aacgtaacat tcaacaaaaa tggtgaaaac tatcacggaa attgttaata ttaatatgtc 1320

ccaaaaatag cctttgtatg tatatgatac taatccatac atctatggta tctataggtg 1380

aaggctcaaa gcctctggct agc 1403

<210> 58
<211> 972
<212> DNA
<213> Bactrocera zonata

<400> 58
cggtaattct aattacttac taaatatagt gatatttaaa aatgctatga gatcttgagg 60

cgaacttaaa tgagacaagg tgttagcttg ctcaaatgac gcaccaatca acttaccaag 120

ataatttgga gcaatatgga atattttctt gtcaaagaac tacaatcaac atgcaactgg 180

tataataaag tgagtataaa gtagaaaggt gcggtataat atttttctct aaagtattaa 240

ggtaagtttt taaaaataat ttaataattt ttcagagtgt gcagtattag tgtctaaaag 300

tattgcaatc tttgtgtttt aaaaatttgt gcagaatatc aaaaaatagg acgacctatt 360

gttagtaata ttatgtatat ttacttattt tcaaataaat aaaattattt tctagccttt 420

agaaacagcc ttatactaca atcaacaaac cataaatact ccctgcaaca attacaaagc 480

aacacaaaca acaatcaaca ttccatgaac tcggacaatt tggaaattga tatcttctgg 540

cacatgcgga ttacaacaat caactcaaac ctattagatg atgtaaataa atataaacct 600

tcatattcaa taacttaatt cgtgatagaa atttagagtt aattactgtt taatcgaaat 660

ttgaacgtct cgtttgaatt cactttaacg tttgaaagat gatctttaaa tagtgactgg 720

agacttaaat tttaatcttt ttaattaaat aatctaacaa agttctttct tattctaatc 780

ttaatatata atcttgtttt aaaatttgaa ttaaacttat ttcgttgagt aacaattaaa 840

tgatttgaat cgtaaaaaat atcattataa ttattcaaca aaaagctgaa ggctgcgatg 900

aaaaattgtt gatatataaa tcaaatttat ttttataaat cactaatagt gacattaacc 960

gtattcacag gt 972

<210> 59
<211> 1312
<212> DNA
<213> Ceratitis rosa

<400> 59
tggtaatttt aaaagcatat tttttttga aattcataag ctatcaatta tcgatggaag 60

tttattcgat ggagaacgtt ttgcctgatg aatgtgtaca aaattatttt accttcaaat 120

ctacaatcaa cacacgctaa cttttgcgac ttgatcaact ctcacctgga aaagcaaaca 180

actacaatca acataacatt ctatgggata atggacaagt gagtaaaatt atagccggaa 240

cacttacaat aggaataaaa ataaggtcag cagtcatgac attcccacta ttttgaatac 300

accttattaa taagacatgt tttgtttcgg ggttaattgg gggccggaaa tcctgctgaa 360

ctccgacaac ggagaccggg gaaggtgccg gtggttcaga aagctgtgaa aaacacctac 420

ttcctgctgc caagtgttag taaaaagttt caaaacttat attaattatt attttaaacg 480

catttatttc ctttaaatat gaccaaaatc gacagctgtg gctgcttaat caaaaagaag 540

ttttgctaag taattagaca tctattaata tctagatttg ttacaagcaa tggagttaat 600

agataagggt taagaagcta ggtatatctg gatgcaaaga gtttatttgt tttctattaa 660

acttatagcg attggttagt aatcactaat agaaatttta caatagtatc aaaaaagtca 720

aacatctttt tcagtctatg tagtaagtaa accaaggaaa aggaaaatat ctccaatcaa 780

caagccattg ttgcaataac aaaacaactg aaactacaat caacattcaa taaacttggg 840

aattcggaat ttaatacttc gggacacctg cggattacaa caatcaactc gaaacttatt 900

atacaatgta aataaaaatt gatacacaca tacatgatga tgaagtaaaa tttcatttaa 960

aaaagaattg attcgaatat ttagtttctt gctttaattt gtcttaaagt aaatagacat 1020

tgcaaattta aattaataaa ttaataatga attgacagtt aagatttaaa aaaagtaaag 1080

ttttacttga taaatttttg aaacgcttta cttgcagttt tattcataaa tacttttaat 1140

aacacatgac tggtaaaatt agttaaaaaa tatttaagtt ttcaaagtaa acattcaaca 1200

aaaatggtaa aaactatcac ggaaattgtt gatattttta acattaatat gccaaaaaaa 1260

aaagcattta tatgtatatg atactaattc atacatccat ggtatcttta gg 1312

<210> 60
<211> 21
<212> DNA
<213> artificial

<220>
<223> spl-agdsx-e3 primer

<400> 60
cgagcccaat ggctgttgga g 21

<210> 61
<211> 22
<212> DNA
<213> artificial

<220>
<223> spl-agdsx-m primer

<400> 61
gtcaaggttc agggcccgat cg 22

<210> 62
<211> 21
<212> DNA
<213> artificial

<220>
<223> primer spl-agdsx-e3

<400> 62
cgagcccaat ggctgttgga g 21

<210> 63
<211> 22
<212> DNA
<213> artificial

<220>
<223> spl-agdsx-m primer

<400> 63
gtcaaggttc agggcccgat cg 22

<210> 64
<211> 20
<212> DNA
<213> artificial

<220>
<223> aedesxF1 primer

<400> 64
tcaatggctc ctggagaagc 20

<210> 65
<211> 25
<212> DNA
<213> artificial

<220>
<223> aedesxR5 primer

<400> 65
accattcttg cagaagtctt gggac 25

<210> 66
<211> 19
<212> DNA
<213> artificial

<220>
<223> aedesxR2 primer

<400> 66
aacattctcc gcgcacagg 19

<210> 67
<211> 23
<212> DNA
<213> artificial

<220>
<223> Agexon1 primer

<400> 67
gacgctcgct ctggtacagt tcg 23

<210> 68
<211> 20
<212> DNA
<213> artificial

<220>
<223> Tra (tTAV) seq+ primer

<400> 68
cctgccagga ctcgccttcc 20

<210> 69
<211> 23
<212> DNA
<213> artificial

<220>
<223> Agexon1 primer

<400> 69
gacgctcgct ctggtacagt tcg 23

<210> 70
<211> 26
<212> DNA
<213> artificial

<220>
<223> Exon 3 primer

<400> 70
gttgtcgctt tgactggcaa tgtcgc 26

<210> 71
<211> 632
<212> DNA
<213> Pectinophora gossypiella

<400> 71
gaactgccac aaactgctgg aaaagttcca ctactcctgg gaaatgatgc ccctggtgct 60

ggtcattcta aactacgccg gctccgacct cgacgaggct tctagaaaaa ttgatgaagg 120

gaagatgatc atcaacgagt acgcgaggga gcacaatctg aacatcttcg atggccacga 180

gctgaggaac tcgactcgcc agaaaatgct gagcgaaatt aataatataa gtggtgtact 240

atcgtcgtcc atgaagttat tttgcgaatg atactttgtt ttgtatgtgc tgtgtgttgt 300

gtggactttt gctgtgcgtt gctgtttgcg atggaaggac tattgtgtcg tcgccacgct 360

ggactattcg cacattgggt ggtccaccag tggcggatgt acgagcggtc gctgtgctcg 420

ctcctggagc tgcaagcgcg caaagggacg tactcggtgt gctgctcacc ccgctacgtc 480

atcgcgcccg agtacgcgtc acacctgttg cctctgccgc ttaccacgca gagatcatcc 540

ccgccgcccg cgcacttgta gcgatgcgaa cctgcgccgc gggaagcggc gcaagaaccc 600

gccgatgccc cggcgtcgtc gtcgggtgcc ac 632

<210> 72
<211> 222
<212> DNA
<213> Drosophila melanogaster

<400> 72
atgcagatct ttgtgaagac tttgaccgga aagaccatca ccctcgaggt agagccatcg 60

gacaccattg agaatgtaaa ggccaagatt caggataagg agggaatccc cccagatcag 120

cagcgtctga tcttcgctgg caagcaactg gaagacggac gcaccctgtc cgattacaac 180

atccagaagg agtccaccct tcacttggtc cttcgtctcc gt 222

<210> 73
<211> 74
<212> PRT

<213> Drosophila melanogaster

<400> 73

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg
65                  70

<210> 74
<211> 34
<212> DNA
<213> artificial

<220>
<223> primer

<400> 74
caagcaaagt gaacacgtcg ctaagcgaaa gcta 34

<210> 75
<211> 22
<212> DNA
<213> artificial

<220>
<223> primer

<400> 75
gcgggtggca gctggtgtac tg 22

<210> 76
<211> 34
<212> DNA
<213> artificial

<220>
<223> primer

<400> 76
caagcaaagt gaacacgtcg ctaagcgaaa gcta 34

<210> 77
<211> 24
<212> DNA

<213> artificial

<220>
<223> primer

<400> 77
gcggaacgac ttggcgttat tgcg 24

<210> 78
<211> 28
<212> DNA
<213> artificial

<220>
<223> primer

<400> 78
ggaagggtcc ttacgctata gagcgcag 28

<210> 79
<211> 31
<212> DNA
<213> artificial

<220>
<223> primer

<400> 79
ccaggcgaag ttgttattaa gcgtagattt g 31

<210> 80
<211> 33
<212> DNA
<213> artificial

<220>
<223> primer

<400> 80
cgtcgctttg aaacagaggc tttgagcctt ctc 33

<210> 81
<211> 48
<212> DNA
<213> artificial

<220>
<223> primer

<400> 81
gctagcaacc accatggcgg taattctaat tacttactaa atatagtg 48

<210> 82
<211> 41
<212> DNA
<213> artificial

<220>

<223> primer

<400> 82
ccgggatgta gaaggccacc tgtgaatacg gttaatgtca c 41

<210> 83
<211> 31
<212> DNA
<213> artificial

<220>
<223> primer

<400> 83
cagtcagtca cgagtttgtt accactgcga c 31

<210> 84
<211> 22
<212> DNA
<213> artificial

<220>
<223> primer

<400> 84
gcgggtggca gctggtgtac tg 22

<210> 85
<211> 21
<212> DNA
<213> artificial

<220>
<223> primer

<400> 85
cggagcacat ctgatagaac g 21

<210> 86
<211> 23
<212> DNA
<213> artificial

<220>
<223> primer

<400> 86
cgcggctgta ggcgctgccg ctc 23

<210> 87
<211> 31
<212> DNA
<213> artificial

<220>
<223> primer

<400> 87

ccaggcgaag ttgttattaa gcgtagattt g 31

<210> 88
<211> 33
<212> DNA
<213> artificial

<220>
<223> primer

<400> 88
cgtcgctttg aaacagaggc tttgagcctt ctc 33

<210> 89
<211> 52
<212> DNA
<213> artificial

<220>
<223> primer

<400> 89
gctagcaacc accatggcgg taattttaaa agcatatttt tttttgaaat tc 52

<210> 90
<211> 41
<212> DNA
<213> artificial

<220>
<223> primer

<400> 90
ccgggatgta gaaggccacc taaagatacc atggatgtat g 41

<210> 91
<211> 31
<212> DNA
<213> artificial

<220>
<223> primer

<400> 91
cagtcagtca cgagtttgtt accactgcga c 31

<210> 92
<211> 22
<212> DNA
<213> artificial

<220>
<223> primer

<400> 92
gcgggtggca gctggtgtac tg 22

<210> 93
<211> 21
<212> DNA
<213> artificial

<220>
<223> primer

<400> 93
gttgcaagtt gacactggcg g 21

<210> 94
<211> 23
<212> DNA
<213> artificial

<220>
<223> primer

<400> 94
aggtgtggga ggttttttaa agc 23

<210> 95
<211> 52
<212> DNA
<213> artificial

<220>
<223> primer

<400> 95
cctgtaatac gactcactat agggcgtttt tttttttttt tttttttttt tt 52

<210> 96
<211> 33
<212> DNA
<213> artificial

<220>
<223> primer

<400> 96
gcaaacggca atcagacggg cccaggctca gga 33

<210> 97
<211> 28
<212> DNA
<213> artificial

<220>
<223> primer

<400> 97
cctgtaatac gactcactat agggcgtt 28

<210> 98
<211> 37
<212> DNA

<213> artificial

<220>
<223> primer

<400> 98
gggatcgagc tagatcggcc tgagccgcca gtggtga 37

<210> 99
<211> 28
<212> DNA
<213> artificial

<220>
<223> primer

<400> 99
cctgtaatac gactcactat agggcgtt 28

<210> 100
<211> 32
<212> DNA
<213> artificial

<220>
<223> primer

<400> 100
cgctccatgg gatcggcgag ctgcgactcc gt 32

<210> 101
<211> 27
<212> DNA
<213> artificial

<220>
<223> primer

<400> 101
gcaacaacca gcggtgtccc ttgaaac 27

<210> 102
<211> 28
<212> DNA
<213> artificial

<220>
<223> primer

<400> 102
cctgtaatac gactcactat agggcgtt 28

<210> 103
<211> 28
<212> DNA
<213> artificial

<220>

<223> primer

<400> 103
gctagtggag aactgccaca aactgctg 28

<210> 104
<211> 34
<212> DNA
<213> artificial

<220>
<223> primer

<400> 104
caagcaaagt gaacacgtcg ctaagcgaaa gcta 34

<210> 105
<211> 25
<212> DNA
<213> artificial

<220>
<223> primer

<400> 105
gccctcgatg gtagacccgt aattg 25

<210> 106
<211> 14874
<212> DNA
<213> artificial

<220>
<223> LA1172 nucleotide sequence, including plasmid backbone

<400> 106
gggctggccg caaccattgt gggaaccgtg cgatcaaaca aacgcgagat accggaagta 60

ctgaaaaaca gtcgctccag gccagtggga acatcgatgt tttgttttga cggacccctt 120

actctcgtct catataaacc gaagccagct aagatggtat acttattatc atcttgtgat 180

gaggatgctt ctatcaacga aagtaccggt aaaccgcaaa tggttatgta ttataatcaa 240

actaaaggcg gagtggacac gctagaccaa atgtgttctg tgatgacctg cagtaggaag 300

acgaataggt ggcctatggc attattgtac ggaatgataa acattgcctg cataaattct 360

tttattatat acagccataa tgtcagtagc aagggagaaa aggtccaaag tcgcaaaaaa 420

tttatgagaa acctttacat gagcctgacg tcatcgttta tgcgtaagcg tttagaagct 480

cctactttga agagatattt gcgcgataat atctctaata ttttgccaaa tgaagtgcct 540

ggtacatcag atgacagtac tgaagagcca gtaatgaaaa aacgtactta ctgtacttac 600

tgcccctcta aaataaggcg aaaggcaaat gcatcgtgca aaaaatgcaa aaaagttatt 660

tgtcgagagc ataatattga tatgtgccaa agttgtttct gactgactaa taagtataat 720

ttgtttctat tatgtataag ttaagctaat tacttatttt ataatacaac atgactgttt 780
ttaaagtaca aaataagttt atttttgtaa aagagagaat gtttaaaagt tttgttactt 840
tatagaagaa attttgagtt tttgtttttt tttaataaat aaataaacat aaataaattg 900
tttgttgaat ttattattag tatgtaagtg taaatataat aaaacttaat atctattcaa 960
attaataaat aaacctcgat atacagaccg ataaaacaca tgcgtcaatt ttacgcatga 1020
ttatctttaa cgtacgtcac aatatgatta tctttctagg gttaaataat agtttctaat 1080
ttttttatta ttcagcctgc tgtcgtgaat accgtatatc tcaacgctgt ctgtgagatt 1140
gtcgtattct agccttttta gtttttcgct catcgacttg atattgtccg acacattttc 1200
gtcgatttgc gttttgatca aagacttgag cagagacacg ttaatcaact gttcaaattg 1260
atccatatta acgatatcaa cccgatgcgt atatggtgcg taaaatatat tttttaaccc 1320
tcttatactt tgcactctgc gttaatacgc gttcgtgtac agacgtaatc atgttttctt 1380
ttttggataa aactcctact gagtttgacc tcatattaga ccctcacaag ttgcaaaacg 1440
tggcattttt taccaatgaa gaatttaaag ttattttaaa aaatttcatc acagatttaa 1500
agaagaacca aaaattaaat tatttcaaca gtttaatcga ccagttaatc aacgtgtaca 1560
cagacgcgtc ggcaaaaaac acgcagcccg acgtgttggc taaaattatt aaatcaactt 1620
gtgttatagt cacggatttg ccgtccaacg tgttcctcaa aaagttgaag accaacaagt 1680
ttacggacac tattaattat ttgattttgc ccccacttcat tttgtgggat cacaattttg 1740
ttatatttta aacaaagctt ggcactggcc gtcgttttac aacgtcgtga ctgggaaaac 1800
cctggcgtta cccaacttaa tcgccttgca gcacatcccc ctttcgccag ctggcgtaat 1860
agcgaagagg cccgcaccga tcgcccttcc caacagttgc gcagcctgaa tggcgaatgg 1920
cgcctgatgc ggtattttct ccttacgcat ctgtgcggta tttcacaccg catatatggt 1980
gcactctcag tacaatctgc tctgatgccg catagttaag ccagccccga cacccgccaa 2040
cacccgctga cgcgccctga cgggcttgtc tgctcccggc atccgcttac agacaagctg 2100
tgaccgtctc cgggagctgc atgtgtcaga ggttttcacc gtcatcaccg aaacgcgcga 2160
gacgaaaggg cctcgtgata cgcctatttt tataggttaa tgtcatgata ataatggttt 2220
cttagacgtc aggtggcact tttcggggaa atgtgcgcgg aacccctatt tgtttatttt 2280
tctaaataca ttcaaatatg tatccgctca tgagacaata accctgataa atgcttcaat 2340
aatattgaaa aaggaagagt atgagtattc aacatttccg tgtcgccctt attccctttt 2400
ttgcggcatt ttgccttcct gtttttgctc acccagaaac gctggtgaaa gtaaaagatg 2460
ctgaagatca gttgggtgca cgagtgggtt acatcgaact ggatctcaac agcggtaaga 2520
tccttgagag ttttcgcccc gaagaacgtt ttccaatgat gagcactttt aaagttctgc 2580
tatgtggcgc ggtattatcc cgtattgacg ccgggcaaga gcaactcggt cgccgcatac 2640

actattctca gaatgacttg gttgagtact caccagtcac agaaaagcat cttacggatg 2700
gcatgacagt aagagaatta tgcagtgctg ccataaccat gagtgataac actgcggcca 2760
acttacttct gacaacgatc ggaggaccga aggagctaac cgcttttttg cacaacatgg 2820
gggatcatgt aactcgcctt gatcgttggg aaccggagct gaatgaagcc ataccaaacg 2880
acgagcgtga caccacgatg cctgtagcaa tggcaacaac gttgcgcaaa ctattaactg 2940
gcgaactact tactctagct tcccggcaac aattaataga ctggatggag gcggataaag 3000
ttgcaggacc acttctgcgc tcggcccttc cggctggctg gtttattgct gataaatctg 3060
gagccggtga gcgtgggtct cgcggtatca ttgcagcact ggggccagat ggtaagccct 3120
cccgtatcgt agttatctac acgacgggga gtcaggcaac tatggatgaa cgaaatagac 3180
agatcgctga gataggtgcc tcactgatta agcattggta actgtcagac caagtttact 3240
catatatact ttagattgat ttaaaacttc atttttaatt taaaaggatc taggtgaaga 3300
tcctttttga taatctcatg accaaaatcc cttaacgtga gttttcgttc cactgagcgt 3360
cagaccccgt agaaaagatc aaaggatctt cttgagatcc tttttttctg cgcgtaatct 3420
gctgcttgca aacaaaaaaa ccaccgctac cagcggtggt ttgtttgccg gatcaagagc 3480
taccaactct ttttccgaag gtaactggct tcagcagagc gcagatacca aatactgtcc 3540
ttctagtgta gccgtagtta ggccaccact tcaagaactc tgtagcaccg cctacatacc 3600
tcgctctgct aatcctgtta ccagtggctg ctgccagtgg cgataagtcg tgtcttaccg 3660
ggttggactc aagacgatag ttaccggata aggcgcagcg gtcgggctga acggggggtt 3720
cgtgcacaca gcccagcttg gagcgaacga cctacaccga actgagatac ctacagcgtg 3780
agcattgaga aagcgccacg cttcccgaag ggagaaaggc ggacaggtat ccggtaagcg 3840
gcagggtcgg aacaggagag cgcacgaggg agcttccagg gggaaacgcc tggtatcttt 3900
atagtcctgt cgggtttcgc cacctctgac ttgagcgtcg atttttgtga tgctcgtcag 3960
gggggcggag cctatggaaa aacgccagca acgcggcctt tttacggttc ctggcctttt 4020
gctggccttt tgctcacatg ttctttcctg cgttatcccc tgattctgtg gataaccgta 4080
ttaccgcctt tgagtgagct gataccgctc gccgcagccg aacgaccgag cgcagcgagt 4140
cagtgagcga ggaagcggaa gagcgcccaa tacgcaaacc gcctctcccc gcgcgttggc 4200
cgattcatta atgcagctgg cacgacaggt ttcccgactg gaaagcgggc agtgagcgca 4260
acgcaattaa tgtgagttag ctcactcatt aggcacccca ggctttacac tttatgcttc 4320
cggctcgtat gttgtgtgga attgtgagcg gataacaatt tcacacagga aacagctatg 4380
accatgatta cgaatttcga cctgcaggca tgcaagcttg catgcctgca ggtcgacgct 4440
cgcgcgactt ggtttgccat tctttagcgc gcgtcgcgtc acacagcttg gccacaatgt 4500

ggtttttgtc aaacgaagat tctatgacgt gtttaaagtt taggtcgagt aaagcgcaaa 4560
tcttttttaa ccctagaaag atagtctgcg taaaattgac gcatgcattc ttgaaatatt 4620
gctctctctt tctaaatagc gcgaatccgt cgctgtgcat ttaggacatc tcagtcgccg 4680
cttggagctc ccgtgaggcg tgcttgtcaa tgcggtaagt gtcactgatt ttgaactata 4740
acgaccgcgt gagtcaaaat gacgcatgat tatcttttac gtgactttta agatttaact 4800
catacgataa ttatattgtt atttcatgtt ctacttacgt gataacttat tatatatata 4860
ttttcttgtt atagatatcg tgactaatat ataataaaat gggtagttct ttagacgatg 4920
agcatatcct ctctgctctt ctgcaaagcg atgacgagct tgttggtgag gattctgaca 4980
gtgaaatatc agatcacgta agtgaagatg acgtccagag cgatacagaa gaagcgttta 5040
tagatgaggt acatgaagtg cagccaacgt caagcggtag tgaaatatta gacgaacaaa 5100
atgttattga acaaccaggt tcttcattgg cttctaacag aatcttgacc ttgccacaga 5160
ggactattag aggtaagaat aaacattgtt ggtcaacttc aaagtccacg aggcgtagcc 5220
gagtctctgc actgaacatt gtcagatcgg ccaggccggc cagatttaaa tgagcggccg 5280
catggtacca tactcggtgg cctccccacc accaactttt ttgcactgca aaaaaacacg 5340
cttttgcacg cgggcccata catagtacaa actctacgtt tcgtagacta ttttacataa 5400
atagtctaca ccgttgtata cgctccaaat acactaccac acattgaacc tttttgcagt 5460
gcaaaaaagt acgtgtcggc agtcacgtag gccggcctta tcgggtcgcg tcctgtcacg 5520
tacgaatcac attatcggac cggacgagtg ttgtcttatc gtgacaggac gccagcttcc 5580
tgtgttgcta accgcagccg gacgcaactc cttatcggaa caggacgcgc ctccatatca 5640
gccgcgcgtt atctcatgcg cgtgaccgga cacgaggcgc ccgtcccgct tatcgcgcct 5700
ataaatacag cccgcaacga tctggtaaac acagttgaac agcatctgtt acagcgacac 5760
aacatgagcc ggtccaacaa cgccaacgcg cccacgccat ccaaccgccg ccgcaacctg 5820
tctctggtgg atcccacccc acccaagaag aagcgcaaac cggtcgccac catggcctcc 5880
tccgagaacg tcatcaccga gttcatgcgc ttcaaggtgc gcatggaggg caccgtgaac 5940
ggccacgagt tcgagatcga gggcgagggc gagggccgcc cctacgaggg ccacaacacc 6000
gtgaagctga aggtgaccaa gggcggcccc ctgcccttcg cctgggacat cctgtccccc 6060
cagttccagt acggctccaa ggtgtacgtg aagcaccccg ccgacatccc cgactacaag 6120
aagctgtcct tccccgaggg cttcaagtgg gagcgcgtga tgaacttcga ggacggcggc 6180
gtggcgaccg tgacccagga ctcctccctg caggacggct gcttcatcta caaggtgaag 6240
ttcatcggcg tgaacttccc ctccgacggc cccgtgatgc agaagaagac catgggctgg 6300
gaggcctcca ccgagcgcct gtaccccccgc gacggcgtgc tgaagggcga gacccacaag 6360
gccctgaagc tgaaggacgg cggccactac ctggtggagt tcaagtccat ctacatggcc 6420

aagaagcccg tgcagctgcc cggctactac tacgtggacg ccaagctgga catcacctcc 6480
cacaacgagg actacaccat cgtggagcag tacgagcgca ccgagggccg ccaccacctg 6540
ttcctgagat ctcgacccaa gaaaaagcgg aaggtggagg acccgtaaga tccaccggat 6600
ctagataact gatcataatc agccatacca catttgtaga ggttttactt gctttaaaaa 6660
acctcccaca cctccccctg aacctgaaac ataaaatgaa tgcaattgtt gttgttaact 6720
tgtttattgc agcttataat ggttacaaat aaagcaatag catcacaaat ttcacaaata 6780
aagcattttt ttcactgcat tctagttgtg gtttgtccaa actcatcaat gtatcttaac 6840
gcgagttaat taatccattg ctgggcgagc tgcgccaatc gatgccaacg ccaccctgca 6900
tggcgagcgg caggccggcg gctaccatgg gcgtcaccat gccctgaccg cccccggagg 6960
gcagtgaaaa atgtgtgggg ggtggtgggg gctgcgcagg aactgattgt gattatggtt 7020
gtgcccatgg ccatgttgtc caagtccatg gacgtgggca tgcttgttgt agcccaaatc 7080
ggcgtttccg tttccaccag gaaacatctc tgcttgtagt tcgaatatgc tctttaaatc 7140
ccagctgtat tcctcagtta tcgaggtttt cttcacgagt gaaacgaatt ttcgtcgcct 7200
tctacgccat ttcttgctc agcccgtttt gtcattcgca gcgaagcggt aacagcgggt 7260
cgctcatatg acggtatttt ttaatacact tcagctatac tgttatttca aaaacatatt 7320
tcttttgtta ctttttatgc agttcatttg ccaccaaaaa gtagtctttt ggattgattt 7380
atttcaaaaa atggtgtaat tcaagaaatt cagagggcca agtaatatac ttaatgaccg 7440
ttatttaaaa cacactcaag gagatttatt taaacggcta caatggtttt ccaaataact 7500
tatttactgt tgacttctat aaaacatagg tgtatatatt attatttcct tattgagttt 7560
gagataattt taatttccac aatatttttt cttgtgatta acagagaaag tcaaactaca 7620
taacatttat cgggtaaaag tctctatgaa ggtagcggtt aacagtgaag tcgcaaaagt 7680
ggtggccgta cgccaatcga gcgtagtacc cctaacctgc aatattttta gttggttttt 7740
tccgcaatag ccccagtttt ctcaaagagt gcaacaagtg attctgttta tgttttcaac 7800
aacttctctc tgcggaactt aacgtgagcg gacgtatgcg gacgcgccat ggtttaaact 7860
cgctagcact gggaagttga cgttgatata gagccgaatt gaacttcacc gctgcttggt 7920
aattactcta caagttcatt taggagaacc ggattcgaaa gatgatttc cagcgtttag 7980
ctttcagatg gccgcataca ttttgcacca ccaaaccgaa actcactagc gtatccaatc 8040
gttcgttttt tggtgccggt gtgttacgaa ctttagctat caagctaaag caatttgctc 8100
tggtcttccg tgctaaaaag aaaaaaaaac tgtttttttt ttggttttga tatttgcgct 8160
atttttactt gggccttaat tgaacaaact tttgaaagtt tccacagcga aatcgttttc 8220
gacgatgcca tttttggtaa catttgcatt ttcttgctca aattgcttgc aaaacccgtg 8280

aaagacatta atattcgata gtgtcatcca aaatcacgaa aatgattgtt gcaaaacgtt 8340
gaacaattta cacatgtaaa aaacaaccat cgattaatgt ttattcaaac tttttacaag 8400
aagggttatt ctgatcaatg tcaccccgct gatgaatgtt accccggatt acacttctcg 8460
aaaagtggtt caaaatgcta cttgagaatt tttatctgtc aaaggaagca aattcgagtc 8520
gaattaaatg gtatagtcct gaattaggtt tccatttact tacaggtatt ccactaaata 8580
gctggaagat ttattttaca caataatgat aattcgtacc ccaaagagtg tagccctact 8640
tttttctctc tttttttttt gtaaattttc atcgctgcgt gccagcttac cgacatgtcg 8700
cgacagcata aagagcctgt caagagatga agaaaaatga caaggagtca gtggtcaggt 8760
ctctgtatca atatttgacg tcctgacttt ccaatatacc tttccttaaa gagtagagat 8820
catgcgatac gtgaataaat atcgtttgga cttcgaaata gaacataatt taaggtagct 8880
gatcagtagt tgaacatctt cagacttctg ggacaagaag tgtttttttg tttgtagaaa 8940
aggtttttgt taaattatat ttgtaagata attcaatgaa tatatctctg attcagtaat 9000
caatccgtac cacgcaccgt ttaagaaaca ccctgtaggt ttgcatcacg tctcagacaa 9060
aagtgtatcg atgtgcgaac actgcatacc ggcgctttgc aaataatgcc aaatttagat 9120
atgcattaca ttgtcacttc gcaaaacaca cactcccaaa tgcgtcggaa acctcacccg 9180
aacgcacgat cgtaacgcga tcgatcgccg attgattgat cggaattaac tatctcaatc 9240
gatccttcta tggactgatg catgggccgg cacttccgag tataaaaccc cggtaaaccc 9300
aaggaatcac tcacaatcgg attttgacgc tcgctctggt acagttcgat acggtctagt 9360
gaaaccgagg ataacgacga aggtttttcc ccattgatcc aggtcggtgt ttatgattgg 9420
tggaaaaaga ctcgagaaaa gttccatcga agccgttgga aatgtgccgt cttcctgtga 9480
cgtcttgtgg atccagttcc ttgttcacgt ctggtgatcg tgtaaaatgt gctgtcttgt 9540
ggcgtcatat gtgttccaga tccagtgatt acgatccgat gtgatgttga tcccttgtga 9600
acgtcttatc ctgttccgtg tgcaccatgc ataatgtcgt attacgtaag ttctgaagtg 9660
aaacagaaga gtgaattgaa agttttttta ttcaacatca acctaaatat ggactttact 9720
ttccaagaaa attatgcctg atcaactgtg gatagttaca aaaaaaaaag gtttattaat 9780
taaattttat gattacataa tgtgttgaaa agaacaactg aaattttaga agaagatctt 9840
ttcgtgcatc aggctttgcc aattaattga tgataaatta tcatagcaaa ttaacgtaga 9900
gactaaaagg tatatcgtca aatagggctt cttttgacac tattttggca ttcttgctct 9960
ttgagaactt gcaaccctaa aatgggatct tcatcagcct agtggttaga ttcagcagct 10020
acaaagcaaa accatgctga agggttcgat tcccggtcgt ttcaggatct tttcgtaatt 10080
gaaatatcct tgactaccct aagtatcatt gtgcttgcca tttacgaata tacatattac 10140
gatatacgaa tgagaaaatg acaactttgg aaaataaagc tctcaatgtt tcaataagaa 10200

ataaatacta catcagtatt gaaggctaat aacaattaca gattagaacc tttaaacatc 10260
atttctgcaa caggctggat aaagtacagt tggaggatta aattatgcga ttttgcaatt 10320
ttttccgatt aaattcatat ttattcctgg tttggttttt acaaaaaata tttttacatg 10380
acgtttgacc ccgattccct caactttgat tgttatattt ttttttggac aggttgagtt 10440
tgtgggtttt ttcctagtgt tgctttgctt tatgggctct ggttatttaa aattaaaatt 10500
tgacaatctt actacacact ccgaaaaaat catgcgattt tacgtctttt ggatgcacat 10560
aaaagaagcg agccaaatga ggtgaatttg tgtcacattt taaatacgat ggtgtctgat 10620
tcgggaaatg tcaatgatag tgtcattcaa tcataatgtg aattacgtcc gcagtaattt 10680
tcattatttt taagagtgta ctactattta cactacaaaa attttgatac cccagggggg 10740
aacgaggtcc cggatgtcca gctggccaga ttgttggcaa cgagccctgt acctattgat 10800
cgagtcacca aagcactcct caagtgtttt aatctcgacc agacggtgga cctcggttgt 10860
tctcattctc ggagggcgat ttcgcaatca ttagtaccaa ccacatgtcg aagtcgggag 10920
atgttataaa attataacca attattcaaa aaatgacatc attcaatttg aacaaacgtt 10980
cgatagaaat tatatatgat ttcacatgat attaaactac gaagaaaatt ttacataagg 11040
aagtggtata aaacgtaata tgcttaataa aaactttaac ccttttggga ggataatatt 11100
cagaagttct gattcagaac catctctcat gttatgttcg tttttgttg cttgtccttt 11160
atatgccaca tgaacaataa caccaatatc tatcccattt ccaggaccta acggaccttg 11220
aagcggcgcc aaaacgtgtg acgatgatgc tggtaccctg gcggtaagtt gatcaaagga 11280
aacgcaaagt tttcaagaaa aaacaaaact aatttgattt ataacacctt tagaaaccac 11340
catgggcagc cgcctggata agtccaaagt catcaactcc gcgttggagc tgttgaacga 11400
agttggcatt gagggactga cgacccgcaa gttggcgcag aagctgggcg tggagcagcc 11460
caccctctac tggcacgtga agaataagcg ggcgctgctg gatgccctgg ccatcgagat 11520
gctcgaccgc caccacacgc atttttgccc gttggaaggc gagtcctggc aggacttcct 11580
ccgcaataac gccaagtcgt tccgctgcgc tctgctgtcc caccgagacg gtgccaaagt 11640
ccatctcggc acgcgcccga ccgaaaagca atacgagaca ctggagaacc agctcgcgtt 11700
cctgtgccag caaggcttca gcctggaaaa tgctctctac gctctgagcg ccgtcggtca 11760
ctttaccctg ggctgcgtgc tggaggacca agagcatcaa gtcgcaaaag aggagcgcga 11820
gaccccaaca accgattcga tgcccccact gctgcgtcag gcaatcgagc tgttcgatca 11880
tcaaggagcc gagccggcat tcctgttcgg cttggagctg attatctgcg gattggaaaa 11940
gcaactgaaa tgcgagtcgg gctcgggccc cgcgtacagc cgcgcgcgta cgaaaaacaa 12000
ttacgggtct accatcgagg gcctgctcga tctcccggac gacgacgccc ccgaagaggc 12060

ggggctggcg gctccgcgcc tgtcctttct ccccgcggga cacacgcgca gactgtcgac 12120
ggcccccccg accgatgtca gcctgggggga cgagctccac ttagacggcg aggacgtggc 12180
gatggcgcat gccgacgcgc tagacgattt cgatctggac atgttggggg acggggattc 12240
cccgggtccg ggatttaccc cccacgactc cgccccctac ggcgctctgg atatggccga 12300
cttcgagttt gagcagatgt ttaccgatgc ccttggaatt gacgagtacg gtgggtagtt 12360
ctagaattgt ccaccgcaag tgcttctaag ccgatcccga ttgtactgat taccataagc 12420
gacattgcca gtgaaagcga caacagcagc atcaaagtac atttgtcata ctgattcggc 12480
tactaccacc atccggaatc agcttgcatc gaacatcaaa tcacgttatt caatgtatct 12540
gtcatccagc tcagacaagt cggagctttt ccagtcgcga aaatctgcga ctccagcgga 12600
aagcaccgaa ccacagagag gactcgtatg aaagccaggg aagaaaccat cattcacctt 12660
gcagcaaata ggaaaaaaaa cggacatctt caacaaacaa aagcccatgc gctaacttgg 12720
tttaggagtt tagtgtgaca ccatgacccc gctgatgatc tttacttagc acaccataac 12780
cacctttatg cgttcgttca tccaaaatct acaggatatc actgcagccg cgagaagaac 12840
tcgtgaacca tcctgttttc ttttttatta tattcttact tttaacttca aattattttc 12900
agtaataaaa cgtctcaaaa taataagttc ataatgagtt taattttacg gaataagaac 12960
aaccatttaa gttattaaat ccttagattt aatggaatta gattgattat atggaaccca 13020
gacttggtaa aaaataaact ccacgttaaa tttctttctg agacttaaaa ttctttcggg 13080
aaagctggga gcaattctcg caccggtgct agggccgcat agtcgacatt tcgagtttac 13140
cactccctat cagtgataga gaaaagtgaa agtcgagttt accactccct atcagtgata 13200
gagaaaagtg aaagtcgagt ttaccactcc ctatcagtga tagagaaaag tgaaagtcga 13260
gtttaccact ccctatcagt gatagagaaa agtgaaagtc gagtttacca ctccctatca 13320
gtgatagaga aaagtgaaag tcgagtttac cactccctat cagtgataga gaaaagtgaa 13380
agtcgagttt accactccct atcagtgata gagaaaagtg aaagtcgagc tcggtacccg 13440
ggtcgaggta ggcgtgtacg gtgggaggcc tatataagca gagctcgttt agtgaaccgt 13500
cagatcgcct ggagacgcca tccacgctgt tttgacctcc atagaagaca ccgggaccga 13560
tccagcctcc gcggccccga attcgagctc ggtacccggg gatccccgct cgaccaccat 13620
gggcgctctc ctgggcctgc ccgaaagcca aacggagctt gataatctta cagaatacaa 13680
cacggcccac aatcggcgca tctcaatgct gggcatcgat gatgatacca atatgcgaaa 13740
gcaaaacgcc ttgaaacagg gacggcgcac tcgaaatgtc acatttaacg atgaggagat 13800
tgtcatcaat cctgaggatg tggatcctaa tgtgggacgc ttcaggaact tggtacaaac 13860
cactgtggtg cccgccaaga gggctcgctg cgacgtcaac cattagtgat aacgcgtcta 13920
gctagagctg agaacttcag ggtgagtttg gggacccttg attgttcttt ctttttcgct 13980

attgtaaaat tcatgttata tggagggggc aaagttttca gggtgttgtt tagaatggga 14040

agatgtccct tgtatcacca tggaccctca tgataatttt gtttctttca ctttctactc 14100

tgttgacaac cattgtctcc tcttattttc ttttcatttt ctgtaacttt ttcgttaaac 14160

tttagcttgc atttgtaacg aatttttaaa ttcacttttg tttatttgtc agattgtaag 14220

tactttctct aatcactttt ttttcaaggc aatcagggta tattatattg tacttcagca 14280

cagttttaga gaacaattgt tataattaaa tgataaggta gaatatttct gcatataaat 14340

tctggctggc gtggaaatat tcttattggt agaaacaact acaccctggt catcatcctg 14400

cctttctctt tatggttaca atgatataca ctgtttgaga tgaggataaa atactctgag 14460

tccaaaccgg gcccctctgc taaccatgtt catgccttct tctctttcct acagctcctg 14520

ggcaacgtgc tggttgttgt gctgtctcat cattttggca aagaattcac tcctcaggtg 14580

caggctgcct atcagaaggt ggtggctggt gtggccaatg ccctggctca caaataccac 14640

tgagatcttt ttccctctgc caaaaattat ggggacatca tgaagcccct tgagcatctg 14700

acttctggct aataaaggaa atttattttc attgcaatag tgtgttggaa ttttttgtgt 14760

ctctcactcg gaaggacata tgggagggca aatcatttaa aacatcagaa tgagtatttg 14820

gtttagagtt tggcaacata tgcccatagc ggccctagcg gcgcgccata gccc 14874

<210> 107
<211> 13
<212> DNA
<213> Drosophila sp.

<400> 107
tcaataatcg tca 13

<210> 108
<211> 13
<212> DNA
<213> Drosophila sp.

<400> 108
tcatcaaacg tca 13

<210> 109
<211> 13
<212> DNA
<213> Drosophila sp.

<400> 109
ttatcgttaa aca 13

<210> 110
<211> 13
<212> DNA
<213> Drosophila sp.

<400> 110
taaacagtca ata 13

<210> 111
<211> 13
<212> DNA
<213> Drosophila sp.

<400> 111
tacacgatca gca 13

<210> 112
<211> 13
<212> DNA
<213> Drosophila sp.

<400> 112
aatacaaaca aca 13

<210> 113
<211> 13
<212> DNA
<213> Drosophila sp.

<400> 113
tcatcaacaa gca 13

<210> 114
<211> 13
<212> DNA
<213> Drosophila sp.

<400> 114
tctacaaacc aga 13

<210> 115
<211> 13
<212> DNA
<213> Drosophila sp.

<400> 115
acatcgattc aca 13

<210> 116
<211> 13
<212> DNA
<213> Drosophila sp.

<400> 116
cgctcaatca aca 13

<210> 117
<211> 13
<212> DNA
<213> Drosophila sp.

<400> 117
tctaccataa aaa 13

<210> 118
<211> 13
<212> DNA
<213> Drosophila sp.

<400> 118
aaatgaatca aca 13

<210> 119
<211> 13
<212> DNA
<213> Drosophila sp.

<400> 119
acatcgttca acg 13

<210> 120
<211> 13
<212> DNA
<213> Drosophila sp.

<400> 120
tcttgattca cca 13

<210> 121
<211> 13
<212> DNA
<213> Drosophila sp.

<400> 121
tctgcagaca aca 13

<210> 122
<211> 13
<212> DNA
<213> Drosophila sp.

<400> 122
tcttcggtaa tca 13

<210> 123
<211> 13
<212> DNA
<213> Drosophila sp.

<400> 123
tctataaaca ata 13

<210> 124
<211> 13
<212> DNA
<213> Drosophila sp.

<400> 124
taaacaataa ata 13

<210> 125
<211> 13
<212> DNA
<213> Drosophila sp.

<400> 125
taaacaagca aaa 13

<210> 126
<211> 13
<212> DNA
<213> Drosophila sp.

<400> 126
tcaacgatcg gcg 13

<210> 127
<211> 13
<212> DNA
<213> Drosophila sp.

<400> 127
tgatccatca tca 13

<210> 128
<211> 13
<212> DNA
<213> Drosophila sp.

<400> 128
tcaacatgca aga 13

<210> 129
<211> 13
<212> DNA
<213> Drosophila sp.

<400> 129
tcttaaataa aga 13

<210> 130
<211> 13
<212> DNA
<213> Drosophila sp.

<400> 130
tcaaagatct ata 13

<210> 131
<211> 13
<212> DNA
<213> Drosophila sp.

<400> 131
taatgaatta aca 13

<210> 132
<211> 13
<212> DNA
<213> Drosophila sp.

<400> 132
tttaccatca act 13

<210> 133
<211> 13
<212> DNA
<213> Drosophila sp.

<400> 133
taatgaaaca aca 13

<210> 134
<211> 13
<212> DNA
<213> Drosophila sp.

<400> 134
gtttcaatta aaa 13

<210> 135
<211> 13
<212> DNA
<213> Drosophila sp.

<400> 135
tattcaatta taa 13

<210> 136
<211> 13
<212> DNA
<213> Drosophila sp.

<400> 136
tcttcaatcg ttt 13

<210> 137
<211> 13
<212> DNA
<213> Drosophila sp.

<400> 137
tcaacgatcc ttt 13

<210> 138
<211> 13
<212> DNA
<213> artificial

<220>
<223>  Table 2 consensus sequence

<400>  138
tcwwcratca aca                                                          13

<210>  139
<211>  34
<212>  DNA
<213>  artificial

<220>
<223>  primer HSP

<400>  139
caagcaaagt gaacacgtcg ctaagcgaaa gcta                                   34

<210>  140
<211>  25
<212>  DNA
<213>  artificial

<220>
<223>  VP16 primer

<400>  140
gccctcgatg gtagacccgt aattg                                             25

<210>  141
<211>  24
<212>  DNA
<213>  artificial

<220>
<223>  primer Agexon1F

<400>  141
ggaaaccgag gataacgacg aagg                                              24

<210>  142
<211>  24
<212>  DNA
<213>  artificial

<220>
<223>  primer TETRR1

<400>  142
gcggaacgac ttggcgttat tgcg                                              24

<210>  143
<211>  6243
<212>  DNA
<213>  artificial

<220>
<223>  LA3576 plasmid sequence

<400> 143
cctctacaaa tgtggtatgg ctgattatga tcagttatct agatccggtg gatcttacgg 60
gtcctccacc ttccgctttt tcttgggtcg agatctcagg aacaggtggt ggcggccctc 120
ggtgcgctcg tactgctcca cgatggtgta gtcctcgttg tgggaggtga tgtccagctt 180
ggcgtccacg tagtagtagc cgggcagctg cacgggcttc ttggccatgt agatggactt 240
gaactccacc aggtagtggc cgccgtcctt cagcttcagg gccttgtggg tctcgccctt 300
cagcacgccg tcgcgggggt acaggcgctc ggtggaggcc tcccagccca tggtcttctt 360
ctgcatcacg gggccgtcgg aggggaagtt cacgccgatg aacttcacct tgtagatgaa 420
gcagccgtcc tgcagggagg agtcctgggt cacggtcgcc acgccgccgt cctcgaagtt 480
catcacgcgc tcccacttga agccctcggg gaaggacagc ttcttgtagt cggggatgtc 540
ggcggggtgc ttcacgtaca ccttggagcc gtactggaac tgggggggaca ggatgtccca 600
ggcgaagggc agggggccgc ccttggtcac cttcagcttc acggtgttgt ggccctcgta 660
ggggcggccc tcgccctcgc cctcgatctc gaactcgtgg ccgttcacgg tgccctccat 720
gcgcaccttg aagcgcatga actcggtgat gacgttctcg gaggaggcca tggtggcgac 780
cggtttgcgc ttcttcttgg gtggggtggg atctcccatg gtggcctgaa tctcaacttg 840
cacctggcga tcgcctaaag gtgttataaa tcaaattagt tttgtttttt cttgaaaact 900
ttgcgtttcc tttgatcaac ttaccgccag ggtacctgca gattgtttag cttgttcagc 960
tgcgcttgtt tatttgctta gctttcgctt agcgacgtgt tcactttgct tgtttgaatt 1020
gaattgtcgc tccgtagacg aagcgcctct atttatactc cggcgctgtt taaacatcca 1080
ccatgcgccc gcatcgatct ctatcactga tagggaggtc tctatcactg atagggagtt 1140
ctctatcact gatagggatg tctctatcac tgatagggat ttctctatca ctgataggga 1200
agtctctatc actgataggg acctctctat cactgatagg gaaatctcta tcactgatag 1260
ggatctctct atcactgata gggacttctc tatcactgat agggacgtct ctatcactga 1320
tagggaactc tctatcactg atagggacat ctctatcact gatagggact tctctatcac 1380
tgatagggag tatgttctct ctcttctctt ctctctctct ttctcgaatg ttctctctct 1440
tctcttctct ctctctttct cgatggccgg ggcgcgccag gtttcgactt tcacttttct 1500
ctatcactga tagggagtgg taaactcgac tttcactttt ctctatcact gatagggagt 1560
ggtaaactcg actttcactt ttctctatca ctgataggga gtggtaaact cgactttcac 1620
ttttctctat cactgatagg gagtggtaaa ctcgactttc acttttctct atcactgata 1680
gggagtggta aactcgactt tcacttttct ctatcactga tagggagtgg taaactcgac 1740
tttcactttt ctctatcact gatagggagt ggtaaactcg agcggccgcc accgcggtgg 1800
agctccagct tttgttccct ttagtgaggg ttaattgcgc gcttggcgta atcatggtca 1860

tagctgtttc ctgtgtgaaa ttgttatccg ctcacaattc cacacaacat acgagccgga 1920

agcataaagt gtaaagcctg gggtgcctaa tgagtgagct aactcacatt aattgcgttg 1980

cgctcactgc ccgctttcca gtcgggaaac ctgtcgtgcc agctgcatta atgaatcggc 2040

caacgcgcgg ggagaggcgg tttgcgtatt gggcgctctt ccgcttcctc gctcactgac 2100

tcgctgcgct cggtcgttcg gctgcggcga gcggtatcag ctcactcaaa ggcggtaata 2160

cggttatcca cagaatcagg ggataacgca ggaaagaaca tgtgagcaaa aggccagcaa 2220

aaggccagga accgtaaaaa ggccgcgttg ctggcgtttt tccataggct ccgcccccct 2280

gacgagcatc acaaaaatcg acgctcaagt cagaggtggc gaaacccgac aggactataa 2340

agataccagg cgtttccccc tggaagctcc ctcgtgcgct ctcctgttcc gaccctgccg 2400

cttaccggat acctgtccgc ctttctccct tcgggaagcg tggcgctttc tcatagctca 2460

cgctgtaggt atctcagttc ggtgtaggtc gttcgctcca agctgggctg tgtgcacgaa 2520

ccccccgttc agcccgaccg ctgcgcctta tccggtaact atcgtcttga gtccaacccg 2580

gtaagacacg acttatcgcc actggcagca gccactggta acaggattag cagagcgagg 2640

tatgtaggcg gtgctacaga gttcttgaag tggtggccta actacggcta cactagaagg 2700

acagtatttg gtatctgcgc tctgctgaag ccagttacct tcggaaaaag agttggtagc 2760

tcttgatccg gcaaacaaac caccgctggt agcggtggtt tttttgtttg caagcagcag 2820

attacgcgca gaaaaaaagg atctcaagaa gatcctttga tcttttctac ggggtctgac 2880

gctcagtgga acgaaaactc acgttaaggg attttggtca tgagattatc aaaaaggatc 2940

ttcacctaga tccttttaaa ttaaaaatga agttttaaat caatctaaag tatatatgag 3000

taaacttggt ctgacagtta ccaatgctta atcagtgagg cacctatctc agcgatctgt 3060

ctatttcgtt catccatagt tgcctgactc cccgtcgtgt agataactac gatacgggag 3120

ggcttaccat ctggccccag tgctgcaatg ataccgcgag acccacgctc accggctcca 3180

gatttatcag caataaacca gccagccgga agggccgagc gcagaagtgg tcctgcaact 3240

ttatccgcct ccatccagtc tattaattgt tgccgggaag ctagagtaag tagttcgcca 3300

gttaatagtt tgcgcaacgt tgttgccatt gctacaggca tcgtggtgtc acgctcgtcg 3360

tttggtatgg cttcattcag ctccggttcc caacgatcaa ggcgagttac atgatccccc 3420

atgttgtgca aaaaagcggt tagctccttc ggtcctccga tcgttgtcag aagtaagttg 3480

gccgcagtgt tatcactcat ggttatggca gcactgcata attctcttac tgtcatgcca 3540

tccgtaagat gcttttctgt gactggtgag tactcaacca agtcattctg agaatagtgt 3600

atgcggcgac cgagttgctc ttgcccggcg tcaatacggg ataataccgc gccacatagc 3660

agaactttaa aagtgctcat cattggaaaa cgttcttcgg ggcgaaaact ctcaaggatc 3720

ttaccgctgt tgagatccag ttcgatgtaa cccactcgtg cacccaactg atcttcagca 3780
tcttttactt tcaccagcgt ttctgggtga gcaaaaacag gaaggcaaaa tgccgcaaaa 3840
aagggaataa gggcgacacg gaaatgttga atactcatac tcttcctttt tcaatattat 3900
tgaagcattt atcagggtta ttgtctcatg agcggataca tatttgaatg tatttagaaa 3960
aataaacaaa taggggttcc gcgcacattt ccccgaaaag tgccacctaa attgtaagcg 4020
ttaatatttt gttaaaattc gcgttaaatt tttgttaaat cagctcattt tttaaccaat 4080
aggccgaaat cggcaaaatc ccttataaat caaaagaata gaccgagata gggttgagtg 4140
ttgttccagt ttggaacaag agtccactat taaagaacgt ggactccaac gtcaaagggc 4200
gaaaaaccgt ctatcagggc gatggcccac tacgtgaacc atcaccctaa tcaagttttt 4260
tggggtcgag gtgccgtaaa gcactaaatc ggaaccctaa agggagcccc cgatttagag 4320
cttgacgggg aaagccggcg aacgtggcga gaaaggaagg gaagaaagcg aaaggagcgg 4380
gcgctagggc gctggcaagt gtagcggtca cgctgcgcgt aaccaccaca cccgccgcgc 4440
ttaatgcgcc gctacagggc gcgtcccatt cgccattcag gctgcgcaac tgttgggaag 4500
ggcgatcggt gcgggcctct tcgctattac gccagctggc gaaaggggga tgtgctgcaa 4560
ggcgattaag ttgggtaacg ccagggtttt cccagtcacg acgttgtaaa acgacggcca 4620
gtgagcgcgc gtaatacgac tcactatagg gcgaattggg taccgggccc cccctcgagg 4680
tcgacgatgt aggtcacggt ctcgaagccg cggtgcgggt gccagggcgt gcccttgggc 4740
tccccgggcg cgtactccac ctcacccatc tggtccatca tgatgaacgg gtcgaggtgg 4800
cggtagttga tcccggcgaa cgcgcggcgc accgggaagc cctcgccctc gaaaccgctg 4860
ggcgcggtgg tcacggtgag cacgggacgt gcgacggcgt cggcgggtgc ggatacgcgg 4920
ggcagcgtca gcgggttctc gacggtcacg gcgggcatgt cgacggtatc gataagcttg 4980
ggccccccct cgaggttccc acaatggtta attcgagctc gcccggggat ctaattcaat 5040
tagagactaa ttcaattaga gctaattcaa ttaggatcca agcttatcga tttcgaaccc 5100
tcgaccgccg gagtataaat agaggcgctt cgtctacgga gcgacaattc aattcaaaca 5160
agcaaagtga acacgtcgct aagcgaaagc taagcaaata aacaagcgca gctgaacaag 5220
ctaaacaatc ggggtaccgc tagagtcgat cccaccccac ccaagaagaa gcgcaaaccg 5280
gtaccatggc ctcctccgag aacgtcatca ccgagttcat gcgcttcaag gtgcgcatgg 5340
agggcaccgt gaacggccac gagttcgaga tcgagggcga gggcgagggc cgcccctacg 5400
agggccacaa caccgtgaag ctgaaggtga ccaagggcgg cccccctgccc ttcgcctggg 5460
acatcctgtc cccccagttc cagtacggct ccaaggtgta cgtgaagcac cccgccgaca 5520
tccccgacta caagaagctg tccttccccg agggcttcaa gtgggagcgc gtgatgaact 5580
tcgaggacgg cggcgtggcg accgtgaccc aggactcctc cctgcaggac ggctgcttca 5640

tctacaaggt gaagttcatc ggcgtgaact tcccctccga cggccccgtg atgcagaaga 5700

agaccatggg ctgggaggcc tccaccgagc gcctgtaccc ccgcgacggc gtgctgaagg 5760

gcgagaccca caaggccctg aagctgaagg acggcggcca ctacctggtg gagttcaagt 5820

ccatctacat ggccaagaag cccgtgcagc tgcccggcta ctactacgtg gacgccaagc 5880

tggacatcac ctcccacaac gaggactaca ccatcgtgga gcagtacgag cgcaccgagg 5940

gccgccacca cctgttcctg tgatgatcat aatcagccat accacatttg tagaggtttt 6000

acttgcttta aaaaacctcc cacacctccc cctgaacctg aaacataaaa tgaatgcaat 6060

tgttgttgtt aacttgttta ttgcagctta taatggttac aaataaagca atagcatcac 6120

aaatttcaca aataaagcat ttttttcact gcattctagt tgtggtttgt ccaaactcat 6180

caatgtatct taacgcgagt taattaaggc cgctcattta tcagcgcttt aaatttgcgc 6240

atg 6243

<210> 144
<211> 5746
<212> DNA
<213> artificial

<220>
<223> LA3582 plasmid sequence

<400> 144
cgcctaaagg tgttataaat caaattagtt ttgtttttc ttgaaaactt tgcgtttcct 60

ttgatcaact taccgccagg gtacctgcag attgtttagc ttgttcagct gcgcttgttt 120

atttgcttag ctttcgctta gcgacgtgtt cactttgctt gtttgaattg aattgtcgct 180

ccgtagacga agcgcctcta tttatactcc ggcgctgttt aaacatccac catgcgcccg 240

catcgatctc tatcactgat agggaggtct ctatcactga tagggagttc tctatcactg 300

atagggatgt ctctatcact gatagggatt tctctatcac tgatagggaa gtctctatca 360

ctgataggga cctctctatc actgataggg aaatctctat cactgatagg gatctctcta 420

tcactgatag ggacttctct atcactgata gggacgtctc tatcactgat agggaactct 480

ctatcactga tagggacatc tctatcactg atagggactt ctctatcact gatagggagt 540

atgttctctc tcttctcttc tctctctctt tctcgaatgt tctctctctt ctcttctctc 600

tctctttctc gatggccggg gcgcgccagg tttcgacttt cacttttctc tatcactgat 660

agggagtggt aaactcgact ttcacttttc tctatcactg atagggagtg gtaaactcga 720

ctttcacttt tctctatcac tgatagggag tggtaaactc gactttcact tttctctatc 780

actgataggg agtggtaaac tcgactttca cttttctcta tcactgatag ggagtggtaa 840

actcgacttt cacttttctc tatcactgat agggagtggt aaactcgact ttcacttttc 900

```
tctatcactg atagggagtg gtaaactcga gcggccgcca ccgcggtgga gctccagctt      960
ttgttccctt tagtgagggt taattgcgcg cttggcgtaa tcatggtcat agctgtttcc     1020
tgtgtgaaat tgttatccgc tcacaattcc acacaacata cgagccggaa gcataaagtg     1080
taaagcctgg ggtgcctaat gagtgagcta actcacatta attgcgttgc gctcactgcc     1140
cgctttccag tcgggaaacc tgtcgtgcca gctgcattaa tgaatcggcc aacgcgcggg     1200
gagaggcggt ttgcgtattg ggcgctcttc cgcttcctcg ctcactgact cgctgcgctc     1260
ggtcgttcgg ctgcggcgag cggtatcagc tcactcaaag gcggtaatac ggttatccac     1320
agaatcaggg gataacgcag gaaagaacat gtgagcaaaa ggccagcaaa aggccaggaa     1380
ccgtaaaaag gccgcgttgc tggcgttttt ccataggctc cgcccccctg acgagcatca     1440
caaaaatcga cgctcaagtc agaggtggcg aaacccgaca ggactataaa gataccaggc     1500
gtttccccct ggaagctccc tcgtgcgctc tcctgttccg accctgccgc ttaccggata     1560
cctgtccgcc tttctccctt cgggaagcgt ggcgctttct catagctcac gctgtaggta     1620
tctcagttcg gtgtaggtcg ttcgctccaa gctgggctgt gtgcacgaac cccccgttca     1680
gcccgaccgc tgcgccttat ccggtaacta tcgtcttgag tccaacccgg taagacacga     1740
cttatcgcca ctggcagcag ccactggtaa caggattagc agagcgaggt atgtaggcgg     1800
tgctacagag ttcttgaagt ggtggcctaa ctacggctac actagaagga cagtatttgg     1860
tatctgcgct ctgctgaagc cagttacctt cggaaaaaga gttggtagct cttgatccgg     1920
caaacaaacc accgctggta gcggtggttt ttttgtttgc aagcagcaga ttacgcgcag     1980
aaaaaaagga tctcaagaag atcctttgat cttttctacg gggtctgacg ctcagtggaa     2040
cgaaaactca cgttaaggga ttttggtcat gagattatca aaaaggatct tcacctagat     2100
ccttttaaat taaaaatgaa gttttaaatc aatctaaagt atatatgagt aaacttggtc     2160
tgacagttac caatgcttaa tcagtgaggc acctatctca gcgatctgtc tatttcgttc     2220
atccatagtt gcctgactcc ccgtcgtgta gataactacg atacgggagg gcttaccatc     2280
tggccccagt gctgcaatga taccgcgaga cccacgctca ccggctccag atttatcagc     2340
aataaaccag ccagccggaa gggccgagcg cagaagtggt cctgcaactt tatccgcctc     2400
catccagtct attaattgtt gccgggaagc tagagtaagt agttcgccag ttaatagttt     2460
gcgcaacgtt gttgccattg ctacaggcat cgtggtgtca cgctcgtcgt ttggtatggc     2520
ttcattcagc tccggttccc aacgatcaag gcgagttaca tgatccccca tgttgtgcaa     2580
aaaagcggtt agctccttcg gtcctccgat cgttgtcaga agtaagttgg ccgcagtgtt     2640
atcactcatg gttatggcag cactgcataa ttctcttact gtcatgccat ccgtaagatg     2700
cttttctgtg actggtgagt actcaaccaa gtcattctga gaatagtgta tgcggcgacc     2760
gagttgctct tgcccggcgt caatacggga taataccgcg ccacatagca gaactttaaa     2820
```

```
agtgctcatc attggaaaac gttcttcggg gcgaaaactc tcaaggatct taccgctgtt     2880
gagatccagt tcgatgtaac ccactcgtgc acccaactga tcttcagcat cttttacttt     2940
caccagcgtt tctgggtgag caaaaacagg aaggcaaaat gccgcaaaaa agggaataag     3000
ggcgacacgg aaatgttgaa tactcatact cttccttttt caatattatt gaagcattta     3060
tcagggttat tgtctcatga gcggatacat atttgaatgt atttagaaaa ataaacaaat     3120
aggggttccg cgcacatttc cccgaaaagt gccacctaaa ttgtaagcgt taatattttg     3180
ttaaaattcg cgttaaattt ttgttaaatc agctcatttt ttaaccaata ggccgaaatc     3240
ggcaaaatcc cttataaatc aaaagaatag accgagatag ggttgagtgt tgttccagtt     3300
tggaacaaga gtccactatt aaagaacgtg gactccaacg tcaaagggcg aaaaaccgtc     3360
tatcagggcg atggcccact acgtgaacca tcaccctaat caagtttttt ggggtcgagg     3420
tgccgtaaag cactaaatcg gaaccctaaa gggagccccc gatttagagc ttgacgggga     3480
aagccggcga acgtggcgag aaaggaaggg aagaaagcga aaggagcggg cgctagggcg     3540
ctggcaagtg tagcggtcac gctgcgcgta accaccacac ccgccgcgct taatgcgccg     3600
ctacagggcg cgtcccattc gccattcagg ctgcgcaact gttgggaagg gcgatcggtg     3660
cgggcctctt cgctattacg ccagctggcg aaagggggat gtgctgcaag gcgattaagt     3720
tgggtaacgc cagggttttc ccagtcacga cgttgtaaaa cgacggccag tgagcgcgcg     3780
taatacgact cactataggg cgaattgggt accgggcccc ccctcgaggt cgacgatgta     3840
ggtcacggtc tcgaagccgc ggtgcgggtg ccagggcgtg cccttgggct ccccgggcgc     3900
gtactccacc tcacccatct ggtccatcat gatgaacggg tcgaggtggc ggtagttgat     3960
cccggcgaac gcgcggcgca ccgggaagcc ctcgccctcg aaaccgctgg gcgcggtggt     4020
cacggtgagc acgggacgtg cgacggcgtc ggcgggtgcg gatacgcggg gcagcgtcag     4080
cgggttctcg acggtcacgg cgggcatgtc gacggtatcg ataagcttgg gccccccctc     4140
gaggttccca caatggttaa ttcgagctcg cccggggatc taattcaatt agagactaat     4200
tcaattagag ctaattcaat taggatccaa gcttatcgat ttcgaaccct cgaccgccgg     4260
agtataaata gaggcgcttc gtctacggag cgacaattca attcaaacaa gcaaagtgaa     4320
cacgtcgcta agcgaaagct aagcaaataa acaagcgcag ctgaacaagc taaacaatcg     4380
gggtaccgct agagtcgatc ccaccccacc caagaagaag cgcaaaccgg taccatggcc     4440
tcctccgaga acgtcatcac cgagttcatg cgcttcaagg tgcgcatgga gggcaccgtg     4500
aacggccacg agttcgagat cgagggcgag ggcgagggcc gcccctacga gggccacaac     4560
accgtgaagc tgaaggtgac caagggcggc cccctgccct tcgcctggga catcctgtcc     4620
ccccagttcc agtacggctc caaggtgtac gtgaagcacc ccgccgacat ccccgactac     4680
```

aagaagctgt ccttccccga gggcttcaag tgggagcgcg tgatgaactt cgaggacggc 4740

ggcgtggcga ccgtgaccca ggactcctcc ctgcaggacg gctgcttcat ctacaaggtg 4800

aagttcatcg gcgtgaactt cccctccgac ggccccgtga tgcagaagaa gaccatgggc 4860

tgggaggcct ccaccgagcg cctgtacccc cgcgacggcg tgctgaaggg cgagacccac 4920

aaggccctga agctgaagga cggcggccac tacctggtgg agttcaagtc catctacatg 4980

gccaagaagc ccgtgcagct gcccggctac tactacgtgg acgccaagct ggacatcacc 5040

tcccacaacg aggactacac catcgtggag cagtacgagc gcaccgaggg ccgccaccac 5100

ctgttcctgt gatgatcata atcagccata ccacatttgt agaggtttta cttgctttaa 5160

aaaacctccc acacctcccc ctgaacctga aacataaaat gaatgcaatt gttgttgtta 5220

acttgtttat tgcagcttat aatggttaca aataaagcaa tagcatcaca aatttcacaa 5280

ataaagcatt tttttcactg cattctagtt gtggtttgtc caaactcatc aatgtatctt 5340

aacgcgagtt aattaaggcc gctcatttat cagcgcttta aatttgcgca tgctagttta 5400

atacaccttt cgcagcaagt agtatagctt gttgcacagc agacatcggg aacgggttgg 5460

gttattttct tgagcgtgac ggaacagaat ctcatgaaag gcctgcacca gatggtagcg 5520

gttgtggtga aggctgactt gcgtcatcgt cggagtcagt ggaggagttg gtggaattga 5580

ctccgttgga cttgttggcg acggtggtgg cgaactgaat tggttctgat tttgctgttg 5640

ttgcattaaa atctgctgct gctgttgcat catttgcaac tgatactgct tctcgatttc 5700

atcatcgatg gcgggaatgt agaatgcgat tgccatggtg ggcgat 5746

<210> 145
<211> 15121
<212> DNA
<213> artificial

<220>
<223> LA3596 plasmid sequence

<400> 145
gggcggccgt ttttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600
agttacatga cgagccctaa tgagccgtcg gtggtctata aactgtgcct tacaaatact 660
tgcaactctt ctcgttttga agtcagcaga gttattgcta attgctaatt gctaattgct 720
tttaactgat ttcttcgaaa ttggtgctat gtttatggcg ctattaacaa gtatgaatgt 780
caggtttaac caggggatgc ttaattgtgt tctcaacttc aaaggcagaa atgtttactc 840
ttgaccatgg gtttaggtat aatgttatca agctcctcga gttaacgtta cgttaacgtt 900
aacgttcgag gtcgactcta gacaccggtg ctacccgcca tactcatcga tgcccagcgc 960
gtcggtgaac atttgctcga actcgaagtc ggccatgtcc agggcgccgt acggggcgct 1020
atcgtggggc gtgaagcccg gtcccgggct atctccatcg cccagcatat ccaggtcgaa 1080
atcgtccagg gcgtcggcgt gggccattgc cacatcctct ccatccaggt gcagctcgtc 1140
gcccaggctc acatcggtcg gcggggcggt gctcaggcgg cgcgtgtgtc cggcgggcag 1200
gaagctcagg cgggggcgg ccaggccggc ttcctccggg gcatcgtcat ccggcaggtc 1260
cagcagtccc tcgatggtgc tgccatagtt gttcttggta cgggcgcggc tgtaggcgct 1320
gccgctctcg cacttcagct gcttttccag gccgcagatg atcagctcca ggccgaacag 1380
gaaggccggc tcggcgccct ggtgatcgaa cagctcgatg gcctggcgca gcagcggcgg 1440
catgctatcg gtggtcgggg tctcgcgctc ctccttggcc acctggtgct cctgatcctc 1500
cagcacacag cccagggtga agtggcccac ggcgctcagg gcgtacaggg cgttctccag 1560
gctgaagccc tgctggcaca ggaaggccag ctggttctcc agggtctcgt actgcttctc 1620
ggtcgggcgg gtgcccaggt gcaccttggc gccatcgcgg tgcgacagca gggcgcagcg 1680
gaagctcttg gcgttgttgc gcaggaaatc ctgccagctc tcgccctcca gcgggcagaa 1740
gtgggtgtgg tggcgatcca gcatttcgat ggccagggcg tccagcaggg cgcgcttgtt 1800
cttcacgtgc cagtacaggg tcggctgttc cacgcccagc ttctgggcca gcttgcgggt 1860
ggtcaggccc tcgataccaa cttcgttcag cagctccagg gcgctgttga tcaccttgct 1920
cttgtccagg cggctgacct gtgaatacgg ttaatgtcac tattagtgat ttataaaaat 1980
aaatttgatt tatatatcaa caatttttca tcgcagcctt cagctttttg ttgaataatt 2040
ataatgatat tttttacgat tcaaatcatt taattgttac tcaacgaaat aagtttaatt 2100
caaattttaa aacaagatta tatattaaga ttagaataag aaagaacttt gttagattat 2160
ttaattaaaa agattaaaat ttaagtctcc agtcactatt taaagatcat ctttcaaacg 2220
ttaaagtgaa ttcaaacgag acgttcaaat ttcgattaaa cagtaattaa ctctaaattt 2280
ctatcacgaa ttaagttatt gaatatgaag gtttatattt atttacatca tctaataggt 2340
ttgagttgat tgttgtaatc cgcatgtgcc agaagatatc aatttccaaa ttgtccgagt 2400

tcatggaatg ttgattgttg tttgtgttgc tttgtaattg ttgcagggag tatttatggt 2460
ttgttgattg tagtataagg ctgtttctaa aggctagaaa ataattttat ttatttgaaa 2520
ataagtaaat atacataata ttactaacaa taggtcgtcc tattttttga tattctgcac 2580
aaatttttaa aacacaaaga ttgcaatact tttagacact aatactgcac actctgaaaa 2640
attattaaat tatttttaaa aacttacctt aatactttag agaaaaatat tataccgcac 2700
ctttctactt tatactcact ttattatacc agttgcatgt tgattgtagt tctttgacaa 2760
gaaaatattc catattgctc caaattatct tggtaagttg attggtgcgt catttgagca 2820
agctaacacc ttgtctcatt taagttcgcc tcaagatctc atagcatttt taaatatcac 2880
tatatttagt aagtaattag aattaccatg gtggtttgct agcggtacct gcagattgtt 2940
tagcttgttc agctgcgctt gtttatttgc ttagctttcg cttagcgacg tgttcacttt 3000
gcttgtttga attgaattgt cgctccgtag acgaagcgcc tctatttata ctccggcgct 3060
cggtccgact ctctatcact gatagggagt attgtcctct ctatcactga tagggaatgc 3120
tatgttctct atcactgata gggaagttga tagtctctat cactgatagg gagtggtaat 3180
ttctctatca ctgataggga ttagtgatgt ctctatcact gatagggatt ggaatattct 3240
ctatcactga tagggagtgg taatatctct atcactgata gggactggag ttttctctat 3300
cactgatagg gacacgctga ctctctatca ctgataggga taagcttact ctctatcact 3360
gatagggagt attgtcctct ctatcactga tagggaatgc tatgttctct atcactgata 3420
gggaagttga tagtctctat cactgatagg gagtggtaat ttctctatca ctgataggga 3480
ttagtgatgt ctctatcact gatagggatt ggaatattct ctatcactga tagggagtgg 3540
taatatctct atcactgata gggactggag ttttctctat cactgatagg gacacgctga 3600
ctctctatca ctgataggga taagcggccg catggtaccc attgcttgtc atttattaat 3660
ttggatgatg tcatttgttt ttaaaattga actggcttta cgagtagaat tctacgcgta 3720
aaacacaatc aagtatgagt cataatctga tgtcatgttt tgtacacggc tcataaccga 3780
actggcttta cgagtagaat tctacttgta atgcacgatc agtggatgat gtcatttgtt 3840
tttcaaatcg agatgatgtc atgttttgca cacggctcat aaactcgctt tacgagtaga 3900
attctacgtg taacgcacga tcgattgatg agtcatttgt tttgcaatat gatatcatac 3960
aatatgactc atttgttttt caaaaccgaa cttgatttac gggtagaatt ctacttgtaa 4020
agcacaatca aaaagatgat gtcatttgtt tttcaaaact gaactcgctt tacgagtaga 4080
attctacgtg taaaacacaa tcaagaaatg atgtcatttg ttataaaaat aaaagctgat 4140
gtcatgtttt gcacatggct cataactaaa ctcgctttac gggtagaatt ctacgcgtaa 4200
aacatgattg ataattaaat aattcatttg caagctatac gttaaatcaa acggacgctc 4260
gaggttgcac aacactatta tcgatttgca gttcgggaca taaatgttta aatatatcga 4320

tgtctttgtg atgcgcgcga catttttgta ggttattgat aaaatgaacg gatacgttgc 4380
ccgacattat cattaaatcc ttggcgtaga atttgtcggg tccattgtcc gtgtgcgcta 4440
gcatgcccgt aacggacctc gtacttttgg cttcaaaggt tttgcgcaca gacaaaatgt 4500
gccacacttg cagctctgca tgtgtgcgcg ttaccacaaa tcccaacggc gcagtgtact 4560
tgttgtatgc aaataaatct cgataaaggc gcggcgcgcg aatgcagctg atcacgtacg 4620
ctcctcgtgt tccgttcaag gacggtgtta tcgacctcag attaatgttt atcggccgac 4680
tgttttcgta tccgctcacc aaacgcgttt ttgcattaac attgtatgtc ggcggatgtt 4740
ctatatctaa tttgaataaa taaacgataa ccgcgttggt tttagagggc ataataaaag 4800
aaatattgtt atcgtgttcg ccattagggc agtataaatt gacgttcatg ttggatattg 4860
tttcagttgc aagttgacac tggcggcgac aagcaattct aattggggta agttttcccg 4920
ttcttttctg ggttcttccc ttttgctcat ccttgctgca ctaccttcag gtgcaagttg 4980
agattcaggc caccatggga gatcccaccc cacccaagaa gaagcgcaaa ccggtcgcca 5040
ccatggagag cgacgagagc ggcctgcccg ccatggagat cgagtgccgc atcaccggca 5100
ccctgaacgg cgtggagttc gagctggtgg gcggcggaga gggcaccccc gagcagggcc 5160
gcatgaccaa caagatgaag agcaccaaag gcgccctgac cttcagcccc tacctgctga 5220
gccacgtgat gggctacggc ttctaccact tcggcaccta ccccagcggc tacgagaacc 5280
ccttcctgca cgccatcaac aacggcggct acaccaacac ccgcatcgag aagtacgagg 5340
acggcggcgt gctgcacgtg agcttcagct accgctacga ggccggccgc gtgatcggcg 5400
acttcaaggt gatgggcacc ggcttccccg aggacagcgt gatcttcacc gacaagatca 5460
tccgcagcaa cgccaccgtg gagcacctgc accccatggg cgataacgat ctggatggca 5520
gcttcacccg caccttcagc ctgcgcgacg gcggctacta cagctccgtg gtggacagcc 5580
acatgcactt caagagcgcc atccacccca gcatcctgca gaacgggggc cccatgttcg 5640
ccttccgccg cgtggaggag gatcacagca acaccgagct gggcatcgtg gagtaccagc 5700
acgccttcaa gaccccggat gcagatgccg gtgaagaaag atctcgaccc aagaaaaagc 5760
ggaaggtgga ggacccgtct ggaggcggtg gatccggcgg tggaggcatg cagatctttg 5820
tgaagacttt gaccggaaag accatcaccc tcgaggtaga gccatcggac accattgaga 5880
atgtaaaggc caagattcag gataaggagg gaatcccccc agatcagcag cgtctgatct 5940
tcgctggtaa ttttaaaagc atattttttt ctttgaaatt cataagttat caattatcga 6000
tggaaatgta ttctatggag aacgttttac ccgatgaatg ggtgcaaaaa ttattttacc 6060
ttcaaatcta caatcaacac acgctaactt ttgtgacttg atcaactctc acctggaaaa 6120
gcaaccaact acaatcaaca ttctatggga taatcgacaa gtgagtaaaa ttatagccgg 6180

acctcttagt acagtgtatt taaaagggga ataatattct atcaatagga ataaaaataa 6240
ggtcagcagc catgactttt ccatcatttt gaatatacct tatttgtttc gggattaatt 6300
gggggtcgga aatcctcttg aattcagaaa cgggaaccgg aggaaggtgc cggtctttca 6360
gaaagctgtg aaaaatacca acatttctgc tgccaagagc tcaataagaa gtttcaaaaa 6420
ttgtcttgga tgttgcagct gtggctgcta agtaataaga catctattag tatctagatt 6480
tgttagacca tttaacatag tgttttaaac gatgggggtta atagatgagg gttaagaagc 6540
tagttatatt actgttgctg taacgccttc aattgtcggt tacagagcaa acattattga 6600
atgttaatgt aaagagttta tttgttttct agtaaacata tagcgattgg ttagtaatca 6660
ctaatagaaa tttttcataa gtatcaaaaa agtaaacctc tttttcagtc tatgtaataa 6720
gtaaaccaag gaaagggaaa atatctacaa tcaacaagcc attgttgcag caacaaagca 6780
actgaaacta caatcaacat tcaataaact tgggtaattt ggaatttaat tctctgggac 6840
acctgtggat tacaacaatc aactcgaaac ttattataca atgtaaataa aaattgatat 6900
gcatacatga agatcaagtg aaattccatt tagaatcaat ttttttcgaa tattaagttt 6960
cttgctttaa tttatctgaa agtaaataga cattccaaat tcaagttaac aaattaataa 7020
tgaattgact agtgattttt aagagaaaaa gataagattt aaaaaaggaa agcctttctt 7080
gataaatttt tgaaccactt tatgccgttt caatcataaa aacttttaag aacacatgac 7140
tggtaaaatt aatttaaaac aaatttaaat tttcaacgta acattcaaca aaaatggtga 7200
aaactatcac ggaaattgtt aatattaata tgtcccaaaa atagcctttg tatgtatatg 7260
atactaatcc atacatctat ggtatctata ggtcgccaac tggaagacgg acgcaccctg 7320
tccgattaca acatccagaa ggagtccacc cttcacttgg tccttcgtct ccgcggtggc 7380
atgcagatcg gggatcccac cccacccaag aagaagcgca aaccggtcgc caccatggcc 7440
tcctccgaga acgtcatcac cgagttcatg cgcttcaagg tgcgcatgga gggcaccgtg 7500
aacggccacg agttcgagat cgagggcgag ggcgagggcc gcccctacga gggccacaac 7560
accgtgaagc tgaaggtgac caagggcggc cccctgccct tcgcctggga catcctgtcc 7620
ccccagttcc agtacggctc caaggtgtac gtgaagcacc ccgccgacat ccccgactac 7680
aagaagctgt ccttccccga gggcttcaag tgggagcgcg tgatgaactt cgaggacggc 7740
ggcgtggcga ccgtgaccca ggactcctcc ctgcaggacg gctgcttcat ctacaaggtg 7800
aagttcatcg gcgtgaactt cccctccgac ggccccgtga tgcagaagaa gaccatgggc 7860
tgggaggcct ccaccgagcg cctgtacccc cgcgacggcg tgctgaaggg cgagacccac 7920
aaggccctga agctgaagga cggcggccac tacctggtgg agttcaagtc catctacatg 7980
gccaagaagc ccgtgcagct gcccggctac tactacgtgg acgccaagct ggacatcacc 8040
tcccacaacg aggactacac catcgtggag cagtacgagc gcaccgaggg ccgccaccac 8100

ctgttcctga gatctcgacc caagaaaaag cggaaggtgg aggacccgta agatccaccg 8160

gatctagata actgatcata atcagccata ccacatttgt agaggtttta cttgctttaa 8220

aaaacctccc acacctcccc ctgaacctga aacataaaat gaatgcaatt gttgttgtta 8280

acttgtttat tgcagcttat aatggttaca aataaagcaa tagcatcaca aatttcacaa 8340

ataaagcatt tttttcactg cattctagtt gtggtttgtc caaactcatc aatgtatctt 8400

aacgcgagtt aattaacacc gaaatcgtaa ttcacggcat cattacaaaa tattttgacg 8460

ttttggacct cgtccctaat gacaccataa cggtggcctt gaagtatatt taaccctaga 8520

aagatagtct gcgtaaaatt gacgcatgca ttcttgaaat attgctctct ctttctaaat 8580

agcgcgaatc cgtcgctgtg catttaggac atctcagtcg ccgcttggag ctcccgtgag 8640

gcgtgcttgt caatgcggta agtgtcactg attttgaact ataacgaccg cgtgagtcaa 8700

aatgacgcat gattatcttt tacgtgactt ttaagattta actcatacga taattatatt 8760

gttatttcat gttctactta cgtgataact tattatatat atattttctt gttatagata 8820

tcgtgactaa tatataataa aatgggtagt tctttagacg atgagcatat cctctctgct 8880

cttctgcaaa gcgatgacga gcttgttggt gaggattctg acagtgaaat atcagatcac 8940

gtaagtgaag atgacgtcca ggaaatctgg ccggccgcaa ccattgtggg aaccgtgcga 9000

tcaaacaaac gcgagatacc ggaagtactg aaaaacagtc gctccaggcc agtgggaaca 9060

tcgatgtttt gttttgacgg acccccttact ctcgtctcat ataaaccgaa gccagctaag 9120

atggtatact tattatcatc ttgtgatgag gatgcttcta tcaacgaaag taccggtaaa 9180

ccgcaaatgg ttatgtatta taatcaaact aaaggcggag tggacacgct agaccaaatg 9240

tgttctgtga tgacctgcag taggaagacg aataggtggc ctatggcatt attgtacgga 9300

atgataaaca ttgcctgcat aaattctttt attatataca gccataatgt cagtagcaag 9360

ggagaaaagg tccaaagtcg caaaaaattt atgagaaacc tttacatgag cctgacgtca 9420

tcgtttatgc gtaagcgttt agaagctcct actttgaaga gatatttgcg cgataatatc 9480

tctaatattt tgccaaatga agtgcctggt acatcagatg acagtactga agagccagta 9540

atgaaaaaac gtacttactg tacttactgc ccctctaaaa taaggcgaaa ggcaaatgca 9600

tcgtgcaaaa aatgcaaaaa agttatttgt cgagagcata atattgatat gtgccaaagt 9660

tgtttctgac tgactaataa gtataatttg tttctattat gtataagtta agctaattac 9720

ttattttata atacaacatg actgttttta aagtacaaaa taagtttatt tttgtaaaag 9780

agagaatgtt taaaagtttt gttactttat agaagaaatt ttgagttttt gttttttttt 9840

aataaataaa taaacataaa taaattgttt gttgaattta ttattagtat gtaagtgtaa 9900

atataataaa acttaatatc tattcaaatt aataaataaa cctcgatata cagaccgata 9960

aaacacatgc gtcaatttta cgcatgatta tctttaacgt acgtcacaat atgattatct 10020
ttctagggtt aaataatagt ttctaatttt tttattattc agcctgctgt cgtgaatacc 10080
gtatatctca acgctgtctg tgagattgtc gtattctagc ctttttagtt tttcgctcat 10140
cgacttgata ttgtccgaca cattttcgtc gatttgcgtt ttgatcaaag acttgagcag 10200
agacacgtta atcaactgtt caaattgatc catattaacg atatcaaccc gatgcgtata 10260
tggtgcgtaa aatatatttt ttaaccctct tatactttgc actctgcgtt aatacgcgtt 10320
cgtgtacaga cgtaatcatg ttttcttttt tggataaaac tcctactgag tttgacctca 10380
tattagaccc tcacaagttg caaaacgtgg catttttttac caatgaagaa tttaaagtta 10440
ttttaaaaaa tttcatcaca gatttaaaga agaaccaaaa attaaattat ttcaacagtt 10500
taatcgacca gttaatcaac gtgtacacag acgcgtcggc aaaaaacacg cagcccgacg 10560
tgttggctaa aattattaaa tcaacttgtg ttatagtcac ggatttgccg tccaacgtgt 10620
tcctcaaaaa gttgaagacc aacaagttta cggacactat taattatttg attttgcccc 10680
acttcatttt gtgggatcac aattttgtta tattttaaac aaagcttggc actggccgtc 10740
gttttacaac gtcgtgactg ggaaaaccct ggcgttaccc aacttaatcg ccttgcagca 10800
catccccctt tcgccagctg gcgtaatagc gaagaggccc gcaccgatcg cccttcccaa 10860
cagttgcgca gcctgaatgg cgaatggcgc ctgatgcggt attttctcct tacgcatctg 10920
tgcggtattt cacaccgcat atggtgcact ctcagtacaa tctgctctga tgccgcatag 10980
ttaagccagc cccgacaccc gccaacaccc gctgacgcgc cctgacgggc ttgtctgctc 11040
ccggcatccg cttacagaca agctgtgacc gtctccggga gctgcatgtg tcagaggttt 11100
tcaccgtcat caccgaaacg cgcgagacga aagggcctcg tgatacgcct atttttatag 11160
gttaatgtca tgataataat ggtttcttag acgtcaggtg gcacttttcg gggaaatgtg 11220
cgcggaaccc ctatttgttt atttttctaa atacattcaa atatgtatcc gctcatgaga 11280
caataaccct gataaatgct tcaataatat tgaaaaagga agagtatgag tattcaacat 11340
ttccgtgtcg cccttattcc cttttttgcg gcattttgcc ttcctgtttt tgctcaccca 11400
gaaacgctgg tgaaagtaaa agatgctgaa gatcagttgg gtgcacgagt gggttacatc 11460
gaactggatc tcaacagcgg taagatcctt gagagttttc gccccgaaga acgttttcca 11520
atgatgagca cttttaaagt tctgctatgt ggcgcggtat tatcccgtat tgacgccggg 11580
caagagcaac tcggtcgccg catacactat tctcagaatg acttggttga gtactcacca 11640
gtcacagaaa agcatcttac ggatggcatg acagtaagag aattatgcag tgctgccata 11700
accatgagtg ataacactgc ggccaactta cttctgacaa cgatcggagg accgaaggag 11760
ctaaccgctt ttttgcacaa catgggggat catgtaactc gccttgatcg ttgggaaccg 11820
gagctgaatg aagccatacc aaacgacgag cgtgacacca cgatgcctgt agcaatggca 11880

acaacgttgc gcaaactatt aactggcgaa ctacttactc tagcttcccg gcaacaatta 11940
atagactgga tggaggcgga taaagttgca ggaccacttc tgcgctcggc ccttccggct 12000
ggctggttta ttgctgataa atctggagcc ggtgagcgtg ggtctcgcgg tatcattgca 12060
gcactggggc cagatggtaa gccctcccgt atcgtagtta tctacacgac ggggagtcag 12120
gcaactatgg atgaacgaaa tagacagatc gctgagatag gtgcctcact gattaagcat 12180
tggtaactgt cagaccaagt ttactcatat atactttaga ttgatttaaa acttcatttt 12240
taatttaaaa ggatctaggt gaagatcctt tttgataatc tcatgaccaa aatcccttaa 12300
cgtgagtttt cgttccactg agcgtcagac cccgtagaaa agatcaaagg atcttcttga 12360
gatccttttt ttctgcgcgt aatctgctgc ttgcaaacaa aaaaaccacc gctaccagcg 12420
gtggtttgtt tgccggatca agagctacca actctttttc cgaaggtaac tggcttcagc 12480
agagcgcaga taccaaatac tgtccttcta gtgtagccgt agttaggcca ccacttcaag 12540
aactctgtag caccgcctac atacctcgct ctgctaatcc tgttaccagt ggctgctgcc 12600
agtggcgata agtcgtgtct taccgggttg gactcaagac gatagttacc ggataaggcg 12660
cagcggtcgg gctgaacggg gggttcgtgc acacagccca gcttggagcg aacgacctac 12720
accgaactga gatacctaca gcgtgagcat tgagaaagcg ccacgcttcc cgaagggaga 12780
aaggcggaca ggtatccggt aagcggcagg gtcggaacag gagagcgcac gagggagctt 12840
ccagggggaa acgcctggta tctttatagt cctgtcgggt ttcgccacct ctgacttgag 12900
cgtcgatttt tgtgatgctc gtcagggggg cggagcctat ggaaaaacgc cagcaacgcg 12960
gcctttttac ggttcctggc cttttgctgg ccttttgctc acatgttctt tcctgcgtta 13020
tcccctgatt ctgtggataa ccgtattacc gcctttgagt gagctgatac cgctcgccgc 13080
agccgaacga ccgagcgcag cgagtcagtg agcgaggaag cggaagagcg cccaatacgc 13140
aaaccgcctc tccccgcgcg ttggccgatt cattaatgca gctggcacga caggtttccc 13200
gactggaaag cgggcagtga gcgcaacgca attaatgtga gttagctcac tcattaggca 13260
ccccaggctt tacactttat gcttccggct cgtatgttgt gtggaattgt gagcggataa 13320
caatttcaca caggaaacag ctatgaccat gattacgaat ttcgacctgc aggcatgcaa 13380
gcttgcatgc ctgcaggtcg acgctcgcgc gacttggttt gccattcttt agcgcgcgtc 13440
gcgtcacaca gcttggccac aatgtggttt ttgtcaaacg aagattctat gacgtgttta 13500
aagtttaggt cgagtaaagc gcaaatcttt tttaacccta gaaagatagt ctgcgtaaaa 13560
ttgacgcatg cattcttgaa atattgctct ctctttctaa atagcgcgaa tccgtcgctg 13620
tgcatttagg acatctcagt cgccgcttgg agctcccgtg aggcgtgctt gtcaatgcgg 13680
taagtgtcac tgattttgaa ctataacgac cgcgtgagtc aaaatgacgc atgattatct 13740

tttacgtgac ttttaagatt taactcatac gataattata ttgttatttc atgttctact 13800

tacgtgataa cttattatat atatattttc ttgttataga tatcgtgact aatatataat 13860

aaaatgggta gttctttaga cgatgagcat atcctctctg ctcttctgca aagcgatgac 13920

gagcttgttg gtgaggattc tgacagtgaa atatcagatc acgtaagtga agatgacgtc 13980

cagagcgata cagaagaagc gtttatagat gaggtacatg aagtgcagcc aacgtcaagc 14040

ggtagtgaaa tattagacga acaaaatgtt attgaacaac caggttcttc attggcttct 14100

aacagaatct tgaccttgcc acagaggact attagaggta agaataaaca ttgttggtca 14160

acttcaaagt ccacgaggcg tagccgagtc tctgcactga acattgtcag atcggcccgg 14220

cggagtggac acgctagacc aaatgtgttc tgtgatgacc tgcagtagga agacgaatag 14280

gtggcctatg gcattattgt acggaatgat aaacattgcc tgcataaatt cttttattat 14340

atacagccat aatgtcagta gcaagggaga aaaggtccaa agtcgcaaaa aatttatgag 14400

aaacctttac atgagcctga cgtcatcgtt tatgcgtaag cgtttagaag ctcctacttt 14460

gaagagatat ttgcgcgata atatctctaa tattttgcca aatgaagtgc ctggtacatc 14520

agatgacagt actgaagagc cagtaatgaa aaaacgtact tactgtactt actgcccctc 14580

taaaataagg cgaaaggcaa atgcatcgtg caaaaaatgc aaaaaagtta tttgtcgaga 14640

gcataatatt gatatgtgcc aaagttgttt ctgactgact aataagtata atttgtttct 14700

attatgtata agttaagcta attacttatt ttataataca acatgactgt ttttaaagta 14760

caaaataagt ttatttttgt aaaagagaga atgtttaaaa gttttgttac tttatagaag 14820

aaattttgag tttttgtttt tttttaataa ataaataaac ataaataaat tgtttgttga 14880

atttattatt agtatgtaag tgtaaatata ataaaactta atatctattc aaattaataa 14940

ataaacctcg atatacagac cgataaaaca catgcgtcaa ttttacgcat gattatcttt 15000

aacgtacgtc acaatatgat tatctttcta gggttaaaat gaatgtaagc actttattaa 15060

cgaaatcttt gggaatattt cgctcatcag cattttattt gagcaggagt ccgagatgcc 15120

c 15121

<210> 146
<211> 533
<212> DNA
<213> artificial

<220>
<223> 146 PBW dsx fragment (Fig 6)

<400> 146
gtccaatcga tcacaatgta tcacaacgtt gcgaattcag tttcacaatc acacgcaaca 60

aacrcgrcac gttacaaatt agttactttg aatcgatcga ttatgatgcg gccgactcar 120

cggcccccgg cagcactaac cagtagtgat ttccactttg cagtgaccgg accaaaactt 180

cgaaattcga attgtaaagt gacagttcat ttcccgccaa gtgttgtgcc agtgtcatgt 240

cgatatttat tttattttct tttttgtagg aaaatgctga gcgaaattaa taatataagt 300

ggtgtactat cgtcgtccat gaagttattt tgcgaatgat actttgtttt gtatgtgctg 360

tgtgttgtgt ggacttttgc tgtgcgttgc tgtttgcgat ggaaggacta ttgtgtcgtc 420

gccacgctgg actattcggt gagtggtaga ataatatttt atctatttca tcgcggtaca 480

attgactttt tattactact cactgctatg gaggaatctc aggaacatcg taa 533

<210> 147
<211> 611
<212> DNA
<213> artificial

<220>
<223> Bombyx-dsx fragment (Fig 6)

<400> 147
gcgattattt aattctatat atttttcaaa ttcagtttct attccactaa caatgtacac 60

tacacgtaca catacacaca acaaaatagt gaatcgataa attagtgtgt cataacacat 120

taacaacatt gttacacacc cacacataca aatttgctaa gttgatagtc gaataatcgg 180

aatggttcgc atcacactac taaccagtcg tgatttccac tttacagtga ccggacgaag 240

gtggagaaat tcgaaattta aatataaaag tgacaattcg aatttccacg cgcgcgctct 300

agtgatgtgc cagtgtgtga atatcaatat tattttttat tttctttttt gtaggaaaat 360

gctggaaatt aataatataa gtggtgtact gtcttcgtca atgaagttat tttgcgaatg 420

atacttagtt ttacaagtgc cgtggtgtgt gttgacactt gctgtgcgat gctgtgcgaa 480

tttcaacgga aatatttgtt gtcgtaacat tggatctatg ggtaagttta gtataataac 540

tttactctgt tcacattagt gaaacataca tttgtaaaat ttgtgtttta ctaatgtgaa 600

atttattttt g 611

<210> 148
<211> 570
<212> DNA
<213> artificial

<220>
<223> codling-dsx fragment (Fig 6)

<400> 148
ttacaaacaa tgtacggagc tacaacgttg caagttcggt ccccacacaa cacaatgtgt 60

cataacacat taacaacatt gttacacacc cacacataca aatttgctaa gttgataaaa 120

gagtggtgtg tccgacgaat cagaacatca ctaacccagt cgtgatttca tttccacagt 180

gaccggacga aggtggagaa gttcgaaatt taaaaaaagt gaccacattt tatttaatag 240

tgatgtgcaa gtgatactat ttttattttg tttttctttt gtaggaaaat gctgagcgaa 300

ataaataatt ttagtggtgt gctatcgtca tcgatgaagt tgttttgcga atgatactat 360

gttcttcaag tgctgtgttt tgtggactgt ggggtgactg ttcctgtaaa taagcttcgt 420

tggacattgt gtctcacaca tcggatctca tggtaagtgc tagtgctagc atyrmaactt 480

aactctctga gcgaattcct ttgactctaa agtcacacgr acagccatac aatcaaagct 540

acgctctaat tttaagatga cawtctgtaa 570

<210> 149
<211> 4389
<212> DNA
<213> artificial

<220>
<223> DSX Minigene1 rom construct LA3491

<400> 149
acgacgaact tgtcaaacga tctcaatggc tcctggagaa gctgcgatac ccctgggaga 60

tgatgcccct gatgtacgtg atactgaaag gcgccgacgg agacgtcaat aaagcgcgcc 120

aacggattga cgaaggtatg gggttctta ccggttggga ctgtttccga ggtatcgatc 180

gggtgtcact cacttcctgg gtgctcccat tttgtaactg ctaacgctta ttattgagtt 240

tcaggacatc tgggatcttc ggtcgacgga gtctattccc aacagtgccc tggatcaaac 300

actgccatca tgcagtttcc gtagcctgtt gggctacgct ccccgacttg acatccccca 360

ttcttatcaa acaacaactc aaggcctgag acaacgagtg gtggaatttg cgcacgaagt 420

cattggtttg tcctggtaaa agttaaaagg gttaactgga gggttaattg acacggtttc 480

aactgatggc cttattgaca cacggatgaa agacttgcac gcttgacctt ctgtctgtac 540

taataaaagt tacgttggct gggttttggg gtcataatgg ccccaaaatc gaatcgtcat 600

aacttcttga aatacaactc acgtttaaga ccattcaaga gtattagatc atcgtctata 660

atagcagatt tgaaatttac ttcacatttc ggtattgcag tgccccttgc ttccacaatg 720

gaattaggtc ggtggtgcgt cgatcgtcgc aagtttatcg ttaaacagtc aataaaatga 780

gcattttata tcgtgataca tatgagaaga tagaggtttc aattaaaaca aatccacatg 840

gtgtcgctaa taaaattgtg cattttaagc gagttatatc ctctgatcaa gataaaatag 900

aaaattcgat ttttgaatat tcaattataa gagcctgaat aactacaaca tgtagtgaat 960

cgaaactgat ttatgacggt ttgtgaaggt tacacgtcct aagcatttgg attcaagaaa 1020

agcaagagat atgacgaatg taaactttat cgtatcaatg aagtaactag cgtccagaac 1080

agtacaaacc aacatcgtac cgtcgtattc cactccggtc gttgcaatat ctctaggtcc 1140

accgaaaaac actcatgacc aagatcgtgt cgtcgatctt ggtccaccga aacaccgatg 1200

tccatatcgt ttcgtcgaac ttggaccaac gattcatgca actgatgaca acgcggcccc 1260

cgggtcgtac caatatccga aaaatccaac tgttcttctc tgcctcgcag gtcaagccgt 1320
ggtcaatgaa tactcacgat tgcacaatct gaacatgttc gacggtgtag agttgcgcag 1380
tacgacgcgc cagtccggat gatagacttt ttacacgatc agcacgaccc actgcgctgc 1440
ggcaaaggtc gaaccgaaac aagaataaac cacgaagatc agatcgattc gacggaagaa 1500
gcaatcgaat gcaaagaaga atcggaacga agaaaactct aaagcatcgc atatttacaa 1560
agcataacgg aaaacccgca agttcaaact agtgattagt gtaagatgaa gcaaagcaga 1620
aatgtagtat ctagattttt cgacgttagt ttacaaagat aaaaaatgag gttggacata 1680
caatcgtggg tattcgtctg agttcgtcac aactgcaccg gaaactgtga aacagaatag 1740
agccaacctg tgcgcggaga atgttgaggt cattataagc ttccttagca tccacgggtg 1800
aaagtcgatc gacggaagcc tgcaagactc tgtcgatggg ctttcgtcct agaagaataa 1860
gattaaacct gaaatgtatt ctcccgtgga atggtttcat ttgagtaatt ctgtatcttc 1920
tccttcccaa ttccacgaac gcgacgaact ctaatacaaa caacataatg accacagtgc 1980
aaatgctgtt taacgataat agcgacatgc agccattctg ggctaccac gtgtagctct 2040
acttgtgaga cagcgttcct aaagagtgtg aaagtgcaaa caagtgatga aaccaatagt 2100
gcaaagcaag tttagaggga aaatttaaaa aatgcaaaac agcagtagta cttaactttt 2160
aagattgtgt ttcgaaagcc gaagtgaggc tgttccatct gccaccggaa aaaaacgacg 2220
acagcagaat catcaacaag caacatccat ccgaaaaaat ccgggaaacc ggatcttcaa 2280
ccaaccatcc tacaatctac aaaccagaga ttatatctct tcaatcgttt ccgacatcgg 2340
tcggtttcgg tgcccaaaat gatctgataa acacttatct ctctgtagct tgcatgccat 2400
tgcgagcgta ttttggtagc tggccgttgc caaacggctc cgacaggtac tgctattgga 2460
ggttgtgcac gaccacgttg agtttgcctt ttgagttgga gagtgtgtct tttcgtcata 2520
tattcggcct tttcaagggt gattttcagg ctacgtaatg attgtatagt ttaaccagct 2580
aaaacatatt gatgacaagt tctatttcag caccacaaac aagcctgtta atgtctctca 2640
ccgcaaccat tgttctgcgc gcgttataat cagcatagaa gtttattttc tttgggatga 2700
ttcaaatatt acgtgacgca aagtttgcca attttagaac ccctccctcc tccacgtaac 2760
ggcttttgtg tgaaaaattt aaattttgtg tatagaccgt agcatttcgg aagacccccct 2820
cccttactct gttgagttac gtaaaatttc aacgatcctt ttgtagttct gaattttata 2880
tcagcgtgca gtgttatgaa gatatccaca gtataaaata ttattttatt ttaaattcta 2940
tgctgattat caatgtgtta ctagtggctt ttcatactca tgttgcgagc tcgatttggc 3000
gcacggtact tatcaaggca tgtatgtatg ttgtttgaag caactgtata actgtttgaa 3060
actatctaat tggtgagctc gtttcattta gtatataata atgataattg ctatggagac 3120

gttatttact agcaagtgat ttgacgacct gaaatcggaa caaatagaca acgtttttat 3180

aaatacaata aatcagaact ttccattatt gggtacaaag agttgcgcta tttcgatact 3240

gtcagatcag attttccagc acaacgatac cttgatatgc gataacttag aattagacct 3300

tcaaatccat ctctccagct atgaacagtc atatagataa agccaatggc gttatgaggt 3360

agcggaaagc gtcatctttc caatgctatc taagtacata atttgctata gctttctatt 3420

aatcgtagtt tgagagatgc aaagtcagtt atctcgtatc aaggtttgat tgttttggaa 3480

attagctaaa cagttgacat tatcacccgt ctttagggga taagcgcata caaatgtgta 3540

tttagttgtt cattgaagta acgtaagata ggcaagtatg gaaacgagct caccaaacgt 3600

cgaaatacgt ctaataaatt tgtgttcagc aggatggttc aaaatttatt tgcatcacct 3660

caaaattaca gtacctagtg ctgtttgtga caaacatcaa aaggtaaaat caaactcgtg 3720

gcgtcgtgca atctccatag aatgaacaat ttctaaccgt atttgatgga aagacattga 3780

gtctactatc ctcttaacag cattgcactt gtctataaac aataaataat ttgttctttt 3840

ttacattttc tttccccact ttcgccccccc ccccccccaa aaatcaatcc ctcaaacagg 3900

atacgacatt tgttgcatct actttccgaa gcgttccagc agacacagac actggccgga 3960

cgaggagaac atctccgtca cccgcactcc gtctgcgtca cggtcgccat gtgccgattt 4020

tcgtacccgg tcacagtcca gctcgccgga taacaacggt ggcgcgctca atctggacac 4080

gaaatctacc aaagcgacga ccgccaccac cgacgacgaa gaggttatgt acgagaaacg 4140

cagcccgaag tccattgaat ctaccgagtt gcggtgccgt ctggaggaag ccttacacag 4200

tggcgctgct gctgctgcgg ctgctgaaga acctctggcg ggcggaagcg gttcccactg 4260

gaagagagaa agtttcggct ctacggagga gattcccact cgacccgctc acagtgaacc 4320

ggaagataat ggatttgaaa acggattgga agcgcaccag tcccatattc tgcacagcat 4380

acatcggaa 4389

<210> 150
<211> 2572
<212> DNA
<213> artificial

<220>
<223> DSX Minigene2 from construct LA3534

<400> 150
ctcgatttcc cctcgtttcc aatttcagac gacgaacttg tcaaacgatc tcaatggctc 60

ctggagaagc tgcgataccc ctgggagatg atgcccctga tgtacgtgat actgaaaggc 120

gccgacggag acgtcaataa agcgcgccaa cggattgacg aaggtatggg ggttcttacc 180

ggttgggact gtttccgagg tatcgatcgg gtgtcactca cttcctgggt gctcccattt 240

tgtaactgct aacgcttatt attgagtttc aggacatctg ggatcttcgg tcgacggagt 300

ctattcccaa cagtgccctg gatcaaacac tgccatcatg cagtttccgt agcctgttgg 360
gctacgctcc ccgacttgac atcccccatt cttatcaaac aacaactcaa ggcctgagac 420
aacgagtggt ggaatttgcg cacgaagtca ttggtttgtc ctggtaaaag ttaaaagggt 480
taactggagg gttaattgac acggtttcaa ctgatggcct tattgacaca cggatgaaag 540
acttgcacgc ttgaccttct gtctgtacta ataaaagtta cgttggctgg gttttggggt 600
cataatggcc ccaaaatcga atcgtcataa cttcttgaaa tacaactcac gtttaagacc 660
attcaagagt attagatcat cgtctataat agcagatttg aaatttactt cacatttcgg 720
tattgcagtg ccccttgctt ccacaatgga attagttaaa gtttcgagag cattgtcaat 780
atcaagtgtt gttagcaaac aaatgctaac atcaagatta ctatcgatgt ttgattcaca 840
tgtattccaa tcagctcgta aaaaatggaa agtggagctg atagggttga ggtctcacgt 900
gctccaaatc atcacctcca agttagttct aatacactcc gttatatgaa atatggtggt 960
gcgtcgatcg tcgcaagttt atcgttaaac agtcaataaa atgagcattt tatatcgtga 1020
tacatatgag aagatagagg tttcaattaa aacaaatcca catggtgtcg ctaataaaat 1080
tgtgcatttt aagcgagtta tatcctctga tcaagataaa atagaaaatt cgatttttga 1140
atattcaatt ataagagcct gaataactac aacatgtagt gaatcgaaac tgatttatga 1200
cggtttgtga aggttacacg tcctaagcat ttggattcaa gaaaagcaag agatatgacg 1260
aatgtaaact ttatcgtatc aatgaagtaa ctagcgtcca gaacagtaca aaccaacatc 1320
gtaccgtcgt attccactcc ggtcgttgca atatctctag gtccaccgaa aaacactcat 1380
gaccaagatc gtgtcgtcga tcttggtcca ccgaaacacc gatgtccata tcgtttcgtc 1440
gaacttggac caacgattca tgcaactgat gacaacgcgg cccccgggtc gtaccaatat 1500
ccgaaaaatc caactgttct tctctgcctc gcaggtcaag ccgtggtcaa tgaatactca 1560
cgattgcaca atctgaacat gttcgacggt gtagagttgc gcagtacgac gcgccagtcc 1620
ggatgataga ctttttacac gatcagcacg acccactgcg ctgcggcaaa ggtcgaaccg 1680
aaacaagaat aaaccacgaa gatcagatcg attcgacgga agaagcaatc gaatgcaaag 1740
aagaatcgga atgaagaaaa ctctaaagca tcgcatattt acaaagcata acggaaaacc 1800
cgcaagttca aactagtgat tagtgtaaga tgaagcaaag cagaaatgta gtatctagat 1860
ttttcgacgt tagtttacaa agataagaaa tgaggttgga catacaatcg tgggtattcg 1920
tctgagttcg tcacaactgc accggaaact gtgaaacaga atagagccaa cctgtgcgcg 1980
gagaatgttg aggtcattat aagcttcctt agcatccacg ggtgaaagtc gatcgacgga 2040
agcctgcaag actctgtcga tgggctttcg tcctagaaga ataagattaa acctgaaatg 2100
tattctcccg tggaatggtt tcatttgagt aattctgtat cttctccttc ccaattccac 2160

gaacgcgacg aactctaata caaacaacat aatgaccaca gtgcaaatgc tgtttaacga 2220

taatagcgac atgcagccat tctggggcta ccacgtgtag ctctacttgt gagacagcgt 2280

tcctaaagag tgtgaaagtg caaacaagtg atgaaaccaa tagtgcaaag caagtttaga 2340

gggaaaattt aaaaaatgca aaacagcagt agtacttaac ttttaagatt gtgtttcgaa 2400

agccgaagtg tgttccatct gccaccggaa aaaaacgacg acagcagaat catcaacaag 2460

caacatccat ccgaaaaaat ccgggaaacc ggatcttcaa ccaaccatcc tacaatctac 2520

aaaccagaga ttatatctct tcaatcgttt ccgacatcgg tcggtttcgg tg 2572

<210> 151
<211> 18790
<212> DNA
<213> artificial

<220>
<223> LA3619 whole plasmid sequence

<400> 151
cgcgcctaag atacattgat gagtttggac aaaccacaac tagaatgcag tgaaaaaaat 60

gctttatttg tgaaatttgt gatgctattg ctttatttgt aaccattata agctgcaata 120

aacaagttaa caacaacaat tgcattcatt ttatgtttca ggttcagggg gaggtgtggg 180

aggtttttta aagcaagtaa aacctctaca aatgtggtat ggctgattat gatcgttgca 240

cattccgatg tatgctgtgc agaatatggg actggtgcgc ttccaatccg ttttcaaatc 300

cattatcttc cggttcactg tgagcgggtc gagtgggaat ctcctccgta gagccgaaac 360

tttctctctt ccagtgggaa ccgcttccgc ccgccagagg ttcttcagca gccgcagcag 420

cagcagcgcc actgtgtaag gcttcctcca gacggcaccg caactcggta gattcaatgg 480

acttcgggct gcgtttctcg tacataacct cttcgtcgtc ggtggtggcg gtcgtcgctt 540

tggtagattt cgtgtccaga ttgagcgcgc caccgttgtt atccggcgag ctggactgtg 600

accgggtacg aaaatcggca catggcgacc gtgacgcaga cggagtgcgg gtgacggaga 660

tgttctcctc gtccggccag tgtctgtgtc tgctggaacg cttcggaaag tagatgcaac 720

aaatgtcgta tcctgtttga gggattgatt tttggggggg gggggggcga aagtggggaa 780

agaaaatgta aaaaagaaca aattatttat tgtttataga caagtgcaat gctgttaaga 840

ggatagtaga ctcaatgtct ttccatcaaa tacggttaga aattgttcat tctatggaga 900

ttgcacgacg ccacgagttt gattttacct tttgatgttt gtcacaaaca gcactaggta 960

ctgtaatttt gaggtgatgc aaataaattt tgaaccatcc tgctgaacac aaatttatta 1020

gacgtatttc gacgtttggt gagctcgttt ccatacttgc ctatcttacg ttacttcaat 1080

gaacaactaa atacacattt gtatgcgctt atcccctaaa gacgggtgat aatgtcaact 1140

gtttagctaa tttccaaaac aatcaaacct tgatacgaga taactgactt tgcatctctc 1200

aaactacgat taatagaaag ctatagcaaa ttatgtactt agatagcatt ggaaagatga 1260

cgctttccgc tacctcataa cgccattggc tttatctata tgactgttca tagctggaga 1320

gatggatttg aaggtctaat tctaagttat cgcatatcaa ggtatcgttg tgctggaaaa 1380

tctgatctga cagtatcgaa atagcgcaac tctttgtacc caataatgga aagttctgat 1440

ttattgtatt tataaaaacg ttgtctattt gttccgattt caggtcgtca aatcacttgc 1500

tagtaaataa cgtctccata gcaattatca ttattatata ctaaatgaaa cgagctcacc 1560

aattagatag tttcaaacag ttatacagtt gcttcaaaca acatacatac atgccttgat 1620

aagtaccgtg cgccaaatcg agctcgcaac atgagtatga aaagccacta gtaacacatt 1680

gataatcagc atagaattta aaataaaata atattttata ctgtggatat cttcataaca 1740

ctgcacgctg atataaaatt cagaactaca aaaggatcgt tgaaatttta cgtaactcaa 1800

cagagtaagg gaggggtct tccgaaatgc tacggtctat acacaaaatt taaatttttc 1860

acacaaaagc cgttacgtgg aggagggagg ggttctaaaa ttggcaaact ttgcgtcacg 1920

taatatttga atcatcccaa agaaaataaa cttctatgct gattataacg cgcgcagaac 1980

aatggttgcg gtgagagaca ttaacaggct tgtttgtggt gctgaaatag aacttgtcat 2040

caatatgttt tagctggtta aactatacaa tcattacgta gcctgaaaat caccccttgaa 2100

aaggccgaat atatgacgaa aagacacact ctccaactca aaaggcaaac tcaacgtggt 2160

cgtgcacaac ctccaatagc agtacctgtc ggagccgttt ggcaacggcc agctaccaaa 2220

atacgctcgc aatggcatgc aagctacaga gagataagtg tttatcagat cattttgggc 2280

accgaaaccg accgatgtcg gaaacgattg aagagatata atctctggtt tgtagattgt 2340

aggatggttg gttgaagatc cggtttcccg gattttttcg gatggatgtt gcttgttgat 2400

gattctgctg tcgtcgtttt tttccggtgg cagatggaac agcctcactt cggctttcga 2460

aacacaatct taaaagttaa gtactactgc tgttttgcat tttttaaatt ttccctctaa 2520

acttgctttg cactattggt ttcatcactt gtttgcactt tcacactctt taggaacgct 2580

gtctcacaag tagagcttgc ggtggacaat caccggtgtt agccgccgta ctcatcgatg 2640

cccagggcgt cggtgaacat ctgctcgaac tcgaaatcgg ccatatccag ggcgccgtag 2700

ggggcgctat cgtgcggggt gaatcccggt cccgggctat cgccatcgcc cagcatgtcc 2760

aggtcgaagt cgtccagggc atcggcgtgg gccatcgcca catcctcgcc atccaggtgc 2820

agctcatcgc ccaggctcac gtcggtcggc ggggcggtcg acaggcggcg ggtgtgtccg 2880

gccggcagga agctcaggcg cggggcggcc aggcccgcct cctccggggc atcatcatcc 2940

ggcagatcca gcaggccctc gatggtgctg ccgtagttgt tcttggtgcg ggcgcggctg 3000

taggcggggc ccgagcccga ctcgcatttc agttgctttt ccaatccgca gataatcagc 3060

tccaagccga acaggaatgc cggctcggct ccttgatgat cgaacagctc gattgcctga 3120
cgcagcagtg ggggcatcga atcggttgtt ggggtctcgc gctcctcttt tgcgacttga 3180
tgctcttggt cctccagcac gcagcccagg gtaaagtgac cgacggcgct cagagcgtag 3240
agagcatttt ccaggctgaa gccttgctgg cacaggaacg cgagctggtt ctccagtgtc 3300
tcgtattgct tttcggtcgg gcgcgtgccg agatggactt tggcaccgtc tcggtgggac 3360
agcagagcgc agcggaacga cttggcgtta ttgcggagga agtcctgcca ggactcgcct 3420
tccaacgggc aaaaatgcgt gtggtggcgg tcgagcatct cgatggccag ggcatccagc 3480
agcgcccgct tattcttcac gtgccagtag agggtgggct gctccacgcc cagcttctgc 3540
gccaacttgc gggtcgtcag tccctcaatg ccaacttcgt tcaacagctc caacgcggag 3600
ttgatgactt tggacttatc caggcggctg cccatggtgg ttttccagtg gcgccgcttc 3660
acgtggtagc cccagaatgg ctgcatgtcg ctattatcgt taaacagcat ttgcactgtg 3720
gtcattatgt tgtttgtatt agagttcgtc gcgttcgtgg aattgggaag gagaagatac 3780
agaattactc aaatgaaacc attccacggg agaatacatt tcaggtttaa tcttattctt 3840
ctaggacgaa agcccatcga cagagtcttg caggcttccg tcgatcgact ttcacccgtg 3900
gatgctaagg aagcttataa tgacctcaac attctccgcg cacaggttgg ctctattctg 3960
tttcacagtt tccggtgcag ttgtgacgaa ctcagacgaa tacccacgat tgtatgtcca 4020
acctcatttt ttatctttgt aaactaacgt cgaaaaatct agatactaca tttctgcttt 4080
gcttcatctt acactaatca ctagtttgaa cttgcgggtt ttccgttatg ctttgtaaat 4140
atgcgatgct ttagagtttt cttcgttccg attcttcttt gcattcgatt gcttcttccg 4200
tcgaatcgat ctgatcttcg tggtttattc ttgtttcggt tcgacctttg ccgcagcgca 4260
gtgggtcgtg ctgatcgtgt aaaaagtcta tcatccggac tggcgcgtcg tactgcgcaa 4320
ctctacaccg tcgaacatgt tcagattgtg caatcgtgag tattcattga ccacggcttg 4380
acctgcgagg cagagaagaa cagttggatt tttcggatat tggtacgacc cgggggccgc 4440
gttgtcatca gttgcatgaa tcgttggtcc aagttcgacg aaacgatatg gacatcggtg 4500
tttcggtgga ccaagatcga cgacacgatc ttggtcatga gtgtttttcg gtggacctag 4560
agatattgca acgaccggag tggaatacga cggtacgatg ttggtttgta ctgttctgga 4620
cgctagttac ttcattgata cgataaagtt tacattcgtc atatctcttg cttttcttga 4680
atccaaatgc ttaggacgtg taaccttcac aaaccgtcat aaatcagttt cgattcacta 4740
catgttgtag ttattcaggc tcttataatt gaatattcaa aaatcgaatt ttctatttta 4800
tcttgatcag aggatataac tcgcttaaaa tgcacaattt tattagcgac accatgtgga 4860
tttgttttaa ttgaaacctc tatcttctca tatgtatcac gatataaaat gctcatttta 4920
ttgactgttt aacgataaac ttgcgacgat cgacgcacca ccgacctaat tccattgtgg 4980

aagcaagggg cactgcaata ccgaaatgtg aagtaaattt caaatctgct attatagacg 5040
atgatctaat actcttgaat ggtcttaaac gtgagttgta tttcaagaag ttatgacgat 5100
tcgattttgg ggccattatg accccaaaac ccagccaacg taacttttat tagtacagac 5160
agaaggtcaa gcgtgcaagt ctttcatccg tgtgtcaata aggccatcag ttgaaaccgt 5220
gtcaattaac cctccagtta acccttttaa cttttaccag gacaaaccaa tgacttcgtg 5280
cgcaaattcc accactcgtt gtctcaggcc ttgagttgtt gtttgataag aatgggggat 5340
gtcaagtcgg ggagcgtagc ccaacaggct acggaaactg catgatggca gtgtttgatc 5400
cagggcactg ttgggaatag actccgtcga ccgaagatcc cagatgtcct gaaactcaat 5460
aataagcgtt agcagttaca aaatgggagc acccaggaag tgagtgacac ccgatcgata 5520
cctcggaaac agtcccaacc ggtaagaacc cccatacctt cgtcaatccg ttggcgcgct 5580
ttattgacgt ctccgtcggc gcctttcagt atcacgtaca tcaggggcac cacctcctag 5640
ggcagattgt ttagcttgtt cagctgcgct tgtttatttg cttagctttc gcttagcgac 5700
gtgttcactt tgcttgtttg aattgaattg tcgctccgta gacgaagcgc ctctatttat 5760
actccggcgc tcgttttcga gtttaccact ccctatcagt gatagagaaa agtgaaagtc 5820
gagtttacca ctccctatca gtgatagaga aaagtgaaag tcgagtttac cactccctat 5880
cagtgataga gaaaagtgaa agtcgagttt accactccct atcagtgata gagaaaagtg 5940
aaagtcgagt ttaccactcc ctatcagtga tagagaaaag tgaaagtcga gtttaccact 6000
ccctatcagt gatagagaaa agtgaaagtc gagtttacca ctccctatca gtgatagaga 6060
aaagtgaaag tcgaaacctg gcgcgccccg gccatcgaga aagagagaga gaagagaaga 6120
gagagaacat tcgagaaaga gagagagaag agaagagaga gaacatactc cctatcagtg 6180
atagagaagt ccctatcagt gatagagatg tccctatcag tgatagagag ttccctatca 6240
gtgatagaga cgtccctatc agtgatagag aagtccctat cagtgataga gagatcccta 6300
tcagtgatag agatttccct atcagtgata gagaggtccc tatcagtgat agagacttcc 6360
ctatcagtga tagagaaatc cctatcagtg atagagacat ccctatcagt gatagagaac 6420
tccctatcag tgatagagac ctccctatca gtgatagaga tcgatgcggc cgcgagcgcc 6480
ggagtataaa tagaggcgct tcgtctacgg agcgacaatt caattcaaac aagcaaagtg 6540
aacacgtcgc taagcgaaag ctaagcaaat aaacaagcgc agctgaacaa gctaaacaat 6600
ctgcaggtac cctggcggta agttgatcaa aggaaacgca aagttttcaa gaaaaaacaa 6660
aactaatttg atttataaca cctttagaaa gcggggctag ccaccatggg cagcgcctac 6720
agccgcgccc gtaccaagaa caactatggc agcaccatcg agggactgct ggacctgccg 6780
gatgacgatg ccccggagga agccggcctg gccgcccccc gcctgagctt cctgcccgcc 6840

ggacacacgc gccgcctgag caccgccccg ccgaccgatg tgagcctggg cgacgagctg 6900

cacctggatg gagaggatgt ggcaatggcc cacgccgacg ccctggacga tttcgacctg 6960

gatatgctgg gcgatggaga tagcccggga ccgggcttca cgccccacga tagcgccccg 7020

tacggcgccc tggacatggc cgacttcgag ttcgagcaaa tgttcaccga cgcgctgggc 7080

atcgatgagt atggcgggta ggtttaaact cgcgttaaga tacattgatg agtttggaca 7140

aaccacaact agaatgcagt gaaaaaaatg ctttatttgt gaaatttgtg atgctattgc 7200

tttatttgta accattataa gctgcaataa acaagttaac aacaacaatt gcattcattt 7260

tatgtttcag gttcaggggg aggtgtggga ggttttttaa agcaagtaaa acctctacaa 7320

atgtggtatg gctgattatg atcagttatc tagatccggt ggatcttacg ggtcctccac 7380

cttccgcttt ttcttgggtc gagatctcag gaacaggtgg tggcggccct cggtgcgctc 7440

gtactgctcc acgatggtgt agtcctcgtt gtgggaggtg atgtccagct tggcgtccac 7500

gtagtagtag ccgggcagct gcacgggctt cttggccatg tagatggact tgaactccac 7560

caggtagtgg ccgccgtcct tcagcttcag ggccttgtgg gtctcgccct tcagcacgcc 7620

gtcgcggggg tacaggcgct cggtggaggc ctcccagccc atggtcttct tctgcatcac 7680

ggggccgtcg gaggggaagt tcacgccgat gaacttcacc ttgtagatga agcagccgtc 7740

ctgcagggag gagtcctggg tcacggtcgc cacgccgccg tcctcgaagt tcatcacgcg 7800

ctcccacttg aagccctcgg ggaaggacag cttcttgtag tcggggatgt cggcggggtg 7860

cttcacgtac accttggagc cgtactggaa ctggggggac aggatgtccc aggcgaaggg 7920

caggggggccg cccttggtca ccttcagctt cacggtgttg tggccctcgt aggggcggcc 7980

ctcgccctcg ccctcgatct cgaactcgtg gccgttcacg gtgccctcca tgcgcacctt 8040

gaagcgcatg aactcggtga tgacgttctc ggaggaggcc atggtggcga ccggtttgcg 8100

cttcttcttg ggtggggtgg gatctcccat ggtggcctga atctcaactt gcacctgaag 8160

gtagtgcagc aaggatgagc aaaagggaag aacccagaaa agaacgggaa aacttacccc 8220

aattagaatt gcttgtcgcc gccagtgtca acttgcaact gaaacaatat ccaacatgaa 8280

cgtcaattta tactgcccta atggcgaaca cgataacaat atttctttta ttatgccctc 8340

taaaaccaac gcggttatcg tttatttatt caaattagat atagaacatc cgccgacata 8400

caatgttaat gcaaaaacgc gtttggtgag cggatacgaa aacagtcggc cgataaacat 8460

taatctgagg tcgataacac cgtccttgaa cggaacacga ggagcgtacg tgatcagctg 8520

cattcgcgcg ccgcgccttt atcgagattt atttgcatac aacaagtaca ctgcgccgtt 8580

gggatttgtg gtaacgcgca cacatgcaga gctgcaagtg tggcacattt tgtctgtgcg 8640

caaaaccttt gaagccaaaa gtacgaggtc cgttacgggc atgctactag cgcacacgga 8700

caatggaccc gacaaattct acgccaagga tttaatgata atgtcgggca acgtatccgt 8760

tcattttatc aataacctac aaaaatgtcg cgcgcatcac aaagacatcg atatatttaa 8820
acatttatgt cccgaactgc aaatcgataa tagtgttgtg caacctcgag cgtccgtttg 8880
atttaacgta tagcttgcaa atgaattatt taattatcaa tcatgtttta cgcgtagaat 8940
tctacccgta aagcgagttt agttatgagc catgtgcaaa acatgacatc agcttttatt 9000
tttataacaa atgacatcat ttcttgattg tgttttacac gtagaattct actcgtaaag 9060
cgagttcagt tttgaaaaac aaatgacatc atctttttga ttgtgcttta caagtagaat 9120
tctacccgta aatcaagttc ggttttgaaa aacaaatgag tcatattgta tgatatcata 9180
ttgcaaaaca aatgactcat caatcgatcg tgcgttacac gtagaattct actcgtaaag 9240
cgagtttatg agccgtgtgc aaaacatgac atcatctcga tttgaaaaac aaatgacatc 9300
atccactgat cgtgcattac aagtagaatt ctactcgtaa agccagttcg gttatgagcc 9360
gtgtacaaaa catgacatca gattatgact catacttgat tgtgttttac gcgtagaatt 9420
ctactcgtaa agccagttca attttaaaaa caaatgacat catccaaatt aataaatgac 9480
aagcaatggg taccatgcgg cctggcctcg cgctcgcgcg actgacggtc gtaagcaccc 9540
gcgtacgtgt ccaccccggt cacaacccct tgtgtcatgt cggcgaccct acgcccccaa 9600
ctgagagaac tcaaaggtta ccccagttgg ggcactactc ccgaaaaccg cttctgacct 9660
gggaaaacgt gaagccccgg ggcatccgct gagggttgcc gccggggctt cggtgtgtcc 9720
gtcagtactt aattaacacc gaaatcgtaa ttcacggcat cattacaaaa tattttgacg 9780
ttttggacct cgtccctaat gacaccataa cggtggcctt gaagtatatt taaccctaga 9840
aagatagtct gcgtaaaatt gacgcatgca ttcttgaaat attgctctct ctttctaaat 9900
agcgcgaatc cgtcgctgtg catttaggac atctcagtcg ccgcttggag ctcccgtgag 9960
gcgtgcttgt caatgcggta agtgtcactg attttgaact ataacgaccg cgtgagtcaa 10020
aatgacgcat gattatcttt tacgtgactt ttaagattta actcatacga taattatatt 10080
gttatttcat gttctactta cgtgataact tattatatat atattttctt gttatagata 10140
tcgtgactaa tatataataa aatgggtagt tctttagacg atgagcatat cctctctgct 10200
cttctgcaaa gcgatgacga gcttgttggt gaggattctg acagtgaaat atcagatcac 10260
gtaagtgaag atgacctcga ggatccaagc ttatcgattt cgaaccctcg accgccggag 10320
tataaataga ggcgcttcgt ctacggagcg acaattcaat tcaaacaagc aaagtgaaca 10380
cgtcgctaag cgaaagctaa gcaaataaac aagcgcagct gaacaagcta aacaatcggg 10440
gtaccgctag agtcgatccc accccaccca agaagaagcg caaaccggta ccatggcctc 10500
ctccgagaac gtcatcaccg agttcatgcg cttcaaggtg cgcatggagg gcaccgtgaa 10560
cggccacgag ttcgagatcg agggcgaggg cgagggccgc ccctacgagg gccacaacac 10620

cgtgaagctg aaggtgacca agggcggccc cctgcccttc gcctgggaca tcctgtcccc 10680
ccagttccag tacggctcca aggtgtacgt gaagcacccc gccgacatcc ccgactacaa 10740
gaagctgtcc ttccccgagg gcttcaagtg ggagcgcgtg atgaacttcg aggacggcgg 10800
cgtggcgacc gtgacccagg actcctccct gcaggacggc tgcttcatct acaaggtgaa 10860
gttcatcggc gtgaacttcc cctccgacgg ccccgtgatg cagaagaaga ccatgggctg 10920
ggaggcctcc accgagcgcc tgtacccccg cgacggcgtg ctgaagggcg agacccacaa 10980
ggccctgaag ctgaaggacg gcggccacta cctggtggag ttcaagtcca tctacatggc 11040
caagaagccc gtgcagctgc ccggctacta ctacgtggac gccaagctgg acatcacctc 11100
ccacaacgag gactacacca tcgtggagca gtacgagcgc accgagggcc gccaccacct 11160
gttcctgtga tgatcataat cagccatacc acatttgtag aggttttact tgctttaaaa 11220
aacctcccac acctcccct gaacctgaaa cataaaatga atgcaattgt tgttgttaac 11280
ttgtttattg cagcttataa tggttacaaa taaagcaata gcatcacaaa tttcacaaat 11340
aaagcatttt tttcactgca ttctagttgt ggtttgtcca aactcatcaa tgtatcttaa 11400
cgcgagttaa ttacggccgc tcatttaaat ctggccggcc gcaaccattg tgggaaccgt 11460
gcgatcaaac aaacgcgaga taccggaagt actgaaaaac agtcgctcca ggccagtggg 11520
aacatcgatg ttttgttttg acggacccct tactctcgtc tcatataaac cgaagccagc 11580
taagatggta tacttattat catcttgtga tgaggatgct tctatcaacg aaagtaccgg 11640
taaaccgcaa atggttatgt attataatca aactaaaggc ggagtggaca cgctagacca 11700
aatgtgttct gtgatgacct gcagtaggaa gacgaatagg tggcctatgg cattattgta 11760
cggaatgata aacattgcct gcataaattc ttttattata tacagccata atgtcagtag 11820
caagggagaa aaggtccaaa gtcgcaaaaa atttatgaga aacctttaca tgagcctgac 11880
gtcatcgttt atgcgtaagc gtttagaagc tcctactttg aagagatatt tgcgcgataa 11940
tatctctaat attttgccaa atgaagtgcc tggtacatca gatgacagta ctgaagagcc 12000
agtaatgaaa aaacgtactt actgtactta ctgcccctct aaaataaggc gaaaggcaaa 12060
tgcatcgtgc aaaaaatgca aaaaagttat ttgtcgagag cataatattg atatgtgcca 12120
aagttgtttc tgactgacta ataagtataa tttgtttcta ttatgtataa gttaagctaa 12180
ttacttattt tataatacaa catgactgtt tttaaagtac aaaataagtt tatttttgta 12240
aaagagagaa tgtttaaaag ttttgttact ttatagaaga aattttgagt ttttgttttt 12300
ttttaataaa taaataaaca taaataaatt gtttgttgaa tttattatta gtatgtaagt 12360
gtaaatataa taaaacttaa tatctattca aattaataaa taaacctcga tatacagacc 12420
gataaaacac atgcgtcaat tttacgcatg attatcttta acgtacgtca caatatgatt 12480
atctttctag ggttaaataa tagtttctaa tttttttatt attcagcctg ctgtcgtgaa 12540

taccgtatat ctcaacgctg tctgtgagat tgtcgtattc tagccttttt agtttttcgc 12600
tcatcgactt gatattgtcc gacacatttt cgtcgatttg cgttttgatc aaagacttga 12660
gcagagacac gttaatcaac tgttcaaatt gatccatatt aacgatatca acccgatgcg 12720
tatatggtgc gtaaaatata ttttttaacc ctcttatact ttgcactctg cgttaatacg 12780
cgttcgtgta cagacgtaat catgttttct tttttggata aaactcctac tgagtttgac 12840
ctcatattag accctcacaa gttgcaaaac gtggcatttt ttaccaatga agaatttaaa 12900
gttattttaa aaaatttcat cacagattta aagaagaacc aaaaattaaa ttatttcaac 12960
agtttaatcg accagttaat caacgtgtac acagacgcgt cggcaaaaaa cacgcagccc 13020
gacgtgttgg ctaaaattat taaatcaact tgtgttatag tcacggattt gccgtccaac 13080
gtgttcctca aaaagttgaa gaccaacaag tttacggaca ctattaatta tttgattttg 13140
ccccacttca ttttgtggga tcacaatttt gttatatttt aaacaaagct tggcactggc 13200
cgtcgtttta caacgtcgtg actgggaaaa ccctggcgtt acccaactta atcgccttgc 13260
agcacatccc cctttcgcca gctggcgtaa tagcgaagag gcccgcaccg atcgcccttc 13320
ccaacagttg cgcagcctga atggcgaatg gcgcctgatg cggtattttc tccttacgca 13380
tctgtgcggt atttcacacc gcatatggtg cactctcagt acaatctgct ctgatgccgc 13440
atagttaagc cagccccgac acccgccaac acccgctgac gcgccctgac gggcttgtct 13500
gctcccggca tccgcttaca gacaagctgt gaccgtctcc gggagctgca tgtgtcagag 13560
gttttcaccg tcatcaccga aacgcgcgag acgaaagggc ctcgtgatac gcctattttt 13620
ataggttaat gtcatgataa taatggtttc ttagacgtca ggtggcactt ttcggggaaa 13680
tgtgcgcgga acccctattt gtttattttt ctaaatacat tcaaatatgt atccgctcat 13740
gagacaataa ccctgataaa tgcttcaata atattgaaaa aggaagagta tgagtattca 13800
acatttccgt gtcgccctta ttcccttttt tgcggcattt tgccttcctg tttttgctca 13860
cccagaaacg ctggtgaaag taaaagatgc tgaagatcag ttgggtgcac gagtgggtta 13920
catcgaactg gatctcaaca gcggtaagat ccttgagagt tttcgccccg aagaacgttt 13980
tccaatgatg agcactttta aagttctgct atgtggcgcg gtattatccc gtattgacgc 14040
cgggcaagag caactcggtc gccgcataca ctattctcag aatgacttgg ttgagtactc 14100
accagtcaca gaaaagcatc ttacggatgg catgacagta agagaattat gcagtgctgc 14160
cataaccatg agtgataaca ctgcggccaa cttacttctg acaacgatcg gaggaccgaa 14220
ggagctaacc gcttttttgc acaacatggg ggatcatgta actcgccttg atcgttggga 14280
accggagctg aatgaagcca taccaaacga cgagcgtgac accacgatgc ctgtagcaat 14340
ggcaacaacg ttgcgcaaac tattaactgg cgaactactt actctagctt cccggcaaca 14400

attaatagac tggatggagg cggataaagt tgcaggacca cttctgcgct cggcccttcc 14460

ggctggctgg tttattgctg ataaatctgg agccggtgag cgtgggtctc gcggtatcat 14520

tgcagcactg gggccagatg gtaagccctc ccgtatcgta gttatctaca cgacggggag 14580

tcaggcaact atggatgaac gaaatagaca gatcgctgag ataggtgcct cactgattaa 14640

gcattggtaa ctgtcagacc aagtttactc atatatactt tagattgatt taaaacttca 14700

tttttaattt aaaaggatct aggtgaagat cctttttgat aatctcatga ccaaaatccc 14760

ttaacgtgag ttttcgttcc actgagcgtc agaccccgta gaaaagatca aaggatcttc 14820

ttgagatcct ttttttctgc gcgtaatctg ctgcttgcaa acaaaaaaac caccgctacc 14880

agcggtggtt tgtttgccgg atcaagagct accaactctt tttccgaagg taactggctt 14940

cagcagagcg cagataccaa atactgtcct tctagtgtag ccgtagttag gccaccactt 15000

caagaactct gtagcaccgc ctacatacct cgctctgcta atcctgttac cagtggctgc 15060

tgccagtggc gataagtcgt gtcttaccgg gttggactca agacgatagt taccggataa 15120

ggcgcagcgg tcgggctgaa cggggggttc gtgcacacag cccagcttgg agcgaacgac 15180

ctacaccgaa ctgagatacc tacagcgtga gcattgagaa agcgccacgc ttcccgaagg 15240

gagaaaggcg gacaggtatc cggtaagcgg cagggtcgga acaggagagc gcacgaggga 15300

gcttccaggg ggaaacgcct ggtatcttta tagtcctgtc gggtttcgcc acctctgact 15360

tgagcgtcga tttttgtgat gctcgtcagg ggggcggagc ctatggaaaa acgccagcaa 15420

cgcggccttt ttacggttcc tggccttttg ctggcctttt gctcacatgt tctttcctgc 15480

gttatcccct gattctgtgg ataaccgtat taccgccttt gagtgagctg ataccgctcg 15540

ccgcagccga acgaccgagc gcagcgagtc agtgagcgag gaagcggaag agcgcccaat 15600

acgcaaaccg cctctccccg cgcgttggcc gattcattaa tgcagctggc acgacaggtt 15660

tcccgactgg aaagcgggca gtgagcgcaa cgcaattaat gtgagttagc tcactcatta 15720

ggcaccccag gctttacact ttatgcttcc ggctcgtatg ttgtgtggaa ttgtgagcgg 15780

ataacaattt cacacaggaa acagctatga ccatgattac gaatttcgac ctgcaggcat 15840

gcaagcttgc atgcctgcag gtcgacgctc gcgcgacttg gtttgccatt ctttagcgcg 15900

cgtcgcgtca cacagcttgg ccacaatgtg gtttttgtca aacgaagatt ctatgacgtg 15960

tttaaagttt aggtcgagta aagcgcaaat ctttttttaac cctagaaaga tagtctgcgt 16020

aaaattgacg catgcattct tgaaatattg ctctctcttt ctaaatagcg cgaatccgtc 16080

gctgtgcatt taggacatct cagtcgccgc ttggagctcc cgtgaggcgt gcttgtcaat 16140

gcggtaagtg tcactgattt tgaactataa cgaccgcgtg agtcaaaatg acgcatgatt 16200

atcttttacg tgacttttaa gatttaactc atacgataat tatattgtta tttcatgttc 16260

tacttacgtg ataacttatt atatatatat tttcttgtta tagatatcgt gactaatata 16320

taataaaatg ggtagttctt tagacgatga gcatatcctc tctgctcttc tgcaaagcga 16380

tgacgagctt gttggtgagg attctgacag tgaaatatca gatcacgtaa gtgaagatga 16440

cgtccagagc gatacagaag aagcgtttat agatgaggta catgaagtgc agccaacgtc 16500

aagcggtagt gaaatattag acgaacaaaa tgttattgaa caaccaggtt cttcattggc 16560

ttctaacaga atcttgacct tgccacagag gactattaga ggtaagaata aacattgttg 16620

gtcaacttca aagtccacga ggcgtagccg agtctctgca ctgaacattg tcagatcggc 16680

ccgctcgccc ggggaactag ttcaattaga gactaattca attagagcta attcaattag 16740

gatccaagct tatcgatttc gaaccctcga ccgccggagt ataaatagag gcgcttcgtc 16800

tacggagcga caattcaatt caaacaagca aagtgaacac gtcgctaagc gaaagctaag 16860

caaataaaca agcgcagctg aacaagctaa acaatcgggg taccgctaga gtcgatccca 16920

ccccacccaa gaagaagcgc aaaccggtcg ccaccatggc cctgtccaac aagttcatcg 16980

gcgacgacat gaagatgacc taccacatgg acggctgcgt gaacggccac tacttcaccg 17040

tgaagggcga gggcagcggc aagccctacg agggcaccca gacctccacc ttcaaggtga 17100

ccatggccaa cggcggcccc ctggccttct ccttcgacat cctgtccacc gtgttcatgt 17160

acggcaaccg ctgcttcacc gcctacccca ccagcatgcc cgactacttc aagcaggcct 17220

tccccgacgg catgtcctac gagagaacct tcacctacga ggacggcggc gtggccaccg 17280

ccagctggga gatcagcctg aagggcaact gcttcgagca caagtccacc ttccacggcg 17340

tgaacttccc cgccgacggc cccgtgatgg ccaagaagac caccggctgg gacccctcct 17400

tcgagaagat gaccgtgtgc gacggcatct tgaagggcga cgtgaccgcc ttcctgatgc 17460

tgcagggcgg cggcaactac agatgccagt tccacacctc ctacaagacc aagaagcccg 17520

tgaccatgcc ccccaaccac gtggtggagc accgcatcgc cagaaccgac ctggacaagg 17580

gcggcaacag cgtgcagctg accgagcacg ccgtggccca catcacctcc gtggtgccct 17640

tctccggact cagatcataa tcagccatac cacatttgta gaggttttac ttgctttaaa 17700

aaacctccca cacctccccc tgaacctgaa acataaaatg aatgcaattg ttgttgttaa 17760

cttgtttatt gcagcttata atggttacaa ataaagcaat agcatcacaa atttcacaaa 17820

taaagcattt ttttcactgc attctagttg tggtttgtcc aaactcatca atgtatctta 17880

ccgcggagtg gacacgctag accaaatgtg ttctgtgatg acctgcagta ggaagacgaa 17940

taggtggcct atggcattat tgtacggaat gataaacatt gcctgcataa attcttttat 18000

tatatacagc cataatgtca gtagcaaggg agaaaaggtc caaagtcgca aaaaatttat 18060

gagaaacctt tacatgagcc tgacgtcatc gtttatgcgt aagcgtttag aagctcctac 18120

tttgaagaga tatttgcgcg ataatatctc taatattttg ccaaatgaag tgcctggtac 18180

atcagatgac agtactgaag agccagtaat gaaaaaacgt acttactgta cttactgccc 18240

ctctaaaata aggcgaaagg caaatgcatc gtgcaaaaaa tgcaaaaaag ttatttgtcg 18300

agagcataat attgatatgt gccaaagttg tttctgactg actaataagt ataatttgtt 18360

tctattatgt ataagttaag ctaattactt attttataat acaacatgac tgtttttaaa 18420

gtacaaaata agtttatttt tgtaaaagag agaatgttta aaagttttgt tactttatag 18480

aagaaatttt gagtttttgt ttttttttaa taaataaata aacataaata aattgtttgt 18540

tgaatttatt attagtatgt aagtgtaaat ataataaaac ttaatatcta ttcaaattaa 18600

taaataaacc tcgatataca gaccgataaa acacatgcgt caattttacg catgattatc 18660

tttaacgtac gtcacaatat gattatcttt ctagggttaa aatgaatgta agcactttat 18720

taacgaaatc tttgggaata tttcgctcat cagcatttta tttgagcagg agtccgagat 18780

gcccgggcgg 18790

<210> 152
<211> 19053
<212> DNA
<213> artificial

<220>
<223> LA3612 whole plasmid sequence

<400> 152
gggcatctcg gactcctgct caaataaaat gctgatgagc gaaatattcc caaagatttc 60

gttaataaag tgcttacatt cattttaacc ctagaaagat aatcatattg tgacgtacgt 120

taaagataat catgcgtaaa attgacgcat gtgttttatc ggtctgtata tcgaggttta 180

tttattaatt tgaatagata ttaagtttta ttatatttac acttacatac taataataaa 240

ttcaacaaac aatttattta tgtttattta tttattaaaa aaaaacaaaa actcaaaatt 300

tcttctataa agtaacaaaa cttttaaaca ttctctcttt tacaaaaata aacttatttt 360

gtactttaaa aacagtcatg ttgtattata aaataagtaa ttagcttaac ttatacataa 420

tagaaacaaa ttatacttat tagtcagtca gaaacaactt tggcacatat caatattatg 480

ctctcgacaa ataacttttt tgcatttttt gcacgatgca tttgcctttc gccttatttt 540

agaggggcag taagtacagt aagtacgttt tttcattact ggctcttcag tactgtcatc 600

tgatgtacca ggcacttcat ttggcaaaat attagagata ttatcgcgca aatatctctt 660

caaagtagga gcttctaaac gcttacgcat aaacgatgac gtcaggctca tgtaaaggtt 720

tctcataaat tttttgcgac tttggacctt ttctcccttg ctactgacat tatggctgta 780

tataataaaa gaatttatgc aggcaatgtt tatcattccg tacaataatg ccataggcca 840

cctattcgtc ttcctactgc aggtcatcac agaacacatt tggtctagcg tgtccactcc 900

gcggtaagat acattgatga gtttggacaa accacaacta gaatgcagtg aaaaaaatgc 960

tttatttgtg aaatttgtga tgctattgct ttatttgtaa ccattataag ctgcaataaa 1020

caagttaaca acaacaattg cattcatttt atgtttcagg ttcaggggga ggtgtgggag 1080

gtttttttaaa gcaagtaaaa cctctacaaa tgtggtatgg ctgattatga tctgagtccg 1140

gagaagggca ccacggaggt gatgtgggcc acggcgtgct cggtcagctg cacgctgttg 1200

ccgcccttgt ccaggtcggt tctggcgatg cggtgctcca ccacgtggtt gggggcatg 1260

gtcacgggct tcttggtctt gtaggaggtg tggaactggc atctgtagtt gccgccgccc 1320

tgcagcatca ggaaggcggt cacgtcgccc ttcaagatgc cgtcgcacac ggtcatcttc 1380

tcgaaggagg ggtcccagcc ggtggtcttc ttggccatca cggggccgtc ggcggggaag 1440

ttcacgccgt ggaaggtgga cttgtgctcg aagcagttgc ccttcaggct gatctcccag 1500

ctggcggtgg ccacgccgcc gtcctcgtag gtgaaggttc tctcgtagga catgccgtcg 1560

gggaaggcct gcttgaagta gtcgggcatg ctggtggggt aggcggtgaa gcagcggttg 1620

ccgtacatga acacggtgga caggatgtcg aaggagaagg ccaggggggcc gccgttggcc 1680

atggtcacct tgaaggtgga ggtctgggtg ccctcgtagg gcttgccgct gccctcgccc 1740

ttcacggtga agtagtggcc gttcacgcag ccgtccatgt ggtaggtcat cttcatgtcg 1800

tcgccgatga acttgttgga cagggccatg gtggcgaccg gtttgcgctt cttcttgggt 1860

ggggtgggat cgactctagc ggtaccccga ttgtttagct tgttcagctg cgcttgttta 1920

tttgcttagc tttcgcttag cgacgtgttc actttgcttg tttgaattga attgtcgctc 1980

cgtagacgaa gcgcctctat ttatactccg gcggtcgagg gttcgaaatc gataagcttg 2040

gatcctaatt gaattagctc taattgaatt agtctctaat tgaactagtt ccccgggcga 2100

gcgggccgat ctgacaatgt tcagtgcaga gactcggcta cgcctcgtgg actttgaagt 2160

tgaccaacaa tgtttattct tacctctaat agtcctctgt ggcaaggtca agattctgtt 2220

agaagccaat gaagaacctg gttgttcaat aacattttgt tcgtctaata tttcactacc 2280

gcttgacgtt ggctgcactt catgtacctc atctataaac gcttcttctg tatcgctctg 2340

gacgtcatct tcacttacgt gatctgatat ttcactgtca gaatcctcac caacaagctc 2400

gtcatcgctt tgcagaagag cagagaggat atgctcatcg tctaaagaac tacccatttt 2460

attatatatt agtcacgata tctataacaa gaaaatatat atataataag ttatcacgta 2520

agtagaacat gaaataacaa tataattatc gtatgagtta aatcttaaaa gtcacgtaaa 2580

agataatcat gcgtcatttt gactcacgcg gtcgttatag ttcaaaatca gtgacactta 2640

ccgcattgac aagcacgcct cacgggagct ccaagcggcg actgagatgt cctaaatgca 2700

cagcgacgga ttcgcgctat ttagaaagag agagcaatat ttcaagaatg catgcgtcaa 2760

ttttacgcag actatctttc tagggttaaa aaagatttgc gctttactcg acctaaactt 2820

taaacacgtc atagaatctt cgtttgacaa aaaccacatt gtggccaagc tgtgtgacgc 2880
gacgcgcgct aaagaatggc aaaccaagtc gcgcgagcgt cgacctgcag gcatgcaagc 2940
ttgcatgcct gcaggtcgaa attcgtaatc atggtcatag ctgtttcctg tgtgaaattg 3000
ttatccgctc acaattccac acaacatacg agccggaagc ataaagtgta aagcctgggg 3060
tgcctaatga gtgagctaac tcacattaat tgcgttgcgc tcactgcccg ctttccagtc 3120
gggaaacctg tcgtgccagc tgcattaatg aatcggccaa cgcgcgggga gaggcggttt 3180
gcgtattggg cgctcttccg cttcctcgct cactgactcg ctgcgctcgg tcgttcggct 3240
gcggcgagcg gtatcagctc actcaaaggc ggtaatacgg ttatccacag aatcagggga 3300
taacgcagga aagaacatgt gagcaaaagg ccagcaaaag gccaggaacc gtaaaaaggc 3360
cgcgttgctg gcgtttttcc ataggctccg cccccctgac gagcatcaca aaaatcgacg 3420
ctcaagtcag aggtggcgaa acccgacagg actataaaga taccaggcgt ttccccctgg 3480
aagctccctc gtgcgctctc ctgttccgac cctgccgctt accggatacc tgtccgcctt 3540
tctcccttcg ggaagcgtgg cgctttctca atgctcacgc tgtaggtatc tcagttcggt 3600
gtaggtcgtt cgctccaagc tgggctgtgt gcacgaaccc cccgttcagc ccgaccgctg 3660
cgccttatcc ggtaactatc gtcttgagtc caacccggta agacacgact tatcgccact 3720
ggcagcagcc actggtaaca ggattagcag agcgaggtat gtaggcggtg ctacagagtt 3780
cttgaagtgg tggcctaact acggctacac tagaaggaca gtatttggta tctgcgctct 3840
gctgaagcca gttaccttcg gaaaaagagt tggtagctct tgatccggca aacaaaccac 3900
cgctggtagc ggtggttttt ttgtttgcaa gcagcagatt acgcgcagaa aaaaaggatc 3960
tcaagaagat cctttgatct tttctacggg gtctgacgct cagtggaacg aaaactcacg 4020
ttaagggatt ttggtcatga gattatcaaa aaggatcttc acctagatcc ttttaaatta 4080
aaaatgaagt tttaaatcaa tctaaagtat atatgagtaa acttggtctg acagttacca 4140
atgcttaatc agtgaggcac ctatctcagc gatctgtcta tttcgttcat ccatagttgc 4200
ctgactcccc gtcgtgtaga taactacgat acgggagggc ttaccatctg gccccagtgc 4260
tgcaatgata ccgcgagacc cacgctcacc ggctccagat ttatcagcaa taaaccagcc 4320
agccggaagg gccgagcgca gaagtggtcc tgcaacttta tccgcctcca tccagtctat 4380
taattgttgc cgggaagcta gagtaagtag ttcgccagtt aatagtttgc gcaacgttgt 4440
tgccattgct acaggcatcg tggtgtcacg ctcgtcgttt ggtatggctt cattcagctc 4500
cggttcccaa cgatcaaggc gagttacatg atcccccatg ttgtgcaaaa aagcggttag 4560
ctccttcggt cctccgatcg ttgtcagaag taagttggcc gcagtgttat cactcatggt 4620
tatggcagca ctgcataatt ctcttactgt catgccatcc gtaagatgct tttctgtgac 4680
tggtgagtac tcaaccaagt cattctgaga atagtgtatg cggcgaccga gttgctcttg 4740

cccggcgtca atacgggata ataccgcgcc acatagcaga actttaaaag tgctcatcat 4800
tggaaaacgt tcttcggggc gaaaactctc aaggatctta ccgctgttga gatccagttc 4860
gatgtaaccc actcgtgcac ccaactgatc ttcagcatct tttactttca ccagcgtttc 4920
tgggtgagca aaaacaggaa ggcaaaatgc cgcaaaaaag ggaataaggg cgacacggaa 4980
atgttgaata ctcatactct tcctttttca atattattga agcatttatc agggttattg 5040
tctcatgagc ggatacatat ttgaatgtat ttagaaaaat aaacaaatag gggttccgcg 5100
cacatttccc cgaaaagtgc cacctgacgt ctaagaaacc attattatca tgacattaac 5160
ctataaaaat aggcgtatca cgaggccctt tcgtctcgcg cgtttcggtg atgacggtga 5220
aaacctctga cacatgcagc tcccggagac ggtcacagct tgtctgtaag cggatgccgg 5280
gagcagacaa gcccgtcagg gcgcgtcagc gggtgttggc gggtgtcggg gctggcttaa 5340
ctatgcggca tcagagcaga ttgtactgag agtgcaccat atgcggtgtg aaataccgca 5400
cagatgcgta aggagaaaat accgcatcag gcgccattcg ccattcaggc tgcgcaactg 5460
ttgggaaggg cgatcggtgc gggcctcttc gctattacgc cagctggcga aaggggggatg 5520
tgctgcaagg cgattaagtt gggtaacgcc agggttttcc cagtcacgac gttgtaaaac 5580
gacggccagt gccaagcttt gtttaaaata taacaaaatt gtgatcccac aaaatgaagt 5640
ggggcaaaat caaataatta atagtgtccg taaacttgtt ggtcttcaac tttttgagga 5700
acacgttgga cggcaaatcc gtgactataa cacaagttga tttaataatt ttagccaaca 5760
cgtcgggctg cgtgtttttt gccgacgcgt ctgtgtacac gttgattaac tggtcgatta 5820
aactgttgaa ataatttaat ttttggttct tctttaaatc tgtgatgaaa ttttttaaaa 5880
taactttaaa ttcttcattg gtaaaaaatg ccacgttttg caacttgtga gggtctaata 5940
tgaggtcaaa ctcagtagga gttttatcca aaaaagaaaa catgattacg tctgtacacg 6000
aacgcgtatt aacgcagagt gcaaagtata agagggttaa aaaatatatt ttacgcacca 6060
tatacgcatc gggttgatat cgttaatatg gatcaatttg aacagttgat taacgtgtct 6120
ctgctcaagt ctttgatcaa aacgcaaatc gacgaaaatg tgtcggacaa tatcaagtcg 6180
atgagcgaaa aactaaaaag gctagaatac gacaatctca cagacagcgt tgagatatac 6240
ggtattcacg acagcaggct gaataataaa aaaattagaa actattattt aaccctagaa 6300
agataatcat attgtgacgt acgttaaaga taatcatgcg taaaattgac gcatgtgttt 6360
tatcggtctg tatatcgagg tttatttatt aatttgaata gatattaagt tttattatat 6420
ttacacttac atactaataa taaattcaac aaacaattta tttatgttta tttatttatt 6480
aaaaaaaaac aaaaactcaa aatttcttct ataaagtaac aaaactttta aacattctct 6540
cttttacaaa aataaactta ttttgtactt taaaaacagt catgttgtat tataaaataa 6600

gtaattagct taacttatac ataatagaaa caaattatac ttattagtca gtcagaaaca 6660
actttggcac atatcaatat tatgctctcg acaaataact tttttgcatt ttttgcacga 6720
tgcatttgcc tttcgcctta ttttagaggg gcagtaagta cagtaagtac gtttttttcat 6780
tactggctct tcagtactgt catctgatgt accaggcact tcatttggca aaatattaga 6840
gatattatcg cgcaaatatc tcttcaaagt aggagcttct aaacgcttac gcataaacga 6900
tgacgtcagg ctcatgtaaa ggtttctcat aaattttttg cgactttgga ccttttctcc 6960
cttgctactg acattatggc tgtatataat aaaagaattt atgcaggcaa tgtttatcat 7020
tccgtacaat aatgccatag gccacctatt cgtcttccta ctgcaggtca tcacagaaca 7080
catttggtct agcgtgtcca ctccgccttt agtttgatta taatacataa ccatttgcgg 7140
tttaccggta ctttcgttga tagaagcatc ctcatcacaa gatgataata agtataccat 7200
cttagctggc ttcggtttat atgagacgag agtaaggggt ccgtcaaaac aaaacatcga 7260
tgttcccact ggcctggagc gactgttttt cagtacttcc ggtatctcgc gtttgtttga 7320
tcgcacggtt cccacaatgg ttgcggccgg ccagatttaa atgagcggcc gtaattaact 7380
cgcgttaaga tacattgatg agtttggaca aaccacaact agaatgcagt gaaaaaaatg 7440
ctttatttgt gaaatttgtg atgctattgc tttatttgta accattataa gctgcaataa 7500
acaagttaac aacaacaatt gcattcattt tatgtttcag gttcaggggg aggtgtggga 7560
ggttttttaa agcaagtaaa acctctacaa atgtggtatg gctgattatg atcatcacag 7620
gaacaggtgg tggcggccct cggtgcgctc gtactgctcc acgatggtgt agtcctcgtt 7680
gtgggaggtg atgtccagct tggcgtccac gtagtagtag ccgggcagct gcacgggctt 7740
cttggccatg tagatggact tgaactccac caggtagtgg ccgccgtcct tcagcttcag 7800
ggccttgtgg gtctcgccct tcagcacgcc gtcgcggggg tacaggcgct cggtggaggc 7860
ctcccagccc atggtcttct tctgcatcac ggggccgtcg gaggggaagt tcacgccgat 7920
gaacttcacc ttgtagatga agcagccgtc ctgcagggag gagtcctggg tcacggtcgc 7980
cacgccgccg tcctcgaagt tcatcacgcg ctcccacttg aagccctcgg ggaaggacag 8040
cttcttgtag tcggggatgt cggcggggtg cttcacgtac accttggagc cgtactggaa 8100
ctgggggggac aggatgtccc aggcgaaggg cagggggccg cccttggtca ccttcagctt 8160
cacggtgttg tggccctcgt aggggcggcc ctcgccctcg ccctcgatct cgaactcgtg 8220
gccgttcacg gtgccctcca tgcgcacctt gaagcgcatg aactcggtga tgacgttctc 8280
ggaggaggcc atggtaccgg tttgcgcttc ttcttgggtg gggtgggatc gactctagcg 8340
gtaccccgat tgtttagctt gttcagctgc gcttgtttat ttgcttagct ttcgcttagc 8400
gacgtgttca ctttgcttgt ttgaattgaa ttgtcgctcc gtagacgaag cgcctctatt 8460
tatactccgg cggtcgaggg ttcgaaatcg ataagcttgg atcctcgagg tcatcttcac 8520

ttacgtgatc tgatatttca ctgtcagaat cctcaccaac aagctcgtca tcgctttgca 8580
gaagagcaga gaggatatgc tcatcgtcta aagaactacc cattttatta tatattagtc 8640
acgatatcta taacaagaaa atatatatat aataagttat cacgtaagta gaacatgaaa 8700
taacaatata attatcgtat gagttaaatc ttaaaagtca cgtaaaagat aatcatgcgt 8760
cattttgact cacgcggtcg ttatagttca aaatcagtga cacttaccgc attgacaagc 8820
acgcctcacg ggagctccaa gcggcgactg agatgtccta aatgcacagc gacggattcg 8880
cgctatttag aaagagagag caatatttca agaatgcatg cgtcaatttt acgcagacta 8940
tctttctagg gttaaatata cttcaaggcc accgttatgg tgtcattagg gacgaggtcc 9000
aaaacgtcaa aatattttgt aatgatgccg tgaattacga tttcggtgtt aattaagtac 9060
tgacggacac accgaagccc cggcggcaac cctcagcgga tgccccgggg cttcacgttt 9120
tcccaggtca gaagcggttt tcgggagtag tgccccaact ggggtaacct ttgagttctc 9180
tcagttgggg gcgtagggtc gccgacatga cacaaggggt tgtgaccggg gtggacacgt 9240
acgcgggtgc ttacgaccgt cagtcgcgcg agcgcgaggc caggccgcat ggtacccatt 9300
gcttgtcatt tattaatttg gatgatgtca tttgttttta aaattgaact ggctttacga 9360
gtagaattct acgcgtaaaa cacaatcaag tatgagtcat aatctgatgt catgttttgt 9420
acacggctca taaccgaact ggctttacga gtagaattct acttgtaatg cacgatcagt 9480
ggatgatgtc atttgttttt caaatcgaga tgatgtcatg ttttgcacac ggctcataaa 9540
ctcgctttac gagtagaatt ctacgtgtaa cgcacgatcg attgatgagt catttgtttt 9600
gcaatatgat atcatacaat atgactcatt tgtttttcaa aaccgaactt gatttacggg 9660
tagaattcta cttgtaaagc acaatcaaaa agatgatgtc atttgttttt caaaactgaa 9720
ctcgctttac gagtagaatt ctacgtgtaa aacacaatca agaaatgatg tcatttgtta 9780
taaaaataaa agctgatgtc atgttttgca catggctcat aactaaactc gctttacggg 9840
tagaattcta cgcgtaaaac atgattgata attaaataat tcatttgcaa gctatacgtt 9900
aaatcaaacg gacgctcgag gttgcacaac actattatcg atttgcagtt cgggacataa 9960
atgtttaaat atatcgatgt ctttgtgatg cgcgcgacat ttttgtaggt tattgataaa 10020
atgaacggat acgttgcccg acattatcat taaatccttg gcgtagaatt tgtcgggtcc 10080
attgtccgtg tgcgctagta gcatgcccgt aacggacctc gtacttttgg cttcaaaggt 10140
tttgcgcaca gacaaaatgt gccacacttg cagctctgca tgtgtgcgcg ttaccacaaa 10200
tcccaacggc gcagtgtact tgttgtatgc aaataaatct cgataaaggc gcggcgcgcg 10260
aatgcagctg atcacgtacg ctcctcgtgt tccgttcaag gacggtgtta tcgacctcag 10320
attaatgttt atcggccgac tgttttcgta tccgctcacc aaacgcgttt ttgcattaac 10380

attgtatgtc ggcggatgtt ctatatctaa tttgaataaa taaacgataa ccgcgttggt 10440
tttagagggc ataataaaag aaatattgtt atcgtgttcg ccattagggc agtataaatt 10500
gacgttcatg ttggatattg tttcagttgc aagttgacac tggcggcgac aagcaattct 10560
aattggggta agttttcccg ttcttttctg ggttcttccc ttttgctcat ccttgctgca 10620
ctaccttcag gtgcaagttg agattcaggc caccatggga gatcccaccc cacccaagaa 10680
gaagcgcaaa ccggtcgcca ccatggcctc ctccgagaac gtcatcaccg agttcatgcg 10740
cttcaaggtg cgcatggagg gcaccgtgaa cggccacgag ttcgagatcg agggcgaggg 10800
cgagggccgc ccctacgagg gccacaacac cgtgaagctg aaggtgacca agggcggccc 10860
cctgcccttc gcctgggaca tcctgtcccc ccagttccag tacggctcca aggtgtacgt 10920
gaagcacccc gccgacatcc ccgactacaa gaagctgtcc ttccccgagg gcttcaagtg 10980
ggagcgcgtg atgaacttcg aggacggcgg cgtggcgacc gtgacccagg actcctccct 11040
gcaggacggc tgcttcatct acaaggtgaa gttcatcggc gtgaacttcc cctccgacgg 11100
ccccgtgatg cagaagaaga ccatgggctg ggaggcctcc accgagcgcc tgtacccccg 11160
cgacggcgtg ctgaagggcg agacccacaa ggccctgaag ctgaaggacg gcggccacta 11220
cctggtggag ttcaagtcca tctacatggc caagaagccc gtgcagctgc ccggctacta 11280
ctacgtggac gccaagctgg acatcacctc ccacaacgag gactacacca tcgtggagca 11340
gtacgagcgc accgagggcc gccaccacct gttcctgaga tctcgaccca agaaaaagcg 11400
gaaggtggag gacccgtaag atccaccgga tctagataac tgatcataat cagccatacc 11460
acatttgtag aggttttact tgctttaaaa aacctcccac acctccccct gaacctgaaa 11520
cataaaatga atgcaattgt tgttgttaac ttgtttattg cagcttataa tggttacaaa 11580
taaagcaata gcatcacaaa tttcacaaat aaagcatttt tttcactgca ttctagttgt 11640
ggtttgtcca aactcatcaa tgtatcttaa cgcgagttta aacctacccg ccatactcat 11700
cgatgcccag cgcgtcggtg aacatttgct cgaactcgaa gtcggccatg tccagggcgc 11760
cgtacggggc gctatcgtgg ggcgtgaagc ccggtcccgg gctatctcca tcgcccagca 11820
tatccaggtc gaaatcgtcc agggcgtcgg cgtgggccat tgccacatcc tctccatcca 11880
ggtgcagctc gtcgcccagg ctcacatcgg tcggcggggc ggtgctcagg cggcgcgtgt 11940
gtccggcggg caggaagctc aggcgggggg cggccaggcc ggcttcctcc ggggcatcgt 12000
catccggcag gtccagcagt ccctcgatgg tgctgccata gttgttcttg gtacgggcgc 12060
ggctgtaggc gctgcccatg gtggctagcc ccgctttcta aaggtgttat aaatcaaatt 12120
agttttgttt tttcttgaaa actttgcgtt tcctttgatc aacttaccgc cagggtacct 12180
gcagattgtt tagcttgttc agctgcgctt gtttatttgc ttagctttcg cttagcgacg 12240
tgttcacttt gcttgtttga attgaattgt cgctccgtag acgaagcgcc tctatttata 12300

ctccggcgct cgcggccgca tcgatctcta tcactgatag ggaggtctct atcactgata 12360
gggagttctc tatcactgat agggatgtct ctatcactga tagggatttc tctatcactg 12420
atagggaagt ctctatcact gatagggacc tctctatcac tgatagggaa atctctatca 12480
ctgataggga tctctctatc actgataggg acttctctat cactgatagg gacgtctcta 12540
tcactgatag ggaactctct atcactgata gggacatctc tatcactgat agggacttct 12600
ctatcactga tagggagtat gttctctctc ttctcttctc tctctctttc tcgaatgttc 12660
tctctcttct cttctctctc tctttctcga tggccggggc gcgccaggtt tcgactttca 12720
cttttctcta tcactgatag ggagtggtaa actcgacttt cacttttctc tatcactgat 12780
agggagtggt aaactcgact ttcacttttc tctatcactg atagggagtg gtaaactcga 12840
ctttcacttt tctctatcac tgatagggag tggtaaactc gactttcact tttctctatc 12900
actgataggg agtggtaaac tcgactttca cttttctcta tcactgatag ggagtggtaa 12960
actcgacttt cacttttctc tatcactgat agggagtggt aaactcgaaa acgagcgccg 13020
gagtataaat agaggcgctt cgtctacgga gcgacaattc aattcaaaca agcaaagtga 13080
acacgtcgct aagcgaaagc taagcaaata aacaagcgca gctgaacaag ctaaacaatc 13140
tgccctagga tctcagtggc tcctggagaa gctgcgatac ccctgggaga tgatgcccct 13200
gatgtacgtg atactgaaag gcgccgacgg agacgtcaat aaagcgcgcc aacggattga 13260
cgaaggtatg gggggttctta ccggttggga ctgtttccga ggtatcgatc gggtgtcact 13320
cacttcctgg gtgctcccat tttgtaactg ctaacgctta ttattgagtt tcaggacatc 13380
tgggatcttc ggtcgacgga gtctattccc aacagtgccc tggatcaaac actgccatca 13440
tgcagtttcc gtagcctgtt gggctacgct ccccgacttg acatccccca ttcttatcaa 13500
acaacaactc aaggcctgag acaacgagtg gtggaatttg cgcacgaagt cattggtttg 13560
tcctggtaaa agttaaaagg gttaactgga gggttaattg acacggtttc aactgatggc 13620
cttattgaca cacggatgaa agacttgcac gcttgacctt ctgtctgtac taataaaagt 13680
tacgttggct gggttttggg gtcataatgg ccccaaaatc gaatcgtcat aacttcttga 13740
aatacaactc acgtttaaga ccattcaaga gtattagatc atcgtctata atagcagatt 13800
tgaaatttac ttcacatttc ggtattgcag tgccccttgc ttccacaatg gaattaggtc 13860
ggtggtgcgt cgatcgtcgc aagtttatcg ttaaacagtc aataaaatga gcattttata 13920
tcgtgataca tatgagaaga tagaggtttc aattaaaaca aatccacatg gtgtcgctaa 13980
taaaattgtg cattttaagc gagttatatc ctctgatcaa gataaaatag aaaattcgat 14040
ttttgaatat tcaattataa gagcctgaat aactacaaca tgtagtgaat cgaaactgat 14100
ttatgacggt ttgtgaaggt tacacgtcct aagcatttgg attcaagaaa agcaagagat 14160

atgacgaatg taaactttat cgtatcaatg aagtaactag cgtccagaac agtacaaacc 14220
aacatcgtac cgtcgtattc cactccggtc gttgcaatat ctctaggtcc accgaaaaac 14280
actcatgacc aagatcgtgt cgtcgatctt ggtccaccga aacaccgatg tccatatcgt 14340
ttcgtcgaac ttggaccaac gattcatgca actgatgaca acgcggcccc cgggtcgtac 14400
caatatccga aaaatccaac tgttcttctc tgcctcgcag gtcaagccgt ggtcaatgaa 14460
tactcacgat tgcacaatct gaacatgttc gacggtgtag agttgcgcag tacgacgcgc 14520
cagtccggat gatagacttt ttacacgatc agcacgaccc actgcgctgc ggcaaaggtc 14580
gaaccgaaac aagaataaac cacgaagatc agatcgattc gacggaagaa gcaatcgaat 14640
gcaaagaaga atcggaacga agaaaactct aaagcatcgc atatttacaa agcataacgg 14700
aaaacccgca agttcaaact agtgattagt gtaagatgaa gcaaagcaga aatgtagtat 14760
ctagattttt cgacgttagt ttacaaagat aaaaaatgag gttggacata caatcgtggg 14820
tattcgtctg agttcgtcac aactgcaccg gaaactgtga aacagaatag agccaacctg 14880
tgcgcggaga atgttgaggt cattataagc ttccttagca tccacgggtg aaagtcgatc 14940
gacggaagcc tgcaagactc tgtcgatggg ctttcgtcct agaagaataa gattaaacct 15000
gaaatgtatt ctcccgtgga atggtttcat ttgagtaatt ctgtatcttc tccttcccaa 15060
ttccacgaac gcgacgaact ctaatacaaa caacataatg accacagtgc aaatgctgtt 15120
taacgataat agcgacatgc agccattctg ggctaccac gtgtggctct acttgcgatc 15180
caaaatgcag atcttcgtca agaccctgac cggcaagacc atcaccctgg aggtggagcc 15240
gagcgatacc atcgagaacg tgaaggccaa gatccaggac aaggagggca tcccgccgga 15300
tcagcagcgc ctgatcttcg ccggacgcca gctggaggat ggccgcaccc tgagcgacta 15360
caacatccag aaggagagca ccctgcacct ggtgctgcgc ctgcgcggtg gtatggtcag 15420
ccgcctggat aagtccaaag tcatcaactc cgcgttggag ctgttgaacg aagttggcat 15480
tgagggactg acgacccgca agttggcgca gaagctgggc gtggagcagc ccaccctcta 15540
ctggcacgtg aagaataagc gggcgctgct ggatgccctg gccatcgaga tgctcgaccg 15600
ccaccacacg cattttgcc cgttggaagg cgagtcctgg caggacttcc tccgcaataa 15660
cgccaagtcg ttccgctgcg ctctgctgtc ccaccgagac ggtgccaaag tccatctcgg 15720
cacgcgcccg accgaaaagc aatacgagac actggagaac cagctcgcgt tcctgtgcca 15780
gcaaggcttc agcctggaaa atgctctcta cgctctgagc gccgtcggtc actttaccct 15840
gggctgcgtg ctggaggacc aagagcatca agtcgcaaaa gaggagcgcg agaccccaac 15900
aaccgattcg atgcccccac tgctgcgtca ggcaatcgag ctgttcgatc atcaaggagc 15960
cgagccggca ttcctgttcg gcttggagct gattatctgc ggattggaaa agcaactgaa 16020
atgcgagtcg ggctcgggcc ccgcctacag ccgcgcccgc accaagaaca actacggcag 16080

caccatcgag ggcctgctgg atctgccgga tgatgatgcc ccggaggagg cgggcctggc 16140
cgccccgcgc ctgagcttcc tgccggccgg acacacccgc cgcctgtcga ccgccccgcc 16200
gaccgacgtg agcctgggcg atgagctgca cctggatggc gaggatgtgg cgatggccca 16260
cgccgatgcc ctggacgact tcgacctgga catgctgggc gatggcgata gcccgggacc 16320
gggattcacc ccgcacgata gcgcccccta cggcgccctg gatatggccg atttcgagtt 16380
cgagcagatg ttcaccgacg ccctgggcat cgatgagtac ggcggctaac accggtgatt 16440
gtccaccgca agctctactt gtgagacagc gttcctaaag agtgtgaaag tgcaaacaag 16500
tgatgaaacc aatagtgcaa agcaagttta gagggaaaat ttaaaaaatg caaaacagca 16560
gtagtactta acttttaaga ttgtgtttcg aaagccgaag tgaggctgtt ccatctgcca 16620
ccggaaaaaa acgacgacag cagaatcatc aacaagcaac atccatccga aaaaatccgg 16680
gaaaccggat cttcaaccaa ccatcctaca atctacaaac cagagattat atctcttcaa 16740
tcgtttccga catcggtcgg tttcggtgcc caaaatgatc tgataaacac ttatctctct 16800
gtagcttgca tgccattgcg agcgtatttt ggtagctggc cgttgccaaa cggctccgac 16860
aggtactgct attggaggtt gtgcacgacc acgttgagtt tgccttttga gttggagagt 16920
gtgtcttttc gtcatatatt cggccttttc aagggtgatt ttcaggctac gtaatgattg 16980
tatagtttaa ccagctaaaa catattgatg acaagttcta tttcagcacc acaaacaagc 17040
ctgttaatgt ctctcaccgc aaccattgtt ctgcgcgcgt tataatcagc atagaagttt 17100
atttctttg ggatgattca aatattacgt gacgcaaagt ttgccaattt tagaacccct 17160
ccctcctcca cgtaacggct tttgtgtgaa aaatttaaat tttgtgtata gaccgtagca 17220
tttcggaaga ccccctccct tactctgttg agttacgtaa aatttcaacg atccttttgt 17280
agttctgaat tttatatcag cgtgcagtgt tatgaagata tccacagtat aaaatattat 17340
tttattttaa attctatgct gattatcaat gtgttactag tggcttttca tactcatgtt 17400
gcgagctcga tttggcgcac ggtacttatc aaggcatgta tgtatgttgt ttgaagcaac 17460
tgtataactg tttgaaacta tctaattggt gagctcgttt catttagtat ataataatga 17520
taattgctat ggagacgtta tttactagca agtgatttga cgacctgaaa tcggaacaaa 17580
tagacaacgt ttttataaat acaataaatc agaactttcc attattgggt acaaagagtt 17640
gcgctatttc gatactgtca gatcagattt tccagcacaa cgataccttg atatgcgata 17700
acttagaatt agaccttcaa atccatctct ccagctatga acagtcatat agataaagcc 17760
aatggcgtta tgaggtagcg gaaagcgtca tctttccaat gctatctaag tacataattt 17820
gctatagctt tctattaatc gtagtttgag agatgcaaag tcagttatct cgtatcaagg 17880
tttgattgtt ttggaaatta gctaaacagt tgacattatc acccgtcttt aggggataag 17940

cgcatacaaa tgtgtattta gttgttcatt gaagtaacgt aagataggca agtatggaaa 18000

cgagctcacc aaacgtcgaa atacgtctaa taaatttgtg ttcagcagga tggttcaaaa 18060

tttatttgca tcacctcaaa attacagtac ctagtgctgt ttgtgacaaa catcaaaagg 18120

taaaatcaaa ctcgtggcgt cgtgcaatct ccatagaatg aacaatttct aaccgtattt 18180

gatggaaaga cattgagtct actatcctct taacagcatt gcacttgtct ataaacaata 18240

aataatttgt tctttttac attttctttc cccactttcg cccccccccc ccccaaaaat 18300

caatccctca aacaggatac gacatttgtt gcatctactt tccgaagcgt tccagcagac 18360

acagacactg gccggacgag gagaacatct ccgtcacccg cactccgtct gcgtcacggt 18420

cgccatgtgc cgattttcgt acccggtcac agtccagctc gccggataac aacggtggcg 18480

cgctcaatct ggacacgaaa tctaccaaag cgacgaccgc caccaccgac gacgaagagg 18540

ttatgtacga gaaacgcagc ccgaagtcca ttgaatctac cgagttgcgg tgccgtctgg 18600

aggaagcctt acacagtggc gctgctgctg ctgcggctgc tgaagaacct ctggcgggcg 18660

gaagcggttc ccactggaag agagaaagtt tcggctctac ggaggagatt cccactcgac 18720

ccgctcacag tgaaccggaa gataatggat ttgaaaacgg attggaagcg caccagtccc 18780

atattctgca cagcatacat cggaatgtgc aacgatcata atcagccata ccacatttgt 18840

agaggtttta cttgctttaa aaaacctccc acacctcccc ctgaacctga aacataaaat 18900

gaatgcaatt gttgttgtta acttgtttat tgcagcttat aatggttaca aataaagcaa 18960

tagcatcaca aatttcacaa ataaagcatt tttttcactg cattctagtt gtggtttgtc 19020

caaactcatc aatgtatctt aggcgcgccg ccc 19053

<210> 153
<211> 10540
<212> DNA
<213> artificial

<220>
<223> LA3491 plasmid sequence

<400> 153
ctaggcttta cgagtagaat tctacgcgta aaacacaatc aagtatgagt cataatctga 60

tgtcatgttt tgtacacggc tcataaccga actggcttta cgagtagaat tctacttgta 120

atgcacgatc agtggatgat gtcatttgtt tttcaaatcg agatgatgtc atgttttgca 180

cacggctcat aaactcgctt tacgagtaga attctacgtg taacgcacga tcgattgatg 240

agtcatttgt tttgcaatat gatatcatac aatatgactc atttgttttt caaaaccgaa 300

cttgatttac gggtagaatt ctacttgtaa agcacaatca aaaagatgat gtcatttgtt 360

tttcaaaact gaactcgctt tacgagtaga attctacgtg taaaacacaa tcaagaaatg 420

atgtcatttg ttataaaaat aaaagctgat gtcatgtttt gcacatggct cataactaaa 480

ctcgctttac gggtagaatt ctacgcgtaa aacatgattg ataattaaat aattcatttg 540
caagctatac gttaaatcaa acggacgctc gaggttgcac aacactatta tcgatttgca 600
gttcgggaca taaatgttta aatatatcga tgtctttgtg atgcgcgcga catttttgta 660
ggttattgat aaaatgaacg gatacgttgc ccgacattat cattaaatcc ttggcgtaga 720
atttgtcggg tccattgtcc gtgtgcgcta gcatgcccgt aacggacctc gtacttttgg 780
cttcaaaggt tttgcgcaca gacaaaatgt gccacacttg cagctctgca tgtgtgcgcg 840
ttaccacaaa tcccaacggc gcagtgtact tgttgtatgc aaataaatct cgataaaggc 900
gcggcgcgcg aatgcagctg atcacgtacg ctcctcgtgt tccgttcaag gacggtgtta 960
tcgacctcag attaatgttt atcggccgac tgttttcgta tccgctcacc aaacgcgttt 1020
ttgcattaac attgtatgtc ggcggatgtt ctatatctaa tttgaataaa taaacgataa 1080
ccgcgttggt tttagagggc ataataaaag aaatattgtt atcgtgttcg ccattagggc 1140
agtataaatt gacgttcatg ttggatattg tttcagttgc aagttgacac tggcggcgac 1200
aagcaattct aattggggta agttttcccg ttcttttctg ggttcttccc ttttgctcat 1260
ccttgctgca ctaccttcag gtgcaagttg agattcaggc caccatggga gcttcacgac 1320
gaacttgtca aacgatctca atggctcctg gagaagctgc gataccccctg ggagatgatg 1380
cccctgatgt acgtgatact gaaaggcgcc gacggagacg tcaataaagc gcgccaacgg 1440
attgacgaag gtatgggggt tcttaccggt tgggactgtt tccgaggtat cgatcgggtg 1500
tcactcactt cctgggtgct cccattttgt aactgctaac gcttattatt gagtttcagg 1560
acatctggga tcttcggtcg acggagtcta ttcccaacag tgccctggat caaacactgc 1620
catcatgcag tttccgtagc ctgttgggct acgctccccg acttgacatc cccccattctt 1680
atcaaacaac aactcaaggc ctgagacaac gagtggtgga atttgcgcac gaagtcattg 1740
gtttgtcctg gtaaaagtta aaagggttaa ctggagggtt aattgacacg gtttcaactg 1800
atggccttat tgacacacgg atgaaagact tgcacgcttg accttctgtc tgtactaata 1860
aaagttacgt tggctgggtt ttggggtcat aatggcccca aaatcgaatc gtcataactt 1920
cttgaaatac aactcacgtt taagaccatt caagagtatt agatcatcgt ctataatagc 1980
agatttgaaa tttacttcac atttcggtat tgcagtgccc cttgcttcca caatggaatt 2040
aggtcggtgg tgcgtcgatc gtcgcaagtt tatcgttaaa cagtcaataa aatgagcatt 2100
ttatatcgtg atacatatga gaagatagag gtttcaatta aaacaaatcc acatggtgtc 2160
gctaataaaa ttgtgcattt taagcgagtt atatcctctg atcaagataa aatagaaaat 2220
tcgatttttg aatattcaat tataagagcc tgaataacta caacatgtag tgaatcgaaa 2280
ctgatttatg acggtttgtg aaggttacac gtcctaagca tttggattca agaaaagcaa 2340

gagatatgac gaatgtaaac tttatcgtat caatgaagta actagcgtcc agaacagtac 2400
aaaccaacat cgtaccgtcg tattccactc cggtcgttgc aatatctcta ggtccaccga 2460
aaaacactca tgaccaagat cgtgtcgtcg atcttggtcc accgaaacac cgatgtccat 2520
atcgtttcgt cgaacttgga ccaacgattc atgcaactga tgacaacgcg gcccccgggt 2580
cgtaccaata tccgaaaaat ccaactgttc ttctctgcct cgcaggtcaa gccgtggtca 2640
atgaatactc acgattgcac aatctgaaca tgttcgacgg tgtagagttg cgcagtacga 2700
cgcgccagtc cggatgatag actttttaca cgatcagcac gacccactgc gctgcggcaa 2760
aggtcgaacc gaaacaagaa taaaccacga agatcagatc gattcgacgg aagaagcaat 2820
cgaatgcaaa gaagaatcgg aacgaagaaa actctaaagc atcgcatatt tacaaagcat 2880
aacggaaaac ccgcaagttc aaactagtga ttagtgtaag atgaagcaaa gcagaaatgt 2940
agtatctaga tttttcgacg ttagtttaca aagataaaaa atgaggttgg acatacaatc 3000
gtgggtattc gtctgagttc gtcacaactg caccggaaac tgtgaaacag aatagagcca 3060
acctgtgcgc ggagaatgtt gaggtcatta taagcttcct tagcatccac gggtgaaagt 3120
cgatcgacgg aagcctgcaa gactctgtcg atgggctttc gtcctagaag aataagatta 3180
aacctgaaat gtattctccc gtggaatggt ttcatttgag taattctgta tcttctcctt 3240
cccaattcca cgaacgcgac gaactctaat acaaacaaca taatgaccac agtgcaaatg 3300
ctgtttaacg ataatagcga catgcagcca ttctggggct accacgtgta gctctacttg 3360
tgagacagcg ttcctaaaga gtgtgaaagt gcaaacaagt gatgaaacca atagtgcaaa 3420
gcaagtttag agggaaaatt taaaaaatgc aaaacagcag tagtacttaa cttttaagat 3480
tgtgtttcga aagccgaagt gaggctgttc catctgccac cggaaaaaaa cgacgacagc 3540
agaatcatca acaagcaaca tccatccgaa aaaatccggg aaaccggatc ttcaaccaac 3600
catcctacaa tctacaaacc agagattata tctcttcaat cgtttccgac atcggtcggt 3660
ttcggtgccc aaaatgatct gataaacact tatctctctg tagcttgcat gccattgcga 3720
gcgtattttg gtagctggcc gttgccaaac ggctccgaca ggtactgcta ttggaggttg 3780
tgcacgacca cgttgagttt gccttttgag ttggagagtg tgtcttttcg tcatatattc 3840
ggccttttca agggtgattt tcaggctacg taatgattgt atagtttaac cagctaaaac 3900
atattgatga caagttctat ttcagcacca caaacaagcc tgttaatgtc tctcaccgca 3960
accattgttc tgcgcgcgtt ataatcagca tagaagttta ttttctttgg gatgattcaa 4020
atattacgtg acgcaaagtt tgccaatttt agaacccctc cctcctccac gtaacggctt 4080
ttgtgtgaaa aatttaaatt ttgtgtatag accgtagcat ttcggaagac cccctccctt 4140
actctgttga gttacgtaaa atttcaacga tccttttgta gttctgaatt ttatatcagc 4200
gtgcagtgtt atgaagatat ccacagtata aaatattatt ttattttaaa ttctatgctg 4260

attatcaatg tgttactagt ggcttttcat actcatgttg cgagctcgat ttggcgcacg 4320
gtacttatca aggcatgtat gtatgttgtt tgaagcaact gtataactgt ttgaaactat 4380
ctaattggtg agctcgtttc atttagtata taataatgat aattgctatg gagacgttat 4440
ttactagcaa gtgatttgac gacctgaaat cggaacaaat agacaacgtt tttataaata 4500
caataaatca gaactttcca ttattgggta caaagagttg cgctatttcg atactgtcag 4560
atcagatttt ccagcacaac gataccttga tatgcgataa cttagaatta gaccttcaaa 4620
tccatctctc cagctatgaa cagtcatata gataaagcca atggcgttat gaggtagcgg 4680
aaagcgtcat ctttccaatg ctatctaagt acataatttg ctatagcttt ctattaatcg 4740
tagtttgaga gatgcaaagt cagttatctc gtatcaaggt ttgattgttt tggaaattag 4800
ctaaacagtt gacattatca cccgtcttta ggggataagc gcatacaaat gtgtatttag 4860
ttgttcattg aagtaacgta agataggcaa gtatggaaac gagctcacca aacgtcgaaa 4920
tacgtctaat aaatttgtgt tcagcaggat ggttcaaaat ttatttgcat cacctcaaaa 4980
ttacagtacc tagtgctgtt tgtgacaaac atcaaaaggt aaaatcaaac tcgtggcgtc 5040
gtgcaatctc catagaatga acaatttcta accgtatttg atggaaagac attgagtcta 5100
ctatcctctt aacagcattg cacttgtcta taaacaataa ataatttgtt ctttttttaca 5160
ttttctttcc ccactttcgc cccccccccc cccaaaaatc aatccctcaa acaggatacg 5220
acatttgttg catctacttt ccgaagcgtt ccagcagaca cagacactgg ccggacgagg 5280
agaacatctc cgtcacccgc actccgtctg cgtcacggtc gccatgtgcc gattttcgta 5340
cccggtcaca gtccagctcg ccggataaca acggtggcgc gctcaatctg gacacgaaat 5400
ctaccaaagc gacgaccgcc accaccgacg acgaagaggt tatgtacgag aaacgcagcc 5460
cgaagtccat tgaatctacc gagttgcggt gccgtctgga ggaagcctta cacagtggcg 5520
ctgctgctgc tgcggctgct gaagaacctc tggcgggcgg aagcggttcc cactggaaga 5580
gagaaagttt cggctctacg gaggagattc ccactcgacc cgctcacagt gaaccggaag 5640
ataatggatt tgaaaacgga ttggaagcgc accagtccca tattctgcac agcatacatc 5700
ggaatgtgca acgatcataa tcagccatac cacatttgta gaggttttac ttgctttaaa 5760
aaacctccca cacctccccc tgaacctgaa acataaaatg aatgcaattg ttgttgttaa 5820
cttgtttatt gcagcttata atggttacaa ataaagcaat agcatcacaa atttcacaaa 5880
taaagcattt ttttcactgc attctagttg tggtttgtcc aaactcatca atgtatctta 5940
gggccgccac cgcggtggag ctccagcttt tgttcccttt agtgagggtt aattgcgcgc 6000
ttggcgtaat catggtcata gctgtttcct gtgtgaaatt gttatccgct cacaattcca 6060
cacaacatac gagccggaag cataaagtgt aaagcctggg gtgcctaatg agtgagctaa 6120

ctcacattaa ttgcgttgcg ctcactgccc gctttccagt cgggaaacct gtcgtgccag 6180
ctgcattaat gaatcggcca acgcgcgggg agaggcggtt tgcgtattgg gcgctcttcc 6240
gcttcctcgc tcactgactc gctgcgctcg gtcgttcggc tgcggcgagc ggtatcagct 6300
cactcaaagg cggtaatacg gttatccaca gaatcagggg ataacgcagg aaagaacatg 6360
tgagcaaaag gccagcaaaa ggccaggaac cgtaaaaagg ccgcgttgct ggcgtttttc 6420
cataggctcc gcccccctga cgagcatcac aaaaatcgac gctcaagtca gaggtggcga 6480
aacccgacag gactataaag ataccaggcg tttccccctg gaagctccct cgtgcgctct 6540
cctgttccga ccctgccgct taccggatac ctgtccgcct ttctcccttc gggaagcgtg 6600
gcgctttctc atagctcacg ctgtaggtat ctcagttcgg tgtaggtcgt tcgctccaag 6660
ctgggctgtg tgcacgaacc ccccgttcag cccgaccgct gcgccttatc cggtaactat 6720
cgtcttgagt ccaacccggt aagacacgac ttatcgccac tggcagcagc cactggtaac 6780
aggattagca gagcgaggta tgtaggcggt gctacagagt tcttgaagtg gtggcctaac 6840
tacggctaca ctagaaggac agtatttggt atctgcgctc tgctgaagcc agttaccttc 6900
ggaaaaagag ttggtagctc ttgatccggc aaacaaacca ccgctggtag cggtggtttt 6960
tttgtttgca agcagcagat tacgcgcaga aaaaaaggat ctcaagaaga tcctttgatc 7020
ttttctacgg ggtctgacgc tcagtggaac gaaaactcac gttaagggat tttggtcatg 7080
agattatcaa aaaggatctt cacctagatc cttttaaatt aaaaatgaag ttttaaatca 7140
atctaaagta tatatgagta aacttggtct gacagttacc aatgcttaat cagtgaggca 7200
cctatctcag cgatctgtct atttcgttca tccatagttg cctgactccc cgtcgtgtag 7260
ataactacga tacgggaggg cttaccatct ggccccagtg ctgcaatgat accgcgagac 7320
ccacgctcac cggctccaga tttatcagca ataaaccagc cagccggaag ggccgagcgc 7380
agaagtggtc ctgcaacttt atccgcctcc atccagtcta ttaattgttg ccgggaagct 7440
agagtaagta gttcgccagt taatagtttg cgcaacgttg ttgccattgc tacaggcatc 7500
gtggtgtcac gctcgtcgtt tggtatggct tcattcagct ccggttccca acgatcaagg 7560
cgagttacat gatcccccat gttgtgcaaa aaagcggtta gctccttcgg tcctccgatc 7620
gttgtcagaa gtaagttggc cgcagtgtta tcactcatgg ttatggcagc actgcataat 7680
tctcttactg tcatgccatc cgtaagatgc ttttctgtga ctggtgagta ctcaaccaag 7740
tcattctgag aatagtgtat gcggcgaccg agttgctctt gcccggcgtc aatacgggat 7800
aataccgcgc cacatagcag aactttaaaa gtgctcatca ttggaaaacg ttcttcgggg 7860
cgaaaactct caaggatctt accgctgttg agatccagtt cgatgtaacc cactcgtgca 7920
cccaactgat cttcagcatc ttttactttc accagcgttt ctgggtgagc aaaaacagga 7980
aggcaaaatg ccgcaaaaaa gggaataagg gcgacacgga aatgttgaat actcatactc 8040

ttccttttc aatattattg aagcatttat cagggttatt gtctcatgag cggatacata 8100
tttgaatgta tttagaaaaa taaacaaata ggggttccgc gcacatttcc ccgaaaagtg 8160
ccacctaaat tgtaagcgtt aatattttgt taaaattcgc gttaaatttt tgttaaatca 8220
gctcattttt taaccaatag gccgaaatcg gcaaaatccc ttataaatca aaagaataga 8280
ccgagatagg gttgagtgtt gttccagttt ggaacaagag tccactatta aagaacgtgg 8340
actccaacgt caaagggcga aaaaccgtct atcagggcga tggcccacta cgtgaaccat 8400
caccctaatc aagttttttg gggtcgaggt gccgtaaagc actaaatcgg aaccctaaag 8460
ggagcccccg atttagagct tgacggggaa agccggcgaa cgtggcgaga aaggaaggga 8520
agaaagcgaa aggagcgggc gctagggcgc tggcaagtgt agcggtcacg ctgcgcgtaa 8580
ccaccacacc cgccgcgctt aatgcgccgc tacagggcgc gtcccattcg ccattcaggc 8640
tgcgcaactg ttgggaaggg cgatcggtgc gggcctcttc gctattacgc cagctggcga 8700
aagggggatg tgctgcaagg cgattaagtt gggtaacgcc agggttttcc cagtcacgac 8760
gttgtaaaac gacggccagt gagcgcgcgt aatacgactc actatagggc gaattgggta 8820
ccgggcccaa gcttatcgat accgtcgaca tgcccgccgt gaccgtcgag aacccgctga 8880
cgctgccccg cgtatccgca cccgccgacg ccgtcgcacg tcccgtgctc accgtgacca 8940
ccgcgcccag cggtttcgag ggcgagggct tcccggtgcg ccgcgcgttc gccgggatca 9000
actaccgcca cctcgacccg ttcatcatga tggaccagat gggtgaggtg gagtacgcgc 9060
ccggggagcc caagggcacg ccctggcacc cgcaccgcgg cttcgagacc gtgacctaca 9120
tcgtcgacct cgaggggggg ccccccctcg aggttcccac aatggttaat tcgagctcgc 9180
ccggggatct aattcaatta gagactaatt caattagagc taattcaatt aggatccaag 9240
cttatcgatt tcgaaccctc gaccgccgga gtataaatag aggcgcttcg tctacggagc 9300
gacaattcaa ttcaaacaag caaagtgaac acgtcgctaa gcgaaagcta agcaaataaa 9360
caagcgcagc tgaacaagct aaacaatcgg ggtaccgcta gagtcgatcc caccccaccc 9420
aagaagaagc gcaaaccggt cgccaccatg gcctcctccg agaacgtcat caccgagttc 9480
atgcgcttca aggtgcgcat ggagggcacc gtgaacggcc acgagttcga gatcgagggc 9540
gagggcgagg gccgccccta cgagggccac aacaccgtga agctgaaggt gaccaagggc 9600
ggccccctgc ccttcgcctg ggacatcctg tccccccagt tccagtacgg ctccaaggtg 9660
tacgtgaagc accccgccga catccccgac tacaagaagc tgtccttccc cgagggcttc 9720
aagtgggagc gcgtgatgaa cttcgaggac ggcggcgtgg cgaccgtgac ccaggactcc 9780
tccctgcagg acggctgctt catctacaag gtgaagttca tcggcgtgaa cttcccctcc 9840
gacggccccg tgatgcagaa gaagaccatg ggctgggagg cctccaccga gcgcctgtac 9900

cccgcgacg gcgtgctgaa gggcgagacc cacaaggccc tgaagctgaa ggacggcggc 9960

cactacctgg tggagttcaa gtccatctac atggccaaga agcccgtgca gctgcccggc 10020

tactactacg tggacgccaa gctggacatc acctcccaca acgaggacta caccatcgtg 10080

gagcagtacg agcgcaccga gggccgccac cacctgttcc tgagatctcg acccaagaaa 10140

aagcggaagg tggaggaccc gtaagatcca ccggatctag ataactgatc ataatcagcc 10200

ataccacatt tgtagaggtt ttacttgctt taaaaaacct cccacacctc cccctgaacc 10260

tgaaacataa aatgaatgca attgttgttg ttaacttgtt tattgcagct tataatggtt 10320

acaaataaag caatagcatc acaaatttca caaataaagc atttttttca ctgcattcta 10380

gttgtggttt gtccaaactc atcaatgtat cttatcatgt ctggatcccg tttgacggta 10440

tcgataagct tgatggggat ccggaaccct taattaccgt tcgtataatg tatgctatac 10500

gaagttatta ggtccctcga cctgcagccc gggggatcca 10540

<210> 154
<211> 4446
<212> DNA
<213> artificial

<220>
<223> LA3515 plasmid sequence

<400> 154
ggccgccacc gcggtggagc tccagctttt gttcccttta gtgagggtta attgcgcgct 60

tggcgtaatc atggtcatag ctgtttcctg tgtgaaattg ttatccgctc acaattccac 120

acaacatacg agccggaagc ataaagtgta aagcctgggg tgcctaatga gtgagctaac 180

tcacattaat tgcgttgcgc tcactgcccg ctttccagtc gggaaacctg tcgtgccagc 240

tgcattaatg aatcggccaa cgcgcgggga gaggcggttt gcgtattggg cgctcttccg 300

cttcctcgct cactgactcg ctgcgctcgg tcgttcggct gcggcgagcg gtatcagctc 360

actcaaaggc ggtaatacgg ttatccacag aatcagggga taacgcagga aagaacatgt 420

gagcaaaagg ccagcaaaag gccaggaacc gtaaaaaggc cgcgttgctg gcgtttttcc 480

ataggctccg cccccctgac gagcatcaca aaaatcgacg ctcaagtcag aggtggcgaa 540

acccgacagg actataaaga taccaggcgt ttccccctgg aagctccctc gtgcgctctc 600

ctgttccgac cctgccgctt accggatacc tgtccgcctt tctcccttcg ggaagcgtgg 660

cgctttctca tagctcacgc tgtaggtatc tcagttcggt gtaggtcgtt cgctccaagc 720

tgggctgtgt gcacgaaccc cccgttcagc ccgaccgctg cgccttatcc ggtaactatc 780

gtcttgagtc caacccggta agacacgact tatcgccact ggcagcagcc actggtaaca 840

ggattagcag agcgaggtat gtaggcggtg ctacagagtt cttgaagtgg tggcctaact 900

acggctacac tagaaggaca gtatttggta tctgcgctct gctgaagcca gttaccttcg 960

gaaaaagagt tggtagctct tgatccggca aacaaaccac cgctggtagc ggtggttttt 1020
ttgtttgcaa gcagcagatt acgcgcagaa aaaaaggatc tcaagaagat cctttgatct 1080
tttctacggg gtctgacgct cagtggaacg aaaactcacg ttaagggatt ttggtcatga 1140
gattatcaaa aaggatcttc acctagatcc ttttaaatta aaaatgaagt tttaaatcaa 1200
tctaaagtat atatgagtaa acttggtctg acagttacca atgcttaatc agtgaggcac 1260
ctatctcagc gatctgtcta tttcgttcat ccatagttgc ctgactcccc gtcgtgtaga 1320
taactacgat acgggagggc ttaccatctg gccccagtgc tgcaatgata ccgcgagacc 1380
cacgctcacc ggctccagat ttatcagcaa taaaccagcc agccggaagg gccgagcgca 1440
gaagtggtcc tgcaacttta tccgcctcca tccagtctat taattgttgc cgggaagcta 1500
gagtaagtag ttcgccagtt aatagtttgc gcaacgttgt tgccattgct acaggcatcg 1560
tggtgtcacg ctcgtcgttt ggtatggctt cattcagctc cggttcccaa cgatcaaggc 1620
gagttacatg atcccccatg ttgtgcaaaa aagcggttag ctccttcggt cctccgatcg 1680
ttgtcagaag taagttggcc gcagtgttat cactcatggt tatggcagca ctgcataatt 1740
ctcttactgt catgccatcc gtaagatgct tttctgtgac tggtgagtac tcaaccaagt 1800
cattctgaga atagtgtatg cggcgaccga gttgctcttg cccggcgtca atacgggata 1860
ataccgcgcc acatagcaga actttaaaag tgctcatcat tggaaaacgt tcttcggggc 1920
gaaaactctc aaggatctta ccgctgttga gatccagttc gatgtaaccc actcgtgcac 1980
ccaactgatc ttcagcatct tttactttca ccagcgtttc tgggtgagca aaaacaggaa 2040
ggcaaaatgc cgcaaaaaag ggaataaggg cgacacggaa atgttgaata ctcatactct 2100
tcctttttca atattattga agcatttatc agggttattg tctcatgagc ggatacatat 2160
ttgaatgtat ttagaaaaat aaacaaatag gggttccgcg cacatttccc cgaaaagtgc 2220
cacctaaatt gtaagcgtta atattttgtt aaaattcgcg ttaaattttt gttaaatcag 2280
ctcatttttt aaccaatagg ccgaaatcgg caaaatccct tataaatcaa aagaatagac 2340
cgagataggg ttgagtgttg ttccagtttg gaacaagagt ccactattaa agaacgtgga 2400
ctccaacgtc aaagggcgaa aaaccgtcta tcagggcgat ggcccactac gtgaaccatc 2460
accctaatca agttttttgg ggtcgaggtg ccgtaaagca ctaaatcgga accctaaagg 2520
gagcccccga tttagagctt gacggggaaa gccggcgaac gtggcgagaa aggaagggaa 2580
gaaagcgaaa ggagcgggcg ctagggcgct ggcaagtgta gcggtcacgc tgcgcgtaac 2640
caccacaccc gccgcgctta atgcgccgct acagggcgcg tcccattcgc cattcaggct 2700
gcgcaactgt tgggaagggc gatcggtgcg ggcctcttcg ctattacgcc agctggcgaa 2760
agggggatgt gctgcaaggc gattaagttg ggtaacgcca gggttttccc agtcacgacg 2820

ttgtaaaacg acggccagtg agcgcgcgta atacgactca ctatagggcg aattgggtac 2880

cgggcccccc ctcgaggtcg acgatgtagg tcacggtctc gaagccgcgg tgcgggtgcc 2940

agggcgtgcc cttgggctcc ccgggcgcgt actccacctc acccatctgg tccatcatga 3000

tgaacgggtc gaggtggcgg tagttgatcc cggcgaacgc gcggcgcacc gggaagccct 3060

cgccctcgaa accgctgggc gcggtggtca cggtgagcac gggacgtgcg acggcgtcgg 3120

cgggtgcgga tacgcggggc agcgtcagcg ggttctcgac ggtcacggcg ggcatgtcga 3180

cggtatcgat aagcttgggc ccccccctcga ggttcccaca atggttaatt cgagctcgcc 3240

cggggatcta attcaattag agactaattc aattagagct aattcaatta ggatccaagc 3300

ttatcgattt cgaaccctcg accgccggag tataaataga ggcgcttcgt ctacggagcg 3360

acaattcaat tcaaacaagc aaagtgaaca cgtcgctaag cgaaagctaa gcaaataaac 3420

aagcgcagct gaacaagcta aacaatcggg gtaccgctag agtcgatccc accccaccca 3480

agaagaagcg caaaccggta ccatggcctc ctccgagaac gtcatcaccg agttcatgcg 3540

cttcaaggtg cgcatggagg gcaccgtgaa cggccacgag ttcgagatcg agggcgaggg 3600

cgagggccgc ccctacgagg gccacaacac cgtgaagctg aaggtgacca agggcggccc 3660

cctgcccttc gcctgggaca tcctgtcccc ccagttccag tacggctcca aggtgtacgt 3720

gaagcacccc gccgacatcc ccgactacaa gaagctgtcc ttccccgagg gcttcaagtg 3780

ggagcgcgtg atgaacttcg aggacggcgg cgtggcgacc gtgacccagg actcctccct 3840

gcaggacggc tgcttcatct acaaggtgaa gttcatcggc gtgaacttcc cctccgacgg 3900

ccccgtgatg cagaagaaga ccatgggctg ggaggcctcc accgagcgcc tgtacccccg 3960

cgacggcgtg ctgaagggcg agacccacaa ggccctgaag ctgaaggacg gcggccacta 4020

cctggtggag ttcaagtcca tctacatggc caagaagccc gtgcagctgc ccggctacta 4080

ctacgtggac gccaagctgg acatcacctc ccacaacgag gactacacca tcgtggagca 4140

gtacgagcgc accgagggcc gccaccacct gttcctgtga tgatcataat cagccatacc 4200

acatttgtag aggttttact tgctttaaaa aacctcccac acctccccct gaacctgaaa 4260

cataaaatga atgcaattgt tgttgttaac ttgtttattg cagcttataa tggttacaaa 4320

taaagcaata gcatcacaaa tttcacaaat aaagcatttt tttcactgca ttctagttgt 4380

ggtttgtcca aactcatcaa tgtatcttaa cgcgagttaa ttaaggccgc tcatttaaat 4440

ctggcc 4446

<210> 155
<211> 12991
<212> DNA
<213> artificial

<220>

<223> LA3545 Plasmid sequence

<400> 155

```
gggcggccgt ttttcttgaa atattgctct ctctttctaa atagcgcgaa tccgtcgctg      60
tgcatttagg acatctcagt cgccgcttgg agctcccaaa cgcgccagtg gtagtacaca     120
gtactgtggg tgttcagttt gaaatcctct tgcttctcca ttgtctcggt tacctttggt     180
caaatccatg ggttctattg cctatatact cttgcgatta ccagtgattg cgctattagc     240
tattagatgg attgttggcc aaacttgtcg cttaagtggc tgggaattgt aaccgtaggc     300
ccgagtgtaa tgatccccca taaaaagttt tcgcaatgcc tttatttttt gttgcaaatc     360
tctctttatt ctgcggtatt cttcattatt gcggggatgg ggaaagtgtt tatatagaag     420
caacttacga ttgaacccaa atgcacctga caagcaaggt caaagggcca gatttttaaa     480
tatattattt agtcttagga ctctctattt gcaattaaat tactttgcta cctgagggtt     540
aaatcttccc cattgataat aataattcca ctatatgttc aattgggttt caccgcgctt     600
agttacatga cgagccctaa tgagccgtcg gtggtctata aactgtgcct tacaaatact     660
tgcaactctt ctcgttttga agtcagcaga gttattgcta attgctaatt gctaattgct     720
tttaactgat ttcttcgaaa ttggtgctat gtttatggcg ctattaacaa gtatgaatgt     780
caggtttaac caggggatgc ttaattgtgt tctcaacttc aaaggcagaa atgtttactc     840
ttgaccatgg gtttaggtat aatgttatca agctcctcga gttaacgtta cgttaacgtt     900
aacgttcgag gtcgactcta gcactgggaa gttgacgttg atatagagcc gaattgaact     960
tcaccgctgc ttggtaatta ctctacaagt tcatttagga gaaccggatt cgaaagatga    1020
ttttccagcg tttagctttc agatggccgc atacattttg caccaccaaa ccgaaactca    1080
ctagcgtatc caatcgttcg ttttttggtg ccggtgtgtt acgaacttta gctatcaagc    1140
taaagcaatt tgctctggtc ttccgtgcta aaaagaaaaa aaaactgttt ttttttttggt    1200
tttgatattt gcgctatttt tacttgggcc ttaattgaac aaacttttga aagtttccac    1260
agcgaaatcg ttttcgacga tgccattttt ggtaacattt gcattttctt gctcaaattg    1320
cttgcaaaac ccgtgaaaga cattaatatt cgatagtgtc atccaaaatc acgaaaatga    1380
ttgttgcaaa acgttgaaca atttacacat gtaaaaaaca accatcgatt aatgtttatt    1440
caaacttttt acaagaaggg ttattctgat caatgtcacc ccgctgatga atgttacccc    1500
ggattacact tctcgaaaag tggttcaaaa tgctacttga gaatttttat ctgtcaaagg    1560
aagcaaattc gagtcgaatt aaatggtata gtcctgaatt aggtttccat ttacttacag    1620
gtattccact aaatagctgg aagatttatt ttacacaata atgataattc gtaccccaaa    1680
gagtgtagcc ctactttttt ctctcttttt tttttgtaaa ttttcatcgc tgcgtgccag    1740
cttaccgaca tgtcgcgaca gcataaagag cctgtcaaga gatgaagaaa aatgacaagg    1800
```

agtcagtggt caggtctctg tatcaatatt tgacgtcctg actttccaat ataccttttcc 1860
ttaaagagta gagatcatgc gatacgtgaa taaatatcgt ttggacttcg aaatagaaca 1920
taatttaagg tagctgatca gtagttgaac atcttcagac ttctgggaca agaagtgttt 1980
ttttgtttgt agaaaaggtt tttgttaaat tatatttgta agataattca atgaatatat 2040
ctctgattca gtaatcaatc cgtaccacgc accgtttaag aaacaccctg taggtttgca 2100
tcacgtctca gacaaaagtg tatcgatgtg cgaacactgc ataccggcgc tttgcaaata 2160
atgccaaatt tagatatgca ttacattgtc acttcgcaaa acacacactc ccaaatgcgt 2220
cggaaacctc acccgaacgc acgatcgtaa cgcgatcgat cgccgattga ttgatcggaa 2280
ttaactatct caatcgatcc ttctatggac tgatgcatgg gccggcactt ccgagtataa 2340
aaccccggta aacccaagga atcactcaca atcggatttt gacgctcgct ctggtacagt 2400
tcgatacggt ctagtgaaac cgaggataac gacgaaggtt tttccccatt gatccaggtc 2460
ggtgtttatg attggtggaa aaagactcga gaaaagttcc atcgaagccg ttggaaatgt 2520
gccgtcttcc tgtgacgtct tgtggatcca gttccttgtt cacgtctggt gatcgtgtaa 2580
aatgtgctgt cttgtggcgt catatgtgtt ccagatccag tgattacgat ccgatgtgat 2640
gttgatccct tgtgaacgtc ttatcctgtt ccgtgtgcac catgcataat gtcgtattac 2700
gtaagttctg aagtgaaaca gaagagtgaa ttgaaagttt ttttattcaa catcaaccta 2760
aatatggact ttactttcca agaaaattat gcctgatcaa ctgtggatag ttacaaaaaa 2820
aaaaggttta ttaattaaat tttatgatta cataatgtgt tgaaaagaac aactgaaatt 2880
ttagaagaag atcttttcgt gcatcaggct ttgccaatta attgatgata aattatcata 2940
gcaaattaac gtagagacta aaaggtatat cgtcaaatag ggcttctttt gacactattt 3000
tggcattctt gctctttgag aacttgcaac cctaaaatgg gatcttcatc agcctagtgg 3060
ttagattcag cagctacaaa gcaaaaccat gctgaagggt tcgattcccg gtcgtttcag 3120
gatcttttcg taattgaaat atccttgact accctaagta tcattgtgct tgccatttac 3180
gaatatacat attacgatat acgaatgaga aaatgacaac tttggaaaat aaagctctca 3240
atgtttcaat aagaaataaa tactacatca gtattgaagg ctaataacaa ttacagatta 3300
gaacctttaa acatcatttc tgcaacaggc tggataaagt acagttggag gattaaatta 3360
tgcgattttg caatttttttc cgattaaatt catatttatt cctggtttgg tttttacaaa 3420
aaatattttt acatgacgtt tgaccccgat tccctcaact ttgattgtta tattttttttt 3480
tggacaggtt gagtttgtgg gttttttcct agtgttgctt tgctttatgg gctctggtta 3540
tttaaaatta aaatttgaca atcttactac acactccgaa aaaatcatgc gattttacgt 3600
cttttggatg cacataaaag aagcgagcca aatgaggtga atttgtgtca cattttaaat 3660
acgatggtgt ctgattcggg aaatgtcaat gatagtgtca ttcaatcata atgtgaatta 3720

cgtccgcagt aattttcatt atttttaaga gtgtactact atttacacta caaaaatttt 3780
gataccccag gggggaacga ggtcccggat gtccagctgg ccagattgtt ggcaacgagc 3840
cctgtaccta ttgatcgagt caccaaagca ctcctcaagt gttttaatct cgaccagacg 3900
gtggacctcg gttgttctca ttctcggagg gcgatttcgc aatcattagt accaaccaca 3960
tgtcgaagtc gggagatgtt ataaaattat aaccaattat tcaaaaaatg acatcattca 4020
atttgaacaa acgttcgata gaaattatat atgatttcac atgatattaa actacgaaga 4080
aaattttaca taaggaagtg gtataaaacg taatatgctt aataaaaact ttaacccttt 4140
tgggaggata atattcagaa gttctgattc agaaccatct ctcatgttat gttcgttttt 4200
tgttgcttgt cctttatatg ccacatgaac aataacacca atatctatcc catttccagg 4260
acctaacgga ccttgaagcg gcgccactag taaaccacca tgggcagccg cctggataag 4320
tccaaagtca tcaactccgc gttggagctg ttgaacgaag ttggcattga gggactgacg 4380
acccgcaagt tggcgcagaa gctgggcgtg gagcagccca ccctctactg gcacgtgaag 4440
aataagcggg cgctgctgga tgccctggcc atcgagatgc tcgaccgcca ccacacgcat 4500
ttttgcccgt tggaaggcga gtcctggcag gacttcctcc gcaataacgc caagtcgttc 4560
cgctgcgctc tgctgtccca ccgagacggt gccaaagtcc atctcggcac gcgcccgacc 4620
gaaaagcaat acgagacact ggagaaccag ctcgcgttcc tgtgccagca aggcttcagc 4680
ctggaaaatg ctctctacgc tctgagcgcc gtcggtcact ttaccctggg ctgcgtgctg 4740
gaggaccaag agcatcaagt cgcaaaagag gagcgcgaga ccccaacaac cgattcgatg 4800
cccccactgc tgcgtcaggc aatcgagctg ttcgatcatc aaggagccga gccggcattc 4860
ctgttcggct tggagctgat tatctgcgga ttggaaaagc aactgaaatg cgagtcgggc 4920
tcgggccccg cctacagccg cgcccgcacc aagaacaact acggcagcac catcgagggc 4980
ctgctggatc tgccggatga tgatgccccg gaggaggcgg gcctggccgc cccgcgcctg 5040
agcttcctgc cggccggaca cacccgccgc ctgtcgaccg ccccgccgac cgacgtgagc 5100
ctgggcgatg agctgcacct ggatggcgag gatgtggcga tggcccacgc cgatgccctg 5160
gacgacttcg acctggacat gctgggcgat ggcgatagcc cgggaccggg attcaccccg 5220
cacgatagcg ccccctacgg cgccctggat atggccgatt tcgagttcga gcagatgttc 5280
accgacgccc tgggcatcga tgagtacggc ggctaacacc ggaaactcgc gttaagatac 5340
attgatgagt ttggacaaac cacaactaga atgcagtgaa aaaaatgctt tatttgtgaa 5400
atttgtgatg ctattgcttt atttgtaacc attataagct gcaataaaca agttaacaac 5460
aacaattgca ttcattttat gtttcaggtt cagggggagg tgtgggaggt tttttaaagc 5520
aagtaaaacc tctacaaatg tggtatggct gattatgatc agttatctag atccggtgga 5580

tcttacgggt cctccacctt ccgctttttc ttgggtcgag atctcaggaa caggtggtgg 5640
cggccctcgg tgcgctcgta ctgctccacg atggtgtagt cctcgttgtg ggaggtgatg 5700
tccagcttgg cgtccacgta gtagtagccg ggcagctgca cgggcttctt ggccatgtag 5760
atggacttga actccaccag gtagtggccg ccgtccttca gcttcagggc cttgtgggtc 5820
tcgcccttca gcacgccgtc gcgggggtac aggcgctcgg tggaggcctc ccagcccatg 5880
gtcttcttct gcatcacggg gccgtcggag gggaagttca cgccgatgaa cttcaccttg 5940
tagatgaagc agccgtcctg cagggaggag tcctgggtca cggtcgccac gccgccgtcc 6000
tcgaagttca tcacgcgctc ccacttgaag ccctcgggga aggacagctt cttgtagtcg 6060
gggatgtcgg cggggtgctt cacgtacacc ttggagccgt actggaactg gggggacagg 6120
atgtcccagg cgaagggcag ggggccgccc ttggtcacct tcagcttcac ggtgttgtgg 6180
ccctcgtagg ggcggccctc gccctcgccc tcgatctcga actcgtggcc gttcacggtg 6240
ccctccatgc gcaccttgaa gcgcatgaac tcggtgatga cgttctcgga ggaggccatg 6300
gtggcgaccg gtttgcgctt cttcttgggt ggggtgggat ctcccatggt ggcctgaatc 6360
tcaacttgca cctgaaggta gtgcagcaag gatgagcaaa agggaagaac ccagaaaaga 6420
acgggaaaac ttaccccaat tagaattgct tgtcgccgcc agtgtcaact tgcaactgaa 6480
acaatatcca acatgaacgt caatttatac tgccctaatg gcgaacacga taacaatatt 6540
tcttttatta tgccctctaa aaccaacgcg gttatcgttt atttattcaa attagatata 6600
gaacatccgc cgacatacaa tgttaatgca aaaacgcgtt tggtgagcgg atacgaaaac 6660
agtcggccga taaacattaa tctgaggtcg ataacaccgt ccttgaacgg aacacgagga 6720
gcgtacgtga tcagctgcat tcgcgcgccg cgcctttatc gagatttatt tgcatacaac 6780
aagtacactg cgccgttggg atttgtggta acgcgcacac atgcagagct gcaagtgtgg 6840
cacattttgt ctgtgcgcaa aacctttgaa gccaaaagta cgaggtccgt tacgggcatg 6900
ctagcgcaca cggacaatgg acccgacaaa ttctacgcca aggatttaat gataatgtcg 6960
ggcaacgtat ccgttcattt tatcaataac ctacaaaaat gtcgcgcgca tcacaaagac 7020
atcgatatat ttaaacattt atgtcccgaa ctgcaaatcg ataatagtgt tgtgcaacct 7080
cgagcgtccg tttgatttaa cgtatagctt gcaaatgaat tatttaatta tcaatcatgt 7140
tttacgcgta gaattctacc cgtaaagcga gtttagttat gagccatgtg caaaacatga 7200
catcagcttt tatttttata acaaatgaca tcatttcttg attgtgtttt acacgtagaa 7260
ttctactcgt aaagcgagtt cagttttgaa aaacaaatga catcatcttt ttgattgtgc 7320
tttacaagta gaattctacc cgtaaatcaa gttcggtttt gaaaaacaaa tgagtcatat 7380
tgtatgatat catattgcaa aacaaatgac tcatcaatcg atcgtgcgtt acacgtagaa 7440
ttctactcgt aaagcgagtt tatgagccgt gtgcaaaaca tgacatcatc tcgatttgaa 7500

aaacaaatga catcatccac tgatcgtgca ttacaagtag aattctactc gtaaagccag 7560
ttcggttatg agccgtgtac aaaacatgac atcagattat gactcatact tgattgtgtt 7620
ttacgcgtag aattctactc gtaaagccag ttcaatttta aaaacaaatg acatcatcca 7680
aattaataaa tgacaagcaa tgggtaccat gcggccgctc atttaaatct ggccggcctg 7740
gccgatctga caatgttcag tgcagagact cggctacgcc tcgtggactt tgaagttgac 7800
caacaatgtt tattcttacc tctaatagtc ctctgtggca aggtcaagat tctgttagaa 7860
gccaatgaag aacctggttg ttcaataaca ttttgttcgt ctaatatttc actaccgctt 7920
gacgttggct gcacttcatg tacctcatct ataaacgctt cttctgtatc gctctggacg 7980
tcatcttcac ttacgtgatc tgatatttca ctgtcagaat cctcaccaac aagctcgtca 8040
tcgctttgca gaagagcaga gaggatatgc tcatcgtcta aagaactacc cattttatta 8100
tatattagtc acgatatcta taacaagaaa atatatatat aataagttat cacgtaagta 8160
gaacatgaaa taacaatata attatcgtat gagttaaatc ttaaaagtca cgtaaaagat 8220
aatcatgcgt cattttgact cacgcggtcg ttatagttca aaatcagtga cacttaccgc 8280
attgacaagc acgcctcacg ggagctccaa gcggcgactg agatgtccta aatgcacagc 8340
gacggattcg cgctatttag aaagagagag caatatttca agaatgcatg cgtcaatttt 8400
acgcagacta tctttctagg gttaaaaaag atttgcgctt tactcgacct aaactttaaa 8460
cacgtcatag aatcttcgtt tgacaaaaac cacattgtgg ccaagctgtg tgacgcgacg 8520
cgcgctaaag aatggcaaac caagtcgcgc gagcgtcgac ctgcaggcat gcaagcttgc 8580
atgcctgcag gtcgaaattc gtaatcatgg tcatagctgt ttcctgtgtg aaattgttat 8640
ccgctcacaa ttccacacaa catacgagcc ggaagcataa agtgtaaagc ctggggtgcc 8700
taatgagtga gctaactcac attaattgcg ttgcgctcac tgcccgcttt ccagtcggga 8760
aacctgtcgt gccagctgca ttaatgaatc ggccaacgcg cggggagagg cggtttgcgt 8820
attgggcgct cttccgcttc ctcgctcact gactcgctgc gctcggtcgt tcggctgcgg 8880
cgagcggtat cagctcactc aaaggcggta atacggttat ccacagaatc aggggataac 8940
gcaggaaaga acatgtgagc aaaaggccag caaaaggcca ggaaccgtaa aaaggccgcg 9000
ttgctggcgt ttttccatag gctccgcccc cctgacgagc atcacaaaaa tcgacgctca 9060
agtcagaggt ggcgaaaccc gacaggacta taaagatacc aggcgtttcc ccctggaagc 9120
tccctcgtgc gctctcctgt tccgaccctg ccgcttaccg gatacctgtc cgcctttctc 9180
ccttcgggaa gcgtggcgct ttctcaatgc tcacgctgta ggtatctcag ttcggtgtag 9240
gtcgttcgct ccaagctggg ctgtgtgcac gaaccccccg ttcagcccga ccgctgcgcc 9300
ttatccggta actatcgtct tgagtccaac ccggtaagac acgacttatc gccactggca 9360

gcagccactg gtaacaggat tagcagagcg aggtatgtag gcggtgctac agagttcttg 9420
aagtggtggc ctaactacgg ctacactaga aggacagtat ttggtatctg cgctctgctg 9480
aagccagtta ccttcggaaa aagagttggt agctcttgat ccggcaaaca aaccaccgct 9540
ggtagcggtg gtttttttgt ttgcaagcag cagattacgc gcagaaaaaa aggatctcaa 9600
gaagatcctt tgatcttttc tacggggtct gacgctcagt ggaacgaaaa ctcacgttaa 9660
gggattttgg tcatgagatt atcaaaaagg atcttcacct agatcctttt aaattaaaaa 9720
tgaagtttta aatcaatcta aagtatatat gagtaaactt ggtctgacag ttaccaatgc 9780
ttaatcagtg aggcacctat ctcagcgatc tgtctatttc gttcatccat agttgcctga 9840
ctccccgtcg tgtagataac tacgatacgg gagggcttac catctggccc cagtgctgca 9900
atgataccgc gagacccacg ctcaccggct ccagatttat cagcaataaa ccagccagcc 9960
ggaagggccg agcgcagaag tggtcctgca actttatccg cctccatcca gtctattaat 10020
tgttgccggg aagctagagt aagtagttcg ccagttaata gtttgcgcaa cgttgttgcc 10080
attgctacag gcatcgtggt gtcacgctcg tcgtttggta tggcttcatt cagctccggt 10140
tcccaacgat caaggcgagt tacatgatcc cccatgttgt gcaaaaaagc ggttagctcc 10200
ttcggtcctc cgatcgttgt cagaagtaag ttggccgcag tgttatcact catggttatg 10260
gcagcactgc ataattctct tactgtcatg ccatccgtaa gatgcttttc tgtgactggt 10320
gagtactcaa ccaagtcatt ctgagaatag tgtatgcggc gaccgagttg ctcttgcccg 10380
gcgtcaatac gggataatac cgcgccacat agcagaactt taaaagtgct catcattgga 10440
aaacgttctt cggggcgaaa actctcaagg atcttaccgc tgttgagatc cagttcgatg 10500
taacccactc gtgcacccaa ctgatcttca gcatctttta ctttcaccag cgtttctggg 10560
tgagcaaaaa caggaaggca aaatgccgca aaaaagggaa taagggcgac acggaaatgt 10620
tgaatactca tactcttcct ttttcaatat tattgaagca tttatcaggg ttattgtctc 10680
atgagcggat acatatttga atgtatttag aaaaataaac aaataggggt tccgcgcaca 10740
tttccccgaa aagtgccacc tgacgtctaa gaaaccatta ttatcatgac attaacctat 10800
aaaaataggc gtatcacgag gccctttcgt ctcgcgcgtt tcggtgatga cggtgaaaac 10860
ctctgacaca tgcagctccc ggagacggtc acagcttgtc tgtaagcgga tgccgggagc 10920
agacaagccc gtcagggcgc gtcagcgggt gttggcgggt gtcggggctg gcttaactat 10980
gcggcatcag agcagattgt actgagagtg caccatatat gcggtgtgaa ataccgcaca 11040
gatgcgtaag gagaaaatac cgcatcaggc gccattcgcc attcaggctg cgcaactgtt 11100
gggaagggcg atcggtgcgg gcctcttcgc tattacgcca gctggcgaaa gggggatgtg 11160
ctgcaaggcg attaagttgg gtaacgccag ggttttccca gtcacgacgt tgtaaaacga 11220
cggccagtgc caagctttgt ttaaaatata acaaaattgt gatcccacaa aatgaagtgg 11280

ggcaaaatca aataattaat agtgtccgta aacttgttgg tcttcaactt tttgaggaac 11340

acgttggacg gcaaatccgt gactataaca caagttgatt taataatttt agccaacacg 11400

tcgggctgcg tgtttttgc cgacgcgtct gtgtacacgt tgattaactg gtcgattaaa 11460

ctgttgaaat aatttaattt ttggttcttc tttaaatctg tgatgaaatt ttttaaaata 11520

actttaaatt cttcattggt aaaaaatgcc acgttttgca acttgtgagg gtctaatatg 11580

aggtcaaact cagtaggagt tttatccaaa aaagaaaaca tgattacgtc tgtacacgaa 11640

cgcgtattaa cgcagagtgc aaagtataag agggttaaaa aatatatttt acgcaccata 11700

tacgcatcgg gttgatatcg ttaatatgga tcaatttgaa cagttgatta acgtgtctct 11760

gctcaagtct ttgatcaaaa cgcaaatcga cgaaaatgtg tcggacaata tcaagtcgat 11820

gagcgaaaaa ctaaaaaggc tagaatacga caatctcaca gacagcgttg agatatacgg 11880

tattcacgac agcaggctga ataataaaaa aattagaaac tattatttaa ccctagaaag 11940

ataatcatat tgtgacgtac gttaaagata atcatgcgta aaattgacgc atgtgtttta 12000

tcggtctgta tatcgaggtt tatttattaa tttgaataga tattaagttt tattatattt 12060

acacttacat actaataata aattcaacaa acaatttatt tatgtttatt tatttattaa 12120

aaaaaaacaa aaactcaaaa tttcttctat aaagtaacaa aacttttaaa cattctctct 12180

tttacaaaaa taaacttatt ttgtacttta aaaacagtca tgttgtatta taaaataagt 12240

aattagctta acttatacat aatagaaaca aattatactt attagtcagt cagaaacaac 12300

tttggcacat atcaatatta tgctctcgac aaataacttt tttgcatttt ttgcacgatg 12360

catttgcctt tcgccttatt ttagaggggc agtaagtaca gtaagtacgt tttttcatta 12420

ctggctcttc agtactgtca tctgatgtac caggcacttc atttggcaaa atattagaga 12480

tattatcgcg caaatatctc ttcaaagtag gagcttctaa acgcttacgc ataaacgatg 12540

acgtcaggct catgtaaagg tttctcataa attttttgcg actttggacc ttttctccct 12600

tgctactgac attatggctg tatataataa aagaatttat gcaggcaatg tttatcattc 12660

cgtacaataa tgccataggc cacctattcg tcttcctact gcaggtcatc acagaacaca 12720

tttggtctag cgtgtccact ccgcctttag tttgattata atacataacc atttgcggtt 12780

taccggtact ttcgttgata gaagcatcct catcacaaga tgataataag tataccatct 12840

tagctggctt cggtttatat gagacgagag taaggggtcc gtcaaaacaa aacatcgatg 12900

ttcccactgg cctggagcga ctgtttttca gtacttccgg tatctcgcgt ttgtttgatc 12960

gcacggttcc cacaatggtt gcggccagcc c 12991

<210> 156
<211> 18411
<212> DNA

<213>  artificial

<220>
<223>  LA3604 Plasmid sequence

<400>  156
ttaaaatgaa tgtaagcact ttattaacga aatctttggg aatatttcgc tcatcagcat 60

tttatttgag caggagtccg agatgcccgg gcggcgcgaa actcccctgc aggataactt 120

cgtatagcat acattatacg aagttatcct agggaagttc ctatactttc tagagaatag 180

gaacttcgga ataggaactt cttcgaacgg ccaaaaaggc cggccggggc acgggcgccg 240

tttttcttga aatattgctc tctctttcta aatagcgcga atccgtcgct gtgcatttag 300

gacatctcag tcgccgcttg gagctcccaa acgcgccagt ggtagtacac agtactgtgg 360

gtgttcagtt tgaaatcctc ttgcttctcc attgtctcgg ttacctttgg tcaaatccat 420

gggttctatt gcctatatac tcttgcgatt accagtgatt gcgctattag ctattagatg 480

gattgttggc caaacttgtc gcttaagtgg ctgggaattg taaccgtagg cccgagtgta 540

atgatccccc ataaaaagtt ttcgcaatgc ctttattttt tgttgcaaat ctctctttat 600

tctgcggtat tcttcattat tgcggggatg gggaaagtgt ttatatagaa gcaacttacg 660

attgaaccca aatgcacctg acaagcaagg tcaaagggcc agatttttaa atatattatt 720

tagtcttagg actctctatt tgcaattaaa ttactttgct acctgagggt taaatcttcc 780

ccattgataa taataattcc actatatgtt caattgggtt tcaccgcgct tagttacatg 840

acgagcccta atgagccgtc ggtggtctat aaactgtgcc ttacaaatac ttgcaactct 900

tctcgttttg aagtcagcag agttattgct aattgctaat tgctaattgc ttttaactga 960

tttcttcgaa attggtgcta tgtttatggc gctattaaca agtatgaatg tcaggtttaa 1020

ccaggggatg cttaattgtg ttctcaactt caaaggcaga aatgtttact cttgaccatg 1080

ggtttaggta taatgttatc aagctcctcg agttaacgtt acgttaacgt taacgttcga 1140

ggtcgactct agacaccggt gttagccgcc gtactcatcg atgcccaggg cgtcggtgaa 1200

catctgctcg aactcgaaat cggccatatc cagggcgccg taggggggcgc tatcgtgcgg 1260

ggtgaatccc ggtcccgggc tatcgccatc gcccagcatg tccaggtcga agtcgtccag 1320

ggcatcggcg tgggccatcg ccacatcctc gccatccagg tgcagctcat cgcccaggct 1380

cacgtcggtc ggcggggcgg tcgacaggcg gcgggtgtgt ccggccggca ggaagctcag 1440

gcgcggggcg gccaggcccg cctcctccgg ggcatcatca tccggcagat ccagcaggcc 1500

ctcgatggtg ctgccgtagt tgttcttggt gcgggcgcgg ctgtaggcgg ggcccgagcc 1560

cgactcgcat ttcagttgct tttccaatcc gcagataatc agctccaagc cgaacaggaa 1620

tgccggctcg gctccttgat gatcgaacag ctcgattgcc tgacgcagca gtgggggcat 1680

cgaatcggtt gttggggtct cgcgctcctc ttttgcgact tgatgctctt ggtcctccag 1740

cacgcagccc agggtaaagt gaccgacggc gctcagagcg tagagagcat tttccaggct 1800
gaagccttgc tggcacagga acgcgagctg gttctccagt gtctcgtatt gcttttcggt 1860
cgggcgcgtg ccgagatgga ctttggcacc gtctcggtgg gacagcagag cgcagcggaa 1920
cgacttggcg ttattgcgga ggaagtcctg ccaggactcg ccttccaacg ggcaaaaatg 1980
cgtgtggtgg cggtcgagca tctcgatggc cagggcatcc agcagcgccc gcttattctt 2040
cacgtgccag tagagggtgg gctgctccac gcccagcttc tgcgccaact tgcgggtcgt 2100
cagtccctca atgccaactt cgttcaacag ctccaacgcg gagttgatga ctttggactt 2160
atccaggcgg ctgaccatac caccgcgcag gcgcagcacc aggtgcaggg tgctctcctt 2220
ctggatgttg tagtcgctca gggtgcggcc atcctccagc tggcgtccgg cgaagatcag 2280
gcgctgctga tccggcggga tgccctcctt gtcctggatc ttggccttca cgttctcgat 2340
ggtatcgctc ggctccacct ccagggtgat ggtcttgccg gtcagggtct tgacgaagat 2400
ctgcatcgag ctagccgtca cacgttttgg cgccgcttca aggtccgtta ggtcctggaa 2460
atgggataga tattggtgtt attgttcatg tggcatataa aggacaagca acaaaaaacg 2520
aacataacat gagagatggt tctgaatcag aacttctgaa tattatcctc ccaaaagggt 2580
taaagttttt attaagcata ttacgtttta taccacttcc ttatgtaaaa ttttcttcgt 2640
agtttaatat catgtgaaat catatataat ttctatcgaa cgtttgttca aattgaatga 2700
tgtcattttt tgaataattg gttataattt tataacatct cccgacttcg acatgtggtt 2760
ggtactaatg attgcgaaat cgccctccga gaatgagaac aaccgaggtc caccgtctgg 2820
tcgagattaa aacacttgag gagtgctttg gtgactcgat caataggtac agggctcgtt 2880
gccaacaatc tggccagctg gacatccggg acctcgttcc cccctggggt atcaaaattt 2940
ttgtagtgta aatagtagta cactcttaaa aataatgaaa attactgcgg acgtaattca 3000
cattatgatt gaatgacact atcattgaca tttcccgaat cagacaccat cgtatttaaa 3060
atgtgacaca aattcacctc atttggctcg cttcttttat gtgcatccaa aagacgtaaa 3120
atcgcatgat tttttcggag tgtgtagtaa gattgtcaaa ttttaatttt aaataaccag 3180
agcccataaa gcaaagcaac actaggaaaa aacccacaaa ctcaacctgt ccaaaaaaaa 3240
atataacaat caaagttgag ggaatcgggg tcaaacgtca tgtaaaaata ttttttgtaa 3300
aaaccaaacc aggaataaat atgaatttaa tcggaaaaaa ttgcaaaatc gcataattta 3360
atcctccaac tgtactttat ccagcctgtt gcagaaatga tgtttaaagg ttctaatctg 3420
taattgttat tagccttcaa tactgatgta gtatttattt cttattgaaa cattgagagc 3480
tttattttcc aaagttgtca ttttctcatt cgtatatcgt aatatgtata ttcgtaaatg 3540
gcaagcacaa tgatactcag ggcagtcaag gatatttcaa ttacgaaaag atcctgaaac 3600

gaccgggaat cgaacccttc agcatggctt tgctttgtag ctgctgaatc taaccactag 3660
gctgatgaag atcccatttt agggttgcaa gttctcaaag agcaagaatg ccaaaatagt 3720
gtcaaaagaa gccctatttg acgatatacc ttttagtctc tacgttaatt tgctatgata 3780
atttatcatc aattaattgg caaagcctga tgcacgaaaa gatcttcttc taaaatttca 3840
gttgttcttt tcaacacatt atgtaatcat aaaatttata ataaaccttt tttttttgta 3900
actatccaca gttgatcagg cataattttc ttggaaagta aagtccatat ttaggttgat 3960
gttgaataaa aaaactttca attcactctt ctgtttcact tcagaactta cgtaatacga 4020
cattatgcat ggtgcacacg gaacaggata agacgttcac aagggatcaa catcacatcg 4080
gatcgtaatc actggatctg gaacacatat gacgccacaa gacagcacat tttacacgat 4140
caccagacgt gaacaaggaa ctggatccac aagacgtcac aggaagacgg cacatttcca 4200
acggcttcga tggaactttt ctcgagtctt tttccaccaa tcataaacac cgacctggat 4260
caatggggaa aaaccttcgt cgttatcctc ggtttccatg gtggcggtcc gtatcgaact 4320
gtaccagagc gagcgtcaaa atccgattgt gagtgattcc ttgggtttac cggggtttta 4380
tactcggaag tgccggccca tgcatcagtc catagaagga tcgattgaga tagttaattc 4440
cgatcaatca atcggcgatc gatcgcgtta cgatcgtgcg ttcgggtgag gtttccgacg 4500
catttgggag tgtgtgtttt gcgaagtgac aatgtaatgc atatctaaat ttggcattat 4560
ttgcaaagcg ccggtatgca gtgttcgcac atcgatacac ttttgtctga gacgtgatgc 4620
aaacctacag ggtgtttctt aaacggtgcg tggtacggat tgattactga atcagagata 4680
tattcattga attatcttac aaatataatt taacaaaaac cttttctaca aacaaaaaaa 4740
cacttcttgt cccagaagtc tgaagatgtt caactactga tcagctacct taaattatgt 4800
tctatttcga agtccaaacg atatttattc acgtatcgca tgatctctac tctttaagga 4860
aaggtatatt ggaaagtcag gacgtcaaat attgatacag agacctgacc actgactcct 4920
tgtcattttt cttcatctct tgacaggctc tttatgctgt cgcgacatgt cggtaagctg 4980
gcacgcagcg atgaaaattt acaaaaaaaa aagagagaaa aaagtagggc tacactcttt 5040
ggggtacgaa ttatcattat tgtgtaaaat aaatcttcca gctatttagt ggaatacctg 5100
taagtaaatg gaaacctaat tcaggactat accatttaat tcgactcgaa tttgcttcct 5160
ttgacagata aaaattctca agtagcattt tgaaccactt ttcgagaagt gtaatccggg 5220
gtaacattca tcagcggggt gacattgatc agaataaccc ttcttgtaaa aagtttgaat 5280
aaacattaat cgatggttgt tttttacatg tgtaaattgt tcaacgtttt gcaacaatca 5340
ttttcgtgat tttggatgac actatcgaat attaatgtct ttcacgggtt ttgcaagcaa 5400
tttgagcaag aaaatgcaaa tgttaccaaa aatggcatcg tcgaaaacga tttcgctgtg 5460
gaaactttca aaagtttgtt caattaaggc ccaagtaaaa atagcgcaaa tatcaaaacc 5520

aaaaaaaaaa cagttttttt ttctttttag cacggaagac cagagcaaat tgctttagct 5580

tgatagctaa agttcgtaac acaccggcac caaaaaacga acgattggat acgctagcga 5640

gtttcggttt ggtggtgcaa aatgtatgcg gccatctgaa agctaaacgc tggaaaatca 5700

tctttcgaat ccggttctcc taaatgaact tgtagagtaa ttaccaagca gcggtgaagt 5760

tcaattcggc tctatatcaa cgtcaacttc ccagtgcgcg ccccggccat cgagaaagag 5820

agagagaaga gaagagagag aacattcgag aaagagagag agaagagaag agagagaaca 5880

tactccctat cagtgataga gaagtcccta tcagtgatag agatgtccct atcagtgata 5940

gagagttccc tatcagtgat agagacgtcc ctatcagtga tagagaagtc cctatcagtg 6000

atagagagat ccctatcagt gatagagatt tccctatcag tgatagagag gtccctatca 6060

gtgatagaga cttccctatc agtgatagag aaatccctat cagtgataga gacatcccta 6120

tcagtgatag agaactccct atcagtgata gagacctccc tatcagtgat agagatcgat 6180

gcggccgcga gcgccggagt ataaatagag gcgcttcgtc tacggagcga caattcaatt 6240

caaacaagca aagtgaacac gtcgctaagc gaaagctaag caaataaaca agcgcagctg 6300

aacaagctaa acaatctgca ggtaccctgg cggtaagttg atcaaaggaa acgcaaagtt 6360

ttcaagaaaa aacaaaacta atttgattta taacaccttt agaaagcggg gctagccacc 6420

atgggcagcg cctacagccg cgcccgtacc aagaacaact atggcagcac catcgaggga 6480

ctgctggacc tgccggatga cgatgccccg gaggaagccg gcctggccgc cccccgcctg 6540

agcttcctgc ccgccggaca cacgcgccgc ctgagcaccg ccccgccgac cgatgtgagc 6600

ctgggcgacg agctgcacct ggatggagag gatgtggcaa tggcccacgc cgacgccctg 6660

gacgatttcg acctggatat gctgggcgat ggagatagcc cgggaccggg cttcacgccc 6720

cacgatagcg ccccgtacgg cgccctggac atggccgact tcgagttcga gcaaatgttc 6780

accgacgcgc tgggcatcga tgagtatggc gggtaggttt aaactcgcgt taagatacat 6840

tgatgagttt ggacaaacca caactagaat gcagtgaaaa aaatgcttta tttgtgaaat 6900

ttgtgatgct attgctttat ttgtaaccat tataagctgc aataaacaag ttaacaacaa 6960

caattgcatt cattttatgt ttcaggttca gggggaggtg tgggaggttt tttaaagcaa 7020

gtaaaacctc tacaaatgtg gtatggctga ttatgatcag ttatctagat ccggtggatc 7080

ttacgggtcc tccaccttcc gctttttctt gggtcgagat ctcaggaaca ggtggtggcg 7140

gccctcggtg cgctcgtact gctccacgat ggtgtagtcc tcgttgtggg aggtgatgtc 7200

cagcttggcg tccacgtagt agtagccggg cagctgcacg ggcttcttgg ccatgtagat 7260

ggacttgaac tccaccaggt agtggccgcc gtccttcagc ttcagggcct tgtgggtctc 7320

gcccttcagc acgccgtcgc gggggtacag gcgctcggtg gaggcctccc agcccatggt 7380

cttcttctgc atcacggggc cgtcggaggg gaagttcacg ccgatgaact tcaccttgta 7440
gatgaagcag ccgtcctgca gggaggagtc ctgggtcacg gtcgccacgc cgccgtcctc 7500
gaagttcatc acgcgctccc acttgaagcc ctcggggaag gacagcttct tgtagtcggg 7560
gatgtcggcg gggtgcttca cgtacacctt ggagccgtac tggaactggg gggacaggat 7620
gtcccaggcg aagggcaggg ggccgccctt ggtcaccttc agcttcacgg tgttgtggcc 7680
ctcgtagggg cggccctcgc cctcgccctc gatctcgaac tcgtggccgt tcacggtgcc 7740
ctccatgcgc accttgaagc gcatgaactc ggtgatgacg ttctcggagg aggccatggt 7800
ggcgaccggt ttgcgcttct tcttgggtgg ggtgggatct cccatggtgg cctgaatctc 7860
aacttgcacc tgaaggtagt gcagcaagga tgagcaaaag ggaagaaccc agaaaagaac 7920
gggaaaactt accccaatta gaattgcttg tcgccgccag tgtcaacttg caactgaaac 7980
aatatccaac atgaacgtca atttatactg ccctaatggc gaacacgata acaatatttc 8040
ttttattatg ccctctaaaa ccaacgcggt tatcgtttat ttattcaaat tagatataga 8100
acatccgccg acatacaatg ttaatgcaaa aacgcgtttg gtgagcggat acgaaaacag 8160
tcggccgata aacattaatc tgaggtcgat aacaccgtcc ttgaacggaa cacgaggagc 8220
gtacgtgatc agctgcattc gcgcgccgcg cctttatcga gatttatttg catacaacaa 8280
gtacactgcg ccgttgggat ttgtggtaac gcgcacacat gcagagctgc aagtgtggca 8340
cattttgtct gtgcgcaaaa cctttgaagc caaaagtacg aggtccgtta cgggcatgct 8400
actagcgcac acggacaatg gacccgacaa attctacgcc aaggatttaa tgataatgtc 8460
gggcaacgta tccgttcatt ttatcaataa cctacaaaaa tgtcgcgcgc atcacaaaga 8520
catcgatata tttaaacatt tatgtcccga actgcaaatc gataatagtg ttgtgcaacc 8580
tcgagcgtcc gtttgattta acgtatagct tgcaaatgaa ttatttaatt atcaatcatg 8640
ttttacgcgt agaattctac ccgtaaagcg agtttagtta tgagccatgt gcaaaacatg 8700
acatcagctt ttatttttat aacaaatgac atcatttctt gattgtgttt tacacgtaga 8760
attctactcg taaagcgagt tcagttttga aaaacaaatg acatcatctt tttgattgtg 8820
ctttacaagt agaattctac ccgtaaatca agttcggttt tgaaaaacaa atgagtcata 8880
ttgtatgata tcatattgca aaacaaatga ctcatcaatc gatcgtgcgt tacacgtaga 8940
attctactcg taaagcgagt ttatgagccg tgtgcaaaac atgacatcat ctcgatttga 9000
aaaacaaatg acatcatcca ctgatcgtgc attacaagta gaattctact cgtaaagcca 9060
gttcggttat gagccgtgta caaaacatga catcagatta tgactcatac ttgattgtgt 9120
tttacgcgta gaattctact cgtaaagcca gttcaatttt aaaaacaaat gacatcatcc 9180
aaattaataa atgacaagca atgggtacca tgcggcctgg cctcgcgctc gcgcgactga 9240
cggtcgtaag cacccgcgta cgtgtccacc ccggtcacaa cccettgtgt catgtcggcg 9300

accctacgcc cccaactgag agaactcaaa ggttacccca gttggggcac tactcccgaa 9360
aaccgcttct gacctgggaa aacgtgaagc cccggggcat ccgctgaggg ttgccgccgg 9420
ggcttcggtg tgtccgtcag tacttaatta acaccgaaat cgtaattcac ggcatcatta 9480
caaaatattt tgacgttttg gacctcgtcc ctaatgacac cataacggtg gccttgaagt 9540
atatttaacc ctagaaagat agtctgcgta aaattgacgc atgcattctt gaaatattgc 9600
tctctctttc taaatagcgc gaatccgtcg ctgtgcattt aggacatctc agtcgccgct 9660
tggagctccc gtgaggcgtg cttgtcaatg cggtaagtgt cactgatttt gaactataac 9720
gaccgcgtga gtcaaaatga cgcatgatta tcttttacgt gacttttaag atttaactca 9780
tacgataatt atattgttat ttcatgttct acttacgtga taacttatta tatatatatt 9840
ttcttgttat agatatcgtg actaatatat aataaaatgg gtagttcttt agacgatgag 9900
catatcctct ctgctcttct gcaaagcgat gacgagcttg ttggtgagga ttctgacagt 9960
gaaatatcag atcacgtaag tgaagatgac ctcgaggatc caagcttatc gatttcgaac 10020
cctcgaccgc cggagtataa atagaggcgc ttcgtctacg gagcgacaat tcaattcaaa 10080
caagcaaagt gaacacgtcg ctaagcgaaa gctaagcaaa taaacaagcg cagctgaaca 10140
agctaaacaa tcggggtacc gctagagtcg atcccacccc acccaagaag aagcgcaaac 10200
cggtaccatg gcctcctccg agaacgtcat caccgagttc atgcgcttca aggtgcgcat 10260
ggagggcacc gtgaacggcc acgagttcga gatcgagggc gagggcgagg gccgccccta 10320
cgagggccac aacaccgtga agctgaaggt gaccaagggc ggccccctgc ccttcgcctg 10380
ggacatcctg tccccccagt tccagtacgg ctccaaggtg tacgtgaagc accccgccga 10440
catccccgac tacaagaagc tgtccttccc cgagggcttc aagtgggagc gcgtgatgaa 10500
cttcgaggac ggcggcgtgg cgaccgtgac ccaggactcc tccctgcagg acggctgctt 10560
catctacaag gtgaagttca tcggcgtgaa cttcccctcc gacggccccg tgatgcagaa 10620
gaagaccatg ggctgggagg cctccaccga gcgcctgtac ccccgcgacg gcgtgctgaa 10680
gggcgagacc cacaaggccc tgaagctgaa ggacggcggc cactacctgg tggagttcaa 10740
gtccatctac atggccaaga agcccgtgca gctgcccggc tactactacg tggacgccaa 10800
gctggacatc acctcccaca acgaggacta caccatcgtg gagcagtacg agcgcaccga 10860
gggccgccac cacctgttcc tgtgatgatc ataatcagcc ataccacatt tgtagaggtt 10920
ttacttgctt taaaaaacct cccacacctc cccctgaacc tgaaacataa aatgaatgca 10980
attgttgttg ttaacttgtt tattgcagct tataatggtt acaaataaag caatagcatc 11040
acaaatttca caaataaagc atttttttca ctgcattcta gttgtggttt gtccaaactc 11100
atcaatgtat cttaacgcga gttaattacg gccgctcatt taaatctggc cggccgcaac 11160

cattgtggga accgtgcgat caaacaaacg cgagataccg gaagtactga aaaacagtcg 11220

ctccaggcca gtgggaacat cgatgttttg ttttgacgga ccccttactc tcgtctcata 11280

taaaccgaag ccagctaaga tggtatactt attatcatct tgtgatgagg atgcttctat 11340

caacgaaagt accggtaaac cgcaaatggt tatgtattat aatcaaacta aaggcggagt 11400

ggacacgcta gaccaaatgt gttctgtgat gacctgcagt aggaagacga ataggtggcc 11460

tatggcatta ttgtacggaa tgataaacat tgcctgcata aattctttta ttatatacag 11520

ccataatgtc agtagcaagg gagaaaaggt ccaaagtcgc aaaaaattta tgagaaacct 11580

ttacatgagc ctgacgtcat cgtttatgcg taagcgttta gaagctccta ctttgaagag 11640

atatttgcgc gataatatct ctaatatttt gccaaatgaa gtgcctggta catcagatga 11700

cagtactgaa gagccagtaa tgaaaaaacg tacttactgt acttactgcc cctctaaaat 11760

aaggcgaaag gcaaatgcat cgtgcaaaaa atgcaaaaaa gttatttgtc gagagcataa 11820

tattgatatg tgccaaagtt gtttctgact gactaataag tataatttgt ttctattatg 11880

tataagttaa gctaattact tattttataa tacaacatga ctgtttttaa agtacaaaat 11940

aagtttattt ttgtaaaaga gagaatgttt aaaagttttg ttactttata gaagaaattt 12000

tgagtttttg ttttttttta ataaataaat aaacataaat aaattgtttg ttgaatttat 12060

tattagtatg taagtgtaaa tataataaaa cttaatatct attcaaatta ataaataaac 12120

ctcgatatac agaccgataa aacacatgcg tcaattttac gcatgattat ctttaacgta 12180

cgtcacaata tgattatctt tctagggtta aataatagtt tctaattttt ttattattca 12240

gcctgctgtc gtgaataccg tatatctcaa cgctgtctgt gagattgtcg tattctagcc 12300

tttttagttt ttcgctcatc gacttgatat tgtccgacac attttcgtcg atttgcgttt 12360

tgatcaaaga cttgagcaga gacacgttaa tcaactgttc aaattgatcc atattaacga 12420

tatcaacccg atgcgtatat ggtgcgtaaa atatattttt taaccctctt atactttgca 12480

ctctgcgtta atacgcgttc gtgtacagac gtaatcatgt tttctttttt ggataaaact 12540

cctactgagt ttgacctcat attagaccct cacaagttgc aaaacgtggc atttttttacc 12600

aatgaagaat ttaaagttat tttaaaaaat ttcatcacag atttaaagaa gaaccaaaaa 12660

ttaaattatt tcaacagttt aatcgaccag ttaatcaacg tgtacacaga cgcgtcggca 12720

aaaaacacgc agcccgacgt gttggctaaa attattaaat caacttgtgt tatagtcacg 12780

gatttgccgt ccaacgtgtt cctcaaaaag ttgaagacca acaagtttac ggacactatt 12840

aattatttga ttttgcccca cttcattttg tgggatcaca attttgttat attttaaaca 12900

aagcttggca ctggccgtcg ttttacaacg tcgtgactgg gaaaaccctg gcgttaccca 12960

acttaatcgc cttgcagcac atcccccttt cgccagctgg cgtaatagcg aagaggcccg 13020

caccgatcgc ccttcccaac agttgcgcag cctgaatggc gaatggcgcc tgatgcggta 13080

ttttctcctt acgcatctgt gcggtatttc acaccgcata tggtgcactc tcagtacaat 13140

ctgctctgat gccgcatagt taagccagcc ccgacacccg ccaacacccg ctgacgcgcc 13200

ctgacgggct tgtctgctcc cggcatccgc ttacagacaa gctgtgaccg tctccgggag 13260

ctgcatgtgt cagaggtttt caccgtcatc accgaaacgc gcgagacgaa agggcctcgt 13320

gatacgccta tttttatagg ttaatgtcat gataataatg gtttcttaga cgtcaggtgg 13380

cacttttcgg ggaaatgtgc gcggaacccc tatttgttta tttttctaaa tacattcaaa 13440

tatgtatccg ctcatgagac aataaccctg ataaatgctt caataatatt gaaaaaggaa 13500

gagtatgagt attcaacatt tccgtgtcgc ccttattccc ttttttgcgg cattttgcct 13560

tcctgttttt gctcacccag aaacgctggt gaaagtaaaa gatgctgaag atcagttggg 13620

tgcacgagtg ggttacatcg aactggatct caacagcggt aagatccttg agagttttcg 13680

ccccgaagaa cgttttccaa tgatgagcac ttttaaagtt ctgctatgtg gcgcggtatt 13740

atcccgtatt gacgccgggc aagagcaact cggtcgccgc atacactatt ctcagaatga 13800

cttggttgag tactcaccag tcacagaaaa gcatcttacg gatggcatga cagtaagaga 13860

attatgcagt gctgccataa ccatgagtga taacactgcg gccaacttac ttctgacaac 13920

gatcggagga ccgaaggagc taaccgcttt tttgcacaac atgggggatc atgtaactcg 13980

ccttgatcgt tgggaaccgg agctgaatga agccatacca aacgacgagc gtgacaccac 14040

gatgcctgta gcaatggcaa caacgttgcg caaactatta actggcgaac tacttactct 14100

agcttcccgg caacaattaa tagactggat ggaggcggat aaagttgcag gaccacttct 14160

gcgctcggcc cttccggctg gctggtttat tgctgataaa tctggagccg gtgagcgtgg 14220

gtctcgcggt atcattgcag cactggggcc agatggtaag ccctcccgta tcgtagttat 14280

ctacacgacg gggagtcagg caactatgga tgaacgaaat agacagatcg ctgagatagg 14340

tgcctcactg attaagcatt ggtaactgtc agaccaagtt tactcatata tactttagat 14400

tgatttaaaa cttcattttt aatttaaaag gatctaggtg aagatccttt ttgataatct 14460

catgaccaaa atcccttaac gtgagttttc gttccactga gcgtcagacc ccgtagaaaa 14520

gatcaaagga tcttcttgag atcctttttt tctgcgcgta atctgctgct tgcaaacaaa 14580

aaaaccaccg ctaccagcgg tggtttgttt gccggatcaa gagctaccaa ctctttttcc 14640

gaaggtaact ggcttcagca gagcgcagat accaaatact gtccttctag tgtagccgta 14700

gttaggccac cacttcaaga actctgtagc accgcctaca tacctcgctc tgctaatcct 14760

gttaccagtg gctgctgcca gtggcgataa gtcgtgtctt accgggttgg actcaagacg 14820

atagttaccg gataaggcgc agcggtcggg ctgaacgggg ggttcgtgca cacagcccag 14880

cttggagcga acgacctaca ccgaactgag atacctacag cgtgagcatt gagaaagcgc 14940

cacgcttccc gaagggagaa aggcggacag gtatccggta agcggcaggg tcggaacagg 15000
agagcgcacg agggagcttc caggggggaaa cgcctggtat ctttatagtc ctgtcgggtt 15060
tcgccacctc tgacttgagc gtcgattttt gtgatgctcg tcaggggggc ggagcctatg 15120
gaaaaacgcc agcaacgcgg cctttttacg gttcctggcc ttttgctggc cttttgctca 15180
catgttcttt cctgcgttat cccctgattc tgtggataac cgtattaccg cctttgagtg 15240
agctgatacc gctcgccgca gccgaacgac cgagcgcagc gagtcagtga gcgaggaagc 15300
ggaagagcgc ccaatacgca aaccgcctct ccccgcgcgt tggccgattc attaatgcag 15360
ctggcacgac aggtttcccg actggaaagc gggcagtgag cgcaacgcaa ttaatgtgag 15420
ttagctcact cattaggcac cccaggcttt acactttatg cttccggctc gtatgttgtg 15480
tggaattgtg agcggataac aatttcacac aggaaacagc tatgaccatg attacgaatt 15540
tcgacctgca ggcatgcaag cttgcatgcc tgcaggtcga cgctcgcgcg acttggtttg 15600
ccattcttta gcgcgcgtcg cgtcacacag cttggccaca atgtggtttt tgtcaaacga 15660
agattctatg acgtgtttaa agtttaggtc gagtaaagcg caaatctttt ttaaccctag 15720
aaagatagtc tgcgtaaaat tgacgcatgc attcttgaaa tattgctctc tctttctaaa 15780
tagcgcgaat ccgtcgctgt gcatttagga catctcagtc gccgcttgga gctcccgtga 15840
ggcgtgcttg tcaatgcggt aagtgtcact gattttgaac tataacgacc gcgtgagtca 15900
aaatgacgca tgattatctt ttacgtgact tttaagattt aactcatacg ataattatat 15960
tgttatttca tgttctactt acgtgataac ttattatata tatattttct tgttatagat 16020
atcgtgacta atatataata aaatgggtag ttctttagac gatgagcata tcctctctgc 16080
tcttctgcaa agcgatgacg agcttgttgg tgaggattct gacagtgaaa tatcagatca 16140
cgtaagtgaa gatgacgtcc agagcgatac agaagaagcg tttatagatg aggtacatga 16200
agtgcagcca acgtcaagcg gtagtgaaat attagacgaa caaaatgtta ttgaacaacc 16260
aggttcttca ttggcttcta acagaatctt gaccttgcca cagaggacta ttagaggtaa 16320
gaataaacat tgttggtcaa cttcaaagtc cacgaggcgt agccgagtct ctgcactgaa 16380
cattgtcaga tcggcccgct cgcccgggga actagttcaa ttagagacta attcaattag 16440
agctaattca attaggatcc aagcttatcg atttcgaacc ctcgaccgcc ggagtataaa 16500
tagaggcgct tcgtctacgg agcgacaatt caattcaaac aagcaaagtg aacacgtcgc 16560
taagcgaaag ctaagcaaat aaacaagcgc agctgaacaa gctaaacaat cggggtaccg 16620
ctagagtcga tcccacccca cccaagaaga agcgcaaacc ggtcgccacc atggccctgt 16680
ccaacaagtt catcggcgac gacatgaaga tgacctacca catggacggc tgcgtgaacg 16740
gccactactt caccgtgaag ggcgagggca gcggcaagcc ctacgagggc acccagacct 16800
ccaccttcaa ggtgaccatg gccaacggcg gcccccctggc cttctccttc gacatcctgt 16860

ccaccgtgtt catgtacggc aaccgctgct tcaccgccta ccccaccagc atgcccgact 16920

acttcaagca ggccttcccc gacggcatgt cctacgagag aaccttcacc tacgaggacg 16980

gcggcgtggc caccgccagc tgggagatca gcctgaaggg caactgcttc gagcacaagt 17040

ccaccttcca cggcgtgaac ttccccgccg acggccccgt gatggccaag aagaccaccg 17100

gctgggaccc ctccttcgag aagatgaccg tgtgcgacgg catcttgaag ggcgacgtga 17160

ccgccttcct gatgctgcag ggcggcggca actacagatg ccagttccac acctcctaca 17220

agaccaagaa gcccgtgacc atgcccccca accacgtggt ggagcaccgc atcgccagaa 17280

ccgacctgga caagggcggc aacagcgtgc agctgaccga gcacgccgtg gcccacatca 17340

cctccgtggt gcccttctcc ggactcagat cataatcagc cataccacat ttgtagaggt 17400

tttacttgct ttaaaaaacc tcccacacct ccccctgaac ctgaaacata aaatgaatgc 17460

aattgttgtt gttaacttgt ttattgcagc ttataatggt tacaaataaa gcaatagcat 17520

cacaaatttc acaaataaag catttttttc actgcattct agttgtggtt tgtccaaact 17580

catcaatgta tcttaccgcg gagtggacac gctagaccaa atgtgttctg tgatgacctg 17640

cagtaggaag acgaataggt ggcctatggc attattgtac ggaatgataa acattgcctg 17700

cataaattct tttattatat acagccataa tgtcagtagc aagggagaaa aggtccaaag 17760

tcgcaaaaaa tttatgagaa acctttacat gagcctgacg tcatcgttta tgcgtaagcg 17820

tttagaagct cctactttga agagatattt gcgcgataat atctctaata ttttgccaaa 17880

tgaagtgcct ggtacatcag atgacagtac tgaagagcca gtaatgaaaa aacgtactta 17940

ctgtacttac tgcccctcta aaataaggcg aaaggcaaat gcatcgtgca aaaaatgcaa 18000

aaaagttatt tgtcgagagc ataatattga tatgtgccaa agttgtttct gactgactaa 18060

taagtataat ttgtttctat tatgtataag ttaagctaat tacttatttt ataatacaac 18120

atgactgttt ttaaagtaca aaataagttt atttttgtaa aagagagaat gtttaaaagt 18180

tttgttactt tatagaagaa attttgagtt tttgtttttt tttaataaat aaataaacat 18240

aaataaattg tttgttgaat ttattattag tatgtaagtg taaatataat aaaacttaat 18300

atctattcaa attaataaat aaacctcgat atacagaccg ataaaacaca tgcgtcaatt 18360

ttacgcatga ttatctttaa cgtacgtcac aatatgatta tctttctagg g 18411

<210> 157
<211> 18073
<212> DNA
<213> artificial

<220>
<223> LA3646 Plasmid sequence

<400> 157

ctaggtaaga tacattgatg agtttggaca aaccacaact agaatgcagt gaaaaaaatg 60
ctttatttgt gaaatttgtg atgctattgc tttatttgta accattataa gctgcaataa 120
acaagttaac aacaacaatt gcattcattt tatgtttcag gttcaggggg aggtgtggga 180
ggttttttaa agcaagtaaa acctctacaa atgtggtatg gctgattatg atcagttatc 240
tagatccggt ggatcttacg ggtcctccac cttccgcttt ttcttgggtc gagatctcag 300
gaacaggtgg tggcggccct cggtgcgctc gtactgctcc acgatggtgt agtcctcgtt 360
gtgggaggtg atgtccagct tggcgtccac gtagtagtag ccgggcagct gcacgggctt 420
cttggccatg tagatggact tgaactccac caggtagtgg ccgccgtcct tcagcttcag 480
ggccttgtgg gtctcgccct tcagcacgcc gtcgcggggg tacaggcgct cggtggaggc 540
ctcccagccc atggtcttct tctgcatcac ggggccgtcg gaggggaagt tcacgccgat 600
gaacttcacc ttgtagatga agcagccgtc ctgcagggag gagtcctggg tcacggtcgc 660
cacgccgccg tcctcgaagt tcatcacgcg ctcccacttg aagccctcgg ggaaggacag 720
cttcttgtag tcggggatgt cggcggggtg cttcacgtac accttggagc cgtactggaa 780
ctggggggac aggatgtccc aggcgaaggg cagggggccg cccttggtca ccttcagctt 840
cacggtgttg tggccctcgt aggggcggcc ctcgccctcg ccctcgatct cgaactcgtg 900
gccgttcacg gtgccctcca tgcgcacctt gaagcgcatg aactcggtga tgacgttctc 960
ggaggaggcc atggtggcga ccggtttgcg cttcttcttg ggtggggtgg gatctcccat 1020
ggtggcctga atctcaactt gcacctgaag gtagtgcagc aaggatgagc aaaagggaag 1080
aacccagaaa agaacgggaa aacttacccc aattagaatt gcttgtcgcc gccagtgtca 1140
acttgcaact gaaacaatat ccaacatgaa cgtcaattta tactgcccta atggcgaaca 1200
cgataacaat atttctttta ttatgccctc taaaaccaac gcggttatcg tttatttatt 1260
caaattagat atagaacatc cgccgacata caatgttaat gcaaaaacgc gtttggtgag 1320
cggatacgaa aacagtcggc cgataaacat taatctgagg tcggtaacac cgtccttgaa 1380
cggaacacga ggagcgtacg tgatcagctg cattcgcgcg ccgcgccttt atcgagattt 1440
atttgcatac aacaagtaca ctgcgccgtt gggatttgtg gtaacgcgca cacatgcaga 1500
gctgcaagtg tggcacattt tgtctgtgcg caaaaccttt gaagccaaaa gtacgaggtc 1560
cgttacgggc atgctagcgc acacggacaa tggacccgac aaattctacg ccaaggattt 1620
aatgataatg tcgggcaacg tatccgttca ttttatcaat aacctacaaa aatgtcgcgc 1680
gcatcacaaa gacatcgata tatttaaaca tttatgtccc gaactgcaaa tcgataatag 1740
tgttgtgcaa cctcgagcgt ccgtttgatt taacgtatag cttgcaaatg aattatttaa 1800
ttatcaatca tgttttacgc gtagaattct acccgtaaag cgagtttagt tatgagccat 1860
gtgcaaaaca tgacatcagc ttttattttt ataacaaatg acatcatttc ttgattgtgt 1920

tttacacgta gaattctact cgtaaagcga gttcagtttt gaaaaacaaa tgacatcatc 1980
tttttgattg tgctttacaa gtagaattct acccgtaaat caagttcggt tttgaaaaac 2040
aaatgagtca tattgtatga tatcatattg caaaacaaat gactcatcaa tcgatcgtgc 2100
gttacacgta gaattctact cgtaaagcga gtttatgagc cgtgtgcaaa acatgacatc 2160
atctcgattt gaaaaacaaa tgacatcatc cactgatcgt gcattacaag tagaattcta 2220
ctcgtaaagc cagttcggtt atgagccgtg tacaaaacat gacatcagat tatgactcat 2280
acttgattgt gttttacgcg tagaattcta ctcgtaaagc cagttcaatt ttaaaaacaa 2340
atgacgcggc cgcattaaca ccgaaatcgt aattcacggc atcattacaa aatattttga 2400
cgttttggac ctcgtcccta atgacaccat aacggtggcc ttgaagtata tttaacccta 2460
gaaagatagt ctgcgtaaaa ttgacgcatg cattcttgaa atattgctct ctctttctaa 2520
atagcgcgaa tccgtcgctg tgcatttagg acatctcagt cgccgcttgg agctcccgtg 2580
aggcgtgctt gtcaatgcgg taagtgtcac tgattttgaa ctataacgac cgcgtgagtc 2640
aaaatgacgc atgattatct tttacgtgac ttttaagatt taactcatac gataattata 2700
ttgttatttc atgttctact tacgtgataa cttattatat atatattttc ttgttataga 2760
tatcgtgact aatatataat aaaatgggta gttctttaga cgatgagcat atcctctctg 2820
ctcttctgca aagcgatgac gagcttgttg gtgaggattc tgacagtgaa atatcagatc 2880
acgtaagtga agatgacctc gaggatccaa gcttatcgat ttcgaaccct cgaccgccgg 2940
agtataaata gaggcgcttc gtctacggag cgacaattca attcaaacaa gcaaagtgaa 3000
cacgtcgcta agcgaaagct aagcaaataa acaagcgcag ctgaacaagc taaacaatcg 3060
gggtaccgct agagtcgatc ccaccccacc caagaagaag cgcaaaccgg taccatggcc 3120
tcctccgaga acgtcatcac cgagttcatg cgcttcaagg tgcgcatgga gggcaccgtg 3180
aacggccacg agttcgagat cgagggcgag ggcgagggcc gcccctacga gggccacaac 3240
accgtgaagc tgaaggtgac caagggcggc cccctgccct tcgcctggga catcctgtcc 3300
ccccagttcc agtacggctc caaggtgtac gtgaagcacc ccgccgacat ccccgactac 3360
aagaagctgt ccttccccga gggcttcaag tgggagcgcg tgatgaactt cgaggacggc 3420
ggcgtggcga ccgtgaccca ggactcctcc ctgcaggacg gctgcttcat ctacaaggtg 3480
aagttcatcg gcgtgaactt cccctccgac ggccccgtga tgcagaagaa gaccatgggc 3540
tgggaggcct ccaccgagcg cctgtacccc cgcgacggcg tgctgaaggg cgagacccac 3600
aaggccctga agctgaagga cggcggccac tacctggtgg agttcaagtc catctacatg 3660
gccaagaagc ccgtgcagct gcccggctac tactacgtgg acgccaagct ggacatcacc 3720
tcccacaacg aggactacac catcgtggag cagtacgagc gcaccgaggg ccgccaccac 3780

ctgttcctgt gatgatcata atcagccata ccacatttgt agaggtttta cttgctttaa 3840
aaaacctccc acacctcccc ctgaacctga aacataaaat gaatgcaatt gttgttgtta 3900
acttgtttat tgcagcttat aatggttaca aataaagcaa tagcatcaca aatttcacaa 3960
ataaagcatt tttttcactg cattctagtt gtggtttgtc caaactcatc aatgtatctt 4020
aacgcgagtt aattacggcc gctcatttaa atctggccgg ccgcaaccat tgtgggaacc 4080
gtgcgatcaa acaaacgcga gataccggaa gtactgaaaa acagtcgctc caggccagtg 4140
ggaacatcga tgttttgttt tgacggaccc cttactctcg tctcatataa accgaagcca 4200
gctaagatgg tatacttatt atcatcttgt gatgaggatg cttctatcaa cgaaagtacc 4260
ggtaaaccgc aaatggttat gtattataat caaactaaag gcggagtgga cacgctagac 4320
caaatgtgtt ctgtgatgac ctgcagtagg aagacgaata ggtggcctat ggcattattg 4380
tacggaatga taaacattgc ctgcataaat tcttttatta tatacagcca taatgtcagt 4440
agcaagggag aaaaggtcca aagtcgcaaa aaatttatga gaaaccttta catgagcctg 4500
acgtcatcgt ttatgcgtaa gcgtttagaa gctcctactt tgaagagata tttgcgcgat 4560
aatatctcta atattttgcc aaatgaagtg cctggtacat cagatgacag tactgaagag 4620
ccagtaatga aaaaacgtac ttactgtact tactgcccct ctaaaataag gcgaaaggca 4680
aatgcatcgt gcaaaaaatg caaaaaagtt atttgtcgag agcataatat tgatatgtgc 4740
caaagttgtt tctgactgac taataagtat aatttgtttc tattatgtat aagttaagct 4800
aattacttat tttataatac aacatgactg tttttaaagt acaaaataag tttatttttg 4860
taaaagagag aatgtttaaa agttttgtta ctttatagaa gaaattttga gtttttgttt 4920
ttttttaata aataaataaa cataaataaa ttgtttgttg aatttattat tagtatgtaa 4980
gtgtaaatat aataaaactt aatatctatt caaattaata aataaacctc gatatacaga 5040
ccgataaaac acatgcgtca attttacgca tgattatctt taacgtacgt cacaatatga 5100
ttatctttct agggttaaat aatagtttct aatttttta ttattcagcc tgctgtcgtg 5160
aataccgtat atctcaacgc tgtctgtgag attgtcgtat tctagccttt ttagtttttc 5220
gctcatcgac ttgatattgt ccgacacatt ttcgtcgatt tgcgttttga tcaaagactt 5280
gagcagagac acgttaatca actgttcaaa ttgatccata ttaacgatat caacccgatg 5340
cgtatatggt gcgtaaaata tattttttaa ccctcttata ctttgcactc tgcgttaata 5400
cgcgttcgtg tacagacgta atcatgtttt cttttttgga taaaactcct actgagtttg 5460
acctcatatt agaccctcac aagttgcaaa acgtggcatt ttttaccaat gaagaattta 5520
aagttatttt aaaaaatttc atcacagatt taaagaagaa ccaaaaatta aattatttca 5580
acagtttaat cgaccagtta atcaacgtgt acacagacgc gtcggcaaaa aacacgcagc 5640
ccgacgtgtt ggctaaaatt attaaatcaa cttgtgttat agtcacggat ttgccgtcca 5700

acgtgttcct caaaaagttg aagaccaaca agtttacgga cactattaat tatttgattt 5760

tgccccactt cattttgtgg gatcacaatt ttgttatatt ttaaacaaag cttggcactg 5820

gccgtcgttt tacaacgtcg tgactgggaa aaccctggcg ttacccaact taatcgcctt 5880

gcagcacatc ccccttttcgc cagctggcgt aatagcgaag aggcccgcac cgatcgccct 5940

tcccaacagt tgcgcagcct gaatggcgaa tggcgcctga tgcggtattt tctccttacg 6000

catctgtgcg gtatttcaca ccgcatatgg tgcactctca gtacaatctg ctctgatgcc 6060

gcatagttaa gccagccccg acacccgcca acacccgctg acgcgccctg acgggcttgt 6120

ctgctcccgg catccgctta cagacaagct gtgaccgtct ccgggagctg catgtgtcag 6180

aggttttcac cgtcatcacc gaaacgcgcg agacgaaagg gcctcgtgat acgcctattt 6240

ttataggtta atgtcatgat aataatggtt tcttagacgt caggtggcac ttttcgggga 6300

aatgtgcgcg gaacccctat ttgtttattt ttctaaatac attcaaatat gtatccgctc 6360

atgagacaat aaccctgata aatgcttcaa taatattgaa aaaggaagag tatgagtatt 6420

caacatttcc gtgtcgccct tattcccttt tttgcggcat tttgccttcc tgtttttgct 6480

cacccagaaa cgctggtgaa agtaaaagat gctgaagatc agttgggtgc acgagtgggt 6540

tacatcgaac tggatctcaa cagcggtaag atccttgaga gttttcgccc cgaagaacgt 6600

tttccaatga tgagcacttt taaagttctg ctatgtggcg cggtattatc ccgtattgac 6660

gccgggcaag agcaactcgg tcgccgcata cactattctc agaatgactt ggttgagtac 6720

tcaccagtca cagaaaagca tcttacggat ggcatgacag taagagaatt atgcagtgct 6780

gccataacca tgagtgataa cactgcggcc aacttacttc tgacaacgat cggaggaccg 6840

aaggagctaa ccgctttttt gcacaacatg ggggatcatg taactcgcct tgatcgttgg 6900

gaaccggagc tgaatgaagc cataccaaac gacgagcgtg acaccacgat gcctgtagca 6960

atggcaacaa cgttgcgcaa actattaact ggcgaactac ttactctagc ttcccggcaa 7020

caattaatag actggatgga ggcggataaa gttgcaggac cacttctgcg ctcggccctt 7080

ccggctggct ggtttattgc tgataaatct ggagccggtg agcgtgggtc tcgcggtatc 7140

attgcagcac tggggccaga tggtaagccc tcccgtatcg tagttatcta cacgacgggg 7200

agtcaggcaa ctatggatga acgaaataga cagatcgctg agataggtgc ctcactgatt 7260

aagcattggt aactgtcaga ccaagtttac tcatatatac tttagattga tttaaaactt 7320

catttttaat ttaaaaggat ctaggtgaag atcctttttg ataatctcat gaccaaaatc 7380

ccttaacgtg agttttcgtt ccactgagcg tcagaccccg tagaaaagat caaaggatct 7440

tcttgagatc cttttttttct gcgcgtaatc tgctgcttgc aaacaaaaaa accaccgcta 7500

ccagcggtgg tttgtttgcc ggatcaagag ctaccaactc tttttccgaa ggtaactggc 7560

ttcagcagag cgcagatacc aaatactgtc cttctagtgt agccgtagtt aggccaccac 7620
ttcaagaact ctgtagcacc gcctacatac ctcgctctgc taatcctgtt accagtggct 7680
gctgccagtg gcgataagtc gtgtcttacc gggttggact caagacgata gttaccggat 7740
aaggcgcagc ggtcgggctg aacggggggt tcgtgcacac agcccagctt ggagcgaacg 7800
acctacaccg aactgagata cctacagcgt gagcattgag aaagcgccac gcttcccgaa 7860
gggagaaagg cggacaggta tccggtaagc ggcagggtcg gaacaggaga gcgcacgagg 7920
gagcttccag ggggaaacgc ctggtatctt tatagtcctg tcgggtttcg ccacctctga 7980
cttgagcgtc gatttttgtg atgctcgtca ggggggcgga gcctatggaa aaacgccagc 8040
aacgcggcct ttttacggtt cctggccttt tgctggcctt ttgctcacat gttctttcct 8100
gcgttatccc ctgattctgt ggataaccgt attaccgcct ttgagtgagc tgataccgct 8160
cgccgcagcc gaacgaccga gcgcagcgag tcagtgagcg aggaagcgga agagcgccca 8220
atacgcaaac cgcctctccc cgcgcgttgg ccgattcatt aatgcagctg gcacgacagg 8280
tttcccgact ggaaagcggg cagtgagcgc aacgcaatta atgtgagtta gctcactcat 8340
taggcacccc aggctttaca ctttatgctt ccggctcgta tgttgtgtgg aattgtgagc 8400
ggataacaat ttcacacagg aaacagctat gaccatgatt acgaatttcg acctgcaggc 8460
atgcaagctt gcatgcctgc aggtcgacgc tcgcgcgact tggtttgcca ttctttagcg 8520
cgcgtcgcgt cacacagctt ggccacaatg tggtttttgt caaacgaaga ttctatgacg 8580
tgtttaaagt ttaggtcgag taaagcgcaa atcttttttta accctagaaa gatagtctgc 8640
gtaaaattga cgcatgcatt cttgaaatat tgctctctct ttctaaatag cgcgaatccg 8700
tcgctgtgca tttaggacat ctcagtcgcc gcttggagct cccgtgaggc gtgcttgtca 8760
atgcggtaag tgtcactgat tttgaactat aacgaccgcg tgagtcaaaa tgacgcatga 8820
ttatctttta cgtgactttt aagatttaac tcatacgata attatattgt tatttcatgt 8880
tctacttacg tgataactta ttatatatat attttcttgt tatagatatc gtgactaata 8940
tataataaaa tgggtagttc tttagacgat gagcatatcc tctctgctct tctgcaaagc 9000
gatgacgagc ttgttggtga ggattctgac agtgaaatat cagatcacgt aagtgaagat 9060
gacgtccaga gcgatacaga agaagcgttt atagatgagg tacatgaagt gcagccaacg 9120
tcaagcggta gtgaaatatt agacgaacaa aatgttattg aacaaccagg ttcttcattg 9180
gcttctaaca gaatcttgac cttgccacag aggactatta gaggtaagaa taaacattgt 9240
tggtcaactt caaagtccac gaggcgtagc cgagtctctg cactgaacat tgtcagatcg 9300
gcccgctcgc ccggggaact agttcaatta gagactaatt caattagagc taattcaatt 9360
aggatccaag cttatcgatt tcgaaccctc gaccgccgga gtataaatag aggcgcttcg 9420
tctacggagc gacaattcaa ttcaaacaag caaagtgaac acgtcgctaa gcgaaagcta 9480

agcaaataaa caagcgcagc tgaacaagct aaacaatcgg ggtaccgcta gagtcgatcc 9540
caccccaccc aagaagaagc gcaaaccggt cgccaccatg gccctgtcca acaagttcat 9600
cggcgacgac atgaagatga cctaccacat ggacggctgc gtgaacggcc actacttcac 9660
cgtgaagggc gagggcagcg gcaagcccta cgagggcacc cagacctcca ccttcaaggt 9720
gaccatggcc aacggcggcc ccctggcctt ctccttcgac atcctgtcca ccgtgttcat 9780
gtacggcaac cgctgcttca ccgcctaccc caccagcatg cccgactact tcaagcaggc 9840
cttccccgac ggcatgtcct acgagagaac cttcacctac gaggacggcg gcgtggccac 9900
cgccagctgg gagatcagcc tgaagggcaa ctgcttcgag cacaagtcca ccttccacgg 9960
cgtgaacttc cccgccgacg gccccgtgat ggccaagaag accaccggct gggacccctc 10020
cttcgagaag atgaccgtgt gcgacggcat cttgaagggc gacgtgaccg ccttcctgat 10080
gctgcagggc ggcggcaact acagatgcca gttccacacc tcctacaaga ccaagaagcc 10140
cgtgaccatg ccccccaacc acgtggtgga gcaccgcatc gccagaaccg acctggacaa 10200
gggcggcaac agcgtgcagc tgaccgagca cgccgtggcc cacatcacct ccgtggtgcc 10260
cttctccgga ctcagatcat aatcagccat accacatttg tagaggtttt acttgcttta 10320
aaaaacctcc cacacctccc cctgaacctg aaacataaaa tgaatgcaat tgttgttgtt 10380
aacttgttta ttgcagctta taatggttac aaataaagca atagcatcac aaatttcaca 10440
aataaagcat ttttttcact gcattctagt tgtggtttgt ccaaactcat caatgtatct 10500
taccgcggag tggacacgct agaccaaatg tgttctgtga tgacctgcag taggaagacg 10560
aataggtggc ctatggcatt attgtacgga atgataaaca ttgcctgcat aaattctttt 10620
attatataca gccataatgt cagtagcaag ggagaaaagg tccaaagtcg caaaaaattt 10680
atgagaaacc tttacatgag cctgacgtca tcgtttatgc gtaagcgttt agaagctcct 10740
actttgaaga gatatttgcg cgataatatc tctaatattt tgccaaatga agtgcctggt 10800
acatcagatg acagtactga agagccagta atgaaaaaac gtacttactg tacttactgc 10860
ccctctaaaa taaggcgaaa ggcaaatgca tcgtgcaaaa aatgcaaaaa agttatttgt 10920
cgagagcata atattgatat gtgccaaagt tgtttctgac tgactaataa gtataatttg 10980
tttctattat gtataagtta agctaattac ttattttata atacaacatg actgttttta 11040
aagtacaaaa taagtttatt tttgtaaaag agagaatgtt taaaagtttt gttactttat 11100
agaagaaatt ttgagttttt gttttttttt aataaataaa taaacataaa taaattgttt 11160
gttgaattta ttattagtat gtaagtgtaa atataataaa acttaatatc tattcaaatt 11220
aataaataaa cctcgatata cagaccgata aaacacatgc gtcaatttta cgcatgatta 11280
tctttaacgt acgtcacaat atgattatct ttctagggtt aaaatgaatg taagcacttt 11340

attaacgaaa tctttgggaa tatttcgctc atcagcattt tatttgagca ggagtccgag 11400

atgcccgggc ggcgcgccga attcttaatt aacgccctag ccgcgatcgc atccgccgcg 11460

gtggcggccc taagatacat tgatgagttt ggacaaacca caactagaat gcagtgaaaa 11520

aaatgcttta tttgtgaaat ttgtgatgct attgctttat ttgtaaccat tataagctgc 11580

aataaacaag ttaacaacaa caattgcatt cattttatgt ttcaggttca gggggaggtg 11640

tgggaggttt tttaaagcaa gtaaaacctc tacaaatgtg gtatggctga ttatgatcgt 11700

tgcacattcc gatgtatgct gtgcagaata tgggactggt gcgcttccaa tccgttttca 11760

aatccattat cttccggttc actgtgagcg ggtcgagtgg gaatctcctc cgtagagccg 11820

aaactttctc tcttccagtg ggaaccgctt ccgcccgcca gaggttcttc agcagccgca 11880

gcagcagcag cgccactgtg taaggcttcc tccagacggc accgcaactc ggtagattca 11940

atggacttcg ggctgcgttt ctcgtacata acctcttcgt cgtcggtggt ggcggtcgtc 12000

gctttggtag atttcgtgtc cagattgagc gcgccaccgt tgttatccgg cgagctggac 12060

tgtgaccggg tacgaaaatc ggcacatggc gaccgtgacg cagacggagt gcgggtgacg 12120

gagatgttct cctcgtccgg ccagtgtctg tgtctgctgg aacgcttcgg aaagtagatg 12180

caacaaatgt cgtatcctgt ttgagggatt gatttttggg gggggggggg gcgaaagtgg 12240

ggaaagaaaa tgtaaaaaag aacaaattat ttattgttta tagacaagtg caatgctgtt 12300

aagaggatag tagactcaat gtctttccat caaatacggt tagaaattgt tcattctatg 12360

gagattgcac gacgccacga gtttgatttt accttttgat gtttgtcaca aacagcacta 12420

ggtactgtaa ttttgaggtg atgcaaataa attttgaacc atcctgctga acacaaattt 12480

attagacgta tttcgacgtt tggtgagctc gtttccatac ttgcctatct tacgttactt 12540

caatgaacaa ctaaatacac atttgtatgc gcttatcccc taaagacggg tgataatgtc 12600

aactgtttag ctaatttcca aaacaatcaa accttgatac gagataactg actttgcatc 12660

tctcaaacta cgattaatag aaagctatag caaattatgt acttagatag cattggaaag 12720

atgacgcttt ccgctacctc ataacgccat tggctttatc tatatgactg ttcatagctg 12780

gagagatgga tttgaaggtc taattctaag ttatcgcata tcaaggtatc gttgtgctgg 12840

aaaatctgat ctgacagtat cgaaatagcg caactctttg tacccaataa tggaaagttc 12900

tgatttattg tatttataaa aacgttgtct atttgttccg atttcaggtc gtcaaatcac 12960

ttgctagtaa ataacgtctc catagcaatt atcattatta tatactaaat gaaacgagct 13020

caccaattag atagtttcaa acagttatac agttgcttca aacaacatac atacatgcct 13080

tgataagtac cgtgcgccaa atcgagctcg caacatgagt atgaaaagcc actagtaaca 13140

cattgataat cagcatagaa tttaaaataa aataatattt tatactgtgg atatcttcat 13200

aacactgcac gctgatataa aattcagaac tacaaaagga tcgttgaaat tttacgtaac 13260

tcaacagagt aagggagggg gtcttccgaa atgctacggt ctatacacaa aatttaaatt 13320

tttcacacaa aagccgttac gtggaggagg gaggggttct aaaattggca aactttgcgt 13380

cacgtaatat ttgaatcatc ccaaagaaaa taaacttcta tgctgattat aacgcgcgca 13440

gaacaatggt tgcggtgaga gacattaaca ggcttgtttg tggtgctgaa atagaacttg 13500

tcatcaatat gttttagctg gttaaactat acaatcatta cgtagcctga aaatcaccct 13560

tgaaaaggcc gaatatatga cgaaaagaca cactctccaa ctcaaaaggc aaactcaacg 13620

tggtcgtgca caacctccaa tagcagtacc tgtcggagcc gtttggcaac ggccagctac 13680

caaaatacgc tcgcaatggc atgcaagcta cagagagata agtgtttatc agatcatttt 13740

gggcaccgaa accgaccgat gtcggaaacg attgaagaga tataatctct ggtttgtaga 13800

ttgtaggatg gttggttgaa gatccggttt cccggatttt ttcggatgga tgttgcttgt 13860

tgatgattct gctgtcgtcg ttttttttccg gtggcagatg gaacagcctc acttcggctt 13920

tcgaaacaca atcttaaaag ttaagtacta ctgctgtttt gcatttttta aattttccct 13980

ctaaacttgc tttgcactat tggtttcata gccgccgtac tcatcgatgc ccagggcgtc 14040

ggtgaacatc tgctcgaact cgaaatcggc catatccagg gcgccgtagg gggcgctatc 14100

gtgcggggtg aatcccggtc ccgggctatc gccatcgccc agcatgtcca ggtcgaagtc 14160

gtccagggca tcggcgtggg ccatcgccac atcctcgcca tccaggtgca gctcatcgcc 14220

caggctcacg tcggtcggcg gggcggtcga caggcggcgg gtgtgtccgg ccggcaggaa 14280

gctcaggcgc ggggcggcca ggcccgcctc ctccggggca tcatcatccg gcagatccag 14340

caggccctcg atggtgctgc cgtagttgtt cttggtgcgg gcgcggctgt aggcggggcc 14400

cgagcccgac tcgcatttca gttgcttttc caatccgcag ataatcagct ccaagccgaa 14460

caggaatgcc ggctcggctc cttgatgatc gaacagctcg attgcctgac gcagcagtgg 14520

gggcatcgaa tcggttgttg gggtctcgcg ctcctctttt gcgacttgat gctcttggtc 14580

ctccagcacg cagcccaggg taaagtgacc gacggcgctc agagcgtaga gagcattttc 14640

caggctgaag ccttgctggc acaggaacgc gagctggttc tccagtgtct cgtattgctt 14700

ttcggtcggg cgcgtgccga gatggacttt ggcaccgtct cggtgggaca gcagagcgca 14760

gcggaacgac ttggcgttat tgcggaggaa gtcctgccag gactcgcctt ccaacgggca 14820

aaaatgcgtg tggtggcggt cgagcatctc gatggccagg gcatccagca gcgcccgctt 14880

attcttcacg tgccagtaga gggtgggctg ctccacgccc agcttctgcg ccaacttgcg 14940

ggtcgtcagt ccctcaatgc caacttcgtt caacagctcc aacgcggagt tgatgacttt 15000

ggacttatcc aggcggctgc ccatggtcac ttgtttgcac tttcacactc tttaggaacg 15060

ctgtctcaca agtagagcta cacgtggtag ccccagaatg gctgtatgtc gctattatcg 15120

ttaaacagta tttgcactgt ggtcattatg ttgtttgtat tagagttcgt cgcgttcgtg 15180
gaattgggaa ggagaagata cagaattact caaatgaaac cattccacgg gagaatacat 15240
ttcaggttta atcttattct tctaggacga aagcccatcg acagagtctt gcaggcttcc 15300
gtcgatcgac tttcacccgt ggatgctaag gaagcttata atgacctcaa cattctccgc 15360
gcacaggttg gctctattct gtttcacagt ttccggtgca gttgtgacga actcagacga 15420
atacccacga ttgtatgtcc aacctcattt tttatctttg taaactaacg tcgaaaaatc 15480
tagatactac atttctgctt tgcttcatct tacactaatc actagtttga acttgcgggt 15540
tttccgttat gctttgtaaa tatgcgatgc tttagagttt tcttcgttcc gattcttctt 15600
tgcattcgat tgcttcttcc gtcgaatcga tctgatcttc gtggtttatt cttgtttcgg 15660
ttcgaccttt gccgcagcgc agtgggtcgt gctgatcgtg taaaaagtct atcatccgga 15720
ctggcgcgtc gtactgcgca actctacacc gtcgaacatg ttcagattgt gcaatcgtga 15780
gtattcattg accacggctt gacctgcgag gcagagaaga acagttggat ttttcggata 15840
ttggtacgac ccgggggccg cgttgtcatc agttgcatga atcgttggtc caagttcgac 15900
gaaacgatat ggacatcggt gtttcggtgg accaagatcg acgacacgat cttggtcatg 15960
agtgtttttc ggtggaccta gagatattgc aacgaccgga gtggaatacg acggtacgat 16020
gttggtttgt actgttctgg acgctagtta cttcattgat acgataaagt ttacattcgt 16080
catatctctt gcttttcttg aatccaaatg cttaggacgt gtaaccttca caaaccgtca 16140
taaatcagtt tcgattcact acatgttgta gttattcagg ctcttataat tgaatattca 16200
aaaatcgaat tttctatttt atcttgatca gaggatataa ctcgcttaaa atgcacaatt 16260
ttattagcga caccatgtgg atttgtttta attgaaacct ctatcttctc atatgtatca 16320
cgatataaaa tgctcatttt attgactgtt taacgataaa cttgcgacga tcgacgcacc 16380
accgacctaa ttccattgtg gaagcaaggg gcactgcaat accgaaatgt gaagtaaatt 16440
tcaaatctgc tattatagac gatgatctaa tactcttgaa tggtcttaaa cgtgagttgt 16500
atttcaagaa gttatgacga ttcgattttg gggccattat gaccccaaaa cccagccaac 16560
gtaactttta ttagtacaga cagaaggtca agcgtgcaag tctttcatcc gtgtgtcaat 16620
aaggccatca gttgaaaccg tgtcaattaa ccctccagtt aaccctttta acttttacca 16680
ggacaaacca atgacttcgt gcgcaaattc caccactcgt tgtctcaggc cttgagttgt 16740
tgtttgataa gaatggggga tgtcaagtcg gggagcgtag cccaacaggc tacggaaact 16800
gcatgatggc agtgtttgat ccagggcact gttgggaata gactccgtcg accgaagatc 16860
ccagatgtcc tgaaactcaa taataagcgt tagcagttac aaaatgggag cacccaggaa 16920
gtgagtgaca cccgatcgat acctcggaaa cagtcccaac cggtaagaac ccccatacct 16980
tcgtcaatcc gttggcgcgc tttattgacg tctccgtcgg cgcctttcag tatcacgtac 17040

gtcaggggcg tcgtctccca ggggtatcgc agcttctcca ggagccgttg agatcgtttg 17100

acaagttcgt cgtggtacct ggcctgaatc tcaacttgca cctgaaggta gtgcagcaag 17160

gatgagcaaa agggaagaac ccagaaaaga acgggaaaac ttaccccaat tagaattggc 17220

tagcgcagat tgtttagctt gttcagctgc gcttgtttat ttgcttagct ttcgcttagc 17280

gacgtgttca ctttgcttgt ttgaattgaa ttgtcgctcc gtagacgaag cgcctctatt 17340

tatactccgg cgctcgtttt cgagtttacc actccctatc agtgatagag aaaagtgaaa 17400

gtcgagttta ccactcccta tcagtgatag agaaaagtga aagtcgagtt taccactccc 17460

tatcagtgat agagaaaagt gaaagtcgag tttaccactc cctatcagtg atagagaaaa 17520

gtgaaagtcg agtttaccac tccctatcag tgatagagaa aagtgaaagt cgagtttacc 17580

actccctatc agtgatagag aaaagtgaaa gtcgagttta ccactcccta tcagtgatag 17640

agaaaagtga aagtcgaaac ctggcgcgcc ccggccatcg agaaagagag agagaagaga 17700

agagagagaa cattcgagaa agagagagag aagagaagag agagaacata ctccctatca 17760

gtgatagaga agtccctatc agtgatagag atgtccctat cagtgataga gagttcccta 17820

tcagtgatag agacgtccct atcagtgata gagaagtccc tatcagtgat agagagatcc 17880

ctatcagtga tagagatttc cctatcagtg atagagaggt ccctatcagt gatagagact 17940

tccctatcag tgatagagaa atccctatca gtgatagaga catccctatc agtgatagag 18000

aactccctat cagtgataga gacctcccta tcagtgatag agatcgatgc ggccgcggcg 18060

gatgcgatcg cgg 18073

<210> 158
<211> 13293
<212> DNA
<213> artificial

<220>
<223> LA3054 plasmid sequence

<400> 158
gggcgccgtt tttcttgaaa tattgctctc tctttctaaa tagcgcgaat ccgtcgctgt 60

gcatttagga catctcagtc gccgcttgga gctcccaaac gcgccagtgg tagtacacag 120

tactgtgggt gttcagtttg aaatcctctt gcttctccat tgtctcggtt acctttggtc 180

aaatccatgg gttctattgc ctatatactc ttgcgattac cagtgattgc gctattagct 240

attagatgga ttgttggcca aacttgtcgc ttaagtggct gggaattgta accgtaggcc 300

cgagtgtaat gatcccccat aaaaagtttt cgcaatgcct ttattttttg ttgcaaatct 360

ctctttattc tgcggtattc ttcattattg cggggatggg gaaagtgttt atatagaagc 420

aacttacgat tgaacccaaa tgcacctgac aagcaaggtc aaagggccag atttttaaat 480

atattattta gtcttaggac tctctatttg caattaaatt actttgctac ctgagggtta 540
aatcttcccc attgataata ataattccac tatatgttca attgggtttc accgcgctta 600
gttacatgac gagccctaat gagccgtcgg tggtctataa actgtgcctt acaaatactt 660
gcaactcttc tcgttttgaa gtcagcagag ttattgctaa ttgctaattg ctaattgctt 720
ttaactgatt tcttcgaaat tggtgctatg tttatggcgc tattaacaag tatgaatgtc 780
aggtttaacc aggggatgct taattgtgtt ctcaacttca aaggcagaaa tgtttactct 840
tgaccatggg tttaggtata atgttatcaa gctcctcgac gcgcctctta ctagaactac 900
ccaccgtact cgtcaattcc aagggcatcg gtaaacatct gctcaaactc gaagtcggcc 960
atatccagag cgccgtaggg ggcggagtcg tgggggtaa atcccggacc cggggaatcc 1020
ccgtccccca acatgtccag atcgaaatcg tctagcgcgt cggcatgcgc catcgccacg 1080
tcctcgccgt ctaagtggag ctcgtccccc aggctgacat cggtcggggg ggccgtcgac 1140
agtctgcgcg tgtgtcccgc ggggagaaag gacaggcgcg gagccgccag ccccgcctct 1200
tcgggggcgt cgtcgtccgg gagatcgagc aggccctcga tggtagaccc gtaattgttt 1260
ttcgtacgcg cgcggctgta cgcggggccc gagcccgact cgcatttcag ttgcttttcc 1320
aatccgcaga taatcagctc caagccgaac aggaatgccg gctcggctcc ttgatgatcg 1380
aacagctcga ttgcctgacg cagcagtggg ggcatcgaat cggttgttgg ggtctcgcgc 1440
tcctcttttg cgacttgatg ctcttggtcc tccagcacgc agcccagggt aaagtgaccg 1500
acggcgctca gagcgtagag agcattttcc aggctgaagc cttgctggca caggaacgcg 1560
agctggttct ccagtgtctc gtattgcttt tcggtcgggc gcgtgccgag atggactttg 1620
gcaccgtctc ggtgggacag cagagcgcag cggaacgact tggcgttatt gcggaggaag 1680
tcctgccagg actcgccttc caacgggcaa aaatgcgtgt ggtggcggtc gagcatctcg 1740
atggccaggg catccagcag cgcccgctta ttcttcacgt gccagtagag ggtgggctgc 1800
tccacgccca gcttctgcgc caacttgcgg gtcgtcagtc cctcaatgcc aacttcgttc 1860
aacagctcca acgcggagtt gatgactttg gacttatcca ggcggctgcc accacggaga 1920
cgaaggacca agtgaagggt ggactccttc tggatgttgt aatcggacag ggtgcgtcca 1980
tcctcaagct gcttgccggc gaagatcaga cgctgctgat ctgggggat tccctcctta 2040
tcctgaatct tggccttcac attctcaatg gtgtccgaag gctctacctc gagggtgatg 2100
gtctttccgg tcaaagtctt cacgaaaatc tgcatcgagc tagccagagg ctttgagcct 2160
tcacctatag ataccataga tgtatggatt agtatcatat acatacaaag gctatttttg 2220
ggacatatta atattaacaa tttccgtgat agttttcacc atttttgttg aatgttacgt 2280
tgaaaattta aatttgtttt aaattaattt taccagtcat gtgttcttaa aagtttttat 2340
gattgaaacg gcataaagtg gttcaaaaat ttatcaagaa aggctttcct tttttaaatc 2400

ttatcttttt ctcttaaaaa tcactagtca attcattatt aatttgttaa cttgaatttg 2460

gaatgtctat ttactttcag ataaattaaa gcaagaaact taatattcga aaaaaattga 2520

ttctaaatgg aatttcactt gatcttcatg tatgcatatc aatttttatt tacattgtat 2580

aataagtttc gagttgattg ttgtaatcca caggtgtccc agagaattaa attccaaatt 2640

acccaagttt attgaatgtt gattgtagtt tcagttgctt tgttgctgca acaatggctt 2700

gttgattgta gatattttcc ctttccttgg tttacttatt acatagactg aaaaagaggt 2760

ttactttttt gatacttatg aaaaatttct attagtgatt actaaccaat cgctatatgt 2820

ttactagaaa acaaataaac tctttacatt aacattcaat aatgtttgct ctgtaaccga 2880

caattgaagg cgttacagca acagtaatat aactagcttc ttaaccctca tctattaacc 2940

ccatcgttta aaacactatg ttaaatggtc taacaaatct agatactaat agatgtctta 3000

ttacttagca gccacagctg caacatccaa gacaattttt gaaacttctt attgagctct 3060

tggcagcaga aatgttggta tttttcacag ctttctgaaa gaccggcacc ttcctccggt 3120

tcccgtttct gaattcaaga ggatttccga cccccaatta atcccgaaac aaataaggta 3180

tattcaaaat gatggaaaag tcatggctgc tgaccttatt tttattccta ttgatagaat 3240

attattcccc ttttaaatac actgtactaa gaggtccggc tataatttta ctcacttgtc 3300

gattatccca tagaatgttg attgtagttg gttgcttttc caggtgagag ttgatcaagt 3360

cacaaaagtt agcgtgtgtt gattgtagat ttgaaggtaa aataattttt gcacccattc 3420

atcgggtaaa acgttctcca tagaatacat ttccatcgat aattgataac ttatgaattt 3480

caaagaaaaa aatatgcttt taaaattacc aaatctacgt ttaataacaa cagatctcag 3540

gaacaggtgg tggcggccct cggtgcgctc gtactgctcc acgatggtgt agtcctcgtt 3600

gtgggaggtg atgtccagct tggcgtccac gtagtagtag ccgggcagct gcacgggctt 3660

cttggccatg tagatggact tgaactccac caggtagtgg ccgccgtcct tcagcttcag 3720

ggccttgtgg gtctcgccct tcagcacgcc gtcgcggggg tacaggcgct cggtggaggc 3780

ctcccagccc atggtcttct tctgcatcac ggggccgtcg gaggggaagt tcacgccgat 3840

gaacttcacc ttgtagatga agcagccgtc ctgcagggag gagtcctggg tcacggtcgc 3900

cacgccgccg tcctcgaagt tcatcacgcg ctcccacttg aagccctcgg ggaaggacag 3960

cttcttgtag tcggggatgt cggcggggtg cttcacgtac accttggagc cgtactggaa 4020

ctggggggac aggatgtccc aggcgaaggg cagggggccg cccttggtca ccttcagctt 4080

cacggtgttg tggccctcgt aggggcggcc ctcgccctcg ccctcgatct cgaactcgtg 4140

gccgttcacg gtgccctcca tgcgcacctt gaagcgcatg aactcggtga tgacgttctc 4200

ggaggaggcc atggtggcga ccggtttgcg cttcttcttg ggtggggtgg gatccaccag 4260

agacaggttg cggcggcggt tggatggcgt gggcgcgttg gcgttgttgg accggctcat 4320
gttgtgtcgc tgtaacagat gctgttcaac tgtgtttacc agatcgttgc gggctgtatt 4380
tataggcgcg ataagcggga cgggcgcctc gtgtccggtc acgcgcatga gataacgcgc 4440
ggctgatatg gaggcgcgtc ctgttccgat aaggagttgc gtccggctgc ggttagcaac 4500
acaggaagct ggcgtcctgt cacgataaga caacactcgt ccggtccgat aatgtgattc 4560
gtacgtgaca ggacgcgacc cgataaggcc ggcctacgtg actgccgaca cgtacttttt 4620
tgcactgcaa aaaggttcaa tgtgtggtag tgtatttgga gcgtatacaa cggtgtagac 4680
tatttatgta aaatagtcta cgaaacgtag agtttgtact atgtatgggc ccgcgtgcaa 4740
aagcgtgttt ttttgcagtg caaaaaagtt ggtggtgggg aggccaccga gtatggtacc 4800
gcagattgtt tagcttgttc agctgcgctt gtttatttgc ttagctttcg cttagcgacg 4860
tgttcacttt gcttgtttga attgaattgt cgctccgtag acgaagcgcc tctatttata 4920
ctccggcgct cgttttcgag tttaccactc cctatcagtg atagagaaaa gtgaaagtcg 4980
agtttaccac tccctatcag tgatagagaa aagtgaaagt cgagtttacc actccctatc 5040
agtgatagag aaaagtgaaa gtcgagttta ccactcccta tcagtgatag agaaaagtga 5100
aagtcgagtt taccactccc tatcagtgat agagaaaagt gaaagtcgag tttaccactc 5160
cctatcagtg atagagaaaa gtgaaagtcg agtttaccac tccctatcag tgatagagaa 5220
aagtgaaagt cgaaacctgg cgcgccccgg ccatcgagaa agagagagag aagagaagag 5280
agagaacatt cgagaaagag agagagaaga gaagagagag aacatactcc ctatcagtga 5340
tagagaagtc cctatcagtg atagagatgt ccctatcagt gatagagagt tccctatcag 5400
tgatagagac gtccctatca gtgatagaga agtccctatc agtgatagag agatccctat 5460
cagtgataga gatttcccta tcagtgatag agaggtccct atcagtgata gagacttccc 5520
tatcagtgat agagaaatcc ctatcagtga tagagacatc cctatcagtg atagagaact 5580
ccctatcagt gatagagacc tccctatcag tgatagagat cgatgcggcc gcatggtacc 5640
cattgcttgt catttattaa tttggatgat gtcatttgtt tttaaaattg aactggcttt 5700
acgagtagaa ttctacgcgt aaaacacaat caagtatgag tcataatctg atgtcatgtt 5760
ttgtacacgg ctcataaccg aactggcttt acgagtagaa ttctacttgt aatgcacgat 5820
cagtggatga tgtcatttgt ttttcaaatc gagatgatgt catgttttgc acacggctca 5880
taaactcgct ttacgagtag aattctacgt gtaacgcacg atcgattgat gagtcatttg 5940
ttttgcaata tgatatcata caatatgact catttgtttt tcaaaaccga acttgattta 6000
cgggtagaat tctacttgta aagcacaatc aaaaagatga tgtcatttgt ttttcaaaac 6060
tgaactcgct ttacgagtag aattctacgt gtaaaacaca atcaagaaat gatgtcattt 6120
gttataaaaa taaaagctga tgtcatgttt tgcacatggc tcataactaa actcgcttta 6180

cgggtagaat tctacgcgta aaacatgatt gataattaaa taattcattt gcaagctata 6240
cgttaaatca aacggacgct cgaggttgca caacactatt atcgatttgc agttcgggac 6300
ataaatgttt aaatatatcg atgtctttgt gatgcgcgcg acatttttgt aggttattga 6360
taaaatgaac ggatacgttg cccgacatta tcattaaatc cttggcgtag aatttgtcgg 6420
gtccattgtc cgtgtgcgct agcatgcccg taacggacct cgtacttttg gcttcaaagg 6480
ttttgcgcac agacaaaatg tgccacactt gcagctctgc atgtgtgcgc gttaccacaa 6540
atcccaacgg cgcagtgtac ttgttgtatg caaataaatc tcgataaagg cgcggcgcgc 6600
gaatgcagct gatcacgtac gctcctcgtg ttccgttcaa ggacggtgtt atcgacctca 6660
gattaatgtt tatcggccga ctgttttcgt atccgctcac caaacgcgtt tttgcattaa 6720
cattgtatgt cggcggatgt tctatatcta atttgaataa ataaacgata accgcgttgg 6780
ttttagaggg cataataaaa gaaatattgt tatcgtgttc gccattaggg cagtataaat 6840
tgacgttcat gttggatatt gtttcagttg caagttgaca ctggcggcga caagcaattc 6900
taattggggt aagttttccc gttcttttct gggttcttcc cttttgctca tccttgctgc 6960
actaccttca ggtgcaagtt gagattcagg ccaccatggg agatcccacc ccacccaaga 7020
agaagcgcaa accggtcgcc accatggcct cctccgagaa cgtcatcacc gagttcatgc 7080
gcttcaaggt gcgcatggag ggcaccgtga acggccacga gttcgagatc gagggcgagg 7140
gcgagggccg cccctacgag ggccacaaca ccgtgaagct gaaggtgacc aagggcggcc 7200
ccctgccctt cgcctgggac atcctgtccc cccagttcca gtacggctcc aaggtgtacg 7260
tgaagcaccc cgccgacatc cccgactaca agaagctgtc cttccccgag ggcttcaagt 7320
gggagcgcgt gatgaacttc gaggacggcg gcgtggcgac cgtgacccag gactcctccc 7380
tgcaggacgg ctgcttcatc tacaaggtga agttcatcgg cgtgaacttc ccctccgacg 7440
gccccgtgat gcagaagaag accatgggct gggaggcctc caccgagcgc ctgtaccccc 7500
gcgacggcgt gctgaagggc gagacccaca aggccctgaa gctgaaggac ggcggccact 7560
acctggtgga gttcaagtcc atctacatgg ccaagaagcc cgtgcagctg cccggctact 7620
actacgtgga cgccaagctg gacatcacct cccacaacga ggactacacc atcgtggagc 7680
agtacgagcg caccgagggc cgccaccacc tgttcctgag atctcgaccc aagaaaaagc 7740
ggaaggtgga ggacccgtaa gatccaccgg atctagataa ctgatcataa tcagccatac 7800
cacatttgta gaggttttac ttgctttaaa aaacctccca cacctccccc tgaacctgaa 7860
acataaaatg aatgcaattg ttgttgttaa cttgtttatt gcagcttata atggttacaa 7920
ataaagcaat agcatcacaa atttcacaaa taaagcattt ttttcactgc attctagttg 7980
tggtttgtcc aaactcatca atgtatctta acgcgagtta attaaggccg ctcatttaaa 8040

tctggccggc cgcaaccatt gtgggaaccg tgcgatcaaa caaacgcgag ataccggaag 8100
tactgaaaaa cagtcgctcc aggccagtgg gaacatcgat gttttgtttt gacggacccc 8160
ttactctcgt ctcatataaa ccgaagccag ctaagatggt atacttatta tcatcttgtg 8220
atgaggatgc ttctatcaac gaaagtaccg gtaaaccgca aatggttatg tattataatc 8280
aaactaaagg cggagtggac acgctagacc aaatgtgttc tgtgatgacc tgcagtagga 8340
agacgaatag gtggcctatg gcattattgt acggaatgat aaacattgcc tgcataaatt 8400
cttttattat atacagccat aatgtcagta gcaagggaga aaaggtccaa agtcgcaaaa 8460
aatttatgag aaacctttac atgagcctga cgtcatcgtt tatgcgtaag cgtttagaag 8520
ctcctacttt gaagagatat ttgcgcgata atatctctaa tattttgcca aatgaagtgc 8580
ctggtacatc agatgacagt actgaagagc cagtaatgaa aaaacgtact tactgtactt 8640
actgcccctc taaaataagg cgaaaggcaa atgcatcgtg caaaaaatgc aaaaaagtta 8700
tttgtcgaga gcataatatt gatatgtgcc aaagttgttt ctgactgact aataagtata 8760
atttgtttct attatgtata agttaagcta attacttatt ttataataca acatgactgt 8820
ttttaaagta caaaataagt ttatttttgt aaaagagaga atgtttaaaa gttttgttac 8880
tttatagaag aaattttgag tttttgtttt tttttaataa ataaataaac ataaataaat 8940
tgtttgttga atttattatt agtatgtaag tgtaaatata ataaaactta atatctattc 9000
aaattaataa ataaacctcg atatacagac cgataaaaca catgcgtcaa ttttacgcat 9060
gattatcttt aacgtacgtc acaatatgat tatctttcta gggttaaata atagtttcta 9120
atttttttat tattcagcct gctgtcgtga ataccgtata tctcaacgct gtctgtgaga 9180
ttgtcgtatt ctagcctttt tagtttttcg ctcatcgact tgatattgtc cgacacattt 9240
tcgtcgattt gcgttttgat caaagacttg agcagagaca cgttaatcaa ctgttcaaat 9300
tgatccatat taacgatatc aacccgatgc gtatatggtg cgtaaaatat attttttaac 9360
cctcttatac tttgcactct gcgttaatac gcgttcgtgt acagacgtaa tcatgttttc 9420
tttttggat aaaactccta ctgagtttga cctcatatta gaccctcaca agttgcaaaa 9480
cgtggcattt tttaccaatg aagaatttaa agttatttta aaaaatttca tcacagattt 9540
aaagaagaac caaaaattaa attatttcaa cagtttaatc gaccagttaa tcaacgtgta 9600
cacagacgcg tcggcaaaaa acacgcagcc cgacgtgttg gctaaaatta ttaaatcaac 9660
ttgtgttata gtcacggatt tgccgtccaa cgtgttcctc aaaaagttga agaccaacaa 9720
gtttacggac actattaatt atttgatttt gccccacttc attttgtggg atcacaattt 9780
tgttatattt taaacaaagc ttggcactgg ccgtcgtttt acaacgtcgt gactgggaaa 9840
accctggcgt tacccaactt aatcgccttg cagcacatcc ccctttcgcc agctggcgta 9900
atagcgaaga ggcccgcacc gatcgccctt cccaacagtt gcgcagcctg aatggcgaat 9960

ggcgcctgat gcggtatttt ctccttacgc atctgtgcgg tatttcacac cgcatatggt 10020
gcactctcag tacaatctgc tctgatgccg catagttaag ccagccccga cacccgccaa 10080
cacccgctga cgcgccctga cgggcttgtc tgctcccggc atccgcttac agacaagctg 10140
tgaccgtctc cgggagctgc atgtgtcaga ggttttcacc gtcatcaccg aaacgcgcga 10200
gacgaaaggg cctcgtgata cgcctatttt tataggttaa tgtcatgata ataatggttt 10260
cttagacgtc aggtggcact tttcggggaa atgtgcgcgg aacccctatt tgtttatttt 10320
tctaaataca ttcaaatatg tatccgctca tgagacaata accctgataa atgcttcaat 10380
aatattgaaa aaggaagagt atgagtattc aacatttccg tgtcgccctt attccctttt 10440
ttgcggcatt ttgccttcct gtttttgctc acccagaaac gctggtgaaa gtaaaagatg 10500
ctgaagatca gttgggtgca cgagtgggtt acatcgaact ggatctcaac agcggtaaga 10560
tccttgagag ttttcgcccc gaagaacgtt ttccaatgat gagcactttt aaagttctgc 10620
tatgtggcgc ggtattatcc cgtattgacg ccgggcaaga gcaactcggt cgccgcatac 10680
actattctca gaatgacttg gttgagtact caccagtcac agaaaagcat cttacggatg 10740
gcatgacagt aagagaatta tgcagtgctg ccataaccat gagtgataac actgcggcca 10800
acttacttct gacaacgatc ggaggaccga aggagctaac cgcttttttg cacaacatgg 10860
gggatcatgt aactcgcctt gatcgttggg aaccggagct gaatgaagcc ataccaaacg 10920
acgagcgtga caccacgatg cctgtagcaa tggcaacaac gttgcgcaaa ctattaactg 10980
gcgaactact tactctagct tcccggcaac aattaataga ctggatggag gcggataaag 11040
ttgcaggacc acttctgcgc tcggcccttc cggctggctg gtttattgct gataaatctg 11100
gagccggtga gcgtgggtct cgcggtatca ttgcagcact ggggccagat ggtaagccct 11160
cccgtatcgt agttatctac acgacgggga gtcaggcaac tatggatgaa cgaaatagac 11220
agatcgctga gataggtgcc tcactgatta agcattggta actgtcagac caagtttact 11280
catatatact ttagattgat ttaaaacttc atttttaatt taaaaggatc taggtgaaga 11340
tcctttttga taatctcatg accaaaatcc cttaacgtga gttttcgttc cactgagcgt 11400
cagaccccgt agaaaagatc aaaggatctt cttgagatcc tttttttctg cgcgtaatct 11460
gctgcttgca aacaaaaaaa ccaccgctac cagcggtggt ttgtttgccg gatcaagagc 11520
taccaactct ttttccgaag gtaactggct tcagcagagc gcagatacca aatactgtcc 11580
ttctagtgta gccgtagtta ggccaccact tcaagaactc tgtagcaccg cctacatacc 11640
tcgctctgct aatcctgtta ccagtggctg ctgccagtgg cgataagtcg tgtcttaccg 11700
ggttggactc aagacgatag ttaccggata aggcgcagcg gtcgggctga acggggggtt 11760
cgtgcacaca gcccagcttg gagcgaacga cctacaccga actgagatac ctacagcgtg 11820

agcattgaga aagcgccacg cttcccgaag ggagaaaggc ggacaggtat ccggtaagcg 11880

gcagggtcgg aacaggagag cgcacgaggg agcttccagg gggaaacgcc tggtatcttt 11940

atagtcctgt cgggtttcgc cacctctgac ttgagcgtcg atttttgtga tgctcgtcag 12000

gggggcggag cctatggaaa aacgccagca acgcggcctt tttacggttc ctggcctttt 12060

gctggccttt tgctcacatg ttctttcctg cgttatcccc tgattctgtg gataaccgta 12120

ttaccgcctt tgagtgagct gataccgctc gccgcagccg aacgaccgag cgcagcgagt 12180

cagtgagcga ggaagcggaa gagcgcccaa tacgcaaacc gcctctcccc gcgcgttggc 12240

cgattcatta atgcagctgg cacgacaggt ttcccgactg gaaagcgggc agtgagcgca 12300

acgcaattaa tgtgagttag ctcactcatt aggcacccca ggctttacac tttatgcttc 12360

cggctcgtat gttgtgtgga attgtgagcg gataacaatt tcacacagga aacagctatg 12420

accatgatta cgaatttcga cctgcaggca tgcaagcttg catgcctgca ggtcgacgct 12480

cgcgcgactt ggtttgccat tctttagcgc gcgtcgcgtc acacagcttg gccacaatgt 12540

ggtttttgtc aaacgaagat tctatgacgt gtttaaagtt taggtcgagt aaagcgcaaa 12600

tctttttaa ccctagaaag atagtctgcg taaaattgac gcatgcattc ttgaaatatt 12660

gctctctctt tctaaatagc gcgaatccgt cgctgtgcat ttaggacatc tcagtcgccg 12720

cttggagctc ccgtgaggcg tgcttgtcaa tgcggtaagt gtcactgatt ttgaactata 12780

acgaccgcgt gagtcaaaat gacgcatgat tatcttttac gtgactttta agatttaact 12840

catacgataa ttatattgtt atttcatgtt ctacttacgt gataacttat tatatatata 12900

ttttcttgtt atagatatcg tgactaatat ataataaaat gggtagttct ttagacgatg 12960

agcatatcct ctctgctctt ctgcaaagcg atgacgagct tgttggtgag gattctgaca 13020

gtgaaatatc agatcacgta agtgaagatg acgtccagag cgatacagaa gaagcgttta 13080

tagatgaggt acatgaagtg cagccaacgt caagcggtag tgaaatatta gacgaacaaa 13140

atgttattga acaaccaggt tcttcattgg cttctaacag aatcttgacc ttgccacaga 13200

ggactattag aggtaagaat aaacattgtt ggtcaacttc aaagtccacg aggcgtagcc 13260

gagtctctgc actgaacatt gtcagatcgg ccc 13293

<210> 159
<211> 13515
<212> DNA
<213> artificial

<220>
<223> LA3056 plasmid sequence

<400> 159
gggcgccgtt tttcttgaaa tattgctctc tctttctaaa tagcgcgaat ccgtcgctgt 60

gcatttagga catctcagtc gccgcttgga gctcccaaac gcgccagtgg tagtacacag 120

tactgtgggt gttcagtttg aaatcctctt gcttctccat tgtctcggtt acctttggtc 180
aaatccatgg gttctattgc ctatatactc ttgcgattac cagtgattgc gctattagct 240
attagatgga ttgttggcca aacttgtcgc ttaagtggct gggaattgta accgtaggcc 300
cgagtgtaat gatcccccat aaaaagtttt cgcaatgcct ttattttttg ttgcaaatct 360
ctctttattc tgcggtattc ttcattattg cggggatggg gaaagtgttt atatagaagc 420
aacttacgat tgaacccaaa tgcacctgac aagcaaggtc aaagggccag atttttaaat 480
atattattta gtcttaggac tctctatttg caattaaatt actttgctac ctgagggtta 540
aatcttcccc attgataata ataattccac tatatgttca attgggtttc accgcgctta 600
gttacatgac gagccctaat gagccgtcgg tggtctataa actgtgcctt acaaatactt 660
gcaactcttc tcgttttgaa gtcagcagag ttattgctaa ttgctaattg ctaattgctt 720
ttaactgatt tcttcgaaat tggtgctatg tttatggcgc tattaacaag tatgaatgtc 780
aggtttaacc aggggatgct taattgtgtt ctcaacttca aaggcagaaa tgtttactct 840
tgaccatggg tttaggtata atgttatcaa gctcctcgac gcgcctctta ctagaactac 900
ccaccgtact cgtcaattcc aagggcatcg gtaaacatct gctcaaactc gaagtcggcc 960
atatccagag cgccgtaggg ggcggagtcg tgggggtaa atcccggacc cggggaatcc 1020
ccgtccccca acatgtccag atcgaaatcg tctagcgcgt cggcatgcgc catcgccacg 1080
tcctcgccgt ctaagtggag ctcgtccccc aggctgacat cggtcggggg ggccgtcgac 1140
agtctgcgcg tgtgtcccgc ggggagaaag gacaggcgcg gagccgccag ccccgcctct 1200
tcgggggcgt cgtcgtccgg gagatcgagc aggccctcga tggtagaccc gtaattgttt 1260
ttcgtacgcg cgcggctgta cgcggggccc gagcccgact cgcatttcag ttgcttttcc 1320
aatccgcaga taatcagctc caagccgaac aggaatgccg gctcggctcc ttgatgatcg 1380
aacagctcga ttgcctgacg cagcagtggg ggcatcgaat cggttgttgg ggtctcgcgc 1440
tcctcttttg cgacttgatg ctcttggtcc tccagcacgc agcccagggt aaagtgaccg 1500
acggcgctca gagcgtagag agcattttcc aggctgaagc cttgctggca caggaacgcg 1560
agctggttct ccagtgtctc gtattgcttt tcggtcgggc gcgtgccgag atggactttg 1620
gcaccgtctc ggtgggacag cagagcgcag cggaacgact tggcgttatt gcggaggaag 1680
tcctgccagg actcgccttc caacgggcaa aaatgcgtgt ggtggcggtc gagcatctcg 1740
atggccaggg catccagcag cgcccgctta ttcttcacgt gccagtagag ggtgggctgc 1800
tccacgccca gcttctgcgc caacttgcgg gtcgtcagtc cctcaatgcc aacttcgttc 1860
aacagctcca acgcggagtt gatgactttg gacttatcca ggcggctgcc accacggaga 1920
cgaaggacca agtgaagggt ggactccttc tggatgttgt aatcggacag ggtgcgtcca 1980

tcctcaagct gcttgccggc gaagatcaga cgctgctgat ctgggggat tccctcctta 2040
tcctgaatct tggccttcac attctcaatg gtgtccgaag gctctacctc gagggtgatg 2100
gtctttccgg tcaaagtctt cacgaaaatc tgcatcgagc tagcaaatcg ttctgggctg 2160
ctggaatcct tttaaaaaaa atgatttttt ttttgctata aagctatgaa gtagttcact 2220
tactgtcgat ttgtgacgct ctttgcgcca ttgatttcaa cctcctcttt actgttgtta 2280
ctccgatctt taggctgtgt ttcaaaatga gcacccacat tacttacaac attatcaggg 2340
tttacaacga tgtcgtcgcg ttgaaacaga ggctttgagc cttcacctat agataccata 2400
gatgtatgga ttagtatcat atacatacaa aggctatttt tgggacatat taatattaac 2460
aatttccgtg atagttttca ccatttttgt tgaatgttac gttgaaaatt taaatttgtt 2520
ttaaattaat tttaccagtc atgtgttctt aaaagttttt atgattgaaa cggcataaag 2580
tggttcaaaa atttatcaag aaaggctttc ctttttttaaa tcttatcttt ttctcttaaa 2640
aatcactagt caattcatta ttaatttgtt aacttgaatt tggaatgtct atttactttc 2700
agataaatta aagcaagaaa cttaatattc gaaaaaaatt gattctaaat ggaatttcac 2760
ttgatcttca tgtatgcata tcaattttta tttacattgt ataataagtt tcgagttgat 2820
tgttgtaatc cacaggtgtc ccagagaatt aaattccaaa ttacccaagt ttattgaatg 2880
ttgattgtag tttcagttgc tttgttgctg caacaatggc ttgttgattg tagatatttt 2940
ccctttcctt ggtttactta ttacatagac tgaaaaagag gtttactttt ttgatactta 3000
tgaaaaattt ctattagtga ttactaacca atcgctatat gtttactaga aaacaaataa 3060
actctttaca ttaacattca ataatgtttg ctctgtaacc gacaattgaa ggcgttacag 3120
caacagtaat ataactagct tcttaaccct catctattaa ccccatcgtt taaaacacta 3180
tgttaaatgg tctaacaaat ctagatacta atagatgtct tattacttag cagccacagc 3240
tgcaacatcc aagacaattt ttgaaacttc ttattgagct cttggcagca gaaatgttgg 3300
tatttttcac agctttctga aagaccggca ccttcctccg gttcccgttt ctgaattcaa 3360
gaggatttcc gacccccaat taatcccgaa acaaataagg tatattcaaa atgatggaaa 3420
agtcatggct gctgacctta tttttattcc tattgataga atattattcc ccttttaaat 3480
acactgtact aagaggtccg gctataattt tactcacttg tcgattatcc catagaatgt 3540
tgattgtagt tggttgcttt tccaggtgag agttgatcaa gtcacaaaag ttagcgtgtg 3600
ttgattgtag atttgaaggt aaaataattt ttgcacccat tcatcgggta aaacgttctc 3660
catagaatac atttccatcg ataattgata acttatgaat ttcaaagaaa aaaatatgct 3720
tttaaaatta ccaaatctac gtttaataac aacagatctc aggaacaggt ggtggcggcc 3780
ctcggtgcgc tcgtactgct ccacgatggt gtagtcctcg ttgtgggagg tgatgtccag 3840
cttggcgtcc acgtagtagt agccgggcag ctgcacgggc ttcttggcca tgtagatgga 3900

cttgaactcc accaggtagt ggccgccgtc cttcagcttc agggccttgt gggtctcgcc 3960
cttcagcacg ccgtcgcggg ggtacaggcg ctcggtggag gcctcccagc ccatggtctt 4020
cttctgcatc acggggccgt cggaggggaa gttcacgccg atgaacttca ccttgtagat 4080
gaagcagccg tcctgcaggg aggagtcctg ggtcacggtc gccacgccgc cgtcctcgaa 4140
gttcatcacg cgctcccact tgaagccctc ggggaaggac agcttcttgt agtcggggat 4200
gtcggcgggg tgcttcacgt acaccttgga gccgtactgg aactgggggg acaggatgtc 4260
ccaggcgaag ggcagggggc cgcccttggt caccttcagc ttcacggtgt tgtggccctc 4320
gtaggggcgg ccctcgccct cgccctcgat ctcgaactcg tggccgttca cggtgccctc 4380
catgcgcacc ttgaagcgca tgaactcggt gatgacgttc tcggaggagg ccatggtggc 4440
gaccggtttg cgcttcttct tgggtggggt gggatccacc agagacaggt tgcggcggcg 4500
gttggatggc gtgggcgcgt tggcgttgtt ggaccggctc atgttgtgtc gctgtaacag 4560
atgctgttca actgtgttta ccagatcgtt gcgggctgta tttataggcg cgataagcgg 4620
gacgggcgcc tcgtgtccgg tcacgcgcat gagataacgc gcggctgata tggaggcgcg 4680
tcctgttccg ataaggagtt gcgtccggct gcggttagca acacaggaag ctggcgtcct 4740
gtcacgataa gacaacactc gtccggtccg ataatgtgat tcgtacgtga caggacgcga 4800
cccgataagg ccggcctacg tgactgccga cacgtacttt tttgcactgc aaaaaggttc 4860
aatgtgtggt agtgtatttg gagcgtatac aacggtgtag actatttatg taaaatagtc 4920
tacgaaacgt agagtttgta ctatgtatgg gcccgcgtgc aaaagcgtgt ttttttgcag 4980
tgcaaaaaag ttggtggtgg ggaggccacc gagtatggta ccgcagattg tttagcttgt 5040
tcagctgcgc ttgtttattt gcttagcttt cgcttagcga cgtgttcact ttgcttgttt 5100
gaattgaatt gtcgctccgt agacgaagcg cctctattta tactccggcg ctcgttttcg 5160
agtttaccac tccctatcag tgatagagaa aagtgaaagt cgagtttacc actccctatc 5220
agtgatagag aaaagtgaaa gtcgagttta ccactcccta tcagtgatag agaaaagtga 5280
aagtcgagtt taccactccc tatcagtgat agagaaaagt gaaagtcgag tttaccactc 5340
cctatcagtg atagagaaaa gtgaaagtcg agtttaccac tccctatcag tgatagagaa 5400
aagtgaaagt cgagtttacc actccctatc agtgatagag aaaagtgaaa gtcgaaacct 5460
ggcgcgcccc ggccatcgag aaagagagag agaagagaag agagagaaca ttcgagaaag 5520
agagagagaa gagaagagag agaacatact ccctatcagt gatagagaag tccctatcag 5580
tgatagagat gtccctatca gtgatagaga gttccctatc agtgatagag acgtccctat 5640
cagtgataga gaagtcccta tcagtgatag agagatccct atcagtgata gagatttccc 5700
tatcagtgat agagaggtcc ctatcagtga tagagacttc cctatcagtg atagagaaat 5760

ccctatcagt gatagagaca tccctatcag tgatagagaa ctccctatca gtgatagaga 5820
cctccctatc agtgatagag atcgatgcgg ccgcatggta cccattgctt gtcatttatt 5880
aatttggatg atgtcatttg tttttaaaat tgaactggct ttacgagtag aattctacgc 5940
gtaaaacaca atcaagtatg agtcataatc tgatgtcatg ttttgtacac ggctcataac 6000
cgaactggct ttacgagtag aattctactt gtaatgcacg atcagtggat gatgtcattt 6060
gtttttcaaa tcgagatgat gtcatgtttt gcacacggct cataaactcg ctttacgagt 6120
agaattctac gtgtaacgca cgatcgattg atgagtcatt tgttttgcaa tatgatatca 6180
tacaatatga ctcatttgtt tttcaaaacc gaacttgatt tacgggtaga attctacttg 6240
taaagcacaa tcaaaaagat gatgtcattt gtttttcaaa actgaactcg ctttacgagt 6300
agaattctac gtgtaaaaca caatcaagaa atgatgtcat ttgttataaa aataaaagct 6360
gatgtcatgt tttgcacatg gctcataact aaactcgctt tacgggtaga attctacgcg 6420
taaaacatga ttgataatta aataattcat ttgcaagcta tacgttaaat caaacggacg 6480
ctcgaggttg cacaacacta ttatcgattt gcagttcggg acataaatgt ttaaatatat 6540
cgatgtcttt gtgatgcgcg cgacattttt gtaggttatt gataaaatga acggatacgt 6600
tgcccgacat tatcattaaa tccttggcgt agaatttgtc gggtccattg tccgtgtgcg 6660
ctagcatgcc cgtaacggac ctcgtacttt tggcttcaaa ggttttgcgc acagacaaaa 6720
tgtgccacac ttgcagctct gcatgtgtgc gcgttaccac aaatcccaac ggcgcagtgt 6780
acttgttgta tgcaaataaa tctcgataaa ggcgcggcgc gcgaatgcag ctgatcacgt 6840
acgctcctcg tgttccgttc aaggacggtg ttatcgacct cagattaatg tttatcggcc 6900
gactgttttc gtatccgctc accaaacgcg tttttgcatt aacattgtat gtcggcggat 6960
gttctatatc taatttgaat aaataaacga taaccgcgtt ggttttagag ggcataataa 7020
aagaaatatt gttatcgtgt tcgccattag ggcagtataa attgacgttc atgttggata 7080
ttgtttcagt tgcaagttga cactggcggc gacaagcaat tctaattggg gtaagttttc 7140
ccgttctttt ctgggttctt cccttttgct catccttgct gcactacctt caggtgcaag 7200
ttgagattca ggccaccatg ggagatccca ccccacccaa gaagaagcgc aaaccggtcg 7260
ccaccatggc ctcctccgag aacgtcatca ccgagttcat gcgcttcaag gtgcgcatgg 7320
agggcaccgt gaacggccac gagttcgaga tcgagggcga gggcgagggc cgcccctacg 7380
agggccacaa caccgtgaag ctgaaggtga ccaagggcgg cccctgccc ttcgcctggg 7440
acatcctgtc cccccagttc cagtacggct ccaaggtgta cgtgaagcac cccgccgaca 7500
tccccgacta caagaagctg tccttccccg agggcttcaa gtgggagcgc gtgatgaact 7560
tcgaggacgg cggcgtggcg accgtgaccc aggactcctc cctgcaggac ggctgcttca 7620
tctacaaggt gaagttcatc ggcgtgaact tcccctccga cggccccgtg atgcagaaga 7680

agaccatggg ctgggaggcc tccaccgagc gcctgtaccc ccgcgacggc gtgctgaagg 7740
gcgagaccca caaggccctg aagctgaagg acggcggcca ctacctggtg gagttcaagt 7800
ccatctacat ggccaagaag cccgtgcagc tgcccggcta ctactacgtg gacgccaagc 7860
tggacatcac ctcccacaac gaggactaca ccatcgtgga gcagtacgag cgcaccgagg 7920
gccgccacca cctgttcctg agatctcgac ccaagaaaaa gcggaaggtg gaggacccgt 7980
aagatccacc ggatctagat aactgatcat aatcagccat accacatttg tagaggtttt 8040
acttgcttta aaaaacctcc cacacctccc cctgaacctg aaacataaaa tgaatgcaat 8100
tgttgttgtt aacttgttta ttgcagctta taatggttac aaataaagca atagcatcac 8160
aaatttcaca aataaagcat ttttttcact gcattctagt tgtggtttgt ccaaactcat 8220
caatgtatct taacgcgagt taattaaggc cgctcattta aatctggccg gccgcaacca 8280
ttgtgggaac cgtgcgatca aacaaacgcg agataccgga agtactgaaa aacagtcgct 8340
ccaggccagt gggaacatcg atgttttgtt ttgacggacc ccttactctc gtctcatata 8400
aaccgaagcc agctaagatg gtatacttat tatcatcttg tgatgaggat gcttctatca 8460
acgaaagtac cggtaaaccg caaatggtta tgtattataa tcaaactaaa ggcggagtgg 8520
acacgctaga ccaaatgtgt tctgtgatga cctgcagtag gaagacgaat aggtggccta 8580
tggcattatt gtacggaatg ataaacattg cctgcataaa ttcttttatt atatacagcc 8640
ataatgtcag tagcaaggga gaaaaggtcc aaagtcgcaa aaaatttatg agaaaccttt 8700
acatgagcct gacgtcatcg tttatgcgta agcgtttaga agctcctact ttgaagagat 8760
atttgcgcga taatatctct aatattttgc caaatgaagt gcctggtaca tcagatgaca 8820
gtactgaaga gccagtaatg aaaaaacgta cttactgtac ttactgcccc tctaaaataa 8880
ggcgaaaggc aaatgcatcg tgcaaaaaat gcaaaaaagt tatttgtcga gagcataata 8940
ttgatatgtg ccaaagttgt ttctgactga ctaataagta taatttgttt ctattatgta 9000
taagttaagc taattactta ttttataata caacatgact gtttttaaag tacaaaataa 9060
gtttattttt gtaaaagaga gaatgtttaa aagttttgtt actttataga agaaattttg 9120
agtttttgtt ttttttaat aaataaataa acataaataa attgtttgtt gaatttatta 9180
ttagtatgta agtgtaaata taataaaact taatatctat tcaaattaat aaataaacct 9240
cgatatacag accgataaaa cacatgcgtc aattttacgc atgattatct ttaacgtacg 9300
tcacaatatg attatctttc tagggttaaa taatagtttc taattttttt attattcagc 9360
ctgctgtcgt gaataccgta tatctcaacg ctgtctgtga gattgtcgta ttctagcctt 9420
tttagttttt cgctcatcga cttgatattg tccgacacat tttcgtcgat ttgcgttttg 9480
atcaaagact tgagcagaga cacgttaatc aactgttcaa attgatccat attaacgata 9540

tcaacccgat gcgtatatgg tgcgtaaaat atatttttta accctcttat actttgcact 9600
ctgcgttaat acgcgttcgt gtacagacgt aatcatgttt tctttttttgg ataaaactcc 9660
tactgagttt gacctcatat tagaccctca caagttgcaa aacgtggcat tttttaccaa 9720
tgaagaattt aaagttattt taaaaaattt catcacagat ttaaagaaga accaaaaatt 9780
aaattatttc aacagtttaa tcgaccagtt aatcaacgtg tacacagacg cgtcggcaaa 9840
aaacacgcag cccgacgtgt tggctaaaat tattaaatca acttgtgtta tagtcacgga 9900
tttgccgtcc aacgtgttcc tcaaaaagtt gaagaccaac aagtttacgg acactattaa 9960
ttatttgatt ttgccccact tcattttgtg ggatcacaat tttgttatat tttaaacaaa 10020
gcttggcact ggccgtcgtt ttacaacgtc gtgactggga aaaccctggc gttacccaac 10080
ttaatcgcct tgcagcacat ccccctttcg ccagctggcg taatagcgaa gaggcccgca 10140
ccgatcgccc ttcccaacag ttgcgcagcc tgaatggcga atggcgcctg atgcggtatt 10200
ttctccttac gcatctgtgc ggtatttcac accgcatatg gtgcactctc agtacaatct 10260
gctctgatgc cgcatagtta agccagcccc gacacccgcc aacacccgct gacgcgccct 10320
gacgggcttg tctgctcccg gcatccgctt acagacaagc tgtgaccgtc tccgggagct 10380
gcatgtgtca gaggttttca ccgtcatcac cgaaacgcgc gagacgaaag ggcctcgtga 10440
tacgcctatt tttataggtt aatgtcatga taataatggt ttcttagacg tcaggtggca 10500
cttttcgggg aaatgtgcgc ggaaccccta tttgtttatt tttctaaata cattcaaata 10560
tgtatccgct catgagacaa taaccctgat aaatgcttca ataatattga aaaaggaaga 10620
gtatgagtat tcaacatttc cgtgtcgccc ttattccctt ttttgcggca ttttgccttc 10680
ctgtttttgc tcacccagaa acgctggtga aagtaaaaga tgctgaagat cagttgggtg 10740
cacgagtggg ttacatcgaa ctggatctca acagcggtaa gatccttgag agttttcgcc 10800
ccgaagaacg ttttccaatg atgagcactt ttaaagttct gctatgtggc gcggtattat 10860
cccgtattga cgccgggcaa gagcaactcg gtcgccgcat acactattct cagaatgact 10920
tggttgagta ctcaccagtc acagaaaagc atcttacgga tggcatgaca gtaagagaat 10980
tatgcagtgc tgccataacc atgagtgata acactgcggc caacttactt ctgacaacga 11040
tcggaggacc gaaggagcta accgcttttt tgcacaacat gggggatcat gtaactcgcc 11100
ttgatcgttg ggaaccggag ctgaatgaag ccataccaaa cgacgagcgt gacaccacga 11160
tgcctgtagc aatggcaaca acgttgcgca aactattaac tggcgaacta cttactctag 11220
cttcccggca acaattaata gactggatgg aggcggataa agttgcagga ccacttctgc 11280
gctcggccct tccggctggc tggtttattg ctgataaatc tggagccggt gagcgtgggt 11340
ctcgcggtat cattgcagca ctggggccag atggtaagcc ctcccgtatc gtagttatct 11400
acacgacggg gagtcaggca actatggatg aacgaaatag acagatcgct gagataggtg 11460

cctcactgat taagcattgg taactgtcag accaagttta ctcatatata ctttagattg 11520

atttaaaact tcatttttaa tttaaaagga tctaggtgaa gatccttttt gataatctca 11580

tgaccaaaat cccttaacgt gagttttcgt tccactgagc gtcagacccc gtagaaaaga 11640

tcaaaggatc ttcttgagat cctttttttc tgcgcgtaat ctgctgcttg caaacaaaaa 11700

aaccaccgct accagcggtg gtttgtttgc cggatcaaga gctaccaact ctttttccga 11760

aggtaactgg cttcagcaga gcgcagatac caaatactgt ccttctagtg tagccgtagt 11820

taggccacca cttcaagaac tctgtagcac cgcctacata cctcgctctg ctaatcctgt 11880

taccagtggc tgctgccagt ggcgataagt cgtgtcttac cgggttggac tcaagacgat 11940

agttaccgga taaggcgcag cggtcgggct gaacgggggg ttcgtgcaca cagcccagct 12000

tggagcgaac gacctacacc gaactgagat acctacagcg tgagcattga gaaagcgcca 12060

cgcttcccga agggagaaag gcggacaggt atccggtaag cggcagggtc ggaacaggag 12120

agcgcacgag ggagcttcca gggggaaacg cctggtatct ttatagtcct gtcgggtttc 12180

gccacctctg acttgagcgt cgatttttgt gatgctcgtc aggggggcgg agcctatgga 12240

aaaacgccag caacgcggcc tttttacggt tcctggcctt ttgctggcct tttgctcaca 12300

tgttctttcc tgcgttatcc cctgattctg tggataaccg tattaccgcc tttgagtgag 12360

ctgataccgc tcgccgcagc cgaacgaccg agcgcagcga gtcagtgagc gaggaagcgg 12420

aagagcgccc aatacgcaaa ccgcctctcc ccgcgcgttg gccgattcat taatgcagct 12480

ggcacgacag gtttcccgac tggaaagcgg gcagtgagcg caacgcaatt aatgtgagtt 12540

agctcactca ttaggcaccc caggctttac actttatgct tccggctcgt atgttgtgtg 12600

gaattgtgag cggataacaa tttcacacag gaaacagcta tgaccatgat tacgaatttc 12660

gacctgcagg catgcaagct tgcatgcctg caggtcgacg ctcgcgcgac ttggtttgcc 12720

attctttagc gcgcgtcgcg tcacacagct tggccacaat gtggtttttg tcaaacgaag 12780

attctatgac gtgtttaaag tttaggtcga gtaaagcgca aatctttttt aaccctagaa 12840

agatagtctg cgtaaaattg acgcatgcat tcttgaaata ttgctctctc tttctaaata 12900

gcgcgaatcc gtcgctgtgc atttaggaca tctcagtcgc cgcttggagc tcccgtgagg 12960

cgtgcttgtc aatgcggtaa gtgtcactga ttttgaacta taacgaccgc gtgagtcaaa 13020

atgacgcatg attatctttt acgtgacttt taagatttaa ctcatacgat aattatattg 13080

ttatttcatg ttctacttac gtgataactt attatatata tattttcttg ttatagatat 13140

cgtgactaat atataataaa atgggtagtt ctttagacga tgagcatatc ctctctgctc 13200

ttctgcaaag cgatgacgag cttgttggtg aggattctga cagtgaaata tcagatcacg 13260

taagtgaaga tgacgtccag agcgatacag aagaagcgtt tatagatgag gtacatgaag 13320

tgcagccaac gtcaagcggt agtgaaatat tagacgaaca aaatgttatt gaacaaccag 13380

gttcttcatt ggcttctaac agaatcttga ccttgccaca gaggactatt agaggtaaga 13440

ataaacattg ttggtcaact tcaaagtcca cgaggcgtag ccgagtctct gcactgaaca 13500

ttgtcagatc ggccc 13515

<210> 160
<211> 9423
<212> DNA
<213> artificial

<220>
<223> LA3488 plasmid sequence

<400> 160
cggcgcgccg gctttacgag tagaattcta cgcgtaaaac acaatcaagt atgagtcata 60

atctgatgtc atgttttgta cacggctcat aaccgaactg gctttacgag tagaattcta 120

cttgtaatgc acgatcagtg gatgatgtca tttgtttttc aaatcgagat gatgtcatgt 180

tttgcacacg gctcataaac tcgctttacg agtagaattc tacgtgtaac gcacgatcga 240

ttgatgagtc atttgttttg caatatgata tcatacaata tgactcattt gtttttcaaa 300

accgaacttg atttacgggt agaattctac ttgtaaagca caatcaaaaa gatgatgtca 360

tttgtttttc aaaactgaac tcgctttacg agtagaattc tacgtgtaaa acacaatcaa 420

gaaatgatgt catttgttat aaaaataaaa gctgatgtca tgttttgcac atggctcata 480

actaaactcg ctttacgggt agaattctac gcgtaaaaca tgattgataa ttaaataatt 540

catttgcaag ctatacgtta aatcaaacgg acgctcgagg ttgcacaaca ctattatcga 600

tttgcagttc gggacataaa tgtttaaata tatcgatgtc tttgtgatgc gcgcgacatt 660

tttgtaggtt attgataaaa tgaacggata cgttgcccga cattatcatt aaatccttgg 720

cgtagaattt gtcgggtcca ttgtccgtgt gcgctagcat gcccgtaacg gacctcgtac 780

ttttggcttc aaaggttttg cgcacagaca aaatgtgcca cacttgcagc tctgcatgtg 840

tgcgcgttac cacaaatccc aacggcgcag tgtacttgtt gtatgcaaat aaatctcgat 900

aaaggcgcgg cgcgcgaatg cagctgatca cgtacgctcc tcgtgttccg ttcaaggacg 960

gtgttatcga cctcagatta atgtttatcg gccgactgtt ttcgtatccg ctcaccaaac 1020

gcgtttttgc attaacattg tatgtcggcg gatgttctat atctaatttg aataaataaa 1080

cgataaccgc gttggtttta gagggcataa taaaagaaat attgttatcg tgttcgccat 1140

tagggcagta taaattgacg ttcatgttgg atattgtttc agttgcaagt tgacactggc 1200

ggcgacaagc aattctaatt ggggtaagtt ttcccgttct tttctgggtt cttccctttt 1260

gctcatcctt gctgcactac cttcaggtgc aagttgagat tcaggccacc atgggagatc 1320

ccacccccacc caagaagaag cgcaaaccgg tcgccaccat ggagagcgac gagagcggcc 1380

tgcccgccat ggagatcgag tgccgcatca ccggcaccct gaacggcgtg gagttcgagc 1440

tggtgggcgg cggagagggc acccccgagc agggccgcat gaccaacaag atgaagagca 1500

ccaaaggcgc cctgaccttc agcccctacc tgctgagcca cgtgatgggc tacggcttct 1560

accacttcgg cacctacccc agcggctacg agaacccctt cctgcacgcc atcaacaacg 1620

gcggctacac caacacccgc atcgagaagt acgaggacgg cggcgtgctg cacgtgagct 1680

tcagctaccg ctacgaggcc ggccgcgtga tcggcgactt caaggtgatg ggcaccggct 1740

tccccgagga cagcgtgatc ttcaccgaca agatcatccg cagcaacgcc accgtggagc 1800

acctgcaccc catgggcgat aacgatctgg atggcagctt cacccgcacc ttcagcctgc 1860

gcgacggcgg ctactacagc tccgtggtgg acagccacat gcacttcaag agcgccatcc 1920

accccagcat cctgcagaac gggggcccca tgttcgcctt ccgccgcgtg gaggaggatc 1980

acagcaacac cgagctgggc atcgtggagt accagcacgc cttcaagacc ccggatgcag 2040

atgccggtga agaaagatct cgacccaaga aaaagcggaa ggtggaggac ccgtctggag 2100

gcggtggatc cggcggtgga ggcatgcaga tctttgtgaa gactttgacc ggaaagacca 2160

tcaccctcga ggtagagcca tcggacacca ttgagaatgt aaaggccaag attcaggata 2220

aggagggaat ccccccagat cagcagcgtc tgatcttcgc tggtaatttt aaaagcatat 2280

ttttttcttt gaaattcata agttatcaat tatcgatgga aatgtattct atggagaacg 2340

ttttacccga tgaatgggtg caaaaattat tttaccttca aatctacaat caacacacgc 2400

taacttttgt gacttgatca actctcacct ggaaaagcaa ccaactacaa tcaacattct 2460

atgggataat cgacaagtga gtaaaattat agccggacct cttagtacag tgtatttaaa 2520

aggggaataa tattctatca ataggaataa aaataaggtc agcagccatg acttttccat 2580

cattttgaat ataccttatt tgtttcggga ttaattgggg gtcggaaatc ctcttgaatt 2640

cagaaacggg aaccggagga aggtgccggt ctttcagaaa gctgtgaaaa ataccaacat 2700

ttctgctgcc aagagctcaa taagaagttt caaaaattgt cttggatgtt gcagctgtgg 2760

ctgctaagta ataagacatc tattagtatc tagatttgtt agaccattta acatagtgtt 2820

ttaaacgatg ggttaatag atgagggtta agaagctagt tatattactg ttgctgtaac 2880

gccttcaatt gtcggttaca gagcaaacat tattgaatgt taatgtaaag agtttatttg 2940

ttttctagta aacatatagc gattggttag taatcactaa tagaaatttt tcataagtat 3000

caaaaaagta aacctctttt tcagtctatg taataagtaa accaaggaaa gggaaaatat 3060

ctacaatcaa caagccattg ttgcagcaac aaagcaactg aaactacaat caacattcaa 3120

taaacttggg taatttggaa tttaattctc tgggacacct gtggattaca acaatcaact 3180

cgaaacttat tatacaatgt aaataaaaat tgatatgcat acatgaagat caagtgaaat 3240

tccatttaga atcaattttt ttcgaatatt aagtttcttg ctttaattta tctgaaagta 3300
aatagacatt ccaaattcaa gttaacaaat taataatgaa ttgactagtg atttttaaga 3360
gaaaaagata agatttaaaa aaggaaagcc tttcttgata aatttttgaa ccactttatg 3420
ccgtttcaat cataaaaact tttaagaaca catgactggt aaaattaatt taaaacaaat 3480
ttaaattttc aacgtaacat tcaacaaaaa tggtgaaaac tatcacggaa attgttaata 3540
ttaatatgtc ccaaaaatag cctttgtatg tatatgatac taatccatac atctatggta 3600
tctataggta agcaactgga agacggacgc accctgtccg attacaacat ccagaaggag 3660
tccacccttc acttggtcct tcgtctccgc ggtggcatgc agatcgggga tcccacccca 3720
cccaagaaga agcgcaaacc ggtcgccacc atggcctcct ccgagaacgt catcaccgag 3780
ttcatgcgct tcaaggtgcg catggagggc accgtgaacg gccacgagtt cgagatcgag 3840
ggcgagggcg agggccgccc ctacgagggc cacaacaccg tgaagctgaa ggtgaccaag 3900
ggcggccccc tgcccttcgc ctgggacatc ctgtcccccc agttccagta cggctccaag 3960
gtgtacgtga agcaccccgc cgacatcccc gactacaaga agctgtcctt ccccgagggc 4020
ttcaagtggg agcgcgtgat gaacttcgag gacggcggcg tggcgaccgt gacccaggac 4080
tcctccctgc aggacggctg cttcatctac aaggtgaagt tcatcggcgt gaacttcccc 4140
tccgacggcc ccgtgatgca gaagaagacc atgggctggg aggcctccac cgagcgcctg 4200
taccccgcg acggcgtgct gaagggcgag acccacaagg ccctgaagct gaaggacggc 4260
ggccactacc tggtggagtt caagtccatc tacatggcca agaagcccgt gcagctgccc 4320
ggctactact acgtggacgc caagctggac atcacctccc acaacgagga ctacaccatc 4380
gtggagcagt acgagcgcac cgagggccgc caccacctgt tcctgagatc tcgacccaag 4440
aaaaagcgga aggtggagga cccgtaagat ccaccgggtc tagataactg atcataatca 4500
gccataccac atttgtagag gttttacttg ctttaaaaaa cctcccacac ctccccctga 4560
acctgaaaca taaaatgaat gcaattgttg ttgttaactt gtttattgca gcttataatg 4620
gttacaaata aagcaatagc atcacaaatt tcacaaataa agcatttttt tcactgcatt 4680
ctagttgtgg tttgtccaaa ctcatcaatg tatcttaacg cgagttaatt aagaggcgcg 4740
gtaaaccgca aatggttatg tattataatc aaactaaagg cggagtggac acgctagacc 4800
aaatgtgttc tgtgatgacc tgcagtagga agacgaatag gtggcctatg gcattattgt 4860
acggaatgat aaacattgcc tgcataaatt cttttattat atacagccat aatgtcagta 4920
gcaagggaga aaaggtccaa agtcgcaaaa aatttatgag aaacctttac atgagcctga 4980
cgtcatcgtt tatgcgtaag cgtttagaag ctcctacttt gaagagatat ttgcgcgata 5040
atatctctaa tattttgcca aatgaagtgc ctggtacatc agatgacagt actgaagagc 5100
cagtaatgaa aaaacgtact tactgtactt actgcccctc taaaataagg cgaaaggcaa 5160

atgcatcgtg caaaaaatgc aaaaaagtta tttgtcgaga gcataatatt gatatgtgcc 5220
aaagttgttt ctgactgact aataagtata atttgtttct attatgtata agttaagcta 5280
attacttatt ttataataca acatgactgt ttttaaagta caaaataagt ttatttttgt 5340
aaaagagaga atgtttaaaa gttttgttac tttatagaag aaattttgag tttttgtttt 5400
tttttaataa ataaataaac ataaataaat tgtttgttga atttattatt agtatgtaag 5460
tgtaaatata ataaaactta atatctattc aaattaataa ataaacctcg atatacagac 5520
cgataaaaca catgcgtcaa ttttacgcat gattatcttt aacgtacgtc acaatatgat 5580
tatctttcta gggttaaata atagtttcta atttttttat tattcagcct gctgtcgtga 5640
ataccgtata tctcaacgct gtctgtgaga ttgtcgtatt ctagcctttt tagtttttcg 5700
ctcatcgact tgatattgtc cgacacattt tcgtcgattt gcgttttgat caaagacttg 5760
agcagagaca cgttaatcaa ctgttcaaat tgatccatat taacgatatc aacccgatgc 5820
gtatatggtg cgtaaaatat attttttaac cctcttatac tttgcactct gcgttaatac 5880
gcgttcgtgt acagacgtaa tcatgttttc ttttttggat aaaactccta ctgagtttga 5940
cctcatatta gaccctcaca agttgcaaaa cgtggcattt tttaccaatg aagaatttaa 6000
agttatttta aaaaatttca tcacagattt aaagaagaac caaaaattaa attatttcaa 6060
cagtttaatc gaccagttaa tcaacgtgta cacagacgcg tcggcaaaaa acacgcagcc 6120
cgacgtgttg gctaaaatta ttaaatcaac ttgtgttata gtcacggatt tgccgtccaa 6180
cgtgttcctc aaaaagttga agaccaacaa gtttacggac actattaatt atttgatttt 6240
gccccacttc attttgtggg atcacaattt tgttatattt taaacaaagc ttggcactgg 6300
ccgtcgtttt acaacgtcgt gactgggaaa accctggcgt tacccaactt aatcgccttg 6360
cagcacatcc ccctttcgcc agctggcgta atagcgaaga ggcccgcacc gatcgccctt 6420
cccaacagtt gcgcagcctg aatggcgaat ggcgcctgat gcggtatttt ctccttacgc 6480
atctgtgcgg tatttcacac cgcatatatg gtgcactctc agtacaatct gctctgatgc 6540
cgcatagtta agccagcccc gacacccgcc aacacccgct gacgcgccct gacgggcttg 6600
tctgctcccg gcatccgctt acagacaagc tgtgaccgtc tccgggagct gcatgtgtca 6660
gaggttttca ccgtcatcac cgaaacgcgc gagacgaaag ggcctcgtga tacgcctatt 6720
tttataggtt aatgtcatga taataatggt ttcttagacg tcaggtggca cttttcgggg 6780
aaatgtgcgc ggaaccccta tttgtttatt tttctaaata cattcaaata tgtatccgct 6840
catgagacaa taaccctgat aaatgcttca ataatattga aaaaggaaga gtatgagtat 6900
tcaacatttc cgtgtcgccc ttattccctt ttttgcggca ttttgccttc ctgtttttgc 6960
tcacccagaa acgctggtga aagtaaaaga tgctgaagat cagttgggtg cacgagtggg 7020

ttacatcgaa ctggatctca acagcggtaa gatccttgag agttttcgcc ccgaagaacg 7080

ttttccaatg atgagcactt ttaaagttct gctatgtggc gcggtattat cccgtattga 7140

cgccgggcaa gagcaactcg gtcgccgcat acactattct cagaatgact tggttgagta 7200

ctcaccagtc acagaaaagc atcttacgga tggcatgaca gtaagagaat tatgcagtgc 7260

tgccataacc atgagtgata acactgcggc caacttactt ctgacaacga tcggaggacc 7320

gaaggagcta accgcttttt tgcacaacat gggggatcat gtaactcgcc ttgatcgttg 7380

ggaaccggag ctgaatgaag ccataccaaa cgacgagcgt gacaccacga tgcctgtagc 7440

aatggcaaca acgttgcgca aactattaac tggcgaacta cttactctag cttcccggca 7500

acaattaata gactggatgg aggcggataa agttgcagga ccacttctgc gctcggccct 7560

tccggctggc tggtttattg ctgataaatc tggagccggt gagcgtgggt ctcgcggtat 7620

cattgcagca ctggggccag atggtaagcc ctcccgtatc gtagttatct acacgacggg 7680

gagtcaggca actatggatg aacgaaatag acagatcgct gagataggtg cctcactgat 7740

taagcattgg taactgtcag accaagttta ctcatatata ctttagattg atttaaaact 7800

tcatttttaa tttaaaagga tctaggtgaa gatccttttt gataatctca tgaccaaaat 7860

cccttaacgt gagttttcgt tccactgagc gtcagacccc gtagaaaaga tcaaaggatc 7920

ttcttgagat cctttttttc tgcgcgtaat ctgctgcttg caaacaaaaa aaccaccgct 7980

accagcggtg gtttgtttgc cggatcaaga gctaccaact ctttttccga aggtaactgg 8040

cttcagcaga gcgcagatac caaatactgt ccttctagtg tagccgtagt taggccacca 8100

cttcaagaac tctgtagcac cgcctacata cctcgctctg ctaatcctgt taccagtggc 8160

tgctgccagt ggcgataagt cgtgtcttac cgggttggac tcaagacgat agttaccgga 8220

taaggcgcag cggtcgggct gaacgggggg ttcgtgcaca cagcccagct tggagcgaac 8280

gacctacacc gaactgagat acctacagcg tgagcattga gaaagcgcca cgcttcccga 8340

agggagaaag gcggacaggt atccggtaag cggcagggtc ggaacaggag agcgcacgag 8400

ggagcttcca gggggaaacg cctggtatct ttatagtcct gtcgggtttc gccacctctg 8460

acttgagcgt cgatttttgt gatgctcgtc aggggggcgg agcctatgga aaaacgccag 8520

caacgcggcc tttttacggt tcctggcctt ttgctggcct tttgctcaca tgttctttcc 8580

tgcgttatcc cctgattctg tggataaccg tattaccgcc tttgagtgag ctgataccgc 8640

tcgccgcagc cgaacgaccg agcgcagcga gtcagtgagc gaggaagcgg aagagcgccc 8700

aatacgcaaa ccgcctctcc ccgcgcgttg gccgattcat taatgcagct ggcacgacag 8760

gtttcccgac tggaaagcgg gcagtgagcg caacgcaatt aatgtgagtt agctcactca 8820

ttaggcaccc caggctttac actttatgct tccggctcgt atgttgtgtg gaattgtgag 8880

cggataacaa tttcacacag gaaacagcta tgaccatgat tacgaatttc gacgctcgcg 8940

cgacttggtt tgccattctt tagcgcgcgt cgcgtcacac agcttggcca caatgtggat 9000

gtcgacttaa ccctagaaag atagtctgcg taaaattgac gcatgcattc ttgaaatatt 9060

gctctctctt tctaaatagc gcgaatccgt cgctgtgcat ttaggacatc tcagtcgccg 9120

cttggagctc ccgtgaggcg tgcttgtcaa tgcggtaagt gtcactgatt ttgaactata 9180

acgaccgcgt gagtcaaaat gacgcatgat tatcttttac gtgactttta agatttaact 9240

catacgataa ttatattgtt atttcatgtt ctacttacgt gataacttat tatatatata 9300

ttttcttgtt atagatatct accggtcata ctcggtggcc tccccaccac caactttttt 9360

gcactgcaaa aaaacacgct tttgcacgcg ggcccggcgc gccatctgcc ggccgcatgg 9420

tac 9423

<210> 161
<211> 17781
<212> DNA
<213> artificial

<220>
<223> LA3641plasmid sequence

<400> 161
ttaaaatgaa tgtaagcact ttattaacga aatctttggg aatatttcgc tcatcagcat 60

tttatttgag caggagtccg agatgccccc ttcccttaag tcaatattac aaacaatgtg 120

gttttccgcc aaccacagtg tggttaaatt ttatcaccga tgatatgaaa tttctagctg 180

caacatgtcc acaagaaata ccataattct catggttgct taacaactgt taattataca 240

tcaggcaaag tattcactgg ttttcttaat atatctggga ataattactt caaggaccgg 300

gttaacaaga taaggtaacc gctctccaac ttaatgtccg tgataatata caaatatgcg 360

tgttgtaaca cgtatagcac atataaaact aggtaaagtc cggaatagcc cactcgggtc 420

ctctcgggtc gggctcgggt cggcctcggg cctactcggg tttggccgaa gtagatggct 480

cagtgggctc gtgcgccgtc tgtcgcggcg cggtgtgggg ctacaagtgc gctggcggcg 540

gcgagcgcat gagcggcacg ggcaggtgcg gcgcgtactc gcgcgacagc acatagcgcg 600

gcgagcagca aaacgactct ttgcgcgctt gcagctccag cagcgagcac agtgagcgct 660

cgtacagccg ccactggtgc accacccagg aggctggaat taacaagaca ggtttaaata 720

aaaactacac aaaaaacaaa taccctgcct acatgcccac caagcacttc cacgtgacct 780

gggaaaacta gaaagccaat ttgaatgtac attttgatat ctaaattatg taattttgtt 840

attttgtatt aaatatgcat aacacattac aaattataac aaaatgacct tgttgatgtc 900

acaacgtaag aattggccgt cgtatcgcgt tatcacgttt ttcgatttaa atcgaggctt 960

taacgtagrc ttaggtacga aagaagcact ctaagcgaat taccttgacg ctgacgcttg 1020

```
cagcgttgag cgtaaaaact ataactgagt cacacgaact cgccgcgaaa aagctggcct     1080
aatacaagaa aacagagtga gtagaagttt tgtagtcact ctaatttctt tgtattacaa     1140
tttgtagcaa cgaattgtat tatctatatc cagatataga tatatgttac atgaatattc     1200
ctgtcaaatt gttacagawt gtcatcttaa aattagagcg tagctttgat tgtatggctg     1260
tycgtgtgac tttagagtca aaggaattcg ctcagagagt taagttkyra tgctagcact     1320
agcacttacc atgagatccg atgtgtgaga cacaatgtcc aacgaagctt atttacagga     1380
acagtcaccc cacagtccac aaaacacagc acttgaagaa catagtatca ttcgcaaaac     1440
aacttcatcg atgacgatag cacaccacta aaattattta tttcgctcag cattttccta     1500
caaaagaaaa acaaaataaa aatagtatca cttgcacatc actattaaat aaaatgtggt     1560
cacttttttt aaatttcgaa cttctccacc ttcgtccggt cactgtggaa atgaaatcac     1620
gactgggtta gtgatgttct gattcgtcgg acacaccact cttttatcaa cttagcaaat     1680
ttgtatgtgt gggtgtgtaa caatgttgtt aatgtgttat gacacattgt gttgtgtggg     1740
gaccgaactt gcaacgttgt agctccgtac attgtttgta aacggcaggc tacgttacta     1800
tacgtagtac gtaagcgacg taagcgtgac tcaacttctt atcgattaca gcgtctttat     1860
aaatgtaagt tatttataat acagtggaac ctcgataagg cgaaaataaa acgctgtctc     1920
gctccgctca caccagtgag agtgagagtg agagaagaac ctgaactcgc ggcgccttaa     1980
cggtccccag caagctcggt tgtaagtaaa cgatggatgg attgtgtgaa agaggatatg     2040
agaaagaaag gagtgagaag agaggaacat gttgtgccga ccccacataa cgtgggataa     2100
gagcaggagg aagaagagca agctaacgta gttacgctct cattttaaaa cgactagcta     2160
aattgctctg aaactttgta ctaacaatag gattaggcat atcggagtcg cctttaagag     2220
cttattaccc ctccgtcgaa ataccacggc caatagtcat atgtattgtt tggactgacg     2280
tttaactgac atatttgctc ctccccegta aaatcgttgt acagagaatt acagacaagg     2340
tgtttccagt tgttaaatcc tccaagtcta aggctgtaat tagtttatgt agcctcagat     2400
accaagtata aactaatttc agccctagac atacctcatg tcattgtatg tgcaaagttc     2460
cattacaatc caacacgcag ttttataatg agaacgaaac tccgtttgta tgtgaaattc     2520
agccgagctt accattgcta gttttaggaa taaggggtta aaatttgcaa attcggtcta     2580
agtgtgtgta aaaaacaaag gtcggtttcc gaacagaatt ttggtttctt tttgagtgtt     2640
tctaacggtt ttgagatgat ttaaatggaa ccttactttg agacttgctt aggttgcggt     2700
gggcgttttt catcgccatc cgaaatggag ttagccgccg tattcatcga tgcccagggc     2760
gtcggtgaac atctgctcga actcgaaatc ggccatatcc agggcgccgt agggggcgct     2820
atcgtgcggg gtgaatcccg gtcccgggct atcgccatcg cccagcatgt ccaggtcgaa     2880
gtcgtccagg gcatcggcgt gggccatcgc cacatcctcg ccatccaggt gcagctcatc     2940
```

gcccaggctc acgtcggtcg gcggggcggt cgacaggcgg cgggtgtgtc cggccggcag 3000
gaagctcagg cgcggggcgg ccaggcccgc ctcctccggg gcatcatcat ccggcagatc 3060
cagcaggccc tcgatggtgc tgccgtagtt gttcttggtg cgggcgcggc tgtaggcggg 3120
gcccgagccc gactcgcatt tcagttgctt ttccaatccg cagataatca gctccaagcc 3180
gaacaggaat gccggctcgg ctccttgatg atcgaacagc tcgattgcct gacgcagcag 3240
tgggggcatc gaatcggttg ttggggtctc gcgctcctct tttgcgactt gatgctcttg 3300
gtcctccagc acgcagccca gggtaaagtg accgacggcg ctcagagcgt agagagcatt 3360
ttccaggctg aagccttgct ggcacaggaa cgcgagctgg ttctccagtg tctcgtattg 3420
cttttcggtc gggcgcgtgc cgagatggac tttggcaccg tctcggtggg acagcagagc 3480
gcagcggaac gacttggcgt tattgcggag gaagtcctgc caggactcgc cttccaacgg 3540
gcaaaaatgc gtgtggtggc ggtcgagcat ctcgatggcc agggcatcca gcagcgcccg 3600
cttattcttc acgtgccagt agagggtggg ctgctccacg cccagcttct gcgccaactt 3660
gcgggtcgtc agtccctcaa tgccaacttc gttcaacagc tccaacgcgg agttgatgac 3720
tttggactta tccaggcggc tgaccatttt gcctggggac aacggaaatc gcacagtttg 3780
aacgttcgct tggcggcgcg gagactgcat tttggagaac acgtacatgt atcgggcgat 3840
aaaaaaaacy ttgtcattgt ttcattatga ccatgacaaa ttaaggtggg ttattttttg 3900
ctacttgaat ttaattgtcg aamagtaaaa aaaaacgatg caacttttttt atattgaaat 3960
tgactgatta caaaatgcag ccttgcttta taatagacac aacatacacg gaggaatgga 4020
ctaggaacat ctattttatg taaccttgta cataactaag acctaggtta aaataacgat 4080
gtgttaatat aatatataga gaacaatata aagcattttg taccatttgg cgttgaaact 4140
tttttgcagc aacgaagcgt ttggtatacg tactcgtaaa tggtgaccga aaagctggcg 4200
gcttcctcgc acaagtaatt ccgccagcat ccttagtaca atgcctgaag ggtatatttt 4260
agatttagct aatttattaa tttagtttag tatttgtaca gttactgcaa tacctctgta 4320
ccggaactcc agctgtgacc ttgacttgtt tcatgtgtca actcgccaca gtcccaactt 4380
gcttaccttc atcaatcttc cgtaacaaaa aagtctcggc gaatccctgg gctttgctcc 4440
aatctaagta ctctgcgttt tcgtaatcgt ttcgtgtccg cgagtttacc catattaata 4500
ctccgtacga cataagtgaa tggaagtgag cgtagtatac ggatcttaaa gtatcactat 4560
cagaagacgg cggcatcaac ggcggtggca gaataacagc gtccgttgct agaattcttt 4620
agcgcactct acaaaattat atcctggtgg attagccgag tcacattccc tttcaattcg 4680
tctgtaagca ttgttatgta cttaaattta aacttacctt cgtcaatctt ccgcgaggcc 4740
tcctccaggt cggagccggc gtagttgagg atgaccagca cgagcggcac cacctcccaa 4800

ctgttctagg gcagattgtt tagcttgttc agctgcgctt gtttatttgc ttagctttcg 4860
cttagcgacg tgttcacttt gcttgtttga attgaattgt cgctccgtag acgaagcgcc 4920
tctatttata ctccggcgct cgttttcgag tttaccactc cctatcagtg atagagaaaa 4980
gtgaaagtcg agtttaccac tccctatcag tgatagagaa aagtgaaagt cgagtttacc 5040
actccctatc agtgatagag aaaagtgaaa gtcgagttta ccactcccta tcagtgatag 5100
agaaaagtga aagtcgagtt taccactccc tatcagtgat agagaaaagt gaaagtcgaa 5160
acctggcgcg ccccggccat cgagaaagag agagagaaga gaagagagag aacattcgag 5220
aaagagagag agaagagaag agagagaaca tactccctat cagtgataga gaagtcccta 5280
tcagtgatag agatgtccct atcagtgata gagagttccc tatcagtgat agagacgtcc 5340
ctatcagtga tagagaagtc cctatcagtg atagagagat ccctatcagt gatagagatt 5400
tccctatcag tgatagagag gtccctatca gtgatagaga cttccctatc agtgatagag 5460
aaatccctat cagtgataga gacatcccta tcagtgatag agaactccct atcagtgata 5520
gagacctccc tatcagtgat agagatcgat gcggccgcga gcgccggagt ataaatagag 5580
gcgcttcgtc tacggagcga caattcaatt caaacaagca aagtgaacac gtcgctaagc 5640
gaaagctaag caaataaaca agcgcagctg aacaagctaa acaatctgca ggtaccctgg 5700
cggtaagttg atcaaaggaa acgcaaagtt ttcaagaaaa aacaaaacta atttgattta 5760
taacaccttt agaaagcggg gctagccacc atgggcagcg cctacagccg cgcccgtacc 5820
aagaacaact atggcagcac catcgaggga ctgctggacc tgccggatga cgatgccccg 5880
gaggaagccg gcctggccgc cccccgcctg agcttcctgc ccgccggaca cacgcgccgc 5940
ctgagcaccg ccccgccgac cgatgtgagc ctgggcgacg agctgcacct ggatggagag 6000
gatgtggcaa tggcccacgc cgacgccctg gacgatttcg acctggatat gctgggcgat 6060
ggagatagcc cgggaccggg cttcacgccc cacgatagcg ccccgtacgg cgccctggac 6120
atggccgact tcgagttcga gcaaatgttc accgacgcgc tgggcatcga tgagtatggc 6180
gggtaggttt aaactcgcgt taagatacat tgatgagttt ggacaaacca caactagaat 6240
gcagtgaaaa aaatgcttta tttgtgaaat ttgtgatgct attgctttat ttgtaaccat 6300
tataagctgc aataaacaag ttaacaacaa caattgcatt cattttatgt ttcaggttca 6360
gggggaggtg tgggaggttt tttaaagcaa gtaaaacctc tacaaatgtg gtatggctga 6420
ttatgatcag ttatctagat ccggtggatc ttacgggtcc tccaccttcc gctttttctt 6480
gggtcgagat ctcaggaaca ggtggtggcg gccctcggtg cgctcgtact gctccacgat 6540
ggtgtagtcc tcgttgtggg aggtgatgtc cagcttggcg tccacgtagt agtagccggg 6600
cagctgcacg ggcttcttgg ccatgtagat ggacttgaac tccaccaggt agtggccgcc 6660
gtccttcagc ttcagggcct tgtgggtctc gcccttcagc acgccgtcgc gggggtacag 6720

gcgctcggtg gaggcctccc agcccatggt cttcttctgc atcacggggc cgtcggaggg 6780
gaagttcacg ccgatgaact tcaccttgta gatgaagcag ccgtcctgca gggaggagtc 6840
ctgggtcacg gtcgccacgc cgccgtcctc gaagttcatc acgcgctccc acttgaagcc 6900
ctcggggaag gacagcttct tgtagtcggg gatgtcggcg gggtgcttca cgtacacctt 6960
ggagccgtac tggaactggg gggacaggat gtcccaggcg aagggcaggg ggccgccctt 7020
ggtcaccttc agcttcacgg tgttgtggcc ctcgtagggg cggccctcgc cctcgccctc 7080
gatctcgaac tcgtggccgt tcacggtgcc ctccatgcgc accttgaagc gcatgaactc 7140
ggtgatgacg ttctcggagg aggccatggt ggcgaccggt ttgcgcttct tcttgggtgg 7200
ggtgggatct cccatggtgg cctgaatctc aacttgcacc tgaaggtagt gcagcaagga 7260
tgagcaaaag ggaagaaccc agaaaagaac gggaaaactt accccaatta gaattgcttg 7320
tcgccgccag tgtcaacttg caactgaaac aatatccaac atgaacgtca atttatactg 7380
ccctaatggc gaacacgata acaatatttc ttttattatg ccctctaaaa ccaacgcggt 7440
tatcgtttat ttattcaaat tagatataga acatccgccg acatacaatg ttaatgcaaa 7500
aacgcgtttg gtgagcggat acgaaaacag tcggccgata aacattaatc tgaggtcgat 7560
aacaccgtcc ttgaacggaa cacgaggagc gtacgtgatc agctgcattc gcgcgccgcg 7620
cctttatcga gatttatttg catacaacaa gtacactgcg ccgttgggat ttgtggtaac 7680
gcgcacacat gcagagctgc aagtgtggca cattttgtct gtgcgcaaaa cctttgaagc 7740
caaaagtacg aggtccgtta cgggcatgct actagcgcac acggacaatg gacccgacaa 7800
attctacgcc aaggatttaa tgataatgtc gggcaacgta tccgttcatt ttatcaataa 7860
cctacaaaaa tgtcgcgcgc atcacaaaga catcgatata tttaaacatt tatgtcccga 7920
actgcaaatc gataatagtg ttgtgcaacc tcgagcgtcc gtttgattta acgtatagct 7980
tgcaaatgaa ttatttaatt atcaatcatg ttttacgcgt agaattctac ccgtaaagcg 8040
agtttagtta tgagccatgt gcaaaacatg acatcagctt ttatttttat aacaaatgac 8100
atcatttctt gattgtgttt tacacgtaga attctactcg taaagcgagt tcagttttga 8160
aaaacaaatg acatcatctt tttgattgtg ctttacaagt agaattctac ccgtaaatca 8220
agttcggttt tgaaaaacaa atgagtcata ttgtatgata tcatattgca aaacaaatga 8280
ctcatcaatc gatcgtgcgt tacacgtaga attctactcg taaagcgagt ttatgagccg 8340
tgtgcaaaac atgacatcat ctcgatttga aaaacaaatg acatcatcca ctgatcgtgc 8400
attacaagta gaattctact cgtaaagcca gttcggttat gagccgtgta caaaacatga 8460
catcagatta tgactcatac ttgattgtgt tttacgcgta gaattctact cgtaaagcca 8520
gttcaatttt aaaaacaaat gacatcatcc aaattaataa atgacaagca atgggtacca 8580

tgcggcctgg cctcgcgctc gcgcgactga cggtcgtaag caccccgcgta cgtgtccacc 8640
ccggtcacaa ccccttgtgt catgtcggcg accctacgcc cccaactgag agaactcaaa 8700
ggttacccca gttggggcac tactcccgaa aaccgcttct gacctgggaa aacgtgaagc 8760
cccggggcat ccgctgaggg ttgccgccgg ggcttcggtg tgtccgtcag tacttaatta 8820
acaccgaaat cgtaattcac ggcatcatta caaaatattt tgacgttttg gacctcgtcc 8880
ctaatgacac cataacggtg gccttgaagt atatttaacc ctagaaagat agtctgcgta 8940
aaattgacgc atgcattctt gaaatattgc tctctctttc taaatagcgc gaatccgtcg 9000
ctgtgcattt aggacatctc agtcgccgct tggagctccc gtgaggcgtg cttgtcaatg 9060
cggtaagtgt cactgatttt gaactataac gaccgcgtga gtcaaaatga cgcatgatta 9120
tcttttacgt gacttttaag atttaactca tacgataatt atattgttat ttcatgttct 9180
acttacgtga taacttatta tatatatatt ttcttgttat agatatcgtg actaatatat 9240
aataaaatgg gtagttcttt agacgatgag catatcctct ctgctcttct gcaaagcgat 9300
gacgagcttg ttggtgagga ttctgacagt gaaatatcag atcacgtaag tgaagatgac 9360
ctcgaggatc caagcttatc gatttcgaac cctcgaccgc cggagtataa atagaggcgc 9420
ttcgtctacg gagcgacaat tcaattcaaa caagcaaagt gaacacgtcg ctaagcgaaa 9480
gctaagcaaa taaacaagcg cagctgaaca agctaaacaa tcggggtacc gctagagtcg 9540
atcccacccc acccaagaag aagcgcaaac cggtaccatg gcctcctccg agaacgtcat 9600
caccgagttc atgcgcttca aggtgcgcat ggagggcacc gtgaacggcc acgagttcga 9660
gatcgagggc gagggcgagg gccgccccta cgagggccac aacaccgtga agctgaaggt 9720
gaccaagggc ggccccctgc ccttcgcctg ggacatcctg tccccccagt tccagtacgg 9780
ctccaaggtg tacgtgaagc accccgccga catccccgac tacaagaagc tgtccttccc 9840
cgagggcttc aagtgggagc gcgtgatgaa cttcgaggac ggcggcgtgg cgaccgtgac 9900
ccaggactcc tccctgcagg acggctgctt catctacaag gtgaagttca tcggcgtgaa 9960
cttcccctcc gacggccccg tgatgcagaa gaagaccatg ggctgggagg cctccaccga 10020
gcgcctgtac ccccgcgacg gcgtgctgaa gggcgagacc cacaaggccc tgaagctgaa 10080
ggacggcggc cactacctgg tggagttcaa gtccatctac atggccaaga agcccgtgca 10140
gctgcccggc tactactacg tggacgccaa gctggacatc acctcccaca acgaggacta 10200
caccatcgtg gagcagtacg agcgcaccga gggccgccac cacctgttcc tgtgatgatc 10260
ataatcagcc ataccacatt tgtagaggtt ttacttgctt taaaaaacct cccacacctc 10320
cccctgaacc tgaaacataa aatgaatgca attgttgttg ttaacttgtt tattgcagct 10380
tataatggtt acaaataaag caatagcatc acaaatttca caaataaagc atttttttca 10440
ctgcattcta gttgtggttt gtccaaactc atcaatgtat cttaacgcga gttaattacg 10500

gccgctcatt taaatctggc cggccgcaac cattgtggga accgtgcgat caaacaaacg 10560
cgagataccg gaagtactga aaaacagtcg ctccaggcca gtgggaacat cgatgttttg 10620
ttttgacgga ccccttactc tcgtctcata taaaccgaag ccagctaaga tggtatactt 10680
attatcatct tgtgatgagg atgcttctat caacgaaagt accggtaaac cgcaaatggt 10740
tatgtattat aatcaaacta aaggcggagt ggacacgcta gaccaaatgt gttctgtgat 10800
gacctgcagt aggaagacga ataggtggcc tatggcatta ttgtacggaa tgataaacat 10860
tgcctgcata aattctttta ttatatacag ccataatgtc agtagcaagg gagaaaaggt 10920
ccaaagtcgc aaaaaattta tgagaaacct ttacatgagc ctgacgtcat cgtttatgcg 10980
taagcgttta gaagctccta ctttgaagag atatttgcgc gataatatct ctaatatttt 11040
gccaaatgaa gtgcctggta catcagatga cagtactgaa gagccagtaa tgaaaaaacg 11100
tacttactgt acttactgcc cctctaaaat aaggcgaaag gcaaatgcat cgtgcaaaaa 11160
atgcaaaaaa gttatttgtc gagagcataa tattgatatg tgccaaagtt gtttctgact 11220
gactaataag tataatttgt ttctattatg tataagttaa gctaattact tattttataa 11280
tacaacatga ctgtttttaa agtacaaaat aagtttattt ttgtaaaaga gagaatgttt 11340
aaaagttttg ttactttata gaagaaattt tgagtttttg ttttttttta ataaataaat 11400
aaacataaat aaattgtttg ttgaatttat tattagtatg taagtgtaaa tataataaaa 11460
cttaatatct attcaaatta ataaataaac ctcgatatac agaccgataa aacacatgcg 11520
tcaattttac gcatgattat ctttaacgta cgtcacaata tgattatctt tctagggtta 11580
aataatagtt tctaattttt ttattattca gcctgctgtc gtgaataccg tatatctcaa 11640
cgctgtctgt gagattgtcg tattctagcc tttttagttt ttcgctcatc gacttgatat 11700
tgtccgacac attttcgtcg atttgcgttt tgatcaaaga cttgagcaga gacacgttaa 11760
tcaactgttc aaattgatcc atattaacga tatcaacccg atgcgtatat ggtgcgtaaa 11820
atatattttt taaccctctt atactttgca ctctgcgtta atacgcgttc gtgtacagac 11880
gtaatcatgt tttctttttt ggataaaact cctactgagt ttgacctcat attagaccct 11940
cacaagttgc aaaacgtggc attttttacc aatgaagaat ttaaagttat tttaaaaaat 12000
ttcatcacag atttaaagaa gaaccaaaaa ttaaattatt tcaacagttt aatcgaccag 12060
ttaatcaacg tgtacacaga cgcgtcggca aaaaacacgc agcccgacgt gttggctaaa 12120
attattaaat caacttgtgt tatagtcacg gatttgccgt ccaacgtgtt cctcaaaaag 12180
ttgaagacca acaagtttac ggacactatt aattatttga ttttgcccca cttcattttg 12240
tgggatcaca attttgttat attttaaaca aagcttggca ctggccgtcg ttttacaacg 12300
tcgtgactgg gaaaaccctg gcgttaccca acttaatcgc cttgcagcac atcccccttt 12360

cgccagctgg cgtaatagcg aagaggcccg caccgatcgc ccttcccaac agttgcgcag 12420
cctgaatggc gaatggcgcc tgatgcggta ttttctcctt acgcatctgt gcggtatttc 12480
acaccgcata tggtgcactc tcagtacaat ctgctctgat gccgcatagt taagccagcc 12540
ccgacacccg ccaacacccg ctgacgcgcc ctgacgggct tgtctgctcc cggcatccgc 12600
ttacagacaa gctgtgaccg tctccgggag ctgcatgtgt cagaggtttt caccgtcatc 12660
accgaaacgc gcgagacgaa agggcctcgt gatacgccta tttttatagg ttaatgtcat 12720
gataataatg gtttcttaga cgtcaggtgg cacttttcgg ggaaatgtgc gcggaacccc 12780
tatttgttta tttttctaaa tacattcaaa tatgtatccg ctcatgagac aataaccctg 12840
ataaatgctt caataatatt gaaaaaggaa gagtatgagt attcaacatt tccgtgtcgc 12900
ccttattccc ttttttgcgg cattttgcct tcctgttttt gctcacccag aaacgctggt 12960
gaaagtaaaa gatgctgaag atcagttggg tgcacgagtg ggttacatcg aactggatct 13020
caacagcggt aagatccttg agagttttcg ccccgaagaa cgttttccaa tgatgagcac 13080
ttttaaagtt ctgctatgtg gcgcggtatt atcccgtatt gacgccgggc aagagcaact 13140
cggtcgccgc atacactatt ctcagaatga cttggttgag tactcaccag tcacagaaaa 13200
gcatcttacg gatggcatga cagtaagaga attatgcagt gctgccataa ccatgagtga 13260
taacactgcg gccaacttac ttctgacaac gatcggagga ccgaaggagc taaccgcttt 13320
tttgcacaac atgggggatc atgtaactcg ccttgatcgt tgggaaccgg agctgaatga 13380
agccatacca aacgacgagc gtgacaccac gatgcctgta gcaatggcaa caacgttgcg 13440
caaactatta actggcgaac tacttactct agcttcccgg caacaattaa tagactggat 13500
ggaggcggat aaagttgcag gaccacttct gcgctcggcc cttccggctg gctggtttat 13560
tgctgataaa tctggagccg gtgagcgtgg gtctcgcggt atcattgcag cactggggcc 13620
agatggtaag ccctcccgta tcgtagttat ctacacgacg gggagtcagg caactatgga 13680
tgaacgaaat agacagatcg ctgagatagg tgcctcactg attaagcatt ggtaactgtc 13740
agaccaagtt tactcatata tactttagat tgatttaaaa cttcattttt aatttaaaag 13800
gatctaggtg aagatccttt ttgataatct catgaccaaa atcccttaac gtgagttttc 13860
gttccactga gcgtcagacc ccgtagaaaa gatcaaagga tcttcttgag atcctttttt 13920
tctgcgcgta atctgctgct tgcaaacaaa aaaaccaccg ctaccagcgg tggtttgttt 13980
gccggatcaa gagctaccaa ctctttttcc gaaggtaact ggcttcagca gagcgcagat 14040
accaaatact gtccttctag tgtagccgta gttaggccac cacttcaaga actctgtagc 14100
accgcctaca tacctcgctc tgctaatcct gttaccagtg gctgctgcca gtggcgataa 14160
gtcgtgtctt accgggttgg actcaagacg atagttaccg gataaggcgc agcggtcggg 14220
ctgaacgggg ggttcgtgca cacagcccag cttggagcga acgacctaca ccgaactgag 14280

atacctacag cgtgagcatt gagaaagcgc cacgcttccc gaagggagaa aggcggacag 14340
gtatccggta agcggcaggg tcggaacagg agagcgcacg agggagcttc caggggggaaa 14400
cgcctggtat ctttatagtc ctgtcgggtt tcgccacctc tgacttgagc gtcgattttt 14460
gtgatgctcg tcaggggggc ggagcctatg gaaaaacgcc agcaacgcgg cctttttacg 14520
gttcctggcc ttttgctggc cttttgctca catgttcttt cctgcgttat cccctgattc 14580
tgtggataac cgtattaccg cctttgagtg agctgatacc gctcgccgca gccgaacgac 14640
cgagcgcagc gagtcagtga gcgaggaagc ggaagagcgc ccaatacgca aaccgcctct 14700
ccccgcgcgt tggccgattc attaatgcag ctggcacgac aggtttcccg actggaaagc 14760
gggcagtgag cgcaacgcaa ttaatgtgag ttagctcact cattaggcac cccaggcttt 14820
acactttatg cttccggctc gtatgttgtg tggaattgtg agcggataac aatttcacac 14880
aggaaacagc tatgaccatg attacgaatt tcgacctgca ggcatgcaag cttgcatgcc 14940
tgcaggtcga cgctcgcgcg acttggtttg ccattcttta gcgcgcgtcg cgtcacacag 15000
cttggccaca atgtggtttt tgtcaaacga agattctatg acgtgtttaa agtttaggtc 15060
gagtaaagcg caaatctttt ttaaccctag aaagatagtc tgcgtaaaat tgacgcatgc 15120
attcttgaaa tattgctctc tctttctaaa tagcgcgaat ccgtcgctgt gcatttagga 15180
catctcagtc gccgcttgga gctcccgtga ggcgtgcttg tcaatgcggt aagtgtcact 15240
gattttgaac tataacgacc gcgtgagtca aaatgacgca tgattatctt ttacgtgact 15300
tttaagattt aactcatacg ataattatat tgttatttca tgttctactt acgtgataac 15360
ttattatata tatattttct tgttatagat atcgtgacta atatataata aaatgggtag 15420
ttctttagac gatgagcata tcctctctgc tcttctgcaa agcgatgacg agcttgttgg 15480
tgaggattct gacagtgaaa tatcagatca cgtaagtgaa gatgacgtcc agagcgatac 15540
agaagaagcg tttatagatg aggtacatga agtgcagcca acgtcaagcg gtagtgaaat 15600
attagacgaa caaaatgtta ttgaacaacc aggttcttca ttggcttcta acagaatctt 15660
gaccttgcca cagaggacta ttagaggtaa gaataaacat tgttggtcaa cttcaaagtc 15720
cacgaggcgt agccgagtct ctgcactgaa cattgtcaga tcggcccgct cgcccgggga 15780
actagttcaa ttagagacta attcaattag agctaattca attaggatcc aagcttatcg 15840
atttcgaacc ctcgaccgcc ggagtataaa tagaggcgct tcgtctacgg agcgacaatt 15900
caattcaaac aagcaaagtg aacacgtcgc taagcgaaag ctaagcaaat aaacaagcgc 15960
agctgaacaa gctaaacaat cggggtaccg ctagagtcga tcccacccca cccaagaaga 16020
agcgcaaacc ggtcgccacc atggccctgt ccaacaagtt catcggcgac gacatgaaga 16080
tgacctacca catggacggc tgcgtgaacg gccactactt caccgtgaag ggcgagggca 16140

gcggcaagcc ctacgagggc acccagacct ccaccttcaa ggtgaccatg gccaacggcg 16200
gcccccctggc cttctccttc gacatcctgt ccaccgtgtt catgtacggc aaccgctgct 16260
tcaccgccta ccccaccagc atgcccgact acttcaagca ggccttcccc gacggcatgt 16320
cctacgagag aaccttcacc tacgaggacg gcggcgtggc caccgccagc tgggagatca 16380
gcctgaaggg caactgcttc gagcacaagt ccaccttcca cggcgtgaac ttccccgccg 16440
acggccccgt gatggccaag aagaccaccg gctgggaccc ctccttcgag aagatgaccg 16500
tgtgcgacgg catcttgaag ggcgacgtga ccgccttcct gatgctgcag ggcggcggca 16560
actacagatg ccagttccac acctcctaca agaccaagaa gcccgtgacc atgcccccca 16620
accacgtggt ggagcaccgc atcgccagaa ccgacctgga caagggcggc aacagcgtgc 16680
agctgaccga gcacgccgtg gcccacatca cctccgtggt gcccttctcc ggactcagat 16740
cataatcagc cataccacat ttgtagaggt tttacttgct ttaaaaaacc tcccacacct 16800
ccccctgaac ctgaaacata aaatgaatgc aattgttgtt gttaacttgt ttattgcagc 16860
ttataatggt tacaaataaa gcaatagcat cacaaatttc acaaataaag catttttttc 16920
actgcattct agttgtggtt tgtccaaact catcaatgta tcttaccgcg gagtggacac 16980
gctagaccaa atgtgttctg tgatgacctg cagtaggaag acgaataggt ggcctatggc 17040
attattgtac ggaatgataa acattgcctg cataaattct tttattatat acagccataa 17100
tgtcagtagc aagggagaaa aggtccaaag tcgcaaaaaa tttatgagaa acctttacat 17160
gagcctgacg tcatcgttta tgcgtaagcg tttagaagct cctactttga agagatattt 17220
gcgcgataat atctctaata ttttgccaaa tgaagtgcct ggtacatcag atgacagtac 17280
tgaagagcca gtaatgaaaa aacgtactta ctgtacttac tgcccctcta aaataaggcg 17340
aaaggcaaat gcatcgtgca aaaaatgcaa aaaagttatt tgtcgagagc ataatattga 17400
tatgtgccaa agttgtttct gactgactaa taagtataat ttgtttctat tatgtataag 17460
ttaagctaat tacttatttt ataatacaac atgactgttt ttaaagtaca aaataagttt 17520
atttttgtaa aagagagaat gtttaaaagt tttgttactt tatagaagaa attttgagtt 17580
tttgtttttt tttaataaat aaataaacat aaataaattg tttgttgaat ttattattag 17640
tatgtaagtg taaatataat aaaacttaat atctattcaa attaataaat aaacctcgat 17700
atacagaccg ataaaacaca tgcgtcaatt ttacgcatga ttatctttaa cgtacgtcac 17760
aatatgatta tctttctagg g 17781

<210> 162
<211> 15482
<212> DNA
<213> artificial

<220>

<223> LA3570 plasmid sequence

<220>
<221> misc_feature
<222> (1875)..(1875)
<223> n is a, c, g, or t

<400> 162
gggcggccgt ttttcttgaa atattgctct ctctttctaa atagcgcgaa tccgtcgctg 60

tgcatttagg acatctcagt cgccgcttgg agctcccaaa cgcgccagtg gtagtacaca 120

gtactgtggg tgttcagttt gaaatcctct tgcttctcca ttgtctcggt tacctttggt 180

caaatccatg ggttctattg cctatatact cttgcgatta ccagtgattg cgctattagc 240

tattagatgg attgttggcc aaacttgtcg cttaagtggc tgggaattgt aaccgtaggc 300

ccgagtgtaa tgatccccca taaaaagttt tcgcaatgcc tttatttttt gttgcaaatc 360

tctctttatt ctgcggtatt cttcattatt gcggggatgg ggaaagtgtt tatatagaag 420

caacttacga ttgaacccaa atgcacctga caagcaaggt caaagggcca gatttttaaa 480

tatattattt agtcttagga ctctctattt gcaattaaat tactttgcta cctgagggtt 540

aaatcttccc cattgataat aataattcca ctatatgttc aattgggttt caccgcgctt 600

agttacatga cgagccctaa tgagccgtcg gtggtctata aactgtgcct tacaaatact 660

tgcaactctt ctcgttttga agtcagcaga gttattgcta attgctaatt gctaattgct 720

tttaactgat ttcttcgaaa ttggtgctat gtttatggcg ctattaacaa gtatgaatgt 780

caggtttaac caggggatgc ttaattgtgt tctcaacttc aaaggcagaa atgtttactc 840

ttgaccatgg gtttaggtat aatgttatca agctcctcga gttaacgtta cgttaacgtt 900

aacgttcgag gtcgactcta gggcctctct agatttacag gtctattttg agctctttgt 960

cagacactgt ttgcttgaaa ttcaagtctg tcagcacctt aaaaccaaaa ataaaaagaa 1020

taataaatga aatagtactt acttcccgcg gcgcaggttc gcatcgctac aagtgcgcgg 1080

gcggcgggga tgatctctgc gtggtaagcg gcagaggcaa caggtgtggc gcgtactcgg 1140

gcgcgatgac gtagcggggt gagcagcaca ccgagtacgt ccctttgcgc gcttgcagct 1200

ccaggagcga gcacagcgac cgctcgtaca tccgccactg gtggaccacc caatgtgcac 1260

ccaatgtgct gcaaggaagg cggggttaag tcgtcgagaa gtgatacaag aaatcggtct 1320

ttaaagtcgt aaggtccatt acctttaaaa atcgaaaacc cttaaactac tgtgtctaga 1380

aatctggacc ttacgaggtt aagtcgttag agaattgaaa gaaagcataa agaaactaga 1440

ccatatcatc gccttgtagc gaaaccacgt aagcgttttt ttgaaaatca aattaaaaac 1500

attctgatac gattttcttc aacaaaattt cattacaggt aaaaattaag accacraatt 1560

attgcctggg ttgaattgaa acaagcttgt ctattgtgtg gttttattaa caaaaatcac 1620

atccgaaggc gcttrtgtgg gtttcattat aaagccacga tatacagtct atacatttag 1680
ctgttcaagt tacaggtgaa tcgcaacctc caaggttaca agcggtataa aattwatatt 1740
gttaataatg tcaaatgtac caactatagt tttacattgg tcaaatgagc aatgtacggc 1800
cgtaaaatgg ccagtcgcag tgccagtaat gtagtttttt aaatccgtaa aaattaagtg 1860
ccatacyttt tttanctacc ttaaaataca aaaatattgg gaacmcacga acaccccaat 1920
aatagtgttt aaacagtcgt tgtcataaaa cgatatcaat aatctttgat gttataaaaa 1980
tatatgtttt tctttatttt aattgcccgg tagtcatgtt gtatacgagt attgtataaa 2040
gcaatcgttc tacaaatgac tcgttacgat gttcctgaga ttcctccata gcagtgagta 2100
gtaataaaaa gtcaattgta ccgcgatgaa atagataaaa tattattcta ccactcaccg 2160
aatagtccag cgtggcgacg acacaatagt ccttccatcg caaacagcaa cgcacagcaa 2220
aagtccacac aacacacagc acatacaaaa caaagtatca ttcgcaaaat aacttcatgg 2280
acgacgatag tacaccactt atattattaa tttcgctcag cattttccac cggtgttagc 2340
cgccgtactc atcgatgccc agggcgtcgg tgaacatctg ctcgaactcg aaatcggcca 2400
tatccagggc gccgtagggg gcgctatcgt gcggggtgaa tcccggtccc gggctatcgc 2460
catcgcccag catgtccagg tcgaagtcgt ccagggcatc ggcgtgggcc atcgccacat 2520
cctcgccatc caggtgcagc tcatcgccca ggctcacgtc ggtcggcggg gcggtcgaca 2580
ggcggcgggt gtgtccggcc ggcaggaagc tcaggcgcgg ggcggccagg cccgcctcct 2640
ccggggcatc atcatccggc agatccagca ggccctcgat ggtgctgccg tagttgttct 2700
tggtgcgggc gcggctgtag gcggggcccg agcccgactc gcatttcagt tgcttttcca 2760
atccgcagat aatcagctcc aagccgaaca ggaatgccgg ctcggctcct tgatgatcga 2820
acagctcgat tgcctgacgc agcagtgggg gcatcgaatc ggttgttggg gtctcgcgct 2880
cctcttttgc gacttgatgc tcttggtcct ccagcacgca gcccagggta aagtgaccga 2940
cggcgctcag agcgtagaga gcattttcca ggctgaagcc ttgctggcac aggaacgcga 3000
gctggttctc cagtgtctcg tattgctttt cggtcgggcg cgtgccgaga tggactttgg 3060
caccgtctcg gtgggacagc agagcgcagc ggaacgactt ggcgttattg cggaggaagt 3120
cctgccagga ctcgccttcc aacgggcaaa aatgcgtgtg gtggcggtcg agcatctcga 3180
tggccagggc atccagcagc gcccgcttat tcttcacgtg ccagtagagg gtgggctgct 3240
ccacgcccag cttctgcgcc aacttgcggg tcgtcagtcc ctcaatgcca acttcgttca 3300
acagctccaa cgcggagttg atgactttgg acttatccag gcggctgccc atggtggttt 3360
cggtccgtta gcgagtcgag ttcctcagct cgtggccatc gaagatgttc agattgtgct 3420
tcctcgcgta ctcgttgatg atcatcttcc ctggaaacat atgacgctag ctttacattc 3480
gcacagcggg gtatgaggaa ctgcatttat tacaatttat tatactatta ttataattcc 3540

cgtcgtcata attgtcgtcg gtcatgtcgt atcaggaggt gaaggatttg gtaggaagaa 3600
gagaggaatg gcgattactc caccgacaag agcgcagctc ttaaaaaaaa agagagataa 3660
ttcccgtgac cttaatataa gcatcatggc ttcataacct cgtgagaaaa cgcacataat 3720
ttcccgagaa atgcgtttcg gaggtgacct aaccagccca atacctgtgt tgtttgcctt 3780
cgggttggaa ggtcagatag gcattcaatt ctgtaatgaa ccggacctgt caaatcttca 3840
ggctaagtac agaaattata ccatcaaata aggtaacata attttgatca gatttcttta 3900
ttatttattt atctttagaa gacagagaga tgaggaggaa gggtgcagac aacattgcat 3960
cctacgtgca ctcaagaaca agtagaatgt ctacttgtat ttactaccta aaatacattt 4020
tattggacct cctagattta attacagttt tgaaatctct aacatctaaa ataatagccc 4080
cgggccttca attattgtaa aaggggaatg aatcttatgt tactataggt agtttcgcct 4140
cgagaggcat tcgcaacttg accgaacaaa cggtttcttc ctttagcgaa tgtattataa 4200
ttatccaaca cacaagactg cacgcagtac aagtaggtaa taatgcaata gattgacata 4260
aacggcaatt aacgaacgac agacgtacct accgcggtgt agagttgtag acctatgatt 4320
attcttcacg gagtttttta ttacaaactg tggtaaaacc tttataaacc accgtaatat 4380
acaagaataa agaacgaaac taattatgta taaacaactt atataaatac cactgctgga 4440
cgcagacgtc ccctcaatca actggacagg gaagatcgta ctccaccacg ctgcttcgtt 4500
acgggttggt agagaattaa ataaatgaat tgtatgaaaa aaaaaacgta agtaaacata 4560
taaaaaatgt aagttttcta tcaaaaactt cacctcgtat tcaaagaacg caaagaactt 4620
gtaatcaatc agtaattatc gtaccttcat caatttttcc agaagcctcg tcgaggccta 4680
gggcagattg tttagcttgt tcagctgcgc ttgtttattt gcttagcttt cgcttagcga 4740
cgtgttcact ttgcttgttt gaattgaatt gtcgctccgt agacgaagcg cctctattta 4800
tactccggcg ctcgttttcg agtttaccac tccctatcag tgatagagaa aagtgaaagt 4860
cgagtttacc actccctatc agtgatagag aaaagtgaaa gtcgagttta ccactcccta 4920
tcagtgatag agaaaagtga aagtcgagtt taccactccc tatcagtgat agagaaaagt 4980
gaaagtcgag tttaccactc cctatcagtg atagagaaaa gtgaaagtcg agtttaccac 5040
tccctatcag tgatagagaa aagtgaaagt cgagtttacc actccctatc agtgatagag 5100
aaaagtgaaa gtcgaaacct ggcgcgcccc ggccatcgag aaagagagag agaagagaag 5160
agagagaaca ttcgagaaag agagagagaa gagaagagag agaacatact ccctatcagt 5220
gatagagaag tccctatcag tgatagagat gtccctatca gtgatagaga gttccctatc 5280
agtgatagag acgtccctat cagtgataga gaagtcccta tcagtgatag agagatccct 5340
atcagtgata gagatttccc tatcagtgat agagaggtcc ctatcagtga tagagacttc 5400

cctatcagtg atagagaaat ccctatcagt gatagagaca tccctatcag tgatagagaa 5460

ctccctatca gtgatagaga cctccctatc agtgatagag atcgatgcgg ccgcgagcgc 5520

cggagtataa atagaggcgc ttcgtctacg gagcgacaat tcaattcaaa caagcaaagt 5580

gaacacgtcg ctaagcgaaa gctaagcaaa taaacaagcg cagctgaaca agctaaacaa 5640

tctgcaggta ccctggcggt aagttgatca aaggaaacgc aaagttttca agaaaaaaca 5700

aaactaattt gatttataac acctttagaa agcggggcta gccaccatgg gcagcgccta 5760

cagccgcgcc cgtaccaaga acaactatgg cagcaccatc gagggactgc tggacctgcc 5820

ggatgacgat gccccggagg aagccggcct ggccgccccc cgcctgagct tcctgcccgc 5880

cggacacacg cgccgcctga gcaccgcccc gccgaccgat gtgagcctgg gcgacgagct 5940

gcacctggat ggagaggatg tggcaatggc ccacgccgac gccctggacg atttcgacct 6000

ggatatgctg ggcgatggag atagcccggg accgggcttc acgccccacg atagcgcccc 6060

gtacggcgcc ctggacatgg ccgacttcga gttcgagcaa atgttcaccg acgcgctggg 6120

catcgatgag tatggcgggt aggtttaaac tcgcgttaag atacattgat gagtttggac 6180

aaaccacaac tagaatgcag tgaaaaaaat gctttatttg tgaaatttgt gatgctattg 6240

ctttatttgt aaccattata agctgcaata aacaagttaa caacaacaat tgcattcatt 6300

ttatgtttca ggttcagggg gaggtgtggg aggtttttta aagcaagtaa aacctctaca 6360

aatgtggtat ggctgattat gatcagttat ctagatccgg tggatcttac gggtcctcca 6420

ccttccgctt tttcttgggt cgagatctca ggaacaggtg gtggcggccc tcggtgcgct 6480

cgtactgctc cacgatggtg tagtcctcgt tgtgggaggt gatgtccagc ttggcgtcca 6540

cgtagtagta gccgggcagc tgcacgggct tcttggccat gtagatggac ttgaactcca 6600

ccaggtagtg gccgccgtcc ttcagcttca gggccttgtg ggtctcgccc ttcagcacgc 6660

cgtcgcgggg gtacaggcgc tcggtggagg cctcccagcc catggtcttc ttctgcatca 6720

cggggccgtc ggaggggaag ttcacgccga tgaacttcac cttgtagatg aagcagccgt 6780

cctgcaggga ggagtcctgg gtcacggtcg ccacgccgcc gtcctcgaag ttcatcacgc 6840

gctcccactt gaagccctcg gggaaggaca gcttcttgta gtcggggatg tcggcggggt 6900

gcttcacgta caccttggag ccgtactgga actgggggga caggatgtcc caggcgaagg 6960

gcagggggcc gcccttggtc accttcagct tcacggtgtt gtggccctcg taggggcggc 7020

cctcgccctc gccctcgatc tcgaactcgt ggccgttcac ggtgccctcc atgcgcacct 7080

tgaagcgcat gaactcggtg atgacgttct cggaggaggc catggtggcg accggtttgc 7140

gcttcttctt gggtggggtg ggatctccca tggtggcctg aatctcaact tgcacctgaa 7200

ggtagtgcag caaggatgag caaaagggaa gaacccagaa aagaacggga aaacttaccc 7260

caattagaat tgcttgtcgc cgccagtgtc aacttgcaac tgaaacaata tccaacatga 7320

acgtcaattt atactgccct aatggcgaac acgataacaa tatttctttt attatgccct 7380

ctaaaaccaa cgcggttatc gtttatttat tcaaattaga tatagaacat ccgccgacat 7440

acaatgttaa tgcaaaaacg cgtttggtga gcggatacga aaacagtcgg ccgataaaca 7500

ttaatctgag gtcgataaca ccgtccttga acggaacacg aggagcgtac gtgatcagct 7560

gcattcgcgc gccgcgcctt tatcgagatt tatttgcata caacaagtac actgcgccgt 7620

tgggatttgt ggtaacgcgc acacatgcag agctgcaagt gtggcacatt ttgtctgtgc 7680

gcaaaacctt tgaagccaaa agtacgaggt ccgttacggg catgctacta gcgcacacgg 7740

acaatggacc cgacaaattc tacgccaagg atttaatgat aatgtcgggc aacgtatccg 7800

ttcattttat caataaccta caaaaatgtc gcgcgcatca caaagacatc gatatattta 7860

aacatttatg tcccgaactg caaatcgata atagtgttgt gcaacctcga gcgtccgttt 7920

gatttaacgt atagcttgca aatgaattat ttaattatca atcatgtttt acgcgtagaa 7980

ttctacccgt aaagcgagtt tagttatgag ccatgtgcaa aacatgacat cagcttttat 8040

ttttataaca aatgacatca tttcttgatt gtgttttaca cgtagaattc tactcgtaaa 8100

gcgagttcag ttttgaaaaa caaatgacat catctttttg attgtgcttt acaagtagaa 8160

ttctacccgt aaatcaagtt cggttttgaa aaacaaatga gtcatattgt atgatatcat 8220

attgcaaaac aaatgactca tcaatcgatc gtgcgttaca cgtagaattc tactcgtaaa 8280

gcgagtttat gagccgtgtg caaaacatga catcatctcg atttgaaaaa caaatgacat 8340

catccactga tcgtgcatta caagtagaat tctactcgta aagccagttc ggttatgagc 8400

cgtgtacaaa acatgacatc agattatgac tcatacttga ttgtgtttta cgcgtagaat 8460

tctactcgta aagccagttc aattttaaaa acaaatgaca tcatccaaat taataaatga 8520

caagcaatgg gtaccatgcg gcctggcctc gcgctcgcgc gactgacggt cgtaagcacc 8580

cgcgtacgtg tccaccccgg tcacaacccc ttgtgtcatg tcggcgaccc tacgccccca 8640

actgagagaa ctcaaaggtt accccagttg ggcactact cccgaaaacc gcttctgacc 8700

tgggaaaacg tgaagccccg gggcatccgc tgagggttgc cgccggggct tcggtgtgtc 8760

cgtcagtact taattaacac cgaaatcgta attcacggca tcattacaaa atattttgac 8820

gttttggacc tcgtccctaa tgacaccata acggtggcct tgaagtatat ttaaccctag 8880

aaagatagtc tgcgtaaaat tgacgcatgc attcttgaaa tattgctctc tctttctaaa 8940

tagcgcgaat ccgtcgctgt gcatttagga catctcagtc gccgcttgga gctcccgtga 9000

ggcgtgcttg tcaatgcggt aagtgtcact gattttgaac tataacgacc gcgtgagtca 9060

aaatgacgca tgattatctt ttacgtgact tttaagattt aactcatacg ataattatat 9120

tgttatttca tgttctactt acgtgataac ttattatata tatattttct tgttatagat 9180

atcgtgacta atatataata aaatgggtag ttctttagac gatgagcata tcctctctgc 9240
tcttctgcaa agcgatgacg agcttgttgg tgaggattct gacagtgaaa tatcagatca 9300
cgtaagtgaa gatgacgtcc aggaaatctg gccggccgca accattgtgg gaaccgtgcg 9360
atcaaacaaa cgcgagatac cggaagtact gaaaaacagt cgctccaggc cagtgggaac 9420
atcgatgttt tgttttgacg gaccccttac tctcgtctca tataaaccga agccagctaa 9480
gatggtatac ttattatcat cttgtgatga ggatgcttct atcaacgaaa gtaccggtaa 9540
accgcaaatg gttatgtatt ataatcaaac taaaggcgga gtggacacgc tagaccaaat 9600
gtgttctgtg atgacctgca gtaggaagac gaataggtgg cctatggcat tattgtacgg 9660
aatgataaac attgcctgca taaattcttt tattatatac agccataatg tcagtagcaa 9720
gggagaaaag gtccaaagtc gcaaaaaatt tatgagaaac ctttacatga gcctgacgtc 9780
atcgtttatg cgtaagcgtt tagaagctcc tactttgaag agatatttgc gcgataatat 9840
ctctaatatt ttgccaaatg aagtgcctgg tacatcagat gacagtactg aagagccagt 9900
aatgaaaaaa cgtacttact gtacttactg cccctctaaa ataaggcgaa aggcaaatgc 9960
atcgtgcaaa aaatgcaaaa aagttatttg tcgagagcat aatattgata tgtgccaaag 10020
ttgtttctga ctgactaata agtataattt gtttctatta tgtataagtt aagctaatta 10080
cttattttat aatacaacat gactgttttt aaagtacaaa ataagtttat ttttgtaaaa 10140
gagagaatgt ttaaaagttt tgttacttta tagaagaaat tttgagtttt tgtttttttt 10200
taataaataa ataaacataa ataaattgtt tgttgaattt attattagta tgtaagtgta 10260
aatataataa aacttaatat ctattcaaat taataaataa acctcgatat acagaccgat 10320
aaaacacatg cgtcaatttt acgcatgatt atctttaacg tacgtcacaa tatgattatc 10380
tttctagggt taaataatag tttctaattt ttttattatt cagcctgctg tcgtgaatac 10440
cgtatatctc aacgctgtct gtgagattgt cgtattctag cctttttagt tttcgctca 10500
tcgacttgat attgtccgac acattttcgt cgatttgcgt tttgatcaaa gacttgagca 10560
gagacacgtt aatcaactgt tcaaattgat ccatattaac gatatcaacc cgatgcgtat 10620
atggtgcgta aaatatattt tttaaccctc ttatactttg cactctgcgt taatacgcgt 10680
tcgtgtacag acgtaatcat gttttctttt ttggataaaa ctcctactga gtttgacctc 10740
atattagacc ctcacaagtt gcaaaacgtg gcattttttta ccaatgaaga atttaaagtt 10800
attttaaaaa atttcatcac agatttaaag aagaaccaaa aattaaatta tttcaacagt 10860
ttaatcgacc agttaatcaa cgtgtacaca gacgcgtcgg caaaaaacac gcagcccgac 10920
gtgttggcta aaattattaa atcaacttgt gttatagtca cggatttgcc gtccaacgtg 10980
ttcctcaaaa agttgaagac caacaagttt acggacacta ttaattattt gattttgccc 11040
cacttcattt tgtgggatca caattttgtt atattttaaa caaagcttgg cactggccgt 11100

cgttttacaa cgtcgtgact gggaaaaccc tggcgttacc caacttaatc gccttgcagc 11160

acatccccct ttcgccagct ggcgtaatag cgaagaggcc cgcaccgatc gcccttccca 11220

acagttgcgc agcctgaatg gcgaatggcg cctgatgcgg tattttctcc ttacgcatct 11280

gtgcggtatt tcacaccgca tatggtgcac tctcagtaca atctgctctg atgccgcata 11340

gttaagccag ccccgacacc cgccaacacc cgctgacgcg ccctgacggg cttgtctgct 11400

cccggcatcc gcttacagac aagctgtgac cgtctccggg agctgcatgt gtcagaggtt 11460

ttcaccgtca tcaccgaaac gcgcgagacg aaagggcctc gtgatacgcc tatttttata 11520

ggttaatgtc atgataataa tggtttctta gacgtcaggt ggcacttttc ggggaaatgt 11580

gcgcggaacc cctatttgtt tatttttcta aatacattca aatatgtatc cgctcatgag 11640

acaataaccc tgataaatgc ttcaataata ttgaaaaagg aagagtatga gtattcaaca 11700

tttccgtgtc gcccttattc ccttttttgc ggcattttgc cttcctgttt ttgctcaccc 11760

agaaacgctg gtgaaagtaa aagatgctga agatcagttg ggtgcacgag tgggttacat 11820

cgaactggat ctcaacagcg gtaagatcct tgagagtttt cgccccgaag aacgttttcc 11880

aatgatgagc acttttaaag ttctgctatg tggcgcggta ttatcccgta ttgacgccgg 11940

gcaagagcaa ctcggtcgcc gcatacacta ttctcagaat gacttggttg agtactcacc 12000

agtcacagaa aagcatctta cggatggcat gacagtaaga gaattatgca gtgctgccat 12060

aaccatgagt gataacactg cggccaactt acttctgaca acgatcggag gaccgaagga 12120

gctaaccgct tttttgcaca acatggggga tcatgtaact cgccttgatc gttgggaacc 12180

ggagctgaat gaagccatac caaacgacga gcgtgacacc acgatgcctg tagcaatggc 12240

aacaacgttg cgcaaactat taactggcga actacttact ctagcttccc ggcaacaatt 12300

aatagactgg atggaggcgg ataaagttgc aggaccactt ctgcgctcgg cccttccggc 12360

tggctggttt attgctgata aatctggagc cggtgagcgt gggtctcgcg gtatcattgc 12420

agcactgggg ccagatggta agccctcccg tatcgtagtt atctacacga cggggagtca 12480

ggcaactatg gatgaacgaa atagacagat cgctgagata ggtgcctcac tgattaagca 12540

ttggtaactg tcagaccaag tttactcata tatactttag attgatttaa aacttcattt 12600

ttaatttaaa aggatctagg tgaagatcct ttttgataat ctcatgacca aaatccctta 12660

acgtgagttt tcgttccact gagcgtcaga ccccgtagaa aagatcaaag gatcttcttg 12720

agatcctttt tttctgcgcg taatctgctg cttgcaaaca aaaaaaccac cgctaccagc 12780

ggtggtttgt ttgccggatc aagagctacc aactcttttt ccgaaggtaa ctggcttcag 12840

cagagcgcag ataccaaata ctgtccttct agtgtagccg tagttaggcc accacttcaa 12900

gaactctgta gcaccgccta catacctcgc tctgctaatc ctgttaccag tggctgctgc 12960

cagtggcgat aagtcgtgtc ttaccgggtt ggactcaaga cgatagttac cggataaggc 13020
gcagcggtcg ggctgaacgg ggggttcgtg cacacagccc agcttggagc gaacgaccta 13080
caccgaactg agatacctac agcgtgagca ttgagaaagc gccacgcttc ccgaagggag 13140
aaaggcggac aggtatccgg taagcggcag ggtcggaaca ggagagcgca cgagggagct 13200
tccaggggga aacgcctggt atctttatag tcctgtcggg tttcgccacc tctgacttga 13260
gcgtcgattt ttgtgatgct cgtcaggggg gcggagccta tggaaaaacg ccagcaacgc 13320
ggccttttta cggttcctgg ccttttgctg gccttttgct cacatgttct ttcctgcgtt 13380
atcccctgat tctgtggata accgtattac cgcctttgag tgagctgata ccgctcgccg 13440
cagccgaacg accgagcgca gcgagtcagt gagcgaggaa gcggaagagc gcccaatacg 13500
caaaccgcct ctccccgcgc gttggccgat tcattaatgc agctggcacg acaggtttcc 13560
cgactggaaa gcgggcagtg agcgcaacgc aattaatgtg agttagctca ctcattaggc 13620
accccaggct ttacacttta tgcttccggc tcgtatgttg tgtggaattg tgagcggata 13680
acaatttcac acaggaaaca gctatgacca tgattacgaa tttcgacctg caggcatgca 13740
agcttgcatg cctgcaggtc gacgctcgcg cgacttggtt tgccattctt tagcgcgcgt 13800
cgcgtcacac agcttggcca caatgtggtt tttgtcaaac gaagattcta tgacgtgttt 13860
aaagtttagg tcgagtaaag cgcaaatctt ttttaaccct agaaagatag tctgcgtaaa 13920
attgacgcat gcattcttga aatattgctc tctctttcta aatagcgcga atccgtcgct 13980
gtgcatttag gacatctcag tcgccgcttg gagctcccgt gaggcgtgct tgtcaatgcg 14040
gtaagtgtca ctgattttga actataacga ccgcgtgagt caaaatgacg catgattatc 14100
ttttacgtga cttttaagat ttaactcata cgataattat attgttattt catgttctac 14160
ttacgtgata acttattata tatatatttt cttgttatag atatcgtgac taatatataa 14220
taaaatgggt agttctttag acgatgagca tatcctctct gctcttctgc aaagcgatga 14280
cgagcttgtt ggtgaggatt ctgacagtga aatatcagat cacgtaagtg aagatgacgt 14340
ccagagcgat acagaagaag cgtttataga tgaggtacat gaagtgcagc caacgtcaag 14400
cggtagtgaa atattagacg aacaaaatgt tattgaacaa ccaggttctt cattggcttc 14460
taacagaatc ttgaccttgc cacagaggac tattagaggt aagaataaac attgttggtc 14520
aacttcaaag tccacgaggc gtagccgagt ctctgcactg aacattgtca gatcggcccg 14580
gcggagtgga cacgctagac caaatgtgtt ctgtgatgac ctgcagtagg aagacgaata 14640
ggtggcctat ggcattattg tacggaatga taaacattgc ctgcataaat tcttttatta 14700
tatacagcca taatgtcagt agcaagggag aaaaggtcca aagtcgcaaa aaatttatga 14760
gaaaccttta catgagcctg acgtcatcgt ttatgcgtaa gcgtttagaa gctcctactt 14820
tgaagagata tttgcgcgat aatatctcta atattttgcc aaatgaagtg cctggtacat 14880

cagatgacag tactgaagag ccagtaatga aaaaacgtac ttactgtact tactgcccct 14940

ctaaaataag gcgaaaggca aatgcatcgt gcaaaaaatg caaaaaagtt atttgtcgag 15000

agcataatat tgatatgtgc caaagttgtt tctgactgac taataagtat aatttgtttc 15060

tattatgtat aagttaagct aattacttat tttataatac aacatgactg tttttaaagt 15120

acaaaataag tttatttttg taaaagagag aatgtttaaa agttttgtta ctttatagaa 15180

gaaattttga gtttttgttt ttttttaata aataaataaa cataaataaa ttgtttgttg 15240

aatttattat tagtatgtaa gtgtaaatat aataaaactt aatatctatt caaattaata 15300

aataaacctc gatatacaga ccgataaaac acatgcgtca attttacgca tgattatctt 15360

taacgtacgt cacaatatga ttatctttct agggttaaaa tgaatgtaag cactttatta 15420

acgaaatctt tgggaatatt tcgctcatca gcattttatt tgagcaggag tccgagatgc 15480

cc 15482

<210> 163
<211> 12
<212> DNA
<213> Artificial Sequence

<220>
<223> LA3077 flanking sequence

<400> 163
aacgaagttggt 12

<210> 164
<211> 12
<212> DNA
<213> Artificial Sequence

<220>
<223> LA3077 flanking sequence

<400> 164
aggtattgaggg 12

<210> 165
<211> 12
<212> DNA
<213> Artificial Sequence

<220>
<223> LA1188 flanking sequence

<400> 165
ctactggcacgt 12

<210> 166
<211> 12
<212> DNA

<213> Artificial Sequence

<220>
<223> LA1188 flanking sequence

<400> 166
aggtgaagaata 12

<210> 167
<211> 12
<212> DNA
<213> Artificial Sequence

<220>
<223> native flanking sequence

<400> 167
cgtagatttggt 12

<210> 168
<211> 12
<212> DNA
<213> Artificial Sequence

<220>
<223> native flanking sequence

<400> 168
aggtgaaggctc 12

<210> 169
<211> 12
<212> DNA
<213> Artificial

<220>
<223> LA3097 flanking sequence

<400> 169
agccaccatggt 12

<210> 170
<211> 12
<212> DNA
<213> artificial

<220>
<223> LA3097 flanking sequence

<400> 170
aggtcagccgcc 12